(19) Europäisches Patentamt
European Patent Office
Office européen des brevets

(11) EP 2 810 944 A1

(12) EUROPEAN PATENT APPLICATION
published in accordance with Art. 153(4) EPC

(43) Date of publication:
**10.12.2014 Bulletin 2014/50**

(21) Application number: **13742843.9**

(22) Date of filing: **30.01.2013**

(51) Int Cl.:
***C07D 471/04*** *(2006.01)* ***A61K 31/519*** *(2006.01)*
***A61P 31/18*** *(2006.01)*

(86) International application number:
**PCT/JP2013/052077**

(87) International publication number:
**WO 2013/115265 (08.08.2013 Gazette 2013/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.01.2012 JP 2012018142**
**11.05.2012 JP 2012109638**

(71) Applicant: **Toyama Chemical Co., Ltd.**
**Shinjuku-ku**
**Tokyo 160-0023 (JP)**

(72) Inventors:
- **KAWAI, Hyouei**
  **Toyama-shi,**
  **Toyama (JP)**
- **MURATA, Daigo**
  **Toyama-shi,**
  **Toyama (JP)**
- **SUZUMURA, Yuko**
  **Toyama-shi,**
  **Toyama (JP)**

(74) Representative: **Blodig, Wolfgang**
**Wächtershäuser & Hartz**
**Patentanwaltspartnerschaft mbB**
**Weinstrasse 8**
**80333 München (DE)**

# (54) HETEROCYCLIC COMPOUND HAVING ANTI-HIV ACTIVITY

(57) A heterocyclic compound represented by the general formula (in the formula, $R^1$, $R^2$ and $R^3$ may be the same or different, and each represents a hydrogen atom, a halogen atom, or this general formula ($X^1$-$Y^1$-$R^4$) (in the formula: $X^1$ represents this general formula ($NR^5$) (in the formula, $R^5$ represents a hydrogen atom, etc.) or the like; $Y^1$ represents an optionally substituted $C_{1-6}$ alkylene group or the like; and $R^4$ represents an optionally substituted aryl group or the like), and Z represents a nitrogen atom or this general formula ($CR^6$) (in the formula, $R^6$ represents a hydrogen atom, a halogen atom, or an optionally substituted $C_{1-12}$ alkyl group or the like)), or a salt thereof, exhibits excellent anti-HIV activity and is useful as an anti-HIV agent.

EP 2 810 944 A1

## Description

[Technical Field]

The present invention relates to a heterocyclic compound or a salt thereof which is useful as an anti-HIV agent.

[Background Art]

Human immunodeficiency virus (HIV), which is a retrovirus, infects immunocytes and causes acquired immunodeficiency syndrome (AIDS). The current number of HIV-infected people has reached tens of millions of people in the world. Particularly, the recent spread of infection in Asian and African regions is a matter of importance.

HIV has, in its capsid, RNA genes encoding virus-specific enzymes (e.g., protease, reverse transcriptase and integrase) and invades a target cell via CD4 and a chemokine receptor present on immunocomponent cell surface. After uncoating, HIV releases a complex of RNA and integrase, etc. into the cytoplasm and reversely transcribe its genetic information to double-stranded proviral DNA by use of its own reverse transcriptase. In addition, the HIV-specific integrase incorporates the proviral DNA into the DNA of the target cell. The proviral DNA thus incorporated in the target cell is transcribed into an RNA strand, which then yields a viral protein efficiently by viral regulatory gene products such as Tat and Rev. The viral protein is combined with separately formed viral RNA to bud from the membrane surface of the host cell. The virus emigrated from the cell repetitively infects immunocomponent cells (e.g., CD4-positive T cells and macrophages) and proliferates therein, resulting in immunodeficiency in the host.

A large number of anti-HIV agents have been developed so far. Examples of anti-HIV agents targeting viral factors include: as nucleic acid reverse transcriptase inhibitors, zidovudine, didanosine, lamivudine, sanilvudine, abacavir, tenofovir and emtricitabine; as non-nucleic acid reverse transcriptase inhibitors, nevirapine, delavirdine, efavirenz and etravirine; as protease inhibitors, indinavir, saquinavir, ritonavir, nelfinavir, lopinavir, atazanavir, fosamprenavir, tipranavir and darunavir; as an integrase inhibitor, raltegravir; and as a fusion inhibitor, enfuvirtide. Also, an anti-HIV agent targeting host factors includes an entry inhibitor maraviroc.

Multidrug therapy using these drugs in combination is used in the treatment of AIDS. For example, the combination of any one of non-nucleic acid reverse transcriptase inhibitors, protease inhibitors and integrase inhibitors with two nucleic acid reverse transcriptase inhibitors is recommended (Non Patent Document 1).

HIV, however, becomes resistant easily. Once a virus becomes resistant, the virus is less sensitive to drugs of the same category (Non Patent Document 2). In addition, a limited number of drugs are used in the multidrug therapy and cannot always produce satisfactory effects.

For example, lactic acidosis caused by the nucleic acid reverse transcriptase inhibitors, and abnormal lipid metabolism and complications (e.g., diabetes mellitus) caused by the protease inhibitors have been reported as adverse reactions of the drugs currently used (Non Patent Documents 3 and 4).

[Citation List]

[Non Patent Document]

[Non Patent Document 1] Antibiotics & Chemotherapy, 2011, Vol. 27, p. 25-31
[Non Patent Document 2] Antibiotics & Chemotherapy, 2004, Vol. 20, p. 58-68
[Non Patent Document 3] Antibiotics & Chemotherapy, 2009, Vol. 25, p. 40-53
[Non Patent Document 4] Antibiotics & Chemotherapy, 2009, Vol. 25, p. 54-61

[Summary of Invention]

[Technical Problem]

There is a strong demand for a compound having excellent anti-HIV activity.

[Solution to Problem]

The present inventors have conducted diligent studies to solve the problems mentioned above and consequently completed the present invention by finding that a heterocyclic compound represented by the general formula [1]:

[Formula 1]

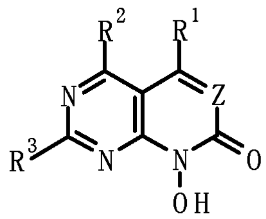

[1

wherein $R^1$ and $R^2$ are the same or different and represent a hydrogen atom, a halogen atom, a cyano group, a nitro group, an optionally substituted $C_{1-12}$ alkyl group, an optionally substituted $C_{2-12}$ alkenyl group, an optionally substituted $C_{2-12}$ alkynyl group, an optionally substituted $C_{1-6}$ alkoxyl group, an optionally substituted acyl group, an optionally substituted $C_{1-6}$ alkylamino group, an optionally substituted di($C_{1-6}$ alkyl)amino group, an optionally substituted $C_{1-6}$ alkylthio group, an optionally substituted $C_{1-6}$ alkylsulfonyl group, an optionally substituted arylsulfonyl group, an optionally substituted $C_{3-8}$cycloalkyl group, an optionally substituted aryl group, an optionally substituted heterocyclic group, an optionally substituted carbamoyl group, an optionally protected amino group, an optionally protected carboxyl group, an optionally protected hydroxyl group or a general formula $X^1$-$Y^1$-$R^4$ (wherein $X^1$ represents a general formula $NR^5$ (wherein $R^5$ represents a hydrogen atom, an optionally substituted $C_{1-6}$ alkyl group or an imino protecting group), an oxygen atom or a sulfur atom; $Y^1$ represents an optionally substituted $C_{1-6}$ alkylene group, an optionally substituted $C_{2-6}$ alkenylene group, an optionally substituted $C_{2-6}$ alkynylene group or a bond; $R^4$ represents an optionally substituted $C_{3-8}$ cycloalkyl group, an optionally substituted aryl group or an optionally substituted heterocyclic group);

$R^3$ represents a hydrogen atom, a halogen atom, a cyano group, a nitro group, an optionally substituted $C_{1-6}$ alkyl group, an optionally substituted $C_{1-6}$ alkoxyl group, an amino group, an optionally protected carboxyl group or an optionally protected hydroxyl group;

Z represents a nitrogen atom or a general formula $CR^6$ (wherein $R^6$ represents a hydrogen atom, a halogen atom, a cyano group, a nitro group, an optionally substituted $C_{1-12}$ alkyl group, an optionally substituted $C_{2-12}$ alkenyl group, an optionally substituted $C_{2-12}$ alkynyl group, an optionally substituted $C_{1-6}$ alkoxyl group, an optionally substituted acyl group, an optionally substituted $C_{1-6}$ alkylamino group, an optionally substituted di($C_{1-6}$ alkyl)amino group, an optionally substituted $C_{1-6}$ alkylthio group, an optionally substituted $C_{1-6}$ alkylsulfonyl group, an optionally substituted arylsulfonyl group, an optionally substituted $C_{3-8}$cycloalkyl group, an optionally substituted aryl group, an optionally substituted heterocyclic group, an optionally substituted carbamoyl group, an optionally protected amino group, an optionally protected carboxyl group, an optionally protected hydroxyl group or a general formula $X^2$-$Y^2$-$R^7$ (wherein $X^2$ represents a general formula $NR^8$ (wherein $R^8$ represents a hydrogen atom, an optionally substituted $C_{1-6}$ alkyl group or an imino protecting group), an oxygen atom or a sulfur atom; $Y^2$ represents an optionally substituted $C_{1-6}$ alkylene group, an optionally substituted $C_{2-6}$ alkenylene group, an optionally substituted $C_{2-6}$ alkynylene group or a bond; $R^7$ represents an optionally substituted $C_{3-8}$cycloalkyl group, an optionally substituted aryl group or an optionally substituted heterocyclic group)),

or a salt thereof has excellent anti-HIV activity and is useful as an anti-HIV agent.

[Advantageous Effects of Invention]

The heterocyclic compound of the present invention or the salt thereof has excellent anti-HIV activity and as such, is useful as an anti-HIV agent. In another aspect, the heterocyclic compound of the present invention or the salt thereof is excellent in safety and kinetics and is useful as an anti-HIV agent.

[Description of Embodiments]

Hereinafter, the present invention will be described in detail.

In the present invention, each term is defined as described below, unless otherwise specified.

A halogen atom means a fluorine atom, a chlorine atom, a bromine atom or an iodine atom.

A $C_{1-12}$ alkyl group means a linear or branched $C_{1-12}$ alkyl group such as methyl, ethyl, propyl, isopropyl, butyl, sec-butyl, isobutyl, tert-butyl, pentyl, isopentyl, hexyl, heptyl and octyl groups.

A $C_{1-6}$ alkyl group means a linear or branched $C_{1-6}$ alkyl group such as methyl, ethyl, propyl, isopropyl, butyl, sec-butyl, isobutyl, tert-butyl, pentyl, isopentyl and hexyl groups.

A $C_{2-12}$ alkenyl group means a linear or branched $C_{2-12}$ alkenyl group such as vinyl, allyl, propenyl, isopropenyl,

butenyl, isobutenyl, 1,3-butadienyl, pentenyl, hexenyl, heptenyl and octenyl groups.

A $C_{2-6}$ alkenyl group means a linear or branched $C_{2-6}$ alkenyl group such as vinyl, allyl, propenyl, isopropenyl, butenyl, isobutenyl, 1,3-butadienyl, pentenyl and hexenyl groups.

A $C_{2-12}$ alkynyl group means a linear or branched $C_{2-12}$ alkynyl group such as ethynyl, propynyl, butynyl, pentynyl, hexynyl, heptynyl and octynyl groups.

A $C_{3-8}$ cycloalkyl group means a $C_{3-8}$ cycloalkyl group such as cyclopropyl, cyclobutyl, cyclopentyl and cyclohexyl groups.

An aryl group means a phenyl, naphthyl, indanyl, indenyl or tetrahydronaphthyl group, etc.

An ar-$C_{1-6}$ alkyl group means an ar-$C_{1-6}$ alkyl group such as benzyl, diphenylmethyl, trityl, phenethyl and naphthylmethyl groups.

A $C_{1-6}$ alkylene group means a linear or branched $C_{1-6}$ alkylene group such as methylene, ethylene, propylene, butylene and hexylene groups.

A $C_{2-6}$ alkenylene group means a linear or branched $C_{2-6}$ alkenylene group such as vinylene, propenylene, butenylene and pentenylene groups.

A $C_{2-6}$ alkynylene group means a linear or branched $C_{2-6}$ alkynylene group such as ethynylene, propynylene, butynylene and pentynylene groups.

A $C_{1-6}$ alkoxy group means a linear or branched $C_{1-6}$ alkyloxy group such as methoxy, ethoxy, propoxy, isopropoxy, butoxy, isobutoxy, sec-butoxy, tert-butoxy, pentyloxy and hexyloxy groups.

An aryloxy group means a phenoxy or naphthyloxy group, etc.

A $C_{1-6}$ alkoxy-$C_{1-6}$ alkyl group means a $C_{1-6}$ alkyloxy-$C_{1-6}$ alkyl group such as methoxymethyl and 1-ethoxyethyl groups.

An ar-$C_{1-6}$ alkoxy-$C_{1-6}$ alkyl group means an ar-$C_{1-6}$ alkyloxy-$C_{1-6}$ alkyl group such as benzyloxymethyl and phenethyloxymethyl groups.

A $C_{2-12}$ alkanoyl group means a linear or branched $C_{2-12}$ alkanoyl group such as acetyl, propionyl, valeryl, isovaleryl and pivaloyl groups.

An aroyl group means a benzoyl or naphthoyl group, etc.

A heterocyclic carbonyl group means a nicotinoyl, thenoyl, pyrrolidinocarbonyl or furoyl group, etc.

An ($\alpha$-substituted) aminoacetyl group means an ($\alpha$-substituted) aminoacetyl group with an optionally protected N terminus derived from an amino acid (examples thereof include amino acids such as glycine, alanine, valine, leucine, isoleucine, serine, threonine, cysteine, methionine, aspartic acid, glutamic acid, asparagine, glutamine, arginine, lysine, histidine, hydroxylysine, phenylalanine, tyrosine, tryptophan, proline and hydroxyproline).

An acyl group means a formyl group, a succinyl group, a glutaryl group, a maleoyl group, a phthaloyl group, a $C_{2-12}$ alkanoyl group, an aroyl group, a heterocyclic carbonyl group or an ($\alpha$-substituted) aminoacetyl group, etc.

An acyl-$C_{1-6}$ alkyl group means an acyl-$C_{1-6}$ alkyl group such as acetylmethyl, benzoylmethyl and 1-benzoylethyl groups.

An acyloxy-$C_{1-6}$ alkyl group means an acyloxy-$C_{1-6}$ alkyl group such as acetoxymethyl, propionyloxymethyl, pivaloyloxymethyl, benzoyloxymethyl and 1-(benzoyloxy)ethyl groups.

A $C_{1-6}$ alkoxycarbonyl group means a linear or branched $C_{1-6}$ alkyloxycarbonyl group such as methoxycarbonyl, ethoxycarbonyl, isopropoxycarbonyl, tert-butoxycarbonyl and 1,1-dimethylpropoxycarbonyl groups.

An ar-$C_{1-6}$ alkoxycarbonyl group means an ar-$C_{1-6}$ alkyloxycarbonyl group such as benzyloxycarbonyl and phenethyloxycarbonyl groups.

An aryloxycarbonyl group means a phenyloxycarbonyl or naphthyloxycarbonyl group, etc.

A $C_{1-6}$ alkylamino group means a linear or branched $C_{1-6}$ alkylamino group such as methylamino, ethylamino, propylamino, isopropylamino, butylamino, sec-butylamino, tert-butylamino, pentylamino and hexylamino groups.

A di($C_{1-6}$ alkyl)amino group means a linear or branched di($C_{1-6}$ alkyl)amino group such as dimethylamino, diethylamino, dipropylamino, diisopropylamino, dibutylamino, di(tert-butyl)amino, dipentylamino, dihexylamino, (ethyl)(methyl)amino and (methyl)(propyl)amino groups.

A $C_{1-6}$ alkylthio group means a $C_{1-6}$ alkylthio group such as methylthio, ethylthio and propylthio groups.

A $C_{1-6}$ alkylsulfonyl group means a $C_{1-6}$ alkylsulfonyl group such as methylsulfonyl, ethylsulfonyl and propylsulfonyl groups.

An arylsulfonyl group means a benzenesulfonyl, p-toluenesulfonyl or naphthalenesulfonyl group, etc.

A $C_{1-6}$ alkylsulfonyloxy group means a $C_{1-6}$ alkylsulfonyloxy group such as methylsulfonyloxy and ethylsulfonyloxy groups.

An arylsulfonyloxy group means a benzenesulfonyloxy or p-toluenesulfonyloxy group, etc.

A silyl group means a trimethylsilyl, triethylsilyl or tributylsilyl group, etc.

A nitrogen-containing monocyclic heterocyclic group means a nitrogen-containing monocyclic heterocyclic group that contains only a nitrogen atom as heteroatom(s) forming the ring, such as azetidinyl, pyrrolidinyl, pyrrolinyl, pyrrolyl, piperidyl, tetrahydropyridyl, pyridyl, homopiperidinyl, octahydroazocinyl, imidazolidinyl, imidazolinyl, imidazolyl,

pyrazolidinyl, pyrazolinyl, pyrazolyl, piperazinyl, pyrazinyl, pyridazinyl, pyrimidinyl, homopiperazinyl, triazolyl and tetrazolyl groups.

An oxygen-containing monocyclic heterocyclic group means a tetrahydrofuranyl, furanyl, tetrahydropyranyl or pyranyl group, etc.

A sulfur-containing monocyclic heterocyclic group means a thienyl group, etc.

A nitrogen- and oxygen-containing monocyclic heterocyclic group means a nitrogen- and oxygen-containing monocyclic heterocyclic group that contains only a nitrogen atom and an oxygen atom as heteroatoms forming the ring, such as oxazolyl, isoxazolyl, oxadiazolyl and morpholinyl groups.

A nitrogen- and sulfur-containing monocyclic heterocyclic group means a nitrogen- and sulfur-containing monocyclic heterocyclic group that contains only a nitrogen atom and a sulfur atom as heteroatoms forming the ring, such as thiazolyl, isothiazolyl, thiadiazolyl, thiomorpholinyl, 1-oxidothiomorpholinyl and 1,1-dioxidothiomorpholinyl groups.

A monocyclic heterocyclic group means a nitrogen-containing monocyclic heterocyclic group, an oxygen-containing monocyclic heterocyclic group, a sulfur-containing monocyclic heterocyclic group, a nitrogen- and oxygen-containing monocyclic heterocyclic group, or a nitrogen- and sulfur-containing monocyclic heterocyclic group.

A nitrogen-containing bicyclic heterocyclic group means a nitrogen-containing bicyclic heterocyclic group that contains only a nitrogen atom as heteroatom(s) forming the ring, such as indolinyl, indolyl, isoindolinyl, isoindolyl, benzimidazolyl, indazolyl, benzotriazolyl, quinolyl, tetrahydroquinolinyl, quinolyl, tetrahydroisoquinolinyl, isoquinolinyl, quinolizinyl, cinnolinyl, phthalazinyl, quinazolinyl, dihydroquinoxalinyl, quinoxalinyl, naphthyridinyl, purinyl, pteridinyl and quinuclidinyl groups.

An oxygen-containing bicyclic heterocyclic group means an oxygen-containing bicyclic heterocyclic group that contains only an oxygen atom as heteroatom(s) forming the ring, such as 2,3-dihydrobenzofuranyl, benzofuranyl, isobenzofuranyl, chromanyl, chromenyl, isochromanyl, 1,3-benzodioxolyl, 1,3-benzodioxanyl and 1,4-benzodioxanyl groups.

A sulfur-containing bicyclic heterocyclic group means a sulfur-containing bicyclic heterocyclic group that contains only a sulfur atom as heteroatom(s) forming the ring, such as 2,3-dihydrobenzothienyl and benzothienyl groups.

A nitrogen- and oxygen-containing bicyclic heterocyclic group means a nitrogen-and oxygen-containing bicyclic heterocyclic group that contains only a nitrogen atom and an oxygen atom as heteroatoms forming the ring, such as benzoxazolyl, benzisoxazolyl, benzoxadiazolyl, benzomorpholinyl, dihydropyranopyridyl, dihydrodioxinopyridyl and dihydropyridooxazinyl groups.

A nitrogen- and sulfur-containing bicyclic heterocyclic group means a nitrogen-and sulfur-containing bicyclic heterocyclic group that contains a nitrogen atom and a sulfur atom as heteroatoms forming the ring, such as benzothiazolyl, benzisothiazolyl and benzothiadiazolyl groups.

A bicyclic heterocyclic group means a nitrogen-containing bicyclic heterocyclic group, an oxygen-containing bicyclic heterocyclic group, a sulfur-containing bicyclic heterocyclic group, a nitrogen- and oxygen-containing bicyclic heterocyclic group, or a nitrogen-and sulfur-containing bicyclic heterocyclic group.

A heterocyclic group means a monocyclic heterocyclic group or a bicyclic heterocyclic group.

Examples of a leaving group include a halogen atom, a $C_{1-6}$ alkylsulfonyloxy group and an arylsulfonyloxy group. The $C_{1-6}$ alkylsulfonyloxy group or the arylsulfonyloxy group may have a substituent.

An amino protecting group includes all groups that may be used as usual protecting groups for the amino group. Examples thereof include groups described in W. Greene et al., Protective Groups in Organic Synthesis, 4th edition, p. 696-926, 2007, John Wiley & Sons, INC. Specifically, an ar-$C_{1-6}$ alkyl group, a $C_{1-6}$ alkoxy-$C_{1-6}$ alkyl group, an acyl group, a $C_{1-6}$ alkoxycarbonyl group, an ar-$C_{1-6}$ alkoxycarbonyl group, an aryloxycarbonyl group, a $C_{1-6}$ alkylsulfonyl group, an arylsulfonyl group or a silyl group is included.

An imino protecting group includes all groups that may be used as usual protecting groups for the imino group. Examples thereof include groups described in W. Greene et al., Protective Groups in Organic Synthesis, 4th edition, p. 696-868, 2007, John Wiley & Sons, INC. Specifically, an ar-$C_{1-6}$ alkyl group, a $C_{1-6}$ alkoxy-$C_{1-6}$ alkyl group, an acyl group, a $C_{1-6}$ alkoxycarbonyl group, an ar-$C_{1-6}$ alkoxycarbonyl group, an aryloxycarbonyl group, a $C_{1-6}$ alkylsulfonyl group, an arylsulfonyl group or a silyl group is included.

A hydroxyl protecting group includes all groups that may be used as usual protecting groups for the hydroxyl group. Examples thereof include groups described in W. Greene et al., Protective Groups in Organic Synthesis, 4th edition, p. 16-299, 2007, John Wiley & Sons, INC. Specifically, a $C_{1-6}$ alkyl group, a $C_{2-6}$ alkenyl group, an ar-$C_{1-6}$ alkyl group, a $C_{1-6}$ alkoxy-$C_{1-6}$ alkyl group, an ar-$C_{1-6}$ alkoxy-$C_{1-6}$ alkyl group, an acyl group, a $C_{1-6}$ alkoxycarbonyl group, an ar-$C_{1-6}$ alkoxycarbonyl group, a $C_{1-6}$ alkylsulfonyl group, an arylsulfonyl group, a silyl group, a tetrahydrofuranyl group or a tetrahydropyranyl group, etc., is included.

A carboxyl protecting group includes all groups that may be used as usual protecting groups for the carboxyl group. Examples thereof include groups described in W. Greene et al., Protective Groups in Organic Synthesis, 4th edition, p. 533-643, 2007, John Wiley & Sons, INC. Specifically, a $C_{1-6}$ alkyl group, a $C_{2-6}$ alkenyl group, an aryl group, an ar-$C_{1-6}$ alkyl group, a $C_{1-6}$ alkoxy-$C_{1-6}$ alkyl group, an ar-$C_{1-6}$ alkoxy-$C_{1-6}$ alkyl group, an acyl-$C_{1-6}$ alkyl group, an

acyloxy-$C_{1-6}$ alkyl group or a silyl group is included.

Examples of aliphatic hydrocarbons include pentane, hexane or cyclohexane, etc.

Examples of halogenated hydrocarbons include methylene chloride, chloroform or dichloroethane, etc.

Examples of alcohols include methanol, ethanol, propanol, 2-propanol, butanol or 2-methyl-2-propanol, etc.

Examples of ethers include diethyl ether, diisopropyl ether, dioxane, tetrahydrofuran, anisole, ethylene glycol dimethyl ether, diethylene glycol dimethyl ether or diethylene glycol diethyl ether, etc.

Examples of ketones include acetone, 2-butanone or 4-methyl-2-pentanone, etc.

Examples of esters include methyl acetate, ethyl acetate, propyl acetate or butyl acetate, etc.

Examples of amides include N,N-dimethylformamide, N,N-dimethylacetamide, or 1-methyl-2-pyrrolidone, etc.

Examples of aromatic hydrocarbons include benzene, toluene or xylene, etc.

Examples of sulfoxides include dimethyl sulfoxide, etc.

Examples of nitriles include acetonitrile, etc.

Examples of a salt of the compound of the general formula [1] can include usually known salts of basic groups such as an amino group or acidic groups such as a hydroxyl or carboxyl group.

Examples of the salts of basic groups include, for example: salts with mineral acids such as hydrochloric acid, hydrobromic acid, nitric acid and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid and trifluoroacetic acid; and salts with sulfonic acids such as methanesulfonic acid, benzenesulfonic acid, p-toluenesulfonic acid, mesitylenesulfonic acid and naphthalenesulfonic acid.

Examples of the salts of acidic groups include, for example: salts with alkali metals such as sodium and potassium; salts with alkaline earth metals such as calcium and magnesium; ammonium salts; and salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine and N,N'-dibenzylethylenediamine.

Among the above-mentioned salts, as a preferable salt, a pharmacologically acceptable salt is included.

The $C_{1-12}$ alkyl group, the $C_{2-12}$ alkenyl group, the $C_{2-12}$ alkynyl group, the $C_{1-6}$ alkoxy group, the acyl group, the $C_{1-6}$ alkylamino group, the di($C_{1-6}$ alkyl)amino group, the $C_{1-6}$ alkylthio group and the $C_{1-6}$ alkylsulfonyl group represented by $R^1$, $R^2$ and $R^6$ may each be substituted by one or more groups selected from a halogen atom, a cyano group, a nitro group, a $C_{1-6}$ alkoxy group, a $C_{1-6}$ alkylamino group, a di($C_{1-6}$ alkyl)amino group, a $C_{3-8}$ cycloalkyl group, an aryl group, a heterocyclic group, an optionally $C_{1-6}$ alkyl group-substituted carbamoyl group, an optionally protected amino group, an optionally protected carboxyl group, an optionally protected hydroxyl group and an oxo group.

The arylsulfonyl group, the $C_{3-8}$ cycloalkyl group, the aryl group and the heterocyclic group represented by $R^1$, $R^2$ and $R^6$ may each be substituted by one or more groups selected from a halogen atom, a cyano group, a nitro group, a $C_{1-12}$ alkyl group, an optionally halogen atom-substituted $C_{2-12}$ alkenyl group, an optionally halogen atom-substituted $C_{2-12}$ alkynyl group, an optionally halogen atom-substituted $C_{1-6}$ alkoxy group, an acyl group, a $C_{1-6}$ alkylamino group, a di($C_{1-6}$ alkyl)amino group, a $C_{1-6}$ alkylthio group, a $C_{1-6}$ alkylsulfonyl group, an arylsulfonyl group, a $C_{3-8}$ cycloalkyl group, an aryl group, a heterocyclic group, an optionally $C_{1-6}$ alkyl group-substituted carbamoyl group, an optionally protected amino group, an optionally protected carboxyl group, an optionally protected hydroxyl group and an oxo group.

The carbamoyl group represented by $R^1$, $R^2$ and $R^6$ may be substituted by one or more groups selected from an optionally halogen atom-substituted $C_{1-12}$ alkyl group, an optionally halogen atom-substituted $C_{2-12}$ alkenyl group, an optionally halogen atom-substituted $C_{2-12}$ alkynyl group, an acyl group, an ar-$C_{1-6}$ alkyl group, a $C_{3-8}$ cycloalkyl group, an aryl group and a heterocyclic group.

The $C_{1-6}$ alkyl group and the $C_{1-6}$ alkoxy group represented by $R^3$ may each be substituted by one or more groups selected from a halogen atom, a cyano group, a nitro group, a $C_{1-6}$ alkoxy group, an optionally $C_{1-6}$ alkyl group-substituted carbamoyl group, an optionally protected amino group, an optionally protected carboxyl group, an optionally protected hydroxyl group and an oxo group.

The $C_{3-8}$ cycloalkyl group, the aryl group and the heterocyclic group represented by $R^4$ and $R^7$ may each be substituted by one or more groups selected from a halogen atom, a cyano group, a nitro group, a $C_{1-12}$ alkyl group optionally substituted by one or more groups selected from Substituent group A, a $C_{2-12}$ alkenyl group optionally substituted by one or more groups selected from Substituent group A, a $C_{2-12}$ alkynyl group optionally substituted by one or more groups selected from Substituent group A, a $C_{1-6}$ alkoxy group optionally substituted by one or more groups selected from Substituent group A, an aryloxy group, an acyl group, a $C_{1-6}$ alkylamino group, a di($C_{1-6}$ alkyl)amino group, a $C_{1-6}$ alkylthio group, a $C_{1-6}$ alkylsulfonyl group, an arylsulfonyl group, a $C_{3-8}$ cycloalkyl group, an aryl group, a heterocyclic group, an optionally $C_{1-6}$ alkyl group-substituted carbamoyl group, an optionally $C_{1-6}$ alkyl group-substituted sulfamoyl group, an optionally protected amino group, an optionally protected carboxyl group, an optionally protected hydroxyl group and an oxo group.

Substituent group A:

a halogen atom, a $C_{1-6}$ alkoxy group, a $C_{1-6}$ alkylamino group, a di($C_{1-6}$ alkyl)amino group, an optionally protected amino

group, an optionally protected carboxyl group and an optionally protected hydroxyl group.

The $C_{1-6}$ alkyl group represented by $R^5$ and $R^8$ may be substituted by one or more groups selected from a halogen atom, a cyano group, a nitro group, a $C_{1-6}$ alkoxy group, an optionally $C_{1-6}$ alkyl group-substituted carbamoyl group, an optionally protected amino group, an optionally protected carboxyl group, an optionally protected hydroxyl group and an oxo group.

The $C_{1-6}$ alkylene group, the $C_{2-6}$ alkenylene group and the $C_{2-6}$ alkynylene group represented by $Y^1$ and $Y^2$ may each be substituted by one or more groups selected from a halogen atom, a cyano group, a nitro group, a $C_{1-6}$ alkoxy group, an aryl group, an optionally $C_{1-6}$ alkyl group-substituted carbamoyl group, an optionally protected amino group, an optionally protected carboxyl group, an optionally protected hydroxyl group and an oxo group.

The groups mentioned above may each be further substituted by one or more groups selected from a halogen atom, a cyano group, a nitro group, a $C_{1-12}$ alkyl group, a $C_{2-12}$ alkenyl group, a $C_{2-12}$ alkynyl group, a $C_{1-6}$ alkoxy group, an acyl group, a $C_{1-6}$ alkylamino group, a di($C_{1-6}$ alkyl)amino group, a $C_{1-6}$ alkylthio group, a $C_{1-6}$ alkylsulfonyl group, an arylsulfonyl group, a $C_{3-8}$ cycloalkyl group, an aryl group, a heterocyclic group, a carbamoyl group, an amino group, a carboxyl group and a hydroxyl group.

Preferable examples of the compound of the general formula [1] of the present invention include the following compounds.

The compound, wherein Z represents a general formula $CR^6$ (wherein $R^6$ represents a hydrogen atom, a halogen atom, a cyano group, a nitro group, an optionally substituted $C_{1-12}$ alkyl group, an optionally substituted $C_{2-12}$ alkenyl group, an optionally substituted $C_{2-12}$ alkynyl group, an optionally substituted $C_{1-6}$ alkoxyl group, an optionally substituted acyl group, an optionally substituted $C_{1-6}$ alkylamino group, an optionally substituted di($C_{1-6}$ alkyl)amino group, an optionally substituted $C_{1-6}$ alkylthio group, an optionally substituted $C_{1-6}$ alkylsulfonyl group, an optionally substituted arylsulfonyl group, an optionally substituted $C_{3-8}$ cycloalkyl group, an optionally substituted aryl group, an optionally substituted heterocyclic group, an optionally substituted carbamoyl group, an optionally protected amino group, an optionally protected carboxyl group, an optionally protected hydroxyl group or a general formula $X^2$-$Y^2$-$R^7$ (wherein $X^2$ represents a general formula $NR^8$ (wherein $R^8$ represents a hydrogen atom, an optionally substituted $C_{1-6}$ alkyl group or an imino protecting group), an oxygen atom or a sulfur atom; $Y^2$ represents an optionally substituted $C_{1-6}$ alkylene group, an optionally substituted $C_{2-6}$ alkenylene group, an optionally substituted $C_{2-6}$ alkynylene group or a bond; $R^7$ represents an optionally substituted $C_{3-8}$ cycloalkyl group, an optionally substituted aryl group or an optionally substituted heterocyclic group)), is preferable.

The compound, wherein $R^1$ represents a general formula $X^1$-$Y^1$-$R^4$ (wherein $X^1$ represents a general formula $NR^5$ (wherein $R^5$ represents a hydrogen atom, an optionally substituted $C_{1-6}$ alkyl group or an imino protecting group), an oxygen atom or a sulfur atom; $Y^1$ represents an optionally substituted $C_{1-6}$ alkylene group, an optionally substituted $C_{2-6}$ alkenylene group, an optionally substituted $C_{2-6}$ alkynylene group or a bond; $R^4$ represents an optionally substituted $C_{3-8}$ cycloalkyl group, an optionally substituted aryl group or an optionally substituted heterocyclic group), is preferable.

The compound, wherein $R^2$ and $R^6$ are the same or different and represent a hydrogen atom, a halogen atom, a cyano group, a nitro group, an optionally substituted $C_{1-12}$ alkyl group, an optionally substituted $C_{1-6}$ alkoxyl group, an optionally substituted $C_{1-6}$ alkylamino group, an optionally substituted di($C_{1-6}$ alkyl)amino group, an optionally substituted $C_{1-6}$ alkylthio group, an optionally substituted aryl group, an optionally substituted heterocyclic group, an optionally substituted carbamoyl group, an optionally protected amino group, an optionally protected carboxyl group or an optionally protected hydroxyl group, is preferable.

The compound, wherein $R^2$ and $R^6$ are the same or different and represent a hydrogen atom, an optionally substituted $C_{1-6}$ alkyl group, an optionally substituted aryl group, an optionally substituted heterocyclic group, an optionally protected amino group or an optionally protected hydroxyl group, is more preferable.

The compound, wherein $R^2$ represents a hydrogen atom or an optionally substituted $C_{1-6}$ alkyl group, is further preferable, and the compound, wherein $R^2$ represents an optionally substituted $C_{1-6}$ alkyl group, is still further preferable.

The compound, wherein $R^6$ represents a hydrogen atom, is further preferable.

The compound, wherein $R^3$ represents a hydrogen atom, a halogen atom, an optionally substituted $C_{1-12}$ alkyl group, an optionally substituted $C_{1-6}$ alkoxy group or an amino group, is preferable.

The compound, wherein $R^3$ represents a hydrogen atom or a halogen atom, is more preferable.

The compound, wherein $R^3$ represents a hydrogen atom, is further preferable.

The compound, wherein $R^4$ represents an optionally substituted aryl group or an optionally substituted heterocyclic group, is preferable.

The compound, wherein $X^1$ represents a general formula $NR^5$ (wherein $R^5$ represents a hydrogen atom, an optionally substituted $C_{1-6}$ alkyl group or an imino protecting group), is preferable.

The compound, wherein $Y^1$ represents an optionally substituted $C_{1-6}$ alkylene group or a bond, is preferable.

The compound, wherein $X^1$ represents NH; and $Y^1$ represents an optionally substituted $C_{1-6}$ alkylene group, is more preferable.

The compound, wherein $R^5$ represents a hydrogen atom, is more preferable.

The compound, wherein $X^2$ represents a general formula $NR^8$ (wherein $R^8$ represents a hydrogen atom, an optionally substituted $C_{1-6}$ alkyl group or an imino protecting group), is preferable.

The compound, wherein $Y^2$ represents an optionally substituted $C_{1-6}$ alkylene group or a bond, is preferable.

The compound, wherein $X^2$ represents NH; and $Y^2$ represents an optionally substituted $C_{1-6}$ alkylene group, is more preferable.

The compound, wherein $R^8$ represents a hydrogen atom, is more preferable.

The compound, wherein $R^7$ represents an optionally substituted aryl group, is preferable.

In another aspect, preferable examples of the compound of the general formula [1] of the present invention include the following compounds.

The compound, wherein $R^1$ represents a general formula $X^1$-$Y^1$-$R^4$ (wherein $X^1$ represents NH; $Y^1$ represents an optionally substituted $C_{1-6}$ alkylene group; and $R^4$ represents an optionally substituted aryl group or an optionally substituted heterocyclic group); $R^2$ represents a hydrogen atom or an optionally substituted $C_{1-6}$ alkyl group; $R^3$ represents a hydrogen atom; and Z represents CH, is preferable.

The compound, wherein $R^1$ represents a general formula $X^1$-$Y^1$-$R^4$ (wherein $X^1$ represents NH; $Y^1$ represents an optionally substituted $C_{1-6}$ alkylene group; and $R^4$ represents an optionally substituted aryl group or an optionally substituted heterocyclic group); $R^2$ represents an optionally substituted $C_{1-6}$ alkyl group; $R^3$ represents a hydrogen atom; and Z represents CH, is more preferable.

Examples of preferable compounds according to the present invention include

8-hydroxy-4-methyl-5-(((2-methylpyridin-3-yl)methyl)amino)pyrido[2,3-d]pyrimidin-7(8H)-one,
5-((((5-fluoropyridin-3-yl)methyl)amino)-8-hydroxy-4-methylpyrido[2,3-d]pyrimidin-7(8H)-one,
8-hydroxy-4-methyl-5-((1-(pyridin-3-yl)ethyl)amino)pyrido[2,3-d]pyrimidin-7(8H)-one,
8-hydroxy-4-methyl-5-(((2-(morpholin-4-yl)pyridin-3-yl)methyl)amino)pyrido[2,3-d]pyrimidin-7(8H)-one,
8-hydroxy-4-methyl-5-(((pyridin-3-yl)methyl)amino)pyrido[2,3-d]pyrimidin-7(8H)-one,
8-hydroxy-5-((2-hydroxybenzyl)amino)-4-methylpyrido[2,3-d]pyrimidin-7(8H)-one,
5-(((3,4-dihydroxybenzyl)amino)-8-hydroxy-4-methylpyrido[2,3-d]pyrimidin-7(8H)-one,
8-hydroxy-5-((3-hydroxybenzyl)amino)-4-methylpyrido[2,3-d]pyrimidin-7(8H)-one,
N-(2-(((8-hydroxy-4-methyl-7-oxo-7,8-dihydropyrido[2,3-d]pyrimidin-5-yl)amino)methyl)phenyl)methanesulfonamide,
8-hydroxy-5-((4-(2-hydroxyethoxy)benzyl)amino)-4-methylpyrido[2,3-d]pyrimidin-7(8H)-one, and
5-((((6-aminopyridin-3-yl)methyl)amino)-8-hydroxy-4-methylpyrido[2,3-d]pyrimidin-7(8H)-one.

The compound of the general formula [1] or the salt thereof may have isomers (e.g., optical isomers, geometric isomers and tautomers). In this case, these isomers may be used. Alternatively, the compound of the general formula [1] or the salt thereof may be any of solvates, hydrates, and crystals in various forms. In this case, these solvates, hydrates, and crystals in various forms may be used.

The compound represented by the general formula [1] or the salt thereof exhibits excellent safety and pharmacokinetics. The safety and the pharmacokinetics are evaluated by various tests and can be evaluated by various tests selected from, for example, cytotoxicity test, hERG test, repeated dose toxicity test, cytochrome P450 (CYP) activity inhibition test, metabolism-dependent inhibition test, *in vivo* mouse micronucleus test, *in vivo* rat liver UDS test, metabolic stability test, and pharmacokinetics tests in mice.

Next, a method for producing the compound of the present invention will be described.

The compound of the present invention is produced by the combination of methods known per se in the art and can be produced according to, for example, a production method shown below.

[Production Method 1]

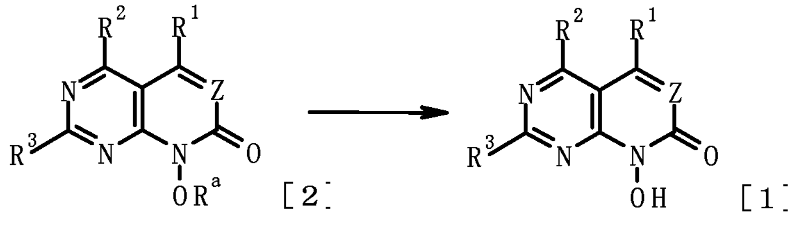

wherein $R^a$ represents a hydroxyl protecting group; and $R^1$, $R^2$, $R^3$ and Z are as defined above.

The compound of the general formula [1] can be produced by deprotecting a compound of the general formula [2]. This reaction can be carried out by, for example, a method described in Protective Groups in Organic Synthesis, 4th edition, p. 16-299, 2007, John Wiley & Sons, INC.

A compound of the general formula [1] wherein $R^a$ is, for example, an ar-$C_{1-6}$ alkyl group can be produced by reducing the compound of the general formula [2] in the presence of a metal catalyst.

The solvent used in this reaction is not particularly limited as long as the solvent has no adverse effect on the reaction. Examples of the solvent include aliphatic hydrocarbons, halogenated hydrocarbons, alcohols, ethers, ketones, esters, amides, aromatic hydrocarbons, sulfoxides, nitriles and water, etc. These solvents may be used as a mixture.

Preferable examples of the solvent include alcohols and ethers.

Examples of the metal catalyst used in this reaction include: metallic palladiums such as palladium-carbon and palladium black; palladium salts such as palladium oxide and palladium hydroxide; nickel metals such as Raney nickel; and platinum salts such as platinum oxide.

The amount of the metal catalyst used can be 0.001 to 5 times (W/W), preferably 0.01 to 1 times (W/W) the amount of the compound of the general formula [2].

Examples of the reducing agent include: hydrogen; formic acid; formates such as sodium formate, ammonium formate and triethylammonium formate; cyclohexene; and cyclohexadiene.

The amount of the reducing agent used can be 2 to 100 times, preferably 2 to 10 times the mol of the compound of the general formula [2].

This reaction can be carried out at 0°C to a temperature equal to or lower than the boiling point of the solvent, preferably 10 to 40°C, for 1 minute to 24 hours.

Next, a method for producing the compound of the general formula [2], which is used as a starting material for the compound of the present invention, will be described.

[Production Method A]

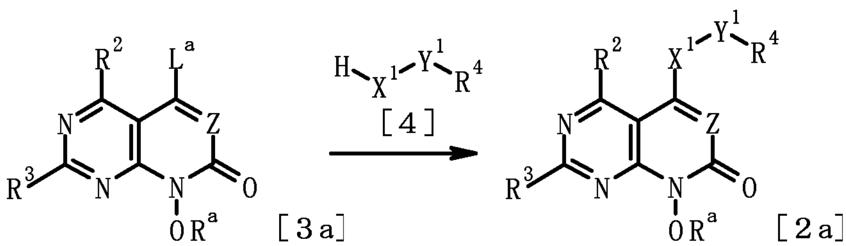

[3a] [4] [2a]

wherein $L^a$ represents a leaving group; and $R^2$, $R^3$, $R^4$, $R^a$, $X^1$, $Y^1$ and Z are as defined above.

For example, 4-methoxybenzylamine and 2-aminomethylpyridine are known as compounds of the general formula [4]. In the case of the compound wherein $X^1$ is represented by, for example, the general formula $NR^5$, the compound of the general formula [4] can be produced from a corresponding nitrile compound by a method known per se in the art, for example, a method described in The Fourth Series of Experimental Chemistry, Vol. 20, ed. by The Chemical Society of Japan, p. 279-282 (1992, Maruzen Co., Ltd.) or a method equivalent thereto.

A compound of the general formula [2a] can be produced by reacting a compound of the general formula [3a] with a compound of the general formula [4] in the presence or absence of a base.

The solvent used in this reaction is not particularly limited as long as the solvent has no adverse effect on the reaction. Examples of the solvent include aliphatic hydrocarbons, halogenated hydrocarbons, alcohols, ethers, ketones, esters, amides, aromatic hydrocarbons, sulfoxides, nitriles and water, etc. These solvents may be used as a mixture.

Preferable examples of the solvent include ethers.

Examples of the base used, if desired, in this reaction, include: inorganic bases such as sodium bicarbonate, sodium carbonate, potassium carbonate, cesium carbonate and tripotassium phosphate; and organic bases such as pyridine, 4-(dimethylamino)pyridine, triethylamine and diisopropylethylamine.

The amount of the base used can be 1 to 50 times, preferably 1 to 2 times the mol of the compound of the general formula [3a].

The amount of the compound of the general formula [4] used can be 1 to 50 times, preferably 1 to 2 times the mol of the compound of the general formula [3a].

This reaction can be carried out at 0°C to a temperature equal to or lower than the boiling point of the solvent, preferably 20 to 120°C, for 1 minute to 24 hours.

[Production Method B]

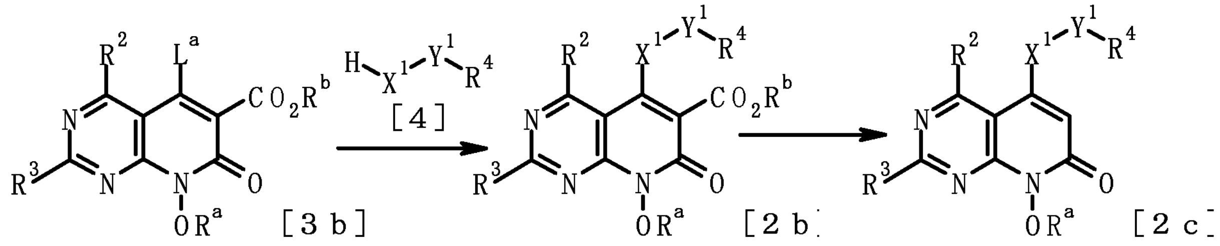

[3b] [4] [2b] [2c]

wherein $R^b$ represents a carboxyl protecting group; and $R^2$, $R^3$, $R^4$, $R^a$, $X^1$, $Y^1$ and $L^a$ are as defined above.

(B-1)

A compound of the general formula [2b] can be produced by reacting a compound of the general formula [3b] with a compound of the general formula [4] according to Production Method A.

(B-2)

A compound of the general formula [2c] can be produced by hydrolyzing the compound of the general formula [2b], followed by decarboxylation reaction.

This reaction can be carried out by a method known per se in the art, for example, a method described in New Experimental Chemistry, Vol. 15, [II], ed. by The Chemical Society of Japan, p. 808-811 (1977, Maruzen Co., Ltd.) or a method equivalent thereto.

[Production Method C]

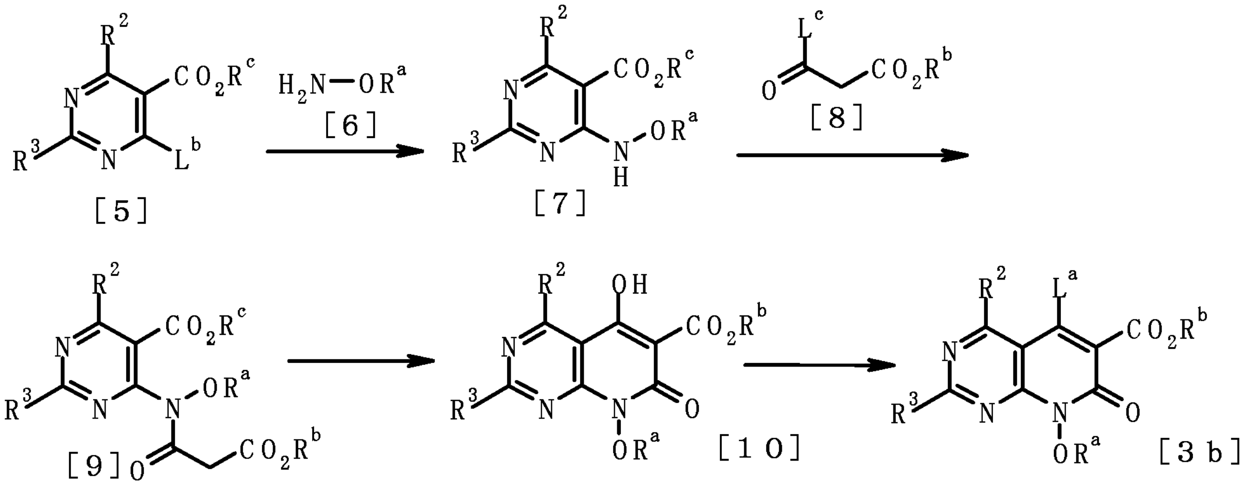

[5] [6] [7] [8] [9] [10] [3b]

wherein $R^c$ represents a carboxyl protecting group; $L^b$ represents a leaving group; $L^c$ represents a halogen atom; and $R^2$, $R^3$, $R^a$, $R^b$ and $L^a$ are as defined above.

(C-1)

For example, ethyl 4-chloropyrimidine-5-carboxylate and ethyl 4-chloro-2-methylpyrimidine-5-carboxylate are known as compounds of the general formula [5].

For example, O-benzylhydroxylamine is known as a compound of the general formula [6].

A compound of the general formula [7] can be produced by reacting a compound of the general formula [5]

with a compound of the general formula [6] in the presence or absence of a base according to Production Method A.

(C-2)

For example, ethylmalonyl chloride is known as a compound of the general formula [8].

A compound of the general formula [9] can be produced by reacting the compound of the general formula [7] with a compound of the general formula [8] in the presence of a base.

The solvent used in this reaction is not particularly limited as long as the solvent has no adverse effect on the reaction. Examples of the solvent include aliphatic hydrocarbons, halogenated hydrocarbons, alcohols, ethers, ketones, esters, amides, aromatic hydrocarbons, sulfoxides, nitriles and water, etc. These solvents may be used as a mixture.

Preferable examples of the solvent include halogenated hydrocarbons.

Examples of the base used in this reaction include: inorganic bases such as sodium bicarbonate, sodium carbonate, potassium carbonate, cesium carbonate and tripotassium phosphate; and organic bases such as pyridine, 4-(dimethylamino)pyridine, triethylamine and diisopropylethylamine.

The amount of the base used can be 1 to 50 times, preferably 1 to 2 times the mol of the compound of the general formula [7].

The amount of the compound of the general formula [8] used can be 1 to 50 times, preferably 1 to 2 times the mol of the compound of the general formula [7].

This reaction can be carried out at -20°C to a temperature equal to or lower than the boiling point of the solvent, preferably -10 to 30°C, for 1 minute to 24 hours.

(C-3)

A compound of the general formula [10] can be produced by subjecting the compound of the general formula [9] to ring-closing reaction in the presence of a base.

The solvent used in this reaction is not particularly limited as long as the solvent has no adverse effect on the reaction. Examples of the solvent include aliphatic hydrocarbons, halogenated hydrocarbons, alcohols, ethers, ketones, esters, amides, aromatic hydrocarbons, sulfoxides, nitriles and water, etc. These solvents may be used as a mixture.

Preferable examples of the solvent include alcohols.

Examples of the base used in this reaction include: metal alkoxides such as sodium methoxide, sodium ethoxide, potassium tert-butoxide and sodium tert-butoxide; inorganic bases such as sodium bicarbonate, sodium carbonate, potassium carbonate, cesium carbonate, and tripotassium phosphate; and organic bases such as pyridine, 4-(dimethylamino)pyridine, triethylamine and diisopropylethylamine.

The amount of the base used can be 1 to 50 times, preferably 1 to 5 times the mol of the compound of the general formula [9].

This reaction can be carried out at 0°C to a temperature equal to or lower than the boiling point of the solvent, preferably 0 to 30°C, for 1 minute to 24 hours.

(C-4)

A compound of the general formula [3b] can be produced by converting the hydroxyl group in the compound of the general formula [10] to a leaving group.

A compound of the general formula [3b] wherein $L^a$ is, for example, a $C_{1-6}$ alkylsulfonyloxy group or an arylsulfonyloxy group can be produced by reacting the compound of the general formula [10] with a sulfonyl chloride compound or sulfonic anhydride in the presence of a base.

The solvent used in this reaction is not particularly limited as long as the solvent has no adverse effect on the reaction. Examples of the solvent include aliphatic hydrocarbons, halogenated hydrocarbons, ethers, ketones, esters, aromatic hydrocarbons, sulfoxides and nitriles, etc. These solvents may be used as a mixture.

Preferable examples of the solvent include halogenated hydrocarbons.

Examples of the base used in this reaction include: inorganic bases such as sodium bicarbonate, sodium carbonate, potassium carbonate, cesium carbonate and tripotassium phosphate; and organic bases such as pyridine, 4-(dimethylamino)pyridine, triethylamine and diisopropylethylamine.

The amount of the base used can be 1 to 50 times, preferably 1 to 2 times the mol of the compound of the general formula [10].

Examples of the sulfonyl chloride compound used in this reaction include: alkanesulfonyl chlorides such as methanesulfonyl chloride and trifluoromethanesulfonyl chloride; and arylsulfonyl chlorides such as benzenesulfonyl chloride and p-toluenesulfonyl chloride.

Examples of the sulfonic anhydride used in this reaction include trifluoromethanesulfonic anhydride.

The amount of the sulfonyl chloride compound or the sulfonic anhydride used can be 1 to 50 times, preferably 1 to 2 times the mol of the compound of the general formula [10].

This reaction can be carried out at 0°C to a temperature equal to or lower than the boiling point of the solvent, preferably 0 to 30°C, for 1 minute to 24 hours.

[Production Method D]

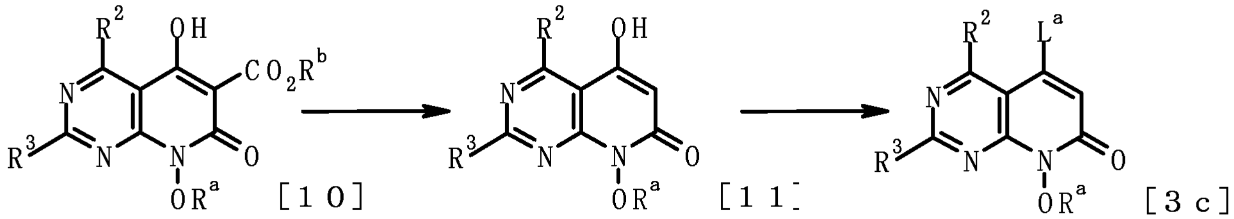

[10] [11] [3c]

wherein $R^2$, $R^3$, $R^a$, $R^b$ and $L^a$ are as defined above.

(D-1)

A compound of the general formula [11] can be produced by hydrolyzing a compound of the general formula [10], followed by decarboxylation reaction.

This reaction can be carried out by a method known per se in the art, for example, a method described in New Experimental Chemistry, Vol. 15, [II], ed. by The Chemical Society of Japan, p. 808-811 (1977, Maruzen Co., Ltd.) or a method equivalent thereto.

(D-2)

A compound of the general formula [3c] can be produced by converting the hydroxyl group in the compound of the general formula [11] to a leaving group.

This reaction can be carried out by a method known per se in the art or a method equivalent thereto and can be carried out by, for example, a method that follows Production Method C-4.

[Production Method E]

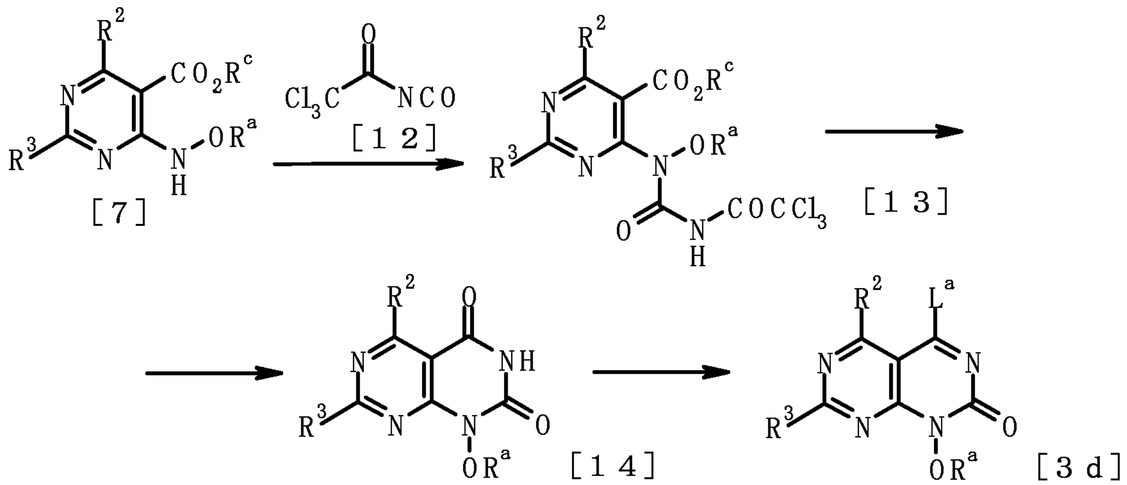

[7] [12] [13] [14] [3d]

wherein $R^2$, $R^3$, $R^a$, $R^c$ and $L^a$ are as defined above.

(E-1)

A compound of the general formula [13] can be produced by reacting a compound of the general formula [7] with a compound of the formula [12] in the presence of a base according to Production Method C-2.

(E-2)

A compound of the general formula [14] can be produced by subjecting the compound of the general formula [13] to ring-closing reaction in the presence of a base according to Production Method C-3.

(E-3)

A compound of the general formula [3d] can be produced by converting the carbonyl group in the tautomeric compound of the general formula [14] to a leaving group.

A compound of the general formula [3d] wherein $L^a$ is, for example, a halogen atom can be produced by reacting the compound of the general formula [14] with a halogenating agent in the presence of a base.

The solvent used in this reaction is not particularly limited as long as the solvent has no adverse effect on the reaction. Examples of the solvent include aliphatic hydrocarbons, halogenated hydrocarbons, ethers, ketones, esters, aromatic hydrocarbons, sulfoxides and nitriles, etc. These solvents may be used as a mixture.

Preferable examples of the solvent include aromatic hydrocarbons.

Examples of the base used in this reaction include: inorganic bases such as sodium bicarbonate, sodium carbonate, potassium carbonate, cesium carbonate and tripotassium phosphate; and organic bases such as pyridine, 4-(dimethylamino)pyridine, triethylamine and diisopropylethylamine.

The amount of the base used can be 1 to 50 times, preferably 1 to 2 times the mol of the compound of the general formula [14].

Examples of the halogenating agent used in this reaction include phosphorus pentaoxide, phosphorus pentachloride, phosphoryl chloride and thionyl chloride, etc.

The amount of the halogenating agent used can be 1 to 50 times, preferably 1 to 2 times the mol of the compound of the general formula [14]. Also, the halogenating agent may be used as a solvent.

This reaction can be carried out at 0°C to a temperature equal to or lower than the boiling point of the solvent, preferably 0 to 80°C, for 1 minute to 24 hours.

[Production Method F]

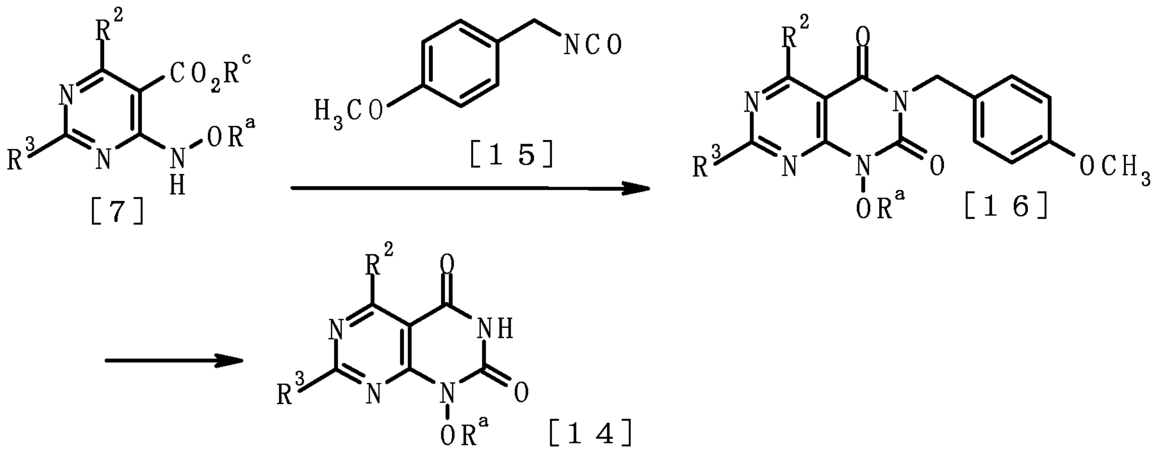
[7] [15] [16] [14]

wherein $R^2$, $R^3$, $R^a$ and $R^c$ are as defined above.

(F-1)

A compound of the general formula [16] can be produced by reacting a compound of the general formula [7] with a compound of the formula [15] in the presence of a base.

The solvent used in this reaction is not particularly limited as long as the solvent has no adverse effect on the reaction. Examples of the solvent include aliphatic hydrocarbons, halogenated hydrocarbons, alcohols, ethers, ketones, esters, amides, aromatic hydrocarbons, sulfoxides, nitriles and water, etc. These solvents may be used as a mixture.
Preferable examples of the solvent include halogenated hydrocarbons.
Examples of the base used in this reaction include: inorganic bases such as sodium bicarbonate, sodium carbonate, potassium carbonate, cesium carbonate and tripotassium phosphate; and organic bases such as pyridine, 4-(dimethylamino)pyridine, triethylamine and diisopropylethylamine.
The amount of the base used can be 1 to 50 times, preferably 1 to 2 times the mol of the compound of the general formula [7].
The amount of the compound of the formula [15] used can be 1 to 50 times, preferably 1 to 2 times the mol of the compound of the general formula [7].
This reaction can be carried out at -20°C to a temperature equal to or lower than the boiling point of the solvent, preferably -10 to 30°C, for 1 minutes to 24 hours.

(F-2)

A compound of the general formula [14] can be produced by deprotecting the compound of the general formula [16]. This reaction can be carried out by, for example, a method described in Protective Groups in Organic Synthesis, 4th edition, p. 696-926, 2007, John Wiley & Sons, INC.
For example, the compound of the general formula [14] can be produced by reacting the compound of the general formula [16] with a deprotecting agent.
The solvent used in this reaction is not particularly limited as long as the solvent has no adverse effect on the reaction. Examples of the solvent include aliphatic hydrocarbons, halogenated hydrocarbons, alcohols, ethers, ketones, esters, amides, aromatic hydrocarbons, sulfoxides, nitriles and water, etc. These solvents may be used as a mixture.
Preferable examples of the solvent include nitriles and water.
Examples of the deprotecting agent used in this reaction include: hydrogen; ammonium formate; zinc; sodium; acid chlorides such as vinyl chloroformate and acetyl chloride; organic silanes such as triethylsilane and trimethylsilyl iodide; tributyltin hydride; alkali metal alkoxides such as potassium tert-butoxide; alkali metal thioalkoxides such as sodium thiomethoxide; 2,3-dichloro-5,6-dicyano-1,4-benzoquinone; sodium borohydride; alkali metal salts such as potassium fluoride and sodium iodide; Lewis acids such as boron tribromide, aluminum chloride, ruthenium chloride and zinc chloride; inorganic acids such as hydrochloric acid, hydrobromic acid and sulfuric acid; organic acids such as trifluoroacetic acid, methanesulfonic acid and para-toluenesulfonic acid; inorganic bases such as potassium carbonate, sodium bicarbonate and sodium hydroxide; organic bases such as piperidine; amines such as ammonia and hydrazine; organic lithium such as methyl lithium; cerium diammonium nitrate; and peroxides such as hydrogen peroxide, ozone and permanganic acid.
The amount of the deprotecting agent used can be 0.01 to 1000 times, preferably 0.1 to 100 times the mol of the compound of the general formula [16].
This reaction can be carried out at 0°C to a temperature equal to or lower than the boiling point of the solvent, preferably 70 to 120°C, for 1 minute to 24 hours.

[Production Method G]

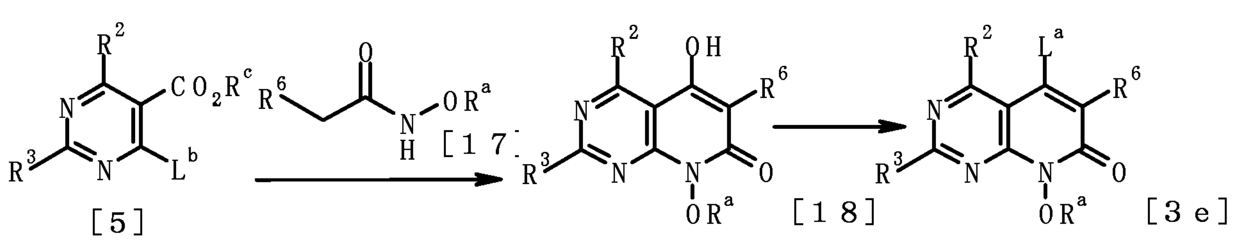

wherein $R^2$, $R^3$, $R^6$, $R^a$, $R^c$, $L^a$ and $L^b$ are as defined above.

(G-1)

For example, N-(benzyloxy)-2-phenylacetamide and N-(benzyloxy)propanamide are known as compounds of

the general formula [17].

A compound of the general formula [18] can be produced by reacting a compound of the general formula [5] with a compound of the general formula [17] in the presence of a base.

The solvent used in this reaction is not particularly limited as long as the solvent has no adverse effect on the reaction. Examples of the solvent include aliphatic hydrocarbons, halogenated hydrocarbons, alcohols, ethers, ketones, esters, amides, aromatic hydrocarbons, sulfoxides, nitriles and water, etc. These solvents may be used as a mixture.

Preferable examples of the solvent include amides.

Examples of the base used in this reaction include: inorganic bases such as sodium bicarbonate, sodium carbonate, potassium carbonate, cesium carbonate and tripotassium phosphate; and organic bases such as pyridine, 4-(dimethylamino)pyridine, triethylamine and diisopropylethylamine.

The amount of the base used can be 1 to 50 times, preferably 2 to 5 times the mol of the compound of the general formula [5].

The amount of the compound of the general formula [17] used can be 1 to 50 times, preferably 2 to 5 times the mol of the compound of the general formula [5].

This reaction can be carried out at 0°C to a temperature equal to or lower than the boiling point of the solvent, preferably 0 to 80°C, for 1 minute to 24 hours.

(G-2)

A compound of the general formula [3e] can be produced by converting the hydroxyl group in the compound of the general formula [18] to a leaving group.

This reaction can be carried out by a method known per se in the art or a method equivalent thereto and can be carried out by, for example, a method that follows Production Method C-4.

[Production Method H]

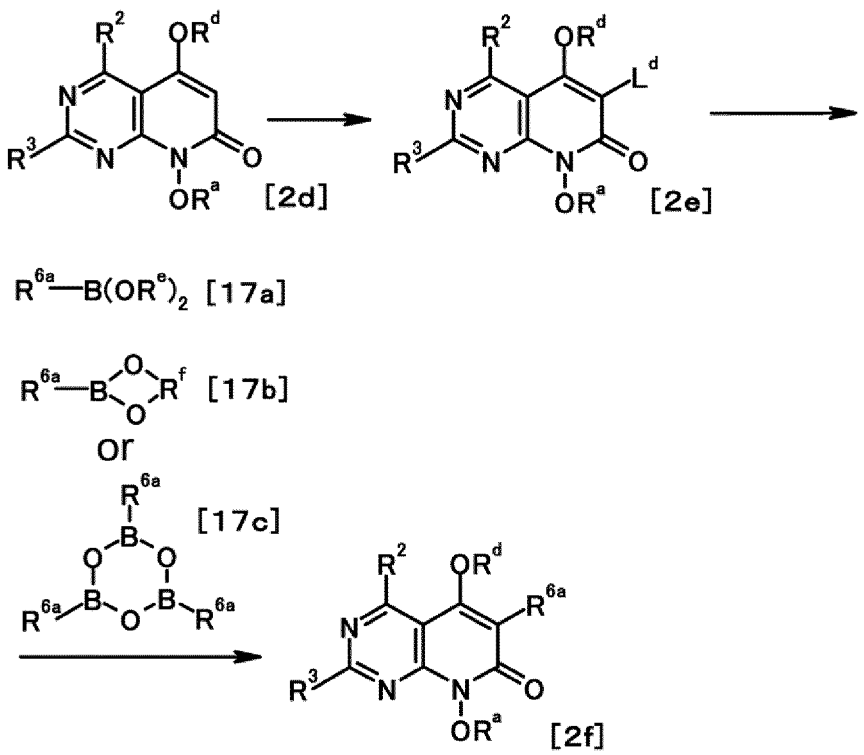

[2d] [2e]

[17a]

[17b]

or

[17c]

[2f]

wherein $R^d$ represents a hydrogen atom or a hydroxyl protecting group; $L^d$ represents a halogen atom; $R^{6a}$ represents an optionally substituted aryl group, an optionally substituted cycloalkyl group, an optionally substituted heterocyclic group, an optionally substituted $C_{1-12}$ alkyl group or an optionally substituted $C_{2-12}$ alkenyl group; $R^e$ represents a hydrogen atom or an optionally substituted $C_{1-6}$ alkyl group; $R^f$ represents an optionally substituted $C_{1-6}$ alkylene group; and $R^2$, $R^3$ and $R^a$ are as defined above.

(H-1)

A compound of the general formula [2e] can be produced by reacting a compound of the general formula [2d] with a halogenating agent in the presence or absence of an additive according to, for example, a method described in The Fourth Series of Experimental Chemistry, Vol. 20, ed. by The Chemical Society of Japan, p. 424-427 and p. 466-467 (1992, Maruzen Co., Ltd.).

The solvent used in this reaction is not particularly limited as long as the solvent has no adverse effect on the reaction. Examples of the solvent include: halogenated hydrocarbons; ethers; alcohols; nitriles; dimethyl sulfoxide; organic acids such as acetic acid, formic acid and trifluoroacetic acid; inorganic acids such as sulfuric acid; and water, etc. These solvents may be used alone or as a mixture of two or more thereof.

The amount of the solvent used can be, but not particularly limited to, preferably 1 to 200 times (v/w) the amount of the compound of the general formula [2d].

The halogenating agent used in this reaction is not particularly limited as long as the agent is a compound usually used in the halogenation reaction of an aromatic compound. Preferable examples of the halogenating agent include iodine, bromine, chlorine, sulfuryl chloride, N-iodosuccinimide, N-bromosuccinimide and N-chlorosuccinimide, etc.

The amount of the halogenating agent used can be 0.05 to 50 times, preferably 0.1 to 20 times the mol of the compound of the general formula [2d].

The additive used, if necessary, in this reaction is not particularly limited as long as the additive is a reagent usually used in the halogenation reaction of an aromatic compound. Preferable examples thereof include sodium bromide, lead tetraacetate, titanium(IV) chloride, aluminum chloride, silver sulfate and trifluoroacetic acid, etc. These additives may be used alone or as a mixture of two or more thereof.

The amount of each additive used in this reaction can be 0.01 to 10 times, preferably 0.1 to 10 times the mol of the compound of the general formula [2d].

This reaction can be carried out at usually -80 to 170°C, preferably -80 to 100°C, for 1 minute to 72 hours, preferably 5 minutes to 48 hours.

(H-2)

For example, phenylboronic acid, 4-methoxyphenylboronic acid and pyridine-3-boronic acid are known as compounds of the general formula [17a].

For example, 3-(4,4,5,5-tetramethyl-1,3,2-dioxaborolan-2-yl)furan is known as a compound of the general formula [17b].

For example, 2,4,6-trimethylboroxine is known as a compound of the general formula [17c].

Alternatively, the compounds of the general formulas [17a] and [17b] can be produced from corresponding halogeno forms according to, for example, a method described in Tetrahedron, Vol. 58, p. 3323-3328 (2002).

A compound of the general formula [2f] can be produced by reacting the compound of the general formula [2e] with a compound of the general formula [17a], [17b] or [17c] in the presence or absence of a base, in the presence of a palladium catalyst, and in the presence or absence of a ligand.

The solvent used in this reaction is not particularly limited as long as the solvent has no effect on the reaction. Examples thereof include halogenated hydrocarbons, ethers, alcohols, aromatic hydrocarbons, acetonitrile and water. These solvents may be used as a mixture.

Preferable examples of the solvent include mixed solvents of alcohols, aromatic hydrocarbons and water, and ethers.

The amount of the solvent used can be, but not particularly limited to, preferably 1 to 150 times (v/w) the amount of the compound of the general formula [2e].

Examples of the palladium catalyst used in this reaction include: metallic palladiums such as palladium-carbon and palladium black; inorganic palladium salts such as palladium chloride; organic palladium salts such as palladium acetate; organic palladium complexes such as tetrakis(triphenylphosphine)palladium(0), bis(triphenylphosphine)palladium(II) dichloride, 1,1'-bis(diphenylphosphino)ferrocene palladium(II) dichloride, tris(dibenzylideneacetone)dipalladium(0) and bis(di-tert-butyl(4-dimethylaminophenyl)phosphine)palladium(II) dichloride; and polymer-immobilized organic palladium complexes such as polymer-supported bis(acetato)triphenylphosphinepalladium(II) and polymer-supported di(acetato)dicyclohexylphenylphosphinepalladium(II), etc. These palladium catalysts may be used in combination.

The amount of the palladium catalyst used can be 0.00001 to 1 times, preferably 0.01 to 0.2 times the mol of the compound of the general formula [2e].

Examples of the ligand used, if desired, in this reaction, include: trialkylphosphines such as trimethylphosphine and tri-tert-butylphosphine; tricycloalkylphosphines such as tricyclohexylphosphine; triarylphosphines such as triphenylphosphine and tritolylphosphine; trialkyl phosphites such as trimethyl phosphite, triethyl phosphite and tributyl phosphite; tricycloalkyl phosphites such as tricyclohexyl phosphite; triaryl phosphites such as triphenyl phosphite; imidazolium salts such as 1,3-bis(2,4,6-trimethylphenyl)imidazolium chloride; diketones such as acetylacetone and octafluoroacetylacetone; amines such as trimethylamine, triethylamine, tripropylamine and tributylamine; 1,1'-bis(diphenylphosphino)ferrocene; 2,2'-bis(diphenylphosphino)-1,1'-binaphthyl; 2-dicyclohexylphosphino-2',6'-dimethoxybiphenyl; 2-dicyclohexylphosphino-2',4',6'-triisopropylbiphenyl; 2-(di-tert-butylphosphino)-2',4',6'-triisopropylbiphenyl; and 2-(di-tert-butylphosphino)biphenyl. These ligands may be used in combination.

The amount of the ligand used can be 0.00001 to 1 times, preferably 0.02 to 0.5 times the mol of the compound of the general formula [2e].

Examples of the base used, if desired, in this reaction include: inorganic bases such as sodium bicarbonate, potassium carbonate, cesium carbonate and tripotassium phosphate; and organic bases such as triethylamine and diisopropylethylamine.

The amount of the base used can be 1 to 50 times, preferably 2 to 10 times the mol of the compound of the general formula [2e].

The amount of the compound of the general formula [17a], [17b] or [17c] used can be 1 to 50 times, preferably 1 to 2 times the mol of the compound of the general formula [2e].

This reaction can be carried out at usually 0 to 160°C, preferably 20 to 120°C, for 1 minute to 96 hours in an inert gas (e.g., nitrogen and/or argon) atmosphere.

[Production Method I]

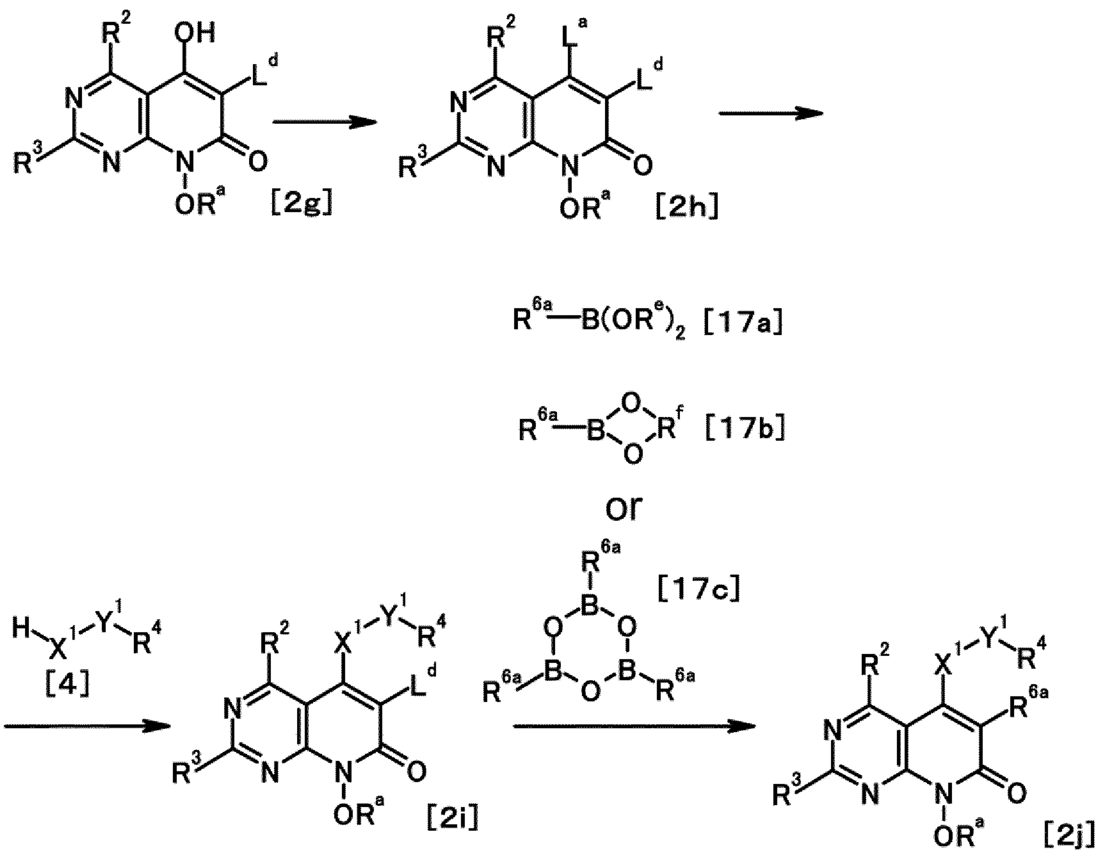

[2g] [2h] [17a] [17b] or [17c] [4] [2i] [2j]

wherein $R^2$, $R^3$, $R^4$, $R^{6a}$, $R^a$, $R^e$, $R^f$, $X^1$, $Y^1$, $L^a$ and $L^d$ are as defined above.

(I-1)

A compound of the general formula [2h] can be produced by converting the hydroxyl group in a compound of the general formula [2g] to a leaving group.

This reaction can be carried out by a method known per se in the art or a method equivalent thereto and can

be carried out by, for example, a method that follows Production Method C-4.

(1-2)

A compound of the general formula [2i] can be produced by reacting the compound of the general formula [2h] with a compound of the general formula [4] according to Production Method A.

(1-3)

A compound of the general formula [2j] can be produced by reacting the compound of the general formula [2i] with a compound of the general formula [17a], [17b] or [17c] in the presence or absence of a base, in the presence of a palladium catalyst, and in the presence or absence of a ligand according to Production Method H-2.

[Production Method J]

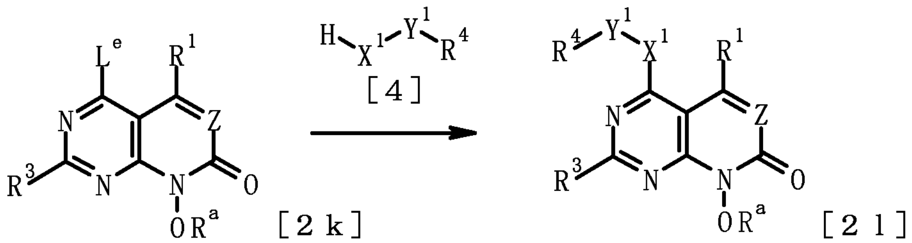

[4]

[2k] [21]

wherein $L^e$ represents a leaving group; and $R^1$, $R^3$, $R^4$, $R^a$, $X^1$, $Y^1$ and Z are as defined above.

A compound of the general formula [21] can be produced by reacting a compound of the general formula [2k] with a compound of the general formula [4] according to Production Method A.

[Production Method K]

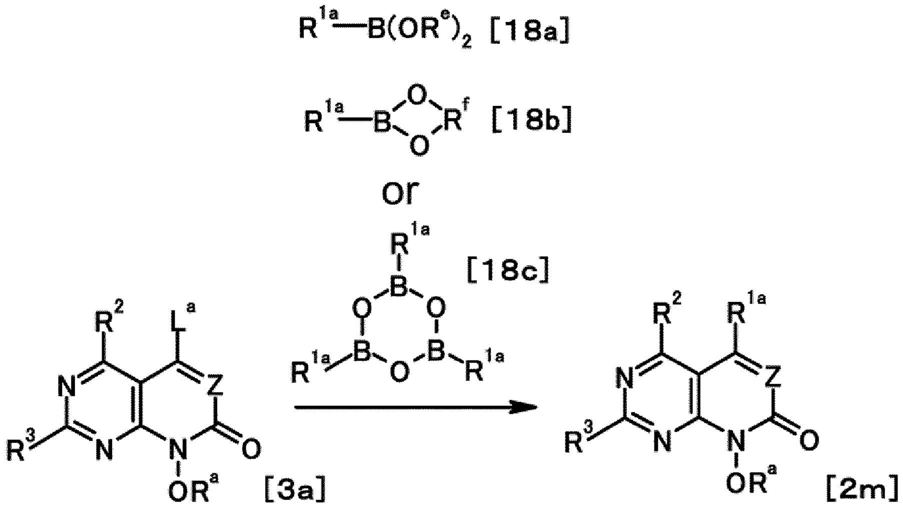

[18a]

[18b]

or

[18c]

[3a] [2m]

wherein $R^{1a}$ represents an optionally substituted aryl group, an optionally substituted heterocyclic group, an optionally substituted $C_{1-12}$ alkyl group or an optionally substituted $C_{2-12}$ alkenyl group; and $R^2$, $R^3$, $R^a$, $R^e$, $R^f$ and Z are as defined above.

A compound of the general formula [2m] can be produced by reacting a compound of the general formula [3a] with a compound of the general formula [18a], [18b] or [18c] in the presence or absence of a base, in the presence of a palladium catalyst, and in the presence or absence of a ligand according to Production Method H-2.

The compounds used in the production methods mentioned above can also be used as salts, if these compounds can take salt forms. Examples of the salts include the same as those exemplified as the salt of the compound of the general formula [1].
The compounds used in the production methods mentioned above may have isomers (e.g., optical isomers, geometric isomers and tautomers). In this case, these isomers may be used. Alternatively, the compounds used in the production methods mentioned above may be any of solvates, hydrates, and crystals in various forms. In this case, these solvates, hydrates, and crystals in various forms may be used.
The compounds obtained by the production methods mentioned above can be converted to other compounds, for example, through a reaction known per se in the art such as condensation, addition, oxidation, reduction, dislocation, substitution, halogenation, dehydration or hydrolysis, or through an appropriate combination of these reactions.
The compounds obtained by the production methods mentioned above and their intermediates may have amino, hydroxyl and/or carboxyl groups. In this case, the reactions can be carried out with their protecting groups appropriately replaced. Also, two or more protecting groups, if any, can each be deprotected selectively through a reaction known per se in the art.
For use as a medicine, the compound of the present invention may be appropriately mixed with pharmaceutical aids usually used in formulation, such as an excipient, a carrier and a diluent. The resulting preparation can be administered orally or parenterally in a form such as tablets, capsules, powders, syrups, granules, pills, suspensions, emulsions, solutions, dusts, suppositories, eye drops, nasal drops, eardrops, patches, ointments or injections according to a routine method. The administration method, dose, and the number of doses of the preparation can be appropriately selected according to the age, body weight and symptoms of a patient. Usually, the compound of the present invention can be administered to an adult once to several times a day at a daily dose of 0.01 to 1000 mg/kg through an oral or parenteral (e.g., injection, drip infusion, and administration to a rectal site) route.
Next, the usefulness of a typical compound of the present invention will be described with reference to the following Test Example.

Test Example 1 Anti-HIV activity

This test was conducted with reference to Journal of Clinical Microbiology, 2007, Vol. 45, p. 477-487.
The anti-HIV activity was evaluated using MaRBLE cells in which a luciferase gene to be expressed under the control of HIV-1 LTR and a CCR5 gene, etc. were introduced in human T lymphocyte-derived HPB-M(a).
A fresh medium or each appropriately diluted test compound was dispensed to 96-well plates to prepare drug solution-containing plates.
The MaRBLE cells were suspended in RPMI1640 containing 10% FCS (fetal calf serum) and penicillin/streptomycin and infected with HIV-1 (JRCSF). Then, the cells were inoculated to the drug solution-containing plates ($1 \times 10^5$ cells/well). The same number of uninfected cells were inoculated as a control group to the drug solution-containing plates.
After culture at 37°C for 7 days in a 5% $CO_2$ atmosphere, the intracellular luciferase activity was measured using Steady-Glo Luciferase assay system (Promega Corp.).
The rate of viral proliferation was determined according to the following expression:

$$\text{Rate of viral proliferation (\%)} = (A / B) \times 100$$

A = (Firefly luciferase activity of the compound-supplemented well) - (Firefly luciferase activity of the uninfected cells)
B = (Firefly luciferase activity of the compound-free well) - (Firefly luciferase activity of the uninfected cells)

$IC_{50}$ of the compound was calculated by a plot with concentration as the logarithm and the rate of viral proliferation as the real part using the FORECAST function (linear regression method) of Microsoft Office Excel 2003.
The results are shown in Table 1.

[Table 1]

| Test compound (Example No.) | Anti-HIV activity $IC_{50}$($\mu$ mol/L) | Test compound (Example No.) | Anti-HIV activity $IC_{50}$($\mu$ mol/L) | Test compound (Example No.) | Anti-HIV activity $IC_{50}$($\mu$ mol/L) |
|---|---|---|---|---|---|
| 1 | 0.11 | 55 | 0.45 | 114 | 0.042 |
| 2 | 0.12 | 56 | 0.90 | 115 | 0.15 |
| 3 | 0.19 | 57 | 0.085 | 122 | 0.10 |

(continued)

| Test compound (Example No.) | Anti-HIV activity $IC_{50}$($\mu$ mol/L) | Test compound (Example No.) | Anti-HIV activity $IC_{50}$($\mu$ mol/L) | Test compound (Example No.) | Anti-HIV activity $IC_{50}$($\mu$ mol/L) |
|---|---|---|---|---|---|
| 4 | 0.41 | 58 | 0.094 | 123 | 0.078 |
| 5 | 0.21 | 59 | 0.16 | 124 | 0.058 |
| 6 | 0.18 | 60 | 0.19 | 125 | 0.063 |
| 7 | 0.17 | 61 | 0.49 | 126 | 0.14 |
| 8 | 0.22 | 63 | 0.87 | 127 | 0.15 |
| 10 | 0.53 | 64 | 0.35 | 128 | 0.27 |
| 16 | 0.28 | 66 | 0.56 | 129 | 0.091 |
| 17 | 0.69 | 67 | 0.16 | 132 | 0.21 |
| 18 | 0.23 | 68 | 0.25 | 133 | 0.059 |
| 21 | 0.88 | 70 | 0.79 | 134 | 0.14 |
| 22 | 0.047 | 72 | 0.15 | 136 | 0.12 |
| 28 | 0.043 | 77 | 0.59 | 137 | 0.082 |
| 29 | 0.050 | 78 | 0.30 | 138 | 0.094 |
| 30 | 0.076 | 79 | 0.82 | 140 | 0.19 |
| 31 | 0.23 | 80 | 0.57 | 141 | 0.046 |
| 32 | 0.029 | 81 | 0.36 | 142 | 0.13 |
| 33 | 0.053 | 83 | 0.37 | 143 | 0.12 |
| 34 | 0.065 | 84 | 0.076 | 144 | 0.090 |
| 35 | 0.068 | 85 | 0.31 | 145 | 0.49 |
| 36 | 0.086 | 86 | 0.50 | 146 | 0.10 |
| 37 | 0.11 | 87 | 0.081 | 147 | 0.083 |
| 38 | 0.042 | 88 | 0.063 | 148 | 0.071 |
| 39 | 0.062 | 89 | 0.085 | 149 | 0.026 |
| 40 | 0.048 | 90 | 0.58 | 150 | 0.10 |
| 41 | 0.048 | 92 | 0.031 | 151 | 0.054 |
| 42 | 0.072 | 93 | 0.045 | 152 | 0.095 |
| 43 | 0.039 | 94 | 0.043 | 153 | 0.13 |
| 44 | 0.029 | 95 | 0.11 | 154 | 0.72 |
| 45 | 0.17 | 97 | 0.20 | 155 | 0.23 |
| 46 | 0.040 | 102 | 0.21 | 156 | 0.59 |
| 47 | 0.059 | 103 | 0.20 | 157 | 0.044 |
| 48 | 0.093 | 105 | 0.064 | 158 | 0.074 |
| 49 | 0.061 | 106 | 0.080 | 160 | 0.068 |
| 50 | 0.11 | 108 | 0.57 | 161 | 0.18 |
| 51 | 0.097 | 110 | 0.049 | 162 | 0.067 |
| 52 | 0.020 | 111 | 0.0084 | 165 | 0.16 |
| 53 | 0.080 | 112 | 0.028 | | |
| 54 | 0.11 | 113 | 0.073 | | |

The compound of the present invention had excellent anti-HIV activity.

Next, the present invention will be described with reference to Reference Examples and Examples. However, the present invention is not intended to be limited by these examples.

A mixing ratio for an eluent is indicated by volume ratio. For example, "eluent: 75-0% hexane/ethyl acetate" means that the eluent was changed from 75% hexane/25% ethyl acetate to finally 0% hexane/100% ethyl acetate. The carrier used for silica gel column chromatography was a silica gel Purif-Pack SI (60 $\mu$m) from Fuji Silysia Chemical Ltd., unless otherwise specified.

Each abbreviation in each Example is as defined below.

Bn: Benzyl

Boc: tert-Butoxycarbonyl
CAN: Cerium(IV) diammonium nitrate
DMF: N,N-Dimethylformamide
DMSO-$d_6$: Dimethyl-$d_6$ sulfoxide
Et: Ethyl
HATU: O-(7-Azabenzotriazol-1-yl)-N,N,N',N'-tetramethyluronium hexafluorophosphate
Me: Methyl
MOM: Methoxymethyl
NBS: N-Bromosuccinimide
Tf: Trifluoromethylsulfonyl
TFA: Trifluoroacetic acid
THF: Tetrahydrofuran
s: Singlet
br: Broad
brs: Broad singlet
d: Doublet
dd: Double doublet
ddd: Double double doublet
t: Triplet
q: Quartet
m: Multiplet

Reference Example 1

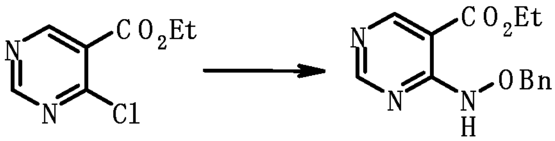

A suspension of ethyl 4-chloropyrimidine-5-carboxylate (1.00 g) and O-benzylhydroxylamine hydrochloride (1.45 g) in N-ethyldiisopropylamine (20 mL) was heated with stirring at 110 to 120°C for 3 hours. After cooling of the reaction mixture, chloroform and water were added thereto, followed by separation of an organic layer. An aqueous layer was extracted with chloroform, combined with the organic layer and dried over sodium sulfate, and the solvent was distilled off under reduced pressure. The obtained residue was purified by silica gel column chromatography [Yamazen Corp., HI-FLASH COLUMNS, W001, eluent: 75-0% hexane/ethyl acetate] to obtain a yellow oil of ethyl 4-((benzyloxy)amino)pyrimidine-5-carboxylate (1.06 g).
$^1$H-NMR ($CDCl_3$) $\delta$ value: 1.36 (t, J=7.1 Hz, 3H), 4.33 (q, J=7.2 Hz, 2H), 5.06 (s, 2H), 7.34-7.44 (m, 3H), 7.44-7.50 (m, 2H), 8.84 (s, 1H), 8.89 (s, 1H), 10.24 (brs, 1H).

Reference Example 2

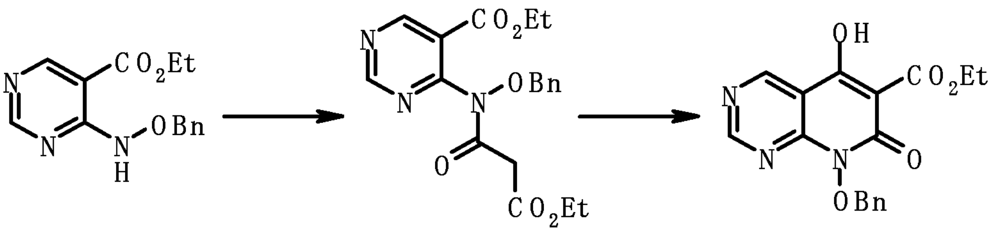

To a solution of ethyl 4-((benzyloxy)amino)pyrimidine-5-carboxylate (1.06 g) and triethylamine 1.08 (mL) in methylene chloride (15 mL), ethylmalonyl chloride (0.99 mL) was added dropwise under ice cooling, methylene chloride (5 mL) was added, and the mixture was stirred at room temperature for 3 hours and 10 minutes. To the reaction mixture,

triethylamine (1.08 mL) was added, ethylmalonyl chloride (0.99 mL) was added dropwise under ice cooling, and the mixture was stirred at room temperature for 8 hours. Insoluble matter was filtered off, and the solvent was distilled off under reduced pressure. The obtained residue was purified by silica gel column chromatography [Yamazen Corp., HI-FLASH COLUMNS, W004, eluent: 80-0% hexane/ethyl acetate] to obtain a yellow oil (1.54 g). To a solution of the obtained yellow oil (1.54 g) in ethanol (30 mL), a 20% solution of sodium ethoxide in ethanol (3.41 g) was added dropwise, and the mixture was stirred at room temperature for 1 hour and 50 minutes. The reaction mixture was pH-adjusted to 2 by the addition of 2 mol/L hydrochloric acid and stirred at room temperature for 30 minutes. The deposit was collected by filtration to obtain a white solid of ethyl 8-(benzyloxy)-5-hydroxy-7-oxo-7,8-dihydropyrido[2,3-d]pyrimidine-6-carboxylate (0.73 g).
$^1$H-NMR (DMSO-$d_6$) δ value: 1.32 (t, J=7.1 Hz, 3H), 4.34 (q, J=7.2 Hz, 2H), 5.16 (s, 2H), 7.39-7.50 (m, 3H), 7.62-7.70 (m, 2H), 9.24 (s, 1H), 9.30 (s, 1H).

Reference Example 3

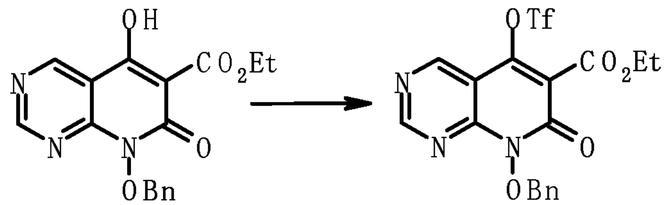

To a solution of ethyl 8-(benzyloxy)-5-hydroxy-7-oxo-7,8-dihydropyrido[2,3-d]pyrimidine-6-carboxylate (150 mg) and triethylamine (122μL) in methylene chloride (7 mL), trifluoromethanesulfonic anhydride (144 μL) was added dropwise under ice cooling, and the mixture was stirred at room temperature for 4 hours and 30 minutes. The solvent in the reaction mixture was distilled off under reduced pressure. The obtained residue was purified by silica gel column chromatography [Yamazen Corp., HI-FLASH COLUMNS, W003, eluent: 90-50% hexane/ethyl acetate] to obtain a pale yellow solid of ethyl 8-(benzyloxy)-7-oxo-5-(((trifluoromethyl)sulfonyl)oxy)-7,8-dihydropyrido[2,3-d]pyrimidine-6-carboxylate (134 mg). $^1$H-NMR ($CDCl_3$) δ value: 1.43 (t, J=7.2 Hz, 3H), 4.48 (q, J=7.1 Hz, 2H), 5.32 (s, 2H), 7.36-7.46 (m, 3H), 7.61-7.70 (m, 2H), 9.14 (s, 1H), 9.28 (s, 1H).

Reference Example 4

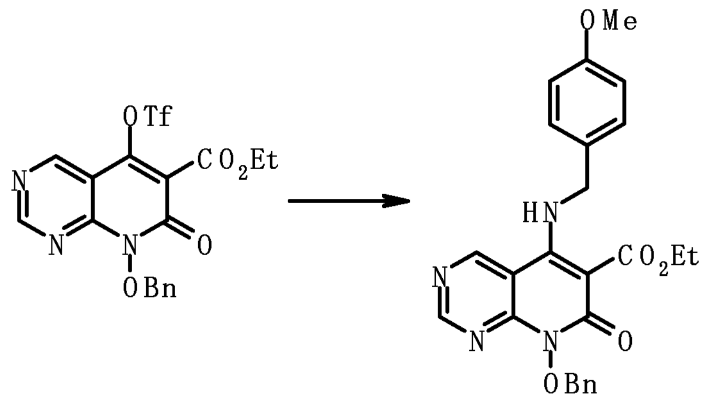

A solution of ethyl 8-(benzyloxy)-7-oxo-5-(((trifluoromethyl)sulfonyl)oxy)-7,8-dihydropyrido[2,3-d]pyrimidine-6-carboxylate (120 mg) and 4-methoxybenzylamine (164 μL) in dioxane (9 mL) was heated with stirring at 90 to 100°C for 3 hours. After cooling of the reaction mixture, the solvent was distilled off under reduced pressure. The obtained residue was purified by silica gel column chromatography [Yamazen Corp., HI-FLASH COLUMNS, W003, eluent: 50-20% hexane/ethyl acetate] to obtain a white solid of ethyl 8-(benzyloxy)-5-((4-methoxybenzyl)amino)-7-oxo-7,8-dihydropyrido[2,3-d]pyrimidine-6-carboxylate (101 mg). $^1$H-NMR ($CDCl_3$) δ value: 1.41 (t, J=7.2 Hz, 3H), 3.83 (s, 3H), 4.40 (q, J=7.2 Hz, 2H), 4.78 (d, J=5.4 Hz, 2H), 5.26 (s, 2H), 6.94 (d, J=8.6 Hz, 2H), 7.22-7.33 (m, 2H), 7.33-7.45 (m, 3H), 7.62-7.78

(m, 2H), 9.04 (s, 1H), 9.15 (s, 1H), 9.70-9.80 (m, 1H).

Reference Example 5

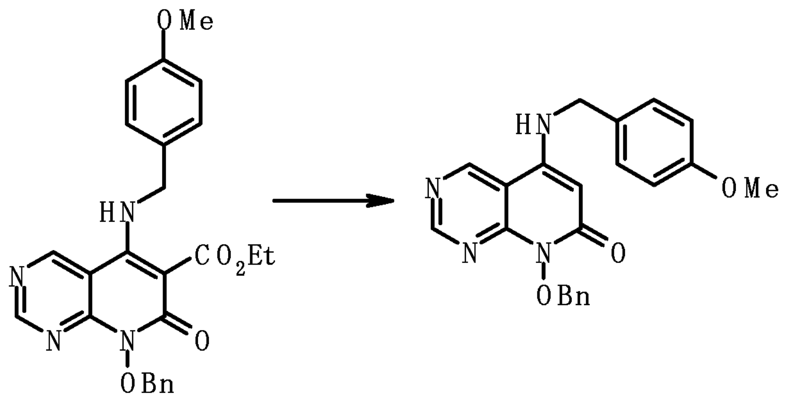

To ethyl 8-(benzyloxy)-5-((4-methoxybenzyl)amino)-7-oxo-7,8-dihydropyrido[2,3-d]pyrimidine-6-carboxylate (235 mg), methanol (10 mL) and a 1 mol/L aqueous sodium hydroxide solution (10 mL) were added, and the mixture was heated to reflux for 1 hour and 40 minutes. After cooling of the reaction mixture, the solvent was distilled off under reduced pressure. To the obtained residue, a 1 mol/L aqueous sodium hydroxide solution (7 mL) was added, and the mixture was pH-adjusted to 1.5 by the dropwise addition of concentrated hydrochloric acid and stirred for 20 minutes under water cooling. The deposit was collected by filtration to obtain a white solid of 8-(benzyloxy)-5-((4-methoxybenzyl)amino)pyrido[2,3-d]pyrimidin-7(8H)-one (144 mg).
$^1$H-NMR ($CDCl_3$) δ value: 3.83 (s, 3H), 4.34 (d, J=3.9 Hz, 2H), 5.12-5.18 (m, 1H), 5.27 (s, 2H), 5.77 (s, 1H), 6.93 (d, J=8.3 Hz, 2H), 7.27-7.32 (m, 2H), 7.34-7.43 (m, 3H), 7.62-7.70 (m, 2H), 8.83 (s, 1H), 9.08 (s, 1H).

Reference Example 6

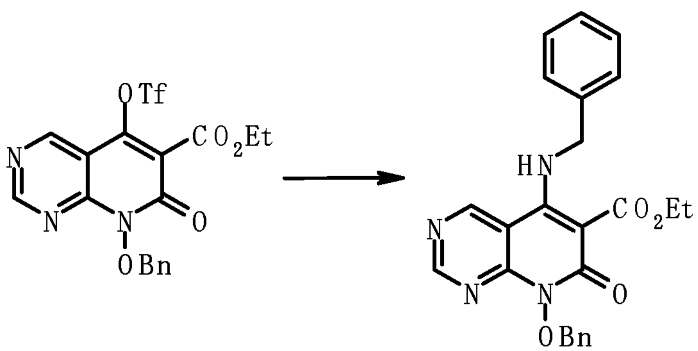

A solution of ethyl 8-(benzyloxy)-7-oxo-5-(((trifluoromethyl)sulfonyl)oxy)-7,8-dihydropyrido[2,3-d]pyrimidine-6-carboxylate (72 mg) and benzylamine (83 μL) in dioxane (7 mL) was heated with stirring at 90 to 100°C for 1 hour and 30 minutes. After cooling of the reaction mixture, the solvent was distilled off under reduced pressure. The obtained residue was purified by silica gel column chromatography [eluent: 80-20% hexane/ethyl acetate] and then suspended in a mixed solvent of 2-propanol and diisopropyl ether, and the deposit was collected by filtration to obtain a white solid of ethyl 5-(benzylamino)-8-(benzyloxy)-7-oxo-7,8-dihydropyrido[2,3-d]pyrimidine-6-carboxylate (48 mg).
$^1$H-NMR ($CDCl_3$) δ value: 1.41 (t, J=7.1 Hz, 3H), 4.41 (q, J=7.2 Hz, 2H), 4.85 (d, J=5.8 Hz, 2H), 5.27 (s, 2H), 7.32-7.46 (m, 8H), 7.66-7.74 (m, 2H), 9.04 (s, 1H), 9.12 (s, 1H), 9.76-9.90 (m, 1H).

Reference Example 7

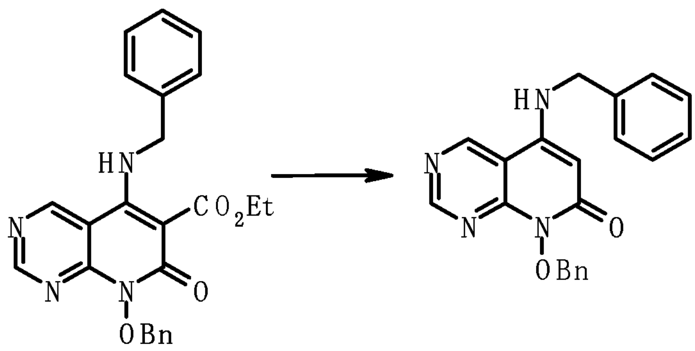

To ethyl 5-(benzylamino)-8-(benzyloxy)-7-oxo-7,8-dihydropyrido[2,3-d]pyrimidine-6-carboxylate (40 mg), methanol (5 mL) and a 1 mol/L aqueous sodium hydroxide solution (5 mL) were added, and the mixture was heated to reflux for 3 hours. After cooling of the reaction mixture, the solvent was distilled off under reduced pressure. To the obtained residue, a 1 mol/L aqueous sodium hydroxide solution (5 mL) was added, and the mixture was pH-adjusted to 1.5 by the dropwise addition of concentrated hydrochloric acid and stirred for 20 minutes under water cooling. The deposit was collected by filtration to obtain a white solid of 5-(benzylamino)-8-(benzyloxy)pyrido[2,3-d]pyrimidin-7(8H)-one (14 mg).
$^1$H-NMR ($CDCl_3$) $\delta$ value: 4.43 (d, J=4.6 Hz, 2H), 5.10-5.18 (m, 1H), 5.27 (s, 2H), 5.78 (s, 1H), 7.31-7.46 (m, 8H), 7.64-7.70 (m, 2H), 8.83 (s, 1H), 9.09 (s, 1H).

Reference Example 8

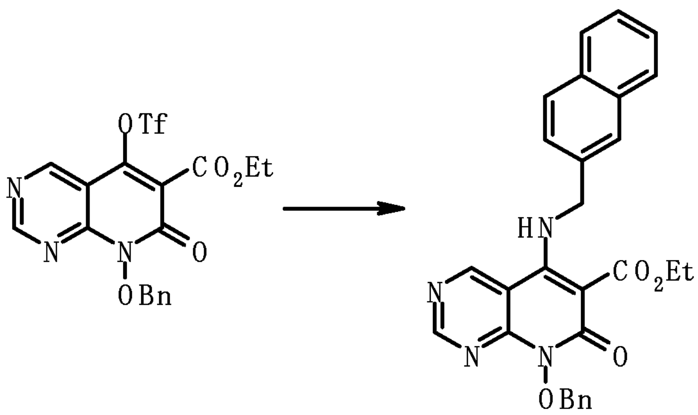

A solution of ethyl 8-(benzyloxy)-7-oxo-5-(((trifluoromethyl)sulfonyl)oxy)-7,8-dihydropyrido[2,3-d]pyrimidine-6-carboxylate (90 mg) and (2-naphthalenemethyl)amine (117 mg) in dioxane (9 mL) was heated to reflux for 2 hours. After cooling of the reaction mixture, the solvent was distilled off under reduced pressure. The obtained residue was purified by silica gel column chromatography [eluent: 90-0% hexane/ethyl acetate] and then suspended in a mixed solvent of 2-propanol and diisopropyl ether, and the deposit was collected by filtration to obtain a white solid of ethyl 8-(benzyloxy)-5-((2-naphthylmethyl)amino)-7-oxo-7,8-dihydropyrido[2,3-d]pyrimidine-6-carboxylate (35 mg).
$^1$H-NMR ($CDCl_3$) $\delta$ value: 1.38-1.44 (m, 3H), 4.41 (q, J=7.2 Hz, 2H), 5.01 (d, J=5.8 Hz, 2H), 5.27 (s, 2H), 7.34-7.43 (m, 4H), 7.48-7.56 (m, 2H), 7.66-7.73 (m, 2H), 7.78-7.94 (m, 4H), 9.03 (s, 1H), 9.16 (s, 1H), 9.94-10.02 (m, 1H).

Reference Example 9

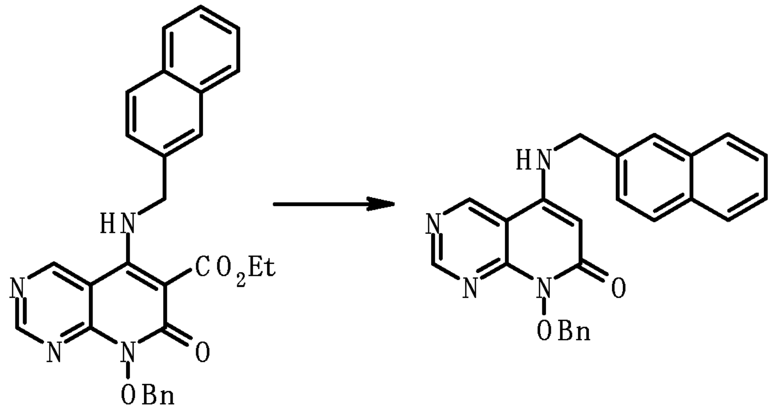

To ethyl 8-(benzyloxy)-5-((2-naphthylmethyl)amino)-7-oxo-7,8-dihydropyrido[2,3-d]pyrimidine-6-carboxylate (33 mg), methanol (5 mL) and a 1 mol/L aqueous sodium hydroxide solution (5 mL) were added, and the mixture was heated to reflux for 3 hours. After cooling of the reaction mixture, the solvent was distilled off under reduced pressure. To the obtained residue, a 1 mol/L aqueous sodium hydroxide solution (5 mL) was added, and the mixture was pH-adjusted to 2 by the dropwise addition of concentrated hydrochloric acid and stirred at room temperature for 10 minutes. The deposit was collected by filtration to obtain a light brown solid of 8-(benzyloxy)-5-((2-naphthylmethyl)amino)pyrido[2,3-d]pyrimidin-7(8H)-one (18 mg).
$^1$H-NMR ($CDCl_3$) δ value: 4.59 (d, J=4.9 Hz, 2H), 5.27 (s, 2H), 5.82 (s, 1H), 7.33-7.44 (m, 4H), 7.44-7.50 (m, 1H), 7.50-7.56 (m, 2H), 7.64-7.70 (m, 2H), 7.80-7.92 (m, 4H), 8.87 (s, 1H), 9.09 (s, 1H).

Reference Example 10

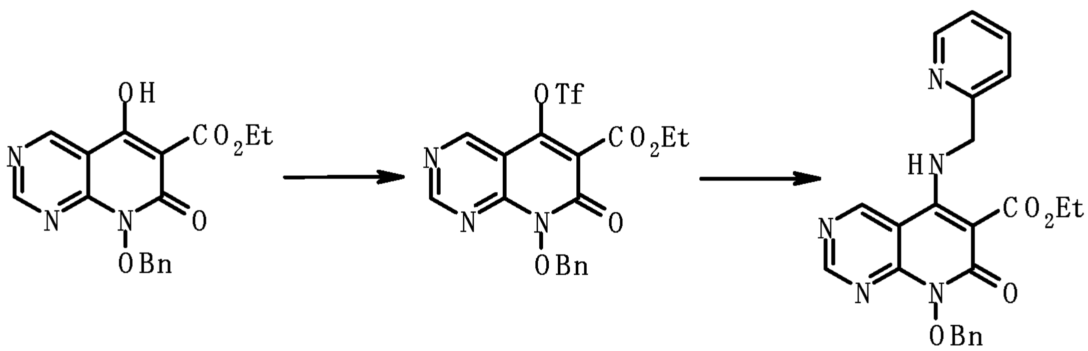

To a solution of ethyl 8-(benzyloxy)-5-hydroxy-7-oxo-7,8-dihydropyrido[2,3-d]pyrimidine-6-carboxylate (1.00 g) and triethylamine (812 μL) in methylene chloride (30 mL), trifluoromethanesulfonic anhydride (961 μL) was added dropwise under ice cooling, methylene chloride (5 mL) was added, and the mixture was stirred at room temperature for 1 hour. The solvent in the reaction mixture was distilled off under reduced pressure. To the obtained residue, 2-aminomethylpyridine (1.48 mL) and dioxane (30 mL) were added, and the mixture was heated with stirring at 80 to 90°C for 30 minutes. After cooling of the reaction mixture, the solvent was distilled off under reduced pressure. The obtained residue was purified by silica gel column chromatography [eluent: 100-80% chloroform/methanol] and then suspended in a mixed solvent of methanol and diisopropyl ether, and the deposit was collected by filtration to obtain a yellow solid of ethyl 8-(benzyloxy)-7-oxo-5-((pyridin-2-ylmethyl)amino)-7,8-dihydropyrido[2,3-d]pyrimidine-6-carboxylate (451 mg).
$^1$H-NMR ($CDCl_3$) δ value: 1.40-1.53 (m, 3H), 4.42-4.58 (m, 2H), 4.68-4.80 (m, 2H), 5.27 (s, 2H), 7.20-7.45 (m, 5H), 7.60-7.80 (m, 3H), 8.60-8.72 (m, 1H), 8.95-9.07 (m, 1H), 9.10 (s, 1H), 9.28 (s, 1H).

Reference Example 11

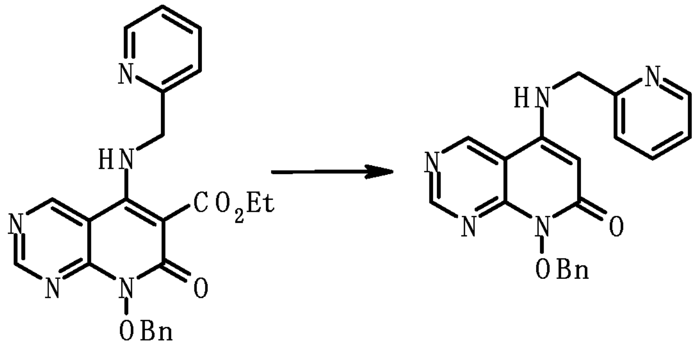

To ethyl 8-(benzyloxy)-7-oxo-5-(((pyridin-2-yl)methyl)amino)-7,8-dihydropyrido[2,3-d]pyrimidine-6-carboxylate (440 mg), methanol (25 mL) and a 1 mol/L aqueous sodium hydroxide solution (25 mL) were added, and the mixture was heated with stirring at 80 to 90°C for 30 minutes. After cooling of the reaction mixture, the solvent was distilled off under reduced pressure. To the obtained residue, chloroform and a saturated aqueous solution of sodium bicarbonate were added, followed by separation of an organic layer. An aqueous layer was extracted with chloroform, combined with the organic layer and dried over sodium sulfate, and the solvent was distilled off under reduced pressure. The obtained residue was purified by silica gel column chromatography [eluent: 99-85% chloroform/methanol] and then suspended in a mixed solvent of 2-propanol and diisopropyl ether, and the deposit was collected by filtration to obtain a brown solid of 8-(benzyloxy)-5-(((pyridin-2-yl)methyl)amino)pyrido[2,3-d]pyrimidin-7(8H)-one (184 mg).
$^1$H-NMR ($CDCl_3$) δ value: 4.50 (d, J=3.9 Hz, 2H), 5.29 (s, 2H), 5.72 (s, 1H), 7.04-7.14 (m, 1H), 7.26-7.46 (m, 5H), 7.64-7.72 (m, 2H), 7.72-7.80 (m, 1H), 8.63 (d, J=4.6 Hz, 1H), 9.08 (s, 1H), 9.11 (s, 1H).

Reference Example 12

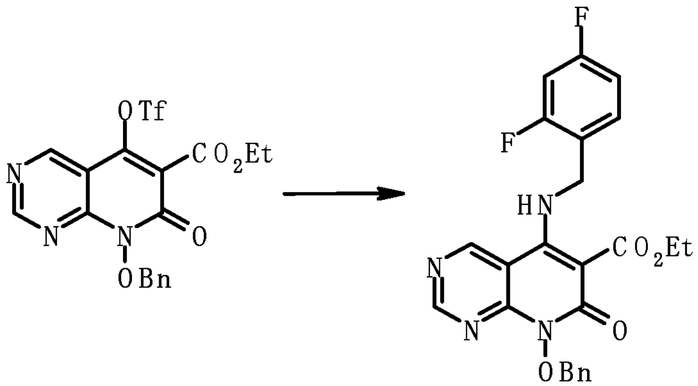

A solution of ethyl 8-(benzyloxy)-7-oxo-5-(((trifluoromethyl)sulfonyl)oxy)-7,8-dihydropyrido[2,3-d]pyrimidine-6-carboxylate (136 mg) and 2,4-difluorobenzylamine (205 mg) in dioxane (14 mL) was heated with stirring at 100 to 110°C for 2 hours. The reaction mixture was cooled, then purified by silica gel column chromatography [eluent: 85-20% hexane/ethyl acetate], and then suspended in a mixed solvent of 2-propanol and diisopropyl ether, and the deposit was collected by filtration to obtain a pale yellow solid of ethyl 8-(benzyloxy)-5-((2,4-difluorobenzyl)amino)-7-oxo-7,8-dihydropyrido[2,3-d]pyrimidine-6-carboxylate (68 mg). $^1$H-NMR ($CDCl_3$) δ value: 1.41 (t, J=7.1 Hz, 3H), 4.40 (q, J=7.1 Hz, 2H), 4.83 (d, J=5.8 Hz, 2H), 5.27 (s, 2H), 6.86-6.99 (m, 2H), 7.33-7.44 (m, 4H), 7.66-7.72 (m, 2H), 9.06 (s, 1H), 9.11 (s, 1H), 9.58-9.68 (m, 1H).

Reference Example 13

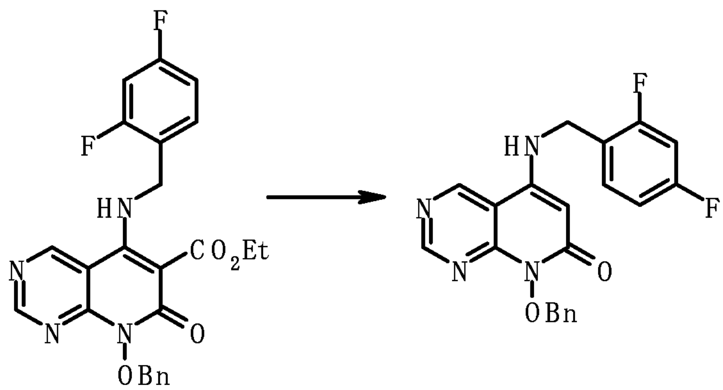

To ethyl 8-(benzyloxy)-5-((2,4-difluorobenzyl)amino)-7-oxo-7,8-dihydropyrido[2,3-d]pyrimidine-6-carboxylate (60 mg), methanol (5 mL) and a 1 mol/L aqueous sodium hydroxide solution (5 mL) were added, and the mixture was heated to reflux for 3 hours. After cooling of the reaction mixture, the solvent was distilled off under reduced pressure. To the obtained residue, a 1 mol/L aqueous sodium hydroxide solution (5 mL) was added, and the mixture was pH-adjusted to 2 by the dropwise addition of concentrated hydrochloric acid and stirred at room temperature for 30 minutes. The deposit was collected by filtration to obtain a white solid of 8-(benzyloxy)-5-((2,4-difluorobenzyl)amino)pyrido[2,3-d]pyrimidin-7(8H)-one (28 mg).
$^1$H-NMR ($CDCl_3$) $\delta$ value: 4.47 (d, J=5.1 Hz, 2H), 5.22-5.29 (m, 3H), 5.76 (s, 1H), 6.85-6.95 (m, 2H), 7.30-7.44 (m, 4H), 7.62-7.70 (m, 2H), 8.86 (s, 1H), 9.09 (s, 1H).

Reference Example 14

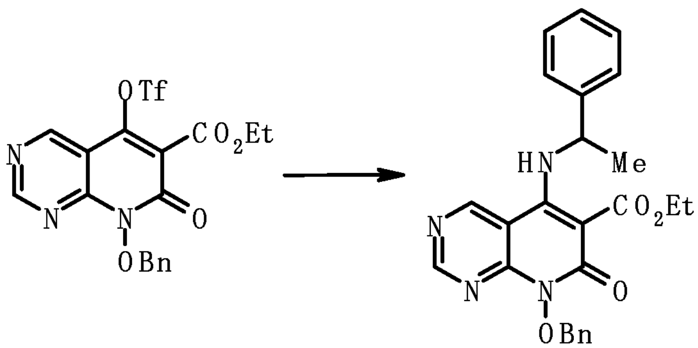

A solution of ethyl 8-(benzyloxy)-7-oxo-5-(((trifluoromethyl)sulfonyl)oxy)-7,8-dihydropyrido[2,3-d]pyrimidine-6-carboxylate (82 mg) and (±)-1-phenylethylamine (1.1 mL) in DMF (4 mL) was heated with stirring at 70 to 80°C for 1 hour. After cooling of the reaction mixture, ethyl acetate and water were added thereto. An organic layer was separated, washed with a saturated aqueous solution of sodium chloride and dried over sodium sulfate, and the solvent was distilled off under reduced pressure. The obtained residue was purified by silica gel column chromatography [eluent: 90-30% hexane/ethyl acetate] and then suspended in a mixed solvent of 2-propanol and diisopropyl ether, and the deposit was collected by filtration to obtain a white solid of ethyl 8-(benzyloxy)-7-oxo-5-((1-phenylethyl)amino)-7,8-dihydropyrido[2,3-d]pyrimidine-6-carboxylate (29 mg).
$^1$H-NMR ($CDCl_3$) $\delta$ value: 1.46 (t, J=7.1 Hz, 3H), 1.71 (d, J=6.6 Hz, 3H), 4.46 (q, J=7.0 Hz, 2H), 5.04-5.14 (m, 1H), 5.20-5.28 (m, 2H), 7.28-7.43 (m, 8H), 7.63-7.72 (m, 2H), 8.96 (s, 1H), 8.99 (s, 1H), 10.26-10.36 (m, 1H).

Reference Example 15

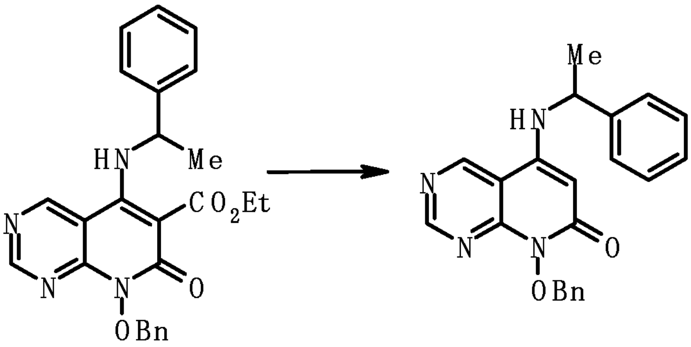

To ethyl 8-(benzyloxy)-7-oxo-5-((1-phenylethyl)amino)-7,8-dihydropyrido[2,3-d]pyrimidine-6-carboxylate (27 mg), methanol (5 mL) and a 1 mol/L aqueous sodium hydroxide solution (5 mL) were added, and the mixture was heated to reflux for 2 hours. After cooling of the reaction mixture, the solvent was distilled off under reduced pressure. To the obtained residue, a 1 mol/L aqueous sodium hydroxide solution (5 mL) was added, and the mixture was pH-adjusted to 2 by the dropwise addition of concentrated hydrochloric acid and stirred at room temperature for 20 minutes. The deposit was collected by filtration to obtain a white solid of 8-(benzyloxy)-5-((1-phenylethyl)amino)pyrido[2,3-d]pyrimidin-7(8H)-one (10 mg).
$^1$H-NMR ($CDCl_3$) δ value: 1.67 (d, J=6.6 Hz, 3H), 4.59-4.66 (m, 1H), 5.05-5.13 (m, 1H), 5.22 (s, 2H), 5.57 (s, 1H), 7.30-7.42 (m, 8H), 7.60-7.68 (m, 2H), 8.92 (s, 1H), 9.09 (s, 1H).

Reference Example 16

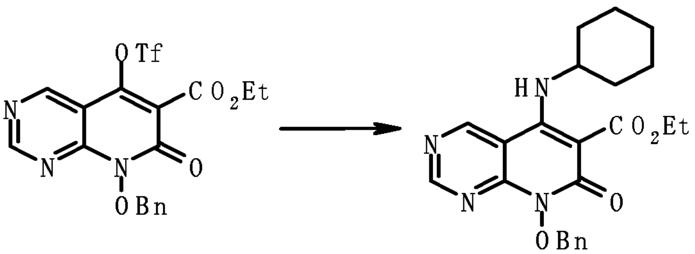

A solution of ethyl 8-(benzyloxy)-7-oxo-5-(((trifluoromethyl)sulfonyl)oxy)-7,8-dihydropyrido[2,3-d]pyrimidine-6-carboxylate (140 mg) and cyclohexylamine (169 μL) in dioxane (8 mL) was heated with stirring at 100 to 110°C for 2 hours. After cooling of the reaction mixture, the solvent was distilled off under reduced pressure. The obtained residue was purified by silica gel column chromatography [eluent: 90-30% hexane/ethyl acetate] to obtain a yellow solid of ethyl 8-(benzyloxy)-5-(cyclohexylamino)-7-oxo-7,8-dihydropyrido[2,3-d]pyrimidine-6-carboxylate (63 mg).
$^1$H-NMR ($CDCl_3$) δ value: 1.20-1.58 (m, 8H), 1.58-1.70 (m, 1H), 1.75-1.88 (m, 2H), 2.00-2.14 (m, 2H), 3.75-3.87 (m, 1H), 4.43 (q, J=7.2 Hz, 2H), 5.25 (s, 2H), 7.32-7.42 (m, 3H), 7.64-7.72 (m, 2H), 9.04 (s, 1H), 9.08 (s, 1H), 9.58-9.68 (m, 1H).

Reference Example 17

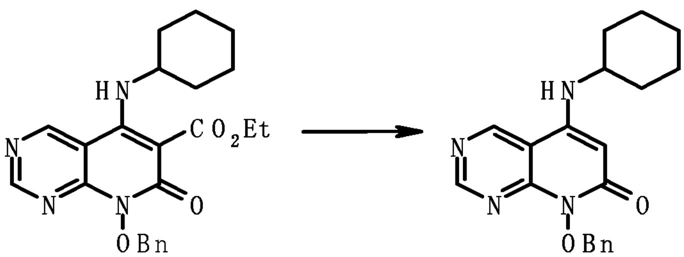

To ethyl 8-(benzyloxy)-5-(cyclohexylamino)-7-oxo-7,8-dihydropyrido[2,3-d]pyrimidine-6-carboxylate (63 mg), methanol (6 mL) and a 1 mol/L aqueous sodium hydroxide solution (6 mL) were added, and the mixture was heated with stirring at 90 to 100°C for 1 hour. After cooling of the reaction mixture, the solvent was distilled off under reduced pressure. To the obtained residue, a 1 mol/L aqueous sodium hydroxide solution (4 mL) was added, and the mixture was pH-adjusted to 3 by the dropwise addition of concentrated hydrochloric acid and stirred at room temperature for 10 minutes. The deposited solid was collected by filtration to obtain a yellow solid of 8-(benzyloxy)-5-(cyclohexylamino)pyrido[2,3-d]pyrimidin-7(8H)-one (31 mg).

$^1$H-NMR (DMSO-$d_6$) δ value: 1.10-1.48 (m, 5H), 1.60-1.70 (m, 1H), 1.70-1.80 (m, 2H), 1.90-2.05 (m, 2H), 3.30-3.48 (m, 1H), 5.12 (s, 2H), 5.57 (s, 1H), 7.09 (d, J=7.6 Hz, 1H), 7.37-7.50 (m, 3H), 7.55-7.70 (m, 2H), 9.08 (s, 1H), 9.45 (s, 1H).

Reference Example 18

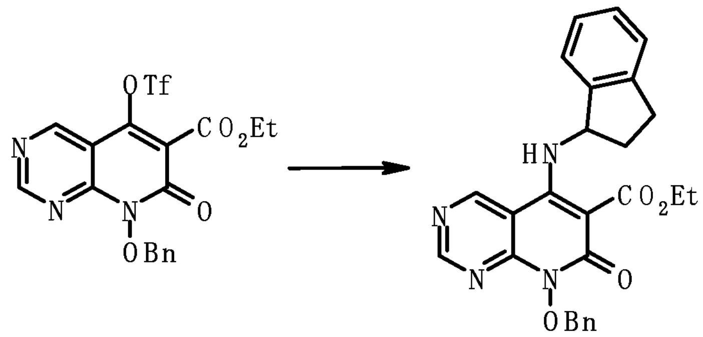

A solution of ethyl 8-(benzyloxy)-7-oxo-5-(((trifluoromethyl)sulfonyl)oxy)-7,8-dihydropyrido[2,3-d]pyrimidine-6-carboxylate (201 mg) and 1-aminoindane (272 μL) in dioxane (8 mL) was heated with stirring at 100 to 110°C for 40 minutes. After cooling of the reaction mixture, the solvent was distilled off under reduced pressure. The obtained residue was purified by silica gel column chromatography [eluent: 90-50% hexane/ethyl acetate] to obtain a pale yellow solid of ethyl 8-(benzyloxy)-5-((2,3-dihydro-1H-inden-1-yl)amino)-7-oxo-7,8-dihydropyrido[2,3-d]pyrimidine-6-carboxylate (136 mg).

$^1$H-NMR ($CDCl_3$) δ value: 1.39 (t, J=7.2 Hz, 3H), 2.14-2.28 (m, 1H), 2.72-2.85 (m, 1H), 2.90-3.05 (m, 1H), 3.05-3.18 (m, 1H), 4.37 (q, J=7.1 Hz, 2H), 5.29 (s, 2H), 5.37-5.50 (m, 1H), 7.20-7.35 (m, 4H), 7.35-7.45 (m, 3H), 7.66-7.78 (m, 2H), 9.07 (s, 1H), 9.31 (s, 1H), 9.55-9.70 (m, 1H).

Reference Example 19

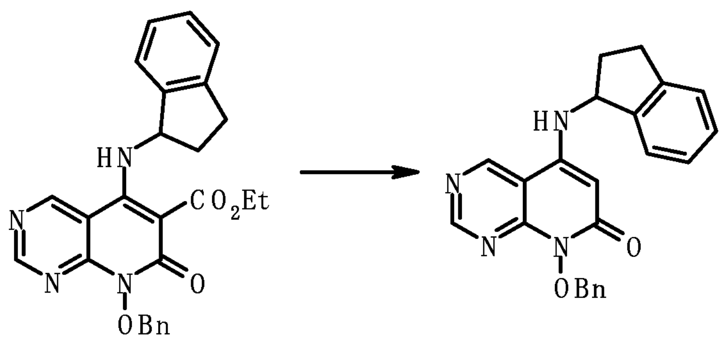

To ethyl 8-(benzyloxy)-5-((2,3-dihydro-1H-inden-1-yl)amino)-7-oxo-7,8-dihydropyrido[2,3-d]pyrimidine-6-carboxylate (135 mg), methanol (7 mL) and a 1 mol/L aqueous sodium hydroxide solution (7 mL) were added, and the mixture was heated with stirring at 70 to 80°C for 30 minutes. After cooling of the reaction mixture, the solvent was distilled off under reduced pressure. To the obtained residue, a 1 mol/L aqueous sodium hydroxide solution (5 mL) was added, and the mixture was pH-adjusted to 1 by the dropwise addition of concentrated hydrochloric acid and stirred at room temperature for 20 minutes. The deposit was collected by filtration to obtain a white solid of 8-(benzyloxy)-5-((2,3-dihydro-1H-inden-1-yl)amino)pyrido[2,3-d]pyrimidin-7(8H)-one (79 mg).
$^1$H-NMR (DMSO-$d_6$) δ value: 1.93-2.06 (m, 1H), 2.56-2.66 (m, 1H), 2.83-2.97 (m, 1H), 2.97-3.08 (m, 1H), 5.15 (s, 2H), 5.24 (q, J=7.6 Hz, 1H), 5.85 (s, 1H), 7.18-7.38 (m, 4H), 7.38-7.48 (m, 3H), 7.58-7.69 (m, 2H), 7.73 (d, J=8.0 Hz, 1H), 9.10 (s, 1H), 9.46 (s, 1H).

Reference Example 20

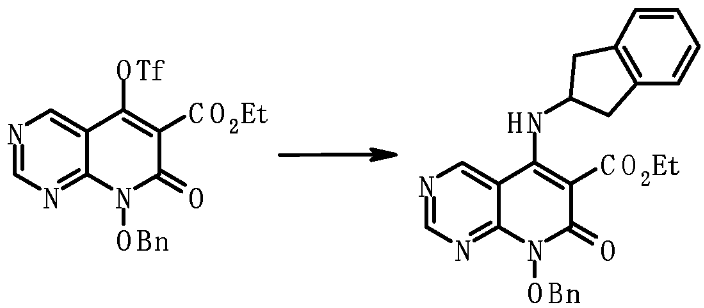

A solution of ethyl 8-(benzyloxy)-7-oxo-5-(((trifluoromethyl)sulfonyl)oxy)-7,8-dihydropyrido[2,3-d]pyrimidine-6-carboxylate (101 mg) and 2-aminoindane (138 μL) in dioxane (5 mL) was heated with stirring at 100 to 110°C for 30 minutes. After cooling of the reaction mixture, the solvent was distilled off under reduced pressure. The obtained residue was purified by silica gel column chromatography [eluent: 90-50% hexane/ethyl acetate] to obtain a yellow oil of ethyl 8-(benzyloxy)-5-((2,3-dihydro-1H-inden-2-yl)amino)-7-oxo-7,8-dihydropyrido[2,3-d]pyrimidine-6-carboxylate (55 mg).
$^1$H-NMR ($CDCl_3$) δ value: 1.38 (t, J=7.2 Hz, 3H), 3.08-3.18 (m, 2H), 3.42-3.53 (m, 2H), 4.36 (q, J=7.1 Hz, 2H), 4.72-4.84 (m, 1H), 5.25 (s, 2H), 7.12-7.30 (m, 4H), 7.32-7.42 (m, 3H), 7.64-7.72 (m, 2H), 9.05 (s, 1H), 9.23 (s, 1H), 9.62-9.70 (m, 1H).

Reference Example 21

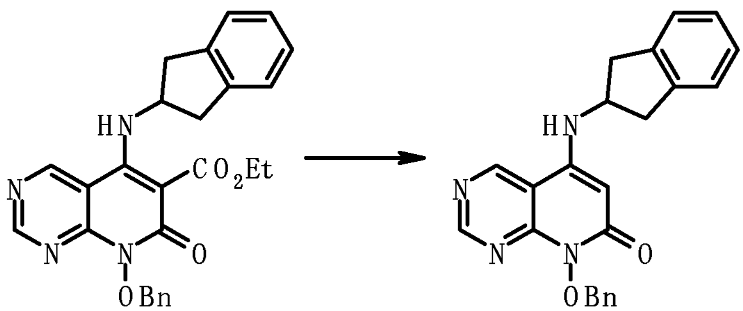

To ethyl 8-(benzyloxy)-5-((2,3-dihydro-1H-inden-2-yl)amino)-7-oxo-7,8-dihydropyrido[2,3-d]pyrimidine-6-carboxylate (55 mg), methanol (5 mL) and a 1 mol/L aqueous sodium hydroxide solution (5 mL) were added, and the mixture was heated with stirring at 80 to 90°C for 30 minutes. After cooling of the reaction mixture, the solvent was distilled off under reduced pressure. To the obtained residue, a 1 mol/L aqueous sodium hydroxide solution (5 mL) was added, and the mixture was pH-adjusted to 3 by the dropwise addition of concentrated hydrochloric acid and stirred at room temperature for 20 minutes. The deposit was collected by filtration to obtain a white solid of 8-(benzyloxy)-5-((2,3-dihydro-1H-inden-2-yl)amino)pyrido[2,3-d]pyrimidin-7(8H)-one (38 mg).
$^1$H-NMR (DMSO-$d_6$) δ value: 3.00-3.10 (m, 2H), 3.17-3.48 (m, 2H), 4.36-4.47 (m, 1H), 5.14 (s, 2H), 5.67 (s, 1H), 7.10-7.21 (m, 2H), 7.21-7.30 (m, 2H), 7.37-7.47 (m, 3H), 7.49 (d, J=6.4 Hz, 1H), 7.57-7.68 (m, 2H), 9.09 (s, 1H), 9.45 (s, 1H).

Reference Example 22

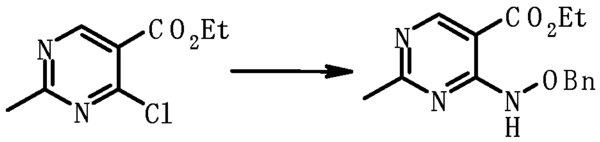

A suspension of ethyl 4-chloro-2-methylpyrimidine-5-carboxylate (1.00 g) and O-benzylhydroxylamine hydrochloride (1.35 g) in N-ethyldiisopropylamine (16 mL) was heated with stirring at 110 to 120°C for 3 hours. After cooling of the reaction mixture, chloroform and water were added thereto, followed by separation of an organic layer. An aqueous layer was extracted with chloroform, combined with the organic layer and dried over sodium sulfate, and the solvent was distilled off under reduced pressure. The obtained residue was purified by silica gel column chromatography [eluent: 90-50% hexane/ethyl acetate] to obtain a yellow oil of ethyl 4-((benzyloxy)amino)-2-methylpyrimidine-5-carboxylate (990 mg).
$^1$H-NMR ($CDCl_3$) δ value: 1.35 (t, J=7.2 Hz, 3H), 2.67 (s, 3H), 4.31 (q, J=7.2 Hz, 2H), 5.05 (s, 2H), 7.35-7.44 (m, 3H), 7.46-7.51 (m, 2H), 8.79 (s, 1H), 10.20 (brs, 1H).

Reference Example 23

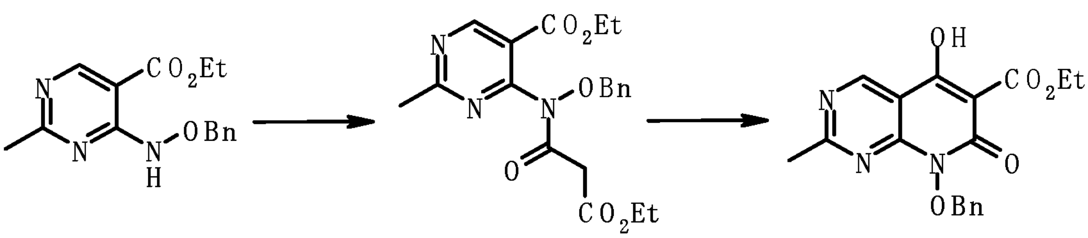

To a solution of ethyl 4-((benzyloxy)amino)-2-methylpyrimidine-5-carboxylate (0.99 g) and triethylamine (955 μL) in methylene chloride (15 mL), ethylmalonyl chloride (882 μL) was added dropwise under ice cooling, and the mixture was stirred at room temperature for 2 hours. To the reaction mixture, triethylamine (955 μL) was added, ethylmalonyl chloride (882 μL) was added dropwise under ice cooling, and the mixture was stirred at room temperature for 1 hour and 30 minutes. Insoluble matter was filtered off, and the solvent was distilled off under reduced pressure. The obtained residue was purified by silica gel column chromatography [eluent: 95-50% hexane/ethyl acetate] to obtain a yellow oil (2.56 g). To a solution of the obtained yellow oil (2.56 g) in ethanol (15 mL), a 20% solution of sodium ethoxide in ethanol (2.12 g) was added dropwise, and the mixture was stirred at room temperature for 4 hours. The reaction mixture was pH-adjusted to 3 by the addition of 2 mol/L hydrochloric acid and stirred at room temperature for 10 minutes. The deposit was collected by filtration to obtain a white solid of ethyl 8-(benzyloxy)-5-hydroxy-2-methyl-7-oxo-7,8-dihydropyrido[2,3-d]pyrimidine-6-carboxylate (865 mg).
$^1$H-NMR (DMSO-$d_6$) δ value: 1.31 (t, J=7.2 Hz, 3H), 2.75 (s, 3H), 4.33 (q, J=7.1 Hz, 2H), 5.14(s, 2H), 7.40-7.47 (m, 3H), 7.63-7.68 (m, 2H), 9.22 (s, 1H).

Reference Example 24

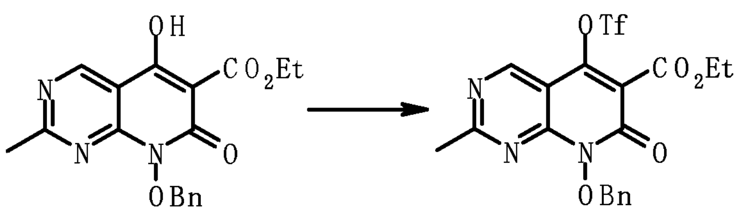

To a solution of ethyl 8-(benzyloxy)-5-hydroxy-2-methyl-7-oxo-7,8-dihydropyrido[2,3-d]pyrimidine-6-carboxylate (300 mg) and triethylamine (234 μL) in methylene chloride (8 mL), trifluoromethanesulfonic anhydride (277 μL) was added dropwise under ice cooling, and the mixture was stirred at room temperature for 1 hour and 20 minutes. The

reaction mixture was purified by silica gel column chromatography [eluent: 100-95% chloroform/methanol] to obtain a brown solid of ethyl 8-(benzyloxy)-2-methyl-7-oxo-5-(((trifluoromethyl)sulfonyl)oxy)-7,8-dihydropyrido[2,3-d]pyrimidine-6-carboxylate (312 mg). $^1$H-NMR ($CDCl_3$) δ value: 1.43 (t, J=7.1 Hz, 3H), 2.89 (s, 3H), 4.47 (q, J=7.2 Hz, 2H), 5.31 (s, 2H), 7.36-7.44 (m, 3H), 7.62-7.70 (m, 2H), 9.03 (s, 1H).

Reference Example 25

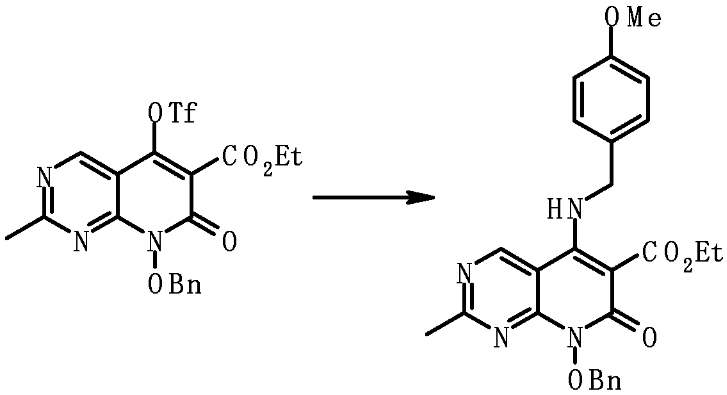

A solution of ethyl 8-(benzyloxy)-2-methyl-7-oxo-5-(((trifluoromethyl)sulfonyl)oxy)-7,8-dihydropyrido[2,3-d]pyrimidine-6-carboxylate (144 mg) and 4-methoxybenzylamine (192 μL) in dioxane (10 mL) was heated with stirring at 100 to 110°C for 1 hour and 30 minutes. After cooling of the reaction mixture, the solvent was distilled off under reduced pressure. The obtained residue was purified by silica gel column chromatography [eluent: 95-25% hexane/ethyl acetate] to obtain a brown oil of ethyl 8-(benzyloxy)-5-((4-methoxybenzyl)amino)-2-methyl-7-oxo-7,8-dihydropyrido[2,3-d]pyrimidine-6-carboxylate (108 mg).
$^1$H-NMR ($CDCl_3$) δ value: 1.44 (t, J=7.1 Hz, 3H), 2.79 (s, 3H), 3.85 (s, 3H), 4.42 (q, J=7.1 Hz, 2H), 4.81 (d, J=5.7 Hz, 2H), 5.28 (s, 2H), 6.94-6.98 (m, 2H), 7.27-7.32 (m, 2H), 7.37-7.44 (m, 3H), 7.70-7.74 (m, 2H), 9.07 (s, 1H), 9.95-10.05 (m, 1H).

Reference Example 26

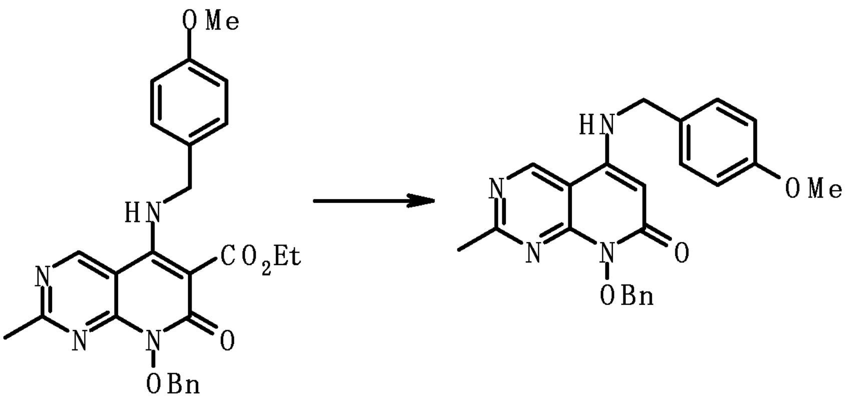

To ethyl 8-(benzyloxy)-5-((4-methoxybenzyl)amino)-2-methyl-7-oxo-7,8-dihydropyrido[2,3-d]pyrimidine-6-carboxylate (108 mg), methanol (7 mL) and a 1 mol/L aqueous sodium hydroxide solution (7 mL) were added, and the mixture was heated with stirring at 80 to 90°C for 10 minutes. After cooling of the reaction mixture, the solvent was distilled off under reduced pressure. To the obtained residue, a 1 mol/L aqueous sodium hydroxide solution (5 mL) was added, and the mixture was pH-adjusted to 2 by the dropwise addition of concentrated hydrochloric acid and stirred at room temperature for 10 minutes. The deposit was collected by filtration to obtain a brown solid of 8-(benzyloxy)-5-((4-methoxybenzyl)amino)-2-methylpyrido[2,3-d]pyrimidin-7(8H)-one (69 mg).

$^1$H-NMR (DMSO-$d_6$) δ value: 2.69 (s, 3H), 3.73 (s, 3H), 4.39 (d, J=5.5 Hz, 2H), 5.07 (s, 2H), 5.35 (s, 1H), 6.90-6.95 (m, 2H), 7.32 (d, J=8.6 Hz, 2H), 7.36-7.44 (m, 3H), 7.60-7.64 (m, 2H), 8.08-8.14 (m, 1H), 9.32 (s, 1H).

Reference Example 27

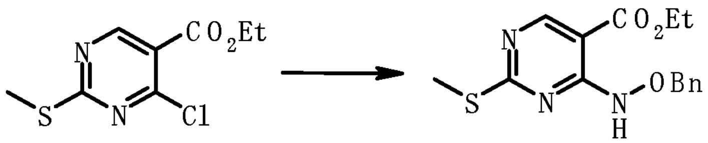

A suspension of ethyl 4-chloro-2-methylthio-5-pyrimidinecarboxylate (2.50 g) and O-benzylhydroxylamine hydrochloride (3.43 g) in N-ethyldiisopropylamine (15 mL) was heated with stirring at 100 to 110°C for 1 hour and 30 minutes. After cooling of the reaction mixture, chloroform and water were added thereto, followed by separation of an organic layer. An aqueous layer was extracted with chloroform, combined with the organic layer, washed with a saturated aqueous solution of sodium chloride and then dried over sodium sulfate, and the solvent was distilled off under reduced pressure. The obtained residue was purified by silica gel column chromatography [eluent: 97-60% hexane/ethyl acetate] to obtain a white solid of ethyl 4-((benzyloxy)amino)-2-(methylthio)pyrimidine-5-carboxylate (2.94 g).
$^1$H-NMR ($CDCl_3$) δ value: 1.35 (t, J=7.2 Hz, 3H), 2.61 (s, 3H), 4.30 (q, J=7.1 Hz, 2H), 5.06 (s, 2H), 7.34-7.43 (m, 3H), 7.43-7.48 (m, 2H), 8.67 (s, 1H), 10.30 (brs, 1H).

Reference Example 28

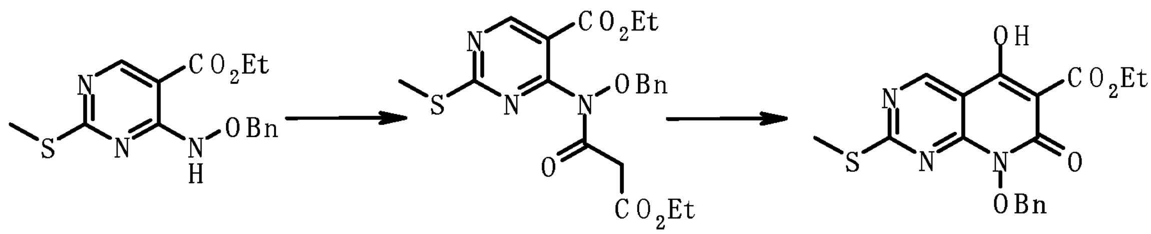

To a solution of ethyl 4-((benzyloxy)amino)-2-(methylthio)pyrimidine-5-carboxylate (2.89 g) and triethylamine (2.51 mL) in methylene chloride (29 mL), ethylmalonyl chloride (2.32 mL) was added dropwise under ice cooling, and the mixture was stirred at room temperature for 3 hours and 30 minutes. The solvent in the reaction mixture was distilled off under reduced pressure. The obtained residue was purified by silica gel column chromatography [eluent: 85-50% hexane/ethyl acetate] to obtain a yellow oil (2.69 g). To a solution of the obtained yellow oil (2.69 g) in ethanol (54 mL), a 20% solution of sodium ethoxide in ethanol (5.28 g) was added dropwise, and the mixture was stirred at room temperature for 2 hours and 10 minutes. The reaction mixture was pH-adjusted to 2 by the addition of 2 mol/L hydrochloric acid and stirred at room temperature for 20 minutes. The deposit was collected by filtration to obtain a brown solid of ethyl 8-(benzyloxy)-5-hydroxy-2-(methylthio)-7-oxo-7,8-dihydropyrido[2,3-d]pyrimidine-6-carboxylate (889 mg).
$^1$H-NMR ($CDCl_3$) δ value: 1.49 (t, J=7.1 Hz, 3H), 2.67 (s, 3H), 4.53 (q, J=7.1 Hz, 2H), 5.25 (s, 2H), 7.37-7.44 (m, 3H), 7.60-7.68 (m, 2H), 9.07 (s, 1H), 14.34 (s, 1H).

Reference Example 29

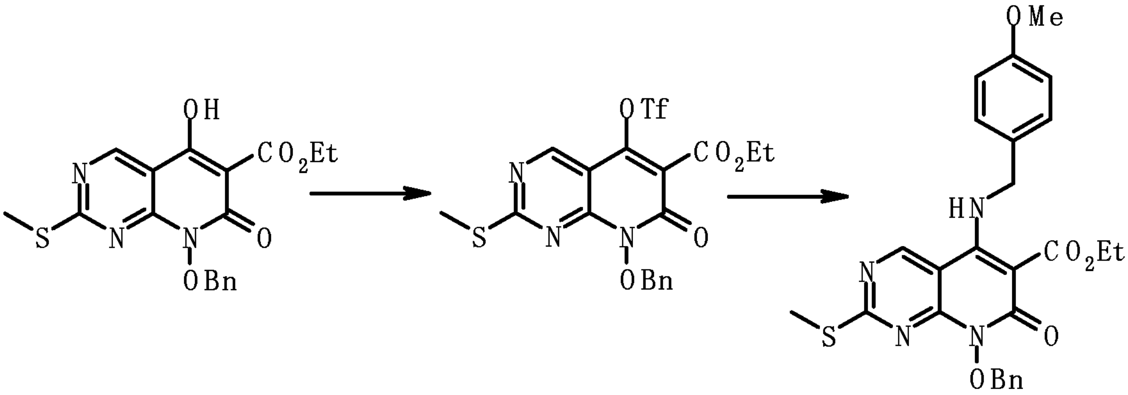

To a solution of ethyl 8-(benzyloxy)-5-hydroxy-2-(methylthio)-7-oxo-7,8-dihydropyrido[2,3-d]pyrimidine-6-carboxylate (481 mg) and triethylamine (344 μL) in methylene chloride (10 mL), trifluoromethanesulfonic anhydride (407 μL) was added dropwise under ice cooling, and the mixture was stirred at room temperature for 3 hours. The solvent in the reaction mixture was distilled off under reduced pressure. The obtained residue was purified by silica gel column chromatography [eluent: 80-50% hexane/ethyl acetate] to obtain a yellow oil (682 mg). A solution of the obtained yellow oil (682 mg) and 4-methoxybenzylamine (852 μL) in dioxane (15 mL) was heated with stirring at 110 to 120°C for 1 hour and 30 minutes. The solvent in the reaction mixture was distilled off under reduced pressure. The obtained residue was suspended in a mixed solvent of methanol and diisopropyl ether, and the deposit was collected by filtration to obtain a white solid of ethyl 8-(benzyloxy)-5-((4-methoxybenzyl)amino)-2-(methylthio)-7-oxo-7,8-dihydropyrido[2,3-d]pyrimidine-6-carboxylate (519 mg).
$^1$H-NMR ($CDCl_3$) δ value: 1.41 (t, J=7.2 Hz, 3H), 2.63 (s, 3H), 3.82 (s, 3H), 4.39 (q, J=7.2 Hz, 2H), 4.76 (d, J=5.6 Hz, 2H), 5.25 (s, 2H), 6.90-6.96 (m, 2H), 7.24-7.28 (m, 3H), 7.36-7.42 (m, 2H), 7.61-7.67 (m, 2H), 8.90 (s, 1H), 10.05-10.18 (m, 1H).

Reference Example 30

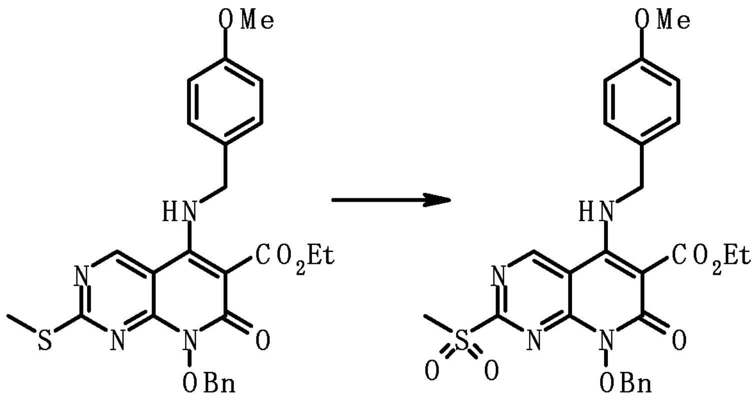

A solution of ethyl 8-(benzyloxy)-5-((4-methoxybenzyl)amino)-2-(methylthio)-7-oxo-7,8-dihydropyrido[2,3-d]pyrimidine-6-carboxylate (510 mg) and meta-chloroperbenzoic acid (677 mg) in methylene chloride (20 mL) was stirred at room temperature for 4 hours. To the reaction mixture, chloroform and a saturated aqueous solution of sodium bicarbonate were added, followed by separation of an organic layer. An aqueous layer was extracted with chloroform, combined with the organic layer and dried over sodium sulfate, and the solvent was distilled off under reduced pressure. The obtained residue was purified by silica gel column chromatography [eluent: 85-25% hexane/ethyl acetate] to obtain a yellow solid of ethyl 8-(benzyloxy)-5-((4-methoxybenzyl)amino)-2-(methylsulfonyl)-7-oxo-7,8-dihydropyrido[2,3-d]pyrimidine-6-carboxylate (387 mg).
$^1$H-NMR ($CDCl_3$) δ value: 1.43 (t, J=7.1 Hz, 3H), 3.34 (s, 3H), 3.83 (s, 3H), 4.42 (q, J=7.2 Hz, 2H), 4.79 (d, J=5.6 Hz, 2H), 5.27 (s, 2H), 6.95 (d, J=8.8 Hz, 2H), 7.24-7.29 (m, 2H), 7.33-7.46 (m, 3H), 7.60-7.68 (m, 2H), 9.24 (s, 1H), 10.00-10.10 (m, 1H).

Reference Example 31

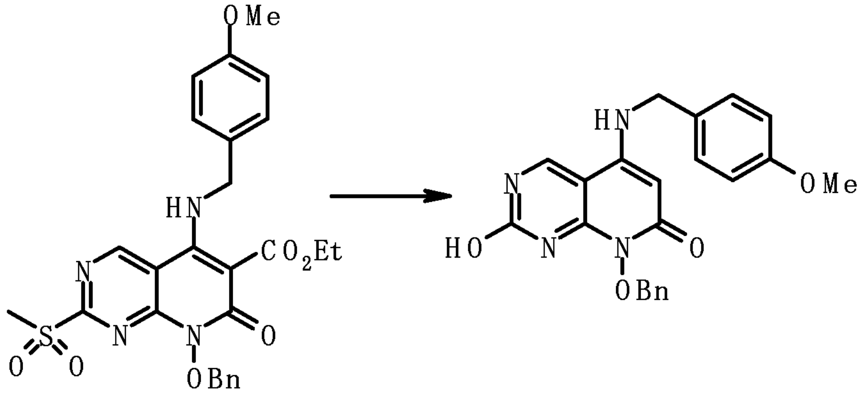

To ethyl 8-(benzyloxy)-5-((4-methoxybenzyl)amino)-2-(methylsulfonyl)-7-oxo-7,8-dihydropyrido[2,3-d]pyrimidine-6-carboxylate (143 mg), dioxane (10 mL) and a 1 mol/L aqueous sodium hydroxide solution (5 mL) were added, and the mixture was stirred at room temperature for 2 hours. The solvent in the reaction mixture was distilled off under reduced pressure. To the obtained residue, a 1 mol/L aqueous sodium hydroxide solution (5 mL) was added, and the mixture was heated to reflux for 40 minutes. The reaction mixture was cooled and then pH-adjusted to 3 by the dropwise addition of concentrated hydrochloric acid and stirred at room temperature for 10 minutes. The deposit was collected by filtration to obtain a white solid of 8-(benzyloxy)-2-hydroxy-5-((4-methoxybenzyl)amino)pyrido[2,3-d]pyrimidin-7(8H)-one (92 mg).
$^1$H-NMR (DMSO-$d_6$) δ value: 3.74 (s, 3H), 4.30 (d, J=5.6 Hz, 2H), 5.00 (s, 2H), 5.05 (s, 1H), 6.89-6.94 (m, 2H), 7.26-7.31 (m, 2H), 7.35-7.44 (m, 3H), 7.57-7.63 (m, 2H), 7.66-7.76 (m, 1H), 8.77 (s, 1H), 12.32 (brs, 1H).

Reference Example 32

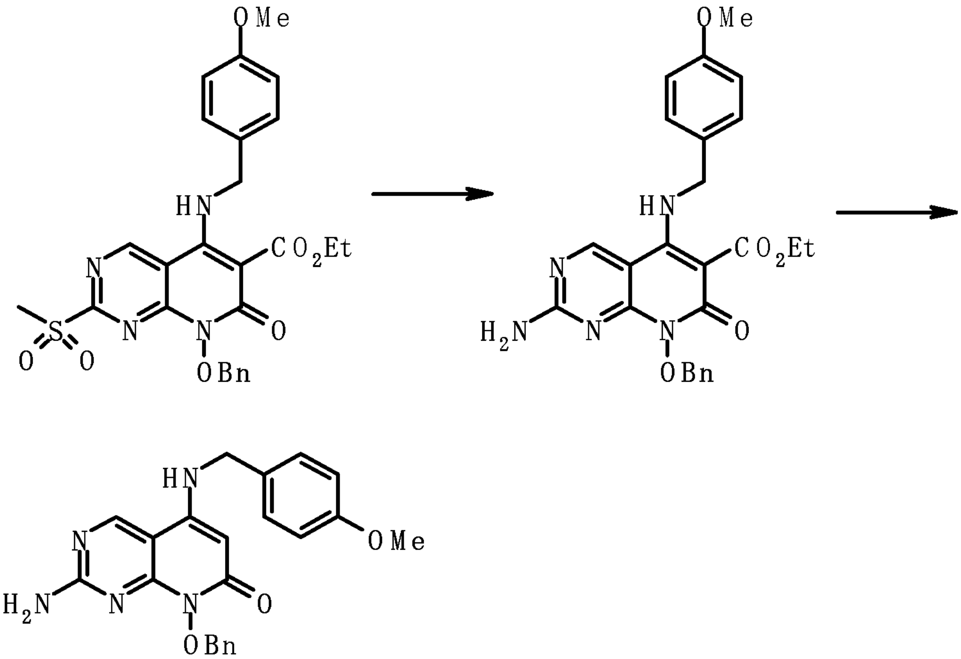

To ethyl 8-(benzyloxy)-5-((4-methoxybenzyl)amino)-2-(methylsulfonyl)-7-oxo-7,8-dihydropyrido[2,3-d]pyrimidine-6-carboxylate (72 mg), ammonium chloride (18 mg), dioxane (10 mL) and N-ethyldiisopropylamine (115 μL) were added, and the mixture was heated with stirring at 50 to 60°C for 2 hours and 30 minutes. After cooling of the reaction

mixture, the solvent was distilled off under reduced pressure. The obtained residue was purified by silica gel column chromatography [eluent: 100-90% chloroform/methanol] to obtain a yellow solid (24 mg). To the obtained yellow solid (24 mg), methanol (5 mL) and a 1 mol/L aqueous sodium hydroxide solution (5 mL) were added, and the mixture was heated to reflux for 2 hours and 30 minutes. After cooling of the reaction mixture, the solvent was distilled off under reduced pressure. To the obtained residue, water (5 mL) was added, and the mixture was stirred at room temperature for 20 minutes. The deposit was collected by filtration to obtain a white solid of 2-amino-8-(benzyloxy)-5-((4-methoxybenzyl)amino)pyrido[2,3-d]pyrimidin-7(8H)-one (18 mg).
$^1$H-NMR (DMSO-$d_6$) δ value: 3.73 (s, 3H), 4.32 (d, J=5.6 Hz, 2H), 5.01 (s, 2H), 5.05 (s, 1H), 6.91 (d, J=8.8 Hz, 2H), 7.24 (brs, 2H), 7.29 (d, J=8.8 Hz, 2H), 7.33-7.46 (m, 3H), 7.55-7.70 (m, 2H), 7.70-7.82 (m, 1H), 8.94 (s, 1H).

Reference Example 33

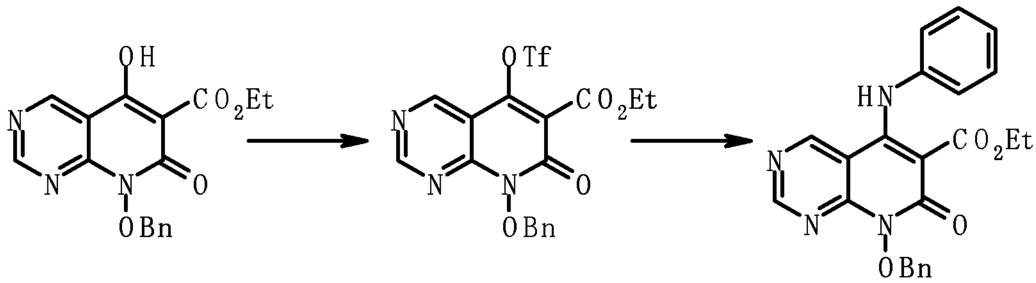

To a solution of ethyl 8-(benzyloxy)-5-hydroxy-7-oxo-7,8-dihydropyrido[2,3-d]pyrimidine-6-carboxylate (100 mg) and triethylamine (81 μL) in methylene chloride (5 mL), trifluoromethanesulfonic anhydride (96 μL) was added dropwise under ice cooling, and the mixture was stirred at room temperature for 40 minutes. The solvent in the reaction mixture was distilled off under reduced pressure. To the obtained residue, aniline (134 μL) and dioxane (7 mL) were added, and the mixture was heated with stirring at 80 to 90°C for 1 hour and 30 minutes. After cooling of the reaction mixture, the solvent was distilled off under reduced pressure. The obtained residue was purified by silica gel column chromatography [eluent: 85-50% hexane/ethyl acetate] and then suspended in a mixed solvent of 2-propanol and diisopropyl ether, and the deposit was collected by filtration to obtain a yellow solid of ethyl 5-anilino-8-(benzyloxy)-7-oxo-7,8-dihydropyrido[2,3-d]pyrimidine-6-carboxylate (52 mg).
$^1$H-NMR (DMSO-$d_6$) δ value: 1.03 (t, J=7.2 Hz, 3H), 3.44 (q, J=7.2 Hz, 2H), 5.17 (s, 2H), 7.12-7.21 (m, 3H), 7.31-7.39 (m, 2H), 7.39-7.51 (m, 3H), 7.58-7.73 (m, 2H), 9.20 (s, 1H), 9.40 (s, 1H), 9.48 (s, 1H).

Reference Example 34

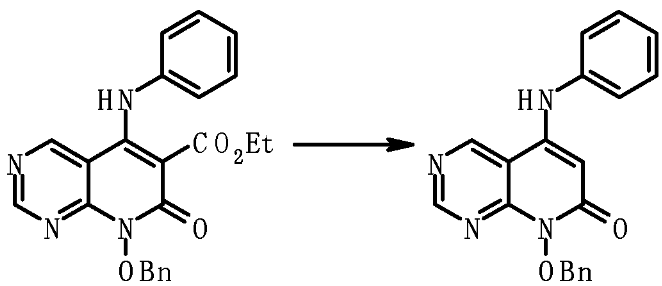

To ethyl 5-anilino-8-(benzyloxy)-7-oxo-7,8-dihydropyrido[2,3-d]pyrimidine-6-carboxylate (48 mg), methanol (5 mL) and a 1 mol/L aqueous sodium hydroxide solution (5 mL) were added, and the mixture was heated with stirring at 50 to 60°C for 2 hours and 30 minutes. After cooling of the reaction mixture, the solvent was distilled off under reduced pressure. The obtained residue was purified by silica gel column chromatography [eluent: 75-25% hexane/ethyl acetate] to obtain a white solid of 5-anilino-8-(benzyloxy)pyrido[2,3-d]pyrimidin-7(8H)-one (10 mg).
$^1$H-NMR ($CDCl_3$) δ value: 5.28 (s, 2H), 6.15 (s, 1H), 6.51 (brs, 1H), 7.22-7.33 (m, 3H), 7.33-7.41 (m, 3H), 7.41-7.51 (m, 2H), 7.61-7.73 (m, 2H), 9.05 (s, 1H), 9.14 (s, 1H).

Reference Example 35

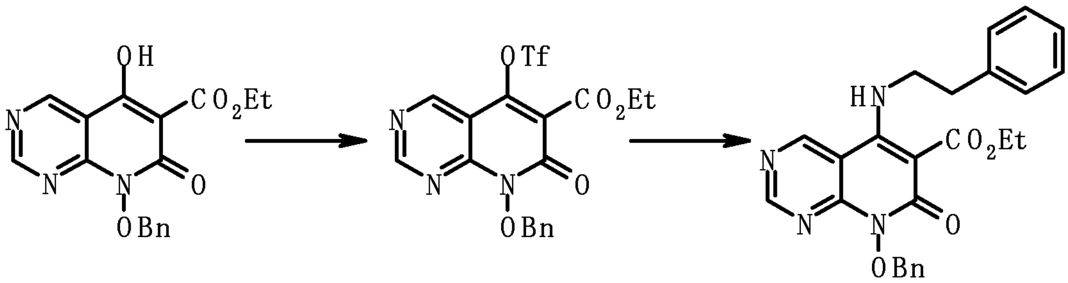

To a solution of ethyl 8-(benzyloxy)-5-hydroxy-7-oxo-7,8-dihydropyrido[2,3-d]pyrimidine-6-carboxylate (100 mg) and triethylamine (81 μL) in methylene chloride (5 mL), trifluoromethanesulfonic anhydride (96 μL) was added dropwise under ice cooling, and the mixture was stirred at room temperature for 4 hours. The solvent in the reaction mixture was distilled off under reduced pressure. To the obtained residue, phenethylamine (185 μL) and dioxane (7 mL) were added, and the mixture was heated with stirring at 80 to 90°C for 3 hours. After cooling of the reaction mixture, the solvent was distilled off under reduced pressure. The obtained residue was purified by silica gel column chromatography [eluent: 90-40% hexane/ethyl acetate] to obtain a yellow oil of ethyl 8-(benzyloxy)-7-oxo-5-((2-phenylethyl)amino)-7,8-dihydropyrido[2,3-d]pyrimidine-6-carboxylate (92 mg).
$^1$H-NMR ($CDCl_3$) δ value: 1.42 (t, J=7.2 Hz, 3H), 3.06 (t, J=7.1 Hz, 2H), 3.88-4.00 (m, 2H), 4.42 (q, J=7.2 Hz, 2H), 5.23 (s, 2H), 7.12-7.46 (m, 8H), 7.63-7.74 (m, 2H), 9.02 (s, 1H), 9.12 (s, 1H), 9.45-9.60 (m, 1H).

Reference Example 36

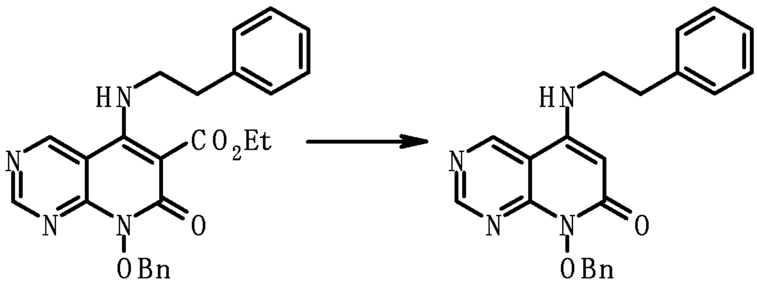

To ethyl 8-(benzyloxy)-7-oxo-5-((2-phenylethyl)amino)-7,8-dihydropyrido[2,3-d]pyrimidine-6-carboxylate (92 mg), methanol (7 mL) and a 1 mol/L aqueous sodium hydroxide solution (7 mL) were added, and the mixture was heated with stirring at 50 to 60°C for 1 hour. After cooling of the reaction mixture, the solvent was distilled off under reduced pressure. To the obtained residue, a 1 mol/L aqueous sodium hydroxide solution (5 mL) was added, and the mixture was pH-adjusted to 1 by the dropwise addition of concentrated hydrochloric acid and stirred at room temperature for 20 minutes. The deposit was collected by filtration to obtain a pale yellow solid of 8-(benzyloxy)-5-((2-phenylethyl)amino)pyrido[2,3-d]pyrimidin-7(8H)-one (48 mg).
$^1$H-NMR (DMSO-$d_6$) δ value: 2.96 (t, J=7.4 Hz, 2H), 3.41-3.52 (m, 2H), 5.13 (s, 2H), 5.60 (s, 1H), 7.12-7.28 (m, 3H), 7.28-7.37 (m, 1H), 7.37-7.48 (m, 4H), 7.54-7.61 (m, 1H), 7.61-7.68 (m, 2H), 9.09 (s, 1H), 9.35 (s, 1H).

Reference Example 37

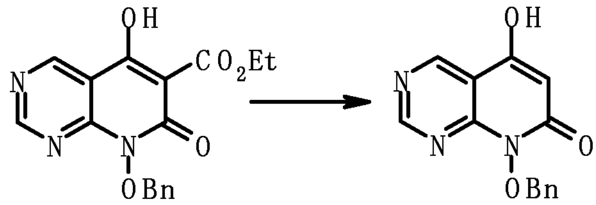

To ethyl 8-(benzyloxy)-5-hydroxy-7-oxo-7,8-dihydropyrido[2,3-d]pyrimidine-6-carboxylate (100 mg), 2 mol/L hydrochloric acid (5 mL) and dioxane (5 mL) were added, and the mixture was heated to reflux for 2 hours. After cooling of the reaction mixture, the solvent was distilled off under reduced pressure. To the obtained residue, ethyl acetate and water were added. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain an orange solid of 8-(benzyloxy)-5-hydroxypyrido[2,3-d]pyrimidin-7(8H)-one (36 mg).
$^1$H-NMR (DMSO-$d_6$) δ value: 5.16 (s, 2H), 5.96 (s, 1H), 7.37-7.47 (m, 3H), 7.59-7.67 (m, 2H), 9.11 (s, 1H), 9.17 (s, 1H), 12.30 (brs, 1H).

Reference Example 38

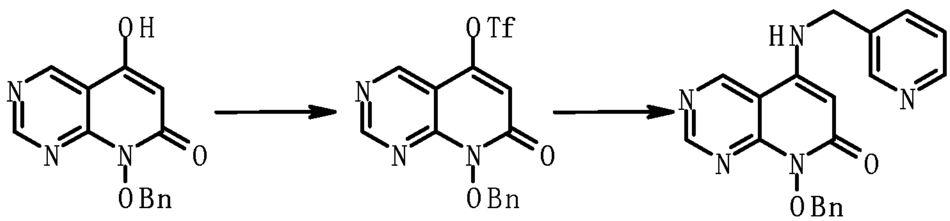

To a solution of 8-(benzyloxy)-5-hydroxypyrido[2,3-d]pyrimidin-7(8H)-one (80 mg) and triethylamine (82 μL) in methylene chloride (5 mL), trifluoromethanesulfonic anhydride (97 μL) was added dropwise under ice cooling, and the mixture was stirred at room temperature for 1 hour and 20 minutes. The solvent in the reaction mixture was distilled off under reduced pressure. To the obtained residue, 3-picolylamine (150 μL) and dioxane (6 mL) were added, and the mixture was heated with stirring at 100 to 110°C for 1 hour. After cooling of the reaction mixture, the solvent was distilled off under reduced pressure. The obtained residue was purified by silica gel column chromatography [eluent: 100-85% chloroform/methanol] and then suspended in a mixed solvent of 2-propanol and diisopropyl ether, and the deposit was collected by filtration to obtain a yellow solid of 8-(benzyloxy)-5-(((pyridin-3-yl)methyl)amino)pyrido[2,3-d]pyrimidin-7(8H)-one (22 mg).
$^1$H-NMR (DMSO-$d_6$) δ value: 4.55 (d, J=5.6 Hz, 2H), 5.09 (s, 2H), 5.49 (s, 1H), 7.35-7.49 (m, 4H), 7.56-7.68 (m, 2H), 7.77-7.89 (m, 1H), 8.14-8.26 (m, 1H), 8.46-8.56 (m, 1H), 8.65 (brs, 1H), 9.12 (s, 1H), 9.42 (s, 1H).

Reference Example 39

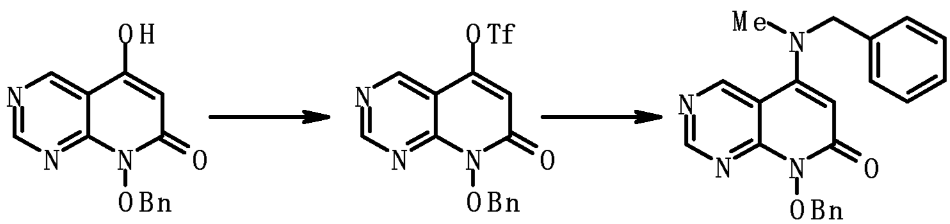

To a solution of 8-(benzyloxy)-5-hydroxypyrido[2,3-d]pyrimidin-7(8H)-one (100 mg) and triethylamine (103 μL) in methylene chloride (5 mL), trifluoromethanesulfonic anhydride (122 μL) was added dropwise under ice cooling, and the mixture was stirred at room temperature for 15 minutes. To the reaction mixture, N-methylbenzylamine (239 μL) was added, and the mixture was stirred at room temperature for 2 hours. The reaction mixture was purified by silica gel

column chromatography [eluent: 85-10% hexane/ethyl acetate] to obtain a brown solid of 5-(benzyl(methyl)amino)-8-(benzyloxy)pyrido[2,3-d]pyrimidin-7(8H)-one (53 mg).
$^1$H-NMR ($CDCl_3$) δ value: 2.88 (s, 3H), 4.49 (s, 2H), 5.30 (s, 2H), 6.08 (s, 1H), 7.28-7.46 (m, 8H), 7.65-7.75 (m, 2H), 9.02 (s, 1H), 9.05 (s, 1H).

Reference Example 40

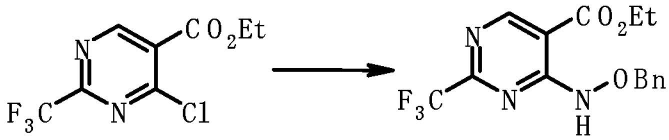

A suspension of ethyl 4-chloro-2-(trifluoromethyl)pyrimidine-5-carboxylate (1.00 g), O-benzylhydroxylamine hydrochloride (0.69 g), and potassium carbonate (1.19 g) in THF (15 mL) was stirred at room temperature for 3 hours. To the reaction mixture, ethyl acetate and water were added. An organic layer was separated, washed with a saturated aqueous solution of sodium chloride and then dried over sodium sulfate, and the solvent was distilled off under reduced pressure. The obtained residue was purified by silica gel column chromatography [eluent: 98-65% hexane/ethyl acetate] to obtain a white solid of ethyl 4-((benzyloxy)amino)-2-(trifluoromethyl)pyrimidine-5-carboxylate (1.27 g).
$^1$H-NMR ($CDCl_3$) δ value: 1.39 (t, J=7.2 Hz, 3H), 4.38 (q, J=7.2 Hz, 2H), 5.06 (s, 2H), 7.35-7.44 (m, 3H), 7.47-7.54 (m, 2H), 8.96 (s, 1H), 10.47 (s, 1H).

Reference Example 41

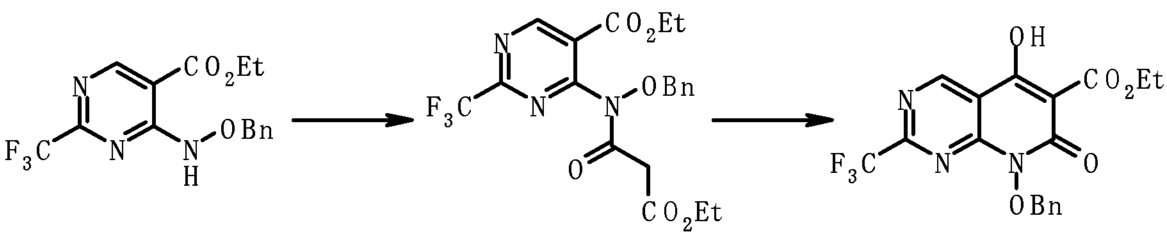

To a solution of ethyl 4-((benzyloxy)amino)-2-(trifluoromethyl)pyrimidine-5-carboxylate (1.27 g) and triethylamine (1.03 mL) in methylene chloride (11 mL), ethylmalonyl chloride (953 μL) was added dropwise under ice cooling, methylene chloride (4 mL) was added, and the mixture was stirred at room temperature for 1 hour and 30 minutes. To the reaction mixture, triethylamine (1.03 mL) was added under ice cooling, ethylmalonyl chloride (953 μL) was added dropwise, and the mixture was stirred at room temperature for 3 hours and 30 minutes. The solvent in the reaction mixture was distilled off under reduced pressure. The obtained residue was purified by silica gel column chromatography [eluent: 95-60% hexane/ethyl acetate] to obtain a yellow oil (1.54 g). To a solution of the obtained yellow oil (1.54 g) in ethanol (20 mL), a 20% solution of sodium ethoxide in ethanol (2.90 g) was added dropwise, and the mixture was stirred at room temperature for 1 hour and 30 minutes. The reaction mixture was pH-adjusted to 3 by the addition of 2 mol/L hydrochloric acid and stirred at room temperature for 10 minutes. The deposit was collected by filtration to obtain a white solid of ethyl 8-(benzyloxy)-5-hydroxy-7-oxo-2-(trifluoromethyl)-7,8-dihydropyrido[2,3-d]pyrimidine-6-carboxylate (0.57 g).
$^1$H-NMR (DMSO-$d_6$) δ value: 1.29 (t, J=7.1 Hz, 3H), 4.27 (q, J=7.2 Hz, 2H), 5.15 (s, 2H), 7.37-7.47 (m, 3H), 7.59-7.70 (m, 2H), 9.42 (s, 1H).

Reference Example 42

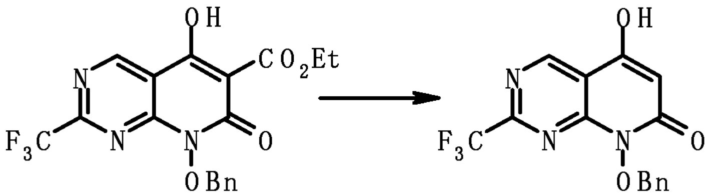

To ethyl 8-(benzyloxy)-5-hydroxy-7-oxo-2-(trifluoromethyl)-7,8-dihydropyrido[2,3-d]pyrimidine-6-carboxylate (100 mg), 2 mol/L hydrochloric acid (5 mL) and dioxane (5 mL) were added, and the mixture was heated to reflux for 3 hours and 30 minutes. After cooling of the reaction mixture, ethyl acetate and water were added thereto. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a white solid of 8-(benzyloxy)-5-hydroxy-2-(trifluoromethyl)pyrido[2,3-d]pyrimidin-7(8H)-one (12 mg).
$^1$H-NMR (DMSO-$d_6$) δ value: 5.17 (s, 2H), 6.06 (s, 1H), 7.35-7.49 (m, 3H), 7.57-7.68 (m, 2H), 9.27 (s, 1H), 12.57 (brs, 1H).

Reference Example 43

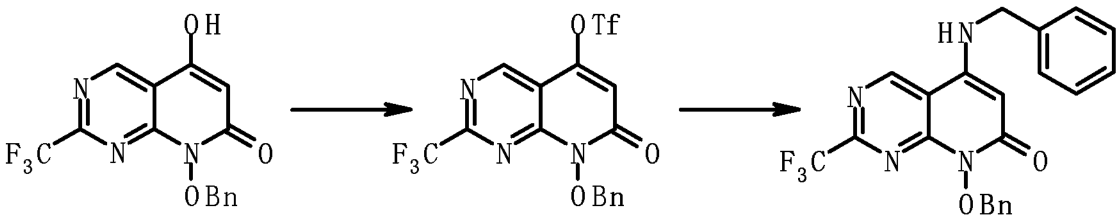

To a solution of 8-(benzyloxy)-5-hydroxy-2-(trifluoromethyl)pyrido[2,3-d]pyrimidin-7(8H)-one (48 mg) and triethylamine (39 μL) in methylene chloride (5 mL), trifluoromethanesulfonic anhydride (47 μL) was added dropwise under ice cooling, and the mixture was stirred at room temperature for 2 hours and 10 minutes. The solvent in the reaction mixture was distilled off under reduced pressure. To the obtained residue, benzylamine (78 μL) and dioxane (6 mL) were added, and the mixture was heated with stirring at 100 to 110°C for 1 hour and 20 minutes. After cooling of the reaction mixture, the solvent was distilled off under reduced pressure. The obtained residue was purified by silica gel column chromatography [eluent: 85-40% hexane/ethyl acetate] to obtain a white solid of 5-(benzylamino)-8-(benzyloxy)-2-(trifluoromethyl)pyrido[2,3-d]pyrimidin-7(8H)-one (31 mg). $^1$H-NMR ($CDCl_3$) δ value: 4.44 (d, J=4.9 Hz, 2H), 5.18-5.26 (m, 1H), 5.27 (s, 2H), 5.83 (s, 1H), 7.33-7.47 (m, 8H), 7.62-7.71 (m, 2H), 8.91 (s, 1H).

Reference Example 44

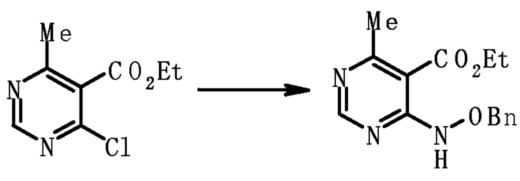

A suspension of ethyl 4-chloro-6-methylpyrimidine-5-carboxylate (517 mg) and O-benzylhydroxylamine hydrochloride (699 mg) in N-ethyldiisopropylamine (5 mL) was heated with stirring at 110 to 120°C for 1 hour and 30 minutes. After cooling of the reaction mixture, chloroform and water were added thereto, followed by separation of an organic layer. An aqueous layer was extracted with chloroform, combined with the organic layer, washed with a saturated aqueous solution of sodium chloride and then dried over sodium sulfate, and the solvent was distilled off under reduced pressure. The obtained residue was purified by silica gel column chromatography [eluent: 70-30% hexane/ethyl acetate] to obtain a yellow oil of ethyl 4-((benzyloxy)amino)-6-methylpyrimidine-5-carboxylate (637 mg).
$^1$H-NMR ($CDCl_3$) δ value: 1.35 (t, J=7.2 Hz, 3H), 2.66 (s, 3H), 4.32 (q, J=7.1 Hz, 2H), 5.02 (s, 2H), 7.33-7.50 (m, 5H),

8.66 (s, 1H), 10.04 (brs, 1H).

Reference Example 45

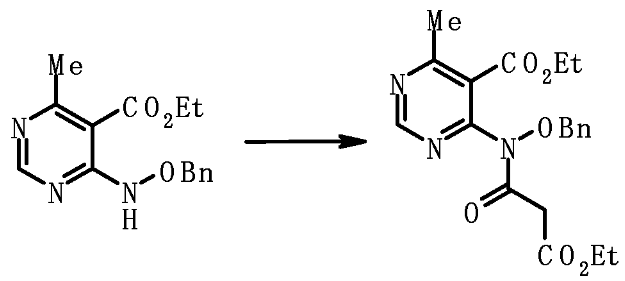

To a solution of ethyl 4-((benzyloxy)amino)-6-methylpyrimidine-5-carboxylate (637 mg) and triethylamine (610 μL) in methylene chloride (6 mL), ethylmalonyl chloride (560 μL) was added dropwise under ice cooling, and the mixture was stirred at room temperature for 3 hours and 30 minutes. The solvent was distilled off under reduced pressure, and the obtained residue was purified by silica gel column chromatography [eluent: 65-0% hexane/ethyl acetate] to obtain a yellow oil of ethyl 4-((benzyloxy)(3-ethoxy-3-oxopropanoyl)amino)-6-methylpyrimidine-5-carboxylate (834 mg).
$^1$H-NMR ($CDCl_3$) δ value: 1.20-1.40 (m, 6H), 2.64 (s, 3H), 3.72 (s, 2H), 4.10-4.32 (m, 4H), 4.96 (s, 2H), 7.31-7.40 (m, 3H), 7.41-7.48 (m, 2H), 8.88 (s, 1H).

Reference Example 46

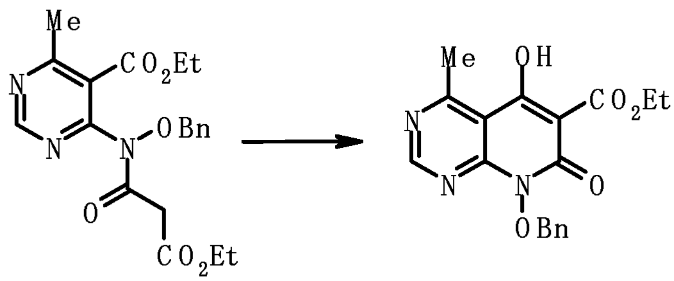

To a solution of ethyl 4-((benzyloxy)(3-ethoxy-3-oxopropanoyl)amino)-6-methylpyrimidine-5-carboxylate (834 mg) in ethanol (16 mL), a 20% solution of sodium ethoxide in ethanol (1.77 g) was added dropwise, and the mixture was stirred at room temperature for 1 hour and 30 minutes. The reaction mixture was pH-adjusted to 2 by the addition of 2 mol/L hydrochloric acid and stirred at room temperature for 30 minutes. The deposit was collected by filtration to obtain a yellow solid of ethyl 8-(benzyloxy)-5-hydroxy-4-methyl-7-oxo-7,8-dihydropyrido[2,3-d]pyrimidine-6-carboxylate (380 mg).
$^1$H-NMR ($CDCl_3$) δ value: 1.50 (t, J=7.2 Hz, 3H), 3.02 (s, 3H), 4.56 (q, J=7.2 Hz, 2H), 5.25 (s, 2H), 7.34-7.44 (m, 3H), 7.66-7.74 (m, 2H), 9.00 (s, 1H).

Reference Example 47

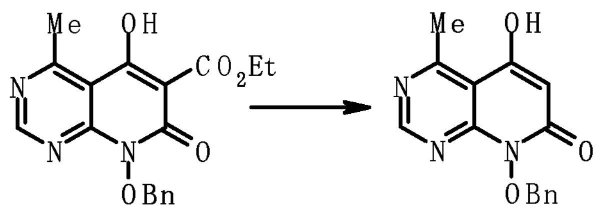

To ethyl 8-(benzyloxy)-5-hydroxy-4-methyl-7-oxo-7,8-dihydropyrido[2,3-d]pyrimidine-6-carboxylate (130 mg), 2 mol/L hydrochloric acid (6 mL) and dioxane (6 mL) were added, and the mixture was heated to reflux for 3 hours. After cooling of the reaction mixture, ethyl acetate and water were added thereto. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a white solid of 8-(benzyloxy)-5-hydroxy-4-methylpyrido[2,3-d]pyrimidin-7(8H)-one (90 mg).
$^1$H-NMR (DMSO-$d_6$) δ value: 2.87 (s, 3H), 5.12 (s, 2H), 5.94 (s, 1H), 7.37-7.49 (m, 3H), 7.58-7.69 (m, 2H), 8.95 (s, 1H), 12.16 (brs, 1H).

Reference Example 48

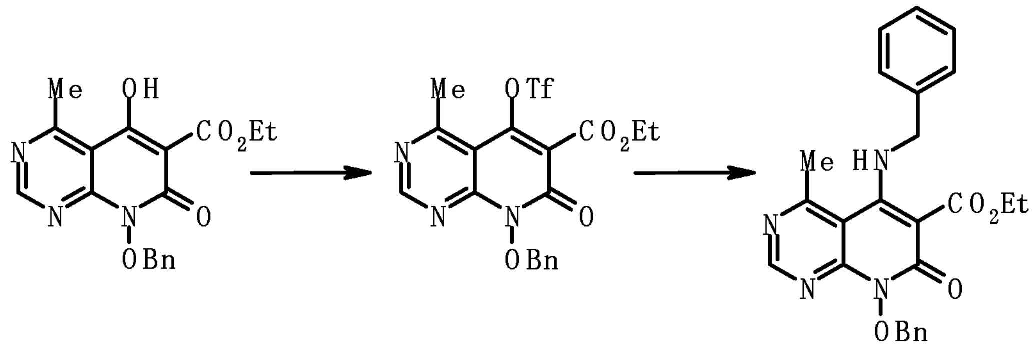

To a solution of ethyl 8-(benzyloxy)-5-hydroxy-4-methyl-7-oxo-7,8-dihydropyrido[2,3-d]pyrimidine-6-carboxylate (140 mg) and triethylamine (109 μL) in methylene chloride (7 mL), trifluoromethanesulfonic anhydride (130 μL) was added dropwise under ice cooling, and the mixture was stirred at room temperature for 55 minutes. The solvent in the reaction mixture was distilled off under reduced pressure. To the obtained residue, benzylamine (213 μL) and dioxane (10 mL) were added, and the mixture was stirred at room temperature for 3 hours. The solvent in the reaction mixture was distilled off under reduced pressure. The obtained residue was purified by silica gel column chromatography [eluent: 35-0% hexane/ethyl acetate] and then suspended in a mixed solvent of 2-propanol and diisopropyl ether, and the deposit was collected by filtration to obtain a yellow solid of ethyl 5-(benzylamino)-8-(benzyloxy)-4-methyl-7-oxo-7,8-dihydropyrido[2,3-d]pyrimidine-6-carboxylate (103 mg).
$^1$H-NMR ($CDCl_3$) δ value: 1.38 (t, J=7.1 Hz, 3H), 2.82 (s, 3H), 4.38 (q, J=7.1 Hz, 2H), 4.44 (d, J=5.6 Hz, 2H), 5.26 (s, 2H), 7.13-7.20 (m, 2H), 7.20-7.42 (m, 6H), 7.65-7.74 (m, 2H), 8.76-8.83 (m, 1H), 8.85 (s, 1H).

Reference Example 49

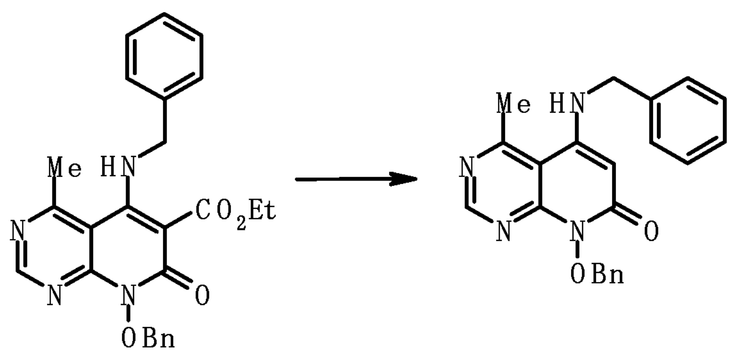

To ethyl 5-(benzylamino)-8-(benzyloxy)-4-methyl-7-oxo-7,8-dihydropyrido[2,3-d]pyrimidine-6-carboxylate (100 mg), methanol (5 mL) and a 1 mol/L aqueous sodium hydroxide solution (5 mL) were added, and the mixture was heated

with stirring at 50 to 60°C for 2 hours and 35 minutes, at 60 to 70°C for 30 minutes, and at 70 to 80°C for 1 hour and 50 minutes. After cooling of the reaction mixture, the solvent was distilled off under reduced pressure. To the obtained residue, a 1 mol/L aqueous sodium hydroxide solution (5 mL) was added, and the mixture was pH-adjusted to 2.1 by the dropwise addition of concentrated hydrochloric acid and stirred for 30 minutes under water cooling. The deposit was collected by filtration. The obtained deposit was suspended in a mixed solvent of 2-propanol and diisopropyl ether and stirred at 40°C for 20 minutes and at room temperature for 20 minutes, and the deposit was then collected by filtration to obtain a pale yellow solid of 5-(benzylamino)-8-(benzyloxy)-4-methylpyrido[2,3-d]pyrimidin-7(8H)-one (45 mg).
$^1$H-NMR ($CDCl_3$) δ value: 2.93 (s, 3H), 4.36-4.42 (m, 2H), 5.25 (s, 2H), 5.30-5.38 (m, 1H), 5.76 (s, 1H), 7.20-7.60 (m, 8H), 7.64-7.74 (m, 2H), 8.90 (s, 1H).

Reference Example 50

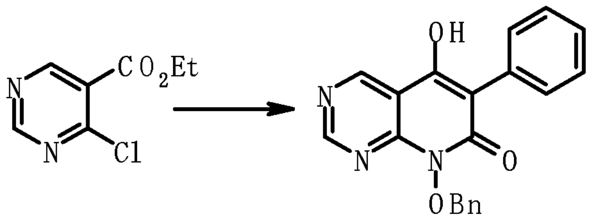

To a solution of ethyl 4-chloropyrimidine-5-carboxylate (490 mg) and N-(benzyloxy)-2-phenylacetamide (1.14 g) in DMF (10 mL), potassium carbonate (909 mg) was added under ice cooling, and the mixture was stirred for 5 hours under ice cooling and at room temperature for 1 hour and 40 minutes. To the reaction mixture, ethyl acetate and water were added. An aqueous layer was separated and pH-adjusted to 2 by the dropwise addition of 2 mol/L hydrochloric acid, and ethyl acetate was added thereto. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a pale yellow solid of 8-(benzyloxy)-5-hydroxy-6-phenylpylido[2,3-d]pyrimidin-7(8H)-one (330 mg).
$^1$H-NMR (DMSO-$d_6$) δ value: 5.20 (s, 2H), 7.34-7.52 (m, 8H), 7.61-7.69 (m, 2H), 9.18 (s, 1H), 9.28 (s, 1H), 11.00 (brs, 1H).

Reference Example 51

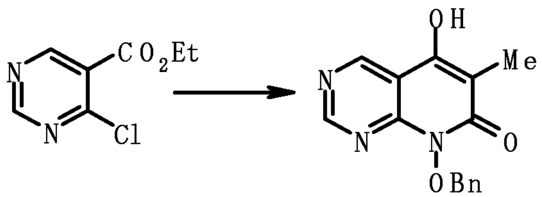

To a solution of ethyl 4-chloropyrimidine-5-carboxylate (507 mg) and N-(benzyloxy)propanamide (877 mg) in DMF (10 mL), potassium carbonate (933 mg) was added under ice cooling, and the mixture was stirred for 5 hours under ice cooling. To the reaction mixture, ethyl acetate and water were added. An aqueous layer was separated and pH-adjusted to 2 by the dropwise addition of 2 mol/L hydrochloric acid, and ethyl acetate was added thereto. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a white solid of 8-(benzyloxy)-5-hydroxy-6-methylpyrido[2,3-d]pyrimidin-7(8H)-one (78 mg).
$^1$H-NMR (DMSO-$d_6$) δ value: 2.08 (s, 3H), 5.16 (s, 2H), 7.38-7.48 (m, 3H), 7.60-7.70 (m, 2H), 9.12 (s, 1H), 9.20 (s, 1H), 11.17 (brs, 1H).

Reference Example 52

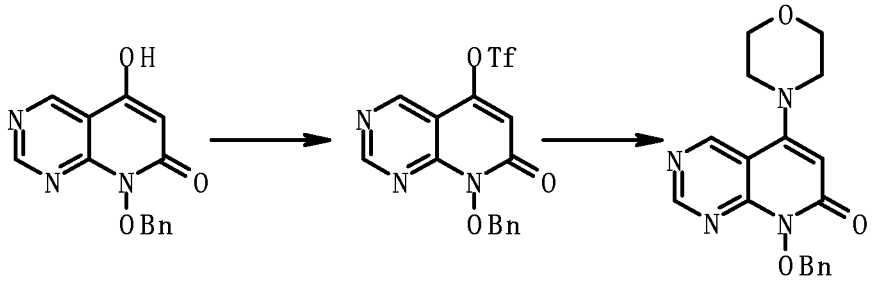

To a solution of 8-(benzyloxy)-5-hydroxypyrido[2,3-d]pyrimidin-7(8H)-one (100 mg) and triethylamine (103 μL) in methylene chloride (5 mL), trifluoromethanesulfonic anhydride (122 μL) was added dropwise under ice cooling, and the mixture was stirred at room temperature for 30 minutes. To the reaction mixture, morpholine (161 μL) was added, and the mixture was stirred at room temperature for 3 hours and 30 minutes. The reaction mixture was purified by silica gel column chromatography [eluent: 95-85% chloroform/methanol] and then suspended in a mixed solvent of 2-propanol and diisopropyl ether, and the deposit was collected by filtration to obtain a brown solid of 8-(benzyloxy)-5-(morpholin-4-yl)pyrido[2,3-d]pyrimidin-7(8H)-one (54 mg).
$^1$H-NMR ($CDCl_3$) δ value: 3.16-3.24 (m, 4H), 3.90-4.00 (m, 4H), 5.29 (s, 2H), 6.14 (s, 1H), 7.34-7.44 (m, 3H), 7.64-7.73 (m, 2H), 8.99 (s, 1H), 9.09 (s, 1H).

Reference Example 53

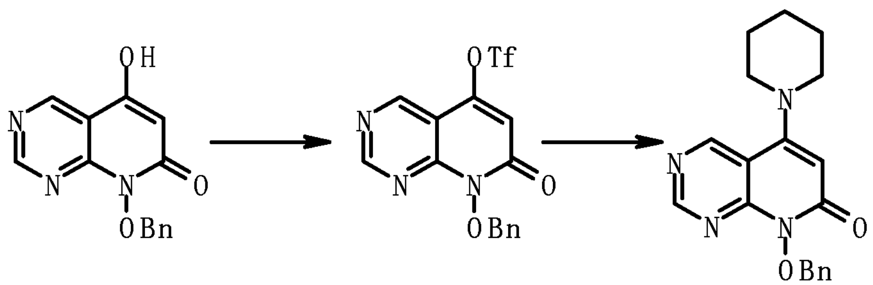

To a solution of 8-(benzyloxy)-5-hydroxypyrido[2,3-d]pyrimidin-7(8H)-one (100 mg) and triethylamine (103 μL) in methylene chloride (5 mL), trifluoromethanesulfonic anhydride (122 μL) was added dropwise under ice cooling, and the mixture was stirred at room temperature for 30 minutes. To the reaction mixture, piperidine (183 μL) was added dropwise, and the mixture was stirred at room temperature for 3 hours. The reaction mixture was purified by silica gel column chromatography [eluent: 65-0% hexane/ethyl acetate] to obtain a yellow oil of 8-(benzyloxy)-5-(piperidin-1-yl)pyrido[2,3-d]pyrimidin-7(8H)-one (32 mg). $^1$H-NMR ($CDCl_3$) δ value: 1.66-1.76 (m, 2H), 1.76-1.88 (m, 4H), 3.12-3.22 (m, 4H), 5.28 (s, 2H), 6.08 (s, 1H), 7.34-7.44 (m, 3H), 7.63-7.72 (m, 2H), 8.96 (s, 1H), 9.06 (s, 1H).

Reference Example 54

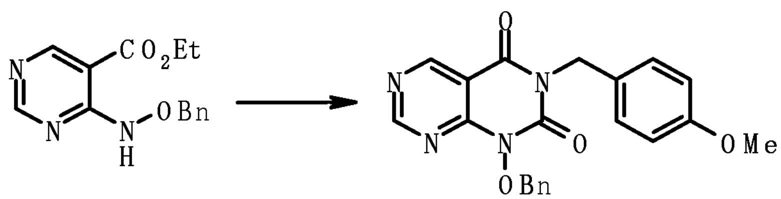

To a solution of ethyl 4-((benzyloxy)amino)pyrimidine-5-carboxylate (400 mg) in methylene chloride (12 mL), 4-methoxybenzyl isocyanate (439 μL) and triethylamine (428 μL) were added under ice cooling, and the mixture was stirred at room temperature for 5 hours. The solvent in the reaction mixture was distilled off under reduced pressure. To the obtained residue, ethyl acetate and a 10% aqueous citric acid solution were added. An organic layer was separated,

washed with a saturated aqueous solution of sodium chloride and dried over sodium sulfate, and the solvent was distilled off under reduced pressure. The obtained residue was suspended in ethanol, and the deposit was collected by filtration to obtain a white solid of 1-(benzyloxy)-3-(4-methoxybenzyl)pyrimido[4,5-d]pyrimidine-2,4(1H,3H)-dione (274 mg). $^1$H-NMR ($CDCl_3$) δ value: 3.79 (s, 3H), 5.16 (s, 2H), 5.28 (s, 2H), 6.82-6.88 (m, 2H), 7.36-7.41 (m, 3H), 7.45-7.50 (m, 2H), 7.57-7.62 (m, 2H), 9.18 (s, 1H), 9.29 (s, 1H).

Reference Example 55

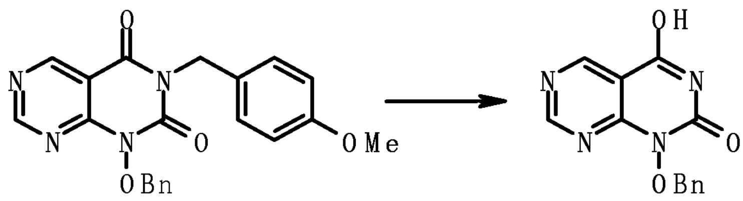

To a suspension of 1-(benzyloxy)-3-(4-methoxybenzyl)pyrimido[4,5-d]pyrimidine-2,4(1H,3H)-dione (97 mg) in acetonitrile (2.9 mL) and water (0.29 mL), CAN (0.68 g) was added, and the mixture was heated to reflux for 5 minutes. After cooling of the reaction mixture, water and ethyl acetate were added thereto. An organic layer was separated and washed with water, and a 5% aqueous potassium carbonate solution was added thereto, followed by separation of an aqueous layer. An organic layer was extracted with a 5% aqueous potassium carbonate solution and combined with the aqueous layer, and ethyl acetate was added thereto. The mixture was pH-adjusted to 3 by the addition of 6 mol/L hydrochloric acid, followed by separation of an organic layer. An aqueous layer was extracted with ethyl acetate, combined with the organic layer, washed with a saturated aqueous solution of sodium chloride, and dried over magnesium sulfate, and the solvent was distilled off under reduced pressure. The obtained residue was suspended in 2-propanol, and the deposit was collected by filtration to obtain a pale yellow solid of 1-(benzyloxy)-4-hydroxypyrimido[4,5-d]pyrimidin-2(1H)-one (22 mg).
$^1$H-NMR ($CDCl_3$) δ value: 5.30 (s, 2H), 7.38-7.44 (m, 3H), 7.59-7.65 (m, 2H), 8.24 (brs, 1H), 9.24 (s, 1H), 9.28 (s, 1H).

Reference Example 56

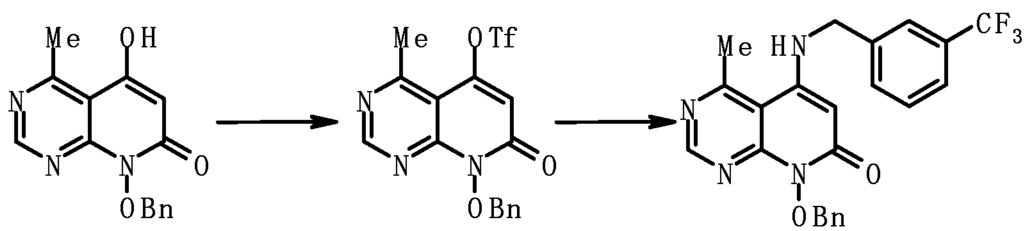

To a solution of 8-(benzyloxy)-5-hydroxy-4-methylpyrido[2,3-d]pyrimidin-7(8H)-one (50 mg) and triethylamine (50 μL) in methylene chloride (3 mL), trifluoromethanesulfonic anhydride (59 μL) was added dropwise under ice cooling, and the mixture was stirred for 1 hour under ice cooling. The solvent in the reaction mixture was distilled off under reduced pressure. To the obtained residue, 3-(trifluoromethyl)benzylamine (129 μL) and dioxane (5 mL) were added, and the mixture was stirred at room temperature for 3 hours and 40 minutes. The solvent in the reaction mixture was distilled off under reduced pressure. The obtained residue was purified by silica gel column chromatography [eluent: 100-90% chloroform/methanol] to obtain a colorless oil of 8-(benzyloxy)-4-methyl-5-((3-(trifluoromethyl)benzyl)amino)pyrido[2,3-d]pyrimidin-7(8H)-one (20 mg).
$^1$H-NMR ($CD_3OD$) δ value: 3.05 (s, 3H), 4.62 (s, 2H), 5.16 (s, 2H), 5.55 (s, 1H), 6.61 (s, 1H), 7.27-7.42 (m, 3H), 7.52-7.66 (m, 4H), 7.66-7.76 (m, 2H), 8.85 (s, 1H).

Reference Example 57

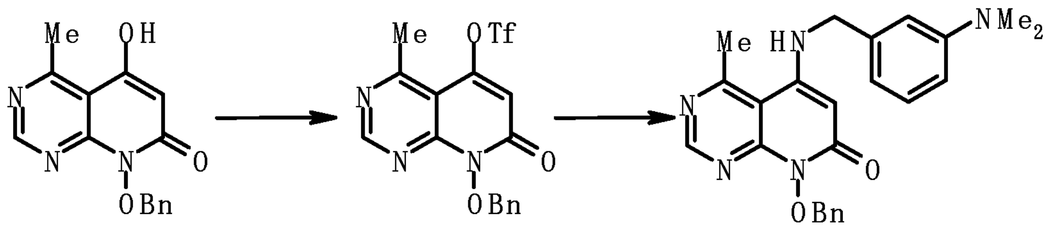

To a solution of 8-(benzyloxy)-5-hydroxy-4-methylpyrido[2,3-d]pyrimidin-7(8H)-one (75 mg) and triethylamine (73 μL) in methylene chloride (5 mL), trifluoromethanesulfonic anhydride (87 μL) was added dropwise under ice cooling, and the mixture was stirred for 15 minutes under ice cooling and at room temperature for 45 minutes. The solvent in the reaction mixture was distilled off under reduced pressure. To the obtained residue, 3-(aminomethyl)-N,N-dimethylaniline (156 mg) and dioxane (7 mL) was added, and the mixture was heated with stirring at 70 to 80°C for 2 hours. After cooling of the reaction mixture, the solvent was distilled off under reduced pressure. The obtained residue was purified by silica gel column chromatography [eluent: 100-85% chloroform/methanol] and then suspended in a mixed solvent of 2-propanol and diisopropyl ether, and the deposit was collected by filtration to obtain a white solid of 8-(benzyloxy)-5-((3-(dimethylamino)benzyl)amino)-4-methylpyrido[2,3-d]pyrimidin-7(8H)-one (20 mg).
$^1$H-NMR ($CDCl_3$) δ value: 2.92 (s, 3H), 2.99 (s, 6H), 4.29 (d, J=4.4 Hz, 2H), 5.26 (s, 2H), 5.30-5.36 (m, 1H), 5.78 (s, 1H), 6.66-6.76 (m, 4H), 7.34-7.44 (m, 3H), 7.64-7.74 (m, 2H), 8.89 (s, 1H).

Reference Example 58

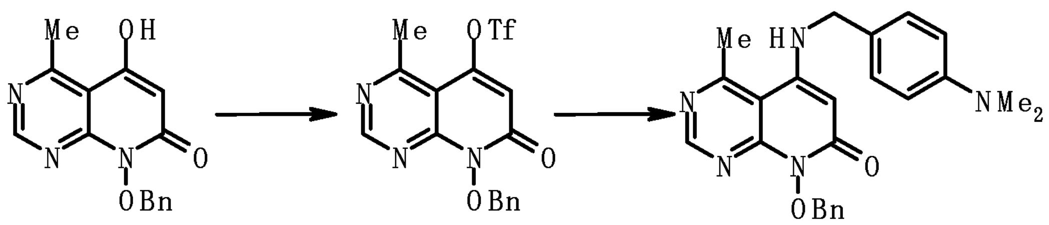

To a solution of 8-(benzyloxy)-5-hydroxy-4-methylpyrido[2,3-d]pyrimidin-7(8H)-one (125 mg) and triethylamine (122 μL) in methylene chloride (5 mL), trifluoromethanesulfonic anhydride (144 μL) was added dropwise under ice cooling, and the mixture was stirred at room temperature for 1 hour. The solvent in the reaction mixture was distilled off under reduced pressure. To the obtained residue, 4-(aminomethyl)-N,N-dimethylaniline (166 mg) and dioxane (10 mL) were added, and the mixture was heated with stirring at 70 to 80°C for 2 hours and 30 minutes. After cooling of the reaction mixture, the solvent was distilled off under reduced pressure. The obtained residue was purified by silica gel column chromatography [eluent: 100-90% chloroform/methanol] and then suspended in a mixed solvent of 2-propanol and diisopropyl ether, and the deposit was collected by filtration to obtain a white solid of 8-(benzyloxy)-5-((4-(dimethylamino)benzyl)amino)-4-methylpyrido[2,3-d]pyrimidin-7(8H)-one (23 mg).
$^1$H-NMR ($CDCl_3$) δ value: 2.88 (s, 3H), 2.98 (s, 6H), 4.23 (d, J=4.4 Hz, 2H), 5.17-5.24 (m, 1H), 5.26 (s, 2H), 5.78 (s, 1H), 6.72-6.79 (m, 2H), 7.22-7.45 (m, 5H), 7.64-7.72 (m, 2H), 8.88 (s, 1H).

Reference Example 59

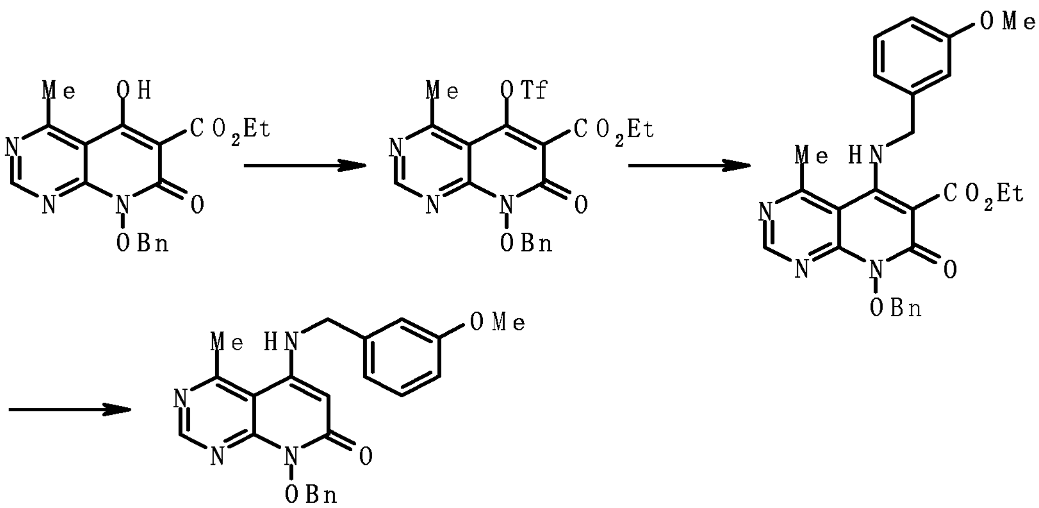

To a solution of ethyl 8-(benzyloxy)-5-hydroxy-4-methyl-7-oxo-7,8-dihydropyrido[2,3-d]pyrimidine-6-carboxylate (175 mg) and triethylamine (136 μL) in methylene chloride (5 mL), trifluoromethanesulfonic anhydride (161 μL) was added dropwise under ice cooling, and the mixture was stirred at room temperature for 1 hour and 10 minutes. The solvent in the reaction mixture was distilled off under reduced pressure. To the obtained residue, 3-methoxybenzylamine (318 μL) and dioxane (10 mL) were added, and the mixture was stirred at room temperature for 4 hours. The solvent in the reaction mixture was distilled off under reduced pressure. The obtained residue was purified by silica gel column chromatography [eluent: 35-0% hexane/ethyl acetate] to obtain a brown oil (177 mg). To the obtained brown oil (177 mg), methanol (9 mL) and a 1 mol/L aqueous sodium hydroxide solution (9 mL) were added, and the mixture was heated with stirring at 60 to 70°C for 3 hours and 30 minutes. After cooling of the reaction mixture, the solvent was distilled off under reduced pressure. To the obtained residue, a 1 mol/L aqueous sodium hydroxide solution (10 mL) was added, and the mixture was pH-adjusted to 2 by the dropwise addition of 2 mol/L hydrochloric acid, followed by addition of chloroform. An organic layer was separated, washed with a saturated aqueous solution of sodium chloride and then dried over sodium sulfate, and the solvent was distilled off under reduced pressure. The obtained residue was suspended in a mixed solvent of methanol, 2-propanol and diisopropyl ether, and the deposit was collected by filtration to obtain a light brown solid of 8-(benzyloxy)-5-((3-methoxybenzyl)amino)-4-methylpyrido[2,3-d]pyrimidin-7(8H)-one (83 mg).
$^1$H-NMR (DMSO-$d_6$) δ value: 3.03 (s, 3H), 3.75 (s, 3H), 4.46 (d, J=4.9 Hz, 2H), 5.07 (s, 2H), 5.41 (s, 1H), 6.82-6.88 (m, 1H), 6.97-7.05 (m, 3H), 7.25-7.33 (m, 1H), 7.36-7.45 (m, 3H), 7.57-7.65 (m, 2H), 8.89 (s, 1H).

Reference Example 60

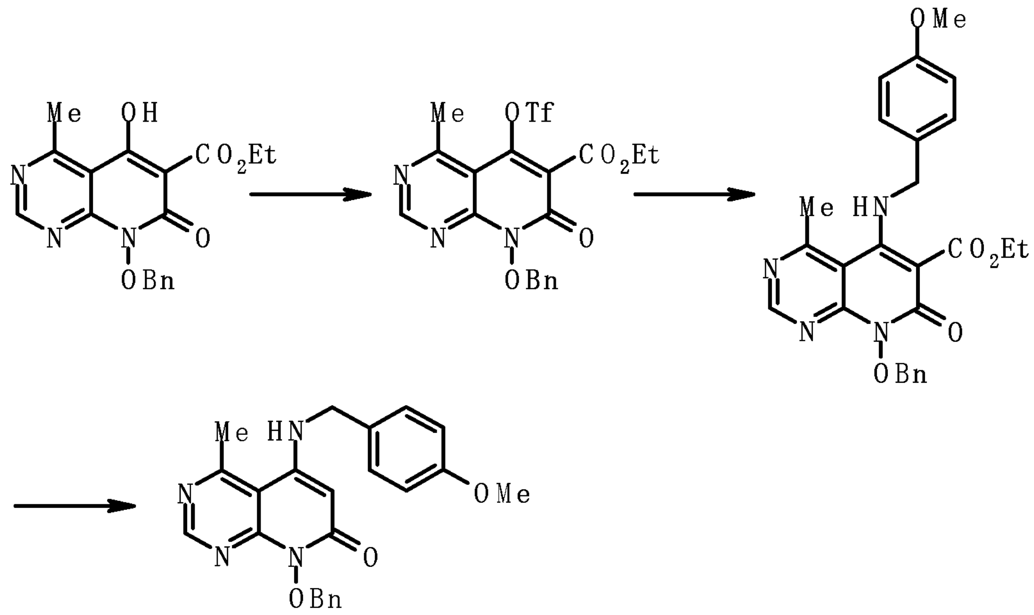

To a solution of ethyl 8-(benzyloxy)-5-hydroxy-4-methyl-7-oxo-7,8-dihydropyrido[2,3-d]pyrimidine-6-carboxylate (175 mg) and triethylamine (136 μL) in methylene chloride (5 mL), trifluoromethanesulfonic anhydride (161 μL) was added dropwise under ice cooling, and the mixture was stirred at room temperature for 50 minutes. The solvent in the reaction mixture was distilled off under reduced pressure. To the obtained residue, 4-methoxybenzylamine (318 μL) and dioxane (10 mL) were added, and the mixture was stirred at room temperature for 3 hours and 10 minutes. The solvent in the reaction mixture was distilled off under reduced pressure. The obtained residue was purified by silica gel column chromatography [eluent: 35-0% hexane/ethyl acetate] to obtain a brown oil (156 mg). To the obtained brown oil (156 mg), methanol (8 mL) and a 1 mol/L aqueous sodium hydroxide solution (8 mL) were added, and the mixture was heated with stirring at 60 to 70°C for 4 hours and 30 minutes. After cooling of the reaction mixture, the solvent was distilled off under reduced pressure. To the obtained residue, a 1 mol/L aqueous sodium hydroxide solution (10 mL) was added, and the mixture was pH-adjusted to 2 by the dropwise addition of 2 mol/L hydrochloric acid, followed by addition of chloroform. An organic layer was separated, washed with a saturated aqueous solution of sodium chloride and then dried over sodium sulfate, and the solvent was distilled off under reduced pressure. The obtained residue was suspended in a mixed solvent of methanol, 2-propanol and diisopropyl ether, and the deposit was collected by filtration to obtain a brown solid of 8-(benzyloxy)-5-((4-methoxybenzyl)amino)-4-methylpyrido[2,3-d]pyrimidin-7(8H)-one (52 mg).
$^1$H-NMR ($CDCl_3$) δ value: 2.90 (s, 3H), 3.84 (s, 3H), 4.29 (d, J=4.4 Hz, 2H), 5.21-5.30 (m, 3H), 5.76 (s, 1H), 6.91-6.99 (m, 2H), 7.21-7.45 (m, 5H), 7.66-7.75 (m, 2H), 8.89 (s, 1H).

Reference Example 61

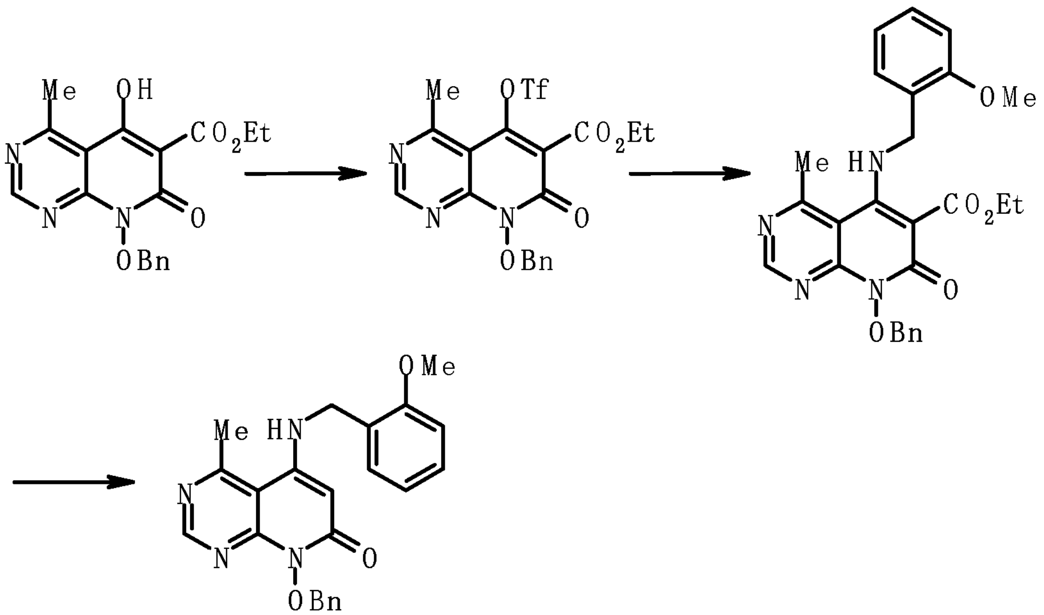

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (134 μL) was added dropwise under ice cooling, and the mixture was stirred at room temperature for 55 minutes. The solvent in the reaction mixture was distilled off under reduced pressure. To the obtained residue, 2-methoxybenzylamine (272 μL) and dioxane (10 mL) were added, and the mixture was stirred at room temperature for 4 hours. The solvent in the reaction mixture was distilled off under reduced pressure. The obtained residue was purified by silica gel column chromatography [eluent: 70-0% hexane/ethyl acetate] to obtain a yellow oil (175 mg). To the obtained yellow oil (175 mg), methanol (8 mL) and a 1 mol/L aqueous sodium hydroxide solution (8 mL) were added, and the mixture was heated with stirring at 60 to 70°C for 3 hours. After cooling of the reaction mixture, the solvent was distilled off under reduced pressure. To the obtained residue, a 1 mol/L aqueous sodium hydroxide solution (10 mL) was added, and the mixture was pH-adjusted to 2 by the dropwise addition of 2 mol/L hydrochloric acid, followed by addition of ethyl acetate. An organic layer was separated, washed with a saturated aqueous solution of sodium chloride and then dried over sodium sulfate, and the solvent was distilled off under reduced pressure. The obtained residue was suspended in a mixed solvent of methanol, 2-propanol and diisopropyl ether, and the deposit was collected by filtration to obtain a pale yellow solid of 8-(benzyloxy)-5-((2-methoxybenzyl)amino)-4-methylpyrido[2,3-d]pyrimidin-7(8H)-one (63 mg).
$^1$H-NMR ($CDCl_3$) δ value: 2.94 (s, 3H), 3.93 (s, 3H), 4.40 (d, J=5.1 Hz, 2H), 5.25 (s, 2H), 5.70-5.79 (m, 1H), 5.81 (s, 1H), 6.94-7.06 (m, 2H), 7.30-7.44 (m, 5H), 7.66-7.74 (m, 2H), 8.89 (s, 1H).

Reference Example 62

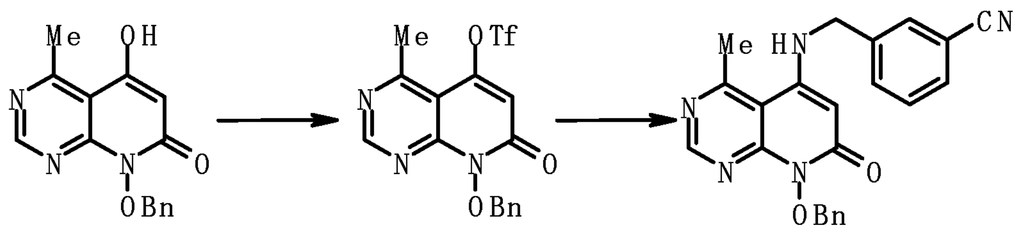

To a solution of 8-(benzyloxy)-5-hydroxy-4-methylpyrido[2,3-d]pyrimidin-7(8H)-one (150 mg) and triethylamine (147 μL) in methylene chloride (5 mL), trifluoromethanesulfonic anhydride (174 μL) was added dropwise under ice cooling, and the mixture was stirred at room temperature for 1 hour. The solvent in the reaction mixture was distilled off under reduced pressure. To the obtained residue, 3-(aminomethyl)benzonitrile (190 mg) and dioxane (10 mL) were added, and the mixture was heated with stirring at 60 to 70°C for 6 hours. After cooling of the reaction mixture, the solvent was distilled off under reduced pressure. The obtained residue was purified by silica gel column chromatography

[eluent: 98-90% chloroform/methanol] and then suspended in a mixed solvent of methanol, 2-propanol and diisopropyl ether, and the deposit was collected by filtration to obtain a pale yellow solid of 3-(((8-(benzyloxy)-4-methyl-7-oxo-7,8-dihydropyrido[2,3-d]pyrimidin-5-yl)amino)methyl)benzonitrile (11 mg).
$^1$H-NMR ($CDCl_3$) δ value: 3.01 (s, 3H), 4.50 (d, J=4.9 Hz, 2H), 5.25 (s, 2H), 5.42-5.50 (m, 1H), 5.63 (s, 1H), 7.24-7.45 (m, 3H), 7.50-7.58 (m, 1H), 7.59-7.74 (m, 5H), 8.93 (s, 1H).

Reference Example 63

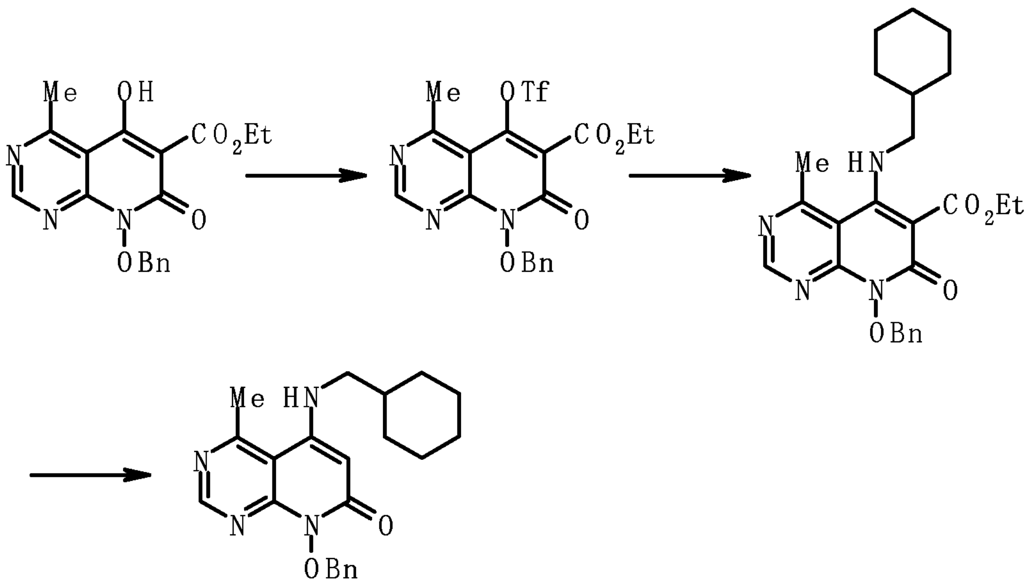

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (134 μL) was added dropwise under ice cooling, and the mixture was stirred at room temperature for 1 hour. The solvent in the reaction mixture was distilled off under reduced pressure. To the obtained residue, (cyclohexylmethyl)amine (273 μL) and dioxane (10 mL) were added, and the mixture was stirred at room temperature for 3 hours and 50 minutes. The solvent in the reaction mixture was distilled off under reduced pressure. The obtained residue was purified by silica gel column chromatography [eluent: 70-0% hexane/ethyl acetate] to obtain a yellow oil (138 mg). To the obtained yellow oil (138 mg), methanol (6 mL) and a 1 mol/L aqueous sodium hydroxide solution (6 mL) were added, and the mixture was heated with stirring at 60 to 70°C for 2 hours and 15 minutes. After cooling of the reaction mixture, the solvent was distilled off under reduced pressure. To the obtained residue, a 1 mol/L aqueous sodium hydroxide solution (10 mL) was added, and the mixture was pH-adjusted to 2 by the dropwise addition of 2 mol/L hydrochloric acid and stirred at room temperature for 10 minutes. The deposit was collected by filtration to obtain a pale yellow solid of 8-(benzyloxy)-5-((cyclohexylmethyl)amino)-4-methylpyrido[2,3-d]pyrimidin-7(8H)-one (75 mg).
$^1$H-NMR ($CDCl_3$) δ value: 1.00-1.40 (m, 5H), 1.66-1.92 (m, 6H), 2.98 (s, 3H), 3.02-3.08 (m, 2H), 5.10-5.18 (m, 1H), 5.25 (s, 2H), 5.66 (s, 1H), 7.32-7.45 (m, 3H), 7.65-7.74 (m, 2H), 8.88 (s, 1H).

Reference Example 64

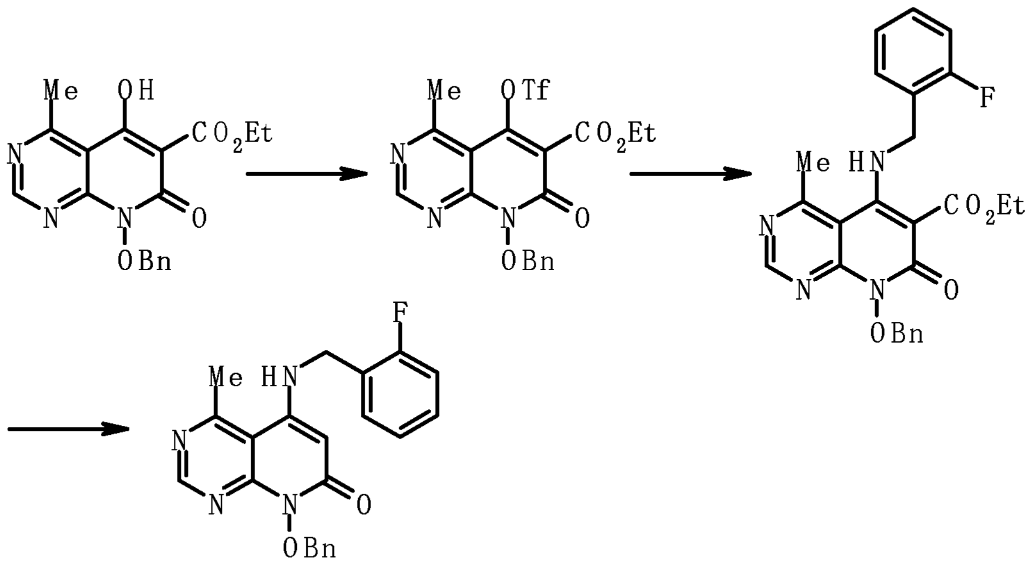

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 25 minutes. The solvent in the reaction mixture was distilled off under reduced pressure. To the obtained residue, 2-fluorobenzylamine (193 μL) and dioxane (7 mL) were added, and the mixture was stirred at room temperature for 1 hour and 30 minutes. The solvent in the reaction mixture was distilled off under reduced pressure. The obtained residue was purified by silica gel column chromatography [eluent: 85-30% hexane/ethyl acetate] to obtain a yellow oil (139 mg). To the obtained yellow oil (139 mg), methanol (8 mL) and a 1 mol/L aqueous sodium hydroxide solution (8 mL) were added, and the mixture was heated with stirring at 70 to 80°C for 3 hours. After cooling of the reaction mixture, the solvent was distilled off under reduced pressure. To the obtained residue, a 1 mol/L aqueous sodium hydroxide solution (8 mL) was added, and the mixture was pH-adjusted to 2 by the dropwise addition of concentrated hydrochloric acid and stirred at room temperature for 20 minutes. The deposit was collected by filtration to obtain a light brown solid of 8-(benzyloxy)-5-((2-fluorobenzyl)amino)-4-methylpyrido[2,3-d]pyrimidin-7(8H)-one (54 mg).
$^1$H-NMR (DMSO-$d_6$) δ value: 2.99 (s, 3H), 4.52 (d, J=5.4 Hz, 2H), 5.08 (s, 2H), 5.45 (s, 1H), 6.90-7.00 (m, 1H), 7.14-7.54 (m, 7H), 7.56-7.68 (m, 2H), 8.90 (s, 1H).

Reference Example 65

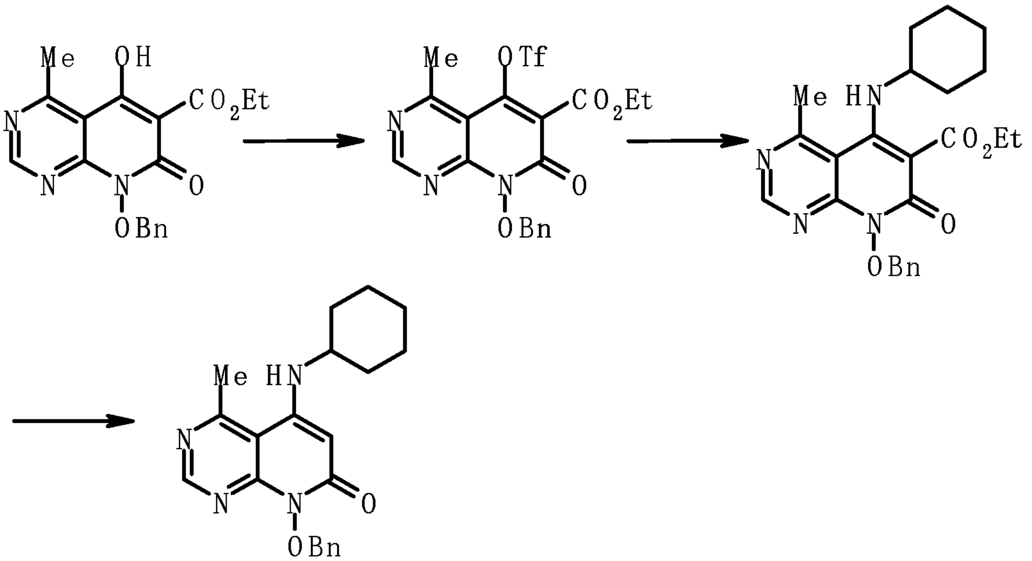

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (134 μL) was added dropwise under ice cooling, and the mixture was stirred at room temperature for 1 hour. The solvent in the reaction mixture was distilled off under reduced pressure. To the obtained residue, cyclohexylamine (240 μL) and dioxane (10 mL) were added, and the mixture was stirred at room temperature for 4 hours. The solvent in the reaction mixture was distilled off under reduced pressure. The obtained residue was purified by silica gel column chromatography [eluent: 70-0% hexane/ethyl acetate] to obtain a yellow oil (128 mg). To the obtained yellow oil (128 mg), methanol (6 mL) and a 1 mol/L aqueous sodium hydroxide solution (6 mL) were added, and the mixture was heated with stirring at 60 to 70°C for 2 hours and 45 minutes. After cooling of the reaction mixture, the solvent was distilled off under reduced pressure. To the obtained residue, a 1 mol/L aqueous sodium hydroxide solution (10 mL) was added, and the mixture was pH-adjusted to 2 by the dropwise addition of 2 mol/L hydrochloric acid and stirred at room temperature for 10 minutes. The deposit was collected by filtration to obtain a brown solid of 8-(benzyloxy)-5-(cyclohexylamino)-4-methylpyrido[2,3-d]pyrimidin-7(8H)-one (62 mg). $^1$H-NMR ($CDCl_3$) δ value: 1.20-1.85 (m, 8H), 2.06-2.18 (m, 2H), 2.96 (s, 3H), 3.32-3.44 (m, 1H), 5.00-5.09 (m, 1H), 5.24 (s, 2H), 5.70 (s, 1H), 7.34-7.42 (m, 3H), 7.64-7.72 (m, 2H), 8.87 (s, 1H).

Reference Example 66

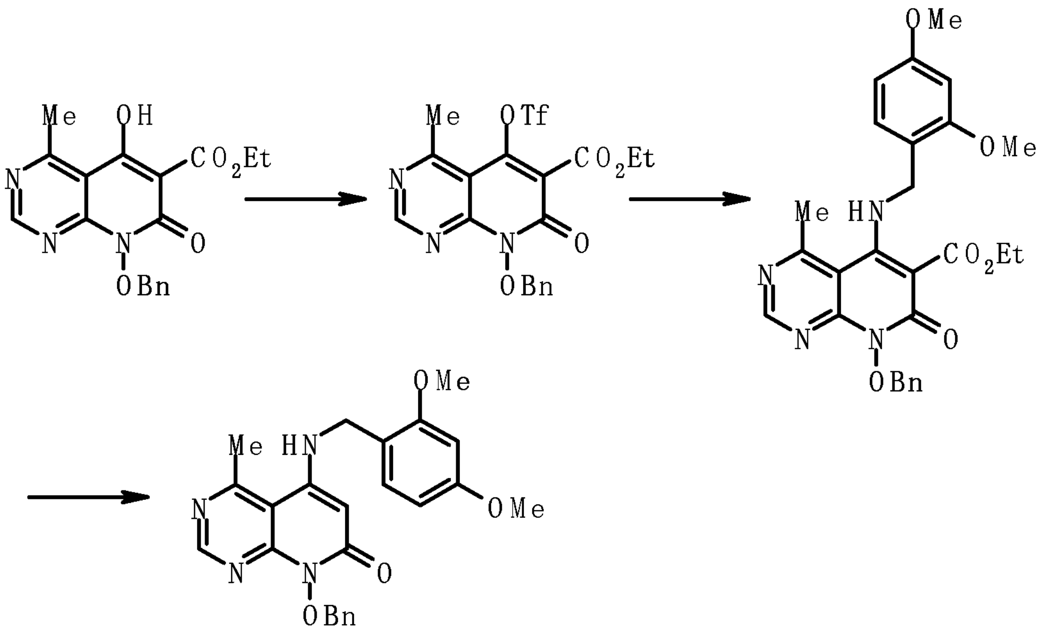

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour. The solvent in the reaction mixture was distilled off under reduced pressure. To the obtained residue, 2,4-dimethoxybenzylamine (253 μL) and dioxane (10 mL) were added, and the mixture was stirred at room temperature for 25 minutes. The solvent in the reaction mixture was distilled off under reduced pressure. The obtained residue was purified by silica gel column chromatography [eluent: 70-0% hexane/ethyl acetate] to obtain a brown oil (125 mg). To the obtained brown oil (125 mg), methanol (5 mL) and a 1 mol/L aqueous sodium hydroxide solution (5 mL) were added, and the mixture was heated with stirring at 60 to 70°C for 3 hours. After cooling of the reaction mixture, the solvent was distilled off under reduced pressure. To the obtained residue, a 1 mol/L aqueous sodium hydroxide solution (10 mL) was added, and the mixture was pH-adjusted to 2 by the dropwise addition of 2 mol/L hydrochloric acid, followed by addition of chloroform. An organic layer was separated, washed with a saturated aqueous solution of sodium chloride and then dried over sodium sulfate, and the solvent was distilled off under reduced pressure. The obtained residue was purified by silica gel column chromatography [eluent: 100-90% chloroform/methanol] and then suspended in a mixed solvent of 2-propanol and diisopropyl ether, and the deposit was collected by filtration to obtain a white solid of 8-(benzyloxy)-5-((2,4-dimethoxybenzyl)amino)-4-methylpyrido[2,3-d]pyrimidin-7(8H)-one (26 mg).
$^1$H-NMR ($CDCl_3$) δ value: 2.90 (s, 3H), 3.83 (s, 3H), 3.88 (s, 3H), 4.30 (d, J=5.1 Hz, 2H), 5.24 (s, 2H), 5.60-5.68 (m, 1H), 5.79 (s, 1H), 6.49 (dd, J=8.2, 2.3 Hz, 1H), 6.53 (d, J=2.2 Hz, 1H), 7.22 (d, J=8.1 Hz, 1H), 7.34-7.43 (m, 3H), 7.65-7.74

(m, 2H), 8.87 (s, 1H).

Reference Example 67

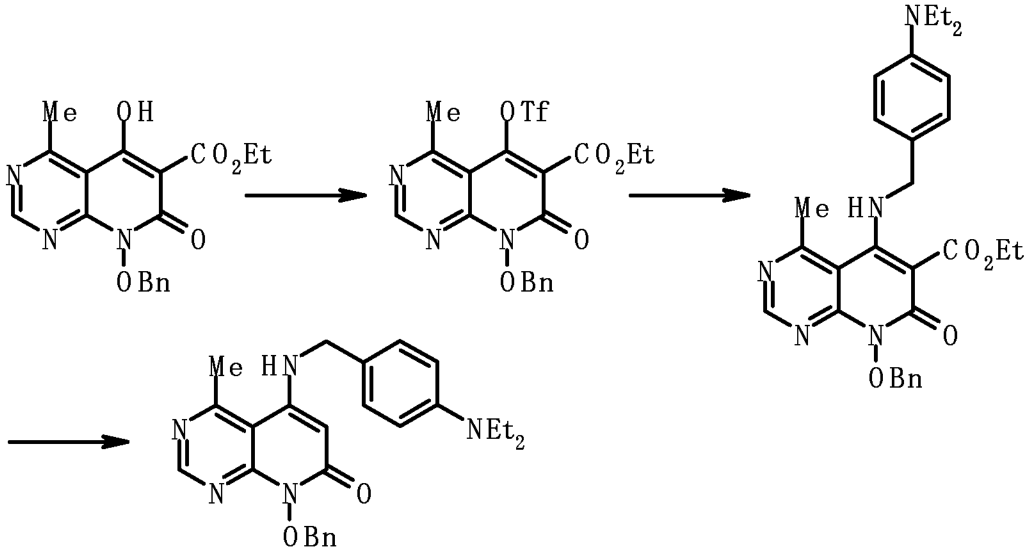

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 50 minutes. The solvent in the reaction mixture was distilled off under reduced pressure. To the obtained residue, 4-(aminomethyl)-N,N-diethylaniline (128 mg), triethylamine (170 μL) and dioxane (10 mL) were added, and the mixture was stirred at room temperature for 4 hours and 20 minutes. The solvent in the reaction mixture was distilled off under reduced pressure. The obtained residue was purified by silica gel column chromatography [eluent: 50-0% hexane/ethyl acetate] to obtain a brown oil (187 mg). To the obtained brown oil (187 mg), methanol (6 mL) and a 1 mol/L aqueous sodium hydroxide solution (6 mL) were added, and the mixture was heated with stirring at 60 to 70°C for 2 hours. After cooling of the reaction mixture, the solvent was distilled off under reduced pressure. To the obtained residue, a 1 mol/L aqueous sodium hydroxide solution (10 mL) was added, and the mixture was pH-adjusted to 2 by the dropwise addition of 2 mol/L hydrochloric acid. Insoluble matter was filtered off, and chloroform was added to the filtrate. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a light brown solid of 8-(benzyloxy)-5-((4-(diethylamino)benzyl)amino)-4-methylpyrido[2,3-d]pyrimidin-7(8H)-one (38 mg).
$^1$H-NMR ($CDCl_3$) δ value: 1.18 (t, J=7.1 Hz, 6H), 2.89 (s, 3H), 3.38 (q, J=7.0 Hz, 4H), 4.20 (d, J=4.2 Hz, 2H), 5.18-5.24 (m, 1H), 5.26 (s, 1H), 5.78 (s, 1H), 6.66-6.73 (m, 2H), 7.18-7.24 (m, 2H), 7.34-7.42 (m, 3H), 7.64-7.75 (m, 2H), 8.88 (s, 1H).

Reference Example 68

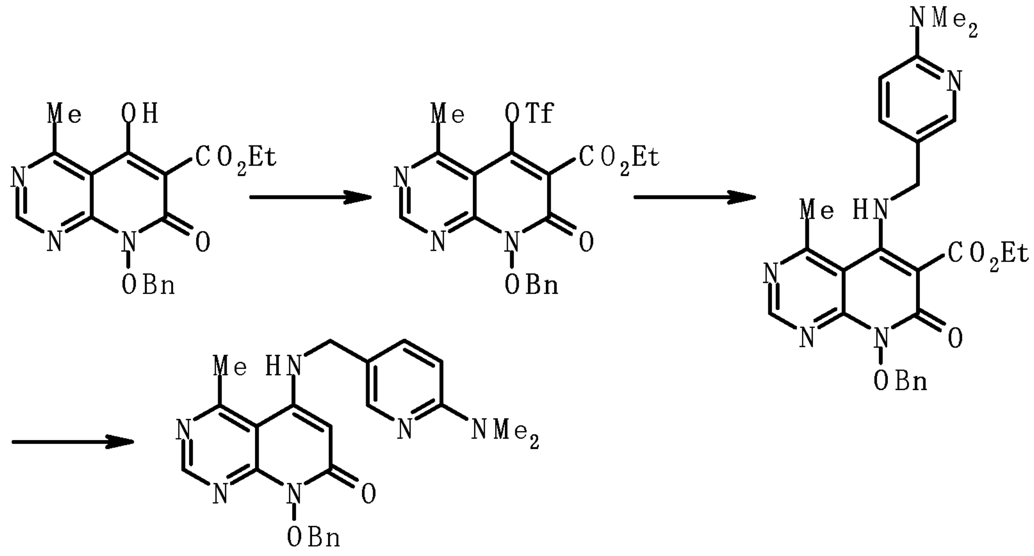

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour and 40 minutes. The solvent in the reaction mixture was distilled off under reduced pressure. To the obtained residue, 5-(aminomethyl)-N,N-dimethylpyridin-2-amine (93 mg), triethylamine (170 μL) and dioxane (10 mL) were added, and the mixture was stirred at room temperature for 6 hours. The solvent in the reaction mixture was distilled off under reduced pressure. The obtained residue was purified by silica gel column chromatography [eluent: 100-80% chloroform/methanol] to obtain a brown oil (198 mg). To the obtained brown oil (198 mg), methanol (8 mL) and a 1 mol/L aqueous sodium hydroxide solution (8 mL) were added, and the mixture was heated with stirring at 60 to 70°C for 3 hours. After cooling of the reaction mixture, the solvent was distilled off under reduced pressure, and ethyl acetate and water were added to the residue. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a yellow solid of 8-(benzyloxy)-5-(((6-(dimethylamino)pyridin-3-yl)methyl)amino)-4-methylpyrido[2,3-d]pyrimidin-7(8H)-one (56 mg).
$^1$H-NMR ($CDCl_3$) δ value: 2.87 (s, 3H), 3.12 (s, 6H), 4.20 (d, J=4.2 Hz, 2H), 5.07-5.16 (m, 1H), 5.25 (s, 2H), 5.78 (s, 1H), 6.55 (d, J=8.8 Hz, 1H), 7.35-7.42 (m, 3H), 7.47 (dd, J=8.8, 2.4 Hz, 1H), 7.65-7.73 (m, 2H), 8.19 (d, J=2.4 Hz, 1H), 8.88 (s, 1H).

Reference Example 69

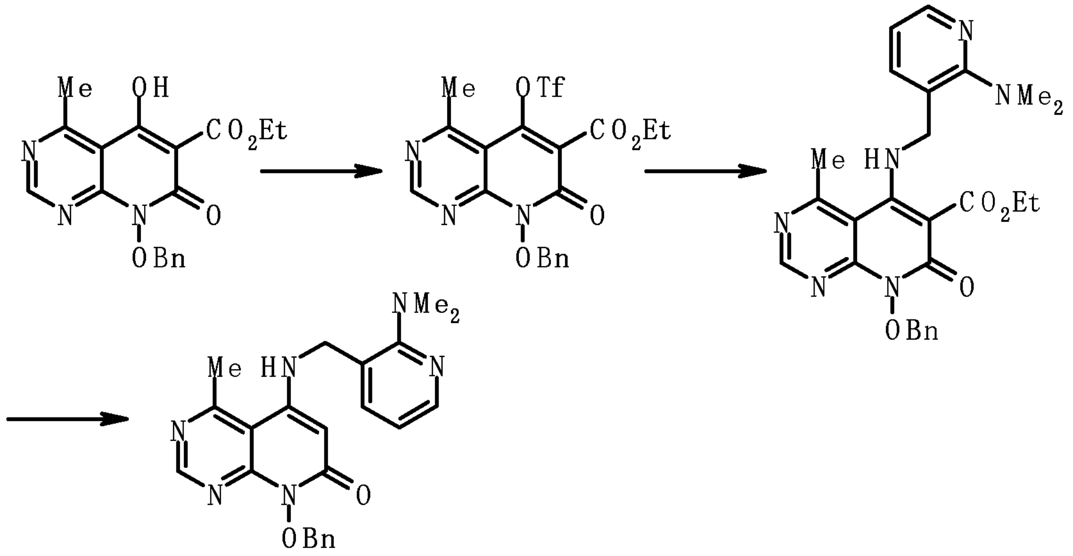

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour. The solvent in the reaction mixture was distilled off under reduced pressure. To the obtained residue, 3-(aminomethyl)-N,N-dimethylpyridin-2-amine (93 mg), triethylamine (170 μL) and dioxane (10 mL) were added, and the mixture was stirred at room temperature for 8 hours and 30 minutes. The solvent in the reaction mixture was distilled off under reduced pressure. The obtained residue was purified by silica gel column chromatography [eluent: 100-80% chloroform/methanol] to obtain a brown oil (248 mg). To the obtained brown oil (248 mg), methanol (10 mL) and a 1 mol/L aqueous sodium hydroxide solution (10 mL) were added, and the mixture was heated with stirring at 60 to 70°C for 3 hours. After cooling of the reaction mixture, the solvent was distilled off under reduced pressure, and chloroform and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 100-80% chloroform/methanol] and then suspended in a mixed solvent of 2-propanol and diisopropyl ether, and the deposit was collected by filtration to obtain a yellow solid of 8-(benzyloxy)-5-(((2-(dimethylamino)pyridin-3-yl)methyl)amino)-4-methylpyrido[2,3-d]pyrimidin-7(8H)-one (29 mg).
$^1$H-NMR ($CDCl_3$) δ value: 2.87 (s, 6H), 2.91 (s, 3H), 4.41 (d, J=3.9 Hz, 2H), 5.26 (s, 2H), 5.75 (s, 1H), 6.48-6.56 (m, 1H), 6.98 (dd, J=7.3, 4.9 Hz, 1H), 7.35-7.42 (m, 3H), 7.57 (dd, J=7.4, 1.8 Hz, 1H), 7.66-7.73 (m, 2H), 8.32 (dd, J=4.8, 1.8 Hz, 1H), 8.90 (s, 1H).

Reference Example 70

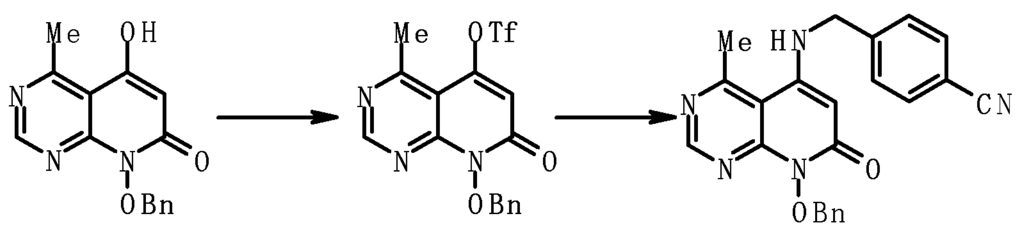

To a solution of 8-(benzyloxy)-5-hydroxy-4-methylpyrido[2,3-d]pyrimidin-7(8H)-one (98 mg) and triethylamine (113 μL) in methylene chloride (5 mL), trifluoromethanesulfonic anhydride (130 μL) was added dropwise under ice cooling, and the mixture was stirred at room temperature for 50 minutes. The solvent in the reaction mixture was distilled off under reduced pressure. To the obtained residue, 4-(aminomethyl)benzonitrile (102 mg), triethylamine (170 μL) and dioxane (10 mL) were added, and the mixture was heated with stirring at 70 to 80°C for 6 hours and 30 minutes. The solvent in the reaction mixture was distilled off under reduced pressure. The obtained residue was purified by silica gel column chromatography [eluent: 100-80% chloroform/methanol] to obtain a yellow solid of 4-(((8-(benzyloxy)-4-methyl-7-oxo-7,8-dihydropyrido[2,3-d]pyrimidin-5-yl)amino)methyl)benzonitrile (57 mg).

$^1$H-NMR ($CDCl_3$) δ value: 2.99 (s, 3H), 4.50 (d, J=5.1 Hz, 2H), 5.22 (s, 2H), 5.50-5.58 (m, 1H), 5.62 (s, 1H), 7.22-7.72 (m, 9H), 8.92 (s, 1H).

Reference Example 71

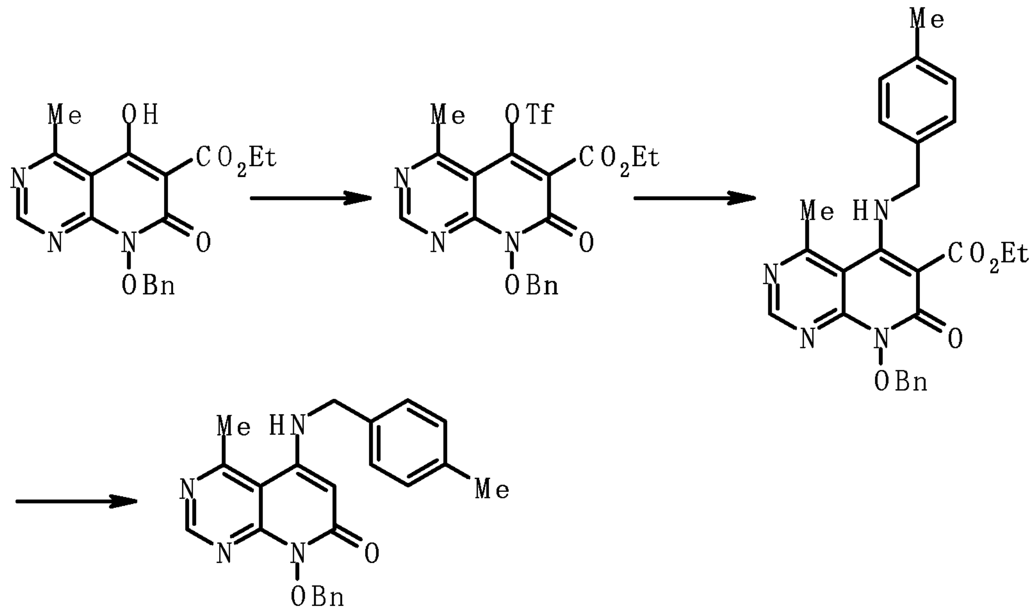

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour and 20 minutes. The solvent in the reaction mixture was distilled off under reduced pressure. To the obtained residue, 4-methylbenzylamine (78 μL), triethylamine (170 μL) and dioxane (12 mL) were added, and the mixture was stirred at room temperature for 3 hours and 30 minutes. The solvent in the reaction mixture was distilled off under reduced pressure. The obtained residue was purified by silica gel column chromatography [eluent: 60-0% hexane/ethyl acetate] to obtain a brown oil (168 mg). To the obtained brown oil (168 mg), methanol (10 mL) and a 1 mol/L aqueous sodium hydroxide solution (10 mL) were added, and the mixture was heated with stirring at 60 to 70°C for 2 hours and 40 minutes. After cooling of the reaction mixture, the solvent was distilled off under reduced pressure. To the obtained residue, a 1 mol/L aqueous sodium hydroxide solution (10 mL) was added, and the mixture was pH-adjusted to 2 by the dropwise addition of 2 mol/L hydrochloric acid and stirred at room temperature for 10 minutes. The deposit was collected by filtration. The obtained solid was suspended in a mixed solvent of 2-propanol and diisopropyl ether, and the deposit was collected by filtration to obtain a yellow solid of 8-(benzyloxy)-4-methyl-5-((4-methylbenzyl)amino)pyrido[2,3-d]pyrimidin-7(8H)-one (46 mg).
$^1$H-NMR ($CDCl_3$) δ value: 2.38 (s, 3H), 2.91 (s, 3H), 4.32 (d, J=4.4 Hz, 2H), 5.22-5.34 (m, 3H), 5.76 (s, 1H), 7.20-7.46 (m, 7H), 7.64-7.74 (m, 2H), 8.89 (s, 1H).

Reference Example 72

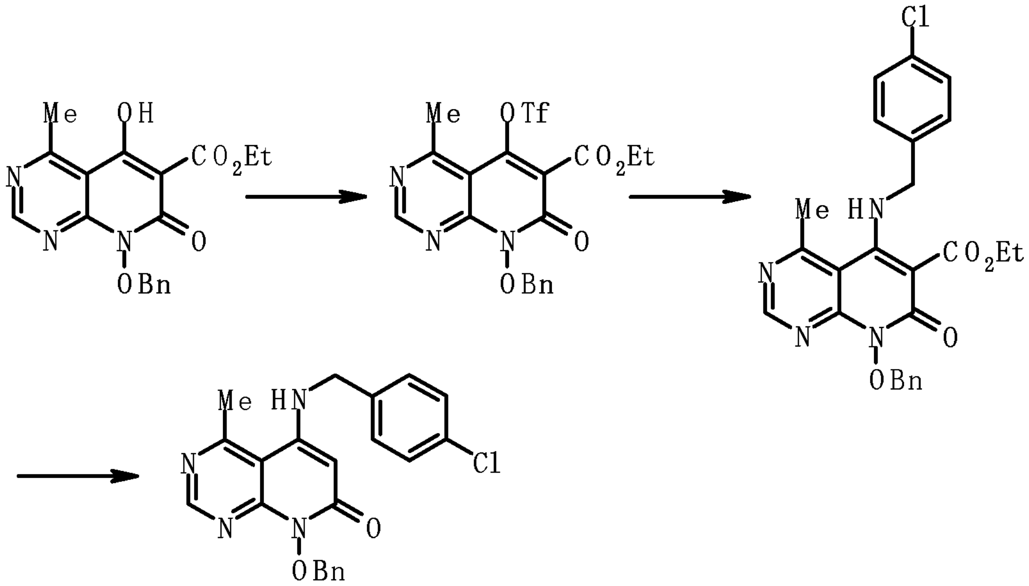

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour and 20 minutes. The solvent in the reaction mixture was distilled off under reduced pressure. To the obtained residue, 4-chlorobenzylamine (75 μL), triethylamine (170 μL) and dioxane (12 mL) were added, and the mixture was stirred at room temperature for 4 hours. The solvent in the reaction mixture was distilled off under reduced pressure. The obtained residue was purified by silica gel column chromatography [eluent: 60-0% hexane/ethyl acetate] to obtain a brown oil (151 mg). To the obtained brown oil (151 mg), methanol (10 mL) and a 1 mol/L aqueous sodium hydroxide solution (10 mL) were added, and the mixture was heated with stirring at 60 to 70°C for 3 hours and 10 minutes. After cooling of the reaction mixture, the solvent was distilled off under reduced pressure. To the obtained residue, a 1 mol/L aqueous sodium hydroxide solution (10 mL) was added, and the mixture was pH-adjusted to 2 by the dropwise addition of 2 mol/L hydrochloric acid and stirred at room temperature for 30 minutes. The deposit was collected by filtration. The obtained solid was suspended in a mixed solvent of 2-propanol and diisopropyl ether, and the deposit was collected by filtration to obtain a pale yellow solid of 8-(benzyloxy)-5-((4-chlorobenzyl)amino)-4-methylpyrido[2,3-d]pyrimidin-7(8H)-one (47 mg).
$^1$H-NMR ($CDCl_3$) δ value: 2.94 (s, 3H), 4.36 (d, J=4.6 Hz, 2H), 5.25 (s, 2H), 5.28-5.35 (m, 1H), 5.71 (s, 1H), 7.20-7.46 (m, 7H), 7.64-7.72 (m, 2H), 8.90 (s, 1H).

Reference Example 73

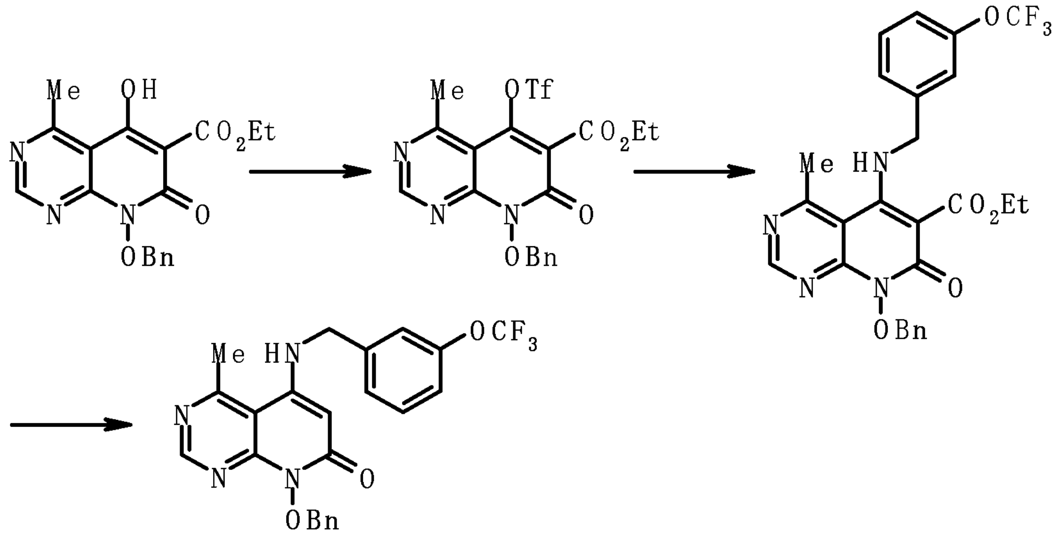

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour and 20 minutes. The solvent in the reaction mixture was distilled off under reduced pressure. To the obtained residue, 3-trifluoromethoxybenzylamine (119 mg), triethylamine (170 μL) and dioxane (12 mL) were added, and the mixture was stirred at room temperature for 6 hours and 15 minutes. The solvent in the reaction mixture was distilled off under reduced pressure. The obtained residue was purified by silica gel column chromatography [eluent: 60-0% hexane/ethyl acetate] to obtain a brown oil (190 mg). To the obtained brown oil (190 mg), methanol (10 mL) and a 1 mol/L aqueous sodium hydroxide solution (10 mL) were added, and the mixture was heated with stirring at 60 to 70°C for 3 hours and 30 minutes. After cooling of the reaction mixture, the solvent was distilled off under reduced pressure, and chloroform and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 100-80% chloroform/methanol] and then suspended in a mixed solvent of 2-propanol and diisopropyl ether, and the deposit was collected by filtration to obtain a yellow solid of 8-(benzyloxy)-4-methyl-5-((3-(trifluoromethoxy)benzyl)amino)pyrido[2,3-d]pyrimidin-7(8H)-one (54 mg). $^1$H-NMR ($CDCl_3$) δ value: 2.96 (s, 3H), 4.43 (d, J=4.9 Hz, 2H), 5.25 (s, 2H), 5.32-5.42 (m, 1H), 5.71 (s, 1H), 7.20-7.55 (m, 7H), 7.64-7.73 (m, 2H), 8.91 (s, 1H).

Reference Example 74

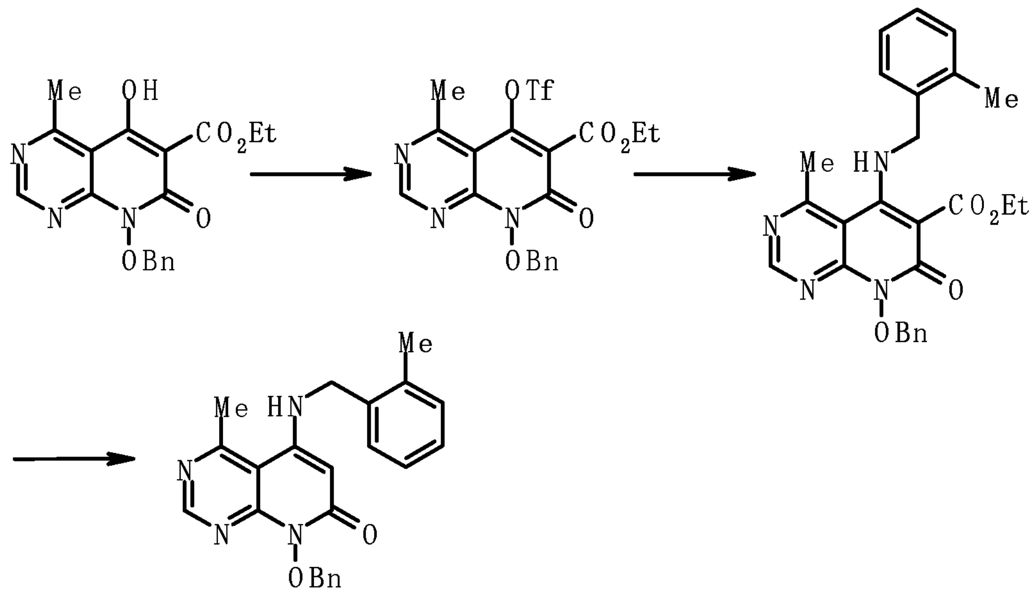

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour and 10 minutes. The solvent in the reaction mixture was distilled off under reduced pressure. To the obtained residue, 2-methylbenzylamine (77 μL), triethylamine (170 μL) and dioxane (12 mL) were added, and the mixture was stirred at room temperature for 6 hours and 30 minutes. The solvent in the reaction mixture was distilled off under reduced pressure. The obtained residue was purified by silica gel column chromatography [eluent: 75-0% hexane/ethyl acetate] to obtain a yellow solid (140 mg). To the obtained yellow solid (140 mg), methanol (10 mL) and a 1 mol/L aqueous sodium hydroxide solution (10 mL) were added, and the mixture was heated with stirring at 60 to 70°C for 3 hours and 5 minutes. After cooling of the reaction mixture, the solvent was distilled off under reduced pressure. To the obtained residue, a 1 mol/L aqueous sodium hydroxide solution (10 mL) was added, and the mixture was pH-adjusted to 2 by the dropwise addition of 2 mol/L hydrochloric acid and stirred at room temperature for 15 minutes. The deposit was collected by filtration. The obtained solid was suspended in a mixed solvent of 2-propanol and diisopropyl ether, and the deposit was collected by filtration to obtain a pale yellow solid of 8-(benzyloxy)-4-methyl-5-((2-methylbenzyl)amino)pyrido[2,3-d]pyrimidin-7(8H)-one (48 mg). $^1$H-NMR ($CDCl_3$) δ value: 2.40 (s, 3H), 2.87 (s, 3H), 4.33 (d, J=4.4 Hz, 2H), 5.11-5.20 (m, 1H), 5.26 (s, 2H), 5.78 (s, 1H), 7.20-7.48 (m, 7H), 7.66-7.74 (m, 2H), 8.90 (s, 1H).

Reference Example 75

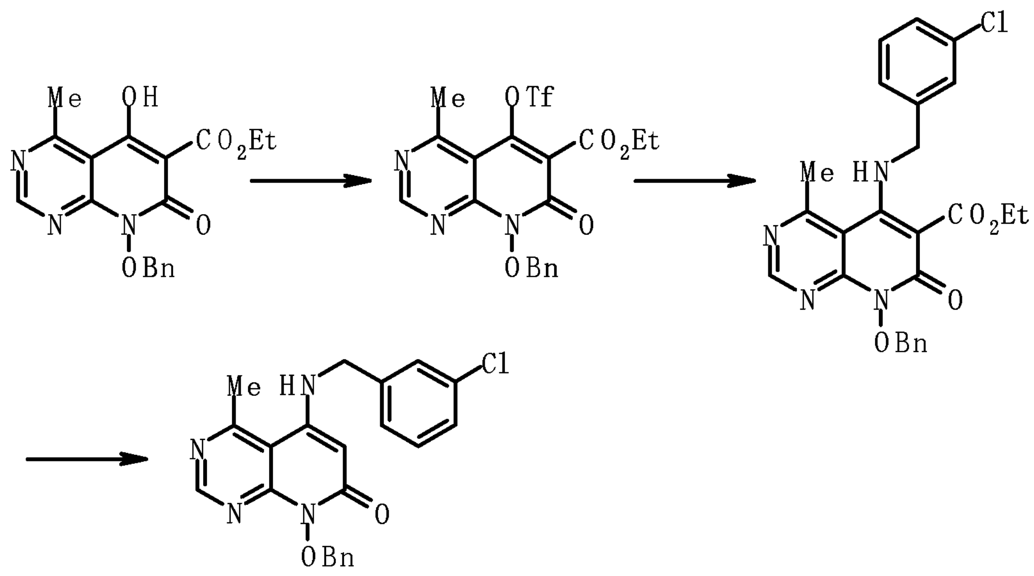

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour and 10 minutes. The solvent in the reaction mixture was distilled off under reduced pressure. To the obtained residue, 3-chlorobenzylamine (76 μL), triethylamine (170 μL) and dioxane (12 mL) were added, and the mixture was stirred at room temperature for 6 hours and 30 minutes. The solvent in the reaction mixture was distilled off under reduced pressure. The obtained residue was purified by silica gel column chromatography [eluent: 50-0% hexane/ethyl acetate] to obtain a brown oil (271 mg). To the obtained brown oil (271 mg), methanol (10 mL) and a 1 mol/L aqueous sodium hydroxide solution (10 mL) were added, and the mixture was heated with stirring at 60 to 70°C for 3 hours. After cooling of the reaction mixture, the solvent was distilled off under reduced pressure. To the obtained residue, a 1 mol/L aqueous sodium hydroxide solution (10 mL) was added, and the mixture was pH-adjusted to 2 by the dropwise addition of 2 mol/L hydrochloric acid and stirred at room temperature for 10 minutes. The deposit was collected by filtration. The obtained solid was suspended in a mixed solvent of 2-propanol and diisopropyl ether, and the deposit was collected by filtration to obtain a yellow solid of 8-(benzyloxy)-5-((3-chlorobenzyl)amino)-4-methylpyrido[2,3-d]pyrimidin-7(8H)-one (45 mg).

$^1$H-NMR ($CDCl_3$) δ value: 2.97 (s, 3H), 4.39 (d, J=5.1 Hz, 2H), 5.25 (s, 2H), 5.33-5.42 (m, 1H), 5.70 (s, 1H), 7.24-7.43 (m, 7H), 7.65-7.73 (m, 2H), 8.91 (s, 1H).

Reference Example 76

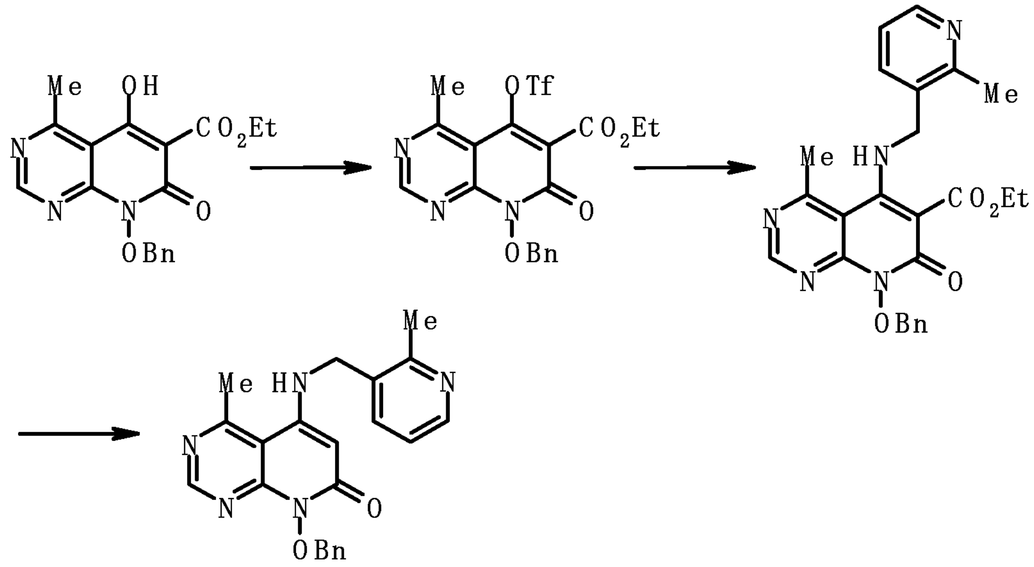

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour and 10 minutes. The solvent in the reaction mixture was distilled off under reduced pressure. To the obtained residue, ((2-methylpyridin-3-yl)methyl)amine (86 mg), triethylamine (170 μL) and dioxane (12 mL) were added, and the mixture was stirred at room temperature for 8 hours and 30 minutes. The solvent in the reaction mixture was distilled off under reduced pressure. The obtained residue was purified by silica gel column chromatography [eluent: 100-85% chloroform/methanol] to obtain a brown oil (232 mg). To the obtained brown oil (232 mg), methanol (10 mL) and a 1 mol/L aqueous sodium hydroxide solution (10 mL) were added, and the mixture was heated with stirring at 60 to 70°C for 3 hours. After cooling of the reaction mixture, the solvent was distilled off under reduced pressure, and chloroform and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 100-80% chloroform/methanol] and then suspended in a mixed solvent of 2-propanol and diisopropyl ether, and the deposit was collected by filtration to obtain a pale yellow solid of 8-(benzyloxy)-4-methyl-5-(((2-methylpyridin-3-yl)methyl)amino)pyrido[2,3-d]pyrimidin-7(8H)-one (20 mg).
$^1$H-NMR ($CDCl_3$) δ value: 2.64 (s, 3H), 2.92 (s, 3H), 4.39 (d, J=4.6 Hz, 2H), 5.17-5.24 (m, 1H), 5.26 (s, 2H), 5.72 (s, 1H), 7.15-7.22 (m, 1H), 7.34-7.42 (m, 3H), 7.55-7.62 (m, 1H), 7.65-7.72 (m, 2H), 8.50-8.57 (m, 1H), 8.92 (s, 1H).

Reference Example 77

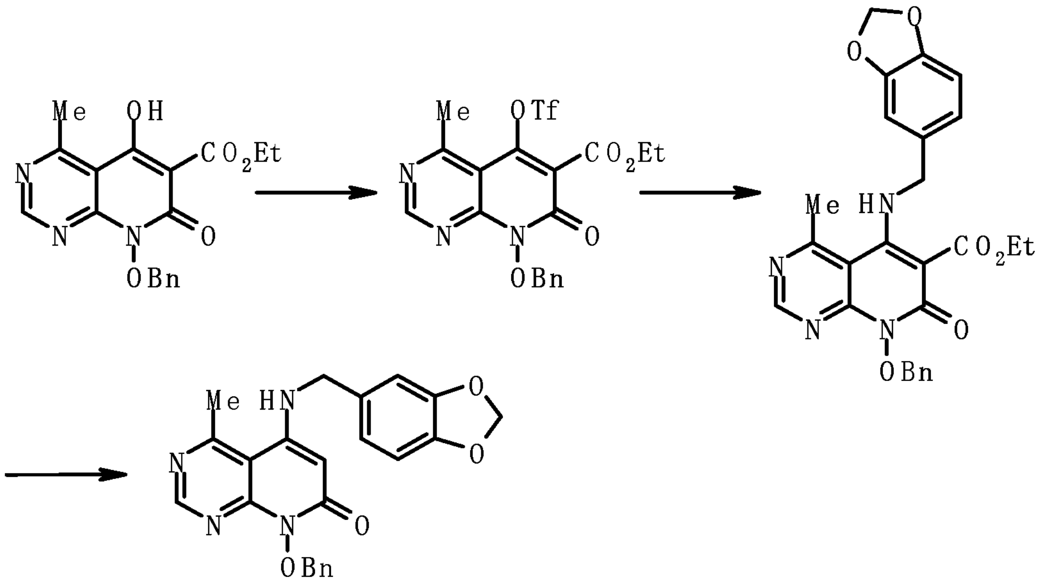

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour. The solvent in the reaction mixture was distilled off under reduced pressure. To the obtained residue, piperonylamine (77 μL), triethylamine (170 μL) and dioxane (10 mL) were added, and the mixture was stirred at room temperature for 4 hours. The solvent in the reaction mixture was distilled off under reduced pressure. The obtained residue was purified by silica gel column chromatography [eluent: 60-0% hexane/ethyl acetate] to obtain a brown solid (160 mg). To the obtained brown solid (160 mg), methanol (10 mL) and a 1 mol/L aqueous sodium hydroxide solution (10 mL) were added, and the mixture was heated with stirring at 60 to 70°C for 3 hours. After cooling of the reaction mixture, the solvent was distilled off under reduced pressure, and ethyl acetate and water were added to the residue. An organic layer was separated, washed with a saturated aqueous solution of sodium chloride and then dried over sodium sulfate, and the solvent was distilled off under reduced pressure. The obtained residue was suspended in a mixed solvent of methanol, 2-propanol and diisopropyl ether, and the deposit was collected by filtration to obtain a pale yellow solid of 5-(((1,3-benzodioxol-5-yl)methyl)amino)-8-(benzyloxy)-4-methylpyrido[2,3-d]pyrimidin-7(8H)-one (42 mg).
$^1$H-NMR ($CDCl_3$) δ value: 2.92 (s, 3H), 4.27 (d, J=4.4 Hz, 2H), 5.23-5.30 (m, 3H), 5.74 (s, 1H), 6.01 (s, 2H), 6.82-6.88 (m, 3H), 7.20-7.46 (m, 3H), 7.64-7.76 (m, 2H), 8.89 (s, 1H).

Reference Example 78

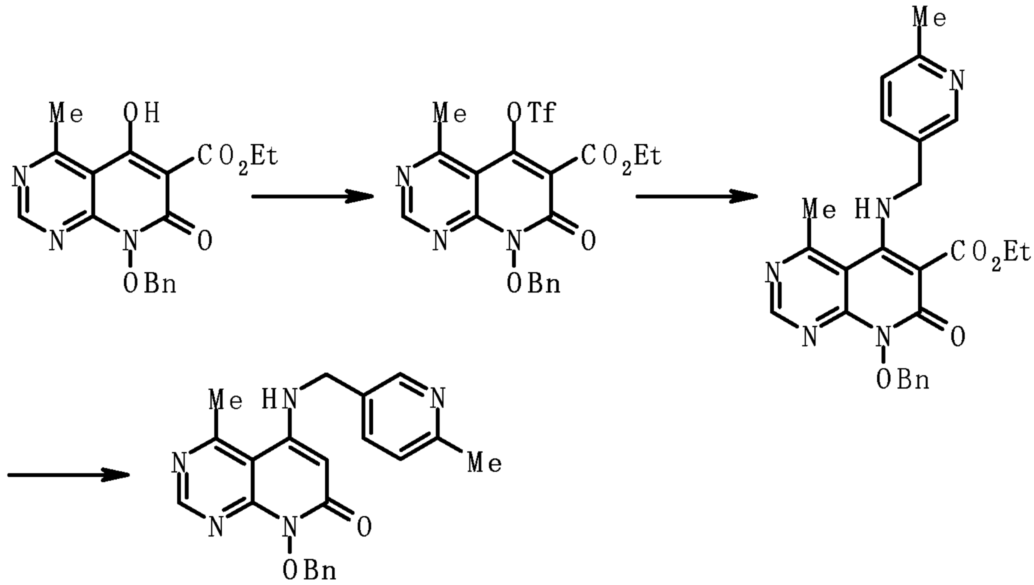

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour. The solvent in the reaction mixture was distilled off under reduced pressure. To the obtained residue, ((6-methylpyridin-3-yl)methyl)amine (76 mg), triethylamine (170 μL) and dioxane (10 mL) were added, and the mixture was stirred at room temperature for 5 hours and 30 minutes. The solvent in the reaction mixture was distilled off under reduced pressure. The obtained residue was purified by silica gel column chromatography [eluent: 100-80% chloroform/methanol] to obtain a brown oil (181 mg). To the obtained brown oil (181 mg), methanol (10 mL) and a 1 mol/L aqueous sodium hydroxide solution (10 mL) were added, and the mixture was heated with stirring at 60 to 70°C for 6 hours and 30 minutes. After cooling of the reaction mixture, the solvent was distilled off under reduced pressure, and chloroform and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 100-80% chloroform/methanol] and then suspended in a mixed solvent of 2-propanol and diisopropyl ether, and the deposit was collected by filtration to obtain a yellow solid of 8-(benzyloxy)-4-methyl-5-(((6-methylpyridin-3-yl)methyl)amino)pyrido[2,3-d]pyrimidin-7(8H)-one (45 mg). $^{1}$H-NMR ($CDCl_3$) δ value: 2.60 (s, 3H), 2.92 (s, 3H), 4.37 (d, J=4.9 Hz, 2H), 5.22-5.30 (m, 3H), 5.74 (s, 1H), 7.22 (d, J=7.8 Hz, 1H), 7.34-7.43 (m, 3H), 7.61 (dd, J=8.0, 2.4 Hz, 1H), 7.64-7.72 (m, 2H), 8.53-8.58 (m, 1H), 8.91 (s, 1H).

Reference Example 79

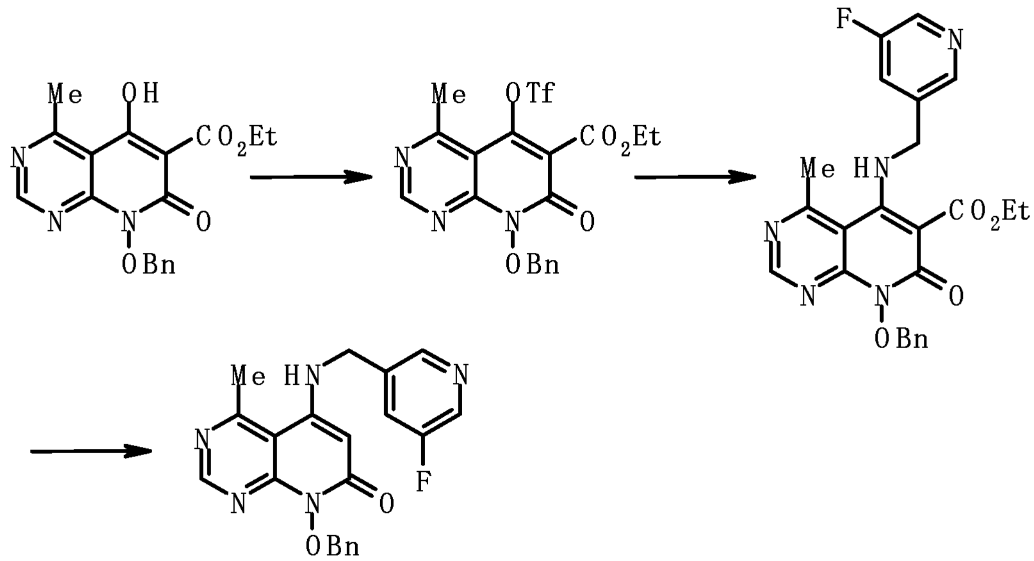

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour. The solvent in the reaction mixture was distilled off under reduced pressure. To the obtained residue, ((5-fluoropyridin-3-yl)methyl)amine (78 mg), triethylamine (170 μL) and dioxane (10 mL) were added, and the mixture was stirred at room temperature for 5 hours and 30 minutes. The solvent in the reaction mixture was distilled off under reduced pressure. The obtained residue was purified by silica gel column chromatography [eluent: 100-80% chloroform/methanol] to obtain a brown oil (250 mg). To the obtained brown oil (250 mg), methanol (10 mL) and a 1 mol/L aqueous sodium hydroxide solution (10 mL) were added, and the mixture was heated with stirring at 60 to 70°C for 6 hours. After cooling of the reaction mixture, the solvent was distilled off under reduced pressure, and chloroform and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 100-80% chloroform/methanol] and then suspended in a mixed solvent of 2-propanol and diisopropyl ether, and the deposit was collected by filtration to obtain a yellow solid of 8-(benzyloxy)-5-(((5-fluoropyridin-3-yl)methyl)amino)-4-methylpyrido[2,3-d]pyrimidin-7(8H)-one (26 mg). $^1$H-NMR ($CDCl_3$) δ value: 2.99 (s, 3H), 4.50 (d, J=5.4 Hz, 2H), 5.25 (s, 2H), 5.35-5.44 (m, 1H), 5.69 (s, 1H), 7.35-7.47 (m, 4H), 7.63-7.72 (m, 2H), 8.47-8.52 (m, 2H), 8.92 (s, 1H).

Reference Example 80

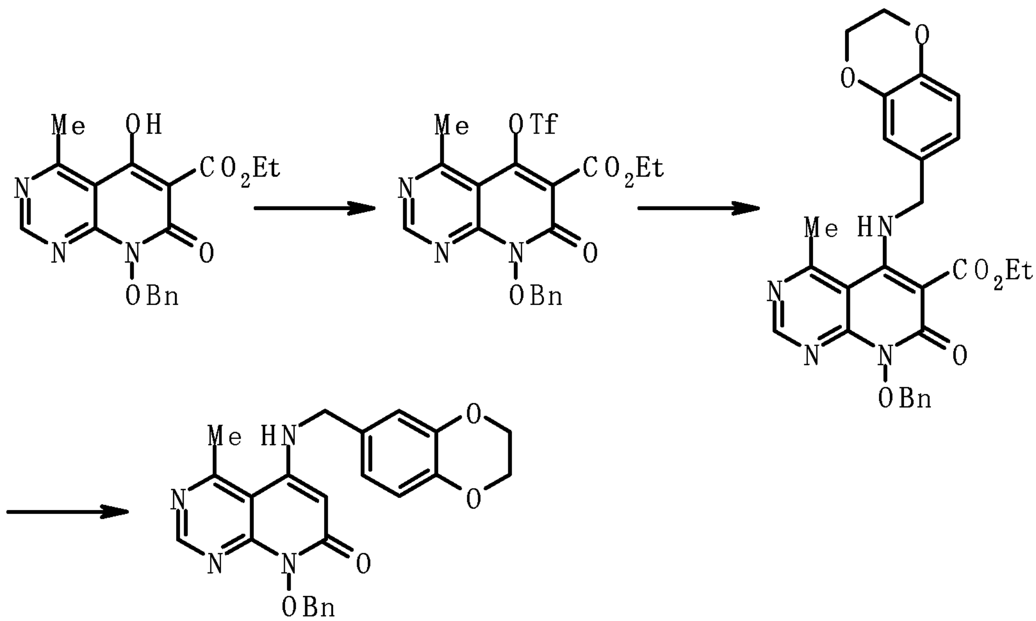

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour. The solvent in the reaction mixture was distilled off under reduced pressure. To the obtained residue, ((2,3-dihydro-1,4-benzodioxin-6-yl)methyl)amine (102 mg), triethylamine (170 μL) and dioxane (10 mL) were added, and the mixture was stirred at room temperature for 3 hours. The solvent in the reaction mixture was distilled off under reduced pressure. The obtained residue was purified by silica gel column chromatography [eluent: 60-0% hexane/ethyl acetate] to obtain a brown oil (208 mg). To the obtained brown oil (208 mg), methanol (10 mL) and a 1 mol/L aqueous sodium hydroxide solution (10 mL) were added, and the mixture was heated with stirring at 60 to 70°C for 2 hours and 30 minutes. After cooling of the reaction mixture, the solvent was distilled off under reduced pressure, and ethyl acetate and water were added to the residue. An organic layer was separated, washed with a saturated aqueous solution of sodium chloride and then dried over sodium sulfate, and the solvent was distilled off under reduced pressure. The obtained residue was suspended in a mixed solvent of methanol, 2-propanol and diisopropyl ether, and the deposit was collected by filtration to obtain a pale yellow solid of 8-(benzyloxy)-5-(((2,3-dihydro-1,4-benzodioxin-6-yl)methyl)amino)-4-methylpyrido[2,3-d]pyrimidin-7(8H)-one (57 mg).
$^1$H-NMR ($CDCl_3$) δ value: 2.93 (s, 3H), 4.25 (d, J=4.6 Hz, 2H), 4.28 (s, 4H), 5.23-5.33 (m, 3H), 5.73 (s, 1H), 6.81-6.94 (m, 3H), 7.35-7.44 (m, 3H), 7.65-7.74 (m, 2H), 8.89 (s, 1H).

Reference Example 81

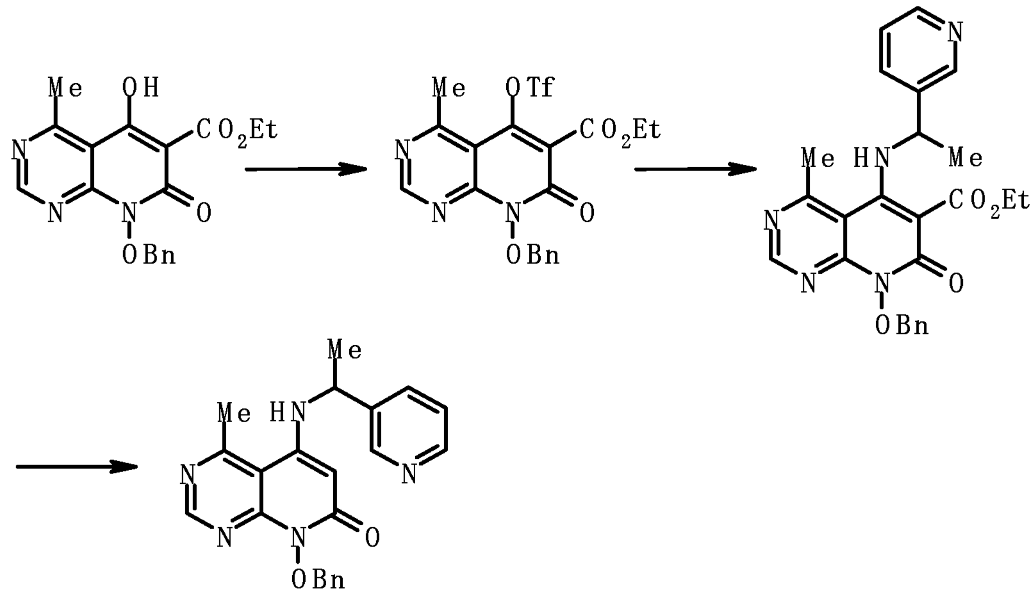

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour. The solvent in the reaction mixture was distilled off under reduced pressure. To the obtained residue, 1-(pyridin-3-yl)ethan-1-amine (87 mg), triethylamine (170 μL) and dioxane (10 mL) were added, and the mixture was stirred at room temperature for 6 hours. The solvent in the reaction mixture was distilled off under reduced pressure. The obtained residue was purified by silica gel column chromatography [eluent: 100-80% chloroform/methanol] to obtain a brown oil (212 mg). To the obtained brown oil (212 mg), methanol (10 mL) and a 1 mol/L aqueous sodium hydroxide solution (10 mL) were added, and the mixture was heated with stirring at 60 to 70°C for 3 hours. After cooling of the reaction mixture, the solvent was distilled off under reduced pressure, and chloroform and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 100-80% chloroform/methanol] to obtain a yellow solid of 8-(benzyloxy)-4-methyl-5-((1-(pyridin-3-yl)ethyl)amino)pyrido[2,3-d]pyrimidin-7(8H)-one (75 mg).
$^{1}H$-NMR ($CDCl_3$) δ value: 1.69 (d, J=6.8 Hz, 3H), 3.08 (s, 3H), 4.55-4.67 (m, 1H), 5.19 (s, 2H), 5.36-5.42 (m, 1H), 5.47 (s, 1H), 7.30-7.41 (m, 4H), 7.58-7.71 (m, 3H), 8.52-8.67 (m, 2H), 8.91 (s, 1H).

Reference Example 82

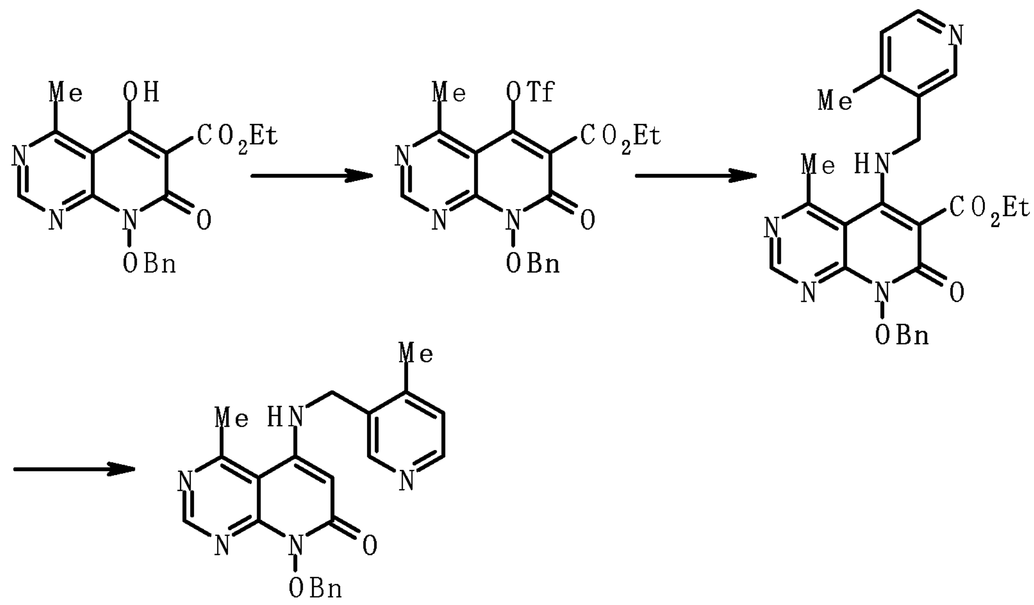

To a solution of ethyl 8-(benzyloxy)-5-hydroxy-4-methyl-7-oxo-7,8-dihydropyrido[2,3-d]pyrimidine-6-carboxylate (145 mg) and triethylamine (113 μL) in methylene chloride (5 mL), trifluoromethanesulfonic anhydride (130 μL) was added dropwise under ice cooling, and the mixture was stirred at room temperature for 1 hour. The solvent in the reaction mixture was distilled off under reduced pressure. To the obtained residue, ((4-methylpyridin-3-yl)methyl)amine (82 mg), triethylamine (170 μL) and dioxane (10 mL) were added, and the mixture was stirred at room temperature for 6 hours. The solvent in the reaction mixture was distilled off under reduced pressure. The obtained residue was purified by silica gel column chromatography [eluent: 100-80% chloroform/methanol] to obtain a brown oil (174 mg). To the obtained brown oil (174 mg), methanol (10 mL) and a 1 mol/L aqueous sodium hydroxide solution (10 mL) were added, and the mixture was heated with stirring at 60 to 70°C for 6 hours and 30 minutes. After cooling of the reaction mixture, the solvent was distilled off under reduced pressure, and chloroform and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 100-80% chloroform/methanol] and then suspended in a mixed solvent of 2-propanol and diisopropyl ether, and the deposit was collected by filtration to obtain a yellow solid of 8-(benzyloxy)-4-methyl-5-(((4-methylpyridin-3-yl)methyl)amino)pyrido[2,3-d]pyrimidin-7(8H)-one (11 mg).
$^1$H-NMR ($CDCl_3$) δ value: 2.42 (s, 3H), 2.88 (s, 3H), 4.38 (d, J=4.4 Hz, 2H), 5.06-5.14 (m, 1H), 5.26 (s, 2H), 5.79 (s, 1H), 7.21 (d, J=5.1 Hz, 1H), 7.34-7.43 (m, 3H), 7.65-7.72 (m, 2H), 8.52 (d, J=5.1 Hz, 1H), 8.53 (s, 1H), 8.91 (s, 1H).

Reference Example 83

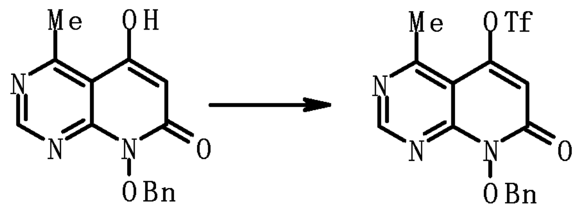

To a solution of 8-(benzyloxy)-5-hydroxy-4-methylpyrido[2,3-d]pyrimidin-7(8H)-one (1.00 g) and triethylamine (978 μL) in methylene chloride (40 mL), trifluoromethanesulfonic anhydride (1.16 mL) was added dropwise under ice cooling, and the mixture was stirred at room temperature for 1 hour and 30 minutes. The solvent in the reaction mixture was distilled off under reduced pressure. The obtained residue was purified by silica gel column chromatography [eluent: 100-80% chloroform/methanol] to obtain a yellow solid of 8-(benzyloxy)-4-methyl-7-oxo-7,8-dihydropyrido[2,3-d]pyrimidin-5-yl trifluoromethanesulfonate (538 mg).
$^1$H-NMR ($CDCl_3$) δ value: 2.95 (s, 3H), 5.29 (s, 2H), 6.89 (s, 1H), 7.36-7.48 (m, 3H), 7.60-7.70 (m, 2H), 9.06 (s, 1H).

Reference Example 84

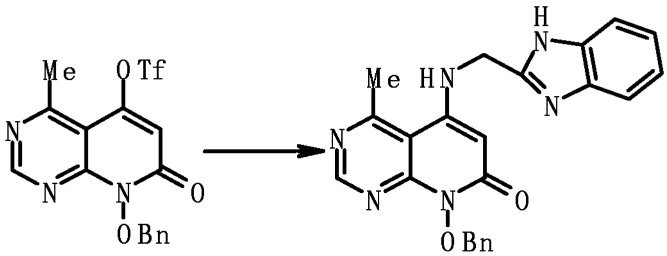

To 8-(benzyloxy)-4-methyl-7-oxo-7,8-dihydropyrido[2,3-d]pyrimidin-5-yl trifluoromethanesulfonate (128 mg) and 2-(aminomethyl)benzimidazole (131 mg), triethylamine (86 μL) and dioxane (12 mL) were added, and the mixture was heated with stirring at 60 to 70°C for 3 hours and 45 minutes. After cooling of the reaction mixture, the solvent was distilled off under reduced pressure. The obtained residue was purified by silica gel column chromatography [eluent: 100-80% chloroform/methanol] and then suspended in a mixed solvent of 2-propanol and diisopropyl ether, and the deposit was collected by filtration to obtain a pale yellow solid of 5-(((1H-benzimidazol-2-yl)methyl)amino)-8-(benzyloxy)-4-methylpyrido[2,3-d]pyrimidin-7(8H)-one (6 mg).
$^1$H-NMR (DMSO-$d_6$) δ value: 3.10 (s, 3H), 4.68-4.76 (m, 2H), 5.07 (s, 2H), 5.56 (s, 1H), 7.00-7.80 (m, 10H), 8.93 (s, 1H).

Reference Example 85

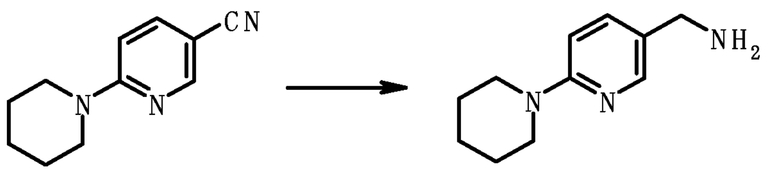

To a solution of 6-(piperidin-1-yl)nicotinonitrile (108 mg) in diethyl ether (15 mL), lithium aluminum hydride (66 mg) was added under ice cooling, and the mixture was stirred at room temperature for 2 hours and 30 minutes in a nitrogen atmosphere. To the reaction mixture, ethyl acetate and water were added under ice cooling, followed by separation of an organic layer. An aqueous layer was extracted twice with ethyl acetate, combined with the organic layer, washed with a saturated aqueous solution of sodium chloride and then dried over sodium sulfate, and the solvent was distilled off under reduced pressure to obtain a white solid of 1-(6-(piperidin-1-yl)pyridin-3-yl)methanamine (111 mg).
$^1$H-NMR ($CDCl_3$) δ value: 1.59-1.70 (m, 6H), 3.46-3.56 (m, 4H), 3.73 (s, 2H), 6.65 (d, J=8.8 Hz, 1H), 7.44 (dd, J=8.6, 2.3 Hz, 1H), 8.09 (d, J=2.2 Hz, 1H).

Reference Example 86

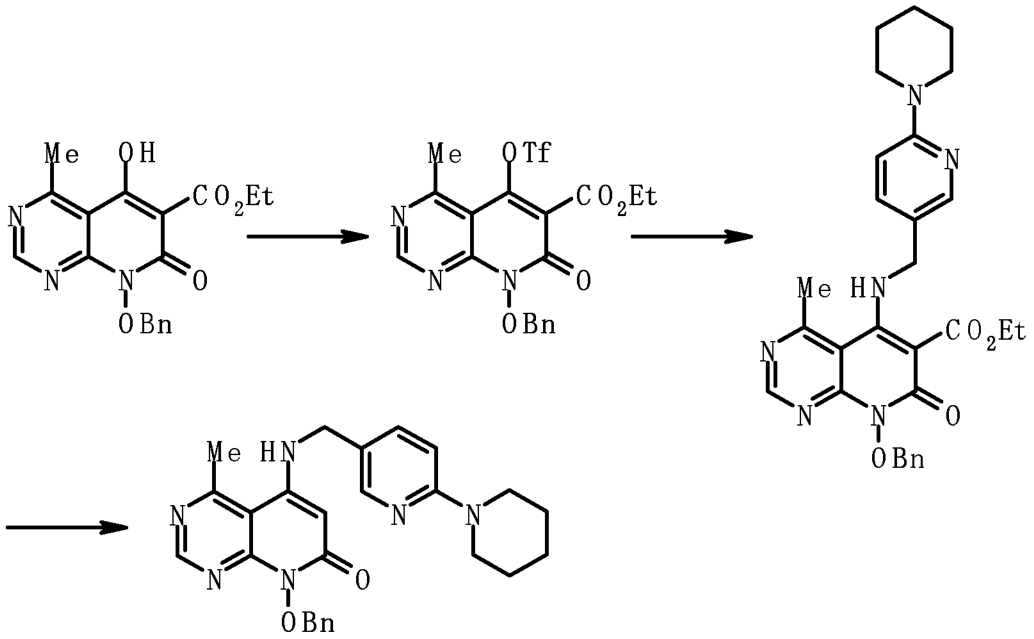

To a solution of ethyl 8-(benzyloxy)-5-hydroxy-4-methyl-7-oxo-7,8-dihydropyrido[2,3-d]pyrimidine-6-carboxylate (150 mg) and triethylamine (116 μL) in methylene chloride (5 mL), trifluoromethanesulfonic anhydride (138 μL) was added dropwise under ice cooling, and the mixture was stirred at room temperature for 1 hour and 20 minutes. The solvent in the reaction mixture was distilled off under reduced pressure. To the obtained residue, 1-(6-(piperidin-1-yl)pyridin-3-yl)methanamine (111 mg), triethylamine (170 μL) and dioxane (10 mL) were added, and the mixture was stirred at room temperature for 5 hours. The solvent in the reaction mixture was distilled off under reduced pressure. The obtained residue was purified by silica gel column chromatography [eluent: 100-80% chloroform/methanol] to obtain a brown solid (270 mg). To the obtained brown solid (270 mg), methanol (10 mL) and a 1 mol/L aqueous sodium hydroxide solution (10 mL) were added, and the mixture was heated with stirring at 60 to 70°C for 5 hours and 15 minutes. After cooling of the reaction mixture, the solvent was distilled off under reduced pressure. The obtained residue was suspended in water, and the deposit was collected by filtration. The obtained solid was suspended in a mixed solvent of methanol, 2-propanol and diisopropyl ether, and the deposit was collected by filtration to obtain a yellow solid of 8-(benzyloxy)-4-methyl-5-(((6-(piperidin-1-yl)pyridin-3-yl)methyl)amino)pyrido[2,3-d]pyrimidin-7(8H)-one (176 mg).
$^1$H-NMR ($CD_3OD$) δ value: 1.56-1.72 (m, 6H), 3.00 (s, 3H), 3.20-3.58 (m, 4H), 4.38 (s, 2H), 5.17 (s, 2H), 5.67 (s, 1H), 6.80-6.86 (m, 1H), 7.33-7.40 (m, 3H), 7.58-7.70 (m, 3H), 8.10-8.14 (m, 1H), 8.84 (s, 1H).

Reference Example 87

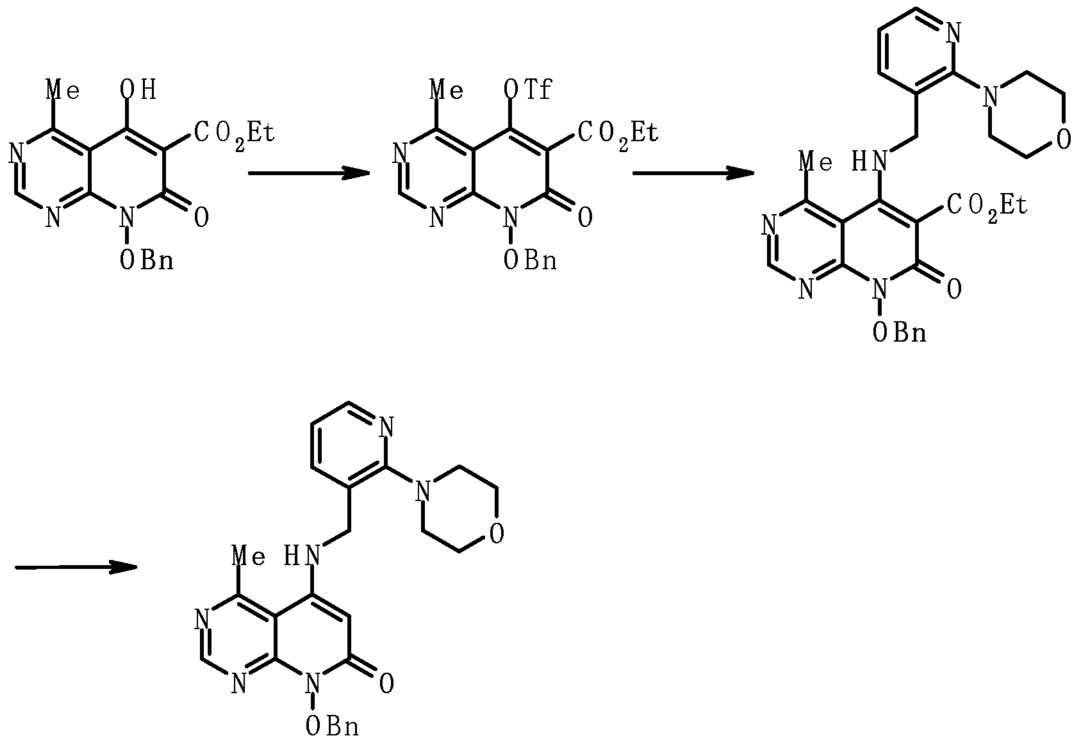

To a solution of ethyl 8-(benzyloxy)-5-hydroxy-4-methyl-7-oxo-7,8-dihydropyrido[2,3-d]pyrimidine-6-carboxylate (107 mg) and triethylamine (83 μL) in methylene chloride (4 mL), trifluoromethanesulfonic anhydride (98 μL) was added dropwise under ice cooling, and the mixture was stirred at room temperature for 1 hour. The solvent in the reaction mixture was distilled off under reduced pressure. To the obtained residue, 1-(2-(morpholin-4-yl)pyridin-3-yl)methanamine (65 mg), triethylamine (125 μL) and dioxane (10 mL) were added, and the mixture was stirred at room temperature for 5 hours. The solvent in the reaction mixture was distilled off under reduced pressure. The obtained residue was purified by silica gel column chromatography [eluent: 100-90% chloroform/methanol] to obtain a brown solid (132 mg). To the obtained brown solid (132 mg), methanol (10 mL) and a 1 mol/L aqueous sodium hydroxide solution (10 mL) were added, and the mixture was heated with stirring at 60 to 70°C for 6 hours and 40 minutes. After cooling of the reaction mixture, the solvent was distilled off under reduced pressure, and ethyl acetate and water were added to the residue, followed by separation of an organic layer. An aqueous layer was extracted twice with ethyl acetate, combined with the organic layer, washed with a saturated aqueous solution of sodium chloride and then dried over sodium sulfate, and the solvent was distilled off under reduced pressure. The obtained residue was purified by silica gel column chromatography [eluent: 100-80% chloroform/methanol] and then suspended in a mixed solvent of 2-propanol and diisopropyl ether, and the deposit was collected by filtration to obtain a white solid of 8-(benzyloxy)-4-methyl-5-(((2-(morpholin-4-yl)pyridin-3-yl)methyl)amino)pyrido[2,3-d]pyrimidin-7(8H)-one (19 mg).

$^1$H-NMR ($CDCl_3$) δ value: 2.97 (s, 3H), 3.16-3.26 (m, 4H), 3.81-3.89 (m, 4H), 4.42 (d, J=4.6 Hz, 2H), 5.26 (s, 2H), 5.73 (s, 1H), 5.88-5.98 (m, 1H), 7.06 (dd, J=7.5, 4.7 Hz, 1H), 7.35-7.45 (m, 3H), 7.60-7.66 (m, 1H), 7.66-7.73 (m, 2H), 8.36 (dd, J=4.6, 1.7 Hz, 1H), 8.92 (s, 1H).

Reference Example 88

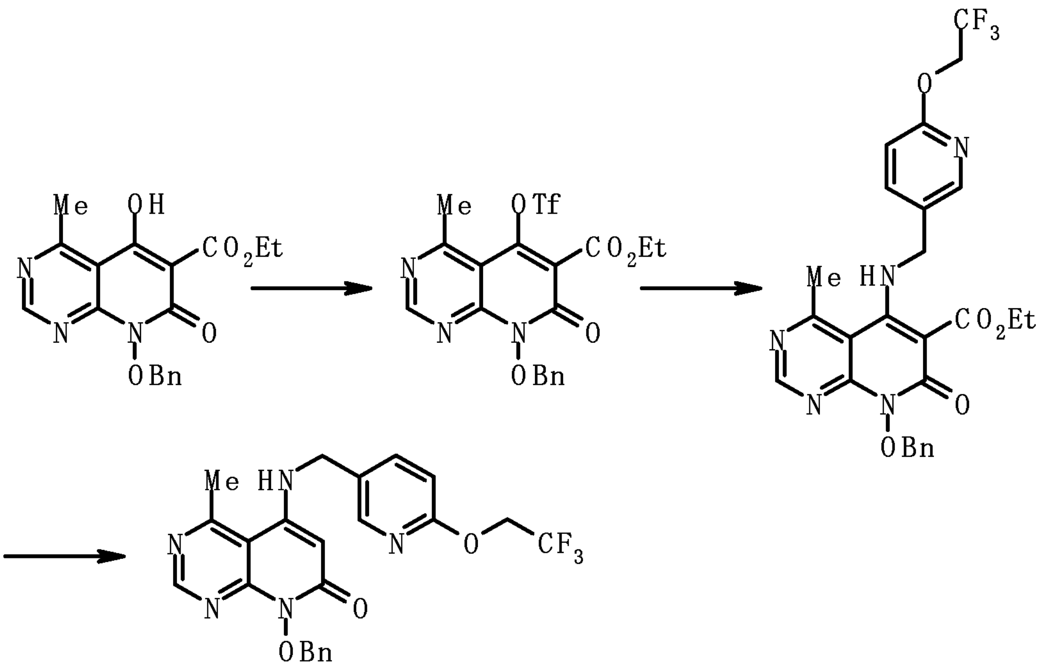

To a solution of ethyl 8-(benzyloxy)-5-hydroxy-4-methyl-7-oxo-7,8-dihydropyrido[2,3-d]pyrimidine-6-carboxylate (193 mg) and triethylamine (149 μL) in methylene chloride (6 mL), trifluoromethanesulfonic anhydride (177 μL) was added dropwise under ice cooling, and the mixture was stirred at room temperature for 1 hour. The solvent in the reaction mixture was distilled off under reduced pressure. To the obtained residue, 1-(6-(2,2,2-trifluoroethoxy)pyridin-3-yl)methanamine (212 mg), triethylamine (225 μL) and dioxane (12 mL) were added, and the mixture was stirred at room temperature for 4 hours. The solvent in the reaction mixture was distilled off under reduced pressure. The obtained residue was purified by silica gel column chromatography [eluent: 100-90% chloroform/methanol] to obtain a brown solid (344 mg). To the obtained brown solid (344 mg), methanol (10 mL) and a 1 mol/L aqueous sodium hydroxide solution (10 mL) were added, and the mixture was heated with stirring at 60 to 70°C for 6 hours. After cooling of the reaction mixture, the solvent was distilled off under reduced pressure. The obtained residue was suspended in water, and the deposit was collected by filtration. The obtained solid was purified by silica gel column chromatography [eluent: 60-0% hexane/ethyl acetate] to obtain a white solid of 8-(benzyloxy)-4-methyl-5-(((6-(2,2,2-trifluoroethoxy)pyridin-3-yl)methyl)amino)pyrido[2,3-d]pyrimidin-7(8H)-one (64 mg).
$^1$H-NMR ($CDCl_3$) δ value: 2.90 (s, 3H), 4.32 (d, J=4.4 Hz, 2H), 4.78 (q, J=8.5 Hz, 2H), 5.19-5.30 (m, 3H), 5.69 (s, 1H), 6.90 (d, J=8.6 Hz, 1H), 7.32-7.42 (m, 3H), 7.61-7.72 (m, 3H), 8.14-8.20 (m, 1H), 8.88 (s, 1H).

Reference Example 89

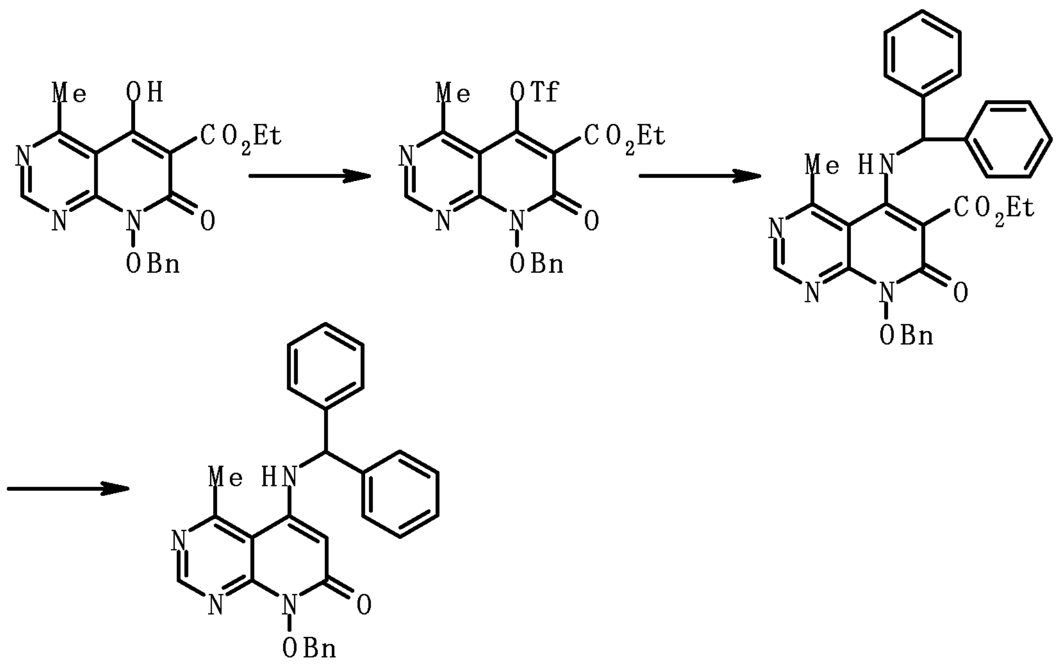

To a solution of ethyl 8-(benzyloxy)-5-hydroxy-4-methyl-7-oxo-7,8-dihydropyrido[2,3-d]pyrimidine-6-carboxylate (150 mg) and triethylamine (116 μL) in methylene chloride (5 mL), trifluoromethanesulfonic anhydride (138 μL) was added dropwise under ice cooling, and the mixture was stirred at room temperature for 1 hour. The solvent in the reaction mixture was distilled off under reduced pressure. To the obtained residue, benzhydrylamine (145 μL), triethylamine (170 μL) and dioxane (10 mL) were added, and the mixture was stirred at room temperature for 4 hours and 30 minutes. The solvent in the reaction mixture was distilled off under reduced pressure. The obtained residue was purified by silica gel column chromatography [eluent: 70-0% hexane/ethyl acetate] to obtain a white solid (200 mg). To the obtained white solid (200 mg), methanol (10 mL) and a 1 mol/L aqueous sodium hydroxide solution (10 mL) were added, and the mixture was heated with stirring at 60 to 70°C for 2 hours and 30 minutes. After cooling of the reaction mixture, the solvent was distilled off under reduced pressure, and ethyl acetate and water were added to the residue. An organic layer was separated, washed with a saturated aqueous solution of sodium chloride and then dried over sodium sulfate, and the solvent was distilled off under reduced pressure. The obtained residue was suspended in a mixed solvent of ethyl acetate and diisopropyl ether, and the deposit was collected by filtration to obtain a pale yellow solid of 8-(benzyloxy)-5-((diphenylmethyl)amino)-4-methylpyrido[2,3-d]pyrimidin-7(8H)-one (94 mg).
$^1$H-NMR ($CD_3OD$) δ value: 3.01 (s, 3H), 5.15 (s, 2H), 5.55 (s, 1H), 5.73 (s, 1H), 7.26-7.52 (m, 13H), 7.56-7.64 (m, 2H), 8.85 (s, 1H).

Reference Example 90

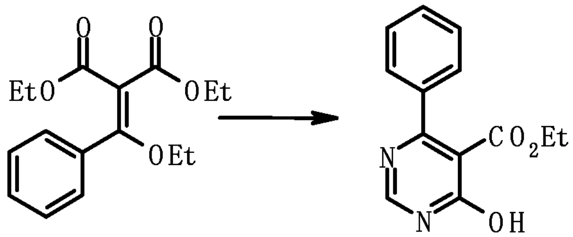

A suspension of diethyl (ethoxy(phenyl)methylene)malonate (14.90 g), formamidine acetate (7.62 g) and potassium tert-butoxide (15.06 g) in ethanol (70 mL) was stirred at room temperature for 1 hour and 35 minutes. The solvent in the reaction mixture was distilled off under reduced pressure, and ethyl acetate and water were added to the residue, followed by separation of an aqueous layer. The separated aqueous layer was pH-adjusted to 2 by the dropwise addition of 2 mol/L hydrochloric acid, and ethyl acetate was added thereto. An organic layer was separated, washed with a saturated aqueous solution of sodium chloride and then dried over sodium sulfate, and the solvent was distilled off under

reduced pressure. The obtained residue was suspended in diisopropyl ether, and the deposit was collected by filtration to obtain a white solid of ethyl 4-hydroxy-6-phenylpyrimidine-5-carboxylate (4.50 g). $^1$H-NMR ($CDCl_3$) $\delta$ value: 1.09 (t, J=7.1 Hz, 3H), 4.23 (q, J=7.1 Hz, 2H), 7.42-7.53 (m, 3H), 7.65-7.72 (m, 2H), 8.35 (s, 1H), 13.42 (brs, 1H).

Reference Example 91

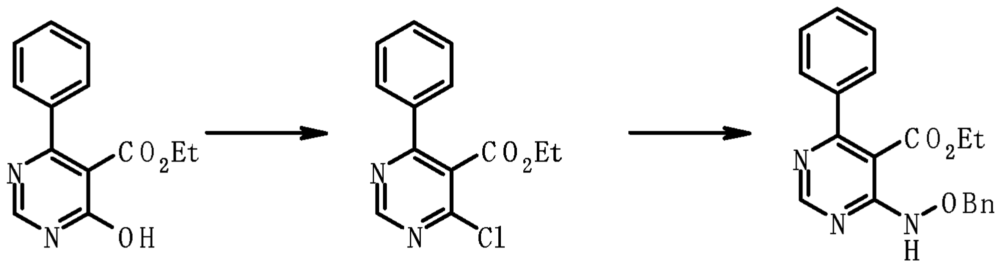

To a suspension of ethyl 4-hydroxy-6-phenylpyrimidine-5-carboxylate (2.50 g) and N-ethyldiisopropylamine (1.93 mL) in toluene (25 mL), phosphorus oxychloride (1.05 mL) was added dropwise, and the mixture was heated with stirring at 70 to 80°C for 1 hour. To the reaction mixture, a 1 mol/L aqueous sodium hydroxide solution (30 mL) was added under ice cooling, and ethyl acetate and water were then added. An organic layer was separated, washed with a saturated aqueous solution of sodium bicarbonate and a saturated aqueous solution of sodium chloride in this order and then dried over sodium sulfate, and the solvent was distilled off under reduced pressure to obtain a brown oil (2.80 g). A suspension of the obtained brown oil (2.70 g) and O-benzylhydroxylamine hydrochloride (2.62 g) in N-ethyldiisopropylamine (22 mL) was heated with stirring at 100 to 110°C for 1 hour. After cooling of the reaction mixture, chloroform and water were added thereto, followed by separation of an organic layer. An aqueous layer was extracted with chloroform, combined with the organic layer, washed with a saturated aqueous solution of sodium chloride and then dried over sodium sulfate, and the solvent was distilled off under reduced pressure. The obtained residue was purified by silica gel column chromatography [eluent: 70-30% hexane/ethyl acetate] to obtain a yellow oil of ethyl 4-((benzyloxy)amino)-6-phenylpyrimidine-5-carboxylate (3.30 g).
$^1$H-NMR ($CDCl_3$) $\delta$ value: 0.79 (t, J=7.2 Hz, 3H), 3.95 (q, J=7.2 Hz, 2H), 5.06 (s, 2H), 7.35-7.53 (m, 10H), 8.83 (s, 1H), 9.57 (s, 1H).

Reference Example 92

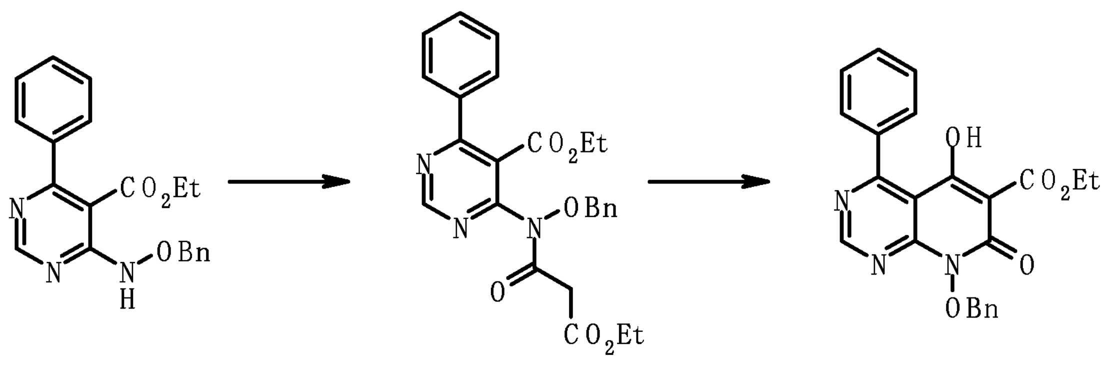

To a solution of ethyl 4-((benzyloxy)amino)-6-phenylpyrimidine-5-carboxylate (3.30 g) and triethylamine (2.62 mL) in methylene chloride (33 mL), ethylmalonyl chloride (2.42 mL) was added dropwise under ice cooling, methylene chloride (2 mL) was added, and the mixture was stirred at room temperature for 6 hours. To the reaction mixture, triethylamine (1.30 mL) was added, ethylmalonyl chloride (1.20 mL) was added dropwise under ice cooling, and the mixture was stirred at room temperature for 3 hours and 30 minutes. The solvent in the reaction mixture was distilled off under reduced pressure. The obtained residue was suspended in ethyl acetate, and insoluble matter was filtered off. The solvent in the filtrate was distilled off under reduced pressure to obtain a brown oil (7.63 g). To a solution of the

obtained brown oil (7.63 g) in ethanol (65 mL), a 20% solution of sodium ethoxide in ethanol (8.0 g) was added dropwise, and the mixture was stirred at room temperature for 2 hours and 45 minutes. The reaction mixture was pH-adjusted to 2 by the addition of 2 mol/L hydrochloric acid. Water (20 mL) was added thereto, and the mixture was stirred at room temperature for 20 minutes and for 10 minutes under ice cooling. The deposit was collected by filtration to obtain a pale yellow solid of ethyl 8-(benzyloxy)-5-hydroxy-7-oxo-4-phenyl-7,8-dihydropyrido[2,3-d]pyrimidine-6-carboxylate (1.11 g).
$^1$H-NMR ($CDCl_3$) δ value: 1.48 (t, J=7.1 Hz, 3H), 4.52 (q, J=7.2 Hz, 2H), 5.31 (s, 2H), 7.36-7.62 (m, 8H), 7.70-7.79 (m, 2H), 9.17 (s, 1H), 14.63 (s, 1H).

Reference Example 93

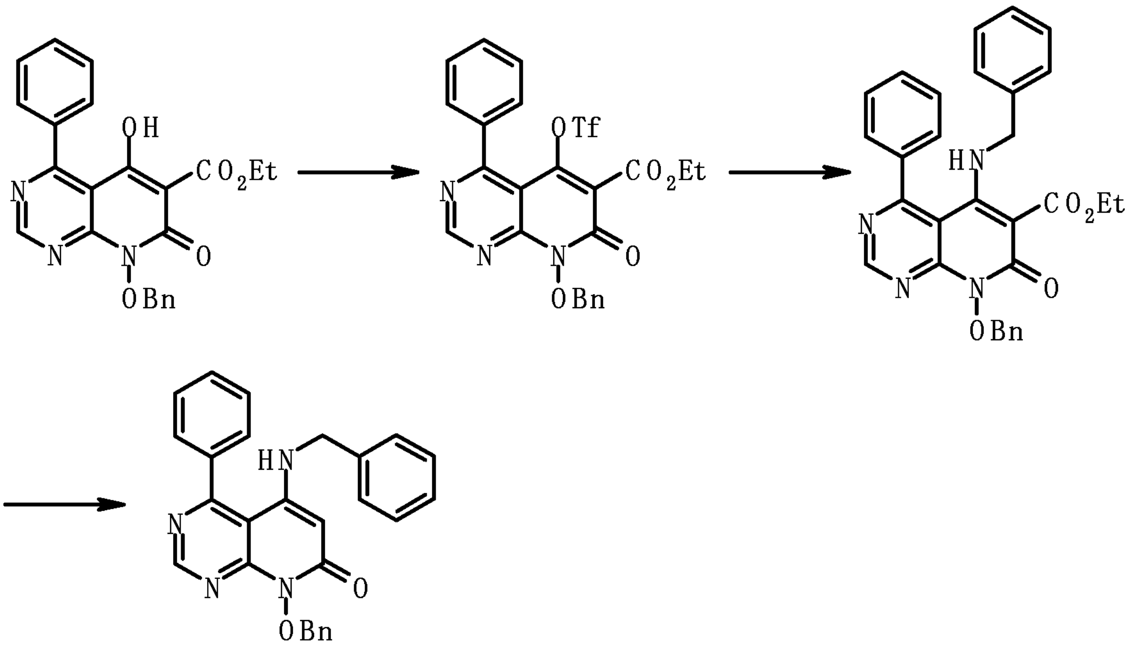

To a solution of ethyl 8-(benzyloxy)-5-hydroxy-7-oxo-4-phenyl-7,8-dihydropyrido[2,3-d]pyrimidine-6-carboxylate (150 mg) and triethylamine (100 μL) in methylene chloride (5 mL), trifluoromethanesulfonic anhydride (118 μL) was added dropwise under ice cooling, and the mixture was stirred for 40 minutes under ice cooling and at room temperature for 30 minutes. The solvent in the reaction mixture was distilled off under reduced pressure. To the obtained residue, benzylamine (197 μL) and dioxane (10 mL) were added, and the mixture was stirred at room temperature for 2 hours and 10 minutes. The solvent in the reaction mixture was distilled off under reduced pressure. The obtained residue was purified by silica gel column chromatography [eluent: 100-70% hexane/ethyl acetate] to obtain a pale yellow solid (48 mg). To the obtained pale yellow solid (48 mg), methanol (5 mL) and a 1 mol/L aqueous sodium hydroxide solution (5 mL) were added, and the mixture was heated with stirring at 60 to 70°C for 1 hour and 30 minutes and at 70 to 80°C for 3 hours. After cooling of the reaction mixture, the solvent was distilled off under reduced pressure. To the obtained residue, a 1 mol/L aqueous sodium hydroxide solution (4 mL) was added, the mixture was pH-adjusted to 3 by the dropwise addition of 6 mol/L hydrochloric acid and stirred for 30 minutes under ice cooling. The deposit was collected by filtration to obtain a pale yellow solid of 5-(benzylamino)-8-(benzyloxy)-4-phenylpyrido[2,3-d]pyrimidin-7(8H)-one (40 mg).
$^1$H-NMR (DMSO-$d_6$) δ value: 4.14 (d, J=4.6 Hz, 2H), 5.12-5.22 (m, 3H), 5.55 (s, 1H), 7.00-7.07 (m, 2H), 7.21-7.34 (m, 3H), 7.38-7.56 (m, 6H), 7.56-7.64 (m, 2H), 7.64-7.72 (m, 2H), 9.06-9.11 (m, 1H).

Reference Example 94

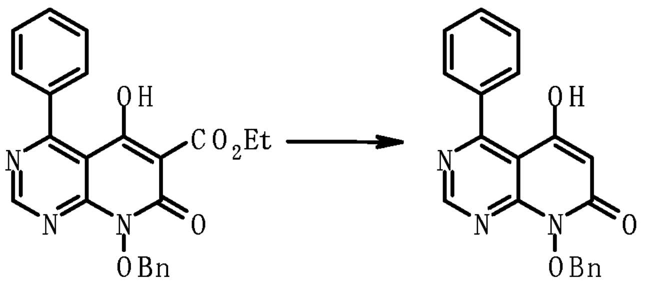

To ethyl 8-(benzyloxy)-5-hydroxy-7-oxo-4-phenyl-7,8-dihydropyrido[2,3-d]pyrimidine-6-carboxylate (246 mg), 2 mol/L hydrochloric acid (6 mL) and dioxane (6 mL) were added, and the mixture was heated to reflux for 5 hours and 15 minutes. After cooling of the reaction mixture, ethyl acetate and water were added thereto, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was suspended in a mixed solvent of methanol and diisopropyl ether, and the deposit was collected by filtration to obtain a pale yellow solid of 8-(benzyloxy)-5-hydroxy-4-phenylpyrido[2,3-d]pyrimidin-7(8H)-one (78 mg).
$^1$H-NMR (DMSO-$d_6$) δ value: 5.19 (s, 2H), 5.89 (s, 1H), 7.38-7.58 (m, 8H), 7.64-7.74 (m, 2H), 9.14 (s, 1H), 11.73 (brs, 1H).

Reference Example 95

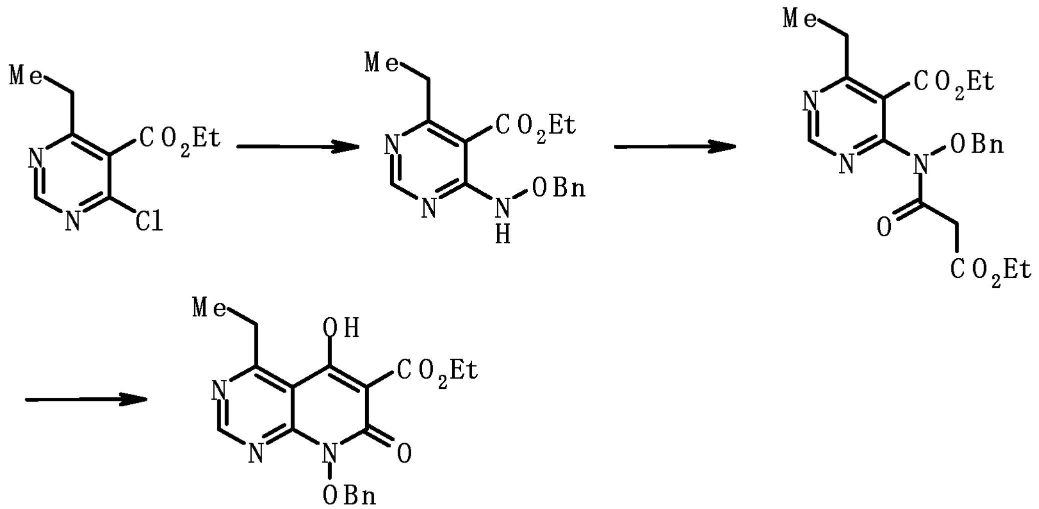

A suspension of ethyl 4-chloro-6-ethylpyrimidine-5-carboxylate (997 mg) and O-benzylhydroxylamine hydrochloride (1.25 g) in N-ethyldiisopropylamine (10 mL) was heated with stirring at 100 to 110°C for 1 hour. After cooling of the reaction mixture, chloroform and water were added thereto. An organic layer was separated, washed with a saturated aqueous solution of sodium chloride and then dried over sodium sulfate, and the solvent was distilled off under reduced pressure. The obtained residue was purified by silica gel column chromatography [eluent: 70-30% hexane/ethyl acetate] to obtain a yellow oil (1.44 g). To a solution of the obtained yellow oil (1.44 g) and triethylamine (1.40 mL) in methylene chloride (15 mL), ethylmalonyl chloride (1.25 mL) was added dropwise under ice cooling, methylene chloride (2 mL) was added, and the mixture was stirred at room temperature for 2 hours and 30 minutes. To the reaction mixture, triethylamine (1.40 mL) was added, ethylmalonyl chloride (1.25 mL) was added dropwise under ice cooling, and the mixture was stirred at room temperature for 3 hours. The solvent in the reaction mixture was distilled off under reduced pressure. The obtained residue was purified by silica gel column chromatography [eluent: 70-30% hexane/ethyl acetate] to obtain a yellow oil (1.90 g). To a solution of the obtained yellow oil (1.90 g) in ethanol (30 mL), a 20% solution of sodium ethoxide in ethanol (3.89 g) was added dropwise, ethanol (8 mL) was added, and the mixture was stirred at room temperature for 2 hours. The reaction mixture was pH-adjusted to 2 by the addition of 2 mol/L hydrochloric acid and stirred at room temperature for 15 minutes and for 15 minutes under ice cooling. The deposit was collected by filtration to obtain a yellow solid of ethyl 8-(benzyloxy)-4-ethyl-5-hydroxy-7-oxo-7,8-dihydropyrido[2,3-d]pyrimidine-6-

carboxylate (889 mg).
$^1$H-NMR ($CDCl_3$) δ value: 1.37 (t, J=7.4 Hz, 3H), 1.50 (t, J=7.1 Hz, 3H), 3.40 (q, J=7.3 Hz, 2H), 4.51-4.61 (m, 2H), 5.25 (s, 2H), 7.34-7.44 (m, 3H), 7.66-7.74 (m, 2H), 9.05 (s, 1H).

Reference Example 96

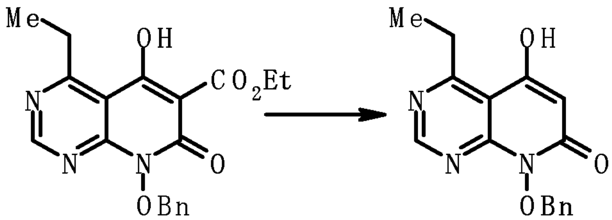

To ethyl 8-(benzyloxy)-4-ethyl-5-hydroxy-7-oxo-7,8-dihydropyrido[2,3-d]pyrimidine-6-carboxylate (880 mg), 2 mol/L hydrochloric acid (20 mL) and dioxane (20 mL) were added, and the mixture was heated to reflux for 3 hours. After cooling of the reaction mixture, ethyl acetate and water were added thereto. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a pale yellow solid of 8-(benzyloxy)-4-ethyl-5-hydroxypyrido[2,3-d]pyrimidin-7(8H)-one (497 mg).
$^1$H-NMR (DMSO-$d_6$) δ value: 1.26 (t, J=7.4 Hz, 3H), 3.29 (q, J=7.4 Hz, 2H), 5.13 (s, 2H), 6.02 (s, 1H), 7.36-7.49 (m, 3H), 7.60-7.69 (m, 2H), 9.02 (s, 1H), 12.28 (brs, 1H).

Reference Example 97

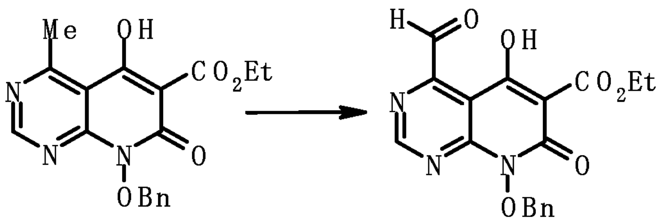

To selenium dioxide (284 mg) and 70% tert-butyl hydroperoxide (152 μL), dioxane (2 mL) was added, and the mixture was stirred at room temperature for 30 minutes. To the reaction mixture, ethyl 8-(benzyloxy)-5-hydroxy-4-methyl-7-oxo-7,8-dihydropyrido[2,3-d]pyrimidine-6-carboxylate (400 mg) and dioxane (6 mL) were added, and the mixture was heated with stirring at 40 to 50°C for 1 hour. The reaction mixture was filtered under reduced pressure, and insoluble matter was filtered off. Then, chloroform and water were added to the filtrate. An organic layer was separated, washed with a saturated aqueous solution of sodium chloride and then dried over sodium sulfate, and the solvent was distilled off under reduced pressure. The obtained residue was purified by silica gel column chromatography [eluent: 100-85% chloroform/methanol], and the obtained residue was suspended in a mixed solvent of 2-propanol and diisopropyl ether. The deposit was collected by filtration to obtain a yellow solid of ethyl 8-(benzyloxy)-4-formyl-5-hydroxy-7-oxo-7,8-dihydropyrido[2,3-d]pyrimidine-6-carboxylate (249 mg).
$^1$H-NMR (DMSO-$d_6$) δ value: 1.20-1.38 (m, 3H), 2.80-4.90 (m, 2H), 5.15 (s, 2H), 7.36-7.52 (m, 3H), 7.60-7.72 (m, 2H), 9.18 (s, 1H), 10.55 (brs, 1H).

Reference Example 98

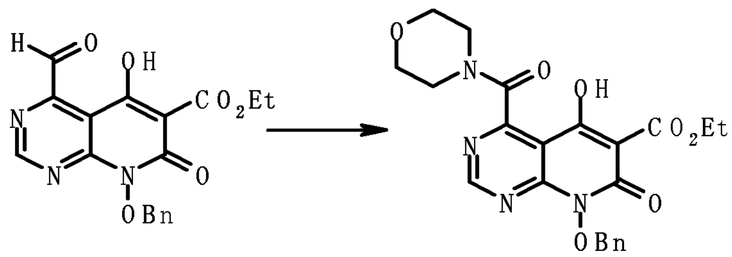

To a solution of ethyl 8-(benzyloxy)-4-formyl-5-hydroxy-7-oxo-7,8-dihydropyrido[2,3-d]pyrimidine-6-carboxylate (60 mg) and morpholine (32 μL) in methylene chloride (4 mL), sodium triacetoxyborohydride (84 mg) was added under ice cooling, and the mixture was stirred at room temperature for 2 hours. To the reaction mixture, chloroform and water were added. An organic layer was separated, washed with a saturated aqueous solution of sodium chloride and then dried over sodium sulfate, and the solvent was distilled off under reduced pressure. The obtained residue was purified by silica gel column chromatography [eluent: 100-70% chloroform/methanol] to obtain a white solid of ethyl 8-(benzyloxy)-5-hydroxy-4-((morpholin-4-yl)carbonyl)-7-oxo-7,8-dihydropyrido[2,3-d]pyrimidine-6-carboxylate (21 mg).
$^1$H-NMR ($CDCl_3$) δ value: 1.50 (t, J=7.2 Hz, 3H), 3.20-3.29 (m, 2H), 3.64-3.74 (m, 2H), 3.85 (s, 4H), 4.56 (q, J=7.2 Hz, 2H), 5.24 (s, 2H), 7.36-7.46 (m, 3H), 7.63-7.74 (m, 2H), 9.17 (s, 1H), 14.90 (s, 1H).

Reference Example 99

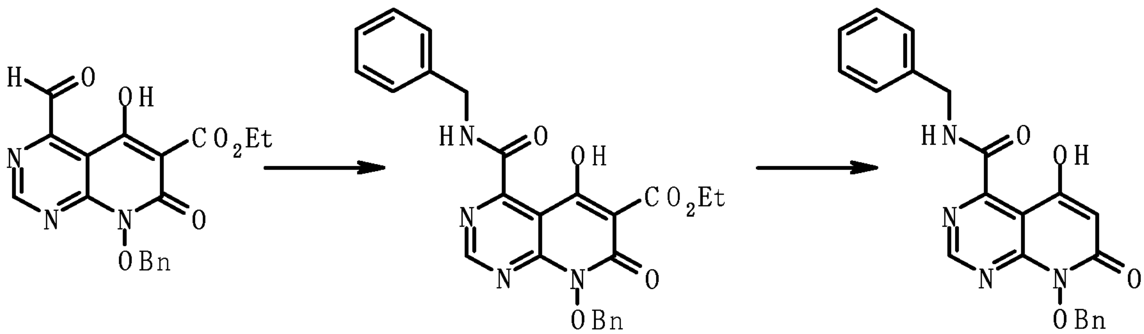

To a solution of ethyl 8-(benzyloxy)-4-formyl-5-hydroxy-7-oxo-7,8-dihydropyrido[2,3-d]pyrimidine-6-carboxylate (45 mg) and benzylamine (30 μL) in methylene chloride (3 mL), sodium triacetoxyborohydride (63 mg) was added under ice cooling, and the mixture was stirred at room temperature for 4 hours. To the reaction mixture, chloroform and water were added. An organic layer was separated, washed with a saturated aqueous solution of sodium chloride and then dried over sodium sulfate, and the solvent was distilled off under reduced pressure. The obtained residue was purified by silica gel column chromatography [eluent: 100-85% chloroform/methanol] to obtain a yellow solid (32 mg). To the obtained yellow solid (32 mg), 2 mol/L hydrochloric acid (2 mL) and dioxane (2 mL) were added, and the mixture was heated to reflux for 8 hours and 45 minutes. After cooling of the reaction mixture, ethyl acetate and water were added thereto. An organic layer was separated, washed with a saturated aqueous solution of sodium chloride and then dried over sodium sulfate, and the solvent was distilled off under reduced pressure. The obtained residue was purified by silica gel column chromatography [eluent: 100-90% chloroform/methanol] to obtain a yellow solid of N-benzyl-8-(benzyloxy)-5-hydroxy-7-oxo-7,8-dihydropyrido[2,3-d]pyrimidine-4-carboxamide (15 mg).
$^1$H-NMR ($CDCl_3$) δ value: 4.72 (d, J=6.1 Hz, 2H), 5.24-5.30 (m, 3H), 6.26 (s, 1H), 7.32-7.50 (m, 8H), 7.62-7.70 (m, 2H), 9.02 (s, 1H), 9.43 (brs, 1H).

Reference Example 100

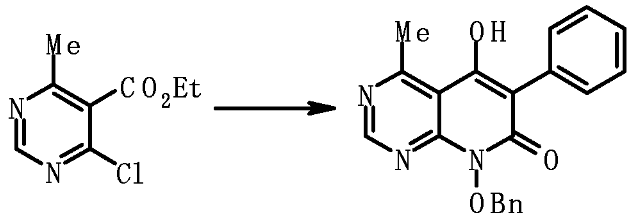

To a solution of ethyl 4-chloro-6-methylpyrimidine-5-carboxylate (250 mg) and N-(benzyloxy)-2-phenylacetamide (543 mg) in DMF (5 mL), potassium carbonate (432 mg) was added under ice cooling, and the mixture was stirred for 3 hours and 15 minutes under ice cooling, at room temperature for 6 hours, at 50 to 60°C for 2 hours and 30 minutes, and at 60 to 70°C for 3 hours and 30 minutes. After cooling of the reaction mixture, ethyl acetate and water were added thereto. An aqueous layer was separated and pH-adjusted to 2 by the dropwise addition of 2 mol/L hydrochloric acid, and ethyl acetate was added thereto. An organic layer was separated, washed with water and a saturated aqueous solution of sodium chloride in this order and then dried over sodium sulfate, and the solvent was distilled off under reduced pressure. The obtained residue was purified by silica gel column chromatography [eluent: 70-0% hexane/ethyl acetate] and then suspended in a mixed solvent of 2-propanol, diisopropyl ether, hexane and ethyl acetate, and the deposit was collected by filtration to obtain a yellow solid of 8-(benzyloxy)-5-hydroxy-4-methyl-6-phenylpyrido[2,3-d]pyrimidin-7(8H)-one (7 mg). $^1$H-NMR ($CD_3OD$) δ value: 3.00 (s, 3H), 5.26 (s, 2H), 7.32-7.56 (m, 8H), 7.60-7.68 (m, 2H), 8.92 (s, 1H).

Reference Example 101

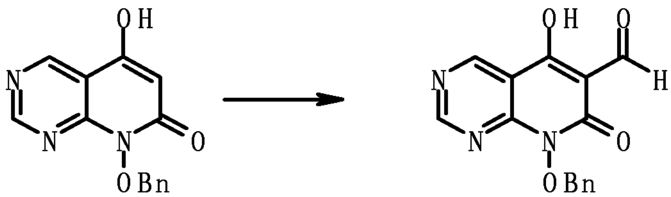

To 8-(benzyloxy)-5-hydroxypyrido[2,3-d]pyrimidin-7(8H)-one (150 mg), N,N-dimethylformamide dimethylacetal (750 μL) and methylene chloride (2.5 mL) were added, and the mixture was heated to reflux for 15 minutes. After cooling of the reaction mixture, the solvent was distilled off under reduced pressure. To the obtained residue, water (10 mL) was added, and the mixture was heated with stirring at 50°C for 5 minutes and then filtered under reduced pressure. Insoluble matter was filtered off. The solvent in the obtained filtrate was distilled off under reduced pressure. The obtained residue was pH-adjusted to 3 by the addition of 2 mol/L hydrochloric acid and stirred for 10 minutes under ice cooling. The deposit was collected by filtration to obtain an orange solid of 8-(benzyloxy)-5-hydroxy-7-oxo-7,8-dihydropyrido[2,3-d]pyrimidine-6-carbaldehyde (139 mg).
$^1$H-NMR (DMSO-$d_6$) δ value: 5.16 (s, 2H), 7.37-7.48 (m, 3H), 7.58-7.69 (m, 2H), 9.21 (s, 1H), 9.23 (s, 1H), 10.04 (s, 1H).

Reference Example 102

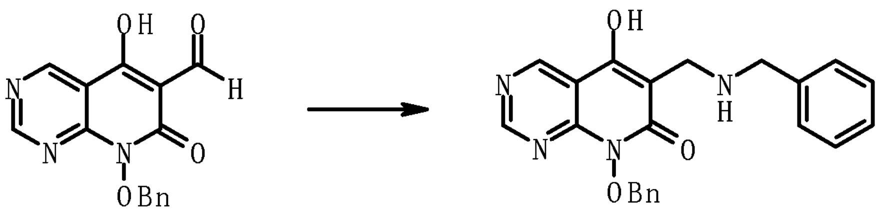

To a solution of 8-(benzyloxy)-5-hydroxy-7-oxo-7,8-dihydropyrido[2,3-d]pyrimidine-6-carbaldehyde (95 mg) and benzylamine (79 μL) in methylene chloride (2 mL), sodium triacetoxyborohydride (170 mg) was added under ice cooling, and the mixture was stirred at room temperature for 50 minutes. To the reaction mixture, ethyl acetate and water

were added. An organic layer was separated, washed with a saturated aqueous solution of sodium chloride and then dried over sodium sulfate, and the solvent was distilled off under reduced pressure. The obtained residue was purified by silica gel column chromatography [eluent: 100-80% chloroform/methanol] and then suspended in a mixed solvent of 2-propanol and diisopropyl ether, and the deposit was collected by filtration to obtain an orange solid of 6-((benzylamino)methyl)-8-(benzyloxy)-5-hydroxypyrido[2,3-d]pyrimidin-7(8H)-one (28 mg). $^1$H-NMR (DMSO-$d_6$) δ value: 3.95 (s, 2H), 4.11 (s, 2H), 5.11 (s, 2H), 7.34-7.50 (m, 6H), 7.52-7.60 (m, 2H), 7.60-7.70 (m, 2H), 8.96 (s, 1H), 8.99 (s, 1H).

Reference Example 103

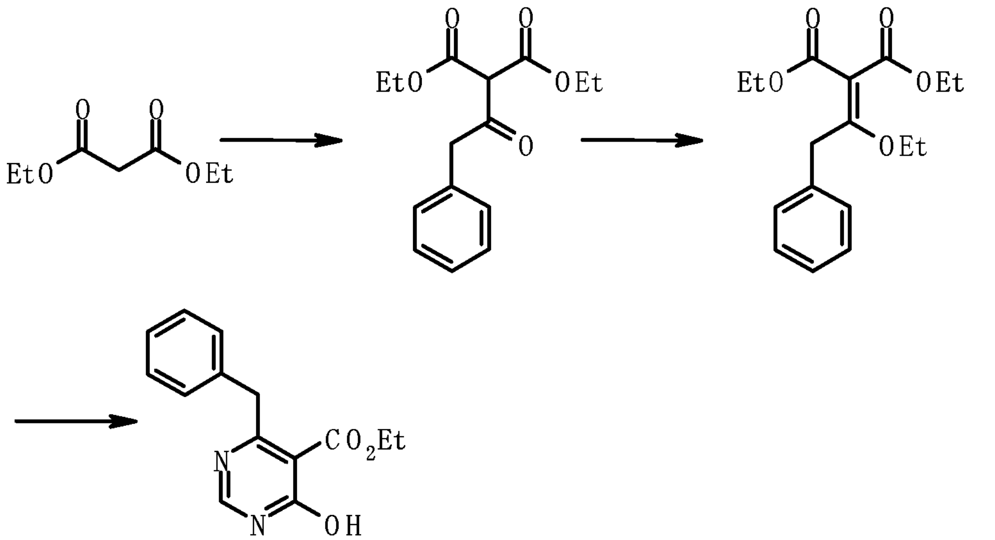

To a suspension of diethyl malonate (5.29 mL) and magnesium chloride (3.30 g) in acetonitrile (33 mL), triethylamine (9.70 mL) was added under ice cooling, and the mixture was stirred for 15 minutes under ice cooling. To the reaction mixture, phenylacetyl chloride (4.60 mL) was added dropwise, and the mixture was stirred for 40 minutes under ice cooling and at room temperature for 1 hour and 20 minutes. To the reaction mixture, 6 mol/L hydrochloric acid (15 mL) was added dropwise, and diethyl ether and water were added. An organic layer was separated, washed with a saturated aqueous solution of sodium chloride and then dried over sodium sulfate, and the solvent was distilled off under reduced pressure to obtain a pale yellow oil (9.95 g). To a suspension of the obtained pale yellow oil (9.95 g) and potassium carbonate (5.80 g) in DMF (40 mL), diethyl sulfate (6.90 mL) was added dropwise, and the mixture was heated with stirring at 70 to 80°C for 2 hours. After cooling of the reaction mixture, diethyl ether and water were added thereto. An organic layer was separated, washed with a saturated aqueous solution of sodium chloride and then dried over sodium sulfate, and the solvent was distilled off under reduced pressure to obtain a pale yellow oil (9.60 g). To the obtained pale yellow oil (9.60 g), formamidine acetate (4.89 g), potassium tert-butoxide (7.63 g) and ethanol (50 mL) were added, and the mixture was stirred at room temperature for 1 hour and 30 minutes. To the reaction mixture, ethyl acetate and water were added. An aqueous layer was separated and pH-adjusted to 2 by the dropwise addition of 6 mol/L hydrochloric acid, and ethyl acetate was added thereto. An organic layer was separated, washed with a saturated aqueous solution of sodium chloride and then dried over sodium sulfate, and the solvent was distilled off under reduced pressure. The obtained residue was purified by silica gel column chromatography [eluent: 60-0% hexane/ethyl acetate] to obtain a white solid of ethyl 4-benzyl-6-hydroxypyrimidine-5-carboxylate (472 mg).
$^1$H-NMR ($CDCl_3$) δ value: 1.36 (t, J=7.0 Hz, 3H), 3.98 (s, 2H), 4.40 (q, J=7.2 Hz, 2H), 7.20-7.40 (m, 5H), 8.12 (s, 1H), 12.96 (brs, 1H).

Reference Example 104

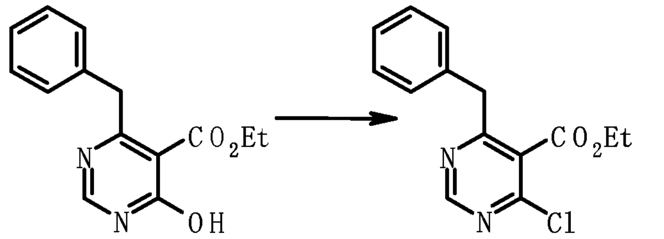

To a suspension of ethyl 4-benzyl-6-hydroxypyrimidine-5-carboxylate (830 mg) and N-ethyldiisopropylamine (0.60 mL) in toluene (17 mL), phosphorus oxychloride (0.33 mL) was added dropwise, and the mixture was heated with stirring at 70 to 80°C for 1 hour. To the reaction mixture, a 1 mol/L aqueous sodium hydroxide solution (10 mL) was added under ice cooling, and ethyl acetate and water were then added. An organic layer was separated, washed with a saturated aqueous solution of sodium bicarbonate and a saturated aqueous solution of sodium chloride in this order and then dried over sodium sulfate, and the solvent was distilled off under reduced pressure to obtain a brown oil of ethyl 4-benzyl-6-chloropyrimidine-5-carboxylate (755 mg).
$^1$H-NMR ($CDCl_3$) δ value: 1.32 (t, J=7.1 Hz, 3H), 4.16 (s, 2H), 4.36 (q, J=7.1 Hz, 2H), 7.18-7.42 (m, 5H), 8.93 (s, 1H).

Reference Example 105

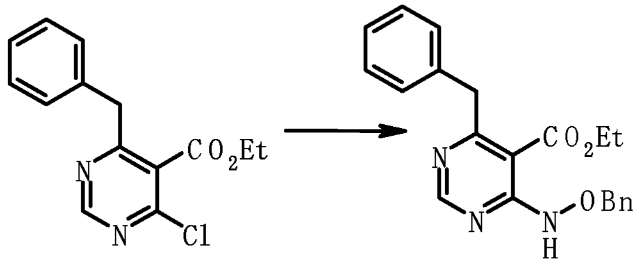

A suspension of ethyl 4-benzyl-6-chloropyrimidine-5-carboxylate (755 mg) and O-benzylhydroxylamine hydrochloride (740 mg) in N-ethyldiisopropylamine (10 mL) was heated with stirring at 100 to 110°C for 2 hours. After cooling of the reaction mixture, chloroform and water were added thereto. An organic layer was separated, washed with a saturated aqueous solution of sodium chloride and then dried over sodium sulfate, and the solvent was distilled off under reduced pressure. The obtained residue was purified by silica gel column chromatography [eluent: 90-30% hexane/ethyl acetate] to obtain a yellow oil of ethyl 4-benzyl-6-((benzyloxy)amino)pyrimidine-5-carboxylate (991 mg).
$^1$H-NMR ($CDCl_3$) δ value: 1.21 (t, J=7.2 Hz, 3H), 4.23 (q, J=7.1 Hz, 2H), 4.34 (s, 2H), 5.01 (s, 2H), 7.14-7.50 (m, 10H), 8.73 (s, 1H), 9.77 (brs, 1H).

Reference Example 106

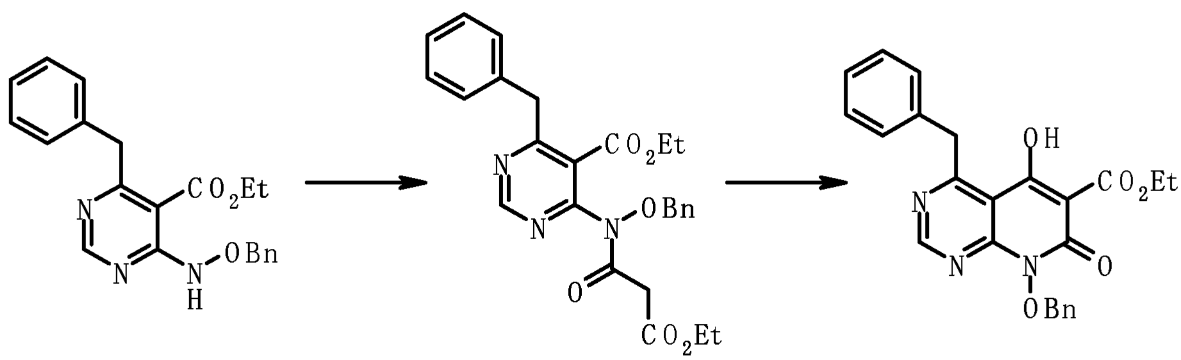

To a solution of ethyl 4-benzyl-6-((benzyloxy)amino)pyrimidine-5-carboxylate (991 mg) and triethylamine (762 μL) in methylene chloride (10 mL), ethylmalonyl chloride (704 μL) was added dropwise under ice cooling, and the mixture was stirred for 35 minutes under ice cooling and at room temperature for 2 hours and 15 minutes. To the reaction mixture, triethylamine (762 μL) was added, ethylmalonyl chloride (704 μL) was added dropwise under ice cooling, and the mixture was stirred at room temperature for 1 hour and 30 minutes. The solvent in the reaction mixture was distilled off under reduced pressure. The obtained residue was purified by silica gel column chromatography [eluent: 75-0% hexane/ethyl acetate] to obtain a brown oil (2.01 g). To a solution of the obtained brown oil (2.01 g) in ethanol (26 mL), a 20% solution of sodium ethoxide in ethanol (2.3 g) was added dropwise, and the mixture was stirred at room temperature for 1 hour and 10 minutes. The reaction mixture was pH-adjusted to 2 by the addition of 2 mol/L hydrochloric acid and stirred at room temperature for 15 minutes and for 15 minutes under ice cooling. The deposit was collected by filtration to obtain a yellow solid of ethyl 4-benzyl-8-(benzyloxy)-5-hydroxy-7-oxo-7,8-dihydropyrido[2,3-d]pyrimidine-6-carboxylate (834 mg).

$^1$H-NMR ($CDCl_3$) δ value: 1.49 (t, J=7.1 Hz, 3H), 4.55 (q, J=7.2 Hz, 2H), 4.75 (s, 2H), 5.23 (s, 2H), 7.20-7.48 (m, 8H), 7.65-7.74 (m, 2H), 9.09 (s, 1H).

Reference Example 107

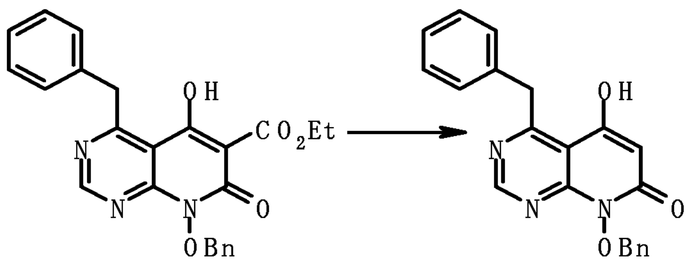

To ethyl 4-benzyl-8-(benzyloxy)-5-hydroxy-7-oxo-7,8-dihydropyrido[2,3-d]pyrimidine-6-carboxylate (150 mg), 2 mol/L hydrochloric acid (5 mL) and dioxane (5 mL) were added, and the mixture was heated to reflux for 3 hours. After cooling of the reaction mixture, ethyl acetate and water were added thereto. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a yellow solid of 4-benzyl-8-(benzyloxy)-5-hydroxypyrido[2,3-d]pyrimidin-7(8H)-one (40 mg).

$^1$H-NMR ($CDCl_3$) δ value: 4.70 (s, 2H), 5.25 (s, 2H), 6.33 (s, 1H), 7.14-7.42 (m, 8H), 7.58-7.68 (m, 2H), 9.05 (s, 1H), 11.17 (brs, 1H).

Reference Example 108

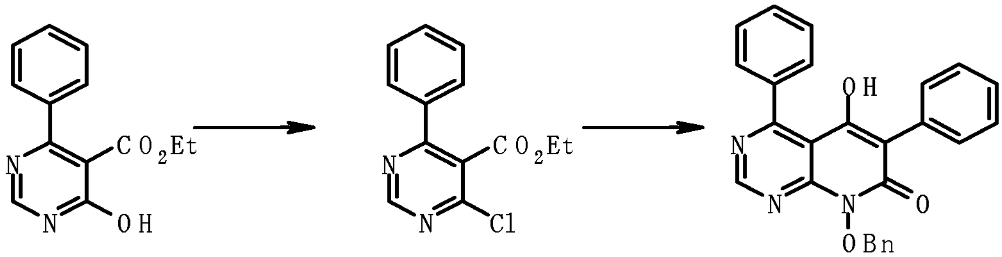

To a suspension of ethyl 4-hydroxy-6-phenylpyrimidine-5-carboxylate (2.50 g) and N-ethyldiisopropylamine (1.93 mL) in toluene (25 mL), phosphorus oxychloride (1.05 mL) was added dropwise, and the mixture was heated with stirring at 70 to 80°C for 1 hour. To the reaction mixture, a 1 mol/L aqueous sodium hydroxide solution (30 mL) was added under ice cooling, and ethyl acetate and water were then added. An organic layer was separated, washed with a saturated aqueous solution of sodium bicarbonate and a saturated aqueous solution of sodium chloride in this order

and then dried over sodium sulfate, and the solvent was distilled off under reduced pressure to obtain a brown oil (2.80 g). To a solution of the obtained brown oil (730 mg) and N-(benzyloxy)-2-phenylacetamide (941 mg) in DMF (10 mL), potassium carbonate (0.97 g) was added under ice cooling, and the mixture was stirred for 30 minutes under ice cooling, at room temperature for 3 hours and 30 minutes, at 50 to 60°C for 3 hours, and at 70 to 80°C for 5 hours and 30 minutes. To the reaction mixture, ethyl acetate and water were added. An aqueous layer was separated, washed four times with ethyl acetate and then pH-adjusted to 2 by the dropwise addition of 2 mol/L hydrochloric acid, and ethyl acetate was added thereto. An organic layer was separated, washed with a saturated aqueous solution of sodium chloride and then dried over sodium sulfate, and the solvent was distilled off under reduced pressure. The obtained residue was purified by silica gel column chromatography [eluent: 80-0% hexane/ethyl acetate] to obtain a yellow oil of 8-(benzyloxy)-5-hydroxy-4,6-diphenylpyrido[2,3-d]pyrimidin-7(8H)-one (79 mg).
$^1$H-NMR ($CDCl_3$) $\delta$ value: 5.36 (s, 2H), 6.22 (brs, 1H), 7.36-7.53 (m, 11H), 7.53-7.60 (m, 2H), 7.68-7.77 (m, 2H), 9.16 (s, 1H).

Reference Example 109

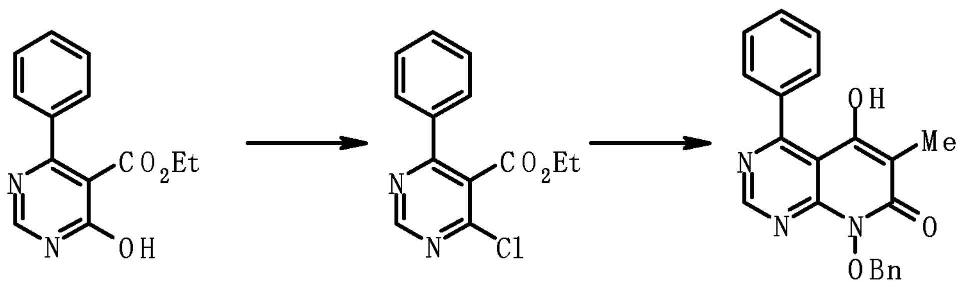

To a suspension of ethyl 4-hydroxy-6-phenylpyrimidine-5-carboxylate (2.50 g) and N-ethyldiisopropylamine (1.93 mL) in toluene (25 mL), phosphorus oxychloride (1.05 mL) was added dropwise, and the mixture was heated with stirring at 70 to 80°C for 1 hour. To the reaction mixture, a 1 mol/L aqueous sodium hydroxide solution (30 mL) was added under ice cooling, and ethyl acetate and water were then added. An organic layer was separated, washed with a saturated aqueous solution of sodium bicarbonate and a saturated aqueous solution of sodium chloride and then dried over sodium sulfate, and the solvent was distilled off under reduced pressure to obtain a brown oil (2.80 g). To a solution of the obtained brown oil (1.16 g) and N-(benzyloxy)propanamide (1.08 g) in DMF (12 mL), potassium carbonate (1.49 g) was added under ice cooling, and the mixture was stirred for 1 hour and 15 minutes under ice cooling, at room temperature for 9 hours, at 50 to 60°C for 1 hour and 30 minutes, at 60 to 70°C for 4 hours and 30 minutes, and at 50 to 60°C for 2 hours. To the reaction mixture, ethyl acetate and water were added. An aqueous layer was separated, washed with ethyl acetate and then pH-adjusted to 2 by the dropwise addition of 2 mol/L hydrochloric acid, and ethyl acetate was added thereto. An organic layer was separated, washed with water and a saturated aqueous solution of sodium chloride in this order and then dried over sodium sulfate, and the solvent was distilled off under reduced pressure. The obtained residue was purified by silica gel column chromatography [eluent: 65-0% hexane/ethyl acetate] to obtain a yellow oil of 8-(benzyloxy)-5-hydroxy-6-methyl-4-phenylpyrido[2,3-d]pyrimidin-7(8H)-one (182 mg).
$^1$H-NMR ($CDCl_3$) $\delta$ value: 2.11 (s, 3H), 5.30 (s, 2H), 7.34-7.47 (m, 3H), 7.56-7.64 (m, 5H), 7.68-7.78 (m, 2H), 9.07 (s, 1H).

Reference Example 110

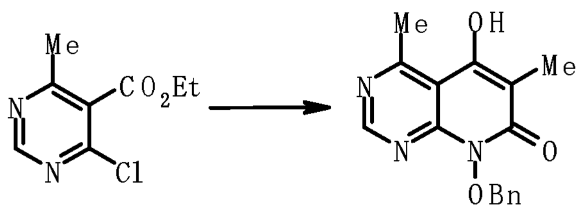

To a solution of ethyl 4-chloro-6-methylpyrimidine-5-carboxylate (598 mg) and N-(benzyloxy)propanamide (961 mg) in DMF (12 mL), potassium carbonate (1.03 g) was added under ice cooling, and the mixture was stirred for 1 hour

under ice cooling, at room temperature for 11 hours, at 50 to 60°C for 1 hour and 30 minutes, and at 60 to 70°C for 6 hours. To the reaction mixture, ethyl acetate and water were added. After separation of an aqueous layer, ethyl acetate was added thereto, and the mixture was pH-adjusted to 2 by the dropwise addition of 2 mol/L hydrochloric acid. An organic layer was separated, washed with water and a saturated aqueous solution of sodium chloride in this order and then dried over sodium sulfate, and the solvent was distilled off under reduced pressure. The obtained residue was purified by silica gel column chromatography [eluent: 60-0% hexane/ethyl acetate] to obtain a pale yellow solid of 8-(benzyloxy)-5-hydroxy-4,6-dimethylpyrido[2,3-d]pyrimidin-7(8H)-one (12 mg). $^1$H-NMR ($CDCl_3$) $\delta$ value: 2.22 (s, 3H), 2.99 (s, 3H), 5.26 (s, 2H), 7.33-7.44 (m, 3H), 7.64-7.74 (m, 2H), 8.95 (s, 1H).

Reference Example 111

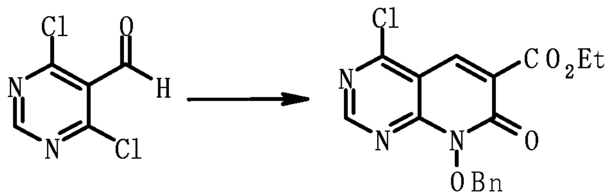

A solution of 4,6-dichloropyrimidine-5-carbaldehyde (619 mg), ethyl 3-((benzyloxy)amino)-3-oxopropanoate (1.08 g) and triethylamine (1.12 mL) in THF (12 mL) was stirred at room temperature for 2 hours. To the reaction mixture, ethyl acetate and water were added. An organic layer was separated, washed with a saturated aqueous solution of sodium chloride and then dried over sodium sulfate, and the solvent was distilled off under reduced pressure. The obtained residue was purified by silica gel column chromatography [eluent: 75-50% hexane/ethyl acetate] and then suspended in diisopropyl ether, and the deposit was collected by filtration to obtain a pale yellow solid of ethyl 8-(benzyloxy)-4-chloro-7-oxo-7,8-dihydropyrido[2,3-d]pyrimidine-6-carboxylate (424 mg).
$^1$H-NMR ($CDCl_3$) $\delta$ value: 1.45 (t, J=7.1 Hz, 3H), 4.48 (q, J=7.1 Hz, 2H), 5.30 (s, 2H), 7.37-7.44 (m, 3H), 7.63-7.72 (m, 2H), 8.65 (s, 1H), 8.94 (s, 1H).

Reference Example 112

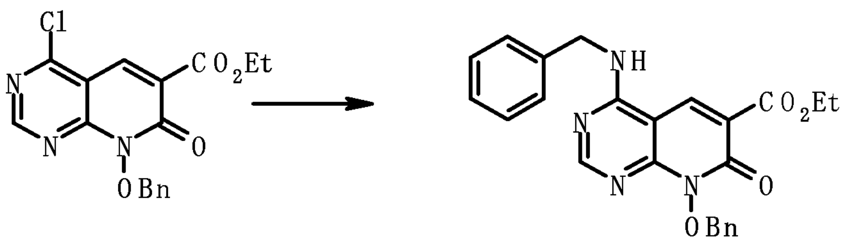

A solution of ethyl 8-(benzyloxy)-4-chloro-7-oxo-7,8-dihydropyrido[2,3-d]pyrimidine-6-carboxylate (153 mg), benzylamine (47 $\mu$L) and triethylamine (59 $\mu$L) in dioxane (5 mL) was stirred at room temperature for 3 hours and 30 minutes. To the reaction mixture, ethyl acetate and water were added. An organic layer was separated, washed with a saturated aqueous solution of sodium chloride and then dried over sodium sulfate, and the solvent was distilled off under reduced pressure. The obtained residue was purified by silica gel column chromatography [eluent: 75-50% hexane/ethyl acetate] to obtain a colorless oil of ethyl 4-(benzylamino)-8-(benzyloxy)-7-oxo-7,8-dihydropyrido[2,3-d]pyrimidine-6-carboxylate (146 mg).
$^1$H-NMR ($CDCl_3$) $\delta$ value: 1.38 (t, J=7.1 Hz, 3H), 4.39 (q, J=7.1 Hz, 2H), 4.84 (q, J=5.4 Hz, 2H), 5.26 (s, 2H), 6.24-6.32 (m, 1H), 7.28-7.43 (m, 8H), 7.64-7.73 (m, 2H), 8.47 (s, 1H), 8.62 (s, 1H).

Reference Example 113

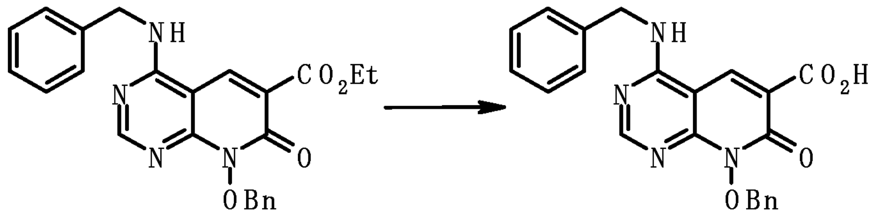

To ethyl 4-(benzylamino)-8-(benzyloxy)-7-oxo-7,8-dihydropyrido[2,3-d]pyrimidine-6-carboxylate (92 mg), dioxane (3 mL) and 2 mol/L hydrochloric acid (3 mL) were added, and the mixture was heated to reflux for 2 hours. After cooling of the reaction mixture, the deposit was collected by filtration to obtain a white solid of 4-(benzylamino)-8-(benzyloxy)-7-oxo-7,8-dihydropyrido[2,3-d]pyrimidine-6-carboxylic acid (43 mg).
$^1$H-NMR (DMSO-$d_6$) δ value: 4.77 (d, J=5.6 Hz, 2H), 5.21 (s, 2H), 7.24-7.30 (m, 1H), 7.30-7.40 (m, 4H), 7.41-7.49 (m, 3H), 7.63-7.69 (m, 2H), 8.60 (s, 1H), 9.20 (s, 1H), 9.47-9.55 (m, 1H).

Reference Example 114

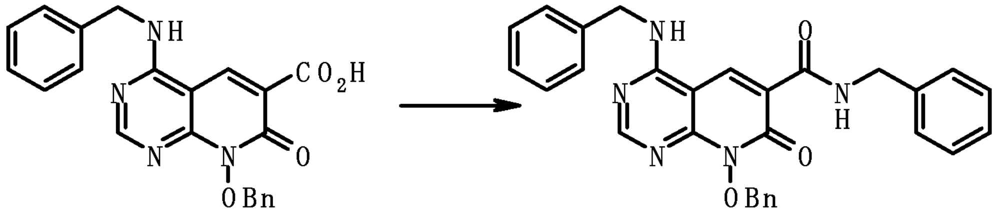

To 4-(benzylamino)-8-(benzyloxy)-7-oxo-7,8-dihydropyrido[2,3-d]pyrimidine-6-carboxylic acid (40 mg), benzylamine (16 μL), N-ethyldiisopropylamine (52 μL) and DMF (0.2 mL), HATU (57 mg) was added under ice cooling, and the mixture was stirred for 1 hour and 30 minutes under ice cooling and at room temperature for 3 hours. To the reaction mixture, chloroform and water were added. An organic layer was separated, washed with a saturated aqueous solution of sodium chloride and then dried over sodium sulfate, and the solvent was distilled off under reduced pressure. The obtained residue was purified by silica gel column chromatography [eluent: 70-40% hexane/ethyl acetate] to obtain a colorless oil of N-benzyl-4-(benzylamino)-8-(benzyloxy)-7-oxo-7,8-dihydropyrido[2,3-d]pyrimidine-6-carboxamide (28 mg).
$^1$H-NMR ($CDCl_3$) δ value: 4.57 (d, J=5.9 Hz, 2H), 4.76 (d, J=5.4 Hz, 2H), 5.28 (s, 2H), 7.01-7.14 (m, 1H), 7.20-7.43 (m, 13H), 7.61-7.71 (m, 2H), 8.63 (s, 1H), 9.04 (s, 1H), 9.78-9.91 (m, 1H).

Reference Example 115

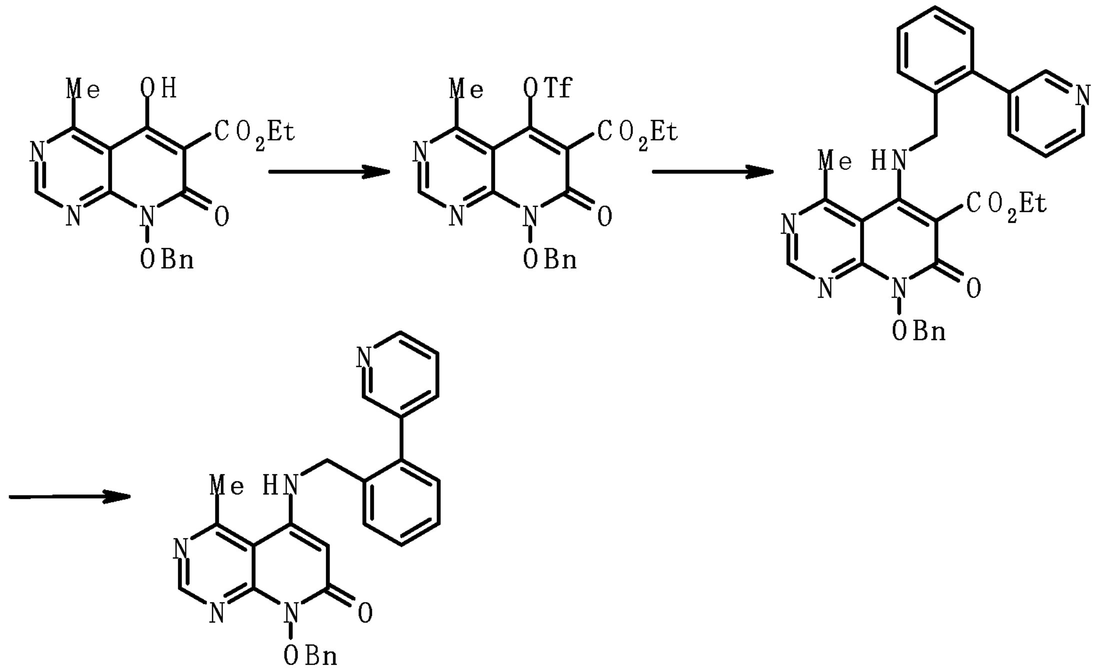

To a solution of ethyl 8-(benzyloxy)-5-hydroxy-4-methyl-7-oxo-7,8-dihydropyrido[2,3-d]pyrimidine-6-carboxylate (150 mg) and triethylamine (116 μL) in methylene chloride (5 mL), trifluoromethanesulfonic anhydride (138 μL) was added dropwise under ice cooling, and the mixture was stirred at room temperature for 1 hour. The solvent in the reaction mixture was distilled off under reduced pressure. To the obtained residue, 1-(2-(pyridin-3-yl)phenyl)methanamine dihydrochloride (162 mg), triethylamine (295 μL) and dioxane (12 mL) were added, and the mixture was stirred at room temperature for 5 hours and 5 minutes. The solvent in the reaction mixture was distilled off under reduced pressure. The obtained residue was purified by silica gel column chromatography [eluent: 100-80% chloroform/methanol] to obtain a brown oil (448 mg). To the obtained brown oil (448 mg), methanol (10 mL) and a 1 mol/L aqueous sodium hydroxide solution (10 mL) were added, and the mixture was heated with stirring at 60 to 70°C for 4 hours. After cooling of the reaction mixture, the solvent was distilled off under reduced pressure, and ethyl acetate and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 100-80% chloroform/methanol] and then suspended in a mixed solvent of 2-propanol and diisopropyl ether, and the deposit was collected by filtration to obtain a pale yellow solid of 8-(benzyloxy)-4-methyl-5-((2-(pyridin-3-yl)benzyl)amino)pyrido[2,3-d]pyrimidin-7(8H)-one (15 mg).
$^1$H-NMR ($CDCl_3$) δ value: 2.78 (s, 3H), 4.34 (d, J=4.9 Hz, 2H), 5.10-5.20 (m, 1H), 5.22 (s, 2H), 5.58 (s, 1H), 7.10-7.60 (m, 7H), 7.60-7.80 (m, 4H), 8.60-8.68 (m, 2H), 8.87 (s, 1H).

Reference Example 116

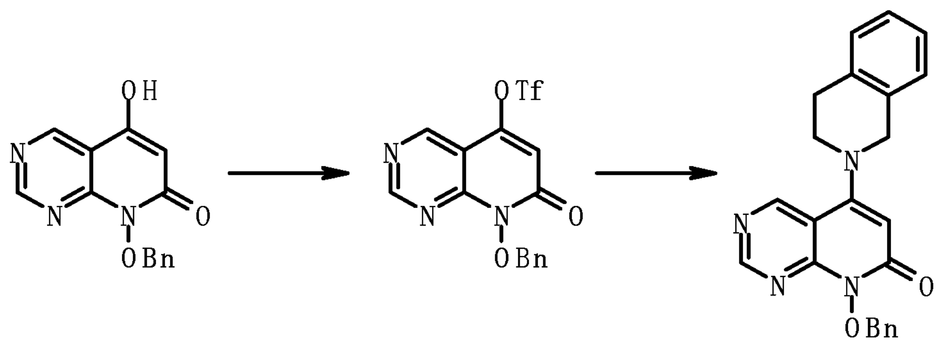

To a solution of 8-(benzyloxy)-5-hydroxypyrido[2,3-d]pyrimidin-7(8H)-one (100 mg) and triethylamine (103 μL)

in methylene chloride (5 mL), trifluoromethanesulfonic anhydride (122 μL) was added dropwise under ice cooling, and the mixture was stirred at room temperature for 3 hours. The solvent in the reaction mixture was distilled off under reduced pressure. To the obtained residue, 1,2,3,4-tetrahydroisoquinoline (236 μL) and dioxane (6 mL) were added, and the mixture was stirred at room temperature for 5 hours and 30 minutes. The solvent in the reaction mixture was distilled off under reduced pressure. The obtained residue was purified by silica gel column chromatography [eluent: 85-30% hexane/ethyl acetate] and then suspended in a mixed solvent of 2-propanol and diisopropyl ether, and the deposit was collected by filtration to obtain a pale yellow solid of 8-(benzyloxy)-5-(3,4-dihydroisoquinolin-2(1H)-yl)pyrido[2,3-d]pyrimidin-7(8H)-one (52 mg).
$^1$H-NMR (DMSO-$d_6$) δ value: 3.06-3.16 (m, 2H), 3.56-3.65 (m, 2H), 4.44 (s, 2H), 5.17 (s, 2H), 6.15 (s, 1H), 7.23 (s, 4H), 7.38-7.48 (m, 3H), 7.62-7.70 (m, 2H), 9.10 (s, 1H), 9.15 (s, 1H).

Reference Example 117

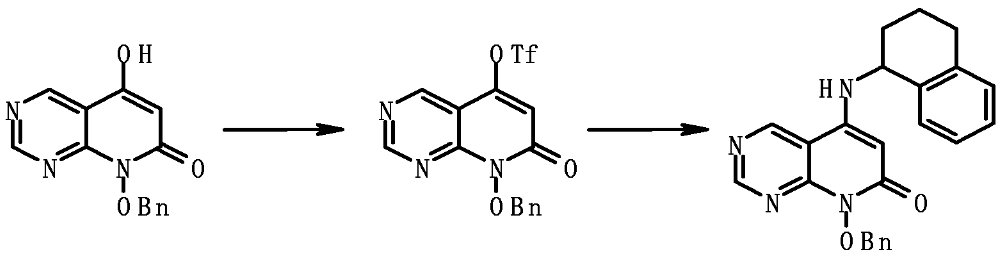

To a solution of 8-(benzyloxy)-5-hydroxypyrido[2,3-d]pyrimidin-7(8H)-one (100 mg) and triethylamine (103 μL) in methylene chloride (5 mL), trifluoromethanesulfonic anhydride (122 μL) was added dropwise under ice cooling, and the mixture was stirred at room temperature for 3 hours. The solvent in the reaction mixture was distilled off under reduced pressure. To the obtained residue, 1,2,3,4-tetrahydronaphthalen-1-amine (266 μL) and dioxane (5 mL) were added, and the mixture was stirred at room temperature for 6 hours. The reaction mixture was purified by silica gel column chromatography [eluent: 85-15% hexane/ethyl acetate] to obtain a brown oil of 8-(benzyloxy)-5-((1,2,3,4-tetrahydronaphthalen-1-yl)amino)pyrido[2,3-d]pyrimidin-7(8H)-one (6 mg).
$^1$H-NMR ($CDCl_3$) δ value: 1.75-2.00 (m, 2H), 2.00-2.20 (m, 2H), 2.65-3.00 (m, 2H), 4.72-4.84 (m, 1H), 5.06-5.14 (m, 1H), 5.28 (s, 2H), 5.91 (s, 1H), 7.05-7.45 (m, 7H), 7.60-7.75 (m, 2H), 8.76 (s, 1H), 9.07 (s, 1H).

Reference Example 118

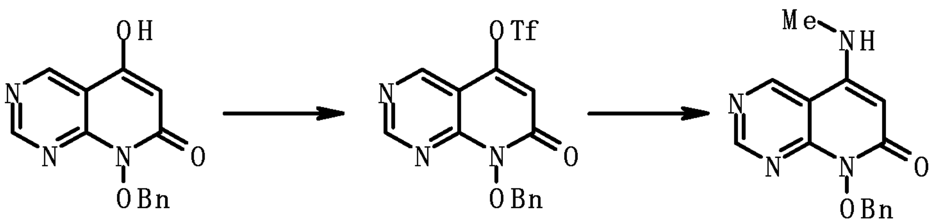

To a solution of 8-(benzyloxy)-5-hydroxypyrido[2,3-d]pyrimidin-7(8H)-one (100 mg) and triethylamine (103 μL) in methylene chloride (5 mL), trifluoromethanesulfonic anhydride (122 μL) was added dropwise under ice cooling, and the mixture was stirred at room temperature for 3 hours. The solvent in the reaction mixture was distilled off under reduced pressure. To the obtained residue, a 40% solution of methylamine in methanol (190 μL) and dioxane (5 mL) were added, and the mixture was stirred at room temperature for 6 hours. The solvent in the reaction mixture was distilled off under reduced pressure. The obtained residue was purified by silica gel column chromatography [eluent: 100-90% chloroform/methanol] to obtain a brown oil of 8-(benzyloxy)-5-(methylamino)pyrido[2,3-d]pyrimidin-7(8H)-one (8 mg).
$^1$H-NMR ($CDCl_3$) δ value: 2.98 (d, J=4.6 Hz, 3H), 5.26 (s, 2H), 5.36-5.50 (m, 1H), 5.68 (s, 1H), 7.30-7.48 (m, 3H), 7.54-7.72 (m, 2H), 8.90 (s, 1H), 9.07 (s, 1H).

Reference Example 119

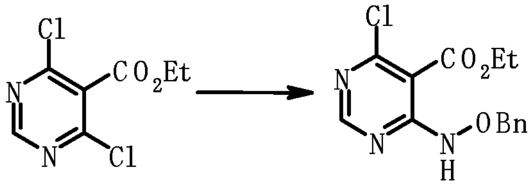

A suspension of ethyl 4,6-dichloropyrimidine-5-carboxylate (3.47 g), O-benzylhydroxylamine hydrochloride (2.51 g) and triethylamine (5.68 mL) in THF (20 mL) was stirred at room temperature for 5 hours and 30 minutes. The solvent in the reaction mixture was distilled off under reduced pressure, and ethyl acetate and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 90-40% hexane/ethyl acetate] to obtain a yellow oil of ethyl 4-((benzyloxy)amino)-6-chloropyrimidine-5-carboxylate (3.40 g).
$^1$H-NMR ($CDCl_3$) δ value: 1.35 (t, J=7.2 Hz, 3H), 4.33 (q, J=7.2 Hz, 2H), 4.98 (s, 2H), 7.30-7.49 (m, 5H), 8.51 (s, 1H), 9.63 (brs, 1H).

Reference Example 120

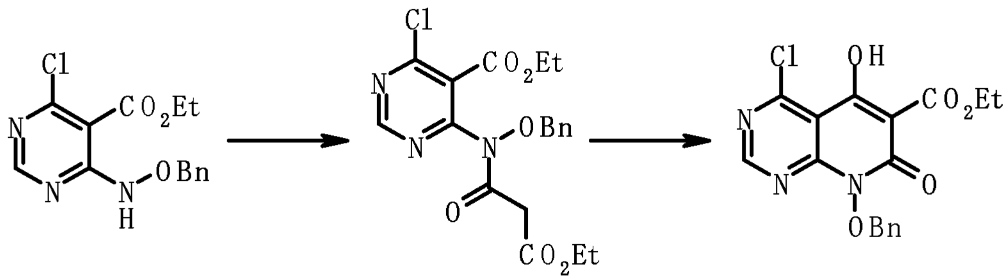

To a solution of ethyl 4-((benzyloxy)amino)-6-chloropyrimidine-5-carboxylate (3.40 g) and triethylamine (3.06 mL) in methylene chloride (25 mL), ethylmalonyl chloride (2.83 mL) was added dropwise under ice cooling, and the mixture was stirred at room temperature for 2 hours and 30 minutes. Insoluble matter was filtered off and washed with methylene chloride. The filtrate and the washes were combined, and the solvent was distilled off under reduced pressure. The obtained residue was purified by silica gel column chromatography [eluent: 95-40% hexane/ethyl acetate] to obtain a yellow oil (4.40 g). A solution of the obtained yellow oil (4.40 g) and triethylamine (2.89 mL) in methylene chloride (25 mL) was stirred at room temperature for 4 hours. To the reaction mixture, chloroform and water were added, and the mixture was pH-adjusted to 2 by the dropwise addition of 2 mol/L hydrochloric acid, followed by separation of an organic layer. An aqueous layer was extracted with chloroform, combined with the organic layer and dried over sodium sulfate, and the solvent was distilled off under reduced pressure. The obtained residue was suspended in diisopropyl ether, and the deposit was collected by filtration to obtain a pale yellow solid of ethyl 8-(benzyloxy)-4-chloro-5-hydroxy-7-oxo-7,8-dihydropyrido[2,3-d]pyrimidine-6-carboxylate (1.64 g).
$^1$H-NMR ($CDCl_3$) δ value: 1.50 (t, J=7.1 Hz, 3H), 4.57 (q, J=7.2 Hz, 2H), 5.24 (s, 2H), 7.35-7.45 (m, 3H), 7.63-7.71 (m, 2H), 8.89 (s, 1H).

Reference Example 121

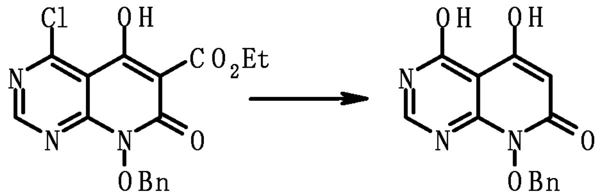

To ethyl 8-(benzyloxy)-4-chloro-5-hydroxy-7-oxo-7,8-dihydropyrido[2,3-d]pyrimidine-6-carboxylate (100 mg), dioxane (5 mL) and 2 mol/L hydrochloric acid (5 mL) were added, and the mixture was heated to reflux for 3 hours and 20 minutes. After cooling of the reaction mixture, ethyl acetate and water were added thereto. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a white solid of 8-(benzyloxy)-4,5-dihydroxypyrido[2,3-d]pyrimidin-7(8H)-one (60 mg).
$^1$H-NMR (DMSO-$d_6$) $\delta$ value: 5.11 (s, 2H), 5.78 (s, 1H), 7.38-7.47 (m, 3H), 7.56-7.64 (m, 2H), 8.52 (s, 1H), 11.84 (s, 1H), 13.50 (brs, 1H).

Reference Example 122

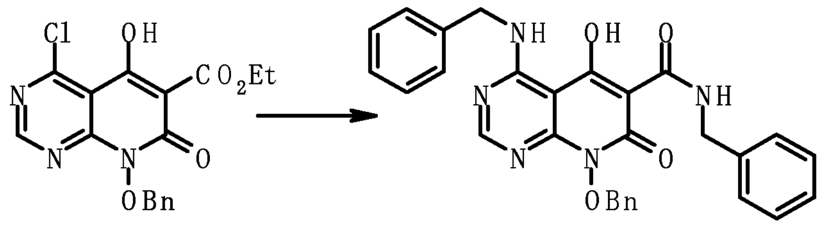

A solution of ethyl 8-(benzyloxy)-4-chloro-5-hydroxy-7-oxo-7,8-dihydropyrido[2,3-d]pyrimidine-6-carboxylate (80 mg), triethylamine (62 $\mu$L) and benzylamine (49 $\mu$L) in dioxane (5 mL) was heated to reflux for 1 hour and 30 minutes. After cooling of the reaction mixture, the solvent was distilled off under reduced pressure. The obtained residue was purified by silica gel column chromatography [eluent: 95-70% hexane/ethyl acetate] and then suspended in a mixed solvent of 2-propanol and diisopropyl ether, and the deposit was collected by filtration to obtain a pale yellow solid of N-benzyl-4-(benzylamino)-8-(benzyloxy)-5-hydroxy-7-oxo-7,8-dihydropyrido[2,3-d]pyrimidine-6-carboxamide (83 mg).
$^1$H-NMR ($CDCl_3$) $\delta$ value: 4.63 (d, J=6.0 Hz, 2H), 4.85 (d, J=5.6 Hz, 2H), 5.22 (s, 2H), 7.28-7.42 (m, 13H), 7.60-7.70 (m, 2H), 8.56 (s, 1H), 8.70-8.88 (m, 1H), 10.34-10.48 (m, 1H).

Reference Example 123

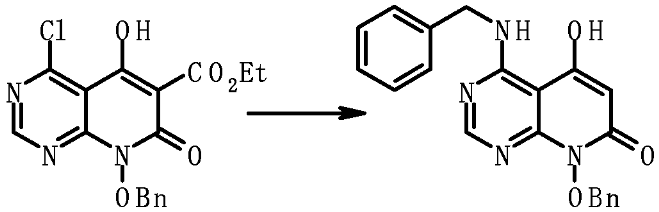

A solution of ethyl 8-(benzyloxy)-4-chloro-5-hydroxy-7-oxo-7,8-dihydropyrido[2,3-d]pyrimidine-6-carboxylate (80 mg), triethylamine (30 $\mu$L) and benzylamine (23 $\mu$L) in dioxane (5 mL) was heated to reflux for 2 hours. After cooling of the reaction mixture, the solvent was distilled off under reduced pressure. The obtained residue was purified by silica gel column chromatography [eluent: 100-95% chloroform/methanol] and then suspended in a mixed solvent of 2-propanol and diisopropyl ether, and the deposit was collected by filtration to obtain an orange solid of 4-(benzylamino)-8-(benzyloxy)-5-hydroxypyrido[2,3-d]pyrimidin-7(8H)-one (30 mg).

$^1$H-NMR (DMSO-$d_6$) δ value: 4.77 (d, J=6.0 Hz, 2H), 5.08 (s, 2H), 5.76 (s, 1H), 7.20-7.28 (m, 1H), 7.28-7.50 (m, 7H), 7.58-7.65 (m, 2H), 8.42 (s, 1H), 8.47-8.58 (m, 1H), 12.60 (brs, 1H).

Reference Example 124

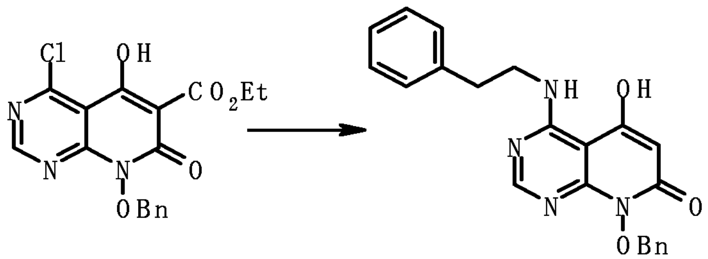

A solution of ethyl 8-(benzyloxy)-4-chloro-5-hydroxy-7-oxo-7,8-dihydropyrido[2,3-d]pyrimidine-6-carboxylate (80 mg), triethylamine (30 μL) and phenethylamine (27 μL) in dioxane (5 mL) was heated to reflux for 3 hours and 40 minutes. After cooling of the reaction mixture, the solvent was distilled off under reduced pressure. The obtained residue was purified by silica gel column chromatography [eluent: 100-90% chloroform/methanol] to obtain a pale yellow oil of 8-(benzyloxy)-5-hydroxy-4-((2-phenylethyl)amino)pyrido[2,3-d]pyrimidin-7(8H)-one (55 mg).
$^1$H-NMR ($CDCl_3$) δ value: 2.85 (t, J=7.4 Hz, 2H), 3.60-3.80 (m, 2H), 5.15 (s, 2H), 6.02 (s, 1H), 7.14-7.40 (m, 9H), 7.51-7.64 (m, 2H), 7.99 (brs, 1H), 8.46 (s, 1H).

Reference Example 125

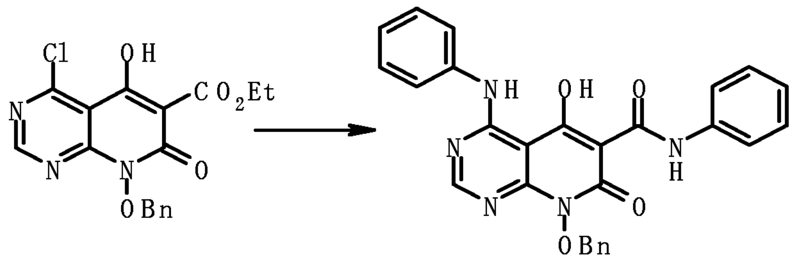

A solution of ethyl 8-(benzyloxy)-4-chloro-5-hydroxy-7-oxo-7,8-dihydropyrido[2,3-d]pyrimidine-6-carboxylate (80 mg), triethylamine (30 μL) and aniline (19 μL) in dioxane (5 mL) was heated to reflux for 30 minutes. After cooling of the reaction mixture, the solvent was distilled off under reduced pressure. The obtained residue was purified by silica gel column chromatography [eluent: 100-92% chloroform/methanol] to obtain a pale yellow solid of 4-anilino-8-(benzyloxy)-5-hydroxy-7-oxo-N-phenyl-7,8-dihydropyrido[2,3-d]pyrimidine-6-carboxamide (37 mg).
$^1$H-NMR ($CDCl_3$) δ value: 5.30 (s, 2H), 7.18-7.28 (m, 3H), 7.36-7.48 (m, 7H), 7.60-7.74 (m, 6H), 8.66 (s, 1H), 10.41 (s, 1H), 12.19 (s, 1H).

Reference Example 126

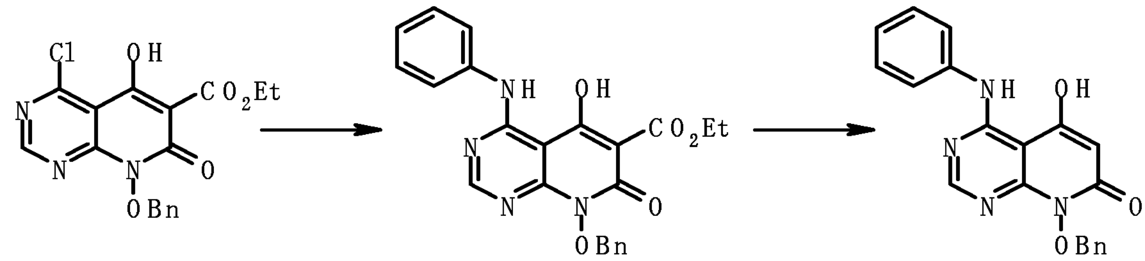

A solution of ethyl 8-(benzyloxy)-4-chloro-5-hydroxy-7-oxo-7,8-dihydropyrido[2,3-d]pyrimidine-6-carboxylate (100 mg), triethylamine (37 μL) and aniline (24 μL) in dioxane (5 mL) was stirred at room temperature for 2 hours. The solvent in the reaction mixture was distilled off under reduced pressure. The obtained residue was purified by silica gel column chromatography [eluent: 100-90% chloroform/methanol] to obtain a pale yellow solid (107 mg). To the obtained pale yellow solid (107 mg), dioxane (6 mL) and 2 mol/L hydrochloric acid (6 mL) were added, and the mixture was heated to reflux for 2 hours. After cooling of the reaction mixture, the solvent was distilled off under reduced pressure, and ethyl acetate and a saturated aqueous solution of sodium bicarbonate were added to the residue. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a light brown solid of 4-anilino-8-(benzyloxy)-5-hydroxypyrido[2,3-d]pyrimidin-7(8H)-one (48 mg).

$^1$H-NMR (DMSO-$d_6$) δ value: 5.07 (s, 2H), 5.28 (brs, 1H), 7.00-7.10 (m, 1H), 7.30-7.47 (m, 6H), 7.59-7.67 (m, 2H), 7.67-7.76 (m, 2H), 8.44 (s, 1H).

Reference Example 127

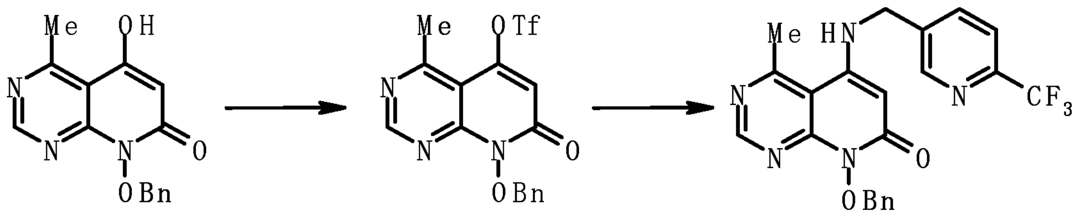

To a solution of 8-(benzyloxy)-5-hydroxy-4-methylpyrido[2,3-d]pyrimidin-7(8H)-one (150 mg) and triethylamine (147 μL) in methylene chloride (5 mL), trifluoromethanesulfonic anhydride (174 μL) was added dropwise under ice cooling, and the mixture was stirred at room temperature for 30 minutes. The solvent in the reaction mixture was distilled off under reduced pressure. To the obtained residue, 1-(6-(trifluoromethyl)pyridin-3-yl)methanamine (280 mg) and dioxane (7 mL) were added, and the mixture was heated with stirring at 70 to 80°C for 1 hour. After cooling of the reaction mixture, the solvent was distilled off under reduced pressure. The obtained residue was purified by silica gel column chromatography [eluent: 100-92% chloroform/methanol] and then suspended in a mixed solvent of 2-propanol and diisopropyl ether, and the deposit was collected by filtration to obtain a white solid of 8-(benzyloxy)-4-methyl-5-(((6-(trifluoromethyl)pyridin-3-yl)methyl)amino)pyrido[2,3-d]pyrimidin-7(8H)-one (18 mg).

$^1$H-NMR (DMSO-$d_6$) δ value: 3.04 (s, 3H), 4.68 (d, J=5.2 Hz, 2H), 5.07 (s, 2H), 5.46 (s, 1H), 7.00-7.12 (m, 1H), 7.34-7.48 (m, 3H), 7.54-7.72 (m, 2H), 7.84-7.98 (m, 1H), 8.09-8.18 (m, 1H), 8.83-8.88 (m, 1H), 8.91 (s, 1H).

Reference Example 128

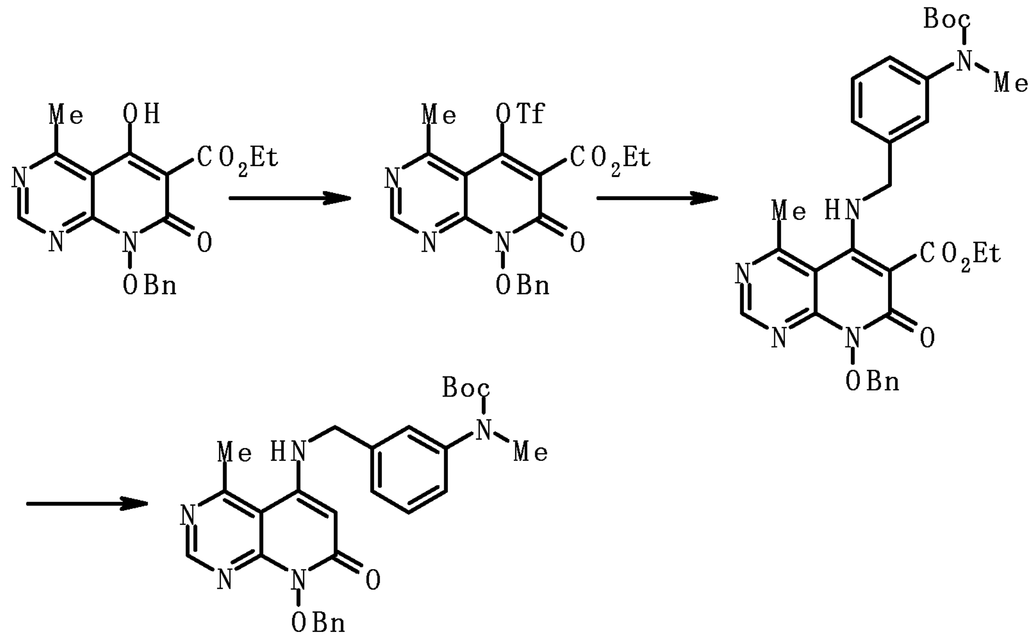

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 3 hours and 30 minutes. The solvent in the reaction mixture was distilled off under reduced pressure. To the obtained residue, tert-butyl (3-(aminomethyl)phenyl)(methyl)carbamate (150 mg), triethylamine (88 μL) and dioxane (10 mL) were added, and the mixture was stirred at room temperature for 4 hours. The solvent in the reaction mixture was distilled off under reduced pressure. The obtained residue was purified by silica gel column chromatography [eluent: 80-50% hexane/ethyl acetate] to obtain a brown oil (222 mg). To the obtained brown oil (222 mg), methanol (9 mL) and a 1 mol/L aqueous sodium hydroxide solution (9 mL) were added, and the mixture was heated with stirring at 50 to 60°C for 6 hours. After cooling of the reaction mixture, the solvent was distilled off under reduced pressure, and ethyl acetate and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 100-95% chloroform/methanol] to obtain a brown oil of tert-butyl (3-(((8-(benzyloxy)-4-methyl-7-oxo-7,8-dihydropyrido[2,3-d]pyrimidin-5-yl)amino)methyl)phenyl)(methyl)carbamate (64 mg). $^1$H-NMR ($CDCl_3$) δ value: 1.39-1.50 (m, 9H), 2.95 (s, 3H), 3.28 (s, 3H), 4.37 (d, J=4.6 Hz, 2H), 5.25 (s, 2H), 5.36-5.42 (m, 1H), 5.73 (s, 1H), 7.12-7.44 (m, 7H), 7.61-7.73 (m, 2H), 8.90 (s, 1H).

Reference Example 129

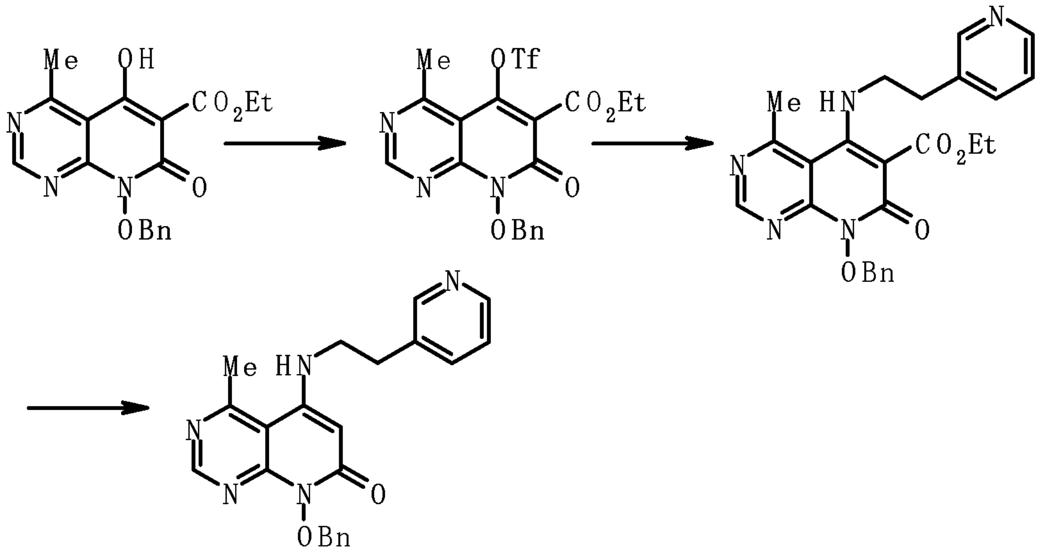

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 40 minutes. The solvent in the reaction mixture was distilled off under reduced pressure. To the obtained residue, 2-(pyridin-3-yl)ethanamine (99 μL), triethylamine (117 μL) and dioxane (6 mL) were added, and the mixture was stirred at room temperature for 2 hours and 15 minutes. The solvent in the reaction mixture was distilled off under reduced pressure. The obtained residue was purified by silica gel column chromatography [eluent: 100-95% chloroform/methanol] to obtain a brown oil (200 mg). To the obtained brown oil (200 mg), methanol (9 mL) and a 1 mol/L aqueous sodium hydroxide solution (9 mL) were added, and the mixture was heated with stirring at 60 to 70°C for 2 hours. After cooling of the reaction mixture, the solvent was distilled off under reduced pressure, and ethyl acetate and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 100-90% chloroform/methanol] to obtain a pale yellow solid of 8-(benzyloxy)-4-methyl-5-((2-(pylidin-3-yl)ethyl)amino)pyrido[2,3-d]pyrimidin-7(8H)-one (65 mg).

$^1$H-NMR ($CDCl_3$) δ value: 2.70 (s, 3H), 3.05 (t, J=6.8 Hz, 2H), 3.44-3.55 (m, 2H), 5.00-5.10 (m, 1H), 5.21 (s, 2H), 5.66 (s, 1H), 7.26-7.32 (m, 1H), 7.32-7.42 (m, 3H), 7.56-7.62 (m, 1H), 7.62-7.74 (m, 2H), 8.48-8.58 (m, 2H), 8.83 (s, 1H).

Reference Example 130

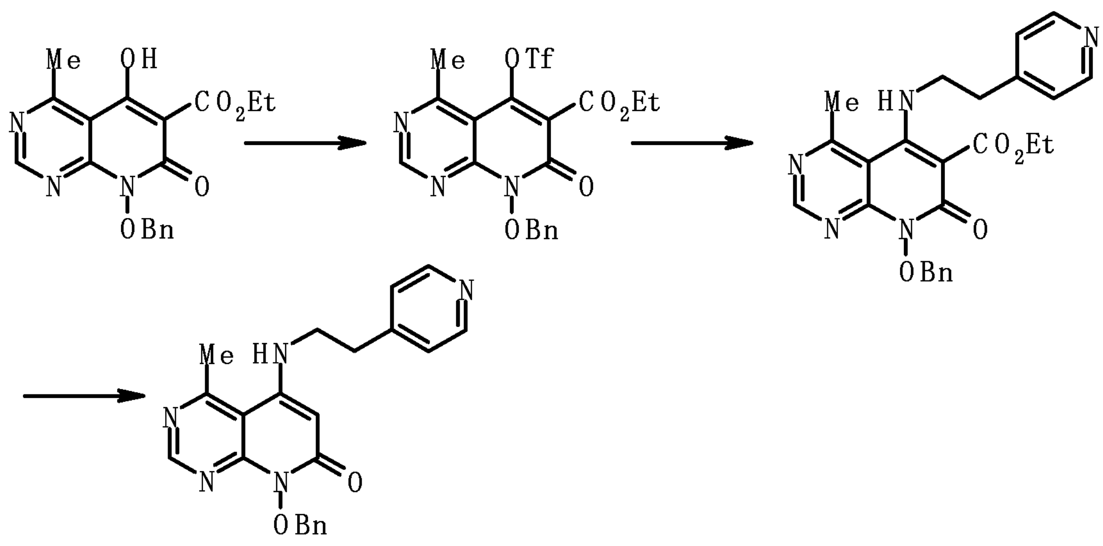

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 40 minutes. The solvent in the reaction mixture was distilled off under reduced pressure. To the obtained residue, 2-(pyridin-4-yl)ethanamine (100 μL), triethylamine (117 μL) and dioxane (6 mL) were added, and the mixture was stirred at room temperature for 3 hours and 40 minutes. The solvent in the reaction mixture was distilled off under reduced pressure. The obtained residue was purified by silica gel column chromatography [eluent: 100-90% chloroform/methanol] to obtain an orange oil (127 mg). To the obtained orange oil (127 mg), methanol (8 mL) and a 1 mol/L aqueous sodium hydroxide solution (8 mL) were added, and the mixture was heated with stirring at 50 to 60°C for 2 hours and 30 minutes. After cooling of the reaction mixture, the solvent was distilled off under reduced pressure, and ethyl acetate and water were added to the residue. An organic layer was separated, washed with a saturated aqueous solution of sodium chloride and then dried over sodium sulfate, and the solvent was distilled off under reduced pressure. The obtained residue was purified by silica gel column chromatography [eluent: 100-90% chloroform/methanol] to obtain a pale yellow oil of 8-(benzyloxy)-4-methyl-5-((2-(pyridin-4-yl)ethyl)amino)pyrido[2,3-d]pyrimidin-7(8H)-one (17 mg).
$^1$H-NMR ($CDCl_3$) δ value: 2.69 (s, 3H), 3.06 (t, J=6.7 Hz, 2H), 3.47-3.60 (m, 2H), 4.90-5.03 (m, 1H), 5.23 (s, 2H), 5.71 (s, 1H), 7.16-7.22 (m, 2H), 7.32-7.42 (m, 3H), 7.62-7.72 (m, 2H), 8.54-8.62 (m, 2H), 8.85 (s, 1H).

Reference Example 131

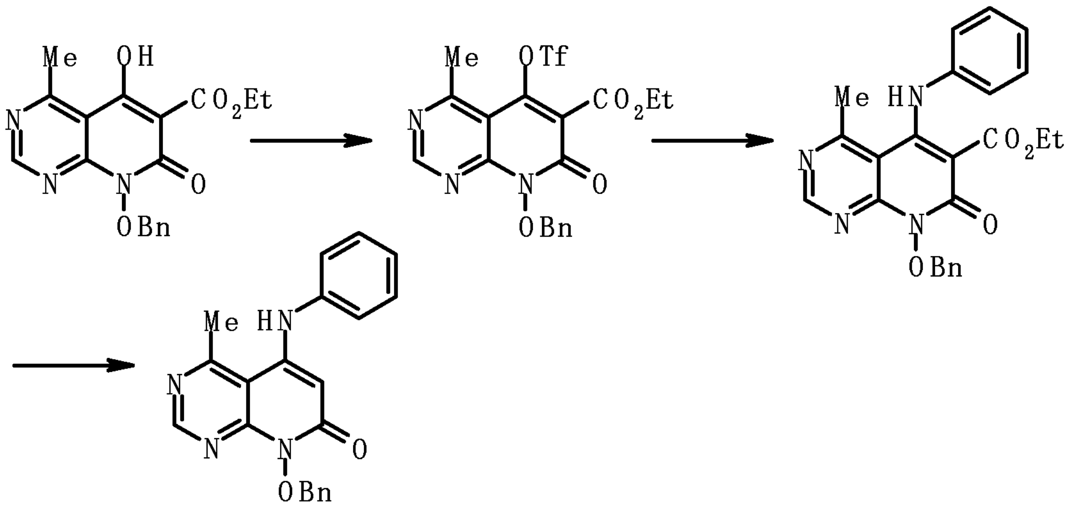

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 30 minutes. The solvent in the reaction mixture was distilled off under reduced pressure. To the obtained residue, aniline (77 μL), triethylamine (117 μL) and dioxane (6 mL) were added, and the mixture was stirred at room temperature for 5 hours and 30 minutes. The solvent in the reaction mixture was distilled off under reduced pressure. The obtained residue was purified by silica gel column chromatography [eluent: 100-95% chloroform/methanol] to obtain a brown oil (79 mg). To the obtained brown oil (79 mg), methanol (7 mL) and a 1 mol/L aqueous sodium hydroxide solution (7 mL) were added, and the mixture was heated with stirring at 50 to 60°C for 5 hours. After cooling of the reaction mixture, the solvent was distilled off under reduced pressure. The obtained residue was purified by silica gel column chromatography [eluent: 100-95% chloroform/methanol] to obtain a yellow oil of 5-anilino-8-(benzyloxy)-4-methylpyrido[2,3-d]pyrimidin-7(8H)-one (4 mg).
$^1$H-NMR ($CDCl_3$) δ value: 3.09 (s, 3H), 5.25 (s, 2H), 6.09 (s, 1H), 6.45 (s, 1H), 7.22-7.33 (m, 2H), 7.33-7.54 (m, 6H), 7.64-7.74 (m, 2H), 8.94 (s, 1H).

Reference Example 132

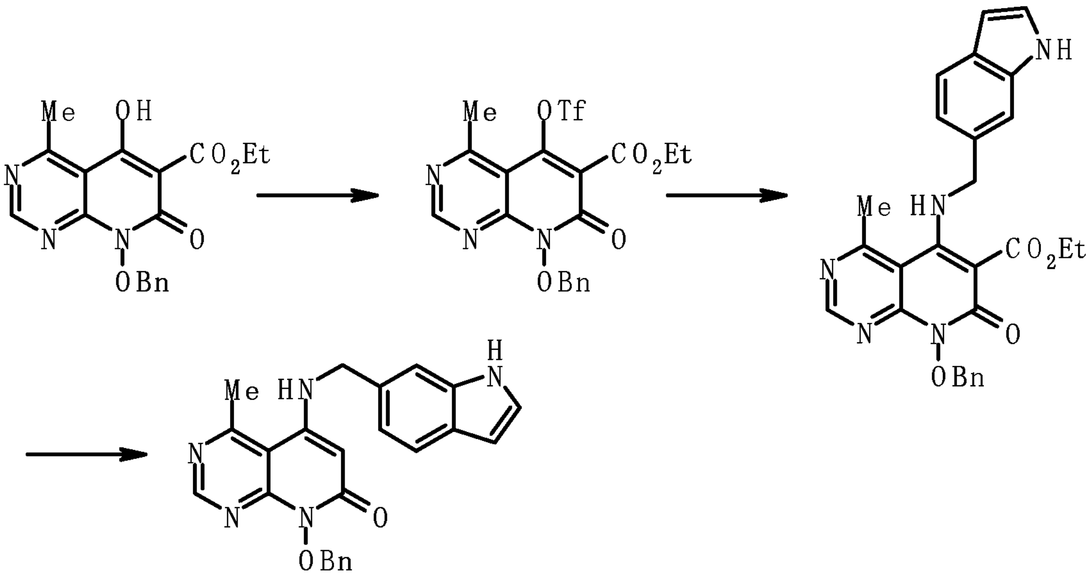

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 40 minutes. The solvent in the reaction mixture was distilled off under reduced pressure. To the obtained residue, 1-(1H-indol-6-yl)methanamine (123 mg), triethylamine (117 μL) and dioxane (7 mL) were added, and the mixture was stirred at room temperature for 1 hour and 10 minutes. The solvent in the reaction mixture was distilled off under reduced pressure. The obtained residue was purified by silica gel column chromatography [eluent: 85-0% hexane/ethyl acetate] to obtain a light brown solid (154 mg). To the obtained light brown solid (154 mg), methanol (8 mL) and a 1 mol/L aqueous sodium hydroxide solution (8 mL) were added, and the mixture was heated with stirring at 50 to 60°C for 3 hours. After cooling of the reaction mixture, the solvent was distilled off under reduced pressure. To the obtained residue, a 1 mol/L aqueous sodium hydroxide solution (5 mL) was added, and the mixture was pH-adjusted to 2 by the dropwise addition of concentrated hydrochloric acid and stirred at room temperature for 20 minutes. Then, the deposit was collected by filtration to obtain a pale yellow solid of 8-(benzyloxy)-5-(((1H-indol-6-yl)methyl)amino)-4-methylpyrido[2,3-d]pyrimidin-7(8H)-one (106 mg).
$^1$H-NMR (DMSO-$d_6$) δ value: 3.04 (s, 3H), 4.56 (d, J=5.1 Hz, 2H), 5.05 (s, 2H), 5.50 (s, 1H), 6.40 (brs, 1H), 6.92-7.04 (m, 1H), 7.09 (d, J=8.3 Hz, 1H), 7.27-7.68 (m, 8H), 8.89 (s, 1H), 11.04 (s, 1H).

Reference Example 133

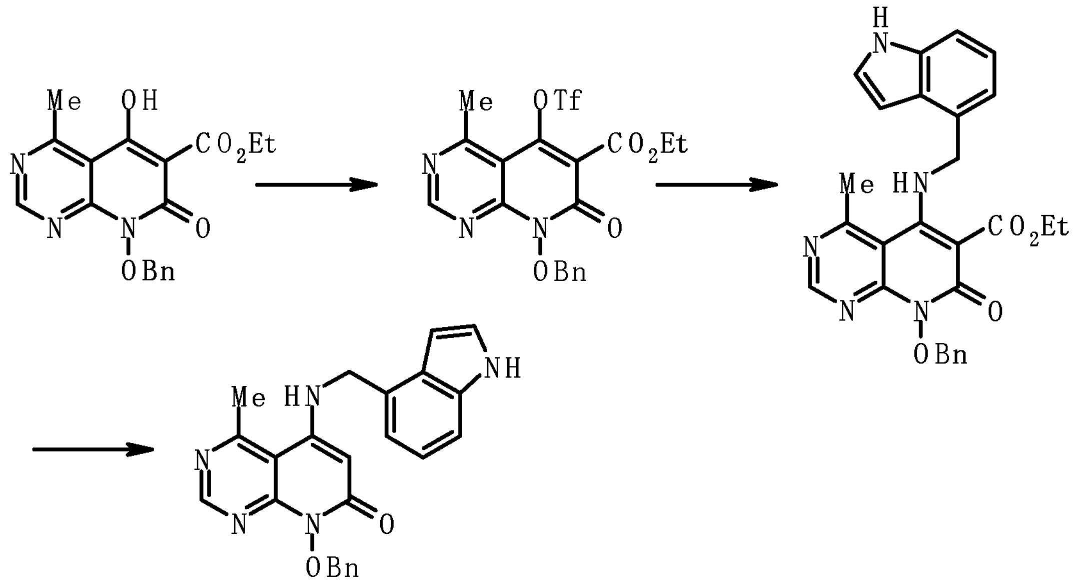

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 30 minutes. The solvent in the reaction mixture was distilled off under reduced pressure. To the obtained residue, 1-(1H-indol-4-yl)methanamine (123 mg), triethylamine (117 μL) and dioxane (6 mL) were added, and the mixture was stirred at room temperature for 2 hours. The solvent in the reaction mixture was distilled off under reduced pressure. The obtained residue was purified by silica gel column chromatography [eluent: 80-0% hexane/ethyl acetate] to obtain a brown oil (172 mg). To the obtained brown oil (170 mg), methanol (8 mL) and a 1 mol/L aqueous sodium hydroxide solution (8 mL) were added, and the mixture was heated with stirring at 50 to 60°C for 2 hours. After cooling of the reaction mixture, the solvent was distilled off under reduced pressure. To the obtained residue, a 1 mol/L aqueous sodium hydroxide solution (5 mL) was added, and the mixture was pH-adjusted to 2 by the dropwise addition of concentrated hydrochloric acid and stirred at room temperature for 20 minutes. Then, the deposit was collected by filtration to obtain a brown solid of 8-(benzyloxy)-5-(((1H-indol-4-yl)methyl)amino)-4-methylpyrido[2,3-d]pyrimidin-7(8H)-one (121 mg).

$^1$H-NMR (DMSO-$d_6$) δ value: 3.01 (s, 3H), 4.70 (d, J=5.1 Hz, 2H), 5.06 (s, 2H), 5.48 (s, 1H), 6.69 (s, 1H), 6.96-7.14 (m, 3H), 7.28-7.50 (m, 5H), 7.56-7.72 (m, 2H), 8.89 (s, 1H), 11.24 (s, 1H).

Reference Example 134

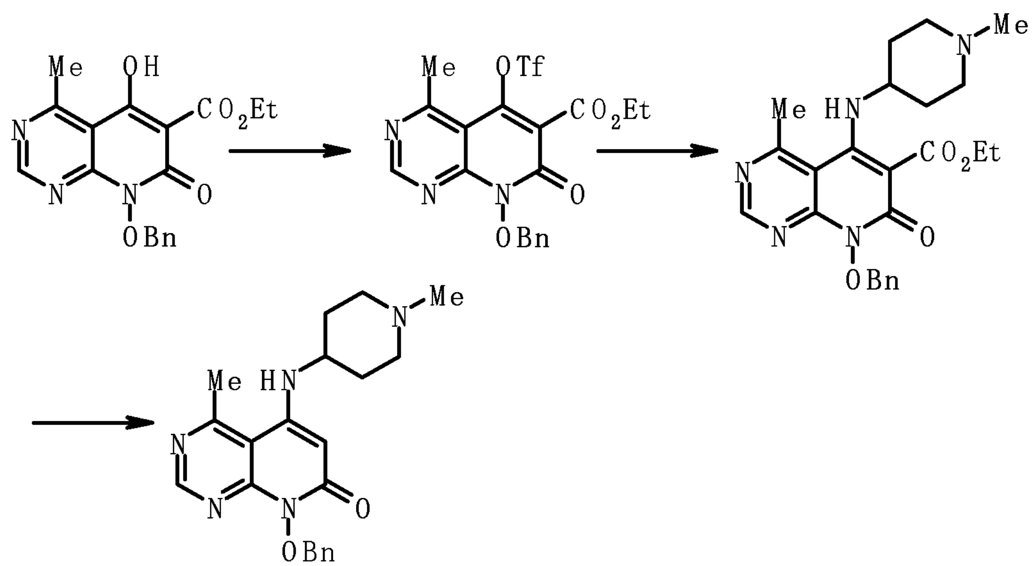

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 30 minutes. The solvent in the reaction mixture was distilled off under reduced pressure. To the obtained residue, 4-amino-1-methylpiperidine (106 μL), triethylamine (117 μL) and dioxane (6 mL) were added, and the mixture was stirred at room temperature for 1 hour and 55 minutes. The solvent in the reaction mixture was distilled off under reduced pressure. The obtained residue was purified by silica gel column chromatography [eluent: 100-90% chloroform/methanol] to obtain a yellow oil (247 mg). To the obtained yellow oil (247 mg), methanol (8 mL) and a 1 mol/L aqueous sodium hydroxide solution (8 mL) were added, and the mixture was heated with stirring at 50 to 60°C for 2 hours. After cooling of the reaction mixture, the solvent was distilled off under reduced pressure, and ethyl acetate and water were added to the residue. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a pale yellow solid of 8-(benzyloxy)-4-methyl-5-((1-methylpiperidin-4-yl)amino)pyrido[2,3-d]pyrimidin-7(8H)-one (46 mg).
$^1$H-NMR (DMSO-$d_6$) δ value: 1.54-1.70 (m, 2H), 1.88-2.04 (m, 2H), 2.06-2.22 (m, 2H), 2.19 (s, 3H), 2.61-2.78 (m, 2H), 2.92 (s, 3H), 3.25-3.50 (m, 1H), 5.10 (s, 2H), 5.61 (s, 1H), 5.98 (d, J=6.8 Hz, 1H), 7.36-7.46 (m, 3H), 7.58-7.68 (m, 2H), 8.86 (s, 1H).

Reference Example 135

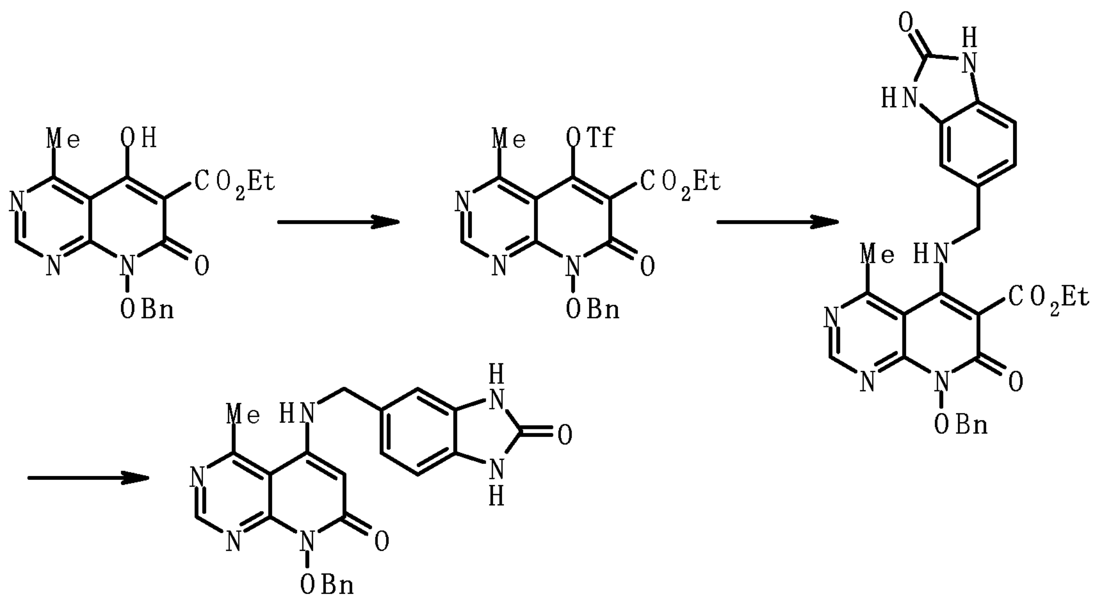

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 50 minutes. The solvent in the reaction mixture was distilled off under reduced pressure. To the obtained residue, 5-(aminomethyl)-1,3-dihydro-2H-benzimidazol-2-one hydrochloride (138 mg), triethylamine (234 μL) and dioxane (8 mL) were added, and the mixture was stirred at room temperature for 2 hours and 30 minutes. The solvent in the reaction mixture was distilled off under reduced pressure. The obtained residue was purified by silica gel column chromatography [eluent: 100-90% chloroform/methanol] to obtain a brown oil (89 mg). To the obtained brown oil (89 mg), methanol (7 mL) and a 1 mol/L aqueous sodium hydroxide solution (7 mL) were added, and the mixture was heated with stirring at 50 to 60°C for 1 hour and 25 minutes. After cooling of the reaction mixture, the solvent was distilled off under reduced pressure. The obtained residue was purified by silica gel column chromatography [eluent: 100-90% chloroform/methanol] to obtain a pale yellow solid of 8-(benzyloxy)-4-methyl-5-(((2-oxo-2,3-dihydro-1H-benzimidazol-5-yl)methyl)amino)pyrido[2,3-d]pyrimidin-7(8H)-one (37 mg).
$^1$H-NMR ($CD_3OD$) δ value: 3.03 (s, 3H), 4.52 (s, 2H), 5.16 (s, 2H), 5.64 (s, 1H), 7.01-7.07 (m, 1H), 7.10-7.16 (m, 2H), 7.32-7.39 (m, 3H), 7.57-7.66 (m, 2H), 8.85 (s, 1H).

Reference Example 136

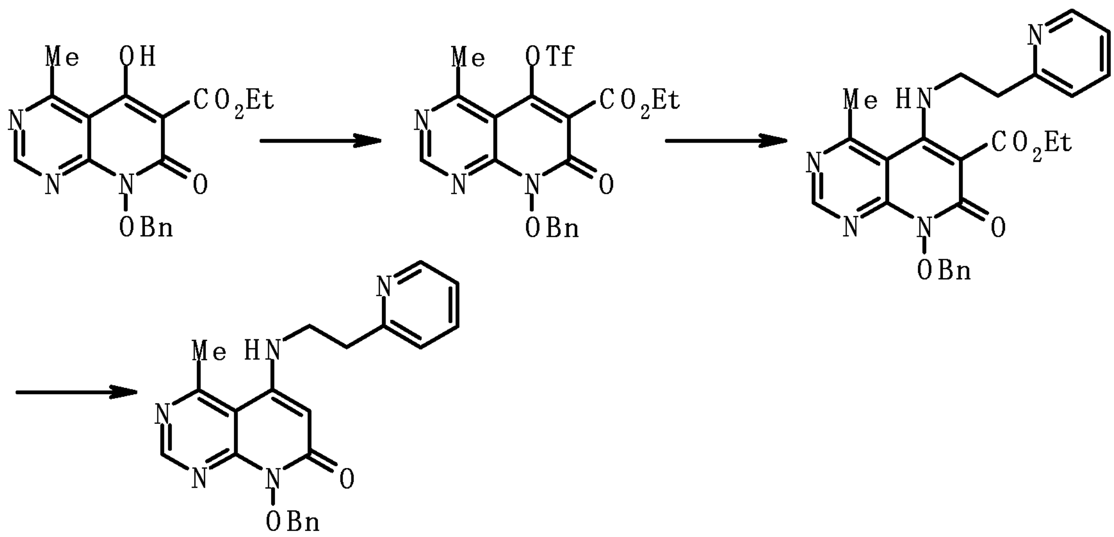

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 40 minutes. The solvent in the reaction mixture was distilled off under reduced pressure. To the obtained residue, 2-(pyridin-2-yl)ethanamine (101 μL), triethylamine (117 μL) and dioxane (6 mL) were added, and the mixture was stirred at room temperature for 1 hour. The solvent in the reaction mixture was distilled off under reduced pressure. The obtained residue was purified by silica gel column chromatography [eluent: 100-95% chloroform/methanol] to obtain a brown oil (142 mg). To the obtained brown oil (142 mg), methanol (7 mL) and a 1 mol/L aqueous sodium hydroxide solution (7 mL) were added, and the mixture was heated with stirring at 50 to 60°C for 1 hour. After cooling of the reaction mixture, the solvent was distilled off under reduced pressure, and ethyl acetate and water were added to the residue. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a pale yellow solid of 8-(benzyloxy)-4-methyl-5-((2-(pyridin-2-yl)ethyl)amino)pyrido[2,3-d]pyrimidin-7(8H)-one (65 mg).
$^1$H-NMR ($CDCl_3$) δ value: 3.08 (s, 3H), 3.21 (t, J=5.9 Hz, 2H), 3.44-3.60 (m, 2H), 5.24 (s, 2H), 5.67 (s, 1H), 7.05-7.18 (m, 1H), 7.18-7.44 (m, 5H), 7.60-7.80 (m, 3H), 8.47-8.57 (m, 1H), 8.88 (s, 1H).

Reference Example 137

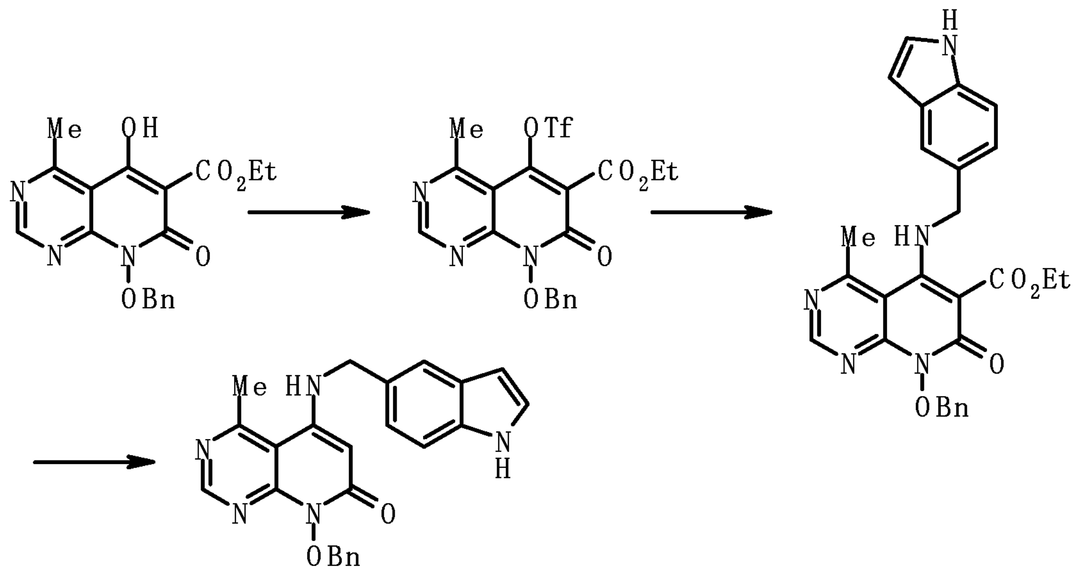

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 50 minutes. The solvent in the reaction mixture was distilled off under reduced pressure. To the obtained residue, 1-(1H-indol-5-yl)methanamine oxalate (199 mg), triethylamine (351 μL) and dioxane (7 mL) were added, and the mixture was stirred at room temperature for 2 hours and 25 minutes. The solvent in the reaction mixture was distilled off under reduced pressure. The obtained residue was purified by silica gel column chromatography [eluent: 85-0% hexane/ethyl acetate] to obtain a brown solid (73 mg). To the obtained brown solid (73 mg), methanol (5 mL) and a 1 mol/L aqueous sodium hydroxide solution (5 mL) were added, and the mixture was heated with stirring at 50 to 60°C for 2 hours. After cooling of the reaction mixture, the solvent was distilled off under reduced pressure. To the obtained residue, a 1 mol/L aqueous sodium hydroxide solution (5 mL) was added, and the mixture was pH-adjusted to 4 by the dropwise addition of concentrated hydrochloric acid and stirred at room temperature for 10 minutes. Then, the deposit was collected by filtration to obtain a light brown solid of 8-(benzyloxy)-5-(((1H-indol-5-yl)methyl)amino)-4-methylpyrido[2,3-d]pyrimidin-7(8H)-one (59 mg).
$^1$H-NMR (DMSO-$d_6$) δ value: 3.03 (s, 3H), 4.52 (d, J=5.1 Hz, 2H), 5.05 (s, 2H), 5.50 (s, 1H), 6.38-6.44 (m, 1H), 6.90-7.00 (m, 1H), 7.17 (dd, J=8.3, 1.5 Hz, 1H), 7.32-7.48 (m, 5H), 7.56-7.70 (m, 3H), 8.88 (s, 1H), 11.09 (s, 1H).

Reference Example 138

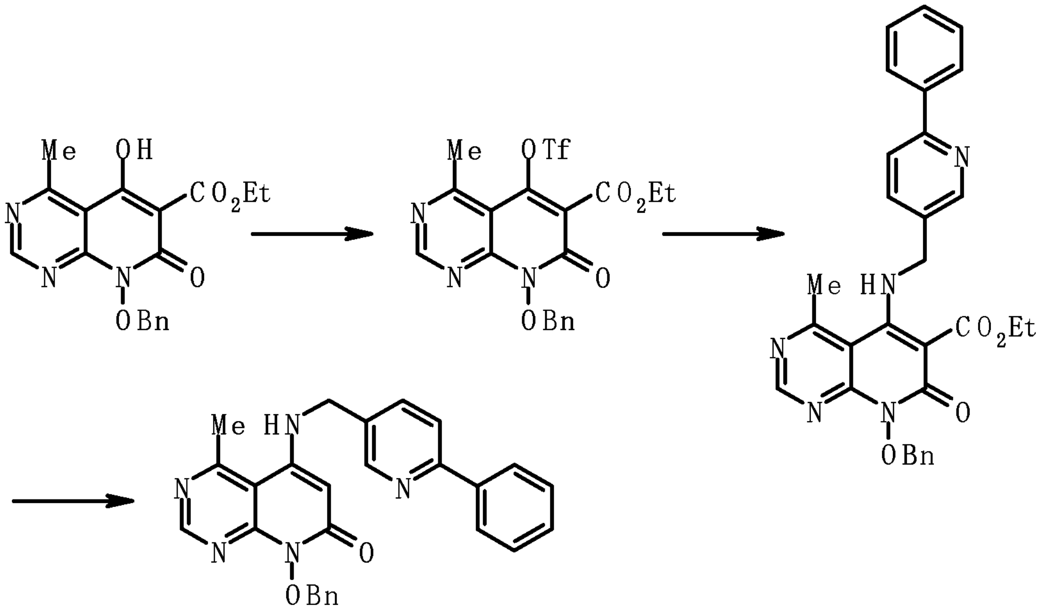

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 50 minutes. The solvent in the reaction mixture was distilled off under reduced pressure. To the obtained residue, 1-(6-phenylpyridin-3-yl)methanamine (156 mg), triethylamine (117 μL) and dioxane (7 mL) were added, and the mixture was stirred at room temperature for 1 hour and 10 minutes. The solvent in the reaction mixture was distilled off under reduced pressure. The obtained residue was purified by silica gel column chromatography [eluent: 100-90% chloroform/methanol] to obtain a pale yellow solid (174 mg). To the obtained pale yellow solid (174 mg), methanol (6 mL) and a 1 mol/L aqueous sodium hydroxide solution (6 mL) were added, and the mixture was heated with stirring at 50 to 60°C for 5 hours and 30 minutes. After cooling of the reaction mixture, the solvent was distilled off under reduced pressure, and ethyl acetate and water were added to the residue, followed by separation of an organic layer. The organic layer was washed with a saturated aqueous solution of sodium chloride and then dried over sodium sulfate, and the solvent was distilled off under reduced pressure. The obtained residue was purified by silica gel column chromatography [eluent: 100-90% chloroform/methanol] to obtain a yellow oil of 8-(benzyloxy)-4-methyl-5-(((6-phenylpyridin-3-yl)methyl)amino)pyrido[2,3-d]pyrimidin-7(8H)-one (90 mg).
$^1$H-NMR ($CDCl_3$) δ value: 2.96 (s, 3H), 4.46 (d, J=4.6 Hz, 2H), 5.26 (s, 2H), 5.30-5.36 (m, 1H), 5.79 (s, 1H), 7.34-7.42 (m, 3H), 7.42-7.54 (m, 3H), 7.64-7.74 (m, 2H), 7.75-7.82 (m, 2H), 7.96-8.04 (m, 2H), 8.72-8.77 (m, 1H), 8.91 (s, 1H).

Reference Example 139

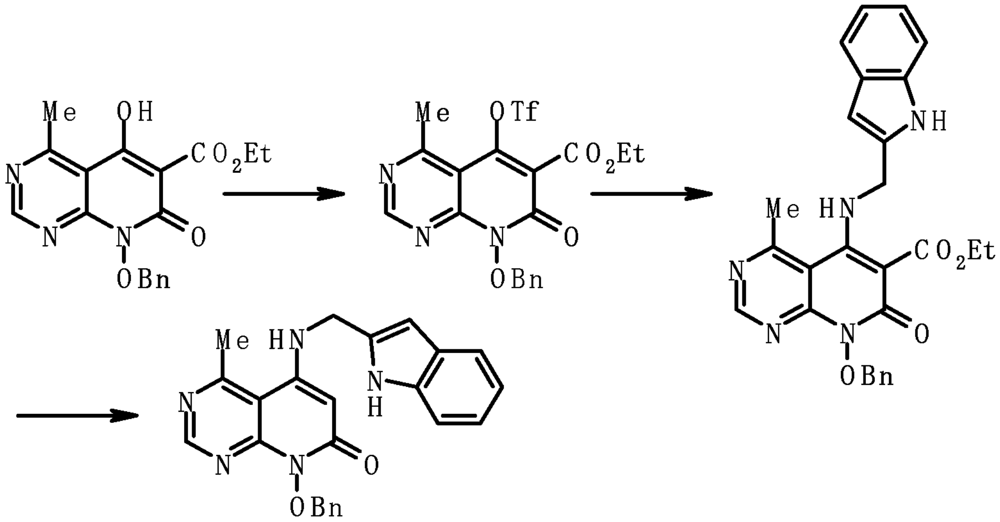

To a solution of ethyl 8-(benzyloxy)-5-hydroxy-4-methyl-7-oxo-7,8-dihydropyrido[2,3-d]pyrimidine-6-carboxylate (150 mg) and triethylamine (117 μL) in methylene chloride (5 mL), trifluoromethanesulfonic anhydride (138 μL) was added dropwise under ice cooling, and the mixture was stirred at room temperature for 1 hour. The solvent in the reaction mixture was distilled off under reduced pressure. To the obtained residue, 1-(1H-indol-2-yl)methanamine (123 mg), triethylamine (117 μL) and dioxane (6 mL) were added, and the mixture was stirred at room temperature for 4 hours. The solvent in the reaction mixture was distilled off under reduced pressure. The obtained residue was purified by silica gel column chromatography [eluent: 80-0% hexane/ethyl acetate] to obtain a brown solid (277 mg). To the obtained brown solid (277 mg), methanol (7 mL) and a 1 mol/L aqueous sodium hydroxide solution (7 mL) were added, and the mixture was heated with stirring at 50 to 60°C for 2 hours. After cooling of the reaction mixture, the solvent was distilled off under reduced pressure. The obtained residue was purified by silica gel column chromatography [eluent: 100-90% chloroform/methanol] and then suspended in a mixed solvent of 2-propanol and diisopropyl ether, and the deposit was collected by filtration to obtain a brown solid of 8-(benzyloxy)-5-(((1H-indol-2-yl)methyl)amino)-4-methylpyrido[2,3-d]pyrimidin-7(8H)-one (72 mg). $^1$H-NMR (DMSO-$d_6$) δ value: 3.06 (s, 3H), 4.63 (d, J=5.1 Hz, 2H), 5.06 (s, 2H), 5.63 (s, 1H), 6.40-6.48 (m, 1H), 6.84-6.92 (m, 1H), 6.92-7.01 (m, 1H), 7.01-7.08 (m, 1H), 7.32-7.51 (m, 5H), 7.57-7.63 (m, 2H), 8.90 (s, 1H), 11.06 (s, 1H).

Reference Example 140

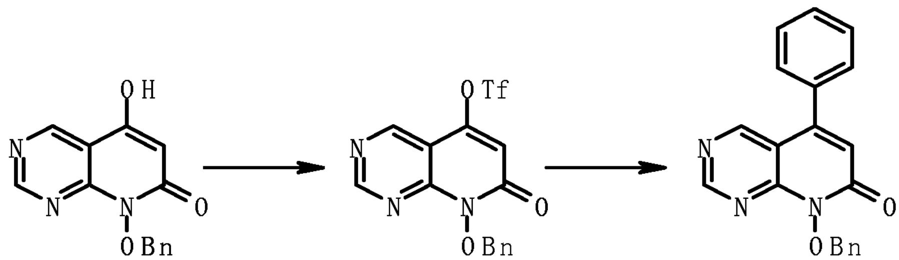

To a solution of 8-(benzyloxy)-5-hydroxypyrido[2,3-d]pyrimidin-7(8H)-one (100 mg) and triethylamine (104 μL) in methylene chloride (5.0 mL), trifluoromethanesulfonic anhydride (122 μL) was added dropwise under ice cooling, and the mixture was stirred at room temperature for 1 hour. To the reaction mixture, chloroform and water were added. An organic layer was separated and dried over magnesium sulfate, and the solvent was distilled off under reduced pressure. To the obtained residue, phenylboronic acid (54 mg), sodium carbonate (79 mg), tetrakis(triphenylphosphine)palladium(0) (21 mg) and dioxane (5.0 mL) were added, and the mixture was heated to reflux for 4 hours in a nitrogen atmosphere. After cooling of the reaction mixture, ethyl acetate and water were added thereto, and insoluble matter was filtered off, followed by separation of an organic layer. An aqueous layer was extracted with ethyl acetate, combined with the organic layer, washed with a saturated aqueous solution of sodium chloride and then dried over magnesium sulfate, and the solvent was distilled off under reduced pressure. The obtained residue was purified by silica gel column chromatography

[Kanto Chemical Co., Inc., silica gel 60 (spherical), eluent: 90-50% hexane/ethyl acetate] and then suspended in diisopropyl ether, and the deposit was collected by filtration to obtain a yellow solid of 8-(benzyloxy)-5-phenylpyrido[2,3-d]pyrimidin-7(8H)-one (46 mg).
$^1$H-NMR ($CDCl_3$) δ value: 5.37 (s, 2H), 6.80 (s, 1H), 7.38-7.50 (m, 5H), 7.54-7.59 (m, 3H), 7.69-7.75 (m, 2H), 8.87 (s, 1H), 9.16 (s, 1H).

Reference Example 141

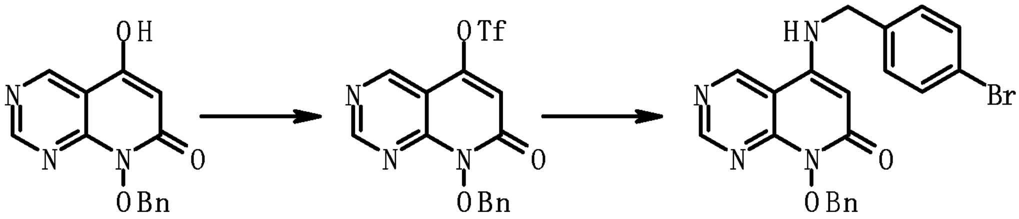

To a solution of 8-(benzyloxy)-5-hydroxypyrido[2,3-d]pyrimidin-7(8H)-one (200 mg) and triethylamine (207 μL) in methylene chloride (10 mL), trifluoromethanesulfonic anhydride (244 μL) was added dropwise under ice cooling, and the mixture was stirred at room temperature for 1 hour. The solvent in the reaction mixture was distilled off under reduced pressure. To the obtained residue, dioxane (10 mL) was added, and the mixture was added dropwise to a suspension of 4-bromobenzylamine hydrochloride (826 mg) and N-ethyldiisopropylamine (647 μL) in dioxane (10 mL) under ice cooling. The reaction mixture was heated with stirring at 90 to 95°C for 2 hours. After cooling of the reaction mixture, ethyl acetate and water were added thereto. An organic layer was separated, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 80-20% hexane/ethyl acetate] and then suspended in 2-propanol, and the deposit was collected by filtration to obtain a brown solid of 8-(benzyloxy)-5-((4-bromobenzyl)amino)pyrido[2,3-d]pyrimidin-7(8H)-one (32 mg).
$^1$H-NMR ($CDCl_3$) δ value: 4.41 (d, J=5.1 Hz, 2H), 5.11-5.19 (m, 1H), 5.26 (s, 2H), 5.72 (s, 1H), 7.22-7.30 (m, 2H), 7.34-7.42 (m, 3H), 7.51-7.56 (m, 2H), 7.63-7.69 (m, 2H), 8.84 (s, 1H), 9.10 (s, 1H).

Reference Example 142

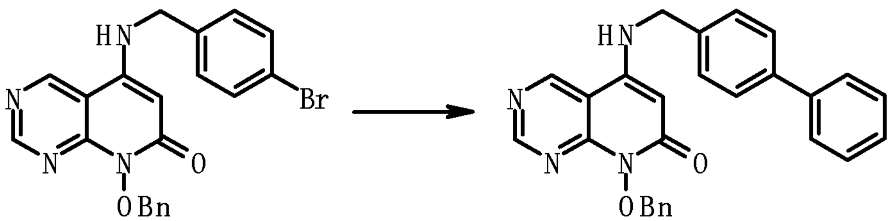

To a suspension of 8-(benzyloxy)-5-((4-bromobenzyl)amino)pyrido[2,3-d]pyrimidin-7(8H)-one (30 mg) in ethylene glycol dimethyl ether (3.0 mL), water (0.90 mL), phenylboronic acid (10 mg), sodium carbonate (15 mg) and bis(triphenylphosphine)palladium(II) dichloride (2.4 mg) were added, and the mixture was heated to reflux for 1 hour and 30 minutes in a nitrogen atmosphere. After cooling of the reaction mixture, ethyl acetate and water were added thereto, and insoluble matter was filtered off. An organic layer was separated, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 80-0% hexane/ethyl acetate] and then suspended in diisopropyl ether, and the deposit was collected by filtration to obtain a yellow solid of 8-(benzyloxy)-5-(((biphenyl-4-yl)methyl)amino)pyrido[2,3-d]pyrimidin-7(8H)-one (17 mg).
$^1$H-NMR ($CDCl_3$) δ value: 4.48 (d, J=5.2 Hz, 2H), 5.14-5.20 (m, 1H), 5.28 (s, 2H), 5.81 (s, 1H), 7.34-7.42 (m, 4H), 7.42-7.50 (m, 4H), 7.57-7.71 (m, 6H), 8.86 (s, 1H), 9.10 (s, 1H).

Reference Example 143

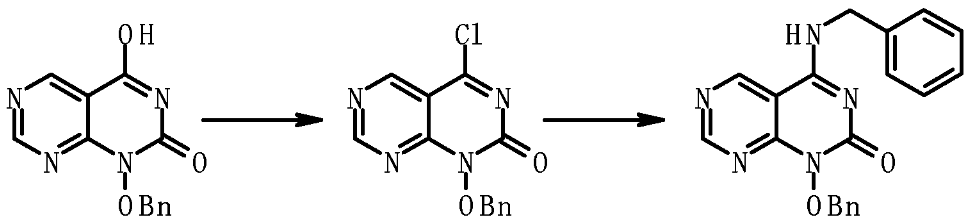

To a suspension of 1-(benzyloxy)-4-hydroxypyrimido[4,5-d]pyrimidin-2(1H)-one (75 mg) in N-ethyldiisopropylamine (0.48 mL), phosphoryl chloride (0.26 mL) was added, and the mixture was heated with stirring at 65°C for 5 minutes and then heated with stirring at 80°C for 5 minutes. After cooling of the reaction mixture, the solvent was distilled off under reduced pressure. To the obtained residue, benzylamine (0.12 mL) and methylene chloride (1.5 mL) were added, and the mixture was stirred at room temperature for 1 hour and 30 minutes. To the reaction mixture, chloroform and water were added. An organic layer was separated, washed with a 10% aqueous citric acid solution and then dried over sodium sulfate, and the solvent was distilled off under reduced pressure. The obtained residue was purified by silica gel column chromatography [Kanto Chemical Co., Inc., silica gel 60 (spherical), eluent: 80-0% hexane/ethyl acetate] and then suspended in a mixed solvent of ethyl acetate and diisopropyl ether, and the deposit was collected by filtration to obtain a yellow solid of 4-(benzylamino)-1-(benzyloxy)pyrimido[4,5-d]pyrimidin-2(1H)-one (39 mg).
$^1$H-NMR ($CDCl_3$) $\delta$ value: 4.85 (d, J=5.4 Hz, 2H), 5.29 (s, 2H), 6.25-6.32 (m, 1H), 7.32-7.43 (m, 8H), 7.62-7.69 (m, 2H), 8.82 (s, 1H), 9.10 (s, 1H).

Reference Example 144

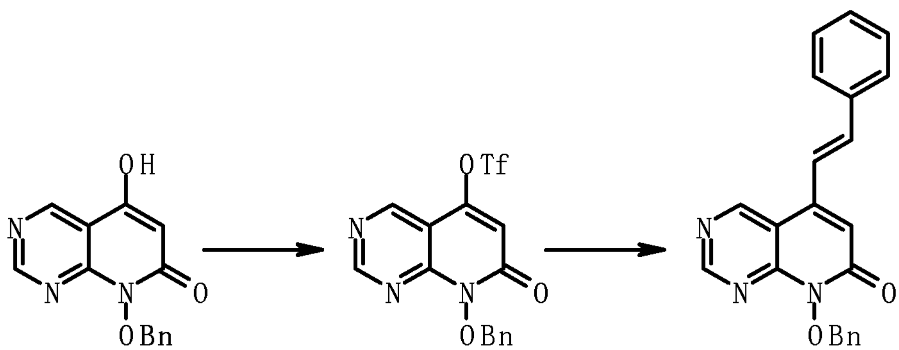

To a solution of 8-(benzyloxy)-5-hydroxypyrido[2,3-d]pyrimidin-7(8H)-one (100 mg) and triethylamine (104 $\mu$L) in methylene chloride (5.0 mL), trifluoromethanesulfonic anhydride (122 $\mu$L) was added dropwise under ice cooling, and the mixture was stirred at room temperature for 1 hour. To the reaction mixture, chloroform and water were added. An organic layer was separated and dried over magnesium sulfate, and the solvent was distilled off under reduced pressure. To the obtained residue, ((E)-2-phenylvinyl)boronic acid (66 mg), sodium carbonate (79 mg), tetrakis(triphenylphosphine)palladium(0) (21 mg) and dioxane (5.0 mL) were added, and the mixture was heated to reflux for 4 hours in a nitrogen atmosphere. After cooling of the reaction mixture, ethyl acetate and water were added thereto, followed by separation of an organic layer. An aqueous layer was extracted with ethyl acetate, combined with the organic layer, washed with a saturated aqueous solution of sodium chloride and then dried over magnesium sulfate, and the solvent was distilled off under reduced pressure. The obtained residue was purified by silica gel column chromatography [Kanto Chemical Co., Inc., silica gel 60 (spherical), eluent: 90-50% hexane/ethyl acetate] and then suspended in a mixed solvent of ethyl acetate and diisopropyl ether, and the deposit was collected by filtration to obtain a pale yellow solid of 8-(benzyloxy)-5-((E)-2-phenylvinyl)pyrido[2,3-d]pyrimidin-7(8H)-one (36 mg).
$^1$H-NMR ($CDCl_3$) $\delta$ value: 5.34 (s, 2H), 7.00 (s, 1H), 7.30-7.49 (m, 8H), 7.57-7.62 (m, 2H), 7.66-7.72 (m, 2H), 9.17 (s, 1H), 9.19 (s, 1H).

Reference Example 145

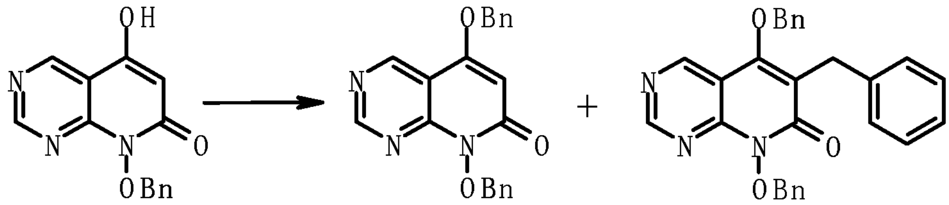

To a solution of 8-(benzyloxy)-5-hydroxypyrido[2,3-d]pyrimidin-7(8H)-one (500 mg) in DMF (7.5 mL), potassium carbonate (308 mg) and benzyl bromide (243 μL) were added, and the mixture was stirred at room temperature for 4 hours and 30 minutes. To the reaction mixture, ethyl acetate, diisopropyl ether and water were added, and the deposit was collected by filtration to obtain a pale red solid of 5,8-bis(benzyloxy)pyrido[2,3-d]pyrimidin-7(8H)-one (326 mg). An organic layer was separated from the filtrate, washed twice with a 10% aqueous citric acid solution, then washed with a saturated aqueous solution of sodium chloride and dried over magnesium sulfate, and the solvent was distilled off under reduced pressure. The obtained residue was purified by silica gel column chromatography [Kanto Chemical Co., Inc., silica gel 60 (spherical), eluent: 90-50% hexane/ethyl acetate] and then suspended in diisopropyl ether, and the deposit was collected by filtration to obtain a white solid of 6-benzyl-5,8-bis(benzyloxy)pyrido[2,3-d]pyrimidin-7(8H)-one (63 mg).
5,8-Bis(benzyloxy)pyrido[2,3-d]pyrimidin-7(8H)-one
$^1$H-NMR ($CDCl_3$) δ value: 5.20 (s, 2H), 5.28 (s, 2H), 6.16 (s, 1H), 7.34-7.48 (m, 8H), 7.63-7.69 (m, 2H), 9.14 (s, 1H), 9.17 (s, 1H).
6-Benzyl-5,8-bis(benzyloxy)pyrido[2,3-d]pyrimidin-7(8H)-one
$^1$H-NMR ($CDCl_3$) δ value: 4.05 (s, 2H), 5.04 (s, 2H), 5.32 (s, 2H), 7.16-7.46 (m, 13H), 7.60-7.67 (m, 2H), 8.96 (s, 1H), 9.10 (s, 1H).

Reference Example 146

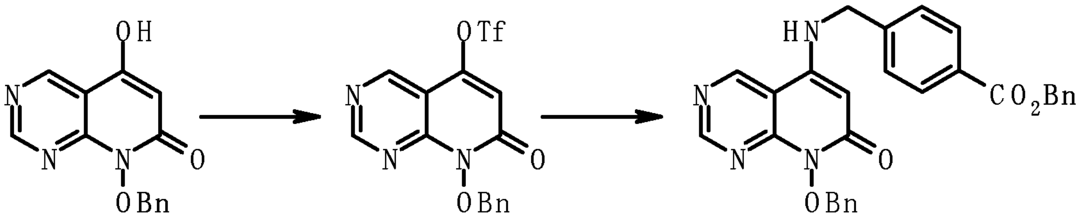

To a solution of 8-(benzyloxy)-5-hydroxypyrido[2,3-d]pyrimidin-7(8H)-one (100 mg) and triethylamine (104 μL) in methylene chloride (5.0 mL), trifluoromethanesulfonic anhydride (122 μL) was added dropwise under ice cooling, and the mixture was stirred at room temperature for 1 hour. The solvent in the reaction mixture was distilled off under reduced pressure. To the obtained residue, dioxane (2.5 mL) was added, and the mixture was added to a suspension of benzyl 4-(aminomethyl)benzoate hydrochloride (516 mg) and N-ethyldiisopropylamine (388 μL) in dioxane (2.5 mL). The reaction mixture was heated with stirring at 70 to 75°C for 1 hour and 30 minutes. After cooling of the reaction mixture, insoluble matter was filtered off and washed with ethyl acetate. The filtrate and the washes were combined, and the solvent was distilled off under reduced pressure. The obtained residue was purified by silica gel column chromatography [Kanto Chemical Co., Inc., silica gel 60 (spherical), eluent: 80-20% hexane/ethyl acetate] and then suspended in a mixed solvent of 2-propanol and diisopropyl ether, and the deposit was collected by filtration to obtain a pale yellow solid of benzyl 4-(((8-(benzyloxy)-7-oxo-7,8-dihydropyrido[2,3-d]pyrimidin-5-yl)amino)methyl)benzoate (12 mg).
$^1$H-NMR ($CDCl_3$) δ value: 4.52 (d, J=4.9 Hz, 2H), 5.18-5.25 (m, 1H), 5.26 (s, 2H), 5.38 (s, 2H), 5.70 (s, 1H), 7.32-7.48 (m, 10H), 7.63-7.68 (m, 2H), 8.08-8.14 (m, 2H), 8.86 (s, 1H), 9.10 (s, 1H).

Reference Example 147

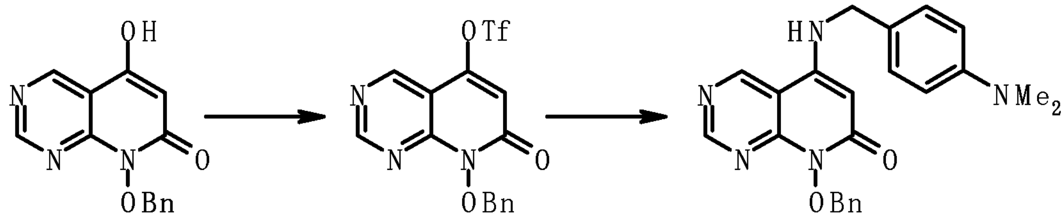

To a solution of 8-(benzyloxy)-5-hydroxypyrido[2,3-d]pyrimidin-7(8H)-one (100 mg) and triethylamine (104 μL) in methylene chloride (5.0 mL), trifluoromethanesulfonic anhydride (122 μL) was added dropwise under ice cooling, and the mixture was stirred at room temperature for 1 hour. The solvent in the reaction mixture was distilled off under reduced pressure. To the obtained residue, 4-(dimethylamino)benzylamine (139 mg) and dioxane (5.0 mL) were added, and the mixture was heated with stirring at 80 to 90°C for 1 hour in a nitrogen atmosphere. After cooling of the reaction mixture, the solvent was distilled off under reduced pressure. The obtained residue was purified by silica gel column chromatography [Kanto Chemical Co., Inc., silica gel 60 (spherical), eluent: 80-0% hexane/ethyl acetate], then purified by silica gel column chromatography [Kanto Chemical Co., Inc., silica gel 60 (spherical), eluent: 100-92% chloroform/methanol], and suspended in a mixed solvent of ethyl acetate, 2-propanol and diisopropyl ether, and the deposit was collected by filtration to obtain an orange solid of 8-(benzyloxy)-5-((4-(dimethylamino)benzyl)amino)pyrido[2,3-d]pyrimidin-7(8H)-one (26 mg). $^1$H-NMR ($CDCl_3$) δ value: 2.98 (s, 6H), 4.28 (d, J=4.6 Hz, 2H), 4.93-4.99 (m, 1H), 5.27 (s, 2H), 5.81 (s, 1H), 6.72-6.77 (m, 2H), 7.22-7.30 (m, 2H), 7.34-7.42 (m, 3H), 7.64-7.71 (m, 2H), 8.77 (s, 1H), 9.07 (s, 1H).

Reference Example 148

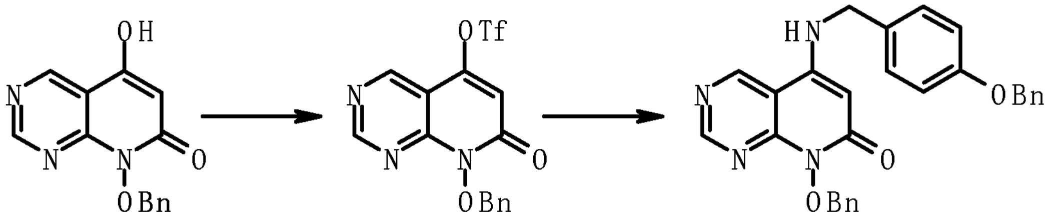

To a solution of 8-(benzyloxy)-5-hydroxypyrido[2,3-d]pyrimidin-7(8H)-one (100 mg) and triethylamine (104 μL) in methylene chloride (5.0 mL), trifluoromethanesulfonic anhydride (122 μL) was added dropwise under ice cooling, and the mixture was stirred at room temperature for 1 hour. The solvent in the reaction mixture was distilled off under reduced pressure. To the obtained residue, 1-(4-(benzyloxy)phenyl)methanamine (309 mg) and dioxane (5.0 mL) were added, and the mixture was heated with stirring at 80 to 90°C for 1 hour in a nitrogen atmosphere. After cooling of the reaction mixture, the solvent was distilled off under reduced pressure. The obtained residue was purified by silica gel column chromatography [Kanto Chemical Co., Inc., silica gel 60 (spherical), eluent: 80-15% hexane/ethyl acetate] and then suspended in 2-propanol, and the deposit was collected by filtration to obtain an orange solid of 8-(benzyloxy)-5-((4-(benzyloxy)benzyl)amino)pyrido[2,3-d]pyrimidin-7(8H)-one (30 mg).
$^1$H-NMR ($CDCl_3$) δ value: 4.34 (d, J=4.9 Hz, 2H), 5.01-5.08 (m, 1H), 5.09 (s, 2H), 5.27 (s, 2H), 5.78 (s, 1H), 6.98-7.03 (m, 2H), 7.25-7.47 (m, 10H), 7.64-7.70 (m, 2H), 8.80 (s, 1H), 9.08 (s, 1H).

Reference Example 149

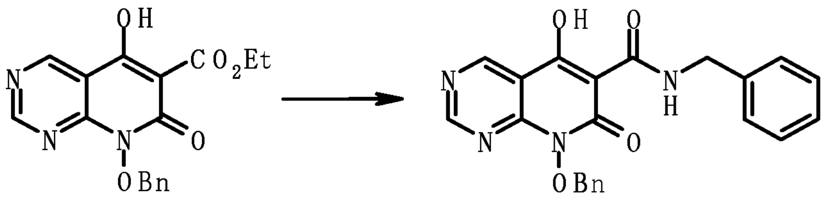

To ethyl 8-(benzyloxy)-5-hydroxy-7-oxo-7,8-dihydropyrido[2,3-d]pyrimidine-6-carboxylate (60 mg), benzylamine (0.60 mL) was added, and the mixture was heated with stirring at 75 to 80°C for 15 minutes. After cooling of the reaction mixture, ethyl acetate and a 10% aqueous citric acid solution were added thereto, and the deposit was collected by filtration. To the obtained solid, chloroform and a 10% aqueous citric acid solution were added. An organic layer was separated and dried over magnesium sulfate, and the solvent was distilled off under reduced pressure. The obtained residue was suspended in 2-propanol, and the deposit was collected by filtration to obtain a white solid of N-benzyl-8-(benzyloxy)-5-hydroxy-7-oxo-7,8-dihydropyrido[2,3-d]pyrimidine-6-carboxamide (57 mg).
$^1$H-NMR ($CDCl_3$) δ value: 4.67 (d, J=6.1 Hz, 2H), 5.26 (s, 2H), 7.29-7.44 (m, 8H), 7.61-7.67 (m, 2H), 9.23 (s, 1H), 9.40 (s, 1H), 10.04-10.14 (m, 1H).

Reference Example 150

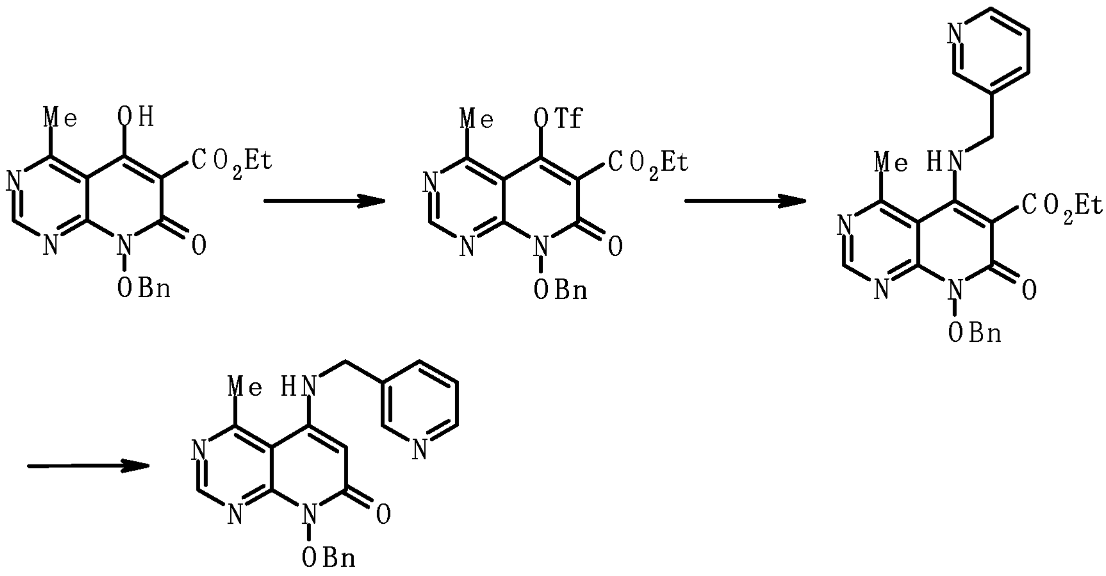

To a solution of ethyl 8-(benzyloxy)-5-hydroxy-4-methyl-7-oxo-7,8-dihydropyrido[2,3-d]pyrimidine-6-carboxylate (120 mg) and triethylamine (94 μL) in methylene chloride (6.0 mL), trifluoromethanesulfonic anhydride (111 μL) was added dropwise under ice cooling, and the mixture was stirred at room temperature for 1 hour. The solvent in the reaction mixture was distilled off under reduced pressure. To the obtained residue, 3-picolylamine (171 μL) and dioxane (6.0 mL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 98-95% chloroform/methanol] and then suspended in a mixed solvent of ethyl acetate, 2-propanol and diisopropyl ether, and the deposit was collected by filtration to obtain an orange solid (92 mg). To the obtained orange solid (90 mg), methanol (4.5 mL) and a 1 mol/L aqueous sodium hydroxide solution (4.5 mL) were added, and the mixture was heated with stirring at 60 to 70°C for 2 hours and then heated with stirring at 70 to 80°C for 1 hour and 30 minutes. After cooling of the reaction mixture, the solvent was distilled off under reduced pressure. To the obtained residue, a saturated aqueous solution of sodium bicarbonate and ethyl acetate were added, followed by separation of an organic layer. An aqueous layer was extracted with ethyl acetate, combined with the organic layer and dried over sodium sulfate, and the solvent was distilled off under reduced pressure. The obtained residue was purified by silica gel column chromatography [Kanto Chemical Co., Inc., silica gel 60 (spherical), eluent: 100-90% chloroform/methanol] and then suspended in a mixed solvent of 2-propanol and diisopropyl ether, and the deposit was collected by filtration to obtain a yellow solid of 8-(benzyloxy)-4-methyl-5-(((pyridin-3-yl)methyl)amino)pyrido[2,3-d]pyrimidin-7(8H)-one (18 mg).
$^1$H-NMR ($CDCl_3$) δ value: 2.95 (s, 3H), 4.44 (d, J=4.9 Hz, 2H), 5.25 (s, 2H), 5.28-5.35 (m, 1H), 5.74 (s, 1H), 7.33-7.42 (m, 4H), 7.65-7.74 (m, 3H), 8.64 (dd, J=4.8, 1.6 Hz, 1H), 8.68 (d, J=2.0 Hz, 1H), 8.91 (s, 1H).

Reference Example 151

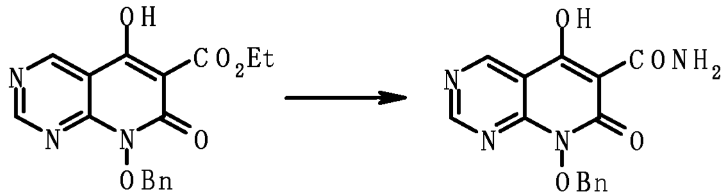

To ethyl 8-(benzyloxy)-5-hydroxy-7-oxo-7,8-dihydropyrido[2,3-d]pyrimidine-6-carboxylate (210 mg), DMF (1.1 mL) and a 25% aqueous ammonia solution (1.1 mL) were added, and the mixture was heated with stirring at 140°C for 1 hour in a sealed tube. After cooling of the reaction mixture, ethyl acetate and 2 mol/L hydrochloric acid were added thereto, followed by separation of an organic layer. An aqueous layer was extracted with ethyl acetate, combined with the organic layer, washed with water and a saturated aqueous solution of sodium chloride in this order and then dried over magnesium sulfate, and the solvent was distilled off under reduced pressure. The obtained residue was purified by silica gel column chromatography [Kanto Chemical Co., Inc., silica gel 60 (spherical), eluent: 100-98% chloroform/methanol] and then suspended in diisopropyl ether, and the deposit was collected by filtration to obtain a yellow solid of 8-(benzyloxy)-5-hydroxy-7-oxo-7,8-dihydropyrido[2,3-d]pyrimidine-6-carboxamide (66 mg).
$^1$H-NMR (DMSO-$d_6$) δ value: 5.20 (s, 2H), 7.39-7.49 (m, 3H), 7.63-7.69 (m, 2H), 9.07 (brs, 1H), 9.25 (brs, 1H), 9.30 (s, 1H), 9.32 (s, 1H).

Reference Example 152

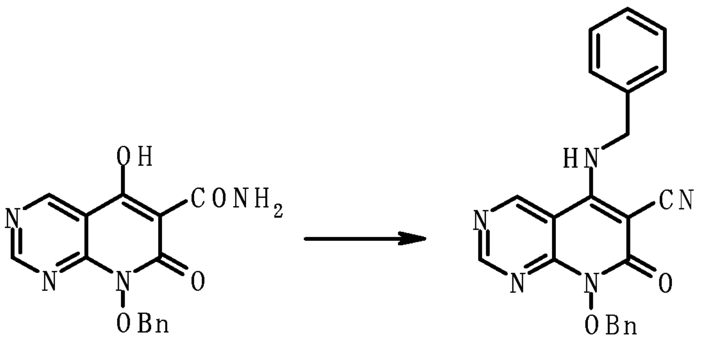

To a solution of 8-(benzyloxy)-5-hydroxy-7-oxo-7,8-dihydropyrido[2,3-d]pyrimidine-6-carboxamide (35 mg) and triethylamine (31 μL) in methylene chloride (1.8 mL), trifluoromethanesulfonic anhydride (37 μL) was added dropwise under ice cooling, and the mixture was stirred at room temperature for 1 hour. The solvent in the reaction mixture was distilled off under reduced pressure. To the obtained residue, benzylamine (61 μL) and methylene chloride (1.8 mL) were added, and the mixture was stirred at room temperature for 1 hour and 30 minutes. The reaction mixture was purified by silica gel column chromatography [Kanto Chemical Co., Inc., silica gel 60 (spherical), eluent: 100-90% chloroform/methanol], then purified by silica gel column chromatography [Kanto Chemical Co., Inc., silica gel 60 (spherical), eluent: 80-20% hexane/ethyl acetate], and suspended in diisopropyl ether, and the deposit was collected by filtration to obtain a pale yellow solid of 5-(benzylamino)-8-(benzyloxy)-7-oxo-7,8-dihydropyrido[2,3-d]pyrimidine-6-carbonitrile (11 mg).
$^1$H-NMR ($CDCl_3$) δ value: 5.12 (d, J=4.9 Hz, 2H), 5.27 (s, 2H), 5.72-5.79 (m, 1H), 7.36-7.49 (m, 8H), 7.62-7.69 (m, 2H), 8.89 (s, 1H), 9.13 (s, 1H).

Reference Example 153

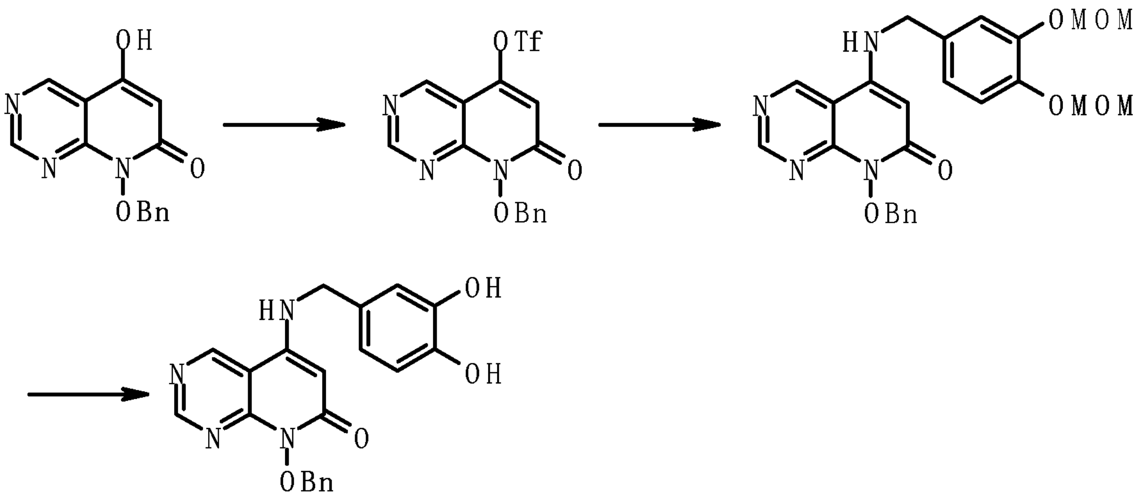

To a solution of 8-(benzyloxy)-5-hydroxypyrido[2,3-d]pyrimidin-7(8H)-one (40 mg) and triethylamine (41 μL) in methylene chloride (2.0 mL), trifluoromethanesulfonic anhydride (49 μL) was added dropwise under ice cooling, and the mixture was stirred at room temperature for 1 hour. The solvent in the reaction mixture was distilled off under reduced pressure. To the obtained residue, 1-(3,4-bis(methoxymethoxy)phenyl)methanamine (135 mg) and dioxane (2.0 mL) were added, and the mixture was heated with stirring at 80 to 90°C for 1 hour in a nitrogen atmosphere. After cooling of the reaction mixture, the solvent was distilled off under reduced pressure. The obtained residue was purified by silica gel column chromatography [Kanto Chemical Co., Inc., silica gel 60 (spherical), eluent: 80-0% hexane/ethyl acetate], and ethyl acetate and a 10% aqueous citric acid solution were then added thereto. An organic layer was separated, washed with a 10% aqueous citric acid solution, water and a saturated aqueous solution of sodium chloride in this order and then dried over sodium sulfate, and the solvent was distilled off under reduced pressure to obtain a brown oil (23 mg). To a solution of the obtained brown oil (23 mg) in methanol (1.0 mL), methanesulfonic acid (16 μL) was added, and the mixture was stirred at room temperature for 2 hours and 30 minutes. The deposit was collected by filtration, and ethyl acetate and a saturated aqueous solution of sodium bicarbonate were added to the obtained solid. An organic layer was separated, washed with a saturated aqueous solution of sodium chloride and then dried over sodium sulfate, and the solvent was distilled off under reduced pressure to obtain a pale yellow solid of 8-(benzyloxy)-5-((3,4-dihydroxybenzyl)amino)pyrido[2,3-d]pyrimidin-7(8H)-one (5 mg).
$^1$H-NMR ($CD_3OD$) δ value: 4.37 (s, 2H), 5.19 (s, 2H), 5.59 (s, 1H), 6.68-6.74 (m, 1H), 6.75 (d, J=8.1 Hz, 1H), 6.81 (d, J=1.7 Hz, 1H), 7.33-7.39 (m, 3H), 7.59-7.64 (m, 2H), 9.06 (s, 1H), 9.32 (s, 1H).

Reference Example 154

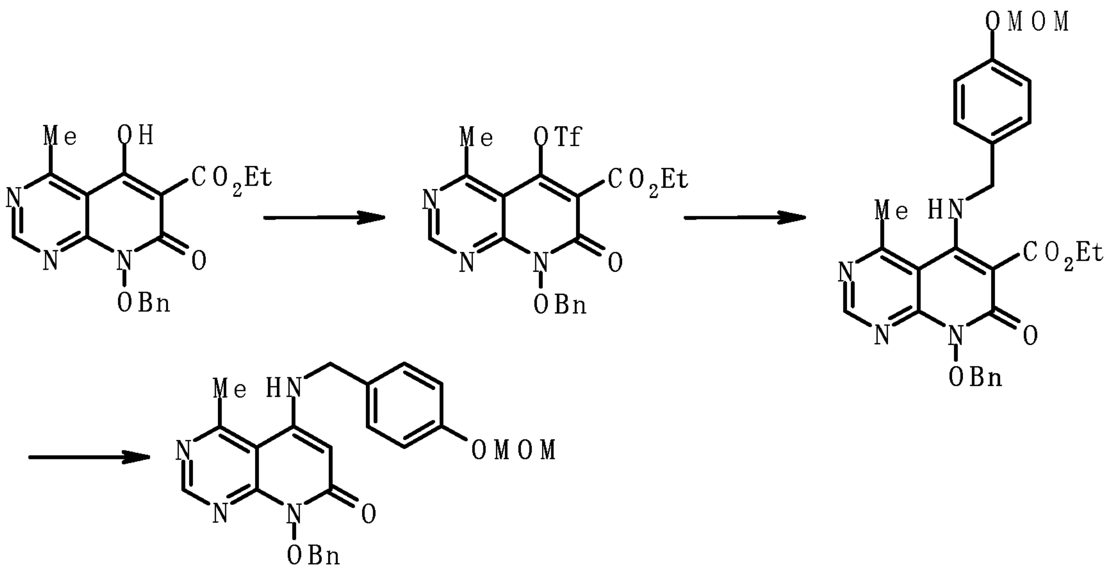

To ethyl 8-(benzyloxy)-5-hydroxy-4-methyl-7-oxo-7,8-dihydropyrido[2,3-d]pyrimidine-6-carboxylate (100 mg), triethylamine (78 μL) and methylene chloride (5.0 mL) were added, then trifluoromethanesulfonic anhydride (92 μL) was added dropwise under ice cooling, and the mixture was stirred at room temperature for 1 hour. The solvent in the reaction mixture was distilled off under reduced pressure. To the obtained residue, 1-(4-(methoxymethoxy)phenyl)methanamine (188 mg) and dioxane (5.0 mL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-40% hexane/ethyl acetate] to obtain a brown oil (73 mg). To the obtained brown oil (72 mg), methanol (3.5 mL) and a 1 mol/L aqueous sodium hydroxide solution (3.5 mL) were added, and the mixture was heated with stirring at 70 to 80°C for 30 minutes in a nitrogen atmosphere. After cooling of the reaction mixture, a saturated aqueous solution of sodium bicarbonate and ethyl acetate were added thereto, followed by separation of an organic layer. An aqueous layer was extracted with ethyl acetate, combined with the organic layer and dried over sodium sulfate, and the solvent was distilled off under reduced pressure. The obtained residue was purified by silica gel column chromatography [Kanto Chemical Co., Inc., silica gel 60 (spherical), eluent: 80-20% hexane/ethyl acetate] and then suspended in a mixed solvent of 2-propanol and diisopropyl ether, and the deposit was collected by filtration to obtain a pale yellow solid of 8-(benzyloxy)-5-((4-(methoxymethoxy)benzyl)amino)-4-methylpyrido[2,3-d]pyrimidin-7(8H)-one (32 mg).
$^1$H-NMR ($CDCl_3$) δ value: 2.91 (s, 3H), 3.50 (s, 3H), 4.30 (d, J=4.6 Hz, 2H), 5.20 (s, 2H), 5.23-5.28 (m, 3H), 5.76 (s, 1H), 7.05-7.11 (m, 2H), 7.24-7.42 (m, 5H), 7.66-7.71 (m, 2H), 8.89 (s, 1H).

Reference Example 155

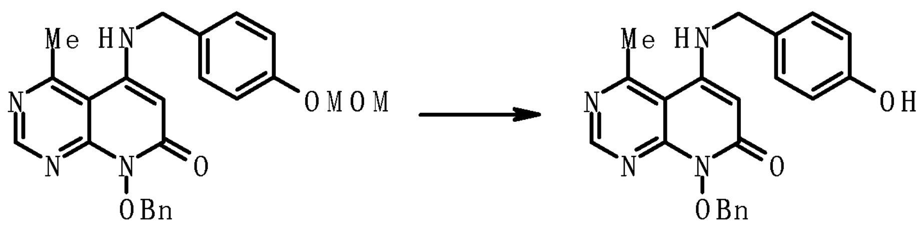

To a solution of 8-(benzyloxy)-5-((4-(methoxymethoxy)benzyl)amino)-4-methylpyrido[2,3-d]pyrimidin-7(8H)-one (31 mg) in methanol (3.0 mL) and dioxane (1.0 mL), methanesulfonic acid (14 μL) was added, and the mixture was stirred at room temperature for 1 hour. To the reaction mixture, methanesulfonic acid (9 μL) was added, and the mixture was stirred at room temperature for 1 hour. To the reaction mixture, methanesulfonic acid (23 μL) was added, and the mixture was stirred at room temperature for 1 hour and 30 minutes. To the reaction mixture, methanesulfonic acid (23 μL) was added, and the mixture was stirred at room temperature for 30 minutes. To the reaction mixture, ethyl acetate and a saturated aqueous solution of sodium bicarbonate were added. An organic layer was separated, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 50-10% hexane/ethyl acetate] and then suspended in a mixed solvent of ethyl acetate and diisopropyl ether, and the deposit was collected by filtration to obtain a white solid of 8-(benzyloxy)-5-((4-hydroxybenzyl)amino)-4-methylpyrido[2,3-d]pyrimidin-7(8H)-one (21 mg).
$^1$H-NMR ($CD_3OD$) δ value: 3.01 (s, 3H), 4.40 (s, 2H), 5.18 (s, 2H), 5.65 (s, 1H), 6.77-6.84 (m, 2H), 7.22-7.29 (m, 2H), 7.33-7.40 (m, 3H), 7.58-7.66 (m, 2H), 8.84 (s, 1H).

Reference Example 156

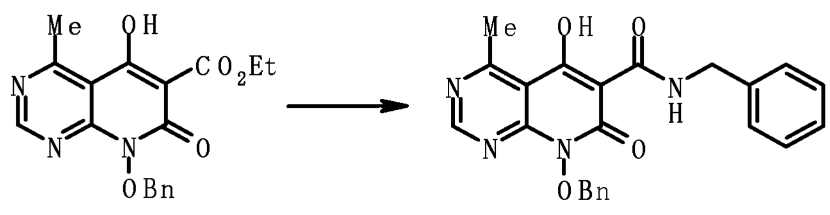

To ethyl 8-(benzyloxy)-5-hydroxy-4-methyl-7-oxo-7,8-dihydropyrido[2,3-d]pyrimidine-6-carboxylate (30 mg), benzylamine (0.30 mL) was added, and the mixture was heated with stirring at 75 to 80°C for 15 minutes. After cooling of the reaction mixture, chloroform and 1 mol/L hydrochloric acid were added thereto. An organic layer was separated, washed twice with 1 mol/L hydrochloric acid, then washed with water and dried over magnesium sulfate, and the solvent was distilled off under reduced pressure. The obtained residue was suspended in 2-propanol, and the deposit was collected by filtration to obtain a pale yellow solid of N-benzyl-8-(benzyloxy)-5-hydroxy-4-methyl-7-oxo-7,8-dihydropyrido[2,3-d]pyrimidine-6-carboxamide (27 mg).
$^1$H-NMR ($CDCl_3$) δ value: 3.05 (s, 3H), 4.67 (d, J=5.9 Hz, 2H), 5.24 (s, 2H), 7.24-7.46 (m, 8H), 7.62-7.68 (m, 2H), 9.02 (s, 1H), 10.26-10.36 (m, 1H).

Reference Example 157

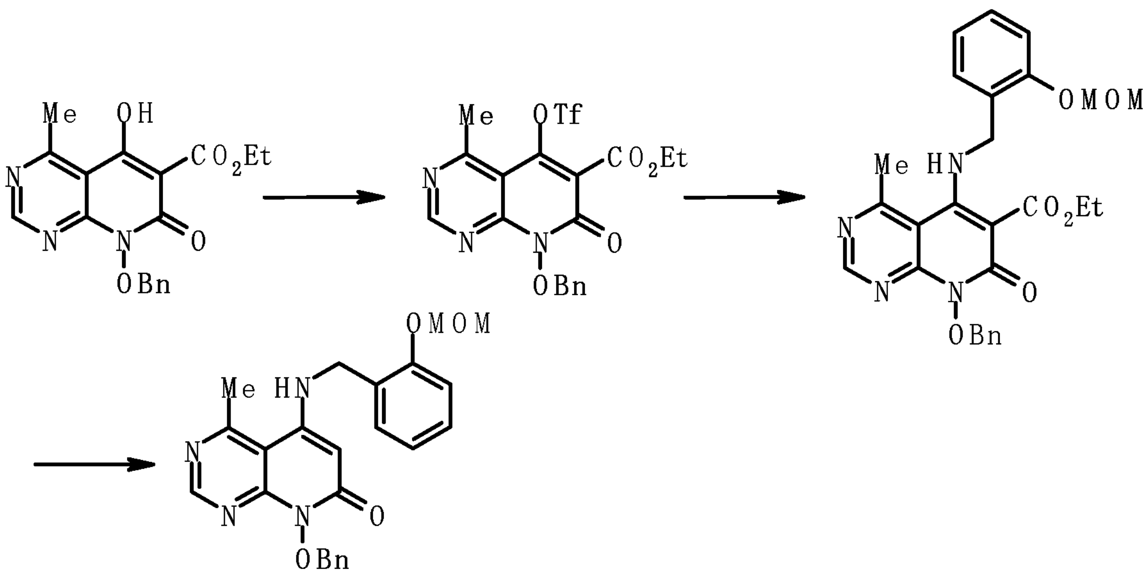

To ethyl 8-(benzyloxy)-5-hydroxy-4-methyl-7-oxo-7,8-dihydropyrido[2,3-d]pyrimidine-6-carboxylate (100 mg), triethylamine (78 μL) and methylene chloride (5.0 mL) were added, then trifluoromethanesulfonic anhydride (92 μL) was added dropwise under ice cooling, and the mixture was stirred at room temperature for 1 hour. The solvent in the reaction mixture was distilled off under reduced pressure. To the obtained residue, 1-(2-(methoxymethoxy)phenyl)methanamine (188 mg) and dioxane (5.0 mL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-40% hexane/ethyl acetate] to obtain an orange oil (65 mg). To the obtained orange oil (64 mg), methanol (3.2 mL) and a 1 mol/L aqueous sodium hydroxide solution (3.2 mL) were added, and the mixture was heated with stirring at 70 to 80°C for 30 minutes in a nitrogen atmosphere. After cooling of the reaction mixture, a saturated aqueous solution of sodium bicarbonate and ethyl acetate were added thereto, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 80-20% hexane/ethyl acetate] and then suspended in a mixed solvent of 2-propanol and diisopropyl ether, and the deposit was collected by filtration to obtain a pale yellow solid of 8-(benzyloxy)-5-((2-(methoxymethoxy)benzyl)amino)-4-methylpyrido[2,3-d]pyrimidin-7(8H)-one (21 mg). $^1$H-NMR ($CDCl_3$) δ value: 2.93 (s, 3H), 3.49 (s, 3H), 4.40 (d, J=4.9 Hz, 2H), 5.24 (s, 2H), 5.27 (s, 2H), 5.54-5.62 (m, 1H), 5.81 (s, 1H), 7.00-7.07 (m, 1H), 7.15-7.21 (m, 1H), 7.23-7.42 (m, 5H), 7.65-7.71 (m, 2H), 8.88 (s, 1H).

Reference Example 158

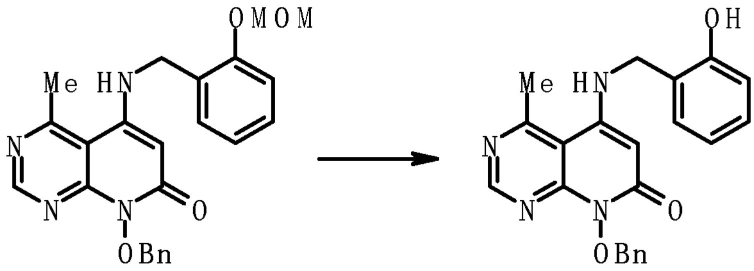

To a solution of 8-(benzyloxy)-5-((2-(methoxymethoxy)benzyl)amino)-4-methylpyrido[2,3-d]pyrimidin-7(8H)-one (20 mg) in methanol (2.0 mL) and dioxane (1.0 mL), methanesulfonic acid (15 μL) was added, and the mixture was stirred at room temperature for 1 hour. To the reaction mixture, methanesulfonic acid (60 μL) was added, and the mixture was stirred at room temperature for 2 hours. To the reaction mixture, methanesulfonic acid (45 μL) was added, and the mixture was stirred at room temperature for 1 hour. To the reaction mixture, methanesulfonic acid (60 μL) was added, and the mixture was stirred at room temperature for 1 hour. To the reaction mixture, ethyl acetate and a saturated aqueous solution of sodium bicarbonate were added. An organic layer was separated, washed with a saturated aqueous solution of sodium chloride and then dried over sodium sulfate, and the solvent was distilled off under reduced pressure. The obtained residue was suspended in a mixed solvent of ethyl acetate and diisopropyl ether, and the deposit was collected by filtration to obtain a white solid of 8-(benzyloxy)-5-((2-hydroxybenzyl)amino)-4-methylpyrido[2,3-d]pyrimidin-7(8H)-one (16 mg). $^1$H-NMR ($CD_3OD$) δ value: 3.02 (s, 3H), 4.48 (s, 2H), 5.18 (s, 2H), 5.72 (s, 1H), 6.80-6.87 (m, 2H), 7.10-7.17 (m, 1H), 7.25-7.30 (m, 1H), 7.33-7.39 (m, 3H), 7.59-7.65 (m, 2H), 8.84 (s, 1H).

Reference Example 159

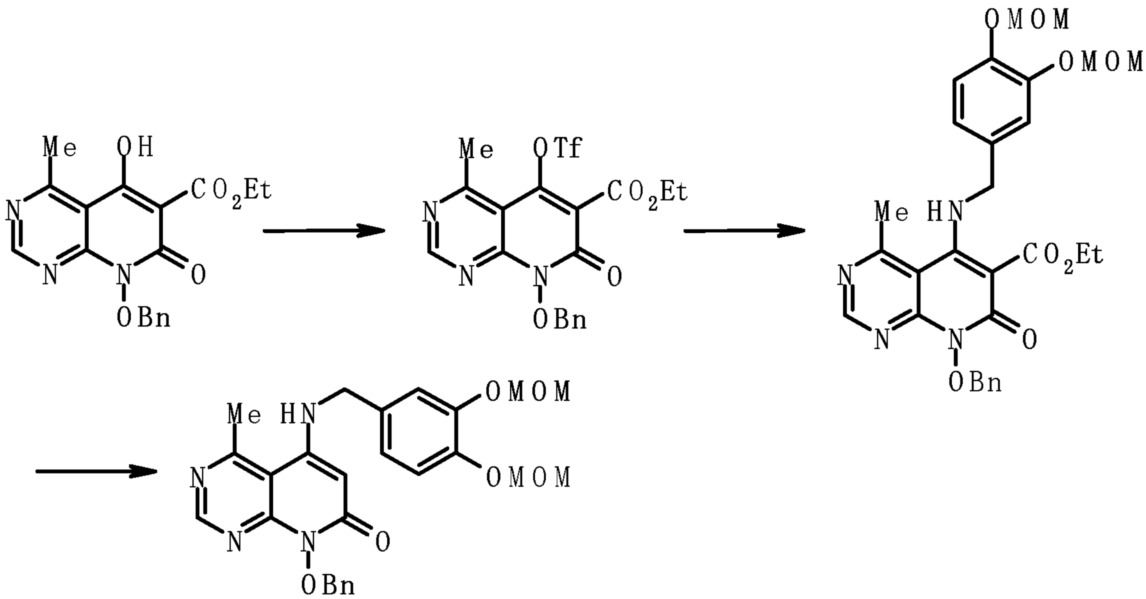

To ethyl 8-(benzyloxy)-5-hydroxy-4-methyl-7-oxo-7,8-dihydropyrido[2,3-d]pyrimidine-6-carboxylate (100 mg), triethylamine (78 μL) and methylene chloride (5.0 mL) were added, then trifluoromethanesulfonic anhydride (92 μL) was added dropwise under ice cooling, and the mixture was stirred at room temperature for 1 hour. The solvent in the reaction mixture was distilled off under reduced pressure. To the obtained residue, 1-(3,4-bis(methoxymethoxy)phenyl)methanamine (256 mg) and dioxane (5.0 mL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-20% hexane/ethyl acetate] to obtain a brown oil (96 mg). To the obtained brown oil (95 mg), methanol (4.0 mL) and a 1 mol/L aqueous sodium hydroxide solution (4.0 mL) were added, and the mixture was heated with stirring at 70 to 80°C for 30 minutes in a nitrogen atmosphere,. After cooling of the reaction mixture, a saturated aqueous solution of sodium bicarbonate and ethyl acetate were added thereto. An organic layer was separated, washed with a saturated aqueous solution of sodium chloride and

then dried over sodium sulfate, and the solvent was distilled off under reduced pressure. The obtained residue was suspended in a mixed solvent of ethyl acetate and 2-propanol, and the deposit was collected by filtration to obtain a pale yellow solid of 8-(benzyloxy)-5-((3,4-bis(methoxymethoxy)benzyl)amino)-4-methylpyrido[2,3-d]pyrimidin-7(8H)-one (24 mg).
$^1$H-NMR ($CDCl_3$) δ value: 2.93 (s, 3H), 3.52 (s, 3H), 3.54 (s, 3H), 4.28 (d, J=4.4 Hz, 2H), 5.22-5.30 (m, 7H), 5.75 (s, 1H), 6.98 (dd, J=8.3, 1.9 Hz, 1H), 7.19 (d, J=8.3 Hz, 1H), 7.21 (d, J=2.2 Hz, 1H), 7.34-7.42 (m, 3H), 7.66-7.71 (m, 2H), 8.89 (s, 1H).

Reference Example 160

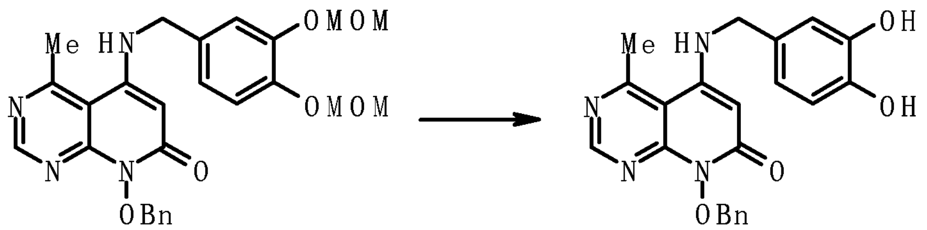

To a suspension of 8-(benzyloxy)-5-((3,4-bis(methoxymethoxy)benzyl)amino)-4-methylpyrido[2,3-d]pyrimidin-7(8H)-one (23 mg) in methanol (2.0 mL) and dioxane (1.0 mL), methanesulfonic acid (15 μL) was added, and the mixture was stirred at room temperature for 1 hour. To the reaction mixture, methanesulfonic acid (15 μL) was added, and the mixture was stirred at room temperature for 2 hours and 30 minutes. To the reaction mixture, ethyl acetate and a saturated aqueous solution of sodium bicarbonate were added. An organic layer was separated, washed with a saturated aqueous solution of sodium chloride and then dried over sodium sulfate, and the solvent was distilled off under reduced pressure. The obtained residue was suspended in a mixed solvent of ethyl acetate and diisopropyl ether, and the deposit was collected by filtration to obtain a yellow solid of 8-(benzyloxy)-5-((3,4-dihydroxybenzyl)amino)-4-methylpyrido[2,3-d]pyrimidin-7(8H)-one (18 mg).
$^1$H-NMR (DMSO-$d_6$) δ value: 3.00 (s, 3H), 4.29 (d, J=5.4 Hz, 2H), 5.07 (s, 2H), 5.43 (s, 1H), 6.68 (dd, J=8.2, 1.8 Hz, 1H), 6.72 (d, J=7.8 Hz, 1H), 6.80 (d, J=1.7 Hz, 1H), 6.81-6.87 (m, 1H), 7.36-7.45 (m, 3H), 7.59-7.64 (m, 2H), 8.83 (brs, 1H), 8.86-8.92 (m, 2H).

Reference Example 161

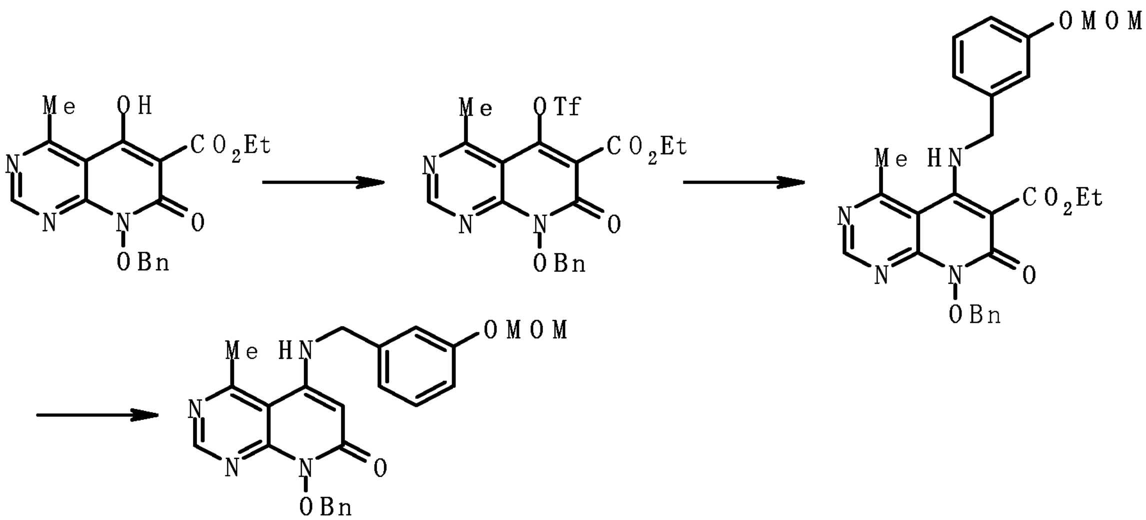

To ethyl 8-(benzyloxy)-5-hydroxy-4-methyl-7-oxo-7,8-dihydropyrido[2,3-d]pyrimidine-6-carboxylate (100 mg), triethylamine (78 μL) and methylene chloride (5.0 mL) were added, then trifluoromethanesulfonic anhydride (92 μL) was

added dropwise under ice cooling, and the mixture was stirred at room temperature for 1 hour. The solvent in the reaction mixture was distilled off under reduced pressure. To the obtained residue, 1-(3-(methoxymethoxy)phenyl)methanamine (188 mg) and dioxane (5.0 mL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-30% hexane/ethyl acetate] to obtain a brown oil (76 mg). To the obtained brown oil (75 mg), methanol (3.8 mL) and a 1 mol/L aqueous sodium hydroxide solution (3.8 mL) were added, and the mixture was heated with stirring at 70 to 80°C for 40 minutes in a nitrogen atmosphere. After cooling of the reaction mixture, a saturated aqueous solution of sodium bicarbonate and ethyl acetate were added thereto, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 80-20% hexane/ethyl acetate] and then suspended in a mixed solvent of 2-propanol and diisopropyl ether, and the deposit was collected by filtration to obtain a pale yellow solid of 8-(benzyloxy)-5-((3-(methoxymethoxy)benzyl)amino)-4-methylpyrido[2,3-d]pyrimidin-7(8H)-one (19 mg). $^1H$-NMR ($CDCl_3$) $\delta$ value: 2.95 (s, 3H), 3.49 (s, 3H), 4.35 (d, J=4.4 Hz, 2H), 5.20 (s, 2H), 5.25 (s, 2H), 5.32-5.37 (m, 1H), 5.74 (s, 1H), 6.99-7.08 (m, 3H), 7.30-7.42 (m, 4H), 7.66-7.70 (m, 2H), 8.90 (s, 1H).

Reference Example 162

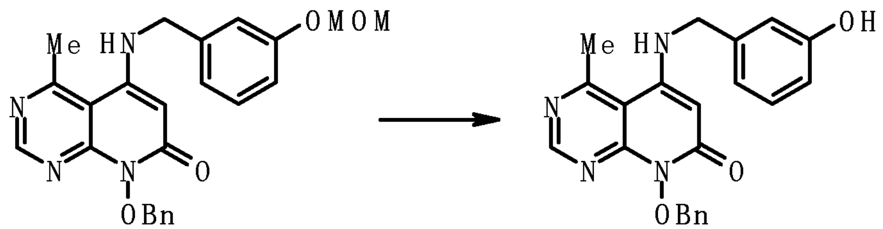

To a suspension of 8-(benzyloxy)-5-((3-(methoxymethoxy)benzyl)amino)-4-methylpyrido[2,3-d]pyrimidin-7(8H)-one (18 mg) in methanol (2.0 mL), methanesulfonic acid (14 $\mu$L) was added, and the mixture was stirred at room temperature for 1 hour. To the reaction mixture, methanesulfonic acid (14 $\mu$L) was added, and the mixture was stirred at room temperature for 2 hours. To the reaction mixture, ethyl acetate and a saturated aqueous solution of sodium bicarbonate were added. An organic layer was separated, washed with a saturated aqueous solution of sodium chloride and then dried over sodium sulfate, and the solvent was distilled off under reduced pressure. The obtained residue was suspended in a mixed solvent of ethyl acetate and diisopropyl ether, and the deposit was collected by filtration to obtain a pale yellow solid of 8-(benzyloxy)-5-((3-hydroxybenzyl)amino)-4-methylpyrido[2,3-d]pyrimidin-7(8H)-one (13 mg).
$^1H$-NMR ($CD_3OD$) $\delta$ value: 3.05 (s, 3H), 4.46 (s, 2H), 5.17 (s, 2H), 5.61 (s, 1H), 6.67-6.74 (m, 1H), 6.81-6.91 (m, 2H), 7.19 (dd, J=7.8, 7.8 Hz, 1H), 7.32-7.39 (m, 3H), 7.58-7.64 (m, 2H), 8.85 (s, 1H).

Reference Example 163

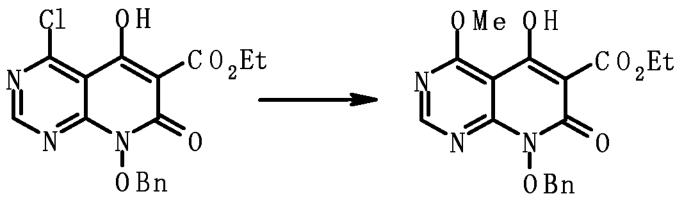

To a suspension of ethyl 8-(benzyloxy)-4-chloro-5-hydroxy-7-oxo-7,8-dihydropyrido[2,3-d]pyrimidine-6-carboxylate (50 mg) in methanol (1.5 mL), a 28% solution of sodium methoxide in methanol (257 mg) was added, and the mixture was stirred at room temperature for 1 hour and 30 minutes. To the reaction mixture, chloroform and 1 mol/L hydrochloric acid were added. An organic layer was separated and dried over magnesium sulfate, and the solvent was distilled off under reduced pressure to obtain a white solid of ethyl 8-(benzyloxy)-5-hydroxy-4-methoxy-7-oxo-7,8-dihydropyrido[2,3-d]pyrimidine-6-carboxylate (35 mg).
$^1$H-NMR ($CDCl_3$) δ value: 1.49 (t, J=7.1 Hz, 3H), 4.20 (s, 3H), 4.49-4.58 (m, 2H), 5.25 (s, 2H), 7.35-7.43 (m, 3H), 7.65-7.71 (m, 2H), 8.72 (s, 1H).

Reference Example 164

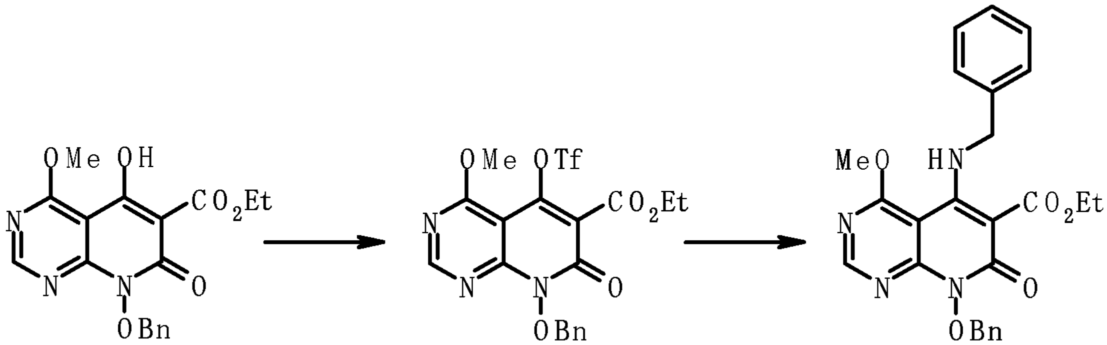

To a solution of ethyl 8-(benzyloxy)-5-hydroxy-4-methoxy-7-oxo-7,8-dihydropyrido[2,3-d]pyrimidine-6-carboxylate (34 mg) and triethylamine (25 μL) in methylene chloride (2.0 mL), trifluoromethanesulfonic anhydride (30 μL) was added dropwise under ice cooling, and the mixture was stirred at room temperature for 1 hour. To the reaction mixture, benzylamine (50 μL) was added, and the mixture was stirred at room temperature for 30 minutes. The reaction mixture was purified by silica gel column chromatography [Kanto Chemical Co., Inc., silica gel 60 (spherical), eluent: 80-40% hexane/ethyl acetate] and then suspended in diisopropyl ether, and the deposit was collected by filtration to obtain a pale yellow solid of ethyl 5-(benzylamino)-8-(benzyloxy)-4-methoxy-7-oxo-7,8-dihydropyrido[2,3-d]pyrimidine-6-carboxylate (36 mg).
$^1$H-NMR ($CDCl_3$) δ value: 1.40 (t, J=7.1 Hz, 3H), 4.01 (s, 3H), 4.34-4.45 (m, 4H), 5.25 (s, 2H), 7.31-7.45 (m, 8H), 7.57-7.64 (m, 1H), 7.66-7.73 (m, 2H), 8.63 (s, 1H).

Reference Example 165

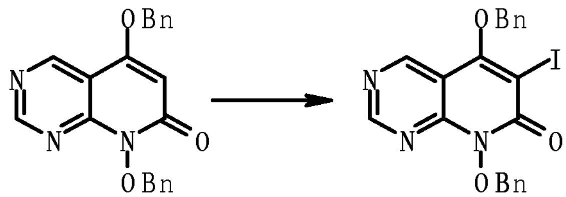

To a suspension of 5,8-bis(benzyloxy)pyrido[2,3-d]pyrimidin-7(8H)-one (25 mg) in acetonitrile (2.5 mL), TFA (5 μL) and N-iodosuccinimide (17 mg) were added, and the mixture was stirred at room temperature for 4 hours and then heated with stirring at 65 to 70°C for 1 hour and at 70 to 75°C for 1 hour. After cooling of the reaction mixture, N-iodosuccinimide (14 mg) was added thereto, and the mixture was heated with stirring at 70 to 75°C for 1 hour. After cooling of the reaction mixture, TFA (5 μL) was added thereto, and the mixture was heated with stirring at 70 to 75°C for 30 minutes. After cooling of the reaction mixture, ethyl acetate and a saturated aqueous solution of sodium bicarbonate were added thereto. An organic layer was separated, washed with a saturated aqueous solution of sodium chloride and then dried over magnesium sulfate, and the solvent was distilled off under reduced pressure. The obtained residue was purified by silica gel column chromatography [Kanto Chemical Co., Inc., silica gel 60 (spherical), eluent: 90-60% hexane/ethyl acetate] and then suspended in diisopropyl ether, and the deposit was collected by filtration to obtain a pale yellow solid of 5,8-bis(benzyloxy)-6-iodopyrido[2,3-d]pyrimidin-7(8H)-one (19 mg).
$^1$H-NMR ($CDCl_3$) δ value: 5.31 (s, 2H), 5.33 (s, 2H), 7.37-7.46 (m, 6H), 7.46-7.53 (m, 2H), 7.65-7.71 (m, 2H), 8.92 (s, 1H), 9.15 (s, 1H).

Reference Example 166

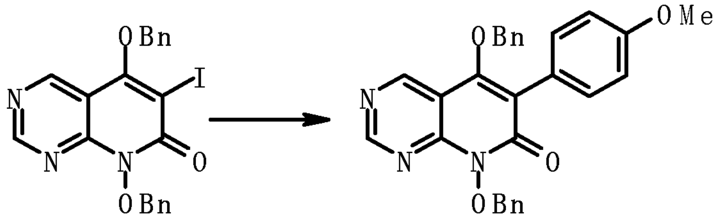

To a suspension of 5,8-bis(benzyloxy)-6-iodopyrido[2,3-d]pyrimidin-7(8H)-one (18 mg) in ethylene glycol dimethyl ether (1.8 mL), water (0.54 mL), 4-methoxyphenylboronic acid (7 mg), sodium carbonate (8 mg) and bis(di-tert-butyl(4-dimethylaminophenyl)phosphine)palladium(II) dichloride (1 mg) were added, and the mixture was heated to reflux for 2 hours in a nitrogen atmosphere. After cooling of the reaction mixture, ethyl acetate and water were added thereto. An organic layer was separated, washed with a saturated aqueous solution of sodium chloride and then dried over magnesium sulfate, and the solvent was distilled off under reduced pressure. The obtained residue was purified by silica gel column chromatography [Kanto Chemical Co., Inc., silica gel 60 (spherical), eluent: 90-50% hexane/ethyl acetate] and then suspended in diisopropyl ether, and the deposit was collected by filtration to obtain a yellow solid of 5,8-bis(benzyloxy)-6-(4-methoxyphenyl)pyrido[2,3-d]pyrimidin-7(8H)-one (10 mg).
$^1$H-NMR ($CDCl_3$) δ value: 3.89 (s, 3H), 4.65 (s, 2H), 5.31 (s, 2H), 7.03-7.08 (m, 2H), 7.13-7.18 (m, 2H), 7.28-7.33 (m, 3H), 7.36-7.42 (m, 3H), 7.50-7.56 (m, 2H), 7.65-7.71 (m, 2H), 9.09 (s, 1H), 9.12 (s, 1H).

Reference Example 167

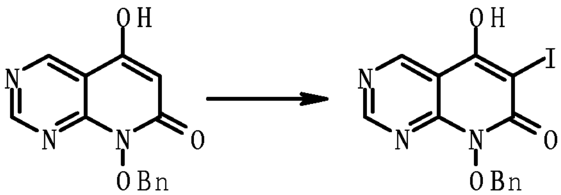

To a solution of 8-(benzyloxy)-5-hydroxypyrido[2,3-d]pyrimidin-7(8H)-one (400 mg) in DMF (4.0 mL), N-iodosuccinimide (351 mg) was added, and the mixture was stirred at room temperature for 30 minutes. To the reaction mixture, ethyl acetate and 0.5 mol/L hydrochloric acid were added, followed by separation of an organic layer. An aqueous layer was extracted with ethyl acetate, combined with the organic layer, washed with 0.5 mol/L hydrochloric acid and a saturated aqueous solution of sodium chloride in this order and then dried over magnesium sulfate, and the solvent was distilled off under reduced pressure. The obtained residue was suspended in a mixed solvent of ethyl acetate and diisopropyl ether, and the deposit was collected by filtration to obtain a red solid of 8-(benzyloxy)-5-hydroxy-6-iodopyrido[2,3-d]pyrimidin-7(8H)-one (488 mg).
$^1$H-NMR (DMSO-$d_6$) δ value: 5.16 (s, 2H), 7.38-7.49 (m, 3H), 7.61-7.68 (m, 2H), 9.18 (s, 1H), 9.25 (s, 1H).

Reference Example 168

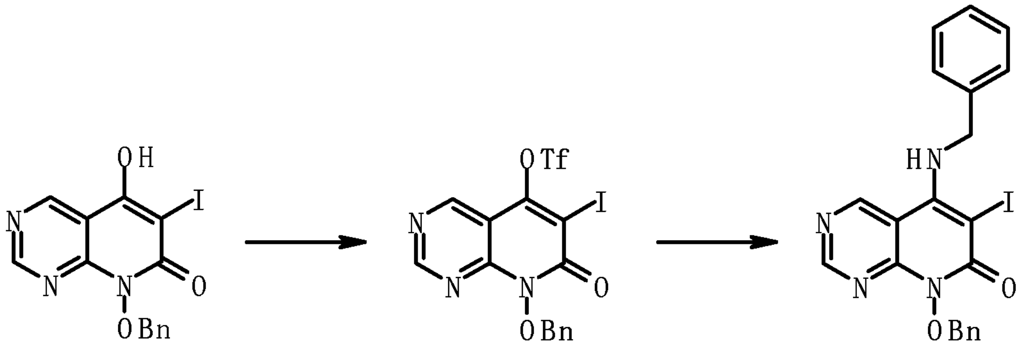

To a solution of 8-(benzyloxy)-5-hydroxy-6-iodopyrido[2,3-d]pyrimidin-7(8H)-one (100 mg) and triethylamine (71 μL) in methylene chloride (5.0 mL), trifluoromethanesulfonic anhydride (83 μL) was added dropwise under ice cooling, and the mixture was stirred at room temperature for 1 hour. The solvent in the reaction mixture was distilled off under reduced pressure. To the obtained residue, benzylamine (138 μL) and dioxane (5.0 mL) were added, and the mixture was heated with stirring at 60°C for 30 minutes. After cooling of the reaction mixture, the solvent was distilled off under reduced pressure. The obtained residue was purified by silica gel column chromatography [Kanto Chemical Co., Inc., silica gel 60 (spherical), eluent: 90-30% hexane/ethyl acetate] and then suspended in a mixed solvent of 2-propanol and diisopropyl ether, and the deposit was collected by filtration to obtain a yellow solid of 5-(benzylamino)-8-(benzyloxy)-6-iodopyrido[2,3-d]pyrimidin-7(8H)-one (19 mg).
$^1$H-NMR ($CDCl_3$) δ value: 4.80 (d, J=6.6 Hz, 2H), 5.29 (s, 2H), 5.32-5.40 (m, 1H), 7.34-7.46 (m, 8H), 7.66-7.72 (m, 2H), 9.11 (s, 1H), 9.19 (s, 1H).

Reference Example 169

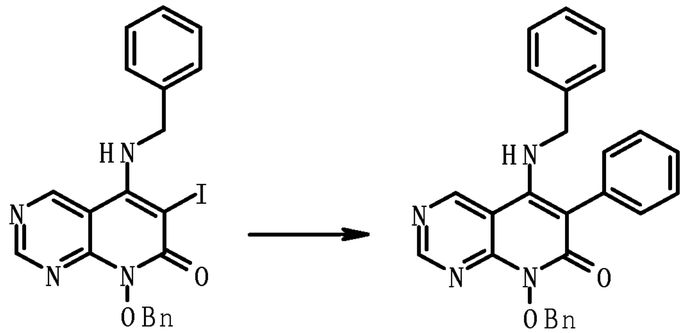

To a solution of 5-(benzylamino)-8-(benzyloxy)-6-iodopyrido[2,3-d]pyrimidin-7(8H)-one (18 mg) in ethylene glycol dimethyl ether (1.8 mL), water (0.54 mL), phenylboronic acid (5 mg), sodium carbonate (8 mg) and bis(di-tert-butyl(4-dimethylaminophenyl)phosphine)palladium(II) dichloride (1 mg) were added, and the mixture was heated to reflux for 2 hours in a nitrogen atmosphere. After cooling of the reaction mixture, ethyl acetate and water were added thereto. An organic layer was separated, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 90-30% hexane/ethyl acetate] and then suspended in diisopropyl ether, and the deposit was collected by filtration to obtain a yellow solid of 5-(benzylamino)-8-(benzyloxy)-6-phenylpyrido[2,3-d]pyrimidin-7(8H)-one (13 mg).
$^1$H-NMR ($CDCl_3$) δ value: 4.27-4.32 (m, 2H), 4.51-4.58 (m, 1H), 5.31 (s, 2H), 7.10-7.15 (m, 2H), 7.22-7.35 (m, 5H), 7.35-7.47 (m, 6H), 7.66-7.71 (m, 2H), 9.07 (s, 1H), 9.11 (s, 1H).

Reference Example 170

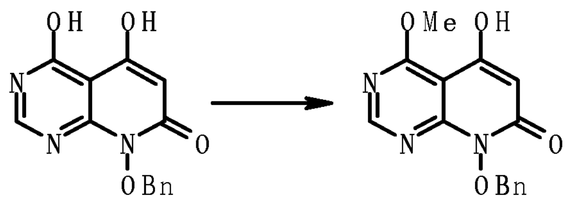

To a solution of 8-(benzyloxy)-4,5-dihydroxypyrido[2,3-d]pyrimidin-7(8H)-one (74 mg) in DMF (3.7 mL), potassium carbonate (36 mg) and methyl iodide (15 μL) were added, and the mixture was stirred at room temperature for 1 hour. To the reaction mixture, ethyl acetate and a 10% aqueous citric acid solution were added. An organic layer was separated, washed with a 10% aqueous citric acid solution and a saturated aqueous solution of sodium chloride in this order and then dried over magnesium sulfate, and the solvent was distilled off under reduced pressure. The obtained residue was purified by silica gel column chromatography [Kanto Chemical Co., Inc., silica gel 60 (spherical), eluent: 90-20% hexane/ethyl acetate] and then suspended in methanol, and the deposit was collected by filtration to obtain a white solid of 8-(benzyloxy)-5-hydroxy-4-methoxypyrido[2,3-d]pyrimidin-7(8H)-one (36 mg).
$^1$H-NMR ($CDCl_3$) δ value: 3.60 (s, 3H), 5.24 (s, 2H), 6.01 (s, 1H), 7.34-7.40 (m, 3H), 7.58-7.63 (m, 2H), 8.15 (s, 1H), 11.29 (s, 1H).

Reference Example 171

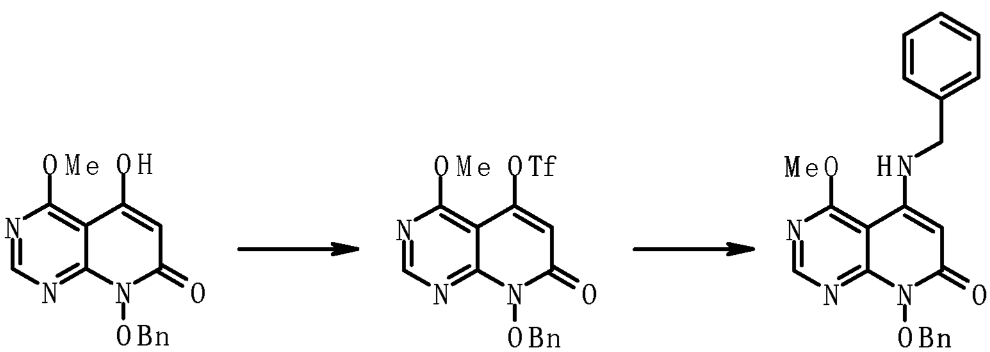

To a solution of 8-(benzyloxy)-5-hydroxy-4-methoxypyrido[2,3-d]pyrimidin-7(8H)-one (26 mg) in methylene chloride (2.6 mL), triethylamine (24 μL) was added, then trifluoromethanesulfonic anhydride (29 μL) was added dropwise under ice cooling, and the mixture was stirred at room temperature for 1 hour. The solvent in the reaction mixture was distilled off under reduced pressure. To the obtained residue, benzylamine (47 μL) and dioxane (1.3 mL) were added, and the mixture was heated with stirring at 65 to 70°C for 30 minutes. After cooling of the reaction mixture, the solvent was distilled off under reduced pressure. The obtained residue was purified by silica gel column chromatography [Kanto Chemical Co., Inc., silica gel 60 (spherical), eluent: 80-20% hexane/ethyl acetate] and then suspended in a mixed solvent of ethyl acetate and 2-propanol, and the deposit was collected by filtration to obtain a white solid of 5-(benzylamino)-8-(benzyloxy)-4-methoxypyrido[2,3-d]pyrimidin-7(8H)-one (22 mg).
$^1$H-NMR ($CDCl_3$) δ value: 3.53 (s, 3H), 4.37 (d, J=5.4 Hz, 2H), 5.21 (s, 2H), 5.51 (s, 1H), 7.24-7.40 (m, 8H), 7.59-7.64 (m, 2H), 8.11 (s, 1H), 8.88-8.95 (m, 1H).

Reference Example 172

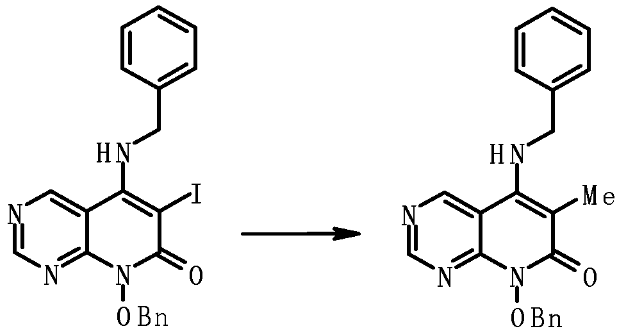

To a solution of 5-(benzylamino)-8-(benzyloxy)-6-iodopyrido[2,3-d]pyrimidin-7(8H)-one (20 mg) in dioxane (2.0 mL), 2,4,6-trimethylboroxine (9 μL), potassium carbonate (11 mg), cesium carbonate (13 mg) and tetrakis(triphenylphosphine)palladium(0) (2 mg) were added, and the mixture was heated to reflux for 3 hours in a nitrogen atmosphere. After cooling of the reaction mixture, ethyl acetate and water were added thereto. An organic layer was separated, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 80-30% hexane/ethyl acetate] to obtain a yellow oil of 5-(benzylamino)-8-(benzyloxy)-6-methylpyrido[2,3-d]pyrimidin-7(8H)-one (10 mg).
$^1$H-NMR ($CDCl_3$) δ value: 2.14 (s, 3H), 4.12-4.20 (m, 1H), 4.58 (d, J=6.6 Hz, 2H), 5.29 (s, 2H), 7.27-7.43 (m, 8H), 7.66-7.72 (m, 2H), 9.06 (s, 1H), 9.06 (s, 1H).

Reference Example 173

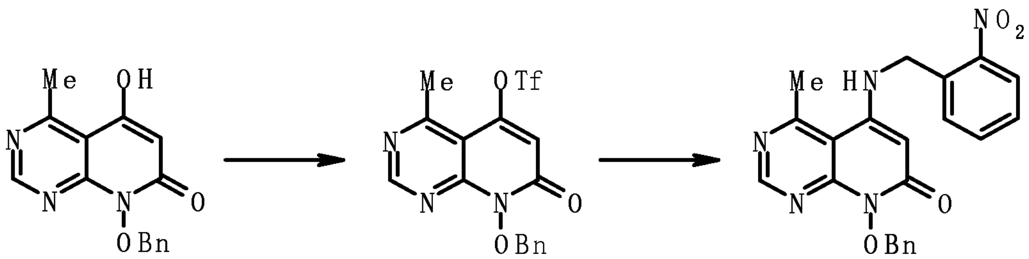

To a suspension of 8-(benzyloxy)-5-hydroxy-4-methylpyrido[2,3-d]pyrimidin-7(8H)-one (100 mg) in methylene chloride (5.0 mL), triethylamine (98 μL) was added, then trifluoromethanesulfonic anhydride (116 μL) was added dropwise under ice cooling, and the mixture was stirred at room temperature for 1 hour. The solvent in the reaction mixture was distilled off under reduced pressure. To the obtained residue, 2-nitrobenzylamine (215 mg) and dioxane (5.0 mL) were added, and the mixture was heated with stirring at 80 to 90°C for 1 hour and at 90 to 100°C for 30 minutes and then heated to reflux for 30 minutes. After cooling of the reaction mixture, 2-nitrobenzylamine (107 mg) was added thereto, and the mixture was heated to reflux for 30 minutes. After cooling of the reaction mixture, the solvent was distilled off under reduced pressure. The obtained residue was purified by silica gel column chromatography [Kanto Chemical Co., Inc., silica gel 60 (spherical), eluent: 80-20% hexane/ethyl acetate] and then suspended in 2-propanol, and the deposit was collected by filtration to obtain a pale yellow solid of 8-(benzyloxy)-4-methyl-5-((2-nitrobenzyl)amino)pyrido[2,3-d]pyrimidin-7(8H)-one (46 mg).
$^1$H-NMR ($CDCl_3$) δ value: 2.99 (s, 3H), 4.80 (d, J=5.8 Hz, 2H), 5.22 (s, 2H), 5.73 (s, 1H), 5.96-6.04 (m, 1H), 7.34-7.41 (m, 3H), 7.52-7.59 (m, 1H), 7.61-7.72 (m, 4H), 8.15 (dd, J=8.3, 1.2 Hz, 1H), 8.90 (s, 1H).

Reference Example 174

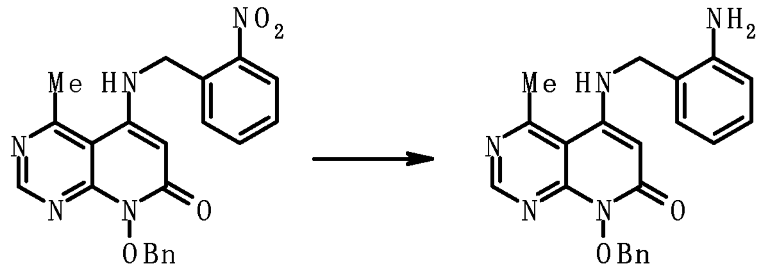

To a suspension of 8-(benzyloxy)-4-methyl-5-((2-nitrobenzyl)amino)pylido[2,3-d]pyrimidin-7(8H)-one (20 mg) in ethanol (1.0 mL), water (0.15 mL), ammonium chloride (2 mg) and an iron powder (8 mg) were added, and the mixture was heated to reflux for 1 hour. After cooling of the reaction mixture, ammonium chloride (2 mg) and an iron powder (8 mg) were added thereto, and the mixture was heated to reflux for 30 minutes. After cooling of the reaction mixture, chloroform and a saturated aqueous solution of sodium bicarbonate were added thereto. Insoluble matter was filtered off. Then, an organic layer was separated and dried over sodium sulfate, and the solvent was distilled off under reduced pressure. The obtained residue was purified by silica gel column chromatography [Kanto Chemical Co., Inc., silica gel 60 (spherical), eluent: 75-0% hexane/ethyl acetate] to obtain a pale yellow solid of 5-((2-aminobenzyl)amino)-8-(benzyloxy)-4-methylpyrido[2,3-d]pyrimidin-7(8H)-one (12 mg). $^1$H-NMR ($CDCl_3$) δ value: 2.89 (s, 3H), 3.80 (brs, 2H), 4.25 (d, J=4.2 Hz, 2H), 5.23-5.29 (m, 3H), 5.83 (s, 1H), 6.77-6.86 (m, 2H), 7.17-7.31 (m, 2H), 7.35-7.42 (m, 3H), 7.67-7.72 (m, 2H), 8.89 (s, 1H).

Reference Example 175

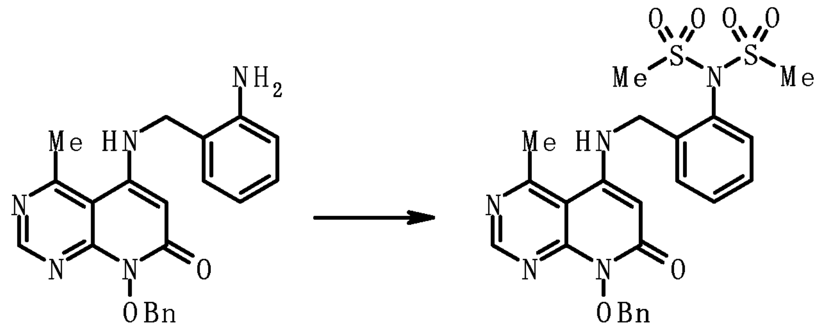

To a solution of 5-((2-aminobenzyl)amino)-8-(benzyloxy)-4-methylpyrido[2,3-d]pyrimidin-7(8H)-one (11 mg) in methylene chloride (1.0 mL), triethylamine (6 μL) was added, then methanesulfonyl chloride (2 μL) was added dropwise under ice cooling, and the mixture was stirred at room temperature for 1 hour. To the reaction mixture, triethylamine (6 μL), 4-(dimethylamino)pyridine (4 mg) and methanesulfonyl chloride (2 μL) were added, and the mixture was stirred at room temperature for 2 hours. To the reaction mixture, triethylamine (6 μL) and methanesulfonyl chloride (2 μL) were added, and the mixture was stirred at room temperature for 1 hour. To the reaction mixture, chloroform and a saturated aqueous solution of sodium bicarbonate were added. An organic layer was separated and dried over sodium sulfate, and the solvent was distilled off under reduced pressure. The obtained residue was purified by silica gel column chromatography [Kanto Chemical Co., Inc., silica gel 60 (spherical), eluent: 100-90% chloroform/methanol] to obtain a white solid of N-(2-(((8-(benzyloxy)-4-methyl-7-oxo-7,8-dihydropyrido[2,3-d]pyrimidin-5-yl)amino)methyl)phenyl)-N-(methylsulfonyl)methanesulfonamide (14 mg).
$^1$H-NMR ($CDCl_3$) δ value: 2.81 (s, 3H), 3.45 (s, 6H), 4.38-4.42 (m, 2H), 5.27 (s, 2H), 5.46-5.52 (m, 1H), 5.87 (s, 1H), 7.35-7.46 (m, 4H), 7.48-7.66 (m, 3H), 7.66-7.72 (m, 2H), 8.89 (s, 1H).

Reference Example 176

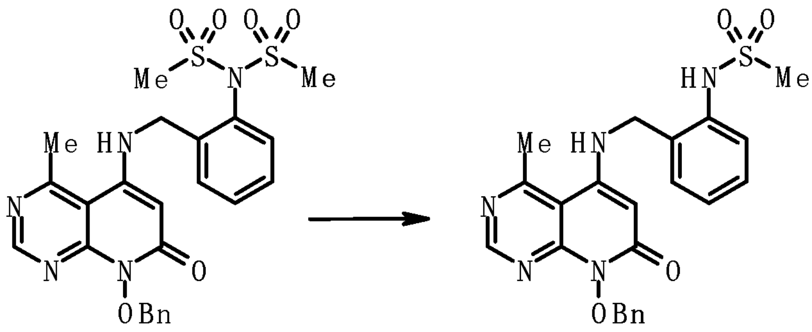

To a suspension of N-(2-(((8-(benzyloxy)-4-methyl-7-oxo-7,8-dihydropyrido[2,3-d]pyrimidin-5-yl)amino)methyl)phenyl)-N-(methylsulfonyl)methanesulfonamide (14 mg) in THF (1.0 mL), a 2 mol/L aqueous sodium hydroxide solution (0.10 mL) was added, and the mixture was stirred at room temperature for 2 hours. The solvent in the reaction mixture was distilled off under reduced pressure, and a saturated aqueous solution of sodium bicarbonate and ethyl acetate were added to the residue. An organic layer was separated, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a pale yellow solid of N-(2-(((8-(benzyloxy)-4-methyl-7-oxo-7,8-dihydropyrido[2,3-d]pyrimidin-5-yl)amino)methyl)phenyl)methanesulfonamide (9 mg).
$^1$H-NMR ($CD_3OD$) δ value: 3.04 (s, 3H), 3.05 (s, 3H), 4.70 (s, 2H), 5.17 (s, 2H), 5.58 (s, 1H), 7.32-7.41 (m, 6H), 7.48-7.53 (m, 1H), 7.58-7.65 (m, 2H), 8.85 (s, 1H).

Reference Example 177

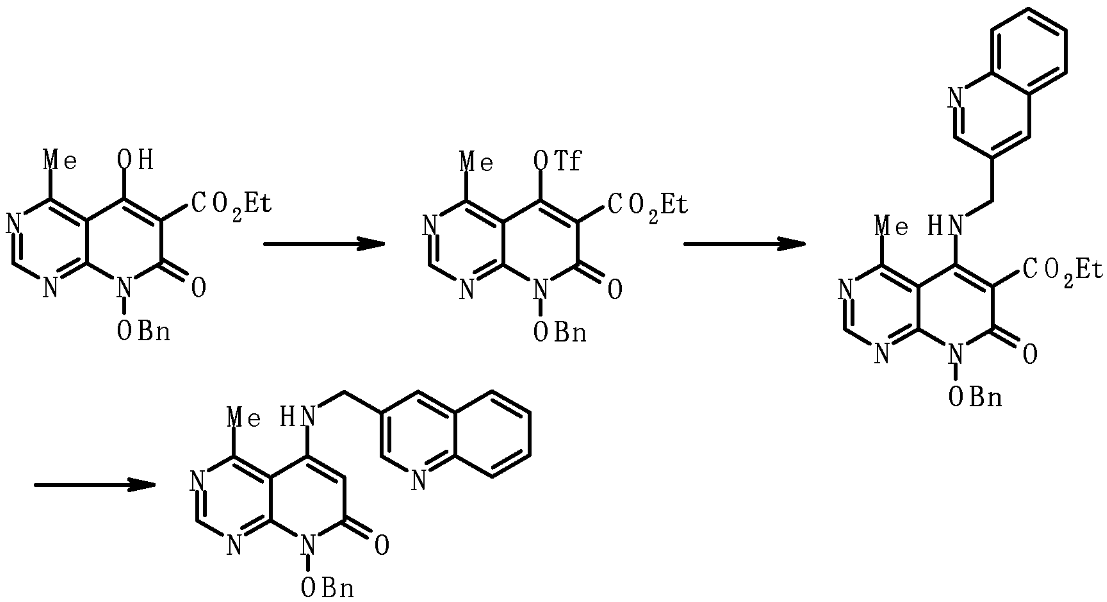

To a solution of ethyl 8-(benzyloxy)-5-hydroxy-4-methyl-7-oxo-7,8-dihydropyrido[2,3-d]pyrimidine-6-carboxylate (100 mg) in methylene chloride (5.0 mL), triethylamine (78 μL) was added, then trifluoromethanesulfonic anhydride (92 μL) was added dropwise under ice cooling, and the mixture was stirred at room temperature for 1 hour. The solvent in the reaction mixture was distilled off under reduced pressure. To the obtained residue, 1-(quinolin-3-yl)methanamine (178 mg) and dioxane (5.0 mL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 100-95% chloroform/methanol] to obtain a brown oil (91 mg). To the obtained brown oil (90 mg), methanol (4.5 mL) and a 1 mol/L aqueous sodium hydroxide solution (4.5 mL) were added, and the mixture was heated with stirring at 55 to 60°C for 6 hours in a nitrogen atmosphere.

After cooling of the reaction mixture, a saturated aqueous solution of sodium bicarbonate and ethyl acetate were added thereto, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 80-0% hexane/ethyl acetate-100-90% chloroform/methanol] and then suspended in a mixed solvent of ethyl acetate and diisopropyl ether, and the deposit was collected by filtration to obtain a yellow solid of 8-(benzyloxy)-4-methyl-5-(((quinolin-3-yl)methyl)amino)pyrido[2,3-d]pyrimidin-7(8H)-one (20 mg).
$^1$H-NMR ($CDCl_3$) δ value: 2.96 (s, 3H), 4.61 (d, J=4.9 Hz, 2H), 5.25 (s, 2H), 5.39-5.45 (m, 1H), 5.81 (s, 1H), 7.32-7.42 (m, 3H), 7.59-7.70 (m, 3H), 7.75-7.81 (m, 1H), 7.83-7.87 (m, 1H), 8.13-8.18 (m, 2H), 8.92 (s, 1H), 8.97 (d, J=2.2 Hz, 1H).

Reference Example 178

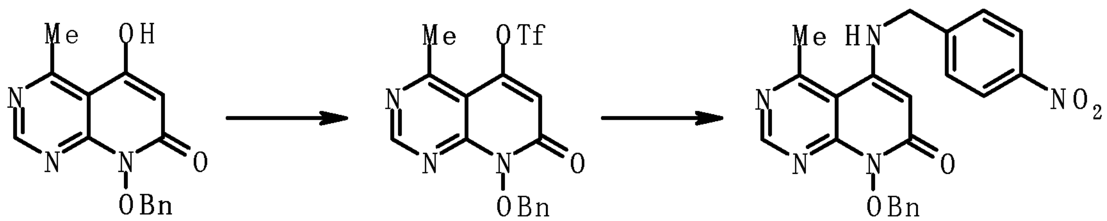

To a suspension of 8-(benzyloxy)-5-hydroxy-4-methylpyrido[2,3-d]pyrimidin-7(8H)-one (100 mg) in methylene chloride (5.0 mL), triethylamine (98 μL) was added, then trifluoromethanesulfonic anhydride (116 μL) was added dropwise under ice cooling, and the mixture was stirred at room temperature for 1 hour. The solvent in the reaction mixture was distilled off under reduced pressure. To the obtained residue, 4-nitrobenzylamine (215 mg) and dioxane (5.0 mL) were added, and the mixture was heated to reflux for 2 hours. After cooling of the reaction mixture, the solvent was distilled off under reduced pressure. The obtained residue was purified by silica gel column chromatography [Kanto Chemical Co., Inc., silica gel 60 (spherical), eluent: 100-94% chloroform/methanol] and then suspended in ethyl acetate, and the deposit was collected by filtration to obtain a pale orange solid of 8-(benzyloxy)-4-methyl-5-((4-nitrobenzyl)amino)pyrido[2,3-d]pyrimidin-7(8H)-one (38 mg).
$^1$H-NMR ($CDCl_3$) δ value: 3.01 (s, 3H), 4.56 (d, J=5.4 Hz, 2H), 5.24 (s, 2H), 5.46-5.52 (m, 1H), 5.63 (s, 1H), 7.35-7.42 (m, 3H), 7.52-7.58 (m, 2H), 7.64-7.69 (m, 2H), 8.25-8.30 (m, 2H), 8.93 (s, 1H).

Reference Example 179

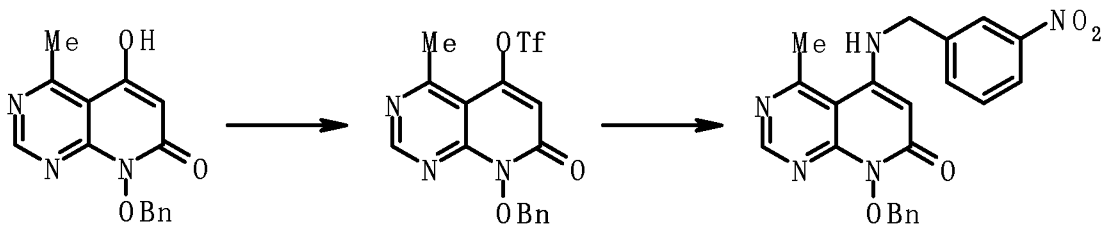

To a suspension of 8-(benzyloxy)-5-hydroxy-4-methylpyrido[2,3-d]pyrimidin-7(8H)-one (150 mg) in methylene chloride (7.5 mL), triethylamine (148 μL) was added, then trifluoromethanesulfonic anhydride (174 μL) was added dropwise under ice cooling, and the mixture was stirred at room temperature for 1 hour. The solvent in the reaction mixture was distilled off under reduced pressure. To the obtained residue, 3-nitrobenzylamine (363 mg) and dioxane (7.5 mL) were added, and the mixture was heated to reflux for 2 hours. After cooling of the reaction mixture, the solvent was distilled off under reduced pressure. The obtained residue was purified by silica gel column chromatography [Kanto Chemical Co., Inc., silica gel 60 (spherical), eluent: 100-90% chloroform/methanol] and then suspended in ethyl acetate, and the deposit was collected by filtration to obtain a pale yellow solid of 8-(benzyloxy)-4-methyl-5-((3-nitrobenzyl)amino)pyrido[2,3-d]pyrimidin-7(8H)-one (40 mg).
$^1$H-NMR (DMSO-$d_6$) δ value: 3.05 (s, 3H), 4.66 (d, J=5.6 Hz, 2H), 5.06 (s, 2H), 5.44 (s, 1H), 7.07-7.13 (m, 1H), 7.35-7.44

(m, 3H), 7.57-7.63 (m, 2H), 7.68 (dd, J=7.9, 7.9 Hz, 1H), 7.89-7.95 (m, 1H), 8.12-8.18 (m, 1H), 8.32-8.36 (m, 1H), 8.91 (s, 1H).

Reference Example 180

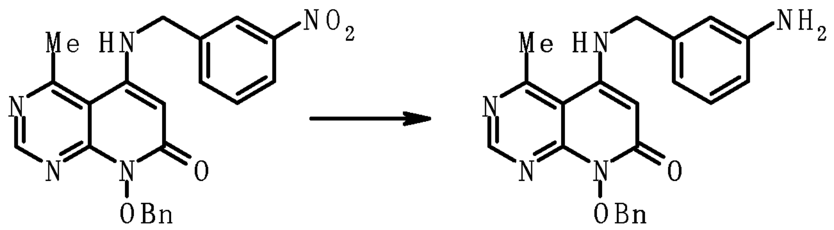

To a suspension of 8-(benzyloxy)-4-methyl-5-((3-nitrobenzyl)amino)pyrido[2,3-d]pyrimidin-7(8H)-one (25 mg) in ethanol (1.2 mL), water (0.18 mL), ammonium chloride (2 mg) and an iron powder (10 mg) were added, and the mixture was heated to reflux for 1 hour. After cooling of the reaction mixture, ammonium chloride (1 mg) and an iron powder (5 mg) were added thereto, and the mixture was heated to reflux for 30 minutes. After cooling of the reaction mixture, chloroform and a saturated aqueous solution of sodium bicarbonate were added thereto, and insoluble matter was filtered off. An organic layer was separated and dried over sodium sulfate, and the solvent was distilled off under reduced pressure. The obtained residue was suspended in a mixed solvent of ethyl acetate and diisopropyl ether, and the deposit was collected by filtration to obtain a white solid of 5-((3-aminobenzyl)amino)-8-(benzyloxy)-4-methylpyrido[2,3-d]pyrimidin-7(8H)-one (19 mg).
$^1$H-NMR ($CDCl_3$) δ value: 2.93 (s, 3H), 3.71-3.79 (m, 2H), 4.27 (d, J=4.4 Hz, 2H), 5.25 (s, 2H), 5.29-5.36 (m, 1H), 5.75 (s, 1H), 6.65-6.70 (m, 2H), 6.73-6.78 (m, 1H), 7.16-7.22 (m, 1H), 7.34-7.42 (m, 3H), 7.66-7.71 (m, 2H), 8.89 (s, 1H).

Reference Example 181

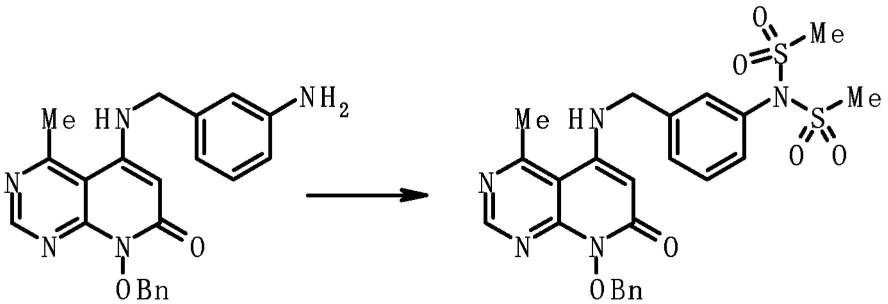

To a suspension of 5-((3-aminobenzyl)amino)-8-(benzyloxy)-4-methylpyrido[2,3-d]pyrimidin-7(8H)-one (18 mg) in methylene chloride (1.8 mL), triethylamine (10 μL) was added, then methanesulfonyl chloride (4 μL) was added dropwise under ice cooling, and the mixture was stirred at room temperature for 1 hour. To the reaction mixture, 4-(dimethylamino)pyridine (6 mg) was added, and the mixture was stirred at room temperature for 1 hour. To the reaction mixture, triethylamine (10 μL) and methanesulfonyl chloride (4 μL) were added, and the mixture was stirred at room temperature for 2 hours. To the reaction mixture, triethylamine (10 μL) and methanesulfonyl chloride (4 μL) were added, and the mixture was stirred at room temperature for 1 hour. To the reaction mixture, triethylamine (10 μL) and methanesulfonyl chloride (4 μL) were added, and the mixture was stirred at room temperature for 1 hour. To the reaction mixture, chloroform and a saturated aqueous solution of sodium bicarbonate were added. An organic layer was separated and dried over sodium sulfate, and the solvent was distilled off under reduced pressure. The obtained residue was purified by silica gel column chromatography [Kanto Chemical Co., Inc., silica gel 60 (spherical), eluent: 100-90% chloroform/methanol] to obtain a pale yellow solid of N-(3-(((8-(benzyloxy)-4-methyl-7-oxo-7,8-dihydropyrido[2,3-d]pyrimidin-5-yl)amino)methyl)phenyl)-N-(methylsulfonyl)methanesulfonamide (25 mg).
$^1$H-NMR ($CDCl_3$) δ value: 2.93 (s, 3H), 3.41 (s, 6H), 4.42 (d, J=4.9 Hz, 2H), 5.24 (s, 2H), 5.34-5.39 (m, 1H), 5.73 (s, 1H), 7.32-7.43 (m, 5H), 7.50-7.55 (m, 2H), 7.63-7.70 (m, 2H), 8.90 (s, 1H).

Reference Example 182

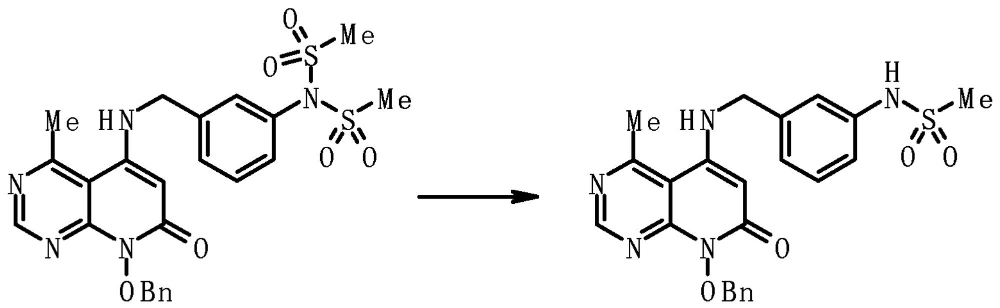

To a solution of N-(3-(((8-(benzyloxy)-4-methyl-7-oxo-7,8-dihydropyrido[2,3-d]pyrimidin-5-yl)amino)methyl)phenyl)-N-(methylsulfonyl)methanesulfonamide (24 mg) in THF (1.2 mL), a 2 mol/L aqueous sodium hydroxide solution (0.12 mL) was added, and the mixture was stirred at room temperature for 2 hours and 30 minutes. To the reaction mixture, a 2 mol/L aqueous sodium hydroxide solution (0.12 mL) was added, and the mixture was stirred at room temperature for 2 hours. To the reaction mixture, a 2 mol/L aqueous sodium hydroxide solution (0.12 mL) was added, and the mixture was stirred at room temperature for 1 hour. The solvent in the reaction mixture was distilled off under reduced pressure. To the obtained residue, a saturated aqueous solution of sodium bicarbonate and ethyl acetate were added thereto, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a pale yellow solid of N-(3-(((8-(benzyloxy)-4-methyl-7-oxo-7,8-dihydropyrido[2,3-d]pyrimidin-5-yl)amino)methyl)phenyl)methanesulfonamide (20 mg).
$^1$H-NMR ($CD_3OD$) δ value: 2.93 (s, 3H), 3.07 (s, 3H), 4.54 (s, 2H), 5.17 (s, 2H), 5.57 (s, 1H), 7.12-7.18 (m, 1H), 7.18-7.23 (m, 1H), 7.32-7.39 (m, 5H), 7.58-7.64 (m, 2H), 8.86 (s, 1H).

Reference Example 183

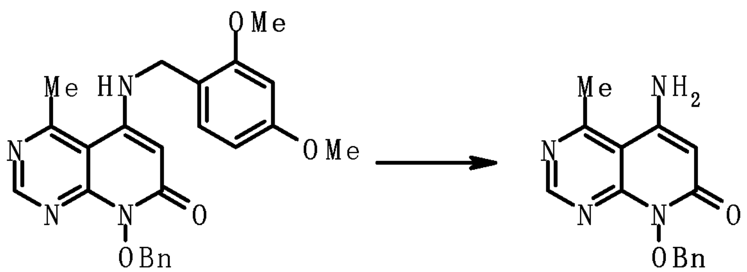

To a suspension of 8-(benzyloxy)-5-((2,4-dimethoxybenzyl)amino)-4-methylpyrido[2,3-d]pyrimidin-7(8H)-one (22 mg) in methanol (2.2 mL), methanesulfonic acid (50 μL) was added, and the mixture was heated with stirring at 50 to 55°C for 3 hours. After cooling of the reaction mixture, ethyl acetate and a saturated aqueous solution of sodium bicarbonate were added thereto, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 50-0% hexane/ethyl acetate - 100-90% chloroform/methanol] and then suspended in a mixed solvent of ethyl acetate and diisopropyl ether, and the deposit was collected by filtration to obtain a yellow solid of 5-amino-8-(benzyloxy)-4-methylpyrido[2,3-d]pyrimidin-7(8H)-one (8 mg).
$^1$H-NMR ($CDCl_3$) δ value: 2.99 (s, 3H), 4.79 (brs, 2H), 5.25 (s, 2H), 5.86 (s, 1H), 7.32-7.42 (m, 3H), 7.65-7.70 (m, 2H), 8.92 (s, 1H).

Reference Example 184

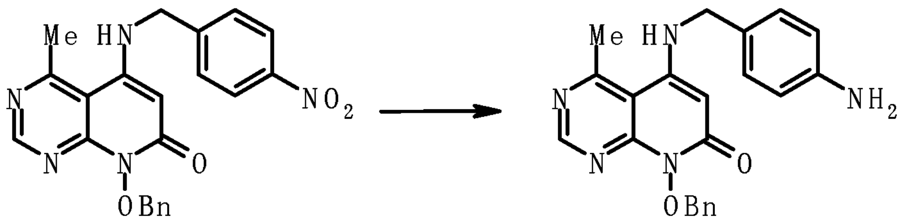

To a suspension of 8-(benzyloxy)-4-methyl-5-((4-nitrobenzyl)amino)pyrido[2,3-d]pyrimidin-7(8H)-one (23 mg) in ethanol (1.2 mL), water (0.18 mL), ammonium chloride (2 mg) and an iron powder (9 mg) were added, and the mixture was heated to reflux for 1 hour. After cooling of the reaction mixture, ammonium chloride (2 mg) and an iron powder (9 mg) were added thereto, and the mixture was heated to reflux for 30 minutes. After cooling of the reaction mixture, chloroform and a saturated aqueous solution of sodium bicarbonate were added thereto, and insoluble matter was filtered off. An organic layer was separated and dried over sodium sulfate, and the solvent was distilled off under reduced pressure. The obtained residue was purified by silica gel column chromatography [Kanto Chemical Co., Inc., silica gel 60 (spherical), eluent: 100-90% chloroform/methanol] to obtain a yellow oil of 5-((4-aminobenzyl)amino)-8-(benzyloxy)-4-methylpyrido[2,3-d]pyrimidin-7(8H)-one (20 mg). $^1$H-NMR ($CDCl_3$) $\delta$ value: 2.88 (s, 3H), 3.77 (brs, 2H), 4.21 (d, J=4.4 Hz, 2H), 5.17-5.26 (m, 1H), 5.25 (s, 2H), 5.75 (s, 1H), 6.68-6.74 (m, 2H), 7.13-7.19 (m, 2H), 7.32-7.42 (m, 3H), 7.64-7.71 (m, 2H), 8.87 (s, 1H).

Reference Example 185

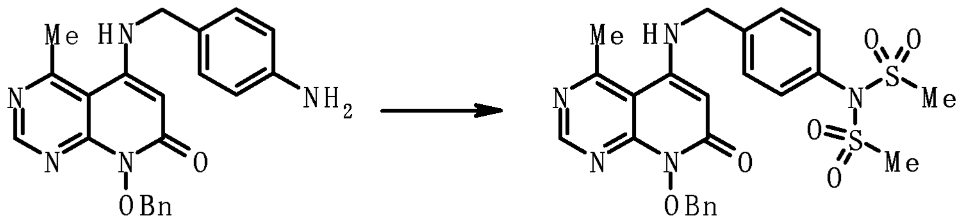

To a solution of 5-((4-aminobenzyl)amino)-8-(benzyloxy)-4-methylpyrido[2,3-d]pyrimidin-7(8H)-one (19 mg) in methylene chloride (1.9 mL), triethylamine (10 $\mu$L) and 4-(dimethylamino)pyridine (6 mg) were added, then methanesulfonyl chloride (4 $\mu$L) was added dropwise under ice cooling, and the mixture was stirred at room temperature for 1 hour. To the reaction mixture, triethylamine (10 $\mu$L) and methanesulfonyl chloride (4 $\mu$L) were added, and the mixture was stirred at room temperature for 1 hour. To the reaction mixture, triethylamine (21 $\mu$L) and methanesulfonyl chloride (8 $\mu$L) were added, and the mixture was stirred at room temperature for 1 hour. To the reaction mixture, chloroform and a saturated aqueous solution of sodium bicarbonate were added. An organic layer was separated and dried over sodium sulfate, and the solvent was distilled off under reduced pressure. The obtained residue was suspended in ethyl acetate, and the deposit was collected by filtration to obtain a pale yellow solid of N-(4-(((8-(benzyloxy)-4-methyl-7-oxo-7,8-dihydropyrido[2,3-d]pyrimidin-5-yl)amino)methyl)phenyl)-N-(methylsulfonyl)methanesulfonamide (19 mg).
$^1$H-NMR (DMSO-$d_6$) $\delta$ value: 3.04 (s, 3H), 3.52 (s, 6H), 4.54-4.59 (m, 2H), 5.08 (s, 2H), 5.46 (s, 1H), 7.00-7.08 (m, 1H), 7.36-7.47 (m, 3H), 7.47-7.66 (m, 6H), 8.90 (s, 1H).

Reference Example 186

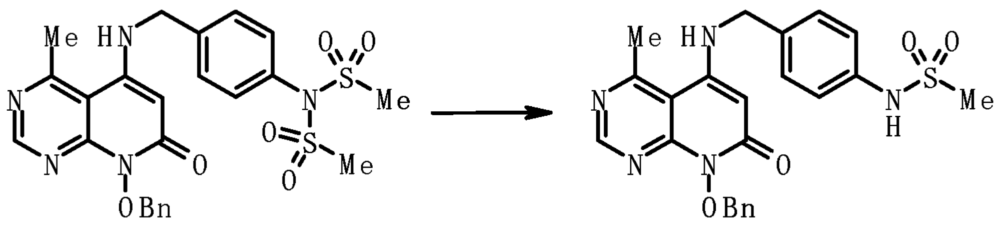

To a suspension of N-(4-(((8-(benzyloxy)-4-methyl-7-oxo-7,8-dihydropyrido[2,3-d]pyrimidin-5-yl)amino)methyl)phenyl)-N-(methylsulfonyl)methanesulfonamide (18 mg) in THF (1.0 mL), a 2 mol/L aqueous sodium hydroxide solution (0.20 mL) was added, and the mixture was stirred at room temperature for 4 hours. The solvent in the reaction mixture was distilled off under reduced pressure. To the obtained residue, a saturated aqueous solution of sodium bicarbonate and ethyl acetate were added, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was suspended in a mixed solvent of 2-propanol and diisopropyl ether, and the deposit was collected by filtration to obtain a pale yellow solid of N-(4-(((8-(benzyloxy)-4-methyl-7-oxo-7,8-dihydropyrido[2,3-d]pyrimidin-5-yl)amino)methyl)phenyl)methanesulfonamide (12 mg).
$^1$H-NMR ($CD_3OD$) δ value: 2.95 (s, 3H), 3.05 (s, 3H), 4.51 (s, 2H), 5.18 (s, 2H), 5.60 (s, 1H), 7.24-7.30 (m, 2H), 7.32-7.44 (m, 5H), 7.58-7.64 (m, 2H), 8.86 (s, 1H).

Reference Example 187

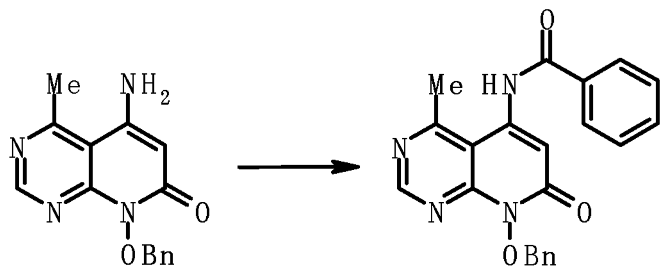

To a suspension of 5-amino-8-(benzyloxy)-4-methylpyrido[2,3-d]pyrimidin-7(8H)-one (10 mg) in methylene chloride (1.0 mL), triethylamine (7 μL) and benzoyl chloride (5 μL) were added, and the mixture was stirred at room temperature for 30 minutes. To the reaction mixture, triethylamine (7 μL), benzoyl chloride (5 μL) and 4-(dimethylamino)pyridine (4 mg) were added, and the mixture was stirred at room temperature for 4 hours. To the reaction mixture, methylene chloride (2.0 mL), triethylamine (15 μL) and benzoyl chloride (5 μL) were added, and the mixture was stirred at room temperature for 4 hours. To the reaction mixture, triethylamine (15 μL) and benzoyl chloride (5 μL) were added, and the mixture was stirred at room temperature for 3 hours. The reaction mixture was purified by silica gel column chromatography [Kanto Chemical Co., Inc., silica gel 60 (spherical), eluent: 100-93% chloroform/methanol] and then purified by silica gel column chromatography [Kanto Chemical Co., Inc., silica gel 60 (spherical), eluent: 80-20% hexane/ethyl acetate] to obtain a white solid of N-(8-(benzyloxy)-4-methyl-7-oxo-7,8-dihydropyrido[2,3-d]pyrimidin-5-yl)benzamide (4 mg). $^1$H-NMR ($CDCl_3$) δ value: 3.02 (s, 3H), 5.29 (s, 2H), 7.35-7.44 (m, 3H), 7.54-7.71 (m, 6H), 7.88-7.94 (m, 2H), 8.36 (brs, 1H), 8.97 (s, 1H).

Reference Example 188

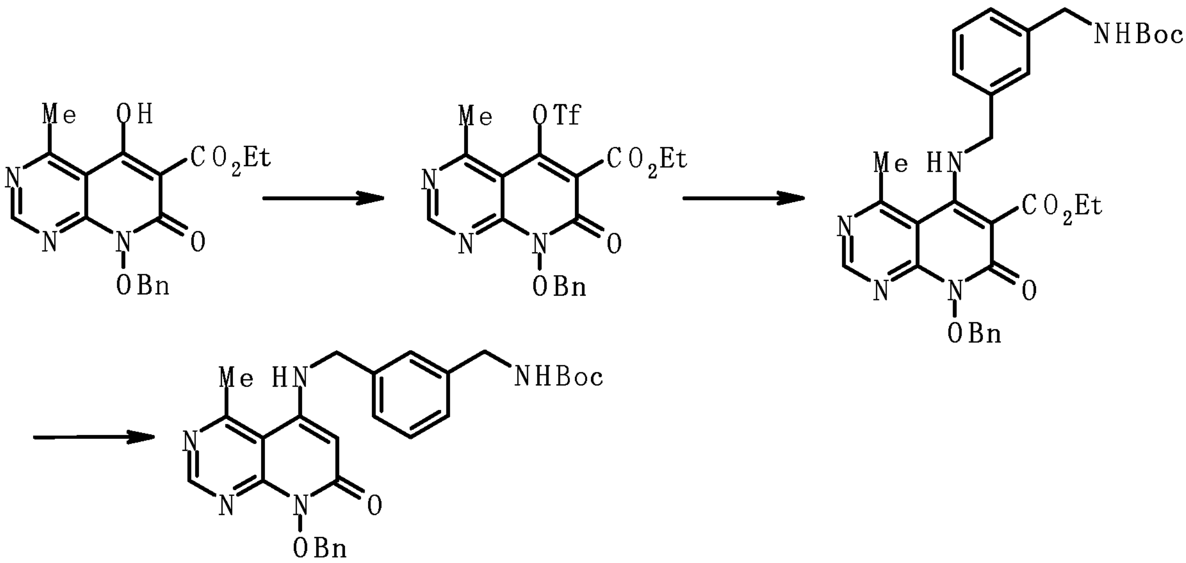

To a solution of ethyl 8-(benzyloxy)-5-hydroxy-4-methyl-7-oxo-7,8-dihydropyrido[2,3-d]pyrimidine-6-carboxylate (100 mg) in methylene chloride (5.0 mL), triethylamine (78 μL) was added, then trifluoromethanesulfonic anhydride (92 μL) was added dropwise under ice cooling, and the mixture was stirred at room temperature for 1 hour. To the reaction mixture, 1-(N-Boc-aminomethyl)-3-(aminomethyl)benzene (94 μL) and triethylamine (59 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-30% hexane/ethyl acetate] and then purified by silica gel column chromatography [Kanto Chemical Co., Inc., silica gel 60 (spherical), eluent: chloroform] to obtain a yellow solid (144 mg). To the obtained yellow solid (143 mg), ethanol (4.0 mL) and a 1 mol/L aqueous sodium hydroxide solution (4.0 mL) were added, and the mixture was heated with stirring in a nitrogen atmosphere at 55 to 60°C for 30 minutes, at 60 to 65°C for 30 minutes, and at 65 to 70°C for 3 hours and 30 minutes. After cooling of the reaction mixture, a saturated aqueous solution of sodium bicarbonate and ethyl acetate were added thereto, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 80-0% hexane/ethyl acetate] to obtain a yellow oil of tert-butyl (3-(((8-(benzyloxy)-4-methyl-7-oxo-7,8-dihydropyrido[2,3-d]pyrimidin-5-yl)amino)methyl)benzyl)carbamate (20 mg).
$^1$H-NMR ($CDCl_3$) δ value: 1.45 (s, 9H), 2.94 (s, 3H), 4.30-4.40 (m, 4H), 4.91 (brs, 1H), 5.25 (s, 2H), 5.31-5.38 (m, 1H), 5.74 (s, 1H), 7.20-7.43 (m, 7H), 7.65-7.71 (m, 2H), 8.90 (s, 1H).

Reference Example 189

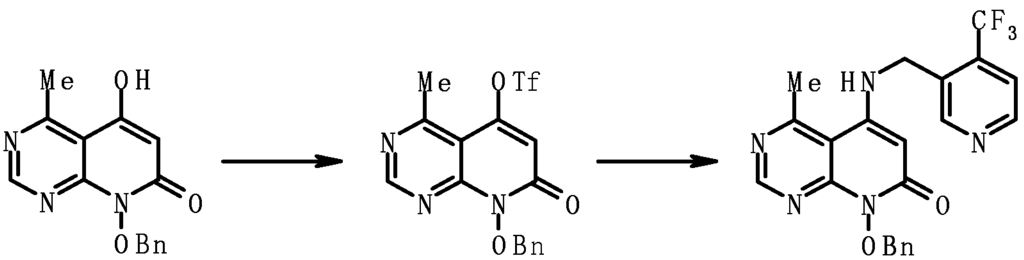

To a suspension of 8-(benzyloxy)-5-hydroxy-4-methylpyrido[2,3-d]pyrimidin-7(8H)-one (120 mg) in methylene chloride (6.0 mL), triethylamine (118 μL) was added, then trifluoromethanesulfonic anhydride (139 μL) was added dropwise under ice cooling, and the mixture was stirred at room temperature for 1 hour. The solvent in the reaction mixture was distilled off under reduced pressure. To the obtained residue, dioxane (6.0 mL), 1-(4-(trifluoromethyl)pyridin-3-yl)methanamine (110 mg) and N-ethyldiisopropylamine (184 μL) were added, and the mixture was heated to reflux

for 3 hours. After cooling of the reaction mixture, the solvent was distilled off under reduced pressure. The obtained residue was purified by silica gel column chromatography [Kanto Chemical Co., Inc., silica gel 60 (spherical), eluent: 80-0% hexane/ethyl acetate - 100-90% chloroform/methanol], and ethyl acetate and water were then added thereto. An organic layer was separated, washed with a 5% aqueous potassium carbonate solution and a saturated aqueous solution of sodium chloride in this order and then dried over sodium sulfate, and the solvent was distilled off under reduced pressure. The obtained residue was suspended in ethyl acetate, and the deposit was collected by filtration to obtain a pale yellow solid of 8-(benzyloxy)-4-methyl-5-(((4-(trifluoromethyl)pyridin-3-yl)methyl)amino)pyrido[2,3-d]pyrimidin-7(8H)-one (7 mg).
$^1$H-NMR ($CDCl_3$) δ value: 2.96 (s, 3H), 4.64-4.71 (m, 2H), 5.25 (s, 2H), 5.39-5.46 (m, 1H), 5.70 (s, 1H), 7.33-7.45 (m, 3H), 7.62-7.72 (m, 3H), 8.81-8.86 (m, 2H), 8.93 (s, 1H).

Reference Example 190

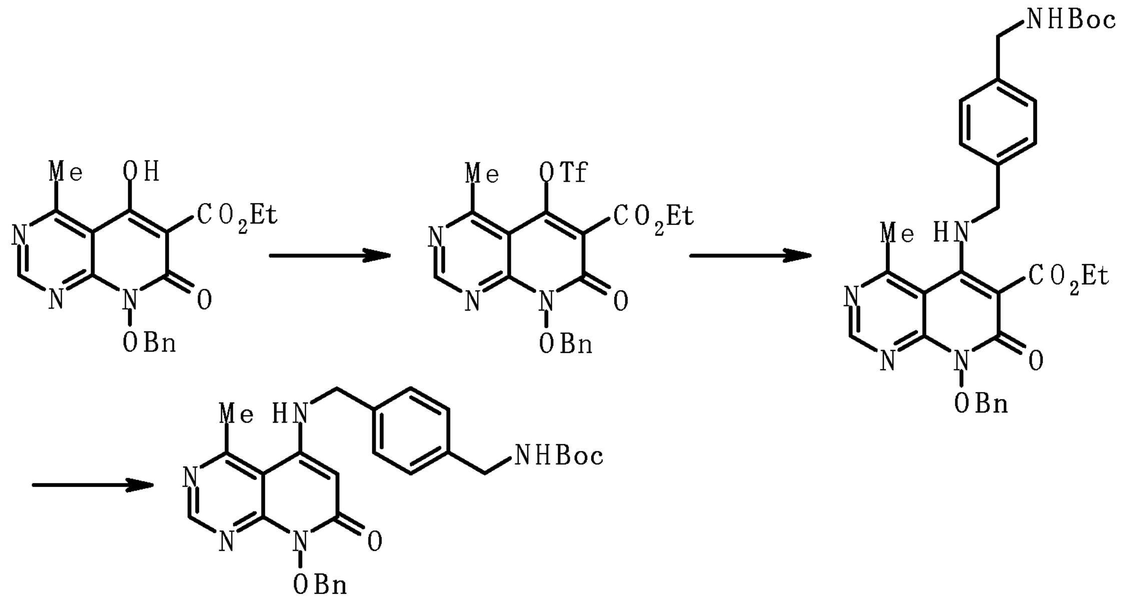

To a solution of ethyl 8-(benzyloxy)-5-hydroxy-4-methyl-7-oxo-7,8-dihydropyrido[2,3-d]pyrimidine-6-carboxylate (120 mg) in methylene chloride (6.0 mL), triethylamine (94 μL) was added, then trifluoromethanesulfonic anhydride (111 μL) was added dropwise under ice cooling, and the mixture was stirred at room temperature for 1 hour. To the reaction mixture, 1-(N-Boc-aminomethyl)-4-(aminomethyl)benzene (120 mg) and triethylamine (71 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-30% hexane/ethyl acetate] to obtain a brown oil (161 mg). To the obtained brown oil (160 mg), methanol (5.0 mL) and a 1 mol/L aqueous sodium hydroxide solution (5.0 mL) were added, and the mixture was heated with stirring at 55 to 60°C for 7 hours in a nitrogen atmosphere,. After cooling of the reaction mixture, a saturated aqueous solution of sodium bicarbonate and ethyl acetate were added thereto. An organic layer was separated, washed with a saturated aqueous solution of sodium chloride and then dried over sodium sulfate, and the solvent was distilled off under reduced pressure. The obtained residue was suspended in a mixed solvent of ethyl acetate and 2-propanol, and the deposit was collected by filtration to obtain a pale yellow solid of tert-butyl (4-(((8-(benzyloxy)-4-methyl-7-oxo-7,8-dihydropyrido[2,3-d]pyrimidin-5-yl)amino)methyl)benzyl)carbamate (68 mg).
$^1$H-NMR ($CDCl_3$) δ value: 1.47 (s, 9H), 2.93 (s, 3H), 4.32-4.38 (m, 4H), 4.89 (brs, 1H), 5.25 (s, 2H), 5.28-5.35 (m, 1H), 5.74 (s, 1H), 7.31-7.42 (m, 7H), 7.66-7.71 (m, 2H), 8.90 (s, 1H).

Reference Example 191

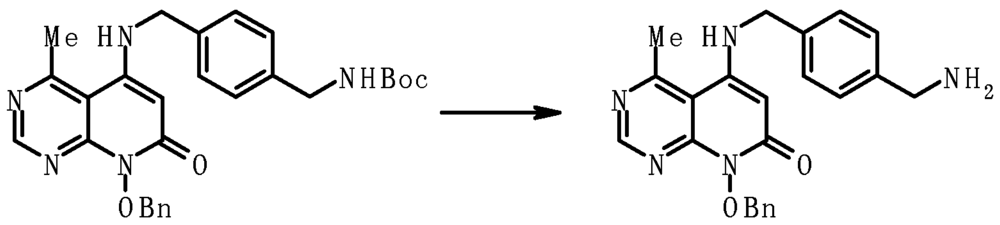

To a solution of tert-butyl (4-(((8-(benzyloxy)-4-methyl-7-oxo-7,8-dihydropyrido[2,3-d]pyrimidin-5-yl)amino)methyl)benzyl)carbamate (30 mg) in methylene chloride (5.0 mL), TFA (0.50 mL) was added under ice cooling, and the mixture was stirred at room temperature for 3 hours. To the reaction mixture, a 1 mol/L aqueous sodium hydroxide solution (10 mL) was added under ice cooling, and chloroform was then added at room temperature, followed by separation of an organic layer. An aqueous layer was extracted with chloroform, combined with the organic layer and dried over sodium sulfate, and the solvent was distilled off under reduced pressure to obtain a white solid of 5-((4-(aminomethyl)benzyl)amino)-8-(benzyloxy)-4-methylpyrido[2,3-d]pyrimidin-7(8H)-one (24 mg).
$^1$H-NMR ($CDCl_3$) δ value: 2.92 (s, 3H), 3.91 (s, 2H), 4.36 (d, J=4.6 Hz, 2H), 5.25 (s, 2H), 5.28-5.35 (m, 1H), 5.75 (s, 1H), 7.32-7.42 (m, 7H), 7.65-7.72 (m, 2H), 8.89 (s, 1H).

Reference Example 192

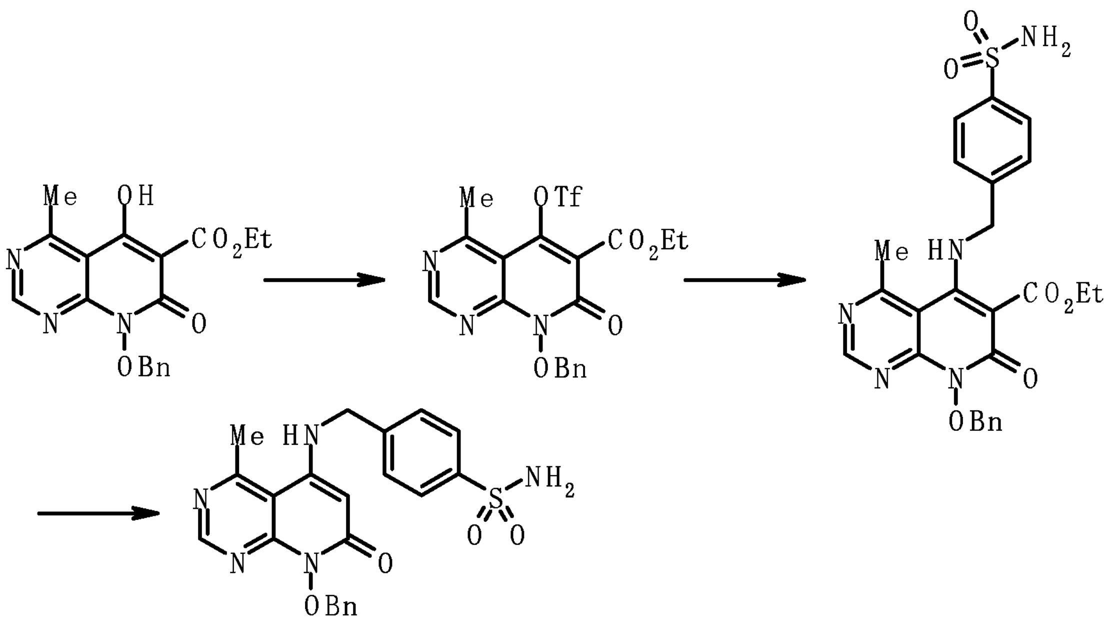

To a solution of ethyl 8-(benzyloxy)-5-hydroxy-4-methyl-7-oxo-7,8-dihydropyrido[2,3-d]pyrimidine-6-carboxylate (100 mg) in methylene chloride (5.0 mL), triethylamine (78 μL) was added, then trifluoromethanesulfonic anhydride (92 μL) was added dropwise under ice cooling, and the mixture was stirred at room temperature for 1 hour. To the reaction mixture, homosulfamine hydrochloride (188 mg) and triethylamine (235 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 100-95% chloroform/methanol] to obtain an orange solid (83 mg). To the obtained orange solid (82 mg), methanol (3.0 mL) and a 1 mol/L aqueous sodium hydroxide solution (3.0 mL) were added, and the mixture was heated with stirring in a nitrogen atmosphere at 60 to 65°C for 2 hours and 30 minutes and at 65 to 70°C for 2 hours. After cooling of the reaction mixture, a saturated aqueous solution of sodium bicarbonate and ethyl acetate were added thereto, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was suspended in ethyl acetate, and the deposit was collected by filtration to obtain a pale yellow solid of 4-(((8-(benzyloxy)-4-methyl-7-oxo-7,8-dihydropylido[2,3-d]pyrimidin-5-yl)amino)methyl)benzenesulfonamide (23 mg).

$^1$H-NMR (DMSO-$d_6$) δ value: 3.04 (s, 3H), 4.59 (d, J=5.4 Hz, 2H), 5.06 (s, 2H), 5.34 (s, 1H), 7.02-7.09 (m, 1H), 7.33 (s, 2H), 7.35-7.45 (m, 3H), 7.57-7.66 (m, 4H), 7.78-7.85 (m, 2H), 8.91 (s, 1H).

Reference Example 193

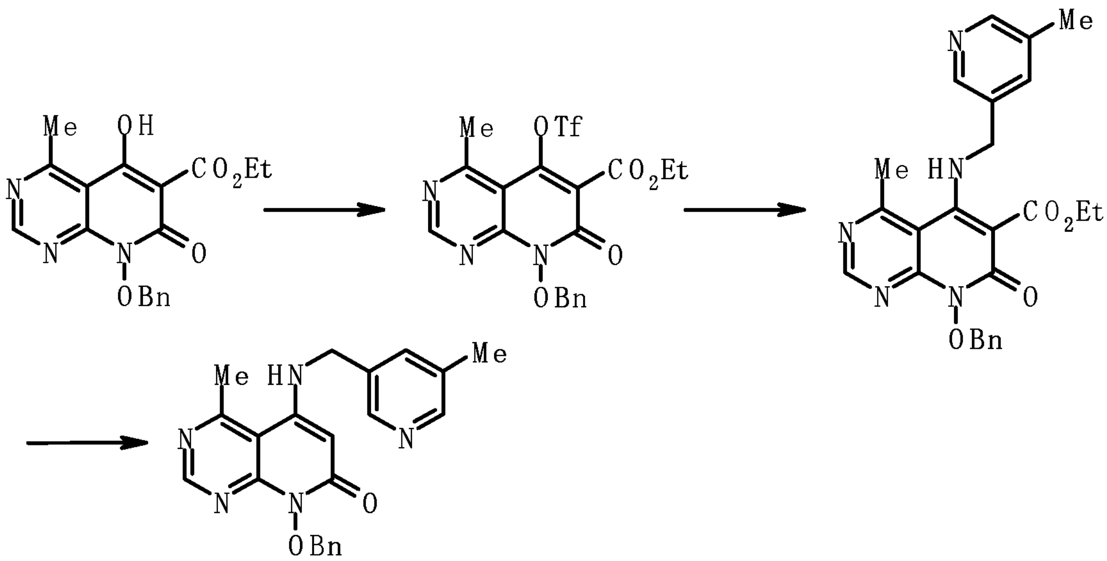

To a solution of ethyl 8-(benzyloxy)-5-hydroxy-4-methyl-7-oxo-7,8-dihydropyrido[2,3-d]pyrimidine-6-carboxylate (100 mg) in methylene chloride (5.0 mL), triethylamine (78 μL) was added, then trifluoromethanesulfonic anhydride (92 μL) was added dropwise under ice cooling, and the mixture was stirred at room temperature for 1 hour. To the reaction mixture, 1-(5-methylpyridin-3-yl)methanamine hydrochloride (67 mg) and triethylamine (118 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 100-96% chloroform/methanol] to obtain a brown oil (54 mg). To the obtained brown oil (53 mg), methanol (2.5 mL) and a 1 mol/L aqueous sodium hydroxide solution (2.5 mL) were added, and the mixture was heated with stirring in a nitrogen atmosphere at 60 to 65°C for 1 hour and at 65 to 70°C for 4 hours. After cooling of the reaction mixture, a saturated aqueous solution of sodium bicarbonate and ethyl acetate were added thereto. An organic layer was separated, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 100-90% chloroform/methanol] and then suspended in diisopropyl ether, and the deposit was collected by filtration to obtain a pale yellow solid of 8-(benzyloxy)-4-methyl-5-(((5-methylpyridin-3-yl)methyl)amino)pyrido[2,3-d]pyrimidin-7(8H)-one (8 mg).
$^1$H-NMR ($CDCl_3$) δ value: 2.39 (s, 3H), 2.95 (s, 3H), 4.39 (d, J=4.6 Hz, 2H), 5.25 (s, 2H), 5.29-5.35 (m, 1H), 5.73 (s, 1H), 7.34-7.43 (m, 3H), 7.49-7.54 (m, 1H), 7.64-7.71 (m, 2H), 8.44-8.50 (m, 2H), 8.91 (s, 1H).

Reference Example 194

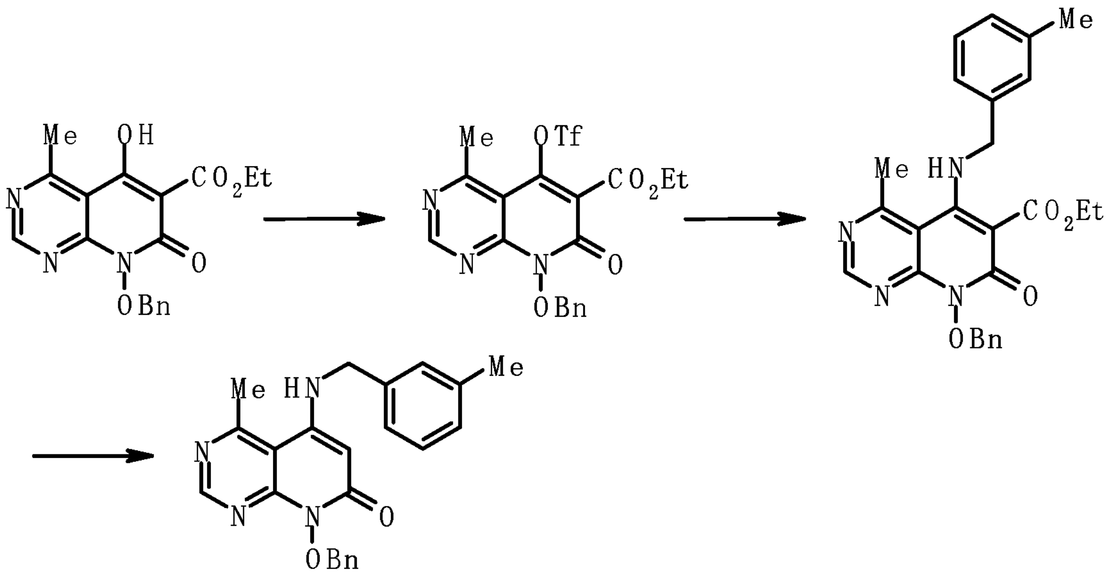

To a solution of ethyl 8-(benzyloxy)-5-hydroxy-4-methyl-7-oxo-7,8-dihydropyrido[2,3-d]pyrimidine-6-carboxylate (120 mg) in methylene chloride (6.0 mL), triethylamine (94 μL) was added, then trifluoromethanesulfonic anhydride (111 μL) was added dropwise under ice cooling, and the mixture was stirred at room temperature for 1 hour. To the reaction mixture, 3-methylbenzylamine (64 μL) and triethylamine (71 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-40% hexane/ethyl acetate] to obtain an orange oil (125 mg). To the obtained orange oil (125 mg), methanol (4.0 mL) and a 1 mol/L aqueous sodium hydroxide solution (4.0 mL) were added, and the mixture was heated with stirring at 65 to 70°C for 2 hours in a nitrogen atmosphere. After cooling of the reaction mixture, a saturated aqueous solution of sodium bicarbonate and ethyl acetate were added thereto. An organic layer was separated, washed with a saturated aqueous solution of sodium chloride and then dried over sodium sulfate, and the solvent was distilled off under reduced pressure. The obtained residue was suspended in a mixed solvent of ethyl acetate and diisopropyl ether, and the deposit was collected by filtration to obtain a pale yellow solid of 8-(benzyloxy)-4-methyl-5-((3-methylbenzyl)amino)pyrido[2,3-d]pyrimidin-7(8H)-one (43 mg).

$^1$H-NMR ($CDCl_3$) δ value: 2.39 (s, 3H), 2.93 (s, 3H), 4.33 (d, J=4.4 Hz, 2H), 5.25 (s, 2H), 5.29-5.36 (m, 1H), 5.75 (s, 1H), 7.15-7.24 (m, 3H), 7.24-7.34 (m, 1H), 7.34-7.42 (m, 3H), 7.65-7.72 (m, 2H), 8.89 (s, 1H).

Reference Example 195

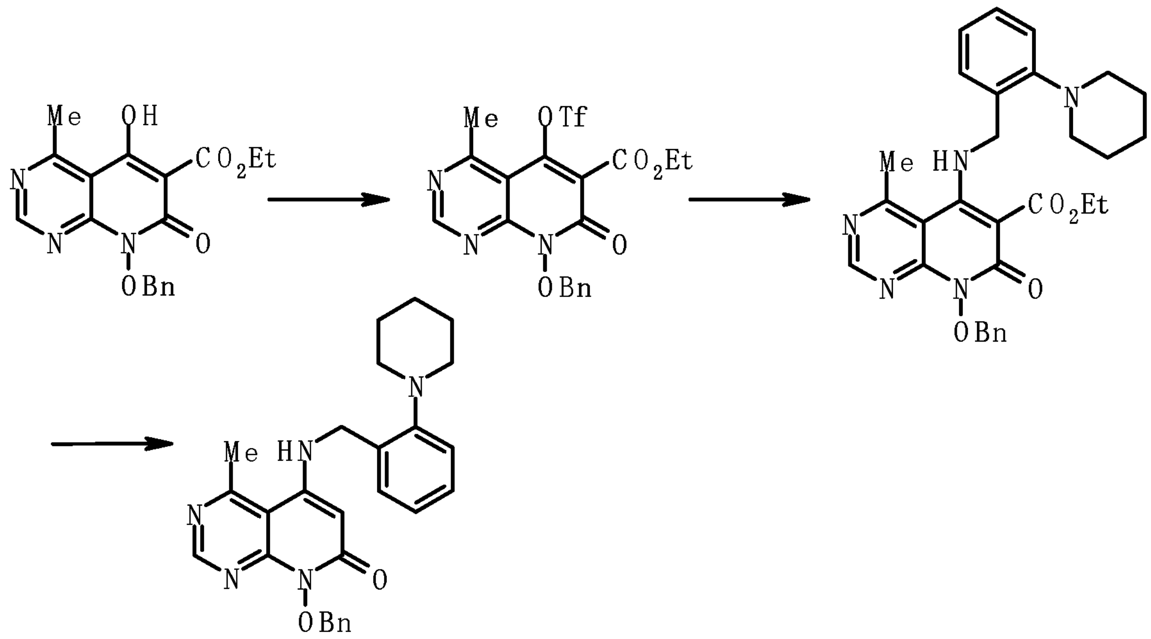

To a solution of ethyl 8-(benzyloxy)-5-hydroxy-4-methyl-7-oxo-7,8-dihydropyrido[2,3-d]pyrimidine-6-carboxylate (120 mg) in methylene chloride (6.0 mL), triethylamine (94 μL) was added, then trifluoromethanesulfonic anhydride (111 μL) was added dropwise under ice cooling, and the mixture was stirred at room temperature for 1 hour. To the reaction mixture, 1-(2-(piperidin-1-yl)phenyl)methanamine (96 mg) and triethylamine (71 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-60% hexane/ethyl acetate] to obtain a yellow oil (157 mg). To the obtained yellow oil (156 mg), methanol (4.0 mL) and a 1 mol/L aqueous sodium hydroxide solution (4.0 mL) were added, and the mixture was heated with stirring at 65 to 70°C for 2 hours in a nitrogen atmosphere,. After cooling of the reaction mixture, the deposit was collected by filtration to obtain a pale yellow solid of 8-(benzyloxy)-4-methyl-5-((2-(piperidin-1-yl)benzyl)amino)pyrido[2,3-d]pyrimidin-7(8H)-one (97 mg).

$^1$H-NMR ($CDCl_3$) δ value: 1.49-1.59 (m, 2H), 1.63-1.72 (m, 4H), 2.86-2.94 (m, 7H), 4.43 (d, J=4.6 Hz, 2H), 5.26 (s, 2H), 5.83 (s, 1H), 6.11-6.18 (m, 1H), 7.08-7.15 (m, 1H), 7.17-7.22 (m, 1H), 7.29-7.42 (m, 5H), 7.66-7.72 (m, 2H), 8.88 (s, 1H).

Reference Example 196

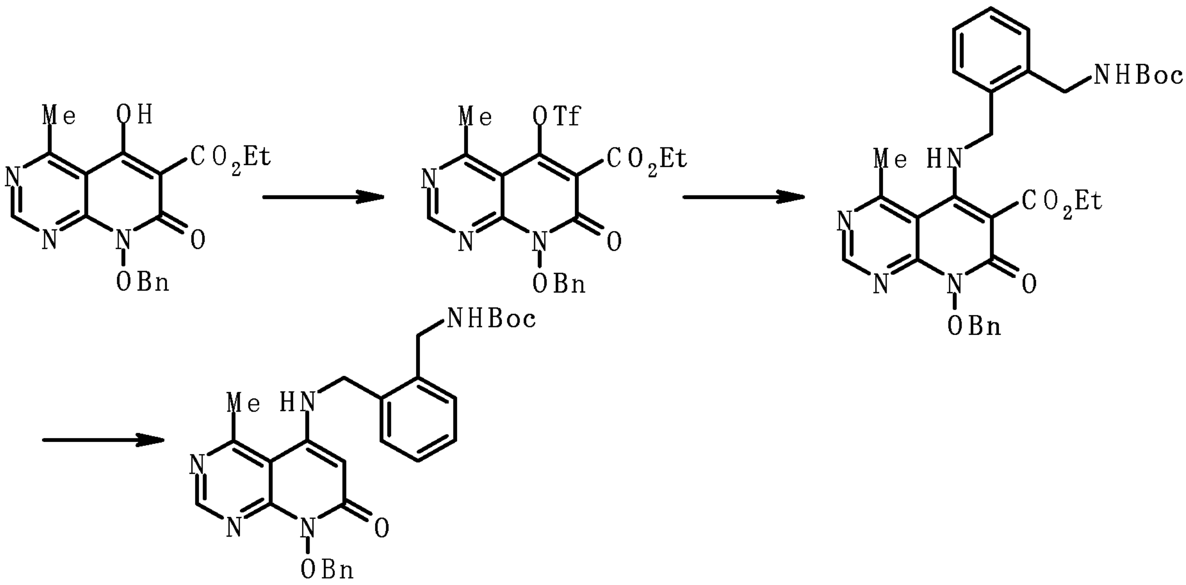

To a solution of ethyl 8-(benzyloxy)-5-hydroxy-4-methyl-7-oxo-7,8-dihydropyrido[2,3-d]pyrimidine-6-carboxylate (120 mg) in methylene chloride (6.0 mL), triethylamine (94 μL) was added, then trifluoromethanesulfonic anhydride (111 μL) was added dropwise under ice cooling, and the mixture was stirred at room temperature for 1 hour. To the reaction mixture, 1-(N-Boc-aminomethyl)-2-(aminomethyl)benzene (120 mg) and triethylamine (71 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-40% hexane/ethyl acetate] to obtain a yellow oil (163 mg). To the obtained yellow oil (162 mg), methanol (3.0 mL) and a 1 mol/L aqueous sodium hydroxide solution (3.0 mL) were added, and the mixture was heated with stirring at 60 to 65°C for 4 hours and 30 minutes in a nitrogen atmosphere. After cooling of the reaction mixture, a saturated aqueous solution of sodium bicarbonate and ethyl acetate were added thereto. An organic layer was separated, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 80-20% hexane/ethyl acetate] to obtain a yellow oil of tert-butyl (2-(((8-(benzyloxy)-4-methyl-7-oxo-7,8-dihydropyrido[2,3-d]pyrimidin-5-yl)amino)methyl)benzyl)carbamate (31 mg).

$^1$H-NMR ($CDCl_3$) δ value: 1.38 (s, 9H), 2.92 (s, 3H), 4.34-4.42 (m, 4H), 4.91-5.02 (m, 1H), 5.25 (s, 2H), 5.74 (brs, 1H), 5.78 (s, 1H), 7.29-7.42 (m, 7H), 7.66-7.72 (m, 2H), 8.88 (s, 1H).

Reference Example 197

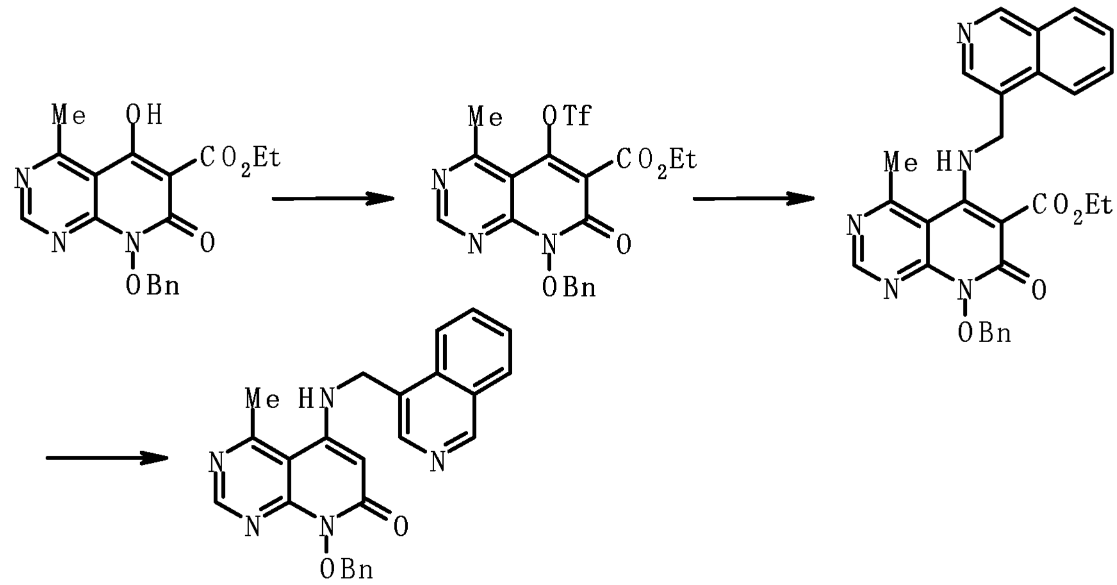

To a solution of ethyl 8-(benzyloxy)-5-hydroxy-4-methyl-7-oxo-7,8-dihydropyrido[2,3-d]pyrimidine-6-carboxylate (200 mg) in methylene chloride (10 mL), triethylamine (157 μL) was added, then trifluoromethanesulfonic anhydride (185 μL) was added dropwise under ice cooling, and the mixture was stirred at room temperature for 1 hour. To the reaction mixture, 1-(isoquinolin-4-yl)methanamine dihydrochloride (195 mg) and triethylamine (471 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-0% hexane/ethyl acetate], and ethyl acetate and a saturated aqueous solution of sodium bicarbonate were then added thereto. An organic layer was separated, washed with a saturated aqueous solution of sodium chloride and then dried over sodium sulfate, and the solvent was distilled off under reduced pressure to obtain an orange oil (232 mg). To the obtained orange oil (231 mg), methanol (5.0 mL) and a 1 mol/L aqueous sodium hydroxide solution (5.0 mL) were added, and the mixture was heated with stirring at 55 to 60°C for 9 hours in a nitrogen atmosphere. After cooling of the reaction mixture, a saturated aqueous solution of sodium bicarbonate and chloroform were added thereto, followed by separation of an organic layer. An aqueous layer was extracted with chloroform, combined with the organic layer and dried over sodium sulfate, and the solvent was distilled off under reduced pressure. The obtained residue was purified by silica gel column chromatography [Kanto Chemical Co., Inc., silica gel 60 (spherical), eluent: 50-0% hexane/ethyl acetate - 100-90% chloroform/methanol] and then suspended in a mixed solvent of ethyl acetate and diisopropyl ether, and the deposit was collected by filtration to obtain a pale yellow solid of 8-(benzyloxy)-5-(((isoquinolin-4-yl)methyl)amino)-4-methylpyrido[2,3-d]pyrimidin-7(8H)-one (33 mg).
$^1$H-NMR ($CDCl_3$) δ value: 2.72 (s, 3H), 4.76 (d, J=4.4 Hz, 2H), 5.20-5.26 (m, 1H), 5.29 (s, 2H), 5.94 (s, 1H), 7.34-7.44 (m, 3H), 7.67-7.77 (m, 3H), 7.80-7.87 (m, 1H), 7.95-8.01 (m, 1H), 8.08-8.14 (m, 1H), 8.60 (s, 1H), 8.90 (s, 1H), 9.33 (s, 1H).

Reference Example 198

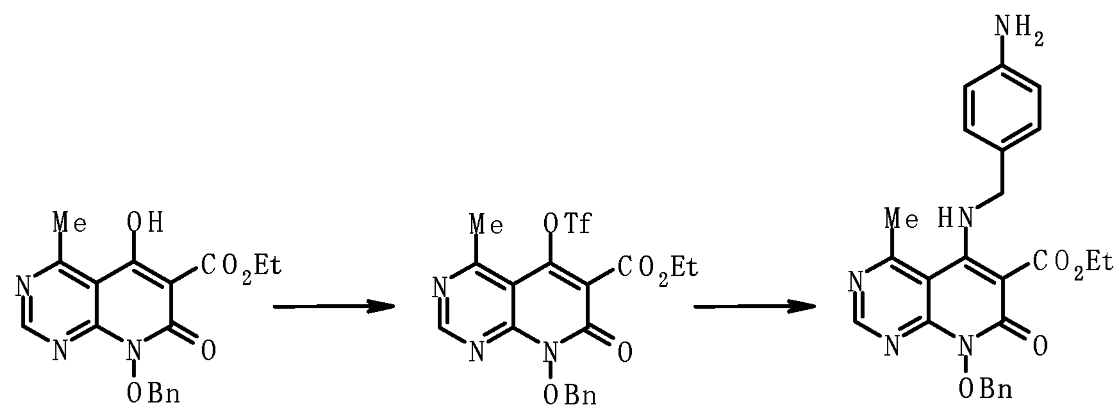

To a solution of ethyl 8-(benzyloxy)-5-hydroxy-4-methyl-7-oxo-7,8-dihydropyrido[2,3-d]pyrimidine-6-carboxylate (200 mg) in methylene chloride (10 mL), triethylamine (157 μL) was added, then trifluoromethanesulfonic anhydride (185 μL) was added dropwise under ice cooling, and the mixture was stirred at room temperature for 1 hour. To the reaction mixture, 4-aminobenzylamine (96 μL) and triethylamine (118 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-0% hexane/ethyl acetate] to obtain an orange oil of ethyl 5-((4-aminobenzyl)amino)-8-(benzyloxy)-4-methyl-7-oxo-7,8-dihydropyrido[2,3-d]pyrimidine-6-carboxylate (200 mg).
$^1$H-NMR ($CDCl_3$) δ value: 1.39 (t, J=7.2 Hz, 3H), 2.80 (s, 3H), 3.67-3.74 (m, 2H), 4.30 (d, J=5.4 Hz, 2H), 4.39 (q, J=7.1 Hz, 2H), 5.26 (s, 2H), 6.60-6.66 (m, 2H), 6.90-6.97 (m, 2H), 7.34-7.42 (m, 3H), 7.66-7.72 (m, 2H), 8.45-8.52 (m, 1H), 8.84 (s, 1H).

Reference Example 199

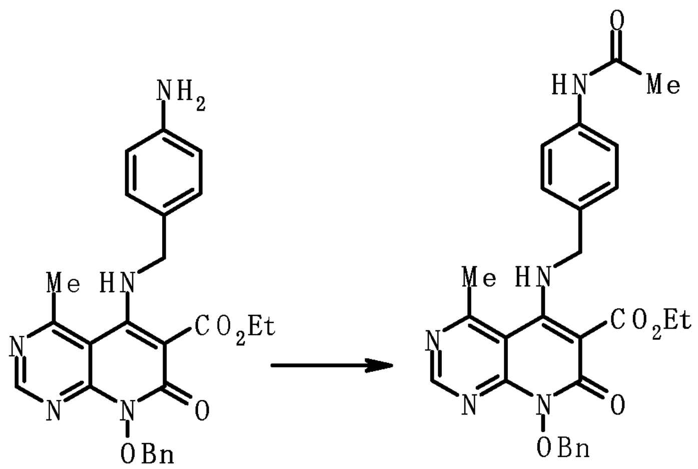

To a solution of ethyl 5-((4-aminobenzyl)amino)-8-(benzyloxy)-4-methyl-7-oxo-7,8-dihydropyrido[2,3-d]pyrimidine-6-carboxylate (50 mg) in methylene chloride (2.0 mL), triethylamine (23 μL) and acetyl chloride (9 μL) were added, and the mixture was stirred at room temperature for 1 hour. The solvent in the reaction mixture was distilled off under reduced pressure. The obtained residue was purified by silica gel column chromatography [Kanto Chemical Co., Inc., silica gel 60 (spherical), eluent: 100-95% chloroform/methanol] to obtain a pale yellow solid of ethyl 5-((4-(acetylamino)benzyl)amino)-8-(benzyloxy)-4-methyl-7-oxo-7,8-dihydropyrido[2,3-d]pyrimidine-6-carboxylate (54 mg).
$^1$H-NMR ($CDCl_3$) δ value: 1.39 (t, J=7.1 Hz, 3H), 2.18 (s, 3H), 2.81 (s, 3H), 4.33-4.42 (m, 4H), 5.26 (s, 2H), 7.06-7.20 (m, 3H), 7.32-7.41 (m, 3H), 7.45-7.53 (m, 2H), 7.64-7.72 (m, 2H), 8.72-8.79 (m, 1H), 8.85 (s, 1H).

Reference Example 200

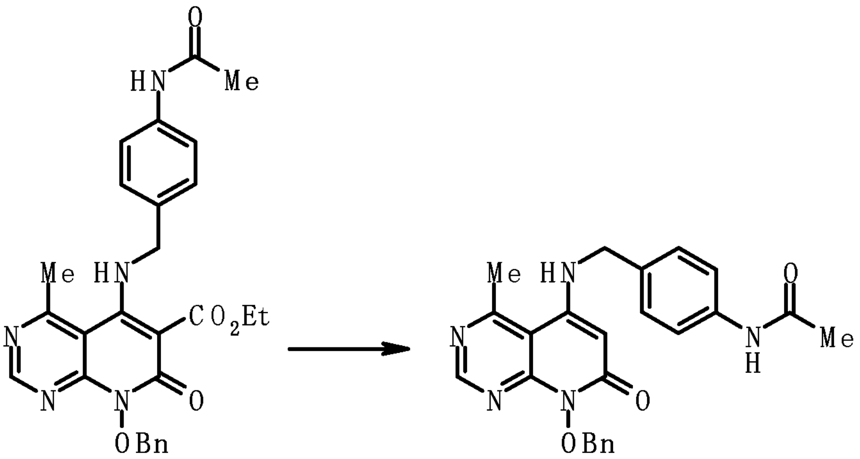

To ethyl 5-((4-(acetylamino)benzyl)amino)-8-(benzyloxy)-4-methyl-7-oxo-7,8-dihydropyrido[2,3-d]pyrimidine-6-carboxylate (53 mg), methanol (2.0 mL) and a 1 mol/L aqueous sodium hydroxide solution (2.0 mL) were added, and the mixture was heated with stirring at 60 to 65°C for 4 hours in a nitrogen atmosphere. After cooling of the reaction mixture, the deposit was collected by filtration to obtain a pale yellow solid of N-(4-(((8-(benzyloxy)-4-methyl-7-oxo-7,8-dihydropyrido[2,3-d]pyrimidin-5-yl)amino)methyl)phenyl)acetamide (23 mg).

$^1$H-NMR ($CDCl_3$) $\delta$ value: 2.20 (s, 3H), 2.91 (s, 3H), 4.32 (d, J=4.4 Hz, 2H), 5.25 (s, 2H), 5.26-5.33 (m, 1H), 5.74 (s, 1H), 7.30-7.42 (m, 5H), 7.52-7.59 (m, 2H), 7.65-7.71 (m, 2H), 8.89 (s, 1H).

Reference Example 201

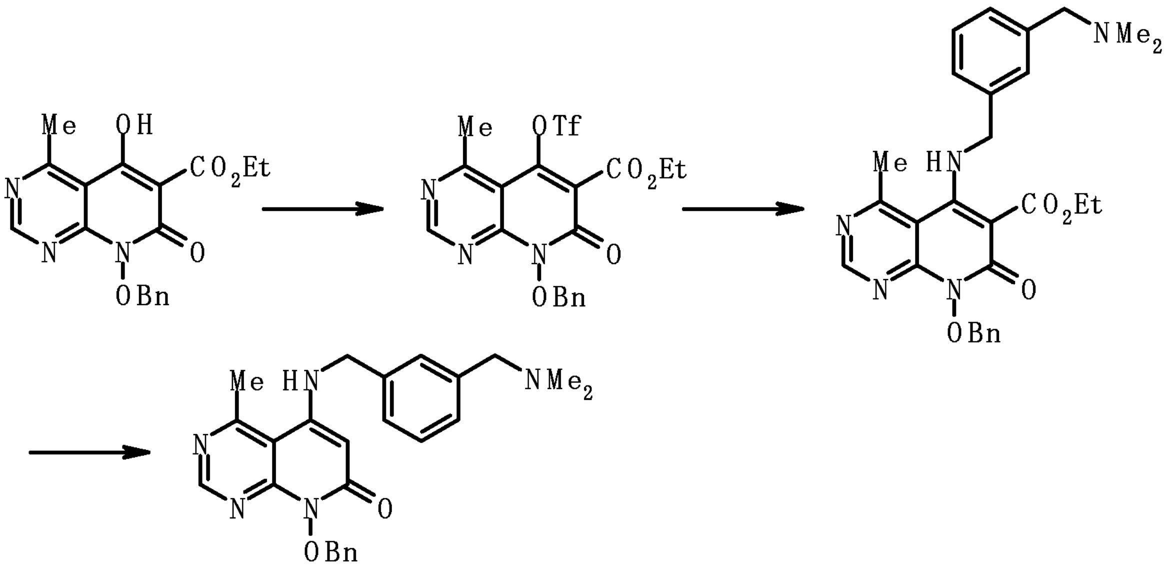

To a solution of ethyl 8-(benzyloxy)-5-hydroxy-4-methyl-7-oxo-7,8-dihydropyrido[2,3-d]pyrimidine-6-carboxylate (150 mg) in methylene chloride (7.5 mL), triethylamine (118 μL) was added, then trifluoromethanesulfonic anhydride (138 μL) was added dropwise under ice cooling, and the mixture was stirred at room temperature for 1 hour. To the reaction mixture, 1-(3-(aminomethyl)phenyl)-N,N-dimethylmethanamine (104 mg) and triethylamine (88 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 100-95% chloroform/methanol], and chloroform and a 0.5 mol/L aqueous sodium hydroxide solution were added thereto, followed by separation of an organic layer. An aqueous layer was extracted with chloroform, combined with the organic layer and dried over sodium sulfate, and the solvent was distilled off under reduced pressure to obtain a yellow oil (205 mg). To the obtained yellow oil (204 mg), methanol (4.0 mL) and a 1 mol/L aqueous sodium hydroxide solution (4.0 mL) were added, and the mixture was heated with stirring at 60 to 65°C for 8 hours in a

nitrogen atmosphere. After cooling of the reaction mixture, water and chloroform were added thereto, followed by separation of an organic layer. An aqueous layer was extracted with chloroform, combined with the organic layer and dried over sodium sulfate, and the solvent was distilled off under reduced pressure. The obtained residue was purified by silica gel column chromatography [Kanto Chemical Co., Inc., silica gel 60 (spherical), eluent: 100-90% chloroform/methanol] to obtain a white solid of 8-(benzyloxy)-5-((3-((dimethylamino)methyl)benzyl)amino)-4-methylpyrido[2,3-d]pyrimidin-7(8H)-one (71 mg). $^1$H-NMR ($CDCl_3$) δ value: 2.25 (s, 6H), 2.92 (s, 3H), 3.45 (s, 2H), 4.36 (d, J=4.6 Hz, 2H), 5.26 (s, 2H), 5.29-5.36 (m, 1H), 5.75 (s, 1H), 7.24-7.32 (m, 2H), 7.33-7.42 (m, 5H), 7.65-7.71 (m, 2H), 8.89 (s, 1H).

Reference Example 202

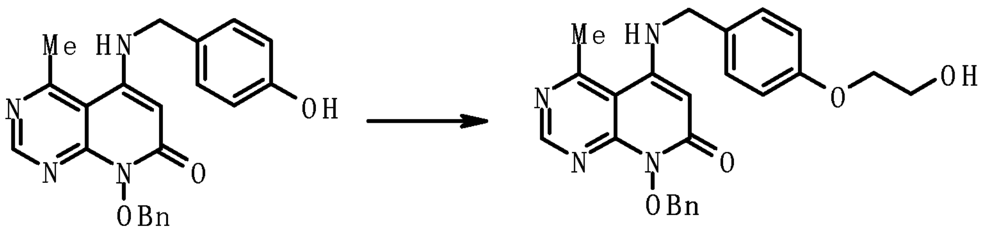

To a solution of 8-(benzyloxy)-5-((4-hydroxybenzyl)amino)-4-methylpyrido[2,3-d]pyrimidin-7(8H)-one (64 mg) in DMF (1.3 mL), potassium carbonate (114 mg) and 2-bromoethanol (35 μL) were added, and the mixture was heated with stirring at 70 to 80°C for 2 hours. After cooling of the reaction mixture, potassium carbonate (114 mg) and 2-bromoethanol (35 μL) were added, and the mixture was heated with stirring at 70 to 80°C for 3 hours. After cooling of the reaction mixture, potassium carbonate (114 mg) and 2-bromoethanol (35 μL) were added thereto, and the mixture was heated with stirring at 80 to 85°C for 7 hours. After cooling of the reaction mixture, ethyl acetate and a 10% aqueous citric acid solution were added thereto. An organic layer was separated, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 50-0% hexane/ethyl acetate - 100-90% chloroform/methanol] and then purified by silica gel column chromatography [Fuji Silysia Chemical Ltd., Chromatolex DIOL 60, eluent: 50-10% hexane/ethyl acetate] to obtain a pale yellow solid of 8-(benzyloxy)-5-((4-(2-hydroxyethoxy)benzyl)amino)-4-methylpyrido[2,3-d]pyrimidin-7(8H)-one (15 mg).
$^1$H-NMR ($CDCl_3$) δ value: 2.06-2.12 (m, 1H), 2.90 (s, 3H), 3.96-4.02 (m, 2H), 4.09-4.14 (m, 2H), 4.29 (d, J=4.4 Hz, 2H), 5.22-5.29 (m, 1H), 5.25 (s, 2H), 5.75 (s, 1H), 6.93-6.98 (m, 2H), 7.28-7.34 (m, 2H), 7.34-7.42 (m, 3H), 7.65-7.71 (m, 2H), 8.89 (s, 1H).

Reference Example 203

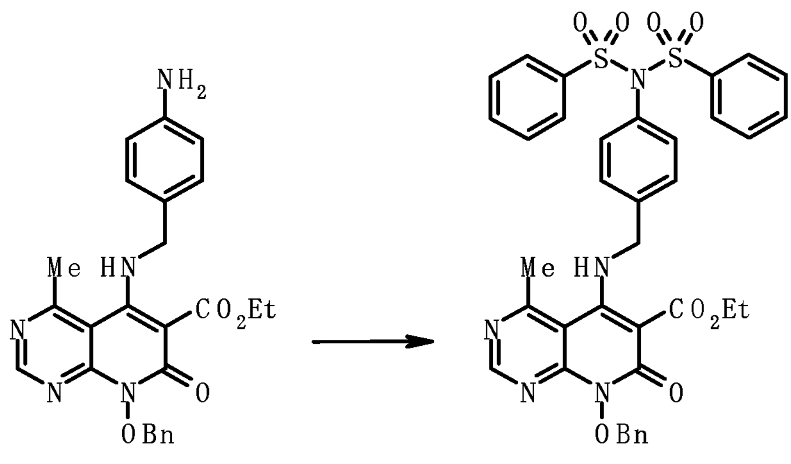

To a solution of ethyl 5-((4-aminobenzyl)amino)-8-(benzyloxy)-4-methyl-7-oxo-7,8-dihydropyrido[2,3-d]pyrimidine-6-carboxylate (60 mg) in methylene chloride (2.4 mL), triethylamine (27 μL) was added, benzenesulfonyl chloride (20 μL) was added under ice cooling, and the mixture was stirred at room temperature for 1 hour. To the reaction mixture, 4-(dimethylamino)pyridine (16 mg) was added, and the mixture was stirred at room temperature for 1 hour and 30 minutes. To the reaction mixture, triethylamine (27 μL) and benzenesulfonyl chloride (13 μL) were added, and the mixture was stirred at room temperature for 2 hours. To the reaction mixture, methanol was added, and the solvent was distilled off under reduced pressure. The obtained residue was purified by silica gel column chromatography [Kanto Chemical Co., Inc., silica gel 60 (spherical), eluent: 50-0% hexane/ethyl acetate] and then suspended in a mixed solvent of ethyl acetate and diisopropyl ether, and the deposit was collected by filtration to obtain a white solid of ethyl 8-(benzyloxy)-5-((4-(bis(phenylsulfonyl)amino)benzyl)amino)-4-methyl-7-oxo-7,8-dihydropyrido[2,3-d]pyrimidine-6-carboxylate (85 mg).
$^1$H-NMR ($CDCl_3$) δ value: 1.42 (t, J=6.7 Hz, 3H), 2.83 (s, 3H), 4.41 (q, J=7.0 Hz, 2H), 4.50 (d, J=5.6 Hz, 2H), 5.27 (s, 2H), 6.98-7.05 (m, 2H), 7.12-7.20 (m, 2H), 7.34-7.43 (m, 3H), 7.51-7.62 (m, 4H), 7.65-7.74 (m, 4H), 7.88-7.97 (m, 4H), 8.86 (s, 1H), 9.15-9.22 (m, 1H).

Reference Example 204

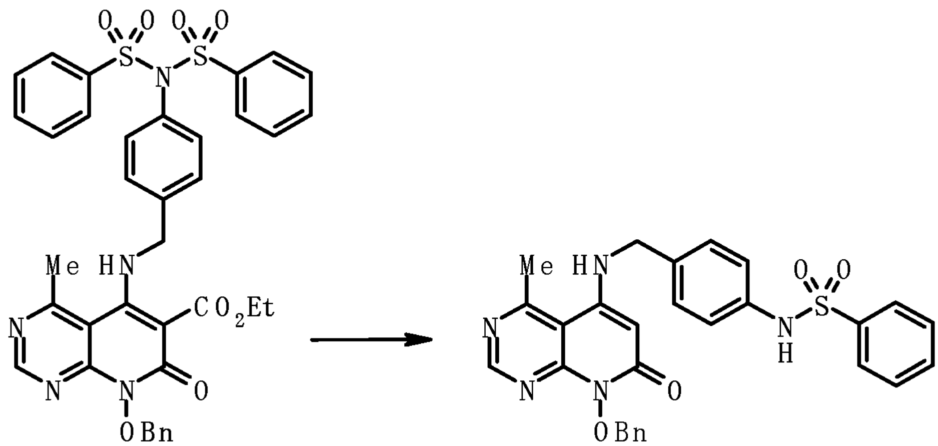

To ethyl 8-(benzyloxy)-5-((4-(bis(phenylsulfonyl)amino)benzyl)amino)-4-methyl-7-oxo-7,8-dihydropyrido[2,3-d]pyrimidine-6-carboxylate (83 mg), methanol (2.5 mL) and a 1 mol/L aqueous sodium hydroxide solution (2.5 mL) were added, and the mixture was heated with stirring at 60 to 65°C for 3 hours in a nitrogen atmosphere. After cooling of the reaction mixture, a saturated aqueous solution of sodium bicarbonate and ethyl acetate were added thereto. An organic layer was separated, washed with a saturated aqueous solution of sodium chloride and then dried over sodium sulfate, and the solvent was distilled off under reduced pressure. The obtained residue was suspended in a mixed solvent of ethyl acetate and diisopropyl ether, and the deposit was collected by filtration to obtain a white solid of N-(4-(((8-(benzyloxy)-4-methyl-7-oxo-7,8-dihydropyrido[2,3-d]pyrimidin-5-yl)amino)methyl)phenyl)benzenesulfonamide (41 mg).
$^1$H-NMR (DMSO-$d_6$) δ value: 2.97 (s, 3H), 4.37 (d, J=4.9 Hz, 2H), 5.06 (s, 2H), 5.34 (s, 1H), 6.83-6.89 (m, 1H), 7.04-7.10 (m, 2H), 7.25-7.31 (m, 2H), 7.35-7.45 (m, 3H), 7.50-7.64 (m, 5H), 7.71-7.78 (m, 2H), 8.88 (s, 1H).

Reference Example 205

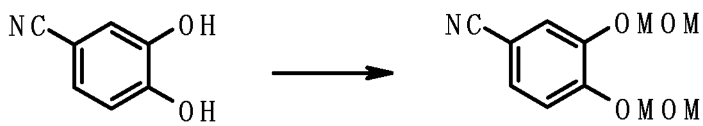

To a solution of 3,4-dihydroxybenzonitrile (700 mg) in acetone (7.0 mL), potassium carbonate (3.58 g) and chloromethyl methyl ether (1.57 mL) were added, and the mixture was heated to reflux for 30 minutes. After cooling of the reaction mixture, potassium carbonate (358 mg) and chloromethyl methyl ether (197 μL) were added, and the mixture was heated to reflux for 30 minutes. After cooling of the reaction mixture, ethyl acetate and water were added thereto.

An organic layer was separated, washed with a saturated aqueous solution of sodium chloride and then dried over magnesium sulfate, and the solvent was distilled off under reduced pressure. The obtained residue was purified by silica gel column chromatography [Kanto Chemical Co., Inc., silica gel 60 (spherical), eluent: 100-80% hexane/ethyl acetate] to obtain a colorless oil of 3,4-bis(methoxymethoxy)benzonitrile (1.16 g). $^1$H-NMR ($CDCl_3$) δ value: 3.51 (s, 3H), 3.52 (s, 3H), 5.25 (s, 2H), 5.29 (s, 2H), 7.22 (d, J=8.5 Hz, 1H), 7.30 (dd, J=8.3, 2.0 Hz, 1H), 7.45 (d, J=1.9 Hz, 1H).

Reference Example 206

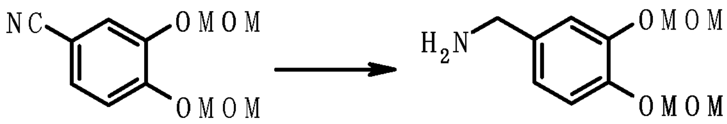

To a suspension of lithium aluminum hydride (592 mg) in THF (40 mL), a solution of 3,4-bis(methoxymethoxy)benzonitrile (1.16 g) in THF (4.0 mL) was added dropwise under ice cooling in a nitrogen atmosphere, and the mixture was stirred for 15 minutes under ice cooling and at room temperature for 2 hours. To the reaction mixture, a mixed solvent of water (0.6 mL) and THF (5 mL), a 2 mol/L aqueous sodium hydroxide solution (0.4 mL) and water (0.6 mL) were added dropwise in this order under ice cooling, and the mixture was stirred at room temperature for 1 hour and 30 minutes. Insoluble matter was filtered off, and the solvent was then distilled off under reduced pressure. To the obtained residue, diethyl ether and a 2 mol/L aqueous sodium hydroxide solution were added, followed by separation of an organic layer. An aqueous layer was extracted with diethyl ether, combined with the organic layer and dried over sodium sulfate, and the solvent was distilled off under reduced pressure to obtain a colorless oil of 1-(3,4-bis(methoxymethoxy)phenyl)methanamine (982 mg).
$^1$H-NMR ($CDCl_3$) δ value: 3.52 (s, 3H), 3.53 (s, 3H), 3.80 (s, 2H), 5.22 (s, 2H), 5.25 (s, 2H), 6.91 (dd, J=8.3, 1.5 Hz, 1H), 7.12 (d, J=8.0 Hz, 1H), 7.12 (d, J=1.9 Hz, 1H).

Reference Example 207

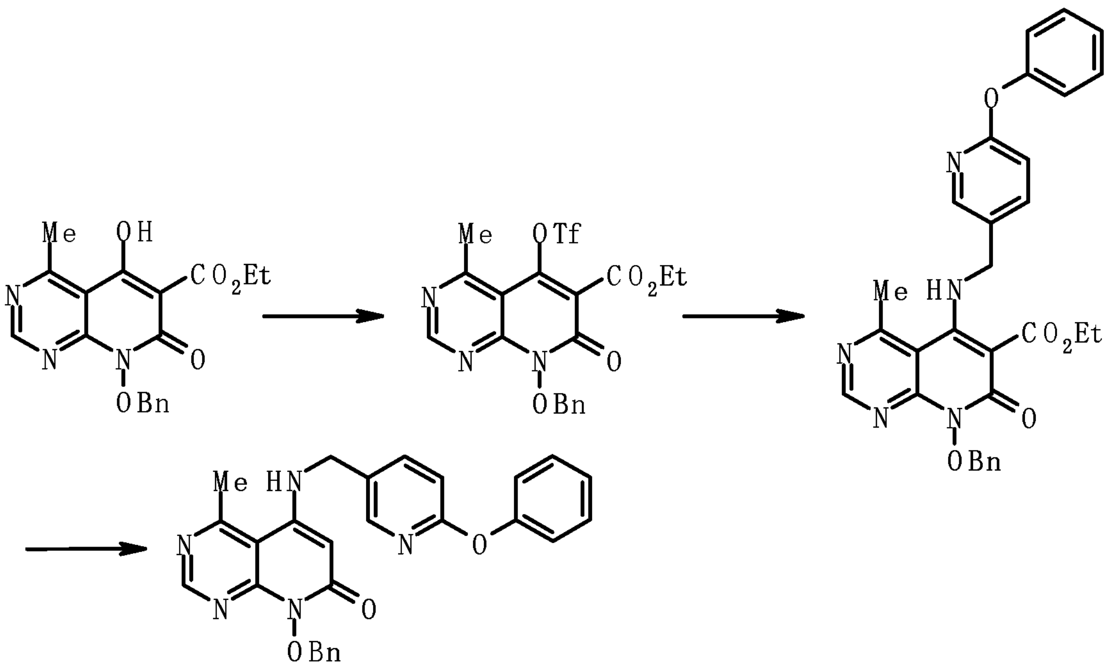

To a solution of ethyl 8-(benzyloxy)-5-hydroxy-4-methyl-7-oxo-7,8-dihydropyrido[2,3-d]pyrimidine-6-carboxylate (200 mg) in methylene chloride (10 mL), triethylamine (157 μL) was added, then trifluoromethanesulfonic anhydride (185 μL) was added dropwise under ice cooling, and the mixture was stirred at room temperature for 1 hour. To the

reaction mixture, 1-(6-phenoxypyridin-3-yl)methanamine (169 mg) and triethylamine (118 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-20% hexane/ethyl acetate] to obtain a yellow oil (267 mg). To the obtained yellow oil (266 mg), methanol (5.0 mL) and a 1 mol/L aqueous sodium hydroxide solution (5.0 mL) were added, and the mixture was heated with stirring at 55 to 60°C for 6 hours in a nitrogen atmosphere,. After cooling of the reaction mixture, the deposit was collected by filtration to obtain a white solid of 8-(benzyloxy)-4-methyl-5-(((6-phenoxypyridin-3-yl)methyl)amino)pyrido[2,3-d]pyrimidin-7(8H)-one (119 mg).
$^1$H-NMR ($CDCl_3$) δ value: 2.92 (s, 3H), 4.35 (d, J=4.6 Hz, 2H), 5.20-5.27 (m, 1H), 5.25 (s, 2H), 5.75 (s, 1H), 6.97 (d, J=8.6 Hz, 1H), 7.13-7.19 (m, 2H), 7.20-7.29 (m, 1H), 7.32-7.47 (m, 5H), 7.64-7.71 (m, 2H), 7.71 (dd, J=8.4, 2.6 Hz, 1H), 8.22 (d, J=2.2 Hz, 1H), 8.90 (s, 1H).

Reference Example 208

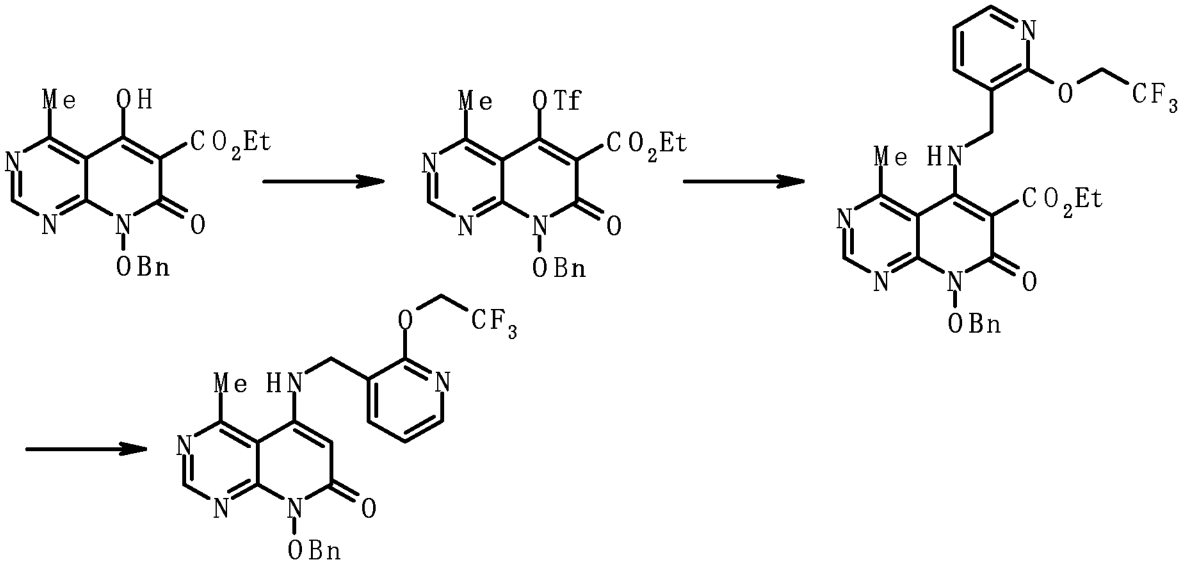

To a solution of ethyl 8-(benzyloxy)-5-hydroxy-4-methyl-7-oxo-7,8-dihydropyrido[2,3-d]pyrimidine-6-carboxylate (200 mg) in methylene chloride (10 mL), triethylamine (157 μL) was added, then trifluoromethanesulfonic anhydride (185 μL) was added dropwise under ice cooling, and the mixture was stirred at room temperature for 1 hour. To the reaction mixture, 1-(2-(2,2,2-trifluoroethoxy)pyridin-3-yl)methanamine (174 mg) and triethylamine (118 μL) were added, and the mixture was stirred at room temperature for 2 hours. The solvent in the reaction mixture was distilled off under reduced pressure. The obtained residue was purified by silica gel column chromatography [Kanto Chemical Co., Inc., silica gel 60 (spherical), eluent: 80-40% hexane/ethyl acetate] to obtain a yellow oil (280 mg). To the obtained yellow oil (279 mg), methanol (5.0 mL) and a 1 mol/L aqueous sodium hydroxide solution (5.0 mL) were added, and the mixture was heated with stirring at 55 to 60°C for 6 hours in a nitrogen atmosphere. After cooling of the reaction mixture, the deposit was collected by filtration to obtain a white solid of 8-(benzyloxy)-4-methyl-5-(((2-(2,2,2-trifluoroethoxy)pyridin-3-yl)methyl)amino)pyrido[2,3-d]pyrimidin-7(8H)-one (123 mg).
$^1$H-NMR ($CDCl_3$) δ value: 2.96 (s, 3H), 4.45 (d, J=5.8 Hz, 2H), 4.86 (q, J=8.5 Hz, 2H), 5.23 (s, 2H), 5.61-5.68 (m, 1H), 5.71 (s, 1H), 7.03 (dd, J=7.3, 5.1 Hz, 1H), 7.32-7.42 (m, 3H), 7.64-7.71 (m, 3H), 8.14 (dd, J=5.1, 1.7 Hz, 1H), 8.90 (s, 1H).

Reference Example 209

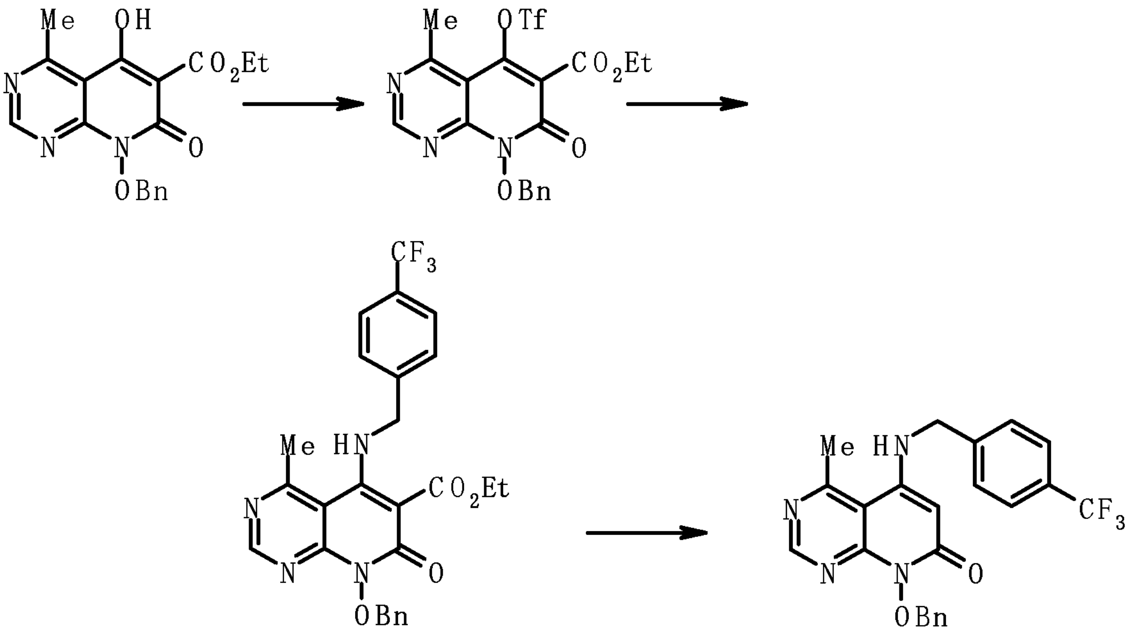

To ethyl 8-(benzyloxy)-5-hydroxy-4-methyl-7-oxo-7,8-dihydropyrido[2,3-d]pyrimidine-6-carboxylate (188 mg), methylene chloride (10 mL) and triethylamine (0.15 mL) were added, trifluoromethanesulfonic anhydride (0.17 mL) was added dropwise under ice cooling, and the mixture was stirred at room temperature for 30 minutes. To the reaction mixture, triethylamine (0.08 mL) was added, and the mixture was stirred at room temperature for 1 hour and 30 minutes. To the reaction mixture, 4-(trifluoromethyl)benzylamine (463 mg) was added, and the mixture was stirred at room temperature for 30 minutes. The solvent in the reaction mixture was distilled off under reduced pressure. The obtained residue was purified by silica gel column chromatography [Kanto Chemical Co., Inc., silica gel 60 (spherical), eluent: 100-90% chloroform/methanol] and then suspended in diisopropyl ether, and the deposit was collected by filtration to obtain a pale yellow solid (180 mg). To the obtained pale yellow solid (180 mg), methanol (9.0 mL) and a 1 mol/L aqueous sodium hydroxide solution (9.0 mL) were added, and the mixture was heated with stirring at 60 to 70°C for 30 minutes and heated to reflux for 2 hours. After cooling of the reaction mixture, the deposit was collected by filtration and washed with water and methanol to obtain a white solid of 8-(benzyloxy)-4-methyl-5-((4-(trifluoromethyl)benzyl)amino)pyrido[2,3-d]pyrimidin-7(8H)-one (70 mg).

$^1$H-NMR ($CDCl_3$) $\delta$ value: 2.98 (s, 3H), 4.48 (d, J=4.4 Hz, 2H), 5.24 (s, 2H), 5.37-5.43 (m, 1H), 5.69 (s, 1H), 7.33-7.43 (m, 3H), 7.47-7.53 (m, 2H), 7.64-7.72 (m, 4H), 8.91 (s, 1H).

Reference Example 210

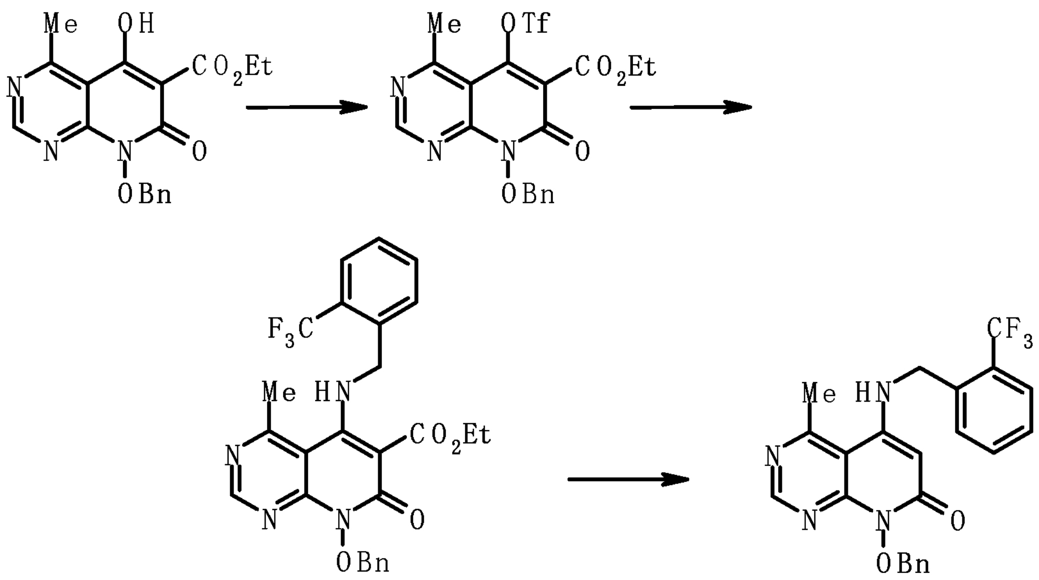

To ethyl 8-(benzyloxy)-5-hydroxy-4-methyl-7-oxo-7,8-dihydropyrido[2,3-d]pyrimidine-6-carboxylate (124 mg), methylene chloride (7.0 mL) and triethylamine (0.14 mL) were added, trifluoromethanesulfonic anhydride (0.11 mL) was added dropwise under ice cooling, and the mixture was stirred at room temperature for 30 minutes. To the reaction mixture, triethylamine (0.07 mL) was added, and the mixture was stirred at room temperature for 15 minutes. To the reaction mixture, 2-(trifluoromethyl)benzylamine (307 mg) was added, and the mixture was stirred at room temperature for 15 minutes. The solvent in the reaction mixture was distilled off under reduced pressure. The obtained residue was purified by silica gel column chromatography [Kanto Chemical Co., Inc., silica gel 60 (spherical), eluent: 100-90% chloroform/methanol] and then suspended in diisopropyl ether, and the deposit was collected by filtration to obtain an orange solid (63 mg). To the obtained orange solid (60 mg), methanol (3.0 mL) and a 1 mol/L aqueous sodium hydroxide solution (3.0 mL) were added, and the mixture was heated to reflux for 2 hours. The reaction mixture was cooled and then pH-adjusted to 7 by the dropwise addition of 1 mol/L hydrochloric acid. To the reaction mixture, ethyl acetate was added. An organic layer was separated, washed with a saturated aqueous solution of sodium chloride and then dried over magnesium sulfate, and the solvent was distilled off under reduced pressure. The obtained residue was suspended in a mixed solvent of ethyl acetate and diisopropyl ether, and the deposit was collected by filtration to obtain a light brown solid of 8-(benzyloxy)-4-methyl-5-((2-(trifluoromethyl)benzyl)amino)pyrido[2,3-d]pyrimidin-7(8H)-one (22 mg).
$^1$H-NMR ($CDCl_3$) δ value: 2.94 (s, 3H), 4.62 (d, J=4.8 Hz, 2H), 5.25 (s, 2H), 5.39-5.47 (m, 1H), 5.72 (s, 1H), 7.33-7.42 (m, 3H), 7.46-7.55 (m, 2H), 7.56-7.62 (m, 1H), 7.65-7.71 (m, 2H), 7.74-7.79 (m, 1H), 8.91 (s, 1H).

Reference Example 211

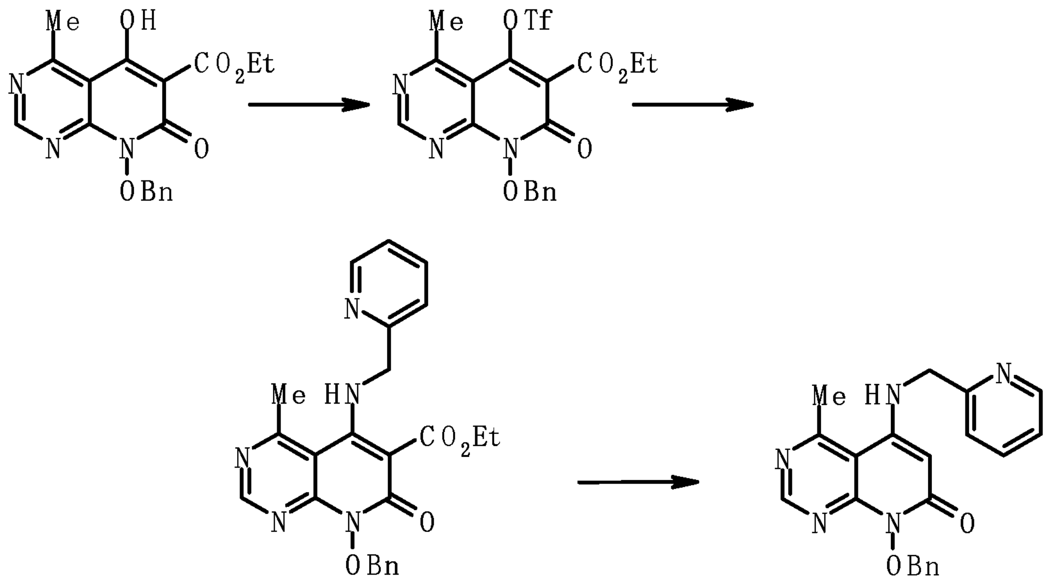

To ethyl 8-(benzyloxy)-5-hydroxy-4-methyl-7-oxo-7,8-dihydropyrido[2,3-d]pyrimidine-6-carboxylate (107 mg), methylene chloride (6.0 mL) and triethylamine (0.17 mL) were added, trifluoromethanesulfonic anhydride (0.10 mL) was added dropwise under ice cooling, and the mixture was stirred at room temperature for 15 minutes. To the reaction mixture, 2-(aminomethyl)pyridine (162 mg) was added, and the mixture was stirred at room temperature for 1 hour and 30 minutes. The solvent in the reaction mixture was distilled off under reduced pressure. The obtained residue was purified by silica gel column chromatography [Kanto Chemical Co., Inc., silica gel 60 (spherical), eluent: 60-0% hexane/ethyl acetate] and suspended in a mixed solvent of diisopropyl ether and ethyl acetate, and the deposit was collected by filtration to obtain a pale yellow solid (77 mg). To the obtained pale yellow solid (75 mg), methanol (3.0 mL) and a 1 mol/L aqueous sodium hydroxide solution (3.0 mL) were added, and the mixture was heated to reflux for 1 hour. The reaction mixture was cooled and then pH-adjusted to 8 by the dropwise addition of 6 mol/L hydrochloric acid, and ethyl acetate and water were added thereto. An organic layer was separated, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 100-90% chloroform/methanol] and then suspended in diisopropyl ether, and the deposit was collected by filtration to obtain a white solid of 8-(benzyloxy)-4-methyl-5-(((pyridin-2-yl)methyl)amino)pyrido[2,3-d]pyrimidin-7(8H)-one (9 mg).
$^1$H-NMR ($CDCl_3$) $\delta$ value: 3.17 (s, 3H), 4.49 (d, J=3.7 Hz, 2H), 5.28 (s, 2H), 5.70 (s, 1H), 7.20-7.43 (m, 5H), 7.50-7.57 (m, 1H), 7.67-7.73 (m, 2H), 7.77 (ddd, J=7.7, 7.7, 1.7 Hz, 1H), 8.59-8.64 (m, 1H), 8.92 (s, 1H).

Reference Example 212

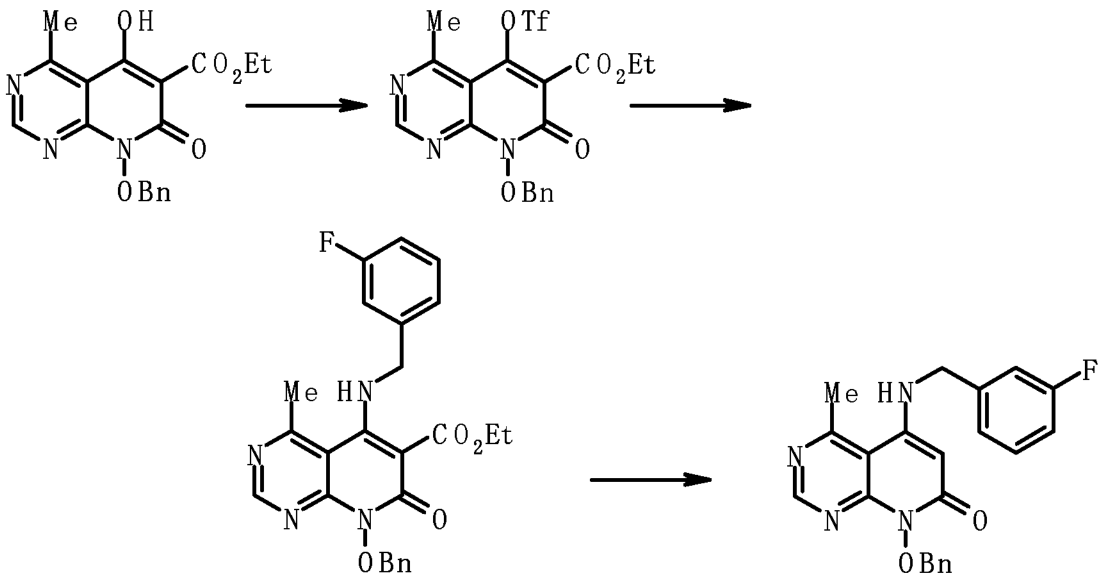

To ethyl 8-(benzyloxy)-5-hydroxy-4-methyl-7-oxo-7,8-dihydropyrido[2,3-d]pyrimidine-6-carboxylate (124 mg), methylene chloride (7.0 mL) and triethylamine (0.19 mL) were added, trifluoromethanesulfonic anhydride (0.11 mL) was added dropwise under ice cooling, and the mixture was stirred at room temperature for 15 minutes. To the reaction mixture, 3-fluorobenzylamine (219 mg) was added, and the mixture was stirred at room temperature for 1 hour. The solvent in the reaction mixture was distilled off under reduced pressure. The obtained residue was purified by silica gel column chromatography [Kanto Chemical Co., Inc., silica gel 60 (spherical), eluent: 70-30% hexane/ethyl acetate] and then suspended in diisopropyl ether, and the deposit was collected by filtration to obtain a yellow solid (92 mg). To the obtained yellow solid (90 mg), methanol (3.0 mL) and a 1 mol/L aqueous sodium hydroxide solution (3.0 mL) were added, and the mixture was heated with stirring at 70°C for 2 hours. The reaction mixture was cooled and then pH-adjusted to 7 by the dropwise addition of 6 mol/L hydrochloric acid, and ethyl acetate was added thereto. An organic layer was separated, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 70-10% hexane/ethyl acetate] and then suspended in diisopropyl ether, and the deposit was collected by filtration to obtain a white solid of 8-(benzyloxy)-5-((3-fluorobenzyl)amino)-4-methylpyrido[2,3-d]pyrimidin-7(8H)-one (32 mg).
$^1$H-NMR ($CDCl_3$) $\delta$ value: 2.96 (s, 3H), 4.40 (d, J=4.6 Hz, 2H), 5.25 (s, 2H), 5.34-5.41 (m, 1H), 5.70 (s, 1H), 7.03-7.10 (m, 2H), 7.14-7.18 (m, 1H), 7.34-7.43 (m, 4H), 7.65-7.70 (m, 2H), 8.91 (s, 1H).

Reference Example 213

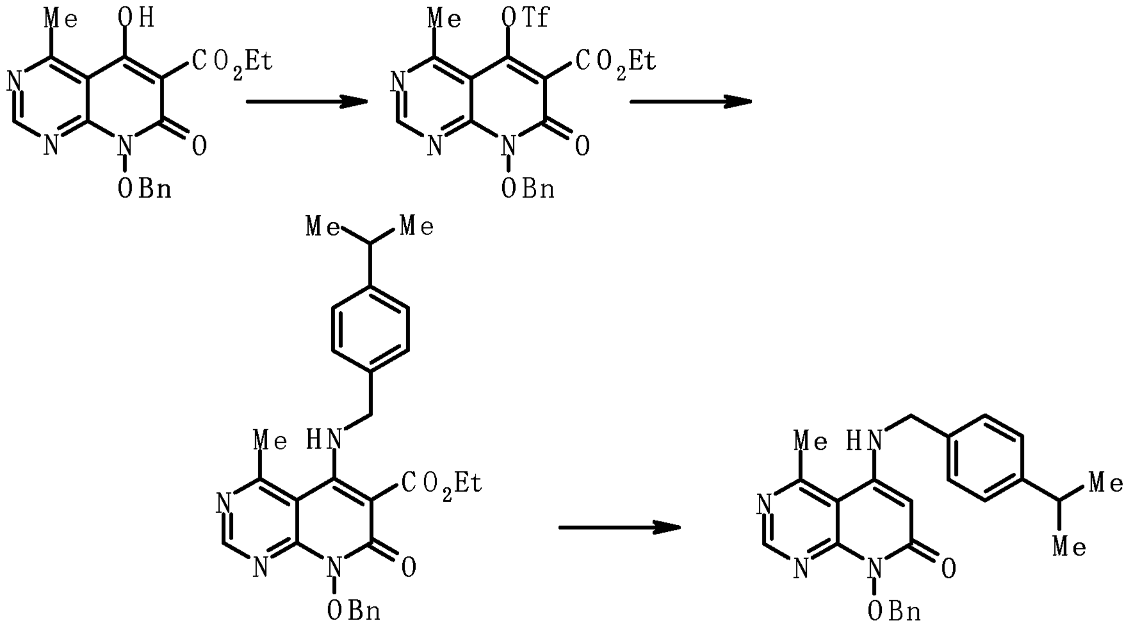

To ethyl 8-(benzyloxy)-5-hydroxy-4-methyl-7-oxo-7,8-dihydropyrido[2,3-d]pyrimidine-6-carboxylate (124 mg), methylene chloride (7.0 mL) and triethylamine (0.19 mL) were added, trifluoromethanesulfonic anhydride (0.11 mL) was added dropwise under ice cooling, and the mixture was stirred at room temperature for 10 minutes. To the reaction mixture, 4-isopropylbenzylamine (131 mg) was added, and the mixture was stirred at room temperature for 30 minutes. The solvent in the reaction mixture was distilled off under reduced pressure. The obtained residue was purified by silica gel column chromatography [Kanto Chemical Co., Inc., silica gel 60 (spherical), eluent: 50-30% hexane/ethyl acetate] and then suspended in diisopropyl ether, and the deposit was collected by filtration to obtain a yellow-red solid (81 mg). To the obtained yellow-red solid (78 mg), methanol (3.0 mL) and a 1 mol/L aqueous sodium hydroxide solution (3.0 mL) were added, and the mixture was heated with stirring at 70°C for 2 hours. After cooling of the reaction mixture, the deposit was collected by filtration and washed with water and methanol to obtain a white solid of 8-(benzyloxy)-5-((4-isopropylbenzyl)amino)-4-methylpyrido[2,3-d]pyrimidin-7(8H)-one (37 mg).
$^1$H-NMR ($CDCl_3$) δ value: 1.27 (d, J=6.8 Hz, 6H), 2.90-2.98 (m, 4H), 4.33 (d, J=4.6 Hz, 2H), 5.25 (s, 2H), 5.28-5.34 (m, 1H), 5.77 (s, 1H), 7.22-7.32 (m, 4H), 7.35-7.42 (m, 3H), 7.66-7.72 (m, 2H), 8.89 (s, 1H).

Reference Example 214

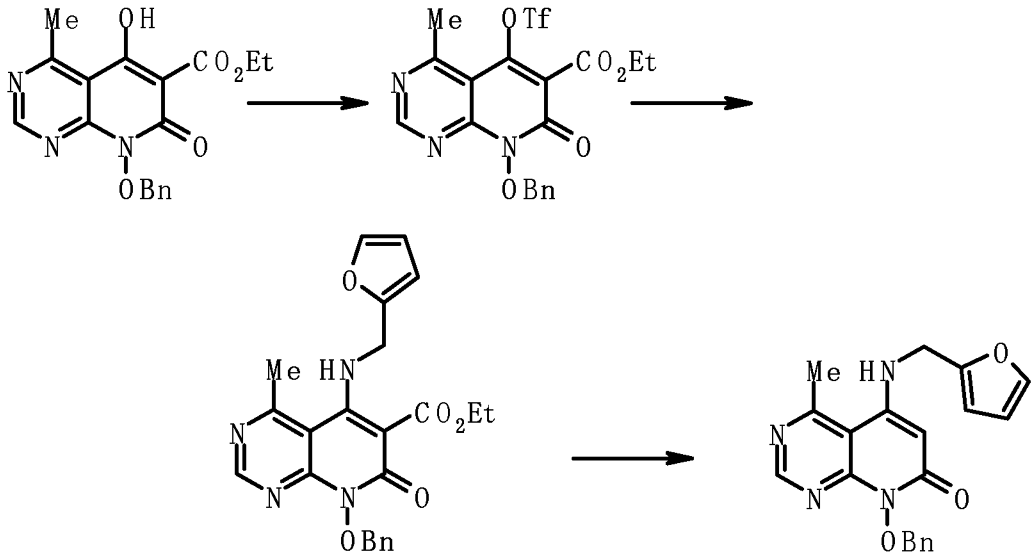

To ethyl 8-(benzyloxy)-5-hydroxy-4-methyl-7-oxo-7,8-dihydropyrido[2,3-d]pyrimidine-6-carboxylate (124 mg), methylene chloride (7.0 mL) and triethylamine (0.19 mL) were added, trifluoromethanesulfonic anhydride (0.11 mL) was added dropwise under ice cooling, and the mixture was stirred at room temperature for 10 minutes. To the reaction mixture, furfurylamine (51 mg) was added, and the mixture was stirred at room temperature for 15 minutes. The solvent in the reaction mixture was distilled off under reduced pressure. The obtained residue was purified by silica gel column chromatography [Kanto Chemical Co., Inc., silica gel 60 (spherical), eluent: 40-20% hexane/ethyl acetate] to obtain a brown oil. To the obtained brown oil, methanol (3.0 mL) and a 1 mol/L aqueous sodium hydroxide solution (3.0 mL) were added, and the mixture was heated with stirring at 70°C for 4 hours. After cooling of the reaction mixture, the deposit was collected by filtration and washed with water and methanol to obtain a white solid of 8-(benzyloxy)-5-((2-furylmethyl)amino)-4-methylpyrido[2,3-d]pyrimidin-7(8H)-one (33 mg).
$^1$H-NMR ($CDCl_3$) δ value: 2.97 (s, 3H), 4.39 (d, J=4.6 Hz, 2H), 5.25 (s, 2H), 5.40-5.46 (m, 1H), 5.79 (s, 1H), 6.37-6.43 (m, 2H), 7.34-7.42 (m, 3H), 7.43-7.46 (m, 1H), 7.65-7.71 (m, 2H), 8.90(s, 1H).

Reference Example 215

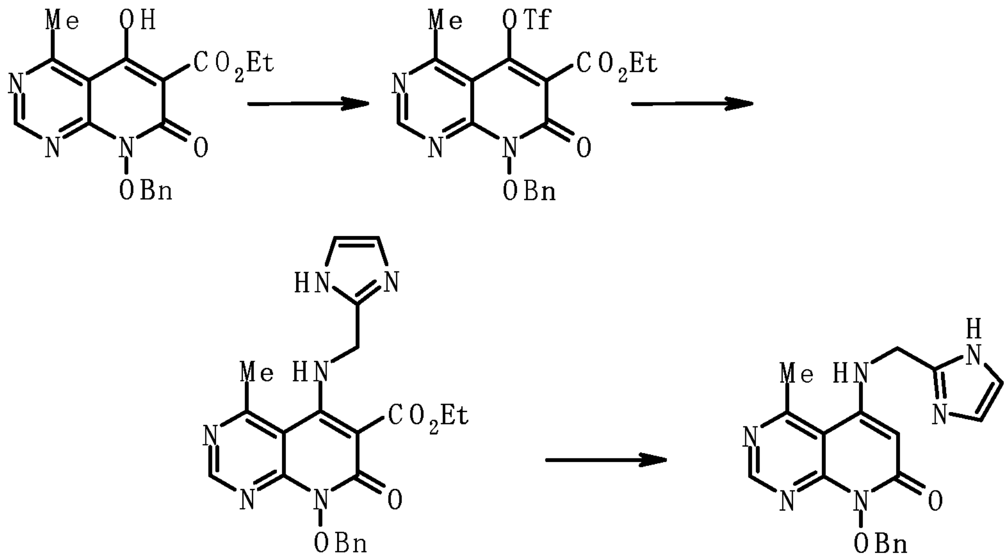

To ethyl 8-(benzyloxy)-5-hydroxy-4-methyl-7-oxo-7,8-dihydropyrido[2,3-d]pyrimidine-6-carboxylate (124 mg), methylene chloride (7.0 mL) and triethylamine (0.19 mL) were added, trifluoromethanesulfonic anhydride (0.11 mL) was added dropwise under ice cooling, and the mixture was stirred at room temperature for 10 minutes. To the reaction mixture, 1-(1H-imidazol-2-yl)methanamine dihydrochloride (89 mg) was added, and the mixture was stirred at room temperature for 15 minutes. To the reaction mixture, triethylamine (0.19 mL) was added, and the mixture was stirred at room temperature for 10 minutes. The solvent in the reaction mixture was distilled off under reduced pressure. The obtained residue was purified by silica gel column chromatography [Kanto Chemical Co., Inc., silica gel 60 (spherical), eluent: 95-90% chloroform/methanol] to obtain a brown oil. To the obtained brown oil, methanol (3.0 mL) and a 1 mol/L aqueous sodium hydroxide solution (3.0 mL) were added, and the mixture was heated with stirring at 70°C for 2 hours. The reaction mixture was cooled and then pH-adjusted to 7 by the dropwise addition of 1 mol/L hydrochloric acid, and ethyl acetate was added thereto. An organic layer was separated, washed with a saturated aqueous solution of sodium chloride and then dried over sodium sulfate, and the solvent was distilled off under reduced pressure. The obtained residue was suspended in a mixed solvent of ethyl acetate and diisopropyl ether, and the deposit was collected by filtration to obtain a white solid of 8-(benzyloxy)-5-(((1H-imidazol-2-yl)methyl)amino)-4-methylpyrido[2,3-d]pyrimidin-7(8H)-one (13 mg).
$^1$H-NMR (DMSO-$d_6$) δ value: 3.03 (s, 3H), 4.46 (d, J=4.9 Hz, 2H), 5.08 (s, 2H), 5.58 (s, 1H), 5.76 (d, J=1.2 Hz, 2H), 6.89-6.94 (m, 1H), 7.38-7.46 (m, 3H), 7.60-7.65 (m, 2H), 8.90 (s, 1H).

Reference Example 216

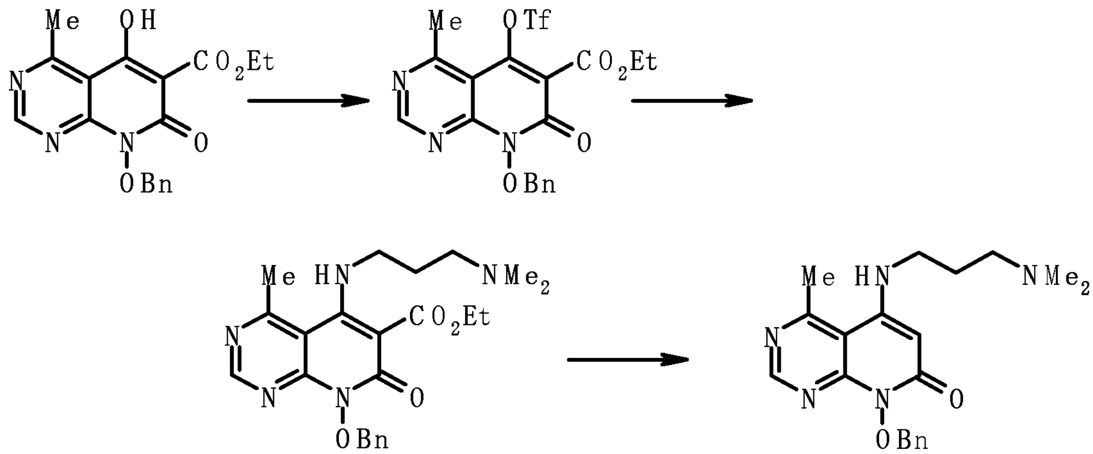

To ethyl 8-(benzyloxy)-5-hydroxy-4-methyl-7-oxo-7,8-dihydropyrido[2,3-d]pyrimidine-6-carboxylate (124 mg), methylene chloride (7.0 mL) and triethylamine (0.19 mL) were added, trifluoromethanesulfonic anhydride (0.11 mL) was added dropwise under ice cooling, and the mixture was stirred at room temperature for 10 minutes. To the reaction mixture, N,N-dimethylpropane-1,3-diamine (54 mg) was added, and the mixture was stirred at room temperature for 15 minutes. The solvent in the reaction mixture was distilled off under reduced pressure. The obtained residue was purified by silica gel column chromatography [Kanto Chemical Co., Inc., silica gel 60 (spherical), eluent: 95-90% chloroform/methanol] to obtain a brown oil (404 mg). To the obtained brown oil (404 mg), methanol (3.0 mL) and a 1 mol/L aqueous sodium hydroxide solution (3.0 mL) were added, and the mixture was heated with stirring at 70°C for 3 hours. After cooling of the reaction mixture, ethyl acetate was added thereto, and the mixture was pH-adjusted to 7 by the dropwise addition of 1 mol/L hydrochloric acid, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was purified by silica gel column chromatography [Kanto Chemical Co., Inc., silica gel 60 (spherical), eluent: 60-20% hexane/ethyl acetate] and then suspended in diisopropyl ether, and the deposit was collected by filtration to obtain a white solid of 8-(benzyloxy)-5-((3-(dimethylamino)propyl)amino)-4-methylpyrido[2,3-d]pyrimidin-7(8H)-one (33 mg).
$^1$H-NMR ($CDCl_3$) δ value: 1.86-1.94 (m, 2H), 2.25 (s, 6H), 2.51-2.57 (m, 2H), 2.92 (s, 3H), 3.22-3.28 (m, 2H), 5.25 (s, 2H), 5.62 (s, 1H), 7.20-7.45 (m, 4H), 7.67-7.72 (m, 2H), 8.86 (s, 1H).

Reference Example 217

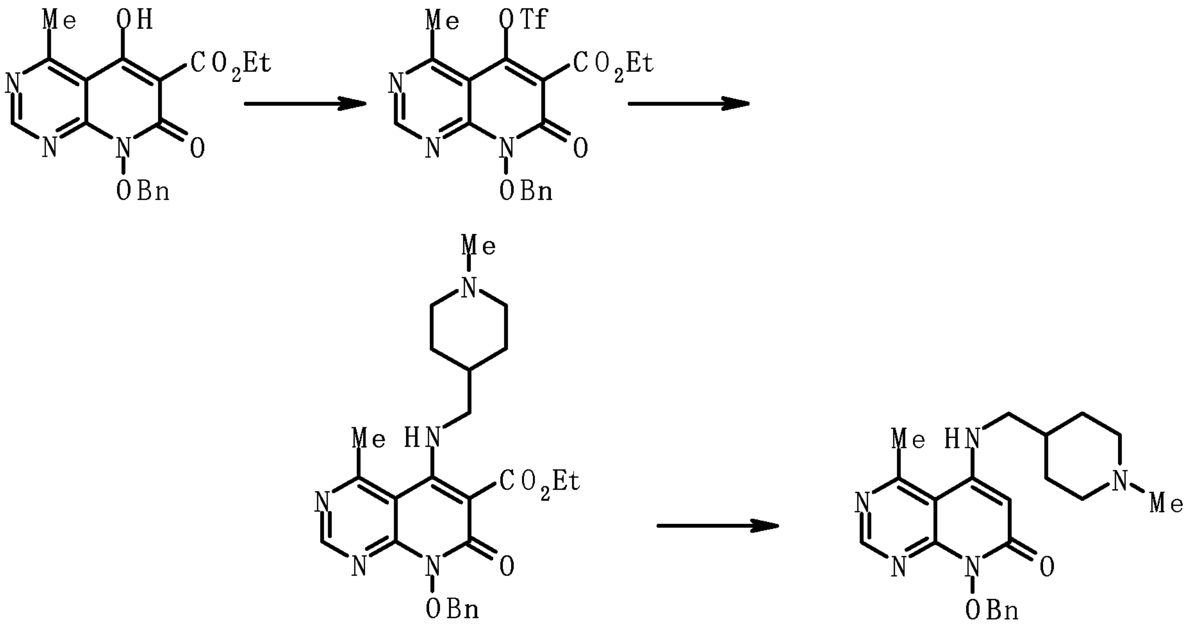

To ethyl 8-(benzyloxy)-5-hydroxy-4-methyl-7-oxo-7,8-dihydropyrido[2,3-d]pyrimidine-6-carboxylate (124 mg), methylene chloride (7.0 mL) and triethylamine (0.19 mL) were added, trifluoromethanesulfonic anhydride (0.11 mL) was added dropwise under ice cooling, and the mixture was stirred at room temperature for 10 minutes. To the reaction mixture, 1-(1-methylpiperidin-4-yl)methanamine (67 mg) was added, and the mixture was stirred at room temperature for 20 minutes. The solvent in the reaction mixture was distilled off under reduced pressure. The obtained residue was purified by silica gel column chromatography [Kanto Chemical Co., Inc., silica gel 60 (spherical), eluent: 95-85% chloroform/methanol] to obtain a brown oil (373 mg). To the obtained brown oil (373 mg), methanol (3.0 mL) and a 1 mol/L aqueous sodium hydroxide solution (3.0 mL) were added, and the mixture was heated with stirring at 70°C for 2 hours. After cooling of the reaction mixture, ethyl acetate was added thereto, and the mixture was pH-adjusted to 7 by the dropwise addition of 1 mol/L hydrochloric acid, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was purified by silica gel column chromatography [Fuji Silysia Chemical Ltd. DNH silica, eluent: 50-10% hexane/ethyl acetate] and then suspended in diisopropyl ether, and the deposit was collected by filtration to obtain a white solid of 8-(benzyloxy)-4-methyl-5-(((1-methylpiperidin-4-yl)methyl)amino)pyrido[2,3-d]pyrimidin-7(8H)-one (4 mg).
$^1$H-NMR ($CDCl_3$) δ value: 1.37-1.51 (m, 2H), 1.70-1.84 (m, 3H), 1.94-2.03 (m, 2H), 2.31 (s, 3H), 2.89-2.96 (m, 2H), 2.98 (s, 3H), 3.10-3.16 (m, 2H), 5.11-5.16 (m, 1H), 5.25 (s, 2H), 5.67 (s, 1H), 7.32-7.42 (m, 3H), 7.66-7.70 (m, 2H), 8.89 (s, 1H).

Reference Example 218

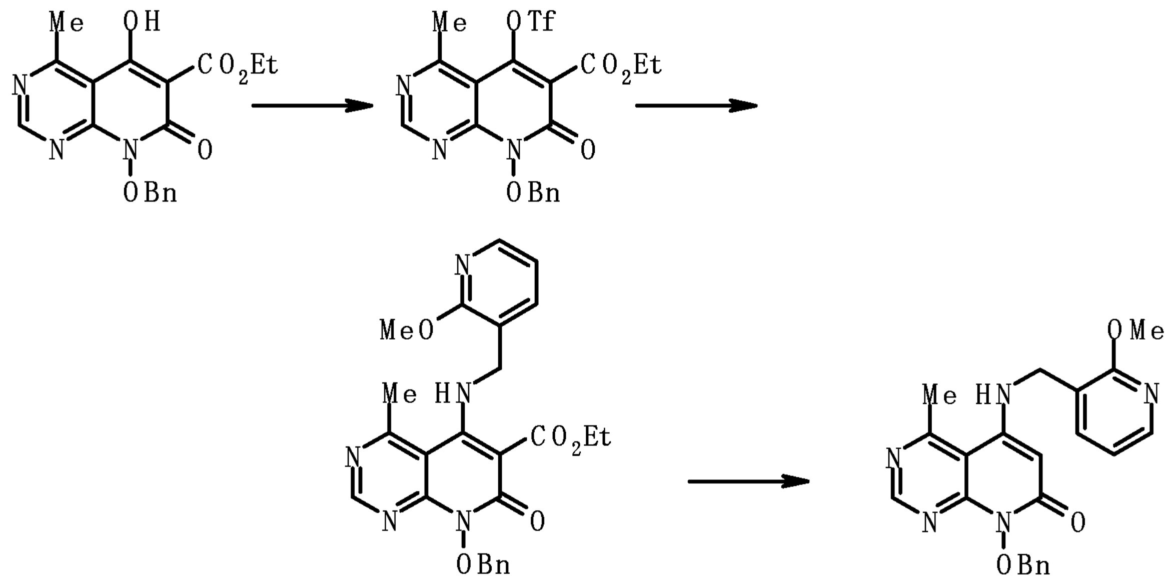

To ethyl 8-(benzyloxy)-5-hydroxy-4-methyl-7-oxo-7,8-dihydropyrido[2,3-d]pyrimidine-6-carboxylate (200 mg), methylene chloride (10 mL) and triethylamine (0.23 mL) were added, trifluoromethanesulfonic anhydride (0.14 mL) was added dropwise under ice cooling, and the mixture was stirred at room temperature for 1 hour and 30 minutes. The reaction mixture was added to 1-(2-methoxypyridin-3-yl)methanamine (117 mg), and the mixture was stirred at room temperature for 4 hours. The reaction mixture was purified by silica gel column chromatography [Kanto Chemical Co., Inc., silica gel 60 (spherical), eluent: 60-20% hexane/ethyl acetate] to obtain a brown oil (296 mg). To the obtained brown oil (296 mg), methanol (6.0 mL) and a 1 mol/L aqueous sodium hydroxide solution (6.0 mL) were added, and the mixture was heated with stirring at 60°C for 3 hours. The reaction mixture was cooled and then pH-adjusted to 7 by the dropwise addition of 6 mol/L hydrochloric acid. The deposit was collected by filtration and washed with water and 2-propanol to obtain a white solid of 8-(benzyloxy)-5-(((2-methoxypyridin-3-yl)methyl)amino)-4-methylpyrido[2,3-d]pyrimidin-7(8H)-one (71 mg).
$^1$H-NMR ($CDCl_3$) δ value: 2.97 (s, 3H), 4.04 (s, 3H), 4.37 (d, J=5.4 Hz, 2H), 5.23 (s, 2H), 5.73 (s, 1H), 5.77-5.83 (m, 1H), 6.92 (dd, J=7.1, 5.1 Hz, 1H), 7.32-7.41 (m, 3H), 7.58 (dd, J=7.2, 1.9 Hz, 1H), 7.65-7.70 (m, 2H), 8.16 (dd, J=5.1, 1.9 Hz, 1H), 8.89 (s, 1H).

Reference Example 219

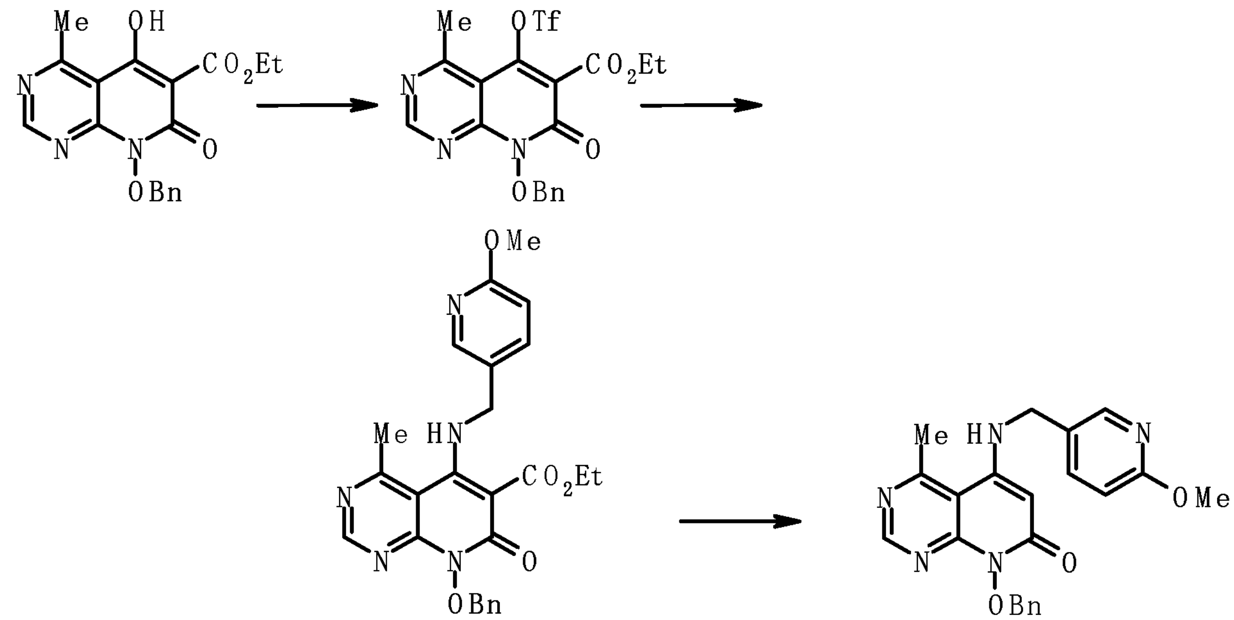

To ethyl 8-(benzyloxy)-5-hydroxy-4-methyl-7-oxo-7,8-dihydropyrido[2,3-d]pyrimidine-6-carboxylate (200 mg), methylene chloride (10 mL) and triethylamine (0.23 mL) were added, trifluoromethanesulfonic anhydride (0.14 mL) was added dropwise under ice cooling, and the mixture was stirred at room temperature for 1 hour and 30 minutes. The reaction mixture was added to 1-(2-methoxypyridin-5-yl)methanamine (117 mg), and the mixture was stirred at room temperature for 4 hours. The reaction mixture was purified by silica gel column chromatography [Kanto Chemical Co., Inc., silica gel 60 (spherical), eluent: 60-20% hexane/ethyl acetate] to obtain a brown oil (259 mg). To the obtained brown oil (259 mg), methanol (6.0 mL) and a 1 mol/L aqueous sodium hydroxide solution (6.0 mL) were added, and the mixture was heated with stirring at 60°C for 3 hours. The reaction mixture was cooled and then pH-adjusted to 7 by the dropwise addition of 6 mol/L hydrochloric acid, and butanol was added thereto. The solvent was distilled off under reduced pressure. The obtained residue was purified by silica gel column chromatography [Fuji Silysia Chemical Ltd. DNH silica, eluent: 80-60% hexane/ethyl acetate] and then suspended in diisopropyl ether, and the deposit was collected by filtration to obtain a white solid of 8-(benzyloxy)-5-(((2-methoxypyridin-5-yl)methyl)amino)-4-methylpyrido[2,3-d]pyrimidin-7(8H)-one (47 mg).
$^1$H-NMR ($CDCl_3$) δ value: 2.91 (s, 3H), 3.96 (s, 3H), 4.31 (d, J=4.4 Hz, 2H), 5.16-5.21 (m, 1H), 5.25 (s, 2H), 5.77 (s, 1H), 6.81 (d, J=8.6 Hz, 1H), 7.35-7.42 (m, 3H), 7.60 (dd, J=8.5, 2.7 Hz, 1H), 7.65-7.71 (m, 2H), 8.21 (d, J=2.4 Hz, 1H), 8.90 (s, 1H).

Reference Example 220

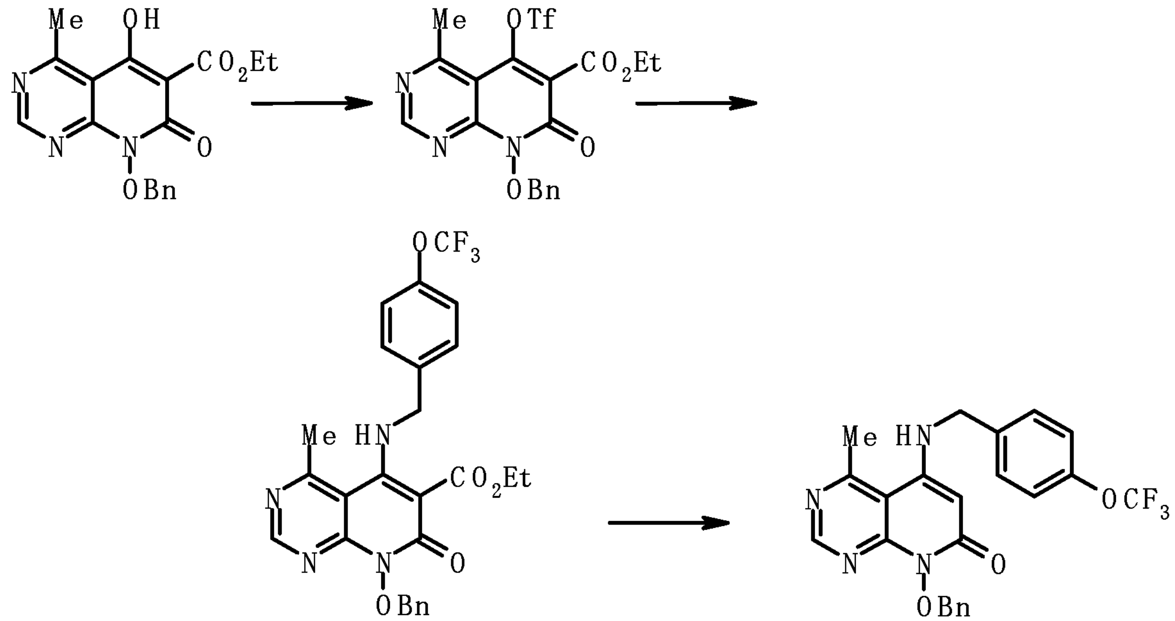

To ethyl 8-(benzyloxy)-5-hydroxy-4-methyl-7-oxo-7,8-dihydropyrido[2,3-d]pyrimidine-6-carboxylate (200 mg), methylene chloride (10 mL) and triethylamine (0.23 mL) were added, trifluoromethanesulfonic anhydride (0.14 mL) was added dropwise under ice cooling, and the mixture was stirred at room temperature for 1 hour and 30 minutes. The reaction mixture was added to 4-(trifluoromethoxy)benzylamine (161 mg), and the mixture was stirred at room temperature for 4 hours. The reaction mixture was purified by silica gel column chromatography [Kanto Chemical Co., Inc., silica gel 60 (spherical), eluent: 70-30% hexane/ethyl acetate] to obtain a brown oil (240 mg). To the obtained brown oil (240 mg), methanol (6.0 mL) and a 1 mol/L aqueous sodium hydroxide solution (6.0 mL) were added, and the mixture was heated with stirring at 60°C for 3 hours. The reaction mixture was cooled and then pH-adjusted to 7 by the dropwise addition of 6 mol/L hydrochloric acid. The deposit was collected by filtration and washed with water and methanol to obtain a white solid of 8-(benzyloxy)-4-methyl-5-((4-(trifluoromethoxy)benzyl)amino)pyrido[2,3-d]pyrimidin-7(8H)-one (74 mg).
$^1$H-NMR ($CDCl_3$) δ value: 2.96 (s, 3H), 4.41 (d, J=4.6 Hz, 2H), 5.25 (s, 2H), 5.31-5.37 (m, 1H), 5.73 (s, 1H), 7.20-7.30 (m, 2H), 7.34-7.45 (m, 5H), 7.65-7.72 (m, 2H), 8.91 (s, 1H).

Reference Example 221

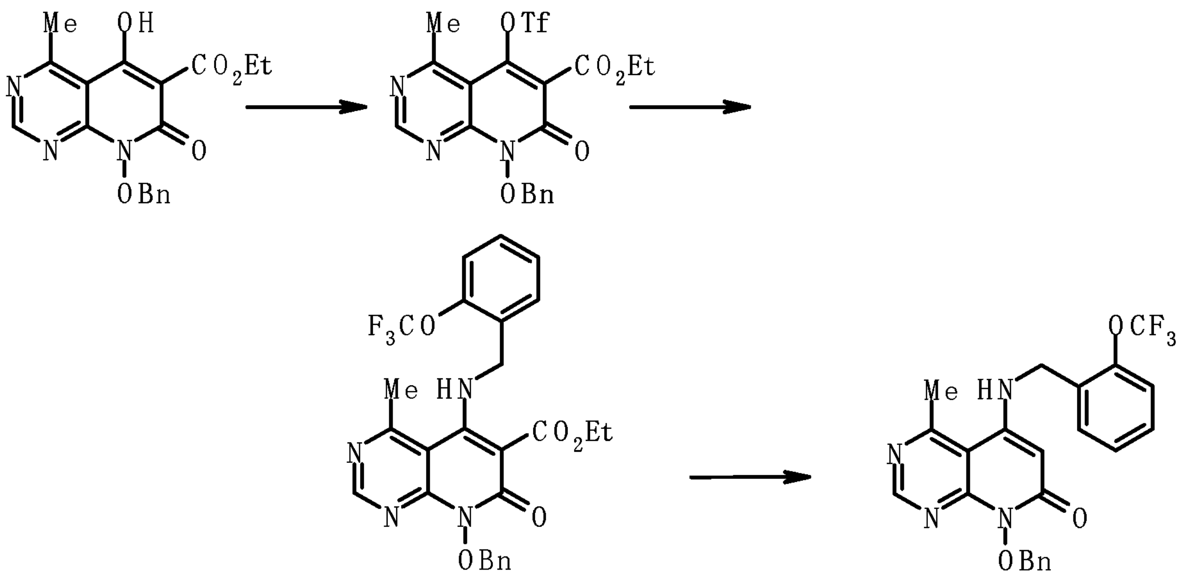

To ethyl 8-(benzyloxy)-5-hydroxy-4-methyl-7-oxo-7,8-dihydropyrido[2,3-d]pyrimidine-6-carboxylate (200 mg), methylene chloride (10 mL) and triethylamine (0.23 mL) were added, trifluoromethanesulfonic anhydride (0.14 mL) was added dropwise under ice cooling, and the mixture was stirred at room temperature for 1 hour and 30 minutes. The reaction mixture was added to 2-(trifluoromethoxy)benzylamine (161 mg), and the mixture was stirred at room temperature for 4 hours. The solvent was distilled off under reduced pressure. The obtained residue was purified by silica gel column chromatography [Kanto Chemical Co., Inc., silica gel 60 (spherical), eluent: 70-30% hexane/ethyl acetate] to obtain a brown oil (293 mg). To the obtained brown oil (293 mg), methanol (6.0 mL) and a 1 mol/L aqueous sodium hydroxide solution (6.0 mL) were added, and the mixture was heated with stirring at 60°C for 3 hours. The reaction mixture was cooled and then pH-adjusted to 7 by the dropwise addition of 6 mol/L hydrochloric acid. The deposit was collected by filtration and washed with water and methanol to obtain a white solid of 8-(benzyloxy)-4-methyl-5-((2-(trifluoromethoxy)benzyl)amino)pyrido[2,3-d]pyrimidin-7(8H)-one (32 mg).
$^1$H-NMR ($CDCl_3$) δ value: 2.95 (s, 3H), 4.49 (d, J=5.1 Hz, 2H), 5.24 (s, 2H), 5.42-5.48 (m, 1H), 5.74 (s, 1H), 7.20-7.48 (m, 7H), 7.65-7.71 (m, 2H), 8.90 (s, 1H).

Reference Example 222

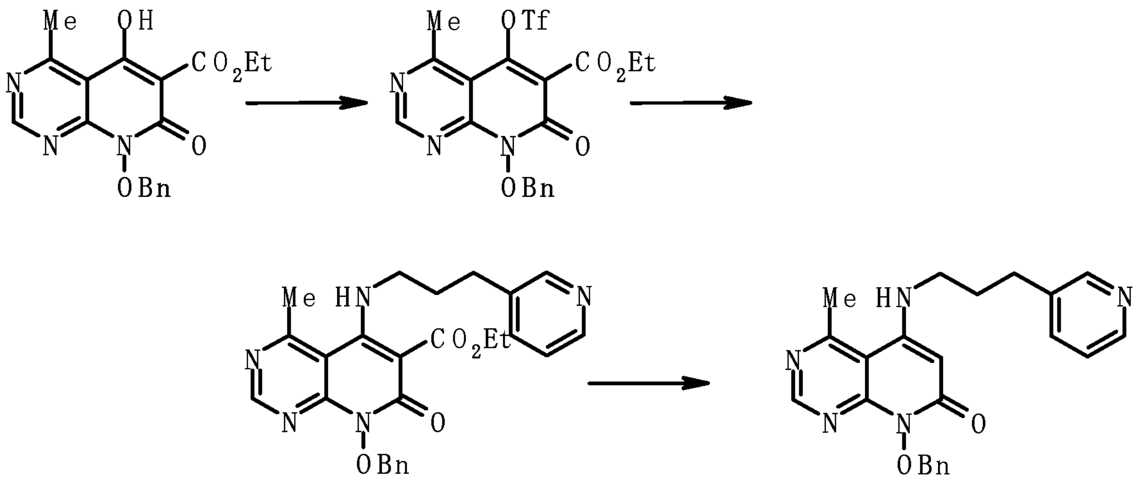

To ethyl 8-(benzyloxy)-5-hydroxy-4-methyl-7-oxo-7,8-dihydropyrido[2,3-d]pyrimidine-6-carboxylate (150 mg), methylene chloride (5 mL) and triethylamine (117 μL) were added, trifluoromethanesulfonic anhydride (138 μL) was added dropwise under ice cooling, and the mixture was stirred at room temperature for 40 minutes. The solvent in the reaction mixture was distilled off under reduced pressure. To the obtained residue, dioxane (7 mL), triethylamine (117 μL) and 3-(pyridin-3-yl)propan-1-amine (115 mg) were added, and the mixture was stirred at room temperature for 2 hours. The solvent in the reaction mixture was distilled off under reduced pressure. The obtained residue was purified by silica gel column chromatography [eluent: 100-95% chloroform/methanol] to obtain a brown oil (153 mg). To the obtained brown oil (153 mg), methanol (6 mL) and a 1 mol/L aqueous sodium hydroxide solution (6 mL) were added, and the mixture was heated with stirring at 50 to 60°C for 3 hours and 20 minutes. After cooling of the reaction mixture, ethyl acetate and water were added thereto. An organic layer was separated, washed with a saturated aqueous solution of sodium chloride and then dried over sodium sulfate, and the solvent was distilled off under reduced pressure. The obtained residue was purified by silica gel column chromatography [eluent: 100-90% chloroform/methanol] to obtain a yellow oil of 8-(benzyloxy)-4-methyl-5-((3-(pyridin-3-yl)propyl)amino)pyrido[2,3-d]pyrimidin-7(8H)-one (19 mg).
$^1$H-NMR ($CDCl_3$) δ value: 2.04-2.16 (m, 2H), 2.79 (t, J=7.6 Hz, 2H), 2.92 (s, 3H), 3.20-3.30 (m, 2H), 5.00-5.05 (m, 1H), 5.24 (s, 2H), 5.65 (s, 1H), 7.22-7.30 (m, 1H), 7.32-7.42 (m, 3H), 7.50-7.56 (m, 1H), 7.65-7.70 (m, 2H), 8.48-8.53 (m, 2H), 8.88 (s, 1H).

Reference Example 223

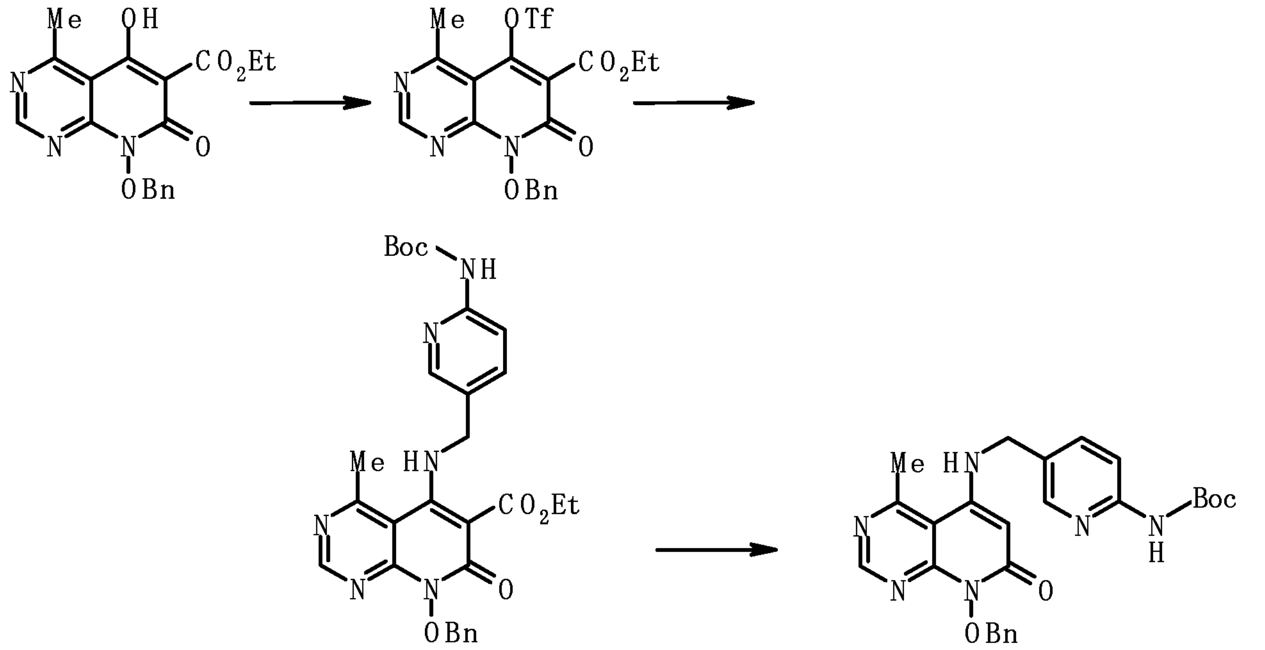

To ethyl 8-(benzyloxy)-5-hydroxy-4-methyl-7-oxo-7,8-dihydropyrido[2,3-d]pyrimidine-6-carboxylate (200 mg), methylene chloride (10 mL) and triethylamine (0.23 mL) were added, trifluoromethanesulfonic anhydride (0.14 mL) was added dropwise under ice cooling, and the mixture was stirred at room temperature for 30 minutes. The reaction mixture was added to tert-butyl (5-(aminomethyl)pyridin-2-yl)carbamate (189 mg), and the mixture was stirred at room temperature for 2 hours and 10 minutes. The solvent in the reaction mixture was distilled off under reduced pressure. The obtained residue was purified by silica gel column chromatography [Kanto Chemical Co., Inc., silica gel 60 (spherical), eluent: 70-0% hexane/ethyl acetate] and then suspended in diisopropyl ether, and the deposit was collected by filtration to obtain a brown solid (181 mg). To the obtained brown solid (174 mg), methanol (3.0 mL) and a 1 mol/L aqueous sodium hydroxide solution (3.0 mL) were added, and the mixture was heated with stirring at 60°C for 4 hours. The reaction mixture was stirred for 30 minutes under ice cooling, and the deposit was collected by filtration and washed with water and 2-propanol to obtain a white solid of tert-butyl (5-(((8-(benzyloxy)-4-methyl-7-oxo-7,8-dihydropyrido[2,3-d]pyrimidin-5-yl)amino)methyl)pyridin-2-yl)carbamate (53 mg).
$^1$H-NMR ($CDCl_3$) δ value: 1.54 (s, 9H), 2.90 (s, 3H), 4.32 (d, J=4.6 Hz, 2H), 5.16-5.21 (m, 1H), 5.25 (s, 2H), 5.76 (s, 1H), 7.35-7.40 (m, 4H), 7.65-7.72 (m, 3H), 8.01 (d, J=8.5 Hz, 1H), 8.28 (d, J=2.2 Hz, 1H), 8.90 (s, 1H).

Reference Example 224

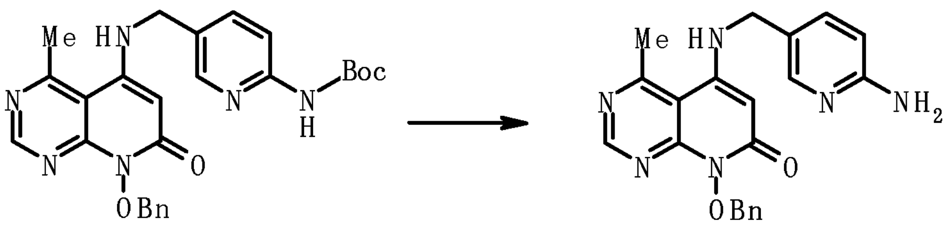

To tert-butyl (5-(((8-(benzyloxy)-4-methyl-7-oxo-7,8-dihydropyrido[2,3-d]pyrimidin-5-yl)amino)methyl)pyridin-2-yl)carbamate (53 mg), methylene chloride (5.0 mL) was added, trifluoroacetic acid (0.5 mL) was added under ice cooling, and the mixture was stirred at room temperature for 2 hours and 30 minutes. To the reaction mixture, chloroform was added, and the mixture was pH-adjusted to 9 by the dropwise addition of 2 mol/L sodium hydroxide. An organic layer was separated, washed with a saturated aqueous solution of sodium chloride and then dried over sodium sulfate, and the solvent was distilled off under reduced pressure. The obtained residue was suspended in diisopropyl ether, and the deposit was collected by filtration to obtain a white solid of 5-(((6-aminopyridin-3-yl)methyl)amino)-8-(benzyloxy)-4-methylpyrido[2,3-d]pyrimidin-7(8H)-one (33 mg).

$^1$H-NMR (DMSO-$d_6$) δ value: 2.96 (s, 3H), 4.26 (d, J=5.1 Hz, 2H), 5.08 (s, 2H), 5.51 (s, 1H), 5.88 (s, 2H), 6.44 (d, J=8.8 Hz, 1H), 6.74 (t, J=5.1 Hz, 1H), 7.37-7.47 (m, 4H), 7.59-7.64 (m, 2H), 7.96-8.00 (m, 1H), 8.87 (s, 1H).

Reference Example 225

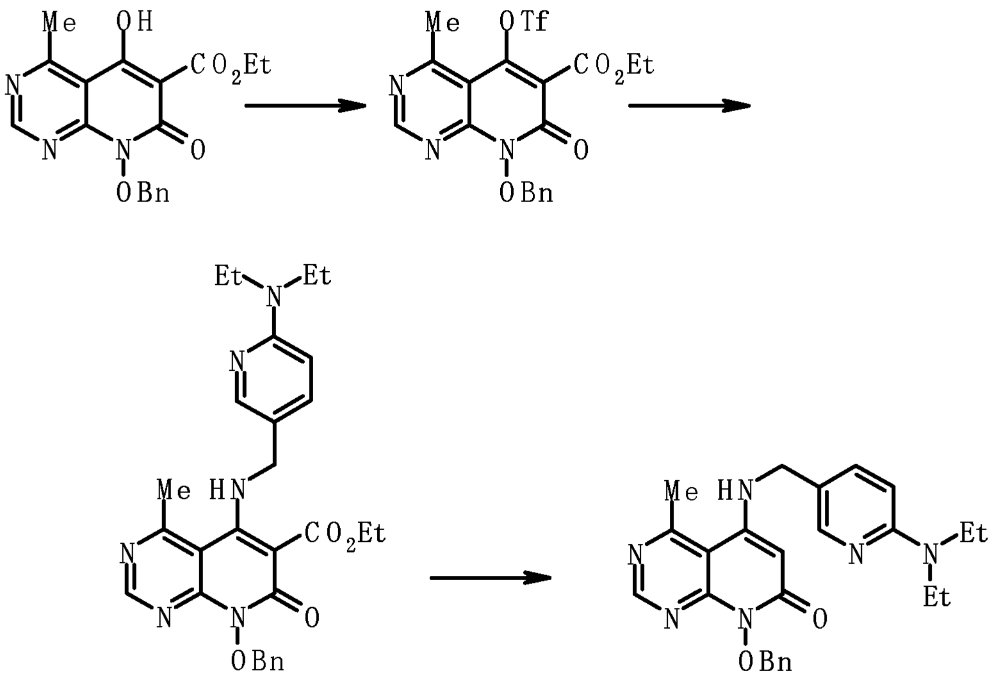

To ethyl 8-(benzyloxy)-5-hydroxy-4-methyl-7-oxo-7,8-dihydropyrido[2,3-d]pyrimidine-6-carboxylate (200 mg), methylene chloride (10 mL) and triethylamine (0.23 mL) were added, trifluoromethanesulfonic anhydride (0.14 mL) was added dropwise under ice cooling, and the mixture was stirred at room temperature for 30 minutes. To the reaction mixture, 5-(aminomethyl)-N,N-diethylpyridin-2-amine (151 mg) was added, and the mixture was stirred at room temperature for 1 hour. The solvent in the reaction mixture was distilled off under reduced pressure. The obtained residue was purified by silica gel column chromatography [Kanto Chemical Co., Inc., silica gel 60 (spherical), eluent: 50-30% hexane/ethyl acetate] to obtain a brown oil (148 mg). To the obtained brown oil (148 mg), methanol (3.0 mL) and a 1 mol/L aqueous sodium hydroxide solution (3.0 mL) were added, and the mixture was heated with stirring at 60°C for 4 hours and 30 minutes. After cooling of the reaction mixture, the deposit was collected by filtration and washed with water and 2-propanol to obtain a white solid of 8-(benzyloxy)-5-(((6-(diethylamino)pyridin-3-yl)methyl)amino)-4-methylpyrido[2,3-d]pyrimidin-7(8H)-one (34 mg).
$^1$H-NMR ($CDCl_3$) δ value: 1.20 (t, J=7.1 Hz, 6H), 2.89 (s, 3H), 3.53 (q, J=7.1 Hz, 4H), 4.18 (d, J=4.2 Hz, 2H), 5.10-5.14 (m, 1H), 5.26 (s, 2H), 5.79 (s, 1H), 6.50 (d, J=8.5 Hz, 1H), 7.33-7.45 (m, 4H), 7.66-7.72 (m, 2H), 8.16 (d, J=2.2 Hz, 1H), 8.88 (s, 1H).

Reference Example 226

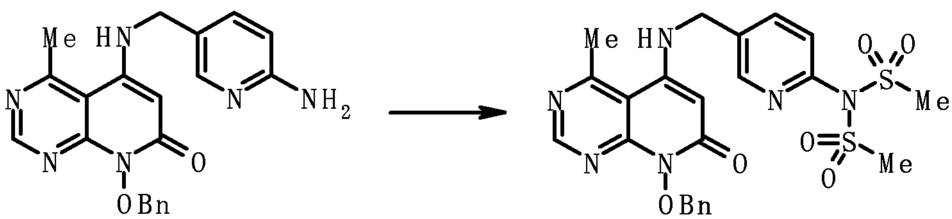

To 5-(((6-aminopyridin-3-yl)methyl)amino)-8-(benzyloxy)-4-methylpyrido[2,3-d]pyrimidin-7(8H)-one (35 mg),

triethylamine (19 μL) and methylene chloride (1.0 mL) were added. To the reaction mixture, methanesulfonyl chloride (7.7 μL) and methylene chloride (1.0 mL) were added dropwise, and the mixture was stirred at room temperature for 10 minutes. To the reaction mixture, methanesulfonyl chloride (7.7 μL) and methylene chloride (1.0 mL) were added, and the mixture was stirred at room temperature for 50 minutes. To the reaction mixture, chloroform and water were added, followed by separation of an organic layer. An aqueous layer was extracted with chloroform, combined with the organic layer, washed with a saturated aqueous solution of sodium chloride and then dried over sodium sulfate, and the solvent was distilled off under reduced pressure. The obtained residue was suspended in chloroform, and the deposit was collected by filtration to obtain a white solid of N-(5-(((8-(benzyloxy)-4-methyl-7-oxo-7,8-dihydropyrido[2,3-d]pyrimidin-5-yl)amino)methyl)pyridin-2-yl)-N-(methylsulfonyl)methanesulfonamide (34 mg).
$^1$H-NMR (DMSO-$d_6$) δ value: 3.03 (s, 3H), 3.65 (s, 6H), 4.63 (d, J=5.4 Hz, 2H), 5.09 (s, 2H), 5.52 (s, 1H), 7.03 (t, J=5.5 Hz, 1H), 7.38-7.44 (m, 3H), 7.59-7.64 (m, 2H), 7.70 (d, J=7.8 Hz, 1H), 8.05 (dd, J=8.3, 2.4 Hz, 1H), 8.63 (d, J=2.2 Hz, 1H), 8.91 (s, 1H).

Reference Example 227

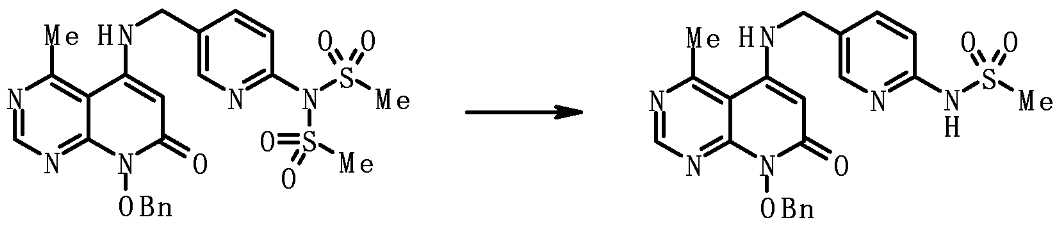

To N-(5-(((8-(benzyloxy)-4-methyl-7-oxo-7,8-dihydropyrido[2,3-d]pyrimidin-5-yl)amino)methyl)pyridin-2-yl)-N-(methylsulfonyl)methanesulfonamide (34 mg), THF (3.4 mL) and 2 mol/L aqueous sodium hydroxide solution (0.17 mL) were added, and the mixture was stirred at room temperature for 2 hours and 30 minutes. The reaction mixture was pH-adjusted to 7 by the dropwise addition of 1 mol/L hydrochloric acid, and the solvent was distilled off under reduced pressure. Ethyl acetate was added to the residue, followed by separation of an organic layer. An aqueous layer was extracted with ethyl acetate, combined with the organic layer, washed with a saturated aqueous solution of sodium chloride and then dried over sodium sulfate, and the solvent was distilled off under reduced pressure. The obtained residue was suspended in ethyl acetate, and the deposit was collected by filtration to obtain a white solid of N-(5-(((8-(benzyloxy)-4-methyl-7-oxo-7,8-dihydropyrido[2,3-d]pyrimidin-5-yl)amino)methyl)pyridin-2-yl)methanesulfonamide (16 mg).
$^1$H-NMR (DMSO-$d_6$) δ value: 3.00 (s, 3H), 3.27 (s, 3H), 4.44 (d, J=5.1 Hz, 2H), 5.07 (s, 2H), 5.49 (s, 1H), 6.91 (t, J=5.4 Hz, 1H), 6.94-7.04 (m, 1H), 7.38-7.44 (m, 4H), 7.58-7.64 (m, 2H), 7.82 (dd, J=8.4, 2.3 Hz, 1H), 8.33 (brs, 1H), 8.89 (s, 1H).

Reference Example 228

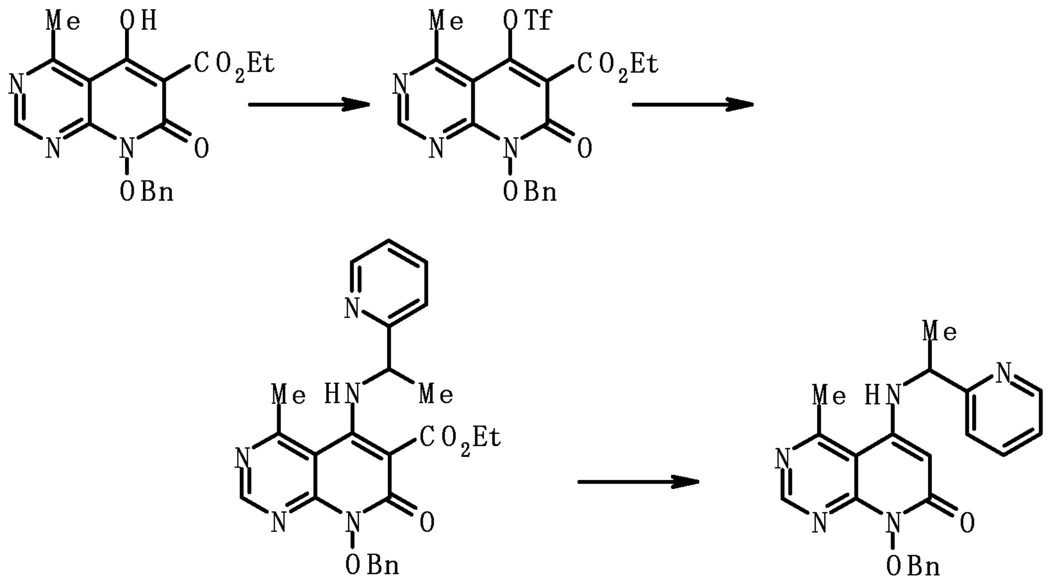

To ethyl 8-(benzyloxy)-5-hydroxy-4-methyl-7-oxo-7,8-dihydropyrido[2,3-d]pyrimidine-6-carboxylate (200 mg), methylene chloride (10 mL) and triethylamine (0.23 mL) were added, trifluoromethanesulfonic anhydride (0.14 mL) was added dropwise under ice cooling, and the mixture was stirred at room temperature for 15 minutes. To the reaction mixture, 1-(pyridin-2-yl)ethanamine (103 mg) was added, and the mixture was stirred at room temperature for 30 minutes. The solvent in the reaction mixture was distilled off under reduced pressure. The obtained residue was purified by silica gel column chromatography [Kanto Chemical Co., Inc., silica gel 60 (spherical), eluent: 50-20% hexane/ethyl acetate] to obtain a pale yellow oil (207 mg). To the obtained pale yellow oil (207 mg), methanol (3.0 mL) and a 1 mol/L aqueous sodium hydroxide solution (3.0 mL) were added, and the mixture was heated to reflux for 4 hours. The reaction mixture was cooled and then pH-adjusted to 8 by the dropwise addition of 6 mol/L hydrochloric acid, and the solvent was distilled off under reduced pressure. To the obtained residue, butanol was added, and the solvent was distilled off under reduced pressure. The obtained residue was purified by silica gel column chromatography [Fuji Silysia Chemical Ltd., DNH silica, eluent: 80-60% hexane/ethyl acetate] and then suspended in a mixed solvent of ethyl acetate and diisopropyl ether, and the deposit was collected by filtration to obtain a white solid of 8-(benzyloxy)-4-methyl-5-((1-(pyridin-2-yl)ethyl)amino)pyrido[2,3-d]pyrimidin-7(8H)-one (83 mg).
$^1$H-NMR ($CDCl_3$) δ value: 1.60 (d, J=6.6 Hz, 3H), 3.17 (s, 3H), 4.71-4.79 (m, 1H), 5.26 (s, 2H), 5.72 (s, 1H), 7.20-7.42 (m, 5H), 7.47-7.53 (m, 1H), 7.67-7.71 (m, 2H), 7.75 (ddd, J=7.7, 7.7, 1.7 Hz, 1H), 8.58-8.61 (m, 1H), 8.90 (s, 1H).

Example 1

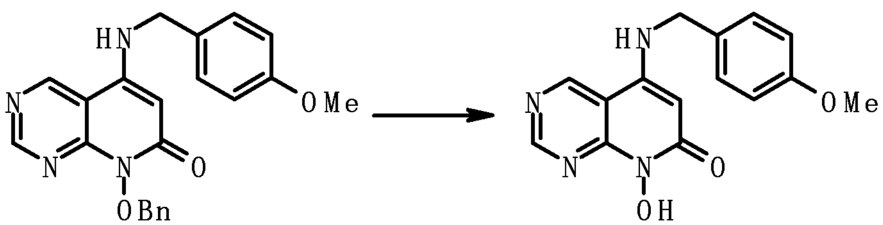

To 8-(benzyloxy)-5-((4-methoxybenzyl)amino)pyrido[2,3-d]pyrimidin-7(8H)-one (138 mg), 10% palladium-carbon (69 mg) and methanol (25 mL) were added, and the mixture was stirred at room temperature for 1 hour and 10 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a white solid of 8-hydroxy-5-((4-methoxybenzyl)amino)pyrido[2,3-d]pyrimidin-7(8H)-one (86 mg).
$^1$H-NMR (DMSO-$d_6$) δ value: 3.73 (s, 3H), 4.39 (d, J=5.6 Hz, 2H), 5.40 (s, 1H), 6.91 (d, J=8.5 Hz, 2H), 7.31 (d, J=8.3 Hz, 2H), 7.60-8.12 (m, 1H), 9.01 (s, 1H), 9.38 (s, 1H), 10.62 (brs, 1H).

Example 2

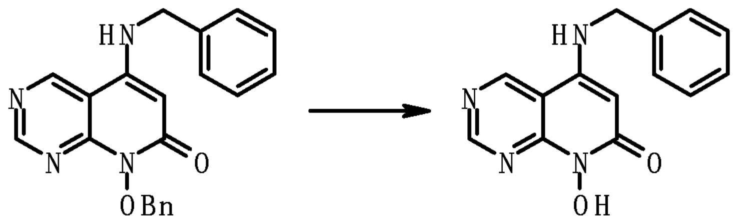

To 5-(benzylamino)-8-(benzyloxy)pyrido[2,3-d]pyrimidin-7(8H)-one (13 mg), 10% palladium-carbon (7 mg) and methanol (7 mL) were added, and the mixture was stirred at room temperature for 5 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a white solid of 5-(benzylamino)-8-hydroxypyrido[2,3-d]pyrimidin-7(8H)-one (6 mg).
$^{1}$H-NMR (DMSO-$d_6$) $\delta$ value: 4.42-4.52 (m, 2H), 5.39 (s, 1H), 7.20-7.45 (m, 5H), 8.04-8.16 (m, 1H), 9.02 (s, 1H), 9.39 (s, 1H), 10.62 (brs, 1H).

Example 3

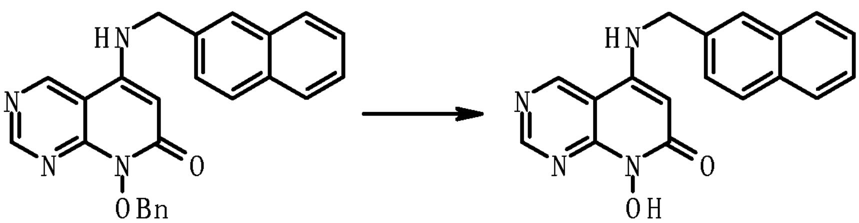

To 8-(benzyloxy)-5-((2-naphthylmethyl)amino)pyrido[2,3-d]pyrimidin-7(8H)-one (17 mg), 10% palladium-carbon (8 mg) and methanol (8 mL) were added, and the mixture was stirred at room temperature for 15 minutes in a hydrogen atmosphere. Ethanol (5 mL) was added thereto, and the mixture was stirred at room temperature for 10 minutes in a hydrogen atmosphere. Palladium-carbon was filtered off and washed with ethanol. The filtrate and the washes were combined, and the solvent was distilled off under reduced pressure. To the obtained residue, 10% palladium-carbon (8 mg) and ethanol (10 mL) were added, and the mixture was stirred at room temperature for 1 hour and 43 minutes in a hydrogen atmosphere. Palladium-carbon was filtered off and washed with ethanol. The filtrate and the washes were combined, and the solvent was distilled off under reduced pressure. The obtained residue was suspended in a mixed solvent of 2-propanol and diisopropyl ether, and the deposit was collected by filtration to obtain a white solid of 8-hydroxy-5-((2-naphthylmethyl)amino)pyrido[2,3-d]pyrimidin-7(8H)-one (5 mg).
$^{1}$H-NMR (DMSO-$d_6$) $\delta$ value: 4.64 (d, J=5.6 Hz, 2H), 5.44 (s, 1H), 7.42-7.62 (m, 3H), 7.84-7.96 (m, 4H), 8.16-8.26 (m, 1H), 9.03 (s, 1H), 9.44 (s, 1H), 10.62 (brs, 1H).

Example 4

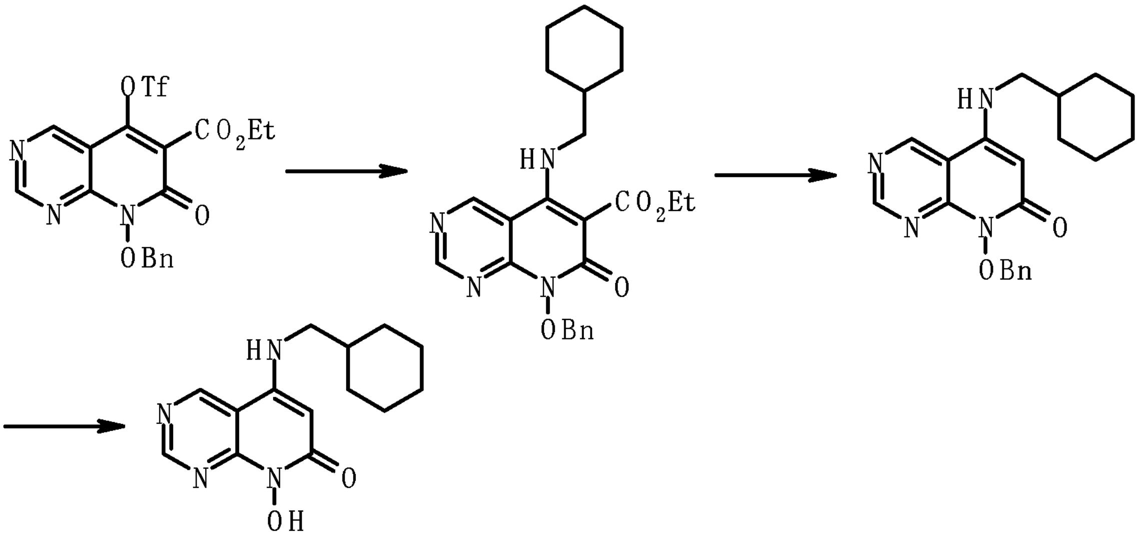

A solution of ethyl 8-(benzyloxy)-7-oxo-5-(((trifluoromethyl)sulfonyl)oxy)-7,8-dihydropyrido[2,3-d]pyrimidine-6-carboxylate (90 mg) and aminomethylcyclohexane (99 μL) in dioxane (9 mL) was heated with stirring at 90 to 100°C for 2 hours and 30 minutes. After cooling of the reaction mixture, the solvent was distilled off under reduced pressure. The obtained residue was purified by silica gel column chromatography [eluent: 95-50% hexane/ethyl acetate] to obtain a yellow oil (329 mg). To the obtained yellow oil (329 mg), methanol (5 mL) and a 1 mol/L aqueous sodium hydroxide solution (5 mL) were added, and the mixture was heated to reflux for 1 hour and 30 minutes. After cooling of the reaction mixture, the solvent was distilled off under reduced pressure. To the obtained residue, a 1 mol/L aqueous sodium hydroxide solution (5 mL) was added, and the mixture was pH-adjusted to 1 by the dropwise addition of concentrated hydrochloric acid and stirred at room temperature for 10 minutes. The deposit was collected by filtration to obtain a brown solid (15 mg). To the obtained brown solid (14 mg), 10% palladium-carbon (7 mg) and methanol (10 mL) were added, and the mixture was stirred at room temperature for 10 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a white solid of 5-((cyclohexylmethyl)amino)-8-hydroxypyrido[2,3-d]pyrimidin-7(8H)-one (7 mg).
$^1$H-NMR (DMSO-$d_6$) δ value: 0.90-1.08 (m, 2H), 1.08-1.30 (m, 3H), 1.55-1.90 (m, 6H), 2.96-3.10 (m, 2H), 5.47 (s, 1H), 7.30-7.45 (m, 1H), 8.99 (s, 1H), 9.36 (s, 1H).

Example 5

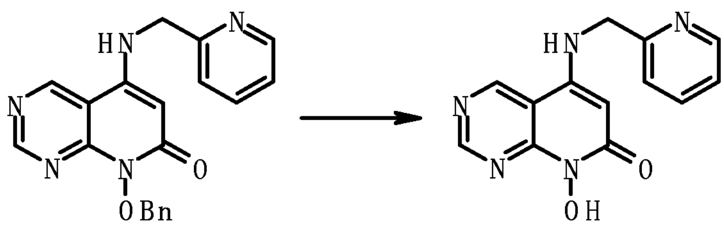

To 8-(benzyloxy)-5-(((pyridin-2-yl)methyl)amino)pyrido[2,3-d]pyrimidin-7(8H)-one (176 mg), 10% palladium-carbon (88 mg) and methanol (35 mL) were added, and the mixture was stirred at room temperature for 1 hour and 30 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a yellow solid of 8-hydroxy-5-(((pyridin-2-yl)methyl)amino)pyrido[2,3-d]pyrimidin-7(8H)-one (55 mg).
$^1$H-NMR (DMSO-$d_6$) δ value: 4.54 (d, J=5.9 Hz, 2H), 5.37 (s, 1H), 7.25-7.35 (m, 1H), 7.42 (d, J=7.8 Hz, 1H), 7.74-7.83 (m, 1H), 8.15-8.23 (m, 1H), 8.56 (d, J=4.4 Hz, 1H), 9.03 (s, 1H), 9.39 (s, 1H), 10.63 (brs, 1H).

Example 6

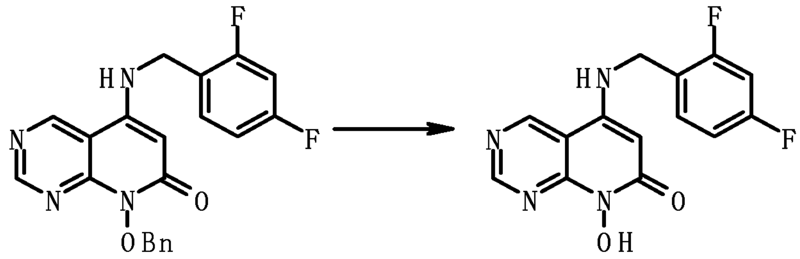

To 8-(benzyloxy)-5-((2,4-difluorobenzyl)amino)pyrido[2,3-d]pyrimidin-7(8H)-one (24 mg), 10% palladium-carbon (12 mg) and methanol (10 mL) were added, and the mixture was stirred at room temperature for 10 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the resulting solid was collected by filtration to obtain a pale yellow solid of 5-((2,4-difluorobenzyl)amino)-8-hydroxypyrido[2,3-d]pyrimidin-7(8H)-one (12 mg).
$^1$H-NMR (DMSO-$d_6$) δ value: 4.46 (d, J=5.4 Hz, 2H), 5.45 (s, 1H), 7.00-7.14 (m, 1H), 7.22-7.34 (m, 1H), 7.37-7.52 (m, 1H), 7.90-8.02 (m, 1H), 9.02 (s, 1H), 9.37 (s, 1H), 10.67 (brs, 1H).

Example 7

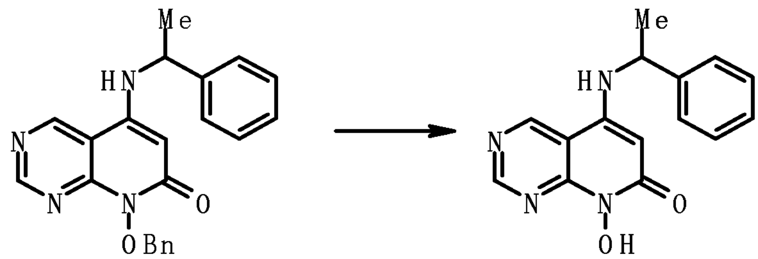

To 8-(benzyloxy)-5-((1-phenylethyl)amino)pyrido[2,3-d]pyrimidin-7(8H)-one (9 mg), 10% palladium-carbon (5 mg) and methanol (8 mL) were added, and the mixture was stirred at room temperature for 15 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a white solid of 8-hydroxy-5-((1-phenylethyl)amino)pyrido[2,3-d]pyrimidin-7(8H)-one (4 mg).
$^1$H-NMR (DMSO-$d_6$) δ value: 1.55 (d, J=6.4 Hz, 3H), 4.64-4.72 (m, 1H), 5.26 (s, 1H), 7.20-7.27 (m, 1H), 7.27-7.38 (m, 2H), 7.38-7.50 (m, 2H), 7.58-7.67 (m, 1H), 9.02 (s, 1H), 9.57 (s, 1H), 10.62 (brs, 1H).

Example 8

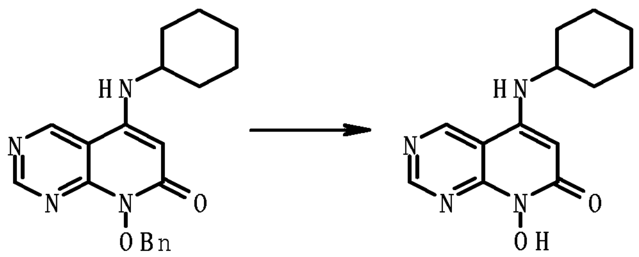

To 8-(benzyloxy)-5-(cyclohexylamino)pyrido[2,3-d]pyrimidin-7(8H)-one (28 mg), 10% palladium-carbon (14 mg) and methanol (10 mL) were added, and the mixture was stirred at room temperature for 10 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a white solid of 5-(cyclohexylamino)-8-hydroxypyrido[2,3-d]pyrimidin-7(8H)-one (17 mg).
$^1$H-NMR (DMSO-$d_6$) δ value: 1.10-1.46 (m, 5H), 1.60-1.68 (m, 1H), 1.72-1.80 (m, 2H), 1.92-2.02 (m, 2H), 3.20-3.40 (m, 1H), 5.55 (s, 1H), 6.97 (d, J=7.3 Hz, 1H), 8.99 (s, 1H), 9.39 (s, 1H), 10.62 (brs, 1H).

Example 9

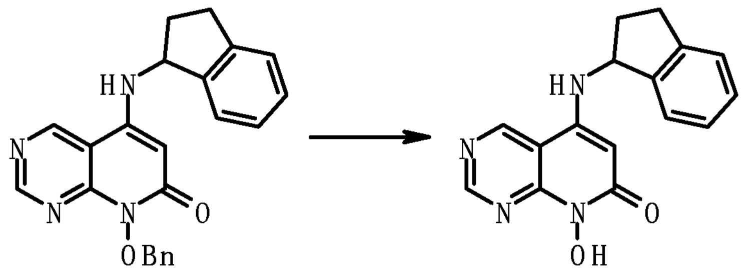

To 8-(benzyloxy)-5-((2,3-dihydro-1H-inden-1-yl)amino)pyrido[2,3-d]pyrimidin-7(8H)-one (75 mg), 10% palladium-carbon (38 mg) and methanol (18 mL) were added, and the mixture was stirred at room temperature for 20 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a white solid of 5-((2,3-dihydro-1H-inden-1-yl)amino)-8-hydroxypyrido[2,3-d]pyrimidin-7(8H)-one (46 mg).
$^1$H-NMR (DMSO-$d_6$) δ value: 1.93-2.06 (m, 1H), 2.54-2.65 (m, 1H), 2.83-2.95 (m, 1H), 2.95-3.07 (m, 1H), 5.16-5.26 (m, 1H), 5.83 (s, 1H), 7.17-7.37 (m, 4H), 7.60 (d, J=8.0 Hz, 1H), 9.01 (s, 1H), 9.40 (s, 1H), 10.63 (brs, 1H).

Example 10

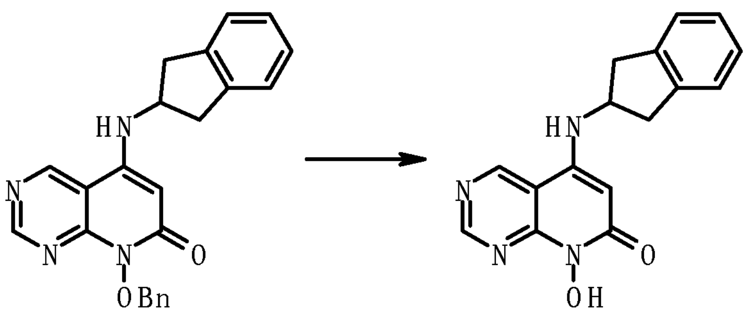

To 8-(benzyloxy)-5-((2,3-dihydro-1H-inden-2-yl)amino)pyrido[2,3-d]pyrimidin-7(8H)-one (36 mg), 10% palladium-carbon (18 mg) and methanol (20 mL) were added, and the mixture was stirred at room temperature for 30 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a white solid of 5-((2,3-dihydro-1H-inden-2-yl)amino)-8-hydroxypyrido[2,3-d]pyrimidin-7(8H)-one (21 mg).
$^1$H-NMR (DMSO-$d_6$) δ value: 3.00-3.08 (m, 2H), 3.37-3.45 (m, 2H), 4.30-4.50 (m, 1H), 5.65 (s, 1H), 7.12-7.21 (m, 2H), 7.21-7.31 (m, 2H), 7.34-7.42 (m, 1H), 8.99 (s, 1H), 9.39 (s, 1H), 10.65 (brs, 1H).

Example 11

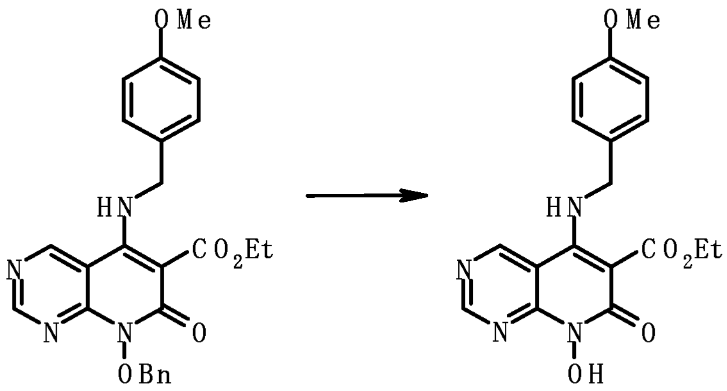

To a solution of ethyl 8-(benzyloxy)-5-((4-methoxybenzyl)amino)-7-oxo-7,8-dihydropyrido[2,3-d]pyrimidine-6-carboxylate (30 mg) in ethanol (10 mL), 10% palladium-carbon (15 mg) was added, and the mixture was stirred at room temperature for 2 hours and 5 minutes in a hydrogen atmosphere. Palladium-carbon was filtered off and washed with ethanol. The filtrate and the washes were combined, and the solvent was distilled off under reduced pressure. The obtained residue was suspended in a mixed solvent of methanol and diisopropyl ether, and the resulting solid was collected by filtration to obtain a pale green solid of ethyl 8-hydroxy-5-((4-methoxybenzyl)amino)-7-oxo-7,8-dihydropyrido[2,3-d]pyrimidine-6-carboxylate (13 mg).
$^1$H-NMR (DMSO-$d_6$) δ value: 1.08 (t, J=7.1 Hz, 3H), 3.73 (s, 3H), 4.04 (q, J=7.2 Hz, 2H), 4.41 (d, J=5.4 Hz, 2H), 6.91 (d, J=8.5 Hz, 2H), 7.22 (d, J=8.3 Hz, 2H), 7.90-8.00 (m, 1H), 9.06 (s, 1H), 9.47 (s, 1H), 10.84 (s, 1H).

Example 12

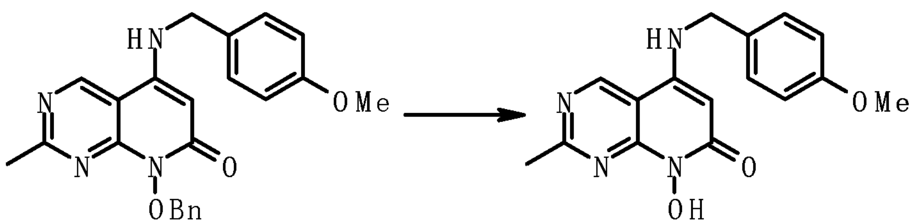

To 8-(benzyloxy)-5-((4-methoxybenzyl)amino)-2-methylpyrido[2,3-d]pyrimidin-7(8H)-one (64 mg), 10% palladium-carbon (32 mg) and methanol (13 mL) were added, and the mixture was stirred at room temperature for 30 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-5-((4-methoxybenzyl)amino)-2-methylpyrido[2,3-d]pyrimidin-7(8H)-one (44 mg).
$^1$H-NMR (DMSO-$d_6$) δ value: 2.64 (s, 3H), 3.73 (s, 3H), 4.36 (d, J=5.4 Hz, 2H), 5.33 (s, 1H), 6.91 (d, J=8.6 Hz, 2H), 7.30 (d, J=8.0 Hz, 2H), 7.92-8.04 (m, 1H), 9.27 (s, 1H), 10.49 (brs, 1H).

Example 13

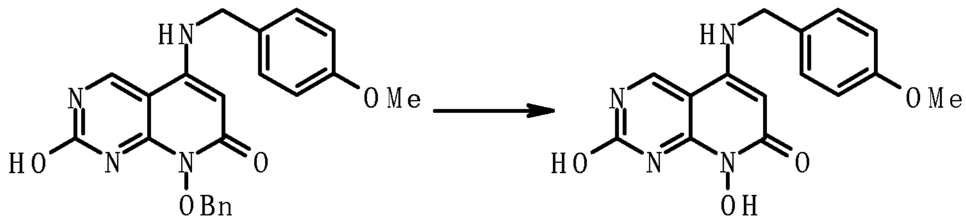

To 8-(benzyloxy)-2-hydroxy-5-((4-methoxybenzyl)amino)pyrido[2,3-d]pyrimidin-7(8H)-one (50 mg), 10% palladium-carbon (25 mg) and ethanol (38 mL) were added, and the mixture was stirred at room temperature for 30 minutes in a hydrogen atmosphere. Palladium-carbon was filtered off and washed with ethanol. The filtrate and the washes were combined, and the solvent was distilled off under reduced pressure. The obtained residue was suspended in a mixed solvent of 2-propanol and diisopropyl ether, and the deposit was collected by filtration to obtain a white solid of 2,8-dihydroxy-5-((4-methoxybenzyl)amino)pyrido[2,3-d]pyrimidin-7(8H)-one (5mg).
$^1$H-NMR (DMSO-$d_6$) δ value: 3.72 (s, 3H), 4.17 (d, J=5.8 Hz, 2H), 4.83 (s, 1H), 6.36-6.46 (m, 1H), 6.83-6.94 (m, 3H), 7.21 (d, J=8.6 Hz, 2H), 7.32 (brs, 1H), 8.74 (s, 1H).

Example 14

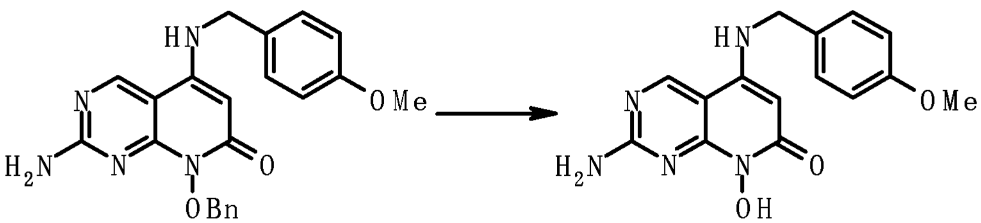

To 2-amino-8-(benzyloxy)-5-((4-methoxybenzyl)amino)pyrido[2,3-d]pyrimidin-7(8H)-one (16 mg), 10% palladium-carbon (8 mg) and methanol (10 mL) were added, and the mixture was stirred at room temperature for 30 minutes in a hydrogen atmosphere. Palladium-carbon was filtered off and washed with methanol, ethanol and chloroform. The filtrate and the washes were combined, and the solvent was distilled off under reduced pressure. The obtained residue was suspended in diisopropyl ether, and the deposit was collected by filtration to obtain a gray solid of 2-amino-8-hydroxy-5-((4-methoxybenzyl)amino)pyrido[2,3-d]pyrimidin-7(8H)-one (1 mg).
$^1$H-NMR (DMSO-$d_6$) δ value: 3.73 (s, 3H), 4.25-4.36 (m, 2H), 5.04 (s, 1H), 6.85-6.94 (m, 2H), 7.10-7.21 (m, 2H), 7.21-7.32 (m, 2H), 7.60-7.68 (m, 1H), 8.90 (s, 1H).

Example 15

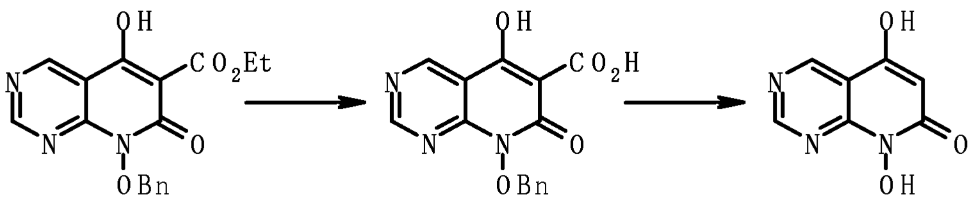

To ethyl 8-(benzyloxy)-5-hydroxy-7-oxo-7,8-dihydropyrido[2,3-d]pyrimidine-6-carboxylate (150 mg), methanol (8 mL) and a 1 mol/L aqueous sodium hydroxide solution (8 mL) were added, and the mixture was heated to reflux for 5 hours. After cooling of the reaction mixture, the solvent was distilled off under reduced pressure. To the obtained residue, a 1 mol/L aqueous sodium hydroxide solution (5 mL) was added, and the mixture was pH-adjusted to 1 by the

dropwise addition of concentrated hydrochloric acid and stirred at room temperature for 20 minutes. The deposit was collected by filtration to obtain a pale yellow solid (109 mg). To the pale yellow solid (109 mg), concentrated hydrochloric acid (8 mL) and TFA (8 mL) were added, and the mixture was heated with stirring at 70 to 80°C for 10 hours. After cooling, the solvent in the reaction mixture was distilled off under reduced pressure. The obtained residue was suspended in a mixed solvent of 2-propanol and diisopropyl ether, and the deposit was collected by filtration to obtain a brown solid of 5,8-dihydroxypyrido[2,3-d]pyrimidin-7(8H)-one (60 mg).
$^1$H-NMR (DMSO-$d_6$) δ value: 5.16 (brs, 1H), 6.07 (s, 1H), 9.10 (s, 1H), 9.12 (s, 1H), 12.39 (brs, 1H).

Example 16

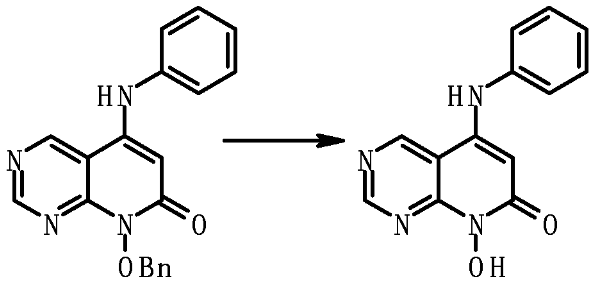

To 5-anilino-8-(benzyloxy)pyrido[2,3-d]pyrimidin-7(8H)-one (10 mg), 10% palladium-carbon (5 mg), ethyl acetate (4 mL) and methanol (4 mL) were added, and the mixture was stirred at room temperature for 20 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale green solid of 5-anilino-8-hydroxypyrido[2,3-d]pyrimidin-7(8H)-one (6 mg). $^1$H-NMR (DMSO-$d_6$) δ value: 5.83 (s, 1H), 7.17-7.28 (m, 1H), 7.31-7.41 (m, 2H), 7.42-7.52 (m, 2H), 9.08 (s, 1H), 9.10 (s, 1H), 9.47 (s, 1H), 10.83 (brs, 1H).

Example 17

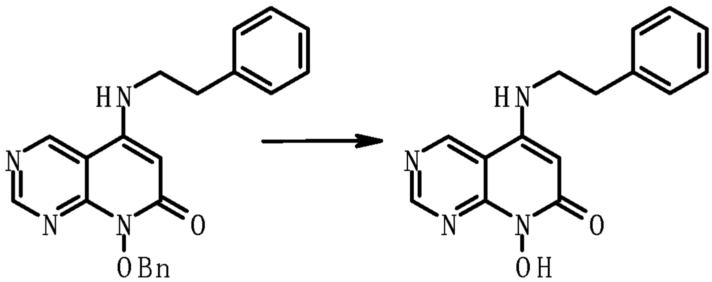

To 8-(benzyloxy)-5-((2-phenylethyl)amino)pyrido[2,3-d]pyrimidin-7(8H)-one (43 mg), 10% palladium-carbon (22 mg) and methanol (15 mL) were added, and the mixture was stirred at room temperature for 1 hours and 20 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-5-((2-phenylethyl)amino)pyrido[2,3-d]pyrimidin-7(8H)-one (16 mg).
$^1$H-NMR (DMSO-$d_6$) δ value: 2.94 (t, J=7.4 Hz, 2H), 3.16-3.50 (m, 2H), 5.57 (s, 1H), 7.08-7.40 (m, 5H), 7.40-7.54 (m, 1H), 9.00 (s, 1H), 9.30 (s, 1H), 10.64 (brs, 1H).

Example 18

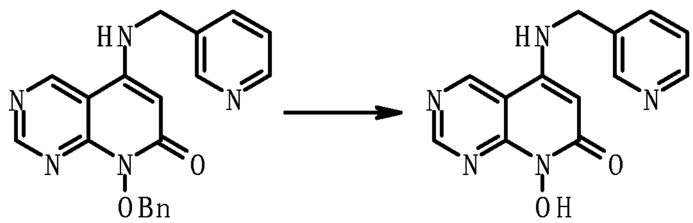

To 8-(benzyloxy)-5-((pyridin-3-ylmethyl)pyrido[2,3-d]pyrimidin-7(8H)-one (20 mg), 10% palladium-carbon (10 mg) and methanol (10 mL) were added, and the mixture was stirred at room temperature for 1 hours and 35 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a gray solid of 8-hydroxy-5-((pyridin-3-ylmethyl)amino)pyrido[2,3-d]pyrimidin-7(8H)-one (10 mg).
$^1$H-NMR (DMSO-$d_6$) δ value: 4.52 (d, J=5.6 Hz, 2H), 5.47 (s, 1H), 7.32-7.46 (m, 1H), 7.76-7.84 (m, 1H), 8.02-8.12 (m, 1H), 8.46-8.52 (m, 1H), 8.60-8.68 (m, 1H), 9.02 (s, 1H), 9.37 (s, 1H), 10.65 (brs, 1H).

Example 19

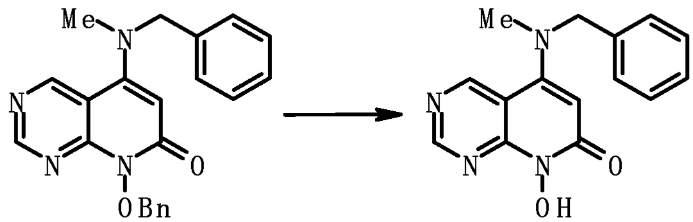

To 5-(benzyl(methyl)amino)-8-(benzyloxy)pyrido[2,3-d]pyrimidin-7(8H)-one (53 mg), 10% palladium-carbon (27 mg) and methanol (12 mL) were added, and the mixture was stirred at room temperature for 25 minutes in a hydrogen atmosphere. Palladium-carbon was filtered off and washed with methanol. The filtrate and the washes were combined, and the solvent was distilled off under reduced pressure. The obtained residue was suspended in a mixed solvent of methanol and diisopropyl ether, and the deposit was collected by filtration to obtain a light brown solid of 5-(benzyl(methyl)amino)-8-hydroxypyrido[2,3-d]pyrimidin-7(8H)-one (28 mg).
$^1$H-NMR (DMSO-$d_6$) δ value: 2.87 (s, 3H), 4.54 (s, 2H), 5.95 (s, 1H), 7.23-7.50 (m, 5H), 9.02 (s, 1H), 9.03 (s, 1H), 10.92 (brs, 1H).

Example 20

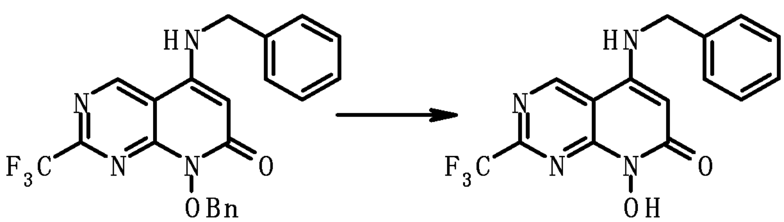

To 5-(benzylamino)-8-(benzyloxy)-2-(trifluoromethyl)pyrido[2,3-d]pyrimidin-7(8H)-one (31 mg), 10% palladium-carbon (16 mg) and methanol (12 mL) were added, and the mixture was stirred at room temperature for 15 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 5-(benzylamino)-8-hydroxy-2-(trifluoromethyl)pyrido[2,3-d]pyrimidin-7(8H)-one (12 mg).
$^1$H-NMR (DMSO-$d_6$) δ value: 4.50 (d, J=5.4 Hz, 2H), 5.50 (s, 1H), 7.22-7.33 (m, 1H), 7.33-7.46 (m, 4H), 8.20-8.34 (m,

1H), 9.55 (s, 1H), 10.93 (brs, 1H).

Example 21

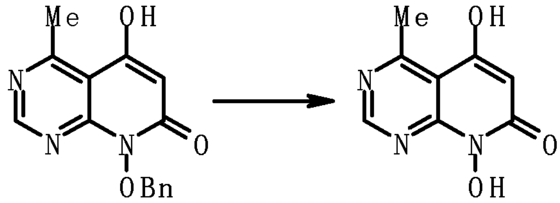

To 8-(benzyloxy)-5-hydroxy-4-methylpyrido[2,3-d]pyrimidin-7(8H)-one (38 mg), 10% palladium-carbon (19 mg) and methanol (20 mL) were added, and the mixture was stirred at room temperature for 7 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a greenish yellow solid of 5,8-dihydroxy-4-methyl pyrido[2,3-d]pyrimidin-7(8H)-one (12 mg).
$^1$H-NMR (DMSO-$d_6$) δ value: 2.85 (s, 3H), 5.91 (s, 1H), 8.85 (s, 1H), 10.83 (brs, 1H).

Example 22

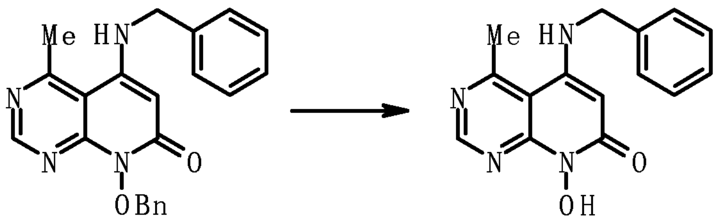

To 5-(benzylamino)-8-(benzyloxy)-4-methylpyrido[2,3-d]pyrimidin-7(8H)-one (52 mg), 10% palladium-carbon (26 mg) and methanol (20 mL) were added, and the mixture was stirred at room temperature for 8 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a light gray solid of 5-(benzylamino)-8-hydroxy-4-methylpyrido[2,3-d]pyrimidin-7(8H)-one (30 mg).
$^1$H-NMR (DMSO-$d_6$) δ value: 2.99 (s, 3H), 4.46 (s, 2H), 5.39 (s, 1H), 6.76-6.94 (m, 1H), 7.20-7.50 (m, 5H), 8.78 (s, 1H), 10.35 (brs, 1H).

Example 23

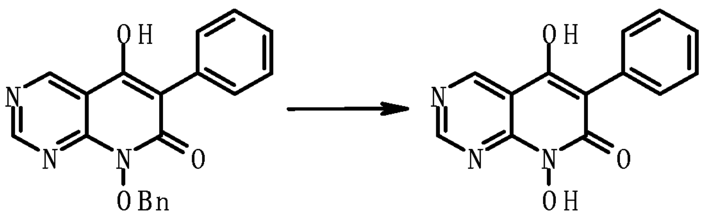

To 8-(benzyloxy)-5-hydroxy-6-phenylpyrido[2,3-d]pyrimidin-7(8H)-one (50 mg), 10% palladium-carbon (25 mg) and methanol (30 mL) were added, and the mixture was stirred at room temperature for 7 minutes in a hydrogen atmosphere. Palladium-carbon was filtered off and washed with methanol. The filtrate and the washes were combined,

and the solvent was distilled off under reduced pressure. The obtained residue was suspended in a mixed solvent of 2-propanol and diisopropyl ether, and the resulting solid was collected by filtration to obtain a pale greenish yellow solid of 5,8-dihydroxy-6-phenylpyrido[2,3-d]pyrimidin-7(8H)-one (19 mg). $^1$H-NMR (DMSO-$d_6$) δ value: 7.29-7.48 (m, 5H), 9.08 (s, 1H), 9.22 (s, 1H), 10.98 (brs, 1H).

Example 24

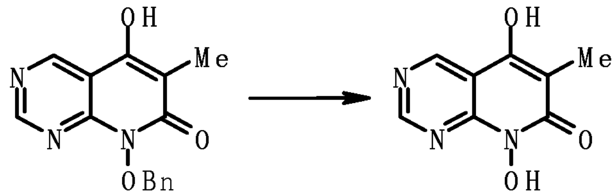

To 8-(benzyloxy)-5-hydroxy-6-methylpyrido[2,3-d]pyrimidin-7(8H)-one (30 mg), 10% palladium-carbon (15 mg) and methanol (20 mL) were added, and the mixture was stirred at room temperature for 7 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a gray solid of 5,8-dihydroxy-6-methyl pyrido[2,3-d]pyrimidin-7(8H)-one (10 mg).
$^1$H-NMR (DMSO-$d_6$) δ value: 2.06 (s, 3H), 9.04 (s, 1H), 9.15 (s, 1H), 10.95 (brs, 1H).

Example 25

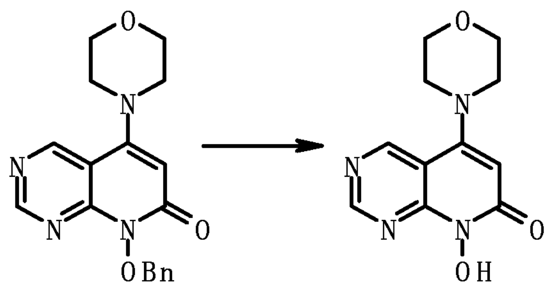

To 8-(benzyloxy)-5-(morpholin-4-yl)pyrido[2,3-d]pyrimidin-7(8H)-one (54 mg), 10% palladium-carbon (27 mg) and methanol (20 mL) were added, and the mixture was stirred at room temperature for 11 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a brown solid of 8-dihydroxy-5-(morpholin-4-yl)pyrido[2,3-d]pyrimidin-7(8H)-one (26 mg).
$^1$H-NMR (DMSO-$d_6$) δ value: 3.10-3.18 (m, 4H), 3.78-3.86 (m, 4H), 6.11 (s, 1H), 9.06 (s, 2H), 11.02 (brs, 1H).

Example 26

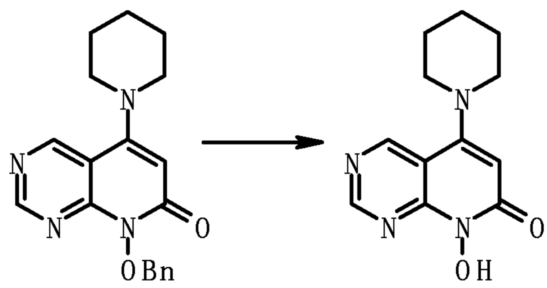

To 8-(benzyloxy)-5-(piperidin-1-yl)pyrido[2,3-d]pyrimidin-7(8H)-one (7 mg), 10% palladium-carbon (7 mg) and methanol (20 mL) were added, and the mixture was stirred at room temperature for 20 minutes in a hydrogen atmosphere. Palladium-carbon was filtered off and washed with methanol. The filtrate and the washes were combined, and the solvent was distilled off under reduced pressure to obtain a brown solid of 8-hydroxy-5-(piperidin-1-yl)pyrido[2,3-d]pyrimidin-7(8H)-one (3 mg).
$^1$H-NMR ($CD_3OD$) $\delta$ value: 1.40-1.70 (m, 2H), 1.70-1.80 (m, 4H), 3.08-3.15 (m, 4H), 6.03 (s, 1H), 8.91 (s, 1H), 8.93 (s, 1H).

Example 27

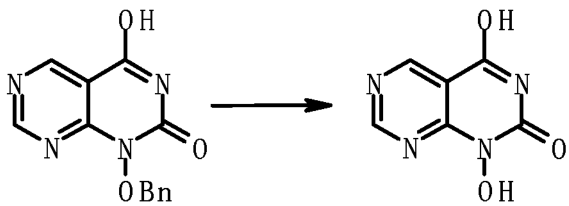

To 1-(benzyloxy)-4-hydroxypyrimido[4,5-d]pyrimidin-2(1H)-one (21 mg), 10% palladium-carbon (11 mg), ethanol (3.2 mL) and ethyl acetate (3.2 mL) were added, and the mixture was stirred at room temperature for 1 hour and 30 minutes in a hydrogen atmosphere. Palladium-carbon was filtered off and washed with a mixed solvent of ethyl acetate and dioxane. The filtrate and the washes were combined, and the solvent was distilled off under reduced pressure. The obtained residue was suspended in a mixed solvent of 2-propanol and ethyl acetate, and the deposit was collected by filtration to obtain a pale yellow solid of 1,4-dihydroxypyrimido[4,5-d]pyrimidin-2(1H)-one (8 mg).
$^1$H-NMR (DMSO-$d_6$) $\delta$ value: 9.06 (s, 1H), 9.15 (s, 1H), 11.11 (brs, 1H), 12.01 (brs, 1H).

Example 28

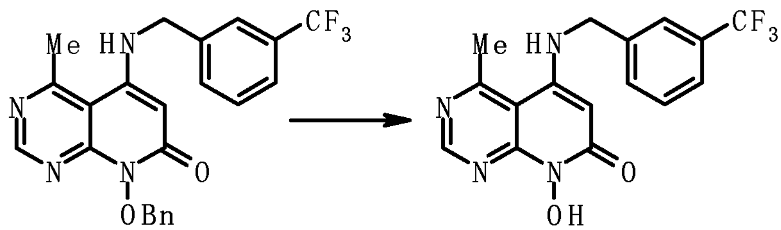

To 8-(benzyloxy)-4-methyl-5-((3-(trifluoromethyl)benzyl)amino) pyrido[2,3-d]pyrimidin-7(8H)-one (20 mg), 20% palladium hydroxide-carbon (10 mg) and methanol (20 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale green solid of 8-hydroxy-4-methyl-5-((3-(trifluoromethyl)benzyl)amino)pyrido[2,3-d]pyrimidin-7(8H)-one (4 mg).
$^1$H-NMR (DMSO-$d_6$) $\delta$ value: 3.01 (s, 3H), 4.58 (d, J=5.6 Hz, 2H), 5.41 (s, 1H), 6.86-6.96 (m, 1H), 7.56-7.70 (m, 2H),

7.70-7.78 (m, 1H), 7.81 (s, 1H), 8.81 (s, 1H), 10.55 (brs, 1H).

Example 29

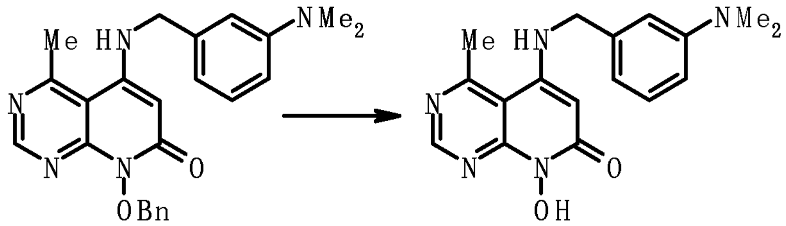

To 8-(benzyloxy)-5-((3-(dimethylamino)benzyl)amino)-4-methylpyrido[2,3-d]pyrimidin-7(8H)-one (20 mg), 20% palladium hydroxide-carbon (10 mg), methanol (16 mL) and dioxane (8 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a light brown solid of 5-((3-(dimethylamino)benzyl)amino)-8-hydroxy-4-methylpyrido[2,3-d]pyrimidin-7(8H)-one (13 mg).
$^1$H-NMR (DMSO-$d_6$) δ value: 2.89 (s, 6H), 2.99 (s, 3H), 4.37 (d, J=4.8 Hz, 2H), 5.44 (s, 1H), 6.59-6.67 (m, 1H), 6.67-6.74 (m, 1H), 6.74-6.84 (m, 2H), 7.12-7.20 (m, 1H), 8.79 (s, 1H), 10.57 (brs, 1H).

Example 30

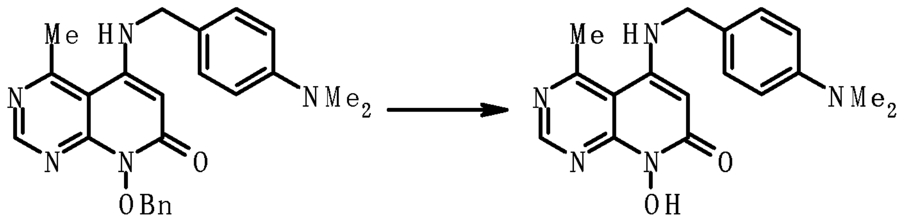

To 8-(benzyloxy)-5-((4-(dimethylamino)benzyl)amino)-4-methylpyrido[2,3-d]pyrimidin-7(8H)-one (20 mg), 20% palladium hydroxide-carbon (10 mg), methanol (50 mL) and dioxane (25 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale green solid of 5-((4-(dimethylamino)benzyl)amino)-8-hydroxy-4-methylpyrido[2,3-d]pyrimidin-7(8H)-one (9 mg).
$^1$H-NMR (DMSO-$d_6$) δ value: 2.87 (s, 6H), 2.96 (s, 3H), 4.31 (d, J=5.6 Hz, 2H), 5.44 (s, 1H), 6.67-6.76 (m, 3H), 7.22-7.27 (m, 2H), 8.78 (s, 1H), 10.57 (brs, 1H).

Example 31

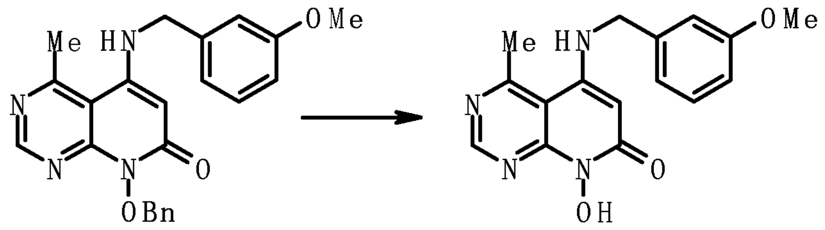

To 8-(benzyloxy)-5-((3-methoxybenzyl)amino)-4-methylpyrido[2,3-d]pyrimidin-7(8H)-one (40 mg), 20% palladium hydroxide-carbon (20 mg) and methanol (25 mL) were added, and the mixture was stirred at room temperature for 7 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a brown solid of 8-hydroxy-5-((3-methoxybenzyl)amino)-4-methylpyrido[2,3-d]pyrimidin-7(8H)-one (19 mg).
$^1$H-NMR (DMSO-d$_6$) δ value: 3.01 (s, 3H), 3.74 (s, 3H), 4.43 (d, J=5.6 Hz, 2H), 5.40 (s, 1H), 6.80-6.90 (m, 2H), 6.96-7.04 (m, 2H), 7.23-7.32 (m, 1H), 8.79 (s, 1H).

Example 32

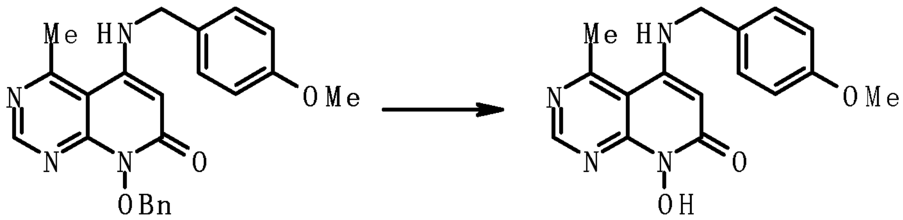

To 8-(benzyloxy)-5-((4-methoxybenzyl)amino)-4-methylpyrido[2,3-d]pyrimidin-7(8H)-one (52 mg), 20% palladium hydroxide-carbon (26 mg) and methanol (25 mL) were added, and the mixture was stirred at room temperature for 7 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and ethyl acetate, and the deposit was collected by filtration to obtain a pale yellow solid of 8-hydroxy-5-((4-methoxybenzyl)amino)-4-methylpyrido[2,3-d]pyrimidin-7(8H)-one (9 mg).
$^1$H-NMR (DMSO-d$_6$) δ value: 2.98 (s, 3H), 3.74 (s, 3H), 4.38 (d, J=5.4 Hz, 2H), 5.42 (s, 1H), 6.77 (t, J=5.2 Hz, 1H), 6.90-6.96 (m, 2H), 7.32-7.38 (m, 2H), 8.79 (s, 1H), 10.57 (brs, 1H).

Example 33

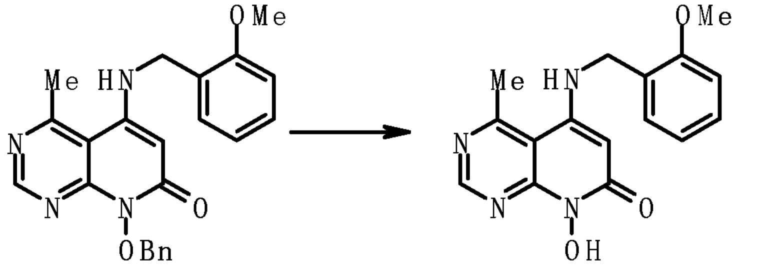

To 8-(benzyloxy)-5-((2-methoxybenzyl)amino)-4-methylpyrido[2,3-d]pyrimidin-7(8H)-one (40 mg), 20% palladium hydroxide-carbon (20 mg) and methanol (25 mL) were added, and the mixture was stirred at room temperature for 7 minutes in a hydrogen atmosphere. Palladium hydroxide-carbon was filtered off and washed with methanol. The filtrate and the washes were combined, and the solvent was distilled off under reduced pressure. The obtained residue

was suspended in a mixed solvent of methanol, 2-propanol and diisopropyl ether, and the deposit was collected by filtration to obtain a pale yellow solid of 8-hydroxy-5-((2-methoxybenzyl)amino)-4-methylpyrido[2,3-d]pyrimidin-7(8H)-one (19 mg).
$^1$H-NMR (DMSO-$d_6$) δ value: 2.97 (s, 3H), 3.88 (s, 3H), 4.40 (d, J=5.4 Hz, 2H), 5.34 (s, 1H), 6.71-6.78 (m, 1H), 6.88-6.97 (m, 1H), 7.03-7.10 (m, 1H), 7.24-7.36 (m, 2H), 8.79 (s, 1H), 10.57 (brs, 1H).

Example 34

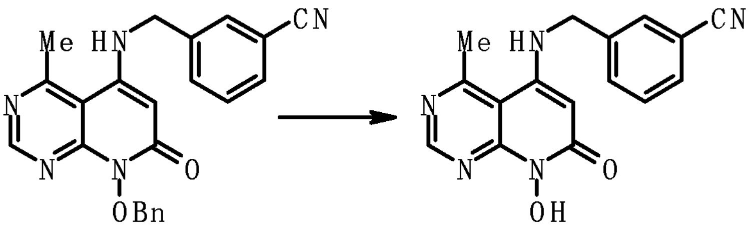

To 3-(((8-(benzyloxy)-4-methyl-7-oxo-7,8-dihydropyrido[2,3-d]pyrimidin-5-yl)amino)methyl)benzonitrile (11 mg), 20% palladium hydroxide-carbon (6 mg), methanol (10 mL) and dioxane (5 mL) were added, and the mixture was stirred at room temperature for 5 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a gray solid of 3-(((8-(benzyloxy)-4-methyl-7-oxo-7,8-dihydropyrido[2,3-d]pyrimidin-5-yl)amino)methyl)benzonitrile (4mg).
$^1$H-NMR (DMSO-$d_6$) δ value: 3.01 (s, 3H), 4.51-4.60 (m, 2H), 5.36 (s, 1H), 6.86-6.94 (m, 1H), 7.54-7.64 (m, 1H), 7.70-7.83 (m, 2H), 7.88-7.95 (m, 1H), 8.81 (s, 1H), 10.56 (brs, 1H).

Example 35

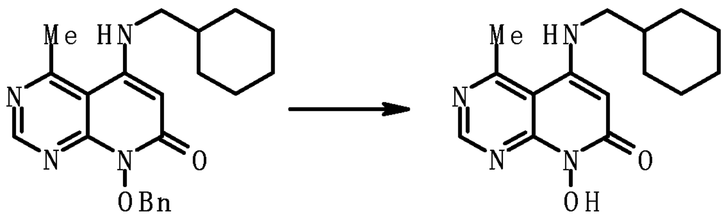

To 8-(benzyloxy)-5-((cyclohexylmethyl)amino)-4-methylpyrido[2,3-d]pyrimidin-7(8H)-one (40 mg), 20% palladium hydroxide-carbon (20 mg), methanol (20 mL) and dioxane (10 mL) were added, and the mixture was stirred at room temperature for 7 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a white solid of 5-((cyclohexylmethyl)amino)-8-hydroxy-4-methylpyrido[2,3-d]pyrimidin-7(8H)-one (15 mg). $^1$H-NMR (DMSO-$d_6$) δ value: 0.92-1.10 (m, 2H), 1.10-1.33 (m, 3H), 1.58-1.88 (m, 6H), 2.90 (s, 3H), 2.95-3.02 (m, 2H), 5.51 (s, 1H), 6.08-6.18 (m, 1H), 8.77 (s, 1H), 10.57 (brs, 1H).

Example 36

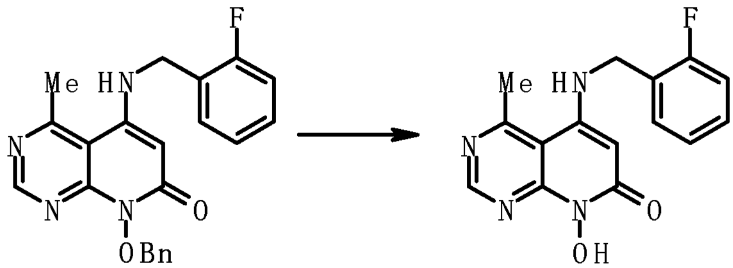

To 8-(benzyloxy)-5-((2-fluorobenzyl)amino)-4-methylpyrido[2,3-d]pyrimidin-7(8H)-one (48 mg), 20% palladium hydroxide-carbon (24 mg), methanol (20 mL) and dioxane (10 mL) were added, and the mixture was stirred at room temperature for 5 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a yellow solid of 5-((2-fluorobenzyl)amino)-8-hydroxy-4-methylpyrido[2,3-d]pyrimidin-7(8H)-one (4 mg).

$^1$H-NMR (DMSO-$d_6$) $\delta$ value: 2.96 (s, 3H), 4.44-4.53 (m, 2H), 5.42 (s, 1H), 6.77-6.85 (m, 1H), 7.13-7.50 (m, 4H), 8.79 (s, 1H).

Example 37

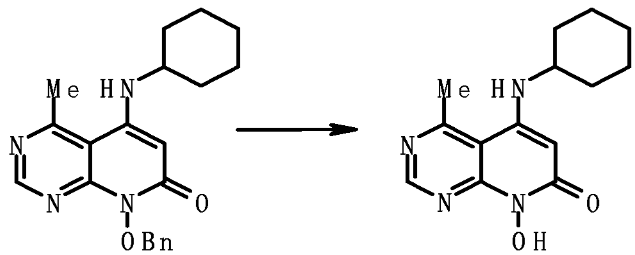

To 8-(benzyloxy)-5-(cyclohexylamino)-4-methylpyrido[2,3-d]pyrimidin-7(8H)-one (28 mg), 20% palladium hydroxide-carbon (14 mg), methanol (20 mL) and dioxane (10 mL) were added, and the mixture was stirred at room temperature for 3 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a greenish yellow solid of 5-(cyclohexylamino)-8-hydroxy-4-methylpyrido[2,3-d]pyrimidin-7(8H)-one (4mg).

$^1$H-NMR (DMSO-$d_6$) $\delta$ value: 1.12-2.02 (m, 10H), 2.90 (s, 3H), 3.17 (d, J=5.1 Hz, 1H), 5.58 (s, 1H), 5.79-5.87 (m, 1H), 8.77 (s, 1H).

Example 38

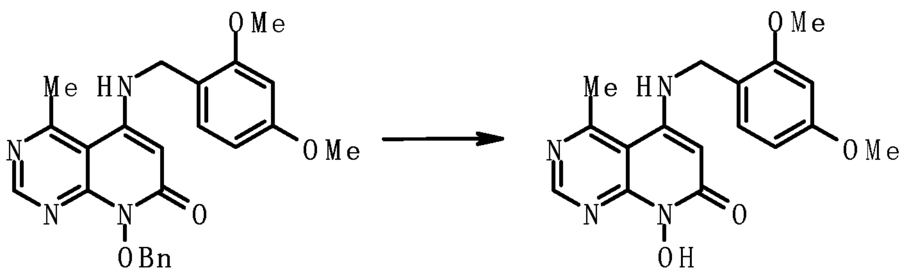

To 8-(benzyloxy)-5-((2,4-dimethoxybenzyl)amino)-4-methylpyrido[2,3-d]pyrimidin-7(8H)-one (26 mg), 20% palladium hydroxide-carbon (13 mg), methanol (20 mL) and dioxane (10 mL) were added, and the mixture was stirred at room temperature for 6 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a gray solid of 5-((2,4-dimethoxybenzyl)amino)-8-hydroxy-4-methylpyrido[2,3-d]pyrimidin-7(8H)-one (12 mg).
$^1$H-NMR (DMSO-$d_6$) δ value: 2.95 (s, 3H), 3.76 (s, 3H), 3.87 (s, 3H), 4.31 (d, J=4.6 Hz, 2H), 5.39 (s, 1H), 6.47-6.53 (m, 1H), 6.60-6.67 (m, 2H), 7.22 (d, J=8.3 Hz, 1H), 8.79 (s, 1H), 10.21 (s, 1H).

Example 39

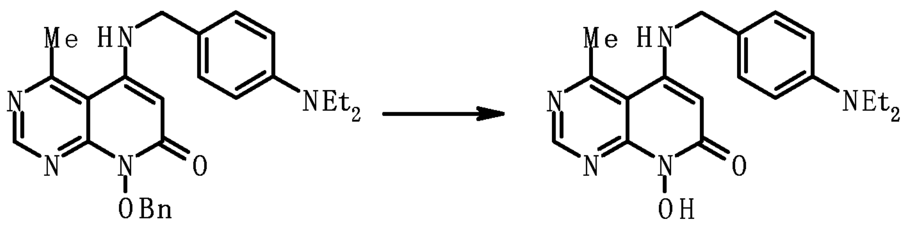

To 8-(benzyloxy)-5-((4-(diethylamino)benzyl)amino)-4-methylpyrido[2,3-d]pyrimidin-7(8H)-one (35 mg), 20% palladium hydroxide-carbon (17 mg), methanol (20 mL) and dioxane (10 mL) were added, and the mixture was stirred at room temperature for 5 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale green solid of 5-((4-(diethylamino)benzyl)amino)-8-hydroxy-4-methylpyrido[2,3-d]pyrimidin-7(8H)-one (10 mg). $^1$H-NMR (DMSO-$d_6$) δ value: 1.07 (t, J=7.0 Hz, 6H), 2.96 (s, 3H), 3.26-3.42 (m, 4H), 4.28 (d, J=5.4 Hz, 2H), 5.46 (s, 1H), 6.60-6.70 (m, 3H), 7.16-7.24 (m, 2H), 8.78 (s, 1H), 10.58 (brs, 1H).

Example 40

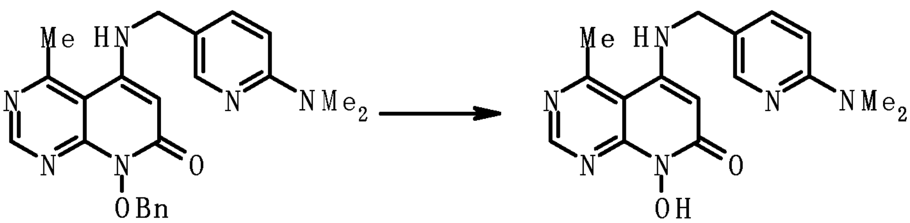

To 8-(benzyloxy)-5-(((6-(dimethylamino)pyridin-3-yl)methyl)amino)-4-methylpyrido[2,3-d]pyrimidin-7(8H)-one (56 mg), 20% palladium hydroxide-carbon (28 mg), methanol (20 mL) and dioxane (10 mL) were added, and the mixture was stirred at room temperature for 5 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a green solid of 5-(((6-(dimethylamino)pyridin-3-yl)methyl)amino)-8-hydroxy-4-methylpyrido[2,3-d]pyrimidin-7(8H)-one (18 mg).
$^1$H-NMR (DMSO-$d_6$) δ value: 2.94 (s, 3H), 3.00 (s, 6H), 4.29 (d, J=5.1 Hz, 2H), 5.50 (s, 1H), 6.58-6.68 (m, 2H), 7.57 (dd, J=8.8, 2.4 Hz, 1H), 8.15 (d, J=2.0 Hz, 1H), 8.77 (s, 1H), 10.62 (brs, 1H).

Example 41

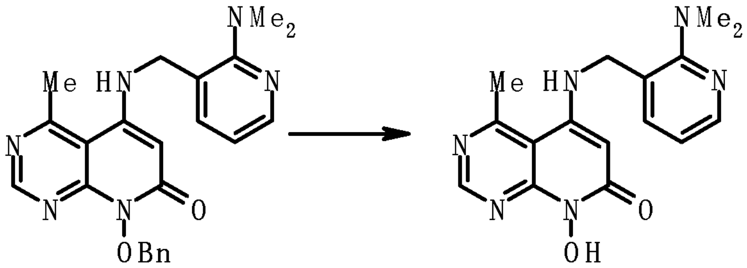

To 8-(benzyloxy)-5-(((2-(dimethylamino)pyridin-3-yl)methyl)amino)-4-methylpyrido[2,3-d]pyrimidin-7(8H)-one (28 mg), 20% palladium hydroxide-carbon (14 mg), methanol (20 mL) and dioxane (10 mL) were added, and the mixture was stirred at room temperature for 5 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a green solid of 5-(((2-(dimethylamino)pyridin-3-yl)methyl)amino)-8-hydroxy-4-methylpyrido[2,3-d]pyrimidin-7(8H)-one (12 mg).
$^1$H-NMR (DMSO-$d_6$) δ value: 2.83 (s, 6H), 2.98 (s, 3H), 4.41 (d, J=5.4 Hz, 2H), 5.27 (s, 1H), 6.86-6.92 (m, 1H), 6.94 (dd, J=7.4, 4.8 Hz, 1H), 7.66-7.76 (m, 1H), 8.14-8.21 (m, 1H), 8.79 (s, 1H), 10.57 (brs, 1H).

Example 42

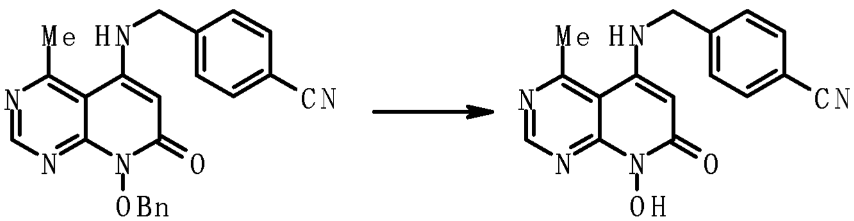

To 4-(((8-(benzyloxy)-4-methyl-7-oxo-7,8-dihydropyrido[2,3-d]pyrimidin-5-yl)amino)methyl)benzonitrile (57 mg), 20% palladium hydroxide-carbon (22 mg), methanol (20 mL) and dioxane (10 mL) were added, and the mixture was stirred at room temperature for 5 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a gray solid of 4-(((8-hydroxy-4-methyl-7-oxo-7,8-dihydropyrido[2,3-d]pyrimidin-5-yl)amino)methyl)benzonitrile (11 mg).
$^1$H-NMR (DMSO-$d_6$) δ value: 3.01 (s, 3H), 4.58 (d, J=5.4 Hz, 2H), 5.33 (s, 1H), 6.88-6.96 (m, 1H), 7.58-7.65 (m, 2H), 7.80-7.86 (m, 2H), 8.81 (s, 1H), 10.59 (brs, 1H).

Example 43

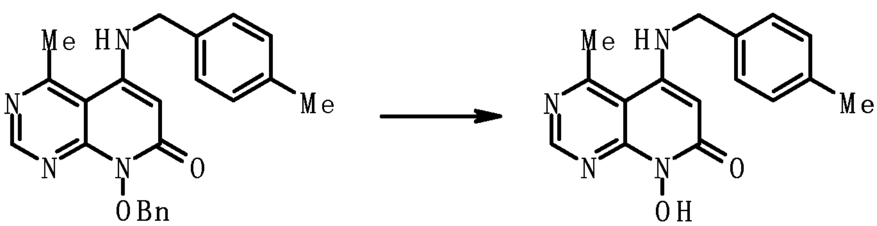

To 8-(benzyloxy)-4-methyl-5-((4-methylbenzyl) amino)pyrido[2,3-d]pyrimidin-7(8H)-one (46 mg), 20% palladium hydroxide-carbon (23 mg), methanol (20 mL) and dioxane (10 mL) were added, and the mixture was stirred at room temperature for 7 minutes in a hydrogen atmosphere. Palladium hydroxide-carbon was filtered off and washed with methanol. The filtrate and the washes were combined, and the solvent was distilled off under reduced pressure. The

obtained residue was suspended in a mixed solvent of methanol, 2-propanol and diisopropyl ether, and the deposit was collected by filtration to obtain a light brown solid of 8-hydroxy-4-methyl-5-((4-methylbenzyl)amino)pyrido[2,3-d]pyrimidin-7(8H)-one (24 mg). $^1$H-NMR (DMSO-$d_6$) δ value: 2.28 (s, 3H), 2.99 (s, 3H), 4.41 (d, J=5.4 Hz, 2H), 5.39 (s, 1H), 6.76-6.85 (m, 1H), 7.14-7.20 (m, 2H), 7.28-7.34 (m, 2H), 8.79 (s, 1H), 10.53 (brs, 1H).

Example 44

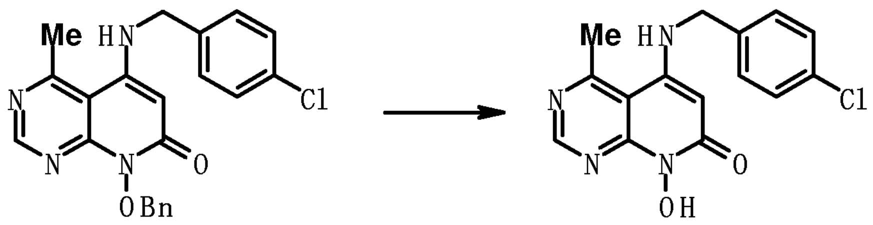

To 8-(benzyloxy)-5-((4-chlorobenzyl)amino)-4-methyl pyrido[2,3-d]pyrimidin-7(8H)-one (47 mg), 20% palladium hydroxide-carbon (23 mg), methanol (20 mL) and dioxane (10 mL) were added, and the mixture was stirred at room temperature for 5 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a light brown solid of 5-((4-chlorobenzyl)amino)-8-hydroxy-4-methylpyrido[2,3-d]pyrimidin-7(8H)-one (12mg).
$^1$H-NMR (DMSO-$d_6$) δ value: 2.99 (s, 3H), 4.47 (d, J=5.6 Hz, 2H), 5.37 (s, 1H), 6.82-6.92 (m, 1H), 7.38-7.48 (m, 4H), 8.80 (s, 1H), 10.59 (brs, 1H).

Example 45

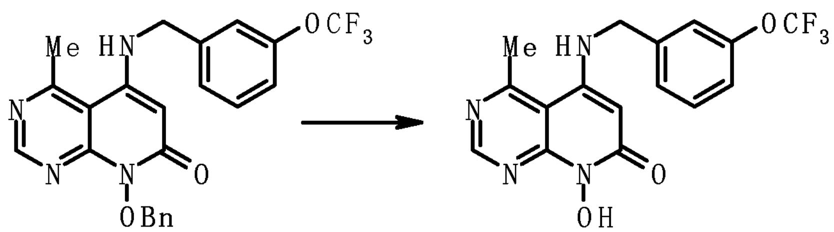

To 8-(benzyloxy)-4-methyl-5-((3-(trifluoromethoxy)benzyl)amino)pyrido[2,3-d]pyrimidin-7(8H)-one (54 mg), 20% palladium hydroxide-carbon (27 mg), methanol (20 mL) and dioxane (10 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of ethyl acetate, 2-propanol and diisopropyl ether, and the deposit was collected by filtration to obtain a green solid of 8-hydroxy-4-methyl-5-((3-(trifluoromethoxy)benzyl)amino)pyrido[2,3-d]pyrimidin-7(8H)-one (16 mg).
$^1$H-NMR (DMSO-$d_6$) δ value: 3.00 (s, 3H), 4.53 (d, J=5.4 Hz, 2H), 5.40 (s, 1H), 6.84-6.94 (m, 1H), 7.24-7.32 (m, 1H), 7.40-7.56 (m, 3H), 8.80 (s, 1H).

Example 46

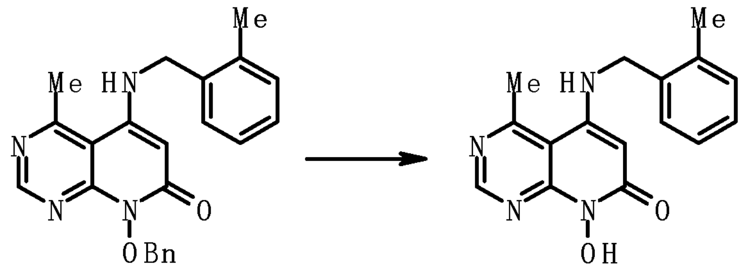

To 8-(benzyloxy)-4-methyl-5-((2-methylbenzyl)amino)pyrido[2,3-d]pyrimidin-7(8H)-one (48 mg), 20% palladium hydroxide-carbon (24 mg), methanol (20 mL) and dioxane (10 mL) were added, and the mixture was stirred at room temperature for 15 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a greenish yellow solid of 8-hydroxy-4-methyl-5-((2-methylbenzyl)amino)pyrido[2,3-d]pyrimidin-7(8H)-one (28 mg). $^{1}$H-NMR (DMSO-$d_6$) δ value: 2.37 (s, 3H), 2.95 (s, 3H), 4.38 (d, J=5.1 Hz, 2H), 5.38 (s, 1H), 6.48-6.62 (m, 1H), 7.12-7.30 (m, 4H), 8.70 (s, 1H).

Example 47

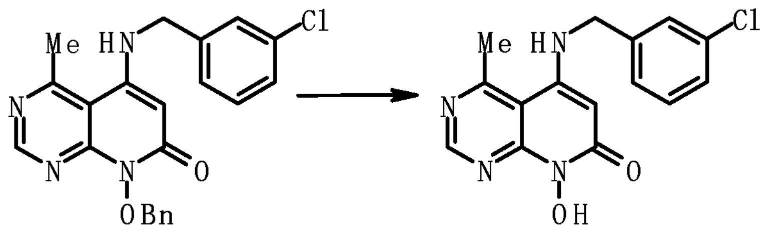

To 8-(benzyloxy)-5-((3-chlorobenzyl)amino) -4-methylpyrido[2,3-d]pyrimidin-7(8H)-one (45 mg), 20% palladium hydroxide-carbon (22 mg), methanol (20 mL) and dioxane (10 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a yellow solid of 5-((3-chlorobenzyl)amino)-8-hydroxy-4-methylpyrido[2,3-d]pyrimidin-7(8H)-one (14mg).
$^{1}$H-NMR (DMSO-$d_6$) δ value: 3.00 (s, 3H), 4.49 (d, J=5.4 Hz, 2H), 5.38 (s, 1H), 6.82-6.94 (m, 1H), 7.22-7.47 (m, 3H), 7.50 (s, 1H), 8.80 (s, 1H), 10.59 (brs, 1H).

Example 48

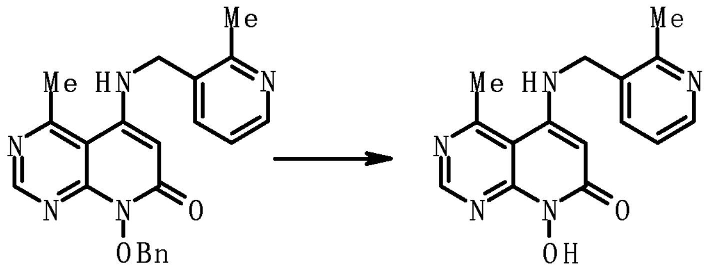

To 8-(benzyloxy)-4-methyl-5-(((2-methylpyridin-3-yl)methyl)amino)pyrido[2,3-d]pyrimidin-7(8H)-one (20 mg), 20% palladium hydroxide-carbon (10 mg), methanol (20 mL) and dioxane (10 mL) were added, and the mixture was

stirred at room temperature for 15 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a white solid of 8-hydroxy-4-methyl-5-(((2-methylpyridin-3-yl)methyl)amino)pyrido[2,3-d]pyrimidin-7(8H)-one (9mg).
$^1$H-NMR (DMSO-$d_6$) δ value: 2.55 (s, 3H), 2.98 (s, 3H), 4.45 (d, J=4.9 Hz, 2H), 5.38 (s, 1H), 6.72-6.80 (m, 1H), 7.16-7.22 (m, 1H), 7.56-7.64 (m, 1H), 8.32-8.38 (m, 1H), 8.81 (s, 1H), 10.61 (brs, 1H).

Example 49

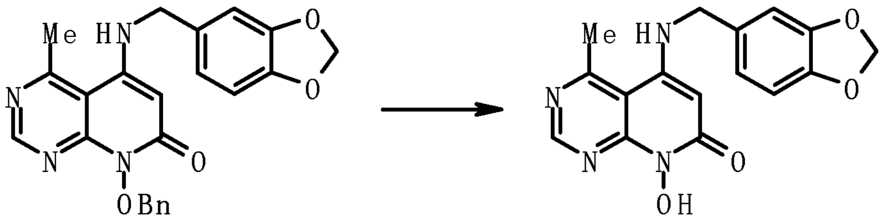

To 5-(((1,3-benzodioxol-5-yl)methyl)amino)-8-(benzyloxy)-4-methylpyrido[2,3-d]pyrimidin-7(8H)-one (42 mg), 20% palladium hydroxide-carbon (21 mg), methanol (20 mL) and dioxane (10 mL) were added, and the mixture was stirred at room temperature for 13 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a light brown solid of 5-(((1,3-benzodioxol-5-yl)methyl)amino)-8-hydroxy-4-methylpyrido[2,3-d]pyrimidin-7(8H)-one (12mg).
$^1$H-NMR (DMSO-$d_6$) δ value: 2.98 (s, 3H), 4.36 (d, J=5.4 Hz, 2H), 5.42 (s, 1H), 6.00 (s, 2H), 6.72-6.80 (m, 1H), 6.85-6.95 (m, 2H), 7.02 (s, 1H), 8.79 (s, 1H), 10.56 (brs, 1H).

Example 50

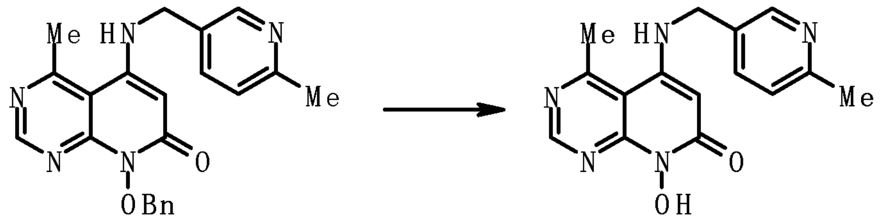

To 8-(benzyloxy)-4-methyl-5-(((6-methylpyridin-3-yl)methyl)amino)pyrido[2,3-d]pyrimidin-7(8H)-one (45 mg), 20% palladium hydroxide-carbon (22 mg), methanol (20 mL) and dioxane (10 mL) were added, and the mixture was stirred at room temperature for 15 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a brown solid of 8-hydroxy-4-methyl-5-(((6-methylpyridin-3-yl)methyl)amino)pyrido[2,3-d]pyrimidin-7(8H)-one (13mg).
$^1$H-NMR (DMSO-$d_6$) δ value: 2.45 (s, 3H), 2.98 (s, 3H), 4.46 (d, J=5.6 Hz, 2H), 5.43 (s, 1H), 6.77-6.86 (m, 1H), 7.25 (d, J=8.0 Hz, 1H), 7.69-7.78 (m, 1H), 8.50-8.56 (m, 1H), 8.80 (s, 1H), 10.60 (brs, 1H).

Example 51

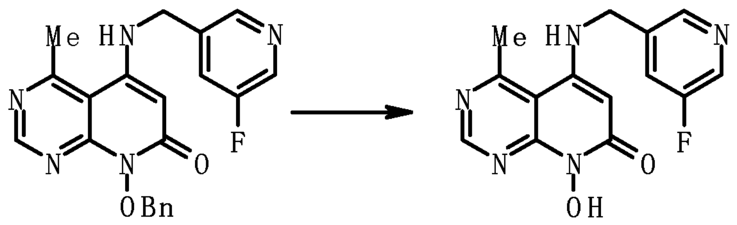

To 8-(benzyloxy) -5-(((5-fluoropyridin-3-yl)methyl)amino)-4-methylpyrido[2,3-d]pyrimidin-7(8H)-one (26 mg), 20% palladium hydroxide-carbon (13 mg), methanol (20 mL) and dioxane (10 mL) were added, and the mixture was stirred at room temperature for 20 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a white solid of 5-(((5-fluoropyridin-3-yl)methyl)amino) -8-hydroxy -4-methylpyrido[2,3-d]pyrimidin-7(8H)-one (5mg).
$^1$H-NMR (DMSO-$d_6$) δ value: 3.00 (s, 3H), 4.53-4.63 (m, 2H), 5.44 (s, 1H), 6.82-6.91 (m, 1H), 7.76-7.84 (m, 1H), 8.46-8.53 (m, 1H), 8.53-8.59 (m, 1H), 8.81 (s, 1H), 10.61 (brs, 1H).

Example 52

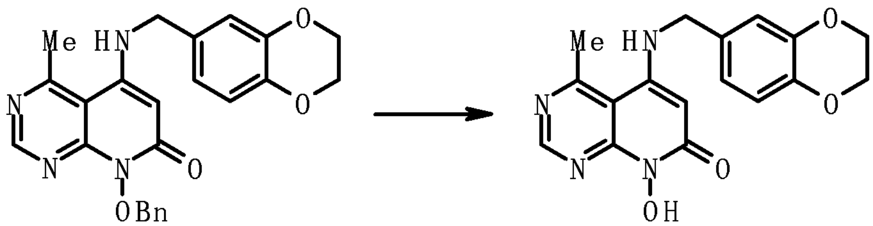

To 8-(benzyloxy)-5-(((2,3-dihydro-1,4-benzodioxin-6-yl)methyl)amino)-4-methylpyrido[2,3-d]pyrimidin-7(8H)-one (57 mg), 20% palladium hydroxide-carbon (28 mg), methanol (20 mL) and dioxane (10 mL) were added, and the mixture was stirred at room temperature for 15 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a gray solid of 5-(((2,3-dihydro-1,4-benzodioxin-6-yl)methyl)amino)-8-hydroxy-4-methylpyrido[2,3-d]pyrimidin-7(8H)-one (18mg).
$^1$H-NMR (DMSO-$d_6$) δ value: 2.97 (s, 3H), 4.22 (s, 4H), 4.33 (d, J=4.9 Hz, 2H), 5.41 (s, 1H), 6.71-6.79 (m, 1H), 6.79-6.95 (m, 3H), 8.78 (s, 1H), 10.62 (brs, 1H).

Example 53

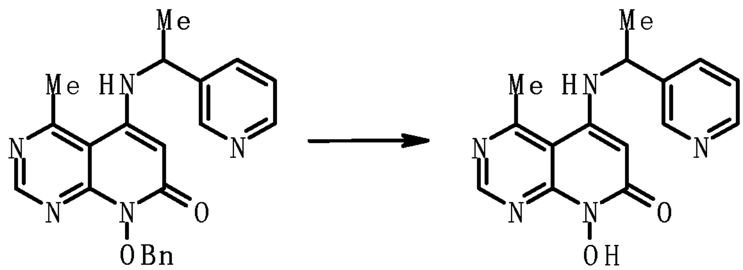

To 8-(benzyloxy)-4-methyl-5-((1-(pyridin-3-yl)ethyl)amino)pyrido[2,3-d]pyrimidin-7(8H)-one (75 mg), 20% palladium hydroxide-carbon (37 mg), methanol (20 mL) and dioxane (10 mL) were added, and the mixture was stirred at room temperature for 25 minutes in a hydrogen atmosphere. Palladium hydroxide-carbon was filtered off and washed

with methanol. The filtrate and the washes were combined, and the solvent was distilled off under reduced pressure. The obtained residue was suspended in a mixed solvent of 2-propanol and diisopropyl ether, and the deposit was collected by filtration to obtain a gray solid of 8-hydroxy-4-methyl-5-((1-(pyridin-3-yl)ethyl)amino)pyrido[2,3-d]pyrimidin-7(8H)-one (4mg).
$^1$H-NMR (DMSO-$d_6$) δ value: 1.58 (d, J=6.6 Hz, 3H), 3.05 (s, 3H), 4.67-4.79 (m, 1H), 5.30 (s, 1H), 6.34-6.45 (m, 1H), 7.36-7.44 (m, 1H), 7.86-7.94 (m, 1H), 8.45-8.52 (m, 1H), 8.69-8.74 (m, 1H), 8.80 (s, 1H), 10.58 (brs, 1H).

Example 54

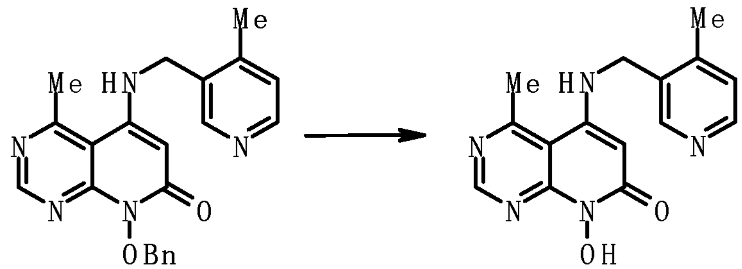

To 8-(benzyloxy)-4-methyl-5-(((4-methylpyridin-3-yl)methyl)amino)pyrido[2,3-d]pyrimidin-7(8H)-one (30 mg), 20% palladium hydroxide-carbon (15 mg), methanol (20 mL) and dioxane (10 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a brown solid of 8-hydroxy-4-methyl-5-(((4-methylpyridin-3-yl)methyl)amino)pyrido[2,3-d]pyrimidin-7(8H)-one (5mg).
$^1$H-NMR (DMSO-$d_6$) δ value: 2.40 (s, 3H), 2.95 (s, 3H), 4.46 (d, J=4.9 Hz, 2H), 5.50 (s, 1H), 6.64-6.71 (m, 1H), 7.28 (d, J=4.9 Hz, 1H), 8.38 (d, J=4.9 Hz, 1H), 8.42 (s, 1H), 8.81 (s, 1H), 10.63 (brs, 1H).

Example 55

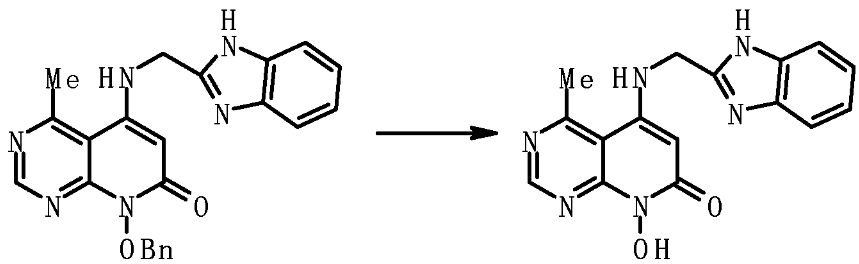

To 5-(((1H-benzimidazol-2-yl)methyl)amino)-8-(benzyloxy)-4-methylpyrido[2,3-d]pyrimidin-7(8H)-one (31 mg), 20% palladium hydroxide-carbon (15 mg), methanol (20 mL) and dioxane (10 mL) were added, and the mixture was stirred at room temperature for 20 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a white solid of 5-(((1H-benzimidazol-2-yl)methyl)amino)-8-hydroxy-4-methylpyrido[2,3-d]pyrimidin-7(8H)-one (4mg).
$^1$H-NMR (DMSO-$d_6$) δ value: 3.07 (s, 3H), 4.69 (d, J=5.6 Hz, 2H), 5.53 (s, 1H), 6.92-7.00 (m, 1H), 7.11-7.22 (m, 2H), 7.41-7.50 (m, 1H), 7.54-7.63 (m, 1H), 8.83 (s, 1H), 10.63 (brs, 1H), 12.34 (brs, 1H).

Example 56

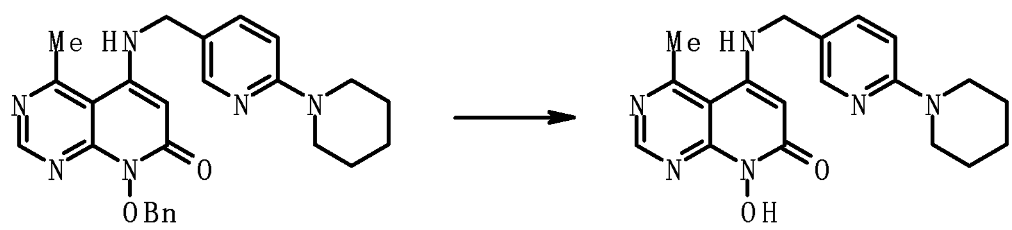

To 8-(benzyloxy)-4-methyl-5-(((6-(piperidin-1-yl)pylidin-3-yl)methyl)amino)pyrido[2,3-d]pyrimidin-7(8H)-one (117 mg), 20% palladium hydroxide-carbon (59 mg), methanol (60 mL) and dioxane (30 mL) were added, and the mixture was stirred at room temperature for 20 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a yellow solid of 8-hydroxy-4-methyl-5-(((6-(piperidin-1-yl)pyridin-3-yl)methyl)amino)pyrido[2,3-d]pyrimidin-7(8H)-one (34mg).
$^1$H-NMR (DMSO-$d_6$) δ value: 1.40-1.68 (m, 6H), 2.94 (s, 3H), 3.20-3.52 (m, 4H), 4.20-4.30 (m, 2H), 5.62 (s, 1H), 6.22-6.40 (m, 1H), 6.74-6.83 (m, 1H), 7.52-7.60 (m, 1H), 8.14 (s, 1H), 8.59 (s, 1H).

Example 57

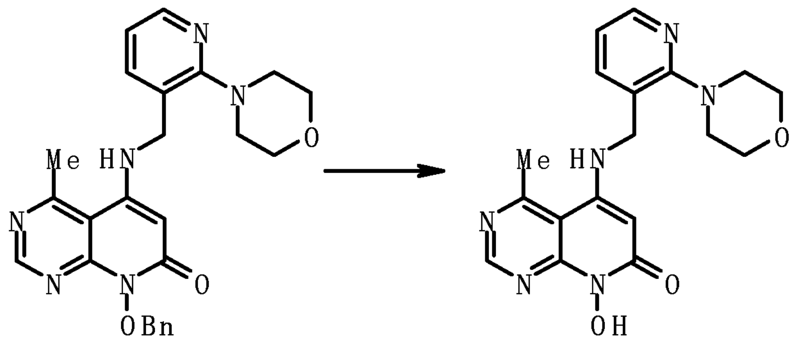

To 8-(benzyloxy)-4-methyl-5-(((2-(morpholin-4-yl)pyridin-3-yl)methyl)amino)pyrido[2,3-d]pyrimidin-7(8H)-one (19 mg), 20% palladium hydroxide-carbon (10 mg), methanol (20 mL) and dioxane (10 mL) were added, and the mixture was stirred at room temperature for 15 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol, ethyl acetate and diisopropyl ether, and the deposit was collected by filtration to obtain a gray solid of 8-hydroxy-4-methyl-5-(((2-(morpholin-4-yl)pyridin-3-yl)methyl)amino)pyrido[2,3-d]pyrimidin-7(8H)-one (6mg).
$^1$H-NMR (DMSO-$d_6$) δ value: 2.98 (s, 3H), 3.05-3.13 (m, 4H), 3.75-3.85 (m, 4H), 4.42 (d, J=5.6 Hz, 2H), 5.41 (s, 1H), 6.85-6.93 (m, 1H), 7.05 (dd, J=7.4, 5.0 Hz, 1H), 7.78 (dd, J=7.6, 1.5 Hz, 1H), 8.23 (dd, J=4.6, 1.7 Hz, 1H), 8.79 (s, 1H), 10.57 (brs, 1H).

Example 58

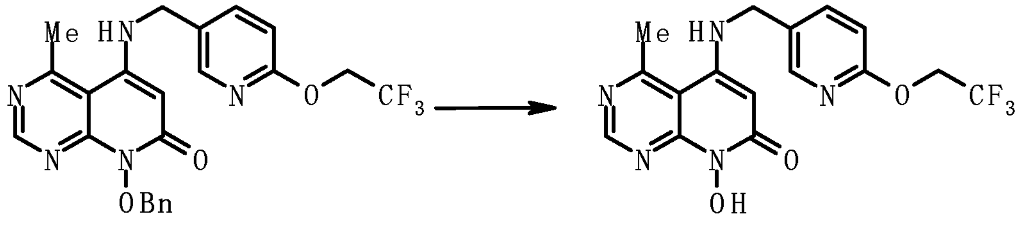

To 8-(benzyloxy)-4-methyl-5-(((6-(2,2,2-trifluoroethoxy)pyridin-3-yl)methyl)amino)pyrido[2,3-d]pyrimidin-7(8H)-one (64 mg), 20% palladium hydroxide-carbon (32 mg), methanol (20 mL) and dioxane (10 mL) were added, and the mixture was stirred at room temperature for 23 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a greenish yellow solid of 8-hydroxy-4-methyl-5-(((6-(2,2,2-trifluoroethoxy)pyridin-3-yl)methyl)amino)pyrido[2,3-d]pyrimidin-7(8H)-one (24mg). $^1$H-NMR (DMSO-$d_6$) $\delta$ value: 2.97 (s, 3H), 4.45 (d, J=5.1 Hz, 2H), 4.91-5.04 (m, 2H), 5.48 (s, 1H), 6.75-6.84 (m, 1H), 6.99 (d, J=8.8 Hz, 1H), 7.84-7.92 (m, 1H), 8.25-8.31 (m, 1H), 8.80 (s, 1H).

Example 59

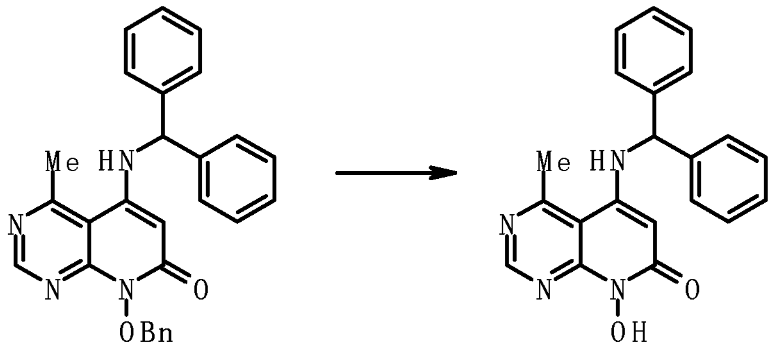

To 8-(benzyloxy)-5-((diphenylmethyl)amino)-4-methylpyrido[2,3-d]pyrimidin-7(8H)-one (60 mg), 20% palladium hydroxide-carbon (30 mg), methanol (20 mL) and dioxane (10 mL) were added, and the mixture was stirred at room temperature for 13 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a gray solid of 5-((diphenylmethyl)amino)-8-hydroxy-4-methylpyrido[2,3-d]pyrimidin-7(8H)-one (12mg).
$^1$H-NMR (DMSO-$d_6$) $\delta$ value: 3.01 (s, 3H), 5.42 (s, 1H), 5.72-5.80 (m, 1H), 6.52-6.64 (m, 1H), 7.21-7.30 (m, 2H), 7.32-7.42 (m, 4H), 7.51-7.60 (m, 4H), 8.75 (s, 1H).

Example 60

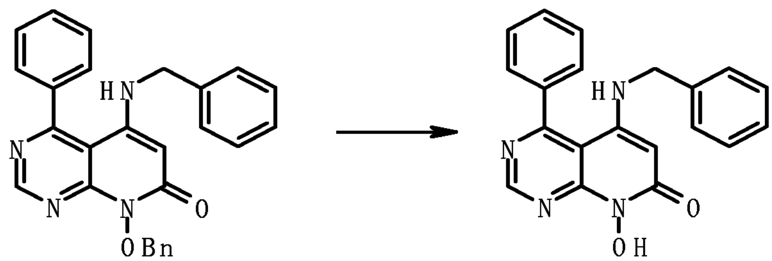

To 5-(benzylamino)-8-(benzyloxy)-4-phenylpyrido[2,3-d]pyrimidin-7(8H)-one (35 mg), 10% palladium hydroxide-carbon (17 mg) and methanol (100 mL) were added, and the mixture was stirred at room temperature for 4 minutes in a hydrogen atmosphere. Palladium-carbon was filtered off and washed with methanol. The filtrate and the washes were combined, and the solvent was distilled off under reduced pressure. The obtained residue was suspended in a mixed solvent of methanol and diisopropyl ether, and the deposit was collected by filtration to obtain a pale yellow solid of 5-(benzylamino)-8-hydroxy-4-phenylpyrido[2,3-d]pyrimidin-7(8H)-one (22mg).
$^1$H-NMR (DMSO-$d_6$) $\delta$ value: 4.11 (d, J=5.1 Hz, 2H), 4.98-5.05 (m, 1H), 5.76 (s, 1H), 6.97-7.07 (m, 2H), 7.21-7.32 (m, 3H), 7.46-7.54 (m, 3H), 7.54-7.63 (m, 2H), 8.98 (s, 1H), 10.79 (brs, 1H).

Example 61

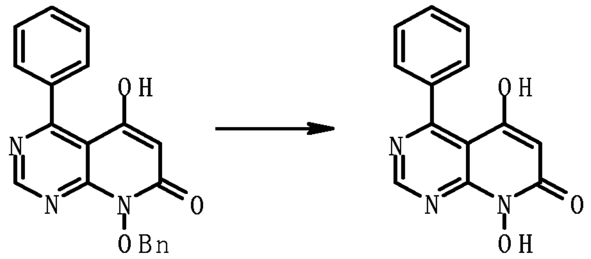

To 8-(benzyloxy)-5-hydroxy-4-phenylpyrido[2,3-d]pyrimidin-7(8H)-one (30 mg), concentrated hydrochloric acid (5 mL) and TFA (5 mL) were added, and the mixture was heated with stirring at 70 to 80°C for 8 hours and 30 minutes. After cooling, the solvent in the reaction mixture was distilled off under reduced pressure. The obtained residue was suspended in a mixed solvent of 2-propanol and diisopropyl ether, and the deposit was collected by filtration to obtain a brown solid of 5,8-dihydroxy-4-phenylpyrido[2,3-d]pyrimidin-7(8H)-one (19 mg). $^1$H-NMR (DMSO-$d_6$) δ value: 5.90 (s, 1H), 7.38-7.57 (m, 5H), 9.05 (s, 1H), 11.54 (brs, 1H).

Example 62

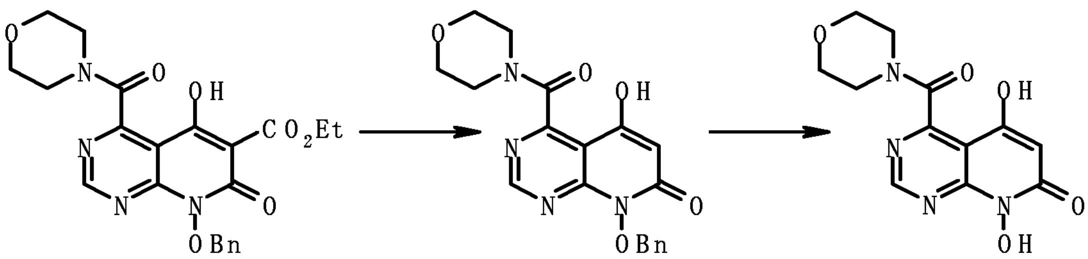

To ethyl 8-(benzyloxy)-5-hydroxy-4-((morpholin-4-yl)carbonyl)-7-oxo-7,8-dihydropyrido[2,3-d]pyrimidine-6-carboxylate (21 mg), 2 mol/L hydrochloric acid (2 mL) and dioxane (2 mL) were added, and the mixture was heated to reflux for 5 hours and 30 minutes. After cooling of the reaction mixture, chloroform and water were added thereto. An organic layer was separated, washed with a saturated aqueous solution of sodium chloride and then dried over sodium sulfate, and the solvent was distilled off under reduced pressure to obtain a pale yellow solid (32 mg). To the obtained solid (32 mg), concentrated hydrochloric acid (3 mL) and TFA (3 mL) were added, and the mixture was heated with stirring at 70 to 80°C for 3 hours and 45 minutes. After cooling, the solvent in the reaction mixture was distilled off under reduced pressure. The obtained residue was suspended in a mixed solvent of 2-propanol and diisopropyl ether, and the deposit was collected by filtration to obtain a brown solid of 5,8-dihydroxy-4-((morpholin-4-yl)carbonyl)pyrido[2,3-d]pyrimidin-7(8H)-one (13 mg).
$^1$H-NMR (DMSO-$d_6$) δ value: 3.04-3.80 (m, 8H), 5.93 (s, 1H), 9.05 (s, 1H), 11.02 (brs, 1H).

Example 63

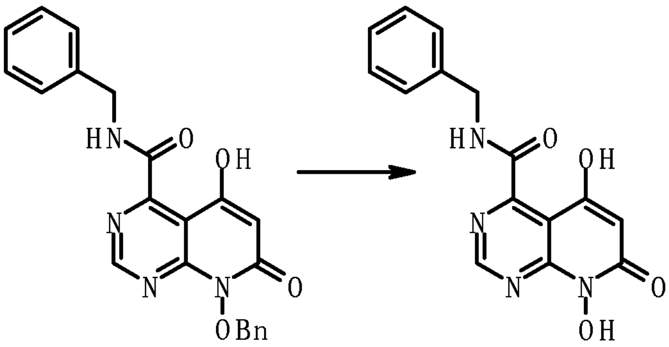

To N-benzyl-8-(benzyloxy)-5-hydroxy-7-oxo-7,8-dihydropyrido[2,3-d]pyrimidine-4-carboxamide (15 mg), concentrated hydrochloric acid (2 mL) and TFA (2 mL) were added, and the mixture was heated with stirring at 70 to 80°C for 4 hours. After cooling of the reaction mixture, the solvent was distilled off under reduced pressure. The obtained residue was suspended in a mixed solvent of 2-propanol and diisopropyl ether, and the deposit was collected by filtration to obtain a brown solid of N-benzyl-5,8-dihydroxy-7-oxo-7,8-dihydropyrido[2,3-d]pyrimidine-4-carboxamide (7 mg).
$^1$H-NMR (DMSO-$d_6$) δ value: 4.45-4.54 (m, 2H), 5.94 (s, 1H), 7.24-7.47 (m, 5H), 9.08 (s, 1H), 9.12-9.23 (m, 1H), 11.01 (brs, 1H).

Example 64

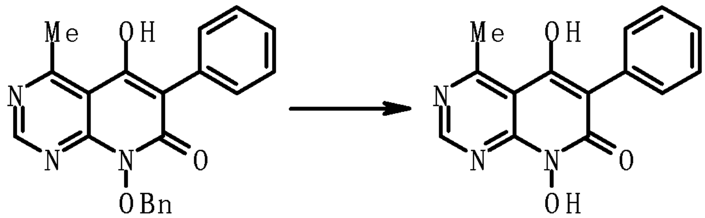

To 8-(benzyloxy)-5-hydroxy-4-methyl-6-phenylpyrido[2,3-d]pyrimidin-7(8H)-one (40 mg), 10% palladium-carbon (20 mg) and methanol (15 mL) were added, and the mixture was stirred at room temperature for 5 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a gray solid of 5,8-dihydroxy-4-methyl-6-phenylpyrido[2,3-d]pyrimidin-7(8H)-one (8 mg).
$^1$H-NMR (DMSO-$d_6$) δ value: 2.89 (s, 3H), 7.29-7.48 (m, 5H), 8.86 (s, 1H), 10.87 (brs, 1H).

Example 65

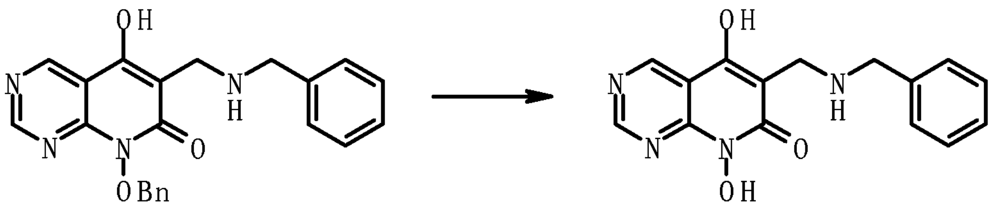

To 6-((benzylamino)methyl)-8-(benzyloxy)-5-hydroxypyrido[2,3-d]pyrimidin-7(8H)-one (14 mg), 10% palladium-carbon (7 mg), methanol (12 mL) and dioxane (6 mL) were added, and the mixture was stirred at room temperature for 10 minutes in a hydrogen atmosphere. Palladium-carbon was filtered off and washed with methanol. The filtrate and the washes were combined, and the solvent was distilled off under reduced pressure. To the obtained residue, 20% palladium hydroxide-carbon (7 mg), methanol (12 mL) and dioxane (6 mL) were added, and the mixture was stirred at

room temperature for 23 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol and diisopropyl ether, and the deposit was collected by filtration to obtain a gray solid of 6-((benzylamino)methyl)-5,8-dihydroxypyrido[2,3-d]pyrimidin-7(8H)-one (4 mg).
$^1$H-NMR (DMSO-$d_6$) δ value: 3.94 (s, 2H), 4.09 (s, 2H), 7.37-7.48 (m, 3H), 7.50-7.59 (m, 2H), 8.86 (s, 1H), 8.93 (s, 1H), 10.17 (brs, 1H).

Example 66

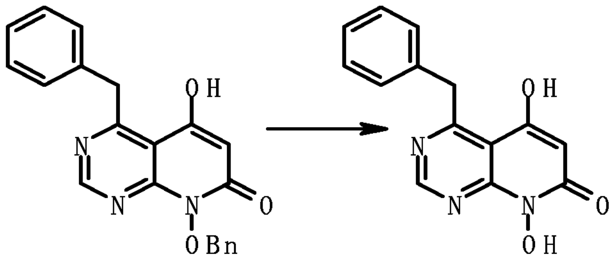

To 4-benzyl-8-(benzyloxy)-5-hydroxypyrido[2,3-d]pyrimidin-7(8H)-one (20 mg), concentrated hydrochloric acid (3 mL) and TFA (3 mL) were added, and the mixture was heated with stirring at 70 to 80°C for 6 hours and 30 minutes. After cooling of the reaction mixture, the solvent was distilled off under reduced pressure. The obtained residue was suspended in a mixed solvent of ethyl acetate, 2-propanol and diisopropyl ether to obtain a dark brown solid of 4-benzyl-5,8-dihydroxypyrido[2,3-d]pyrimidin-7(8H)-one (11 mg).
$^1$H-NMR (DMSO-$d_6$) δ value: 4.63 (s, 2H), 5.95 (s, 1H), 7.10-7.35 (m, 5H), 8.94 (s, 1H), 12.14 (brs, 1H).

Example 67

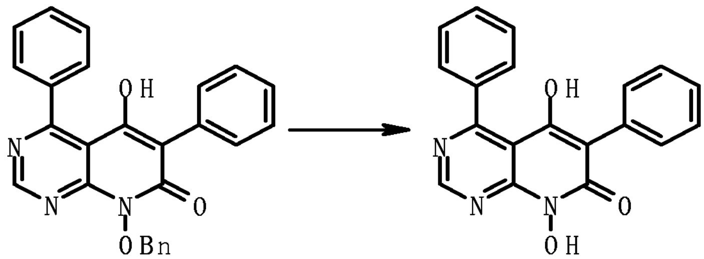

To 8-(benzyloxy)-5-hydroxy-4,6-diphenylpyrido[2,3-d]pyrimidin-7(8H)-one (47 mg), 20% palladium hydroxide-carbon (24 mg) and methanol (15 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol, diisopropyl ether and diethyl ether, and the deposit was collected by filtration to obtain a dark green solid of 5,8-dihydroxy-4,6-diphenylpyrido[2,3-d]pyrimidin-7(8H)-one (3 mg).
$^1$H-NMR ($CD_3OD$) δ value: 7.30-7.58 (m, 10H), 9.02 (s, 1H).

Example 68

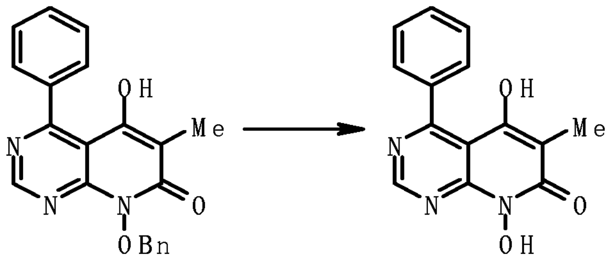

To 8-(benzyloxy)-5-hydroxy-6-methyl-4-phenylpyrido[2,3-d]pyrimidin-7(8H)-one (60 mg), 20% palladium hydroxide-carbon (30 mg) and methanol (20 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale green solid of 5,8-dihydroxy-6-methyl-4-phenylpyrido[2,3-d]pyrimidin-7(8H)-one (35 mg).
$^1$H-NMR (DMSO-$d_6$) δ value: 1.99 (s, 3H), 7.40-7.53 (m, 5H), 9.00 (s, 1H), 10.32 (brs, 1H), 10.99 (brs, 1H).

Example 69

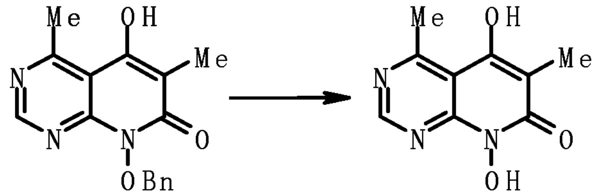

To 8-(benzyloxy)-5-hydroxy-4,6-dimethylpyrido[2,3-d]pyrimidin-7(8H)-one (12 mg), 20% palladium hydroxide-carbon (6 mg) and methanol (20 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a gray solid of 5,8-dihydroxy-4,6-dimethylpyrido[2,3-d]pyrimidin-7(8H)-one (3 mg).
$^1$H-NMR (DMSO-$d_6$) δ value: 2.05 (s, 3H), 2.91 (s, 3H), 8.81 (s, 1H), 10.82 (brs, 1H).

Example 70

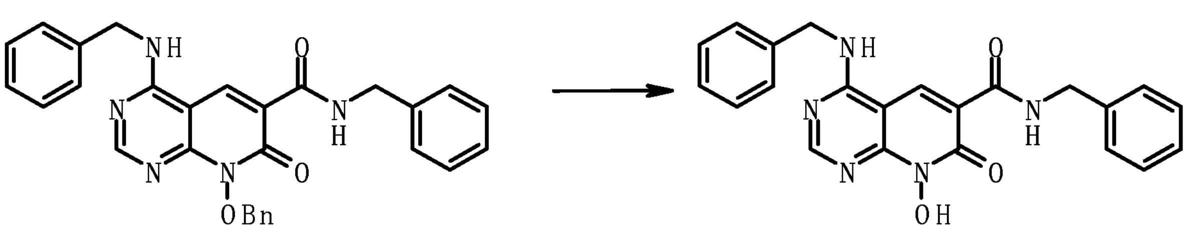

To N-benzyl-4-(benzylamino)-8-(benzyloxy)-7-oxo-7,8-dihydropyrido[2,3-d]pyrimidine-6-carboxamide (28 mg), 20% palladium hydroxide-carbon (14 mg), methanol (20 mL) and dioxane (10 mL) were added, and the mixture was stirred at room temperature for 5 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol and diisopropyl ether, and the deposit was collected by filtration to obtain a green solid of N-benzyl-4-(benzylamino)-8-hydroxy-7-oxo-7,8-dihydropyrido[2,3-d]pyrimidine-6-carboxamide (7 mg).
$^1$H-NMR (DMSO-$d_6$) δ value: 4.58 (d, J=5.8 Hz, 2H), 4.74 (d, J=5.6 Hz, 2H), 7.21-7.44 (m, 10H), 8.48 (s, 1H), 9.19 (s,

1H), 9.38-9.52 (m, 1H), 9.82-9.90 (m, 1H), 11.43 (brs, 1H).

Example 71

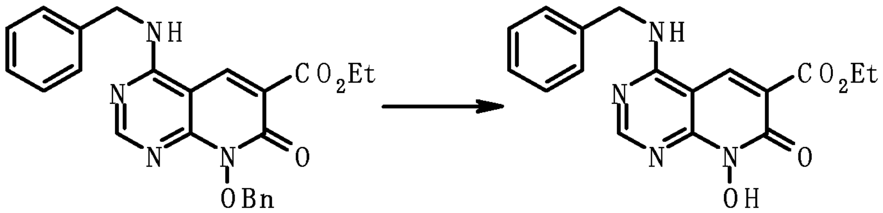

To ethyl 4-(benzylamino)-8-(benzyloxy)-7-oxo-7,8-dihydropyrido[2,3-d]pyrimidine-6-carboxylate (74 mg), 20% palladium hydroxide-carbon (37 mg), methanol (14 mL) and dioxane (7 mL) were added, and the mixture was stirred at room temperature for 5 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a pale yellow solid of ethyl 4-(benzylamino)-8-hydroxy-7-oxo-7,8-dihydropyrido[2,3-d]pyrimidine-6-carboxylate (24 mg).
$^1$H-NMR (DMSO-$d_6$) δ value: 1.31 (t, 6.6 Hz, 3H), 4.23-4.36 (m, 2H), 4.69-4.81 (m, 2H), 7.21-7.42 (m, 5H), 8.45 (s, 1H), 8.87 (s, 1H), 9.18 (brs, 1H), 11.15 (brs, 1H).

Example 72

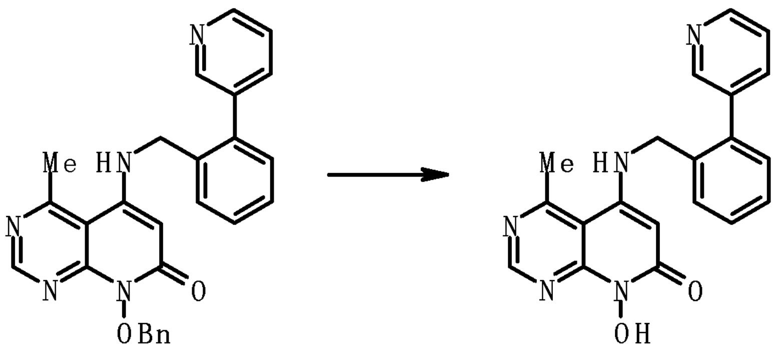

To 8-(benzyloxy)-4-methyl-5-((2-(pyridin-3-yl)benzyl)amino)pyrido[2,3-d]pyrimidin-7(8H)-one (15 mg), 20% palladium-carbon (8 mg), methanol (20 mL) and dioxane (10 mL) were added, and the mixture was stirred at room temperature for 25 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of methanol, 2-propanol and diisopropyl ether, and the deposit was collected by filtration to obtain a pale green solid of 8-hydroxy-4-methyl-5-((2-(pyridin-3-yl)benzyl)amino)pyrido[2,3-d]pyrimidin-7(8H)-one (8 mg). $^1$H-NMR (DMSO-$d_6$) δ value: 2.81 (s, 3H), 4.39 (d, J=5.1 Hz, 2H), 5.24 (s, 1H), 6.56-6.64 (m, 1H), 7.30-7.64 (m, 5H), 7.87-7.96 (m, 1H), 8.58-8.70 (m, 2H), 8.77 (s, 1H), 10.56 (brs, 1H).

Example 73

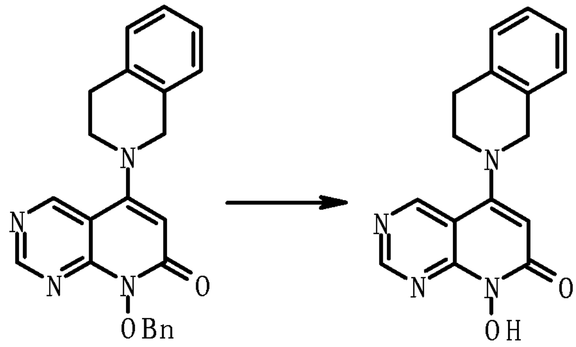

To 8-(benzyloxy)-5-(3,4-dihydroisoquinolin-2(1H)-yl)pyrido[2,3-d]pyrimidin-7(8H)-one (48 mg), 10% palladium hydroxide-carbon (24 mg) and methanol (20 mL) were added, and the mixture was stirred at room temperature for 1 hours and 40 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale green solid of 5-(3,4-dihydroisoquinolin-2(1H)-yl)-8-hydroxypyrido[2,3-d]pyrimidin-7(8H)-one (25 mg).
$^1$H-NMR (DMSO-$d_6$) δ value: 3.02-3.16 (m, 2H), 3.50-3.68 (m, 2H), 4.40 (s, 2H), 6.14 (s, 1H), 7.22 (s, 4H), 9.05 (s, 1H), 9.07 (s, 1H), 10.99 (brs, 1H).

Example 74

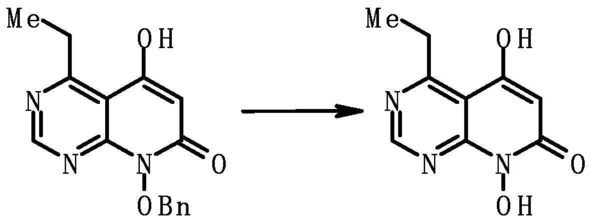

To 8-(benzyloxy)-4-ethyl-5-hydroxypyrido[2,3-d]pyrimidin-7(8H)-one (30 mg), concentrated hydrochloric acid (5 mL) and TFA (5 mL) were added, and the mixture was heated with stirring at 70 to 80°C for 6 hours. After cooling of the reaction mixture, the solvent was distilled off under reduced pressure. The obtained residue was suspended in a mixed solvent of 2-propanol and diisopropyl ether, and the deposit was collected by filtration to obtain a brown solid of 4-ethyl-5,8-dihydroxypyrido[2,3-d]pyrimidin-7(8H)-one (20 mg).
$^1$H-NMR (DMSO-$d_6$) δ value: 1.25 (t, J=7.4 Hz, 3H), 3.29 (q, J=7.4 Hz, 2H), 6.09 (s, 1H), 8.98 (s, 1H), 12.29 (brs, 1H).

Example 75

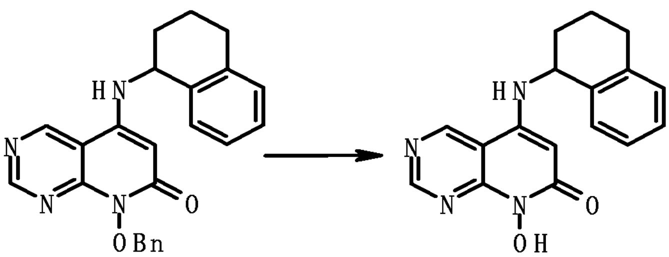

To 8-(benzyloxy)-5-((1,2,3,4-tetrahydronaphthalen-1-yl)amino)pyrido[2,3-d]pyrimidin-7(8H)-one (6 mg), 10% palladium-carbon (3 mg) and methanol (5 mL) were added, and the mixture was stirred at room temperature for 30 minutes in a hydrogen atmosphere. Palladium-carbon was filtered off and washed with methanol. The filtrate and the

washes were combined, and the solvent was distilled off under reduced pressure. The obtained residue was suspended in a mixed solvent of 2-propanol and diisopropyl ether, and the deposit was collected by filtration to obtain a gray solid of 8-hydroxy-5-((1,2,3,4-tetrahydronaphthalen-1-yl)amino)pyrido[2,3-d]pyrimidin-7(8H)-one (3 mg).
$^1H$-NMR (DMSO-$d_6$) δ value: 1.60-2.20 (m, 4H), 2.70-2.90 (m, 2H), 4.80-4.94 (m, 1H), 5.78 (s, 1H), 7.06-7.34 (m, 4H), 7.52-7.68 (m, 1H), 9.00 (s, 1H), 9.43 (s, 1H), 10.65 (brs, 1H).

Example 76

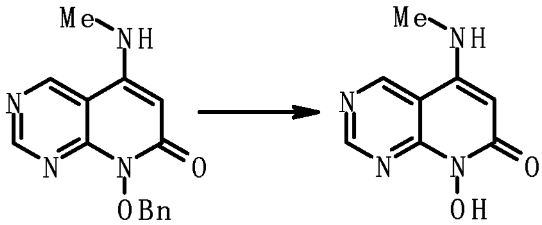

To 8-(benzyloxy)-5-(methylamino)pyrido[2,3-d]pyrimidin-7(8H)-one (8 mg), 10% palladium-carbon (4 mg) and methanol (6 mL) were added, and the mixture was stirred at room temperature for 2 hours and 35 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a light brown solid of 8-hydroxy-5-(methylamino)pyrido[2,3-d]pyrimidin-7(8H)-one (3 mg).
$^1H$-NMR (DMSO-$d_6$) δ value: 2.81 (d, J=4.4 Hz, 3H), 5.43 (s, 1H), 7.45-7.70 (m, 1H), 9.00 (s, 1H), 9.24 (s, 1H), 10.63 (brs, 1H).

Example 77

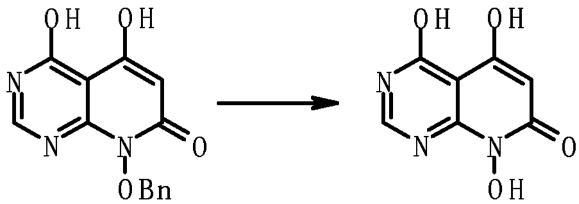

To 8-(benzyloxy)-4,5-dihydroxypyrido[2,3-d]pyrimidin-7(8H)-one (30 mg), concentrated hydrochloric acid (5 mL) and TFA (5 mL) were added, and the mixture was heated with stirring at 70 to 80°C for 4 hours and 30 minutes. After cooling of the reaction mixture, the solvent was distilled off under reduced pressure. The obtained residue was suspended in a mixed solvent of 2-propanol and diisopropyl ether, and the deposit was collected by filtration to obtain a light brown solid of 4,5,8-trihydroxypyrido[2,3-d]pyrimidin-7(8H)-one (18 mg).
$^1H$-NMR (DMSO-$d_6$) δ value: 5.75 (s, 1H), 8.40 (s, 1H), 11.09 (brs, 1H), 11.81 (s, 1H), 13.33 (brs, 1H).

Example 78

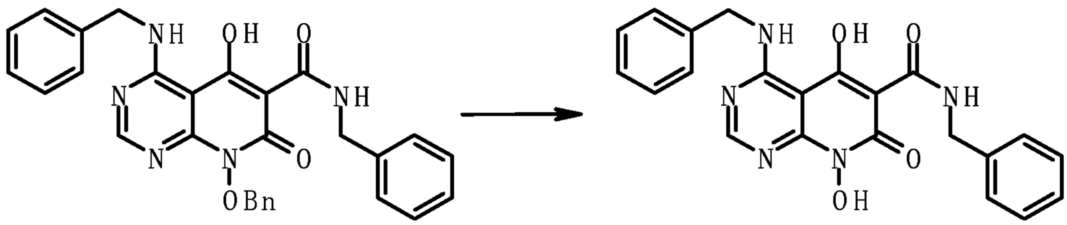

To N-benzyl-4-(benzylamino)-8-(benzyloxy)-5-hydroxy-7-oxo-7,8-dihydropyrido[2,3-d]pyrimidine-6-carboxamide (36 mg), 20% palladium hydroxide-carbon (18 mg), dioxane (6 mL) and methanol (6 mL) were added, and the mixture was stirred at room temperature for 30 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a gray solid of N-benzyl-4-(benzylamino)-5,8-dihydroxy-7-oxo-7,8-dihydropyrido[2,3-d]pyrimidine-6-carboxamide (21 mg).

$^1$H-NMR (DMSO-$d_6$) δ value: 4.62 (d, J=5.6 Hz, 2H), 4.79 (d, J=6.0 Hz, 2H), 7.22-7.42 (m, 10H), 8.45 (s, 1H), 9.07 (brs, 1H), 10.53 (brs, 1H), 10.93 (brs, 1H).

Example 79

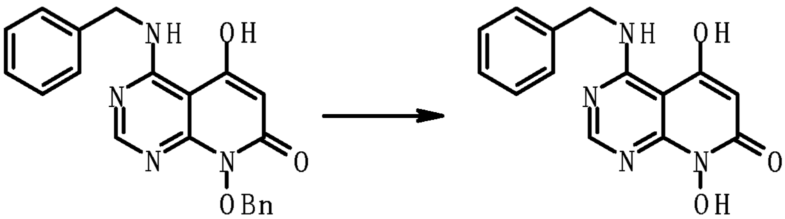

To 4-(benzylamino)-8-(benzyloxy)-5-hydroxypyrido[2,3-d]pyrimidine-7(8H)-one (26 mg), 20% palladium hydroxide-carbon (13 mg), dioxane (6 mL) and methanol (6 mL) were added, and the mixture was stirred at room temperature for 35 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a gray solid of 4-(benzylamino)-5,8-dihydroxypyrido[2,3-d]pyrimidine-7(8H)-one (16 mg).

$^1$H-NMR (DMSO-$d_6$) δ value: 4.75 (d, J=5.6 Hz, 2H), 5.74 (s, 1H), 7.20-7.37 (m, 5H), 8.33 (s, 1H), 8.49 (brs, 1H), 10.57 (brs, 1H), 12.42 (brs, 1H).

Example 80

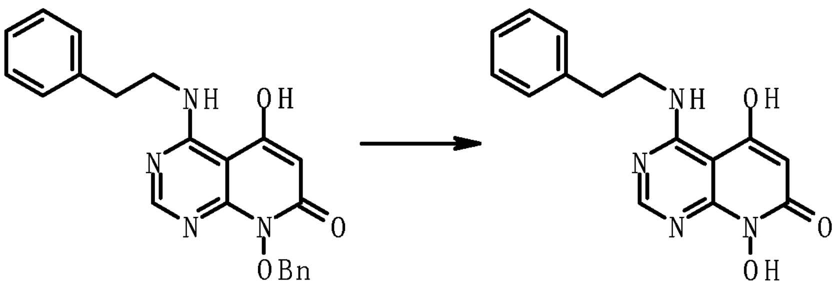

To 8-(benzyloxy)-5-hydroxy-4-((2-phenylethyl)amino)pyrido[2,3-d]pyrimidine-7(8H)-one (55 mg), 20% palladium hydroxide-carbon (28 mg) and methanol (15 mL) were added, and the mixture was stirred at room temperature for 30 minutes in a hydrogen atmosphere. Palladium hydroxide-carbon was filtered off and washed with methanol. The filtrate and the washes were combined, and the solvent was distilled off under reduced pressure. The obtained residue

was suspended in a mixed solvent of 2-propanol and diisopropyl ether, and the deposit was collected by filtration to obtain a gray solid of 5,8-dihydroxy-4-((2-phenylethyl)amino)pyrido[2,3-d]pyrimidine-7(8H)-one (27mg).
$^1$H-NMR (DMSO-$d_6$) δ value: 2.90 (t, J=7.2 Hz, 2H), 3.68-3.82 (m, 2H), 5.71 (s, 1H), 7.18-7.36 (m, 5H), 8.07 (brs, 1H), 8.38 (s, 1H), 10.60 (brs, 1H), 12.36 (brs, 1H).

Example 81

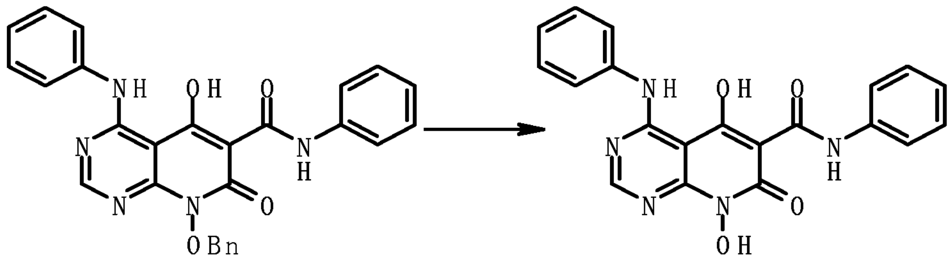

To 4-anilino-8-(benzyloxy)-5-hydroxy-7-oxo-N-phenyl-7,8-dihydropyrido[2,3-d]pyrimidine-6-carboxamide (37 mg), 20% palladium hydroxide-carbon (19 mg), methanol (6 mL) and THF (6 mL) were added, and the mixture was stirred at room temperature for 40 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale green solid of 4-anilino -5,8-dihydroxy-7-oxo-N-phenyl-7,8-dihydropyrido[2,3-d]pyrimidine-6-carboxamide (22 mg).
$^1$H-NMR (DMSO-$d_6$) δ value: 7.14-7.29 (m, 2H), 7.32-7.52 (m, 4H), 7.65 (d, J=8.0 Hz, 2H), 7.74 (d, J=8.0 Hz, 2H), 8.61 (s, 1H), 11.28 (brs, 1H), 12.37 (brs, 1H).

Example 82

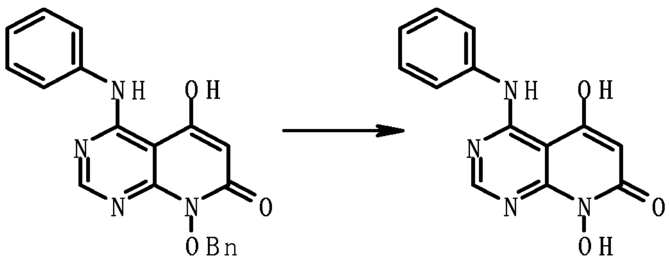

To 4-anilino-8-(benzyloxy)-5-hydroxypyrido[2,3-d]pyrimidine-7(8H)-one (24 mg), 20% palladium hydroxide-carbon (12 mg) and methanol (12 mL) were added, and the mixture was stirred at room temperature for 5 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 4-anilino-5,8-dihydroxypyrido[2,3-d]pyrimidine-7(8H)-one (13mg).
$^1$H-NMR (DMSO-$d_6$) δ value: 5.29 (s, 1H), 6.96-7.08 (m, 1H), 7.25-7.40 (m, 2H), 7.66-7.76 (m, 2H), 8.35 (s, 1H), 12.78 (brs, 1H).

Example 83

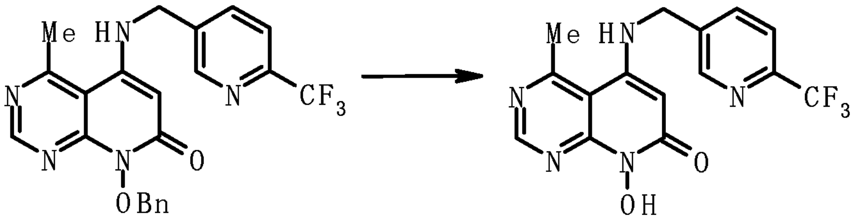

To 8-(benzyloxy)-4-methyl-5-(((6-(trifluoromethyl)pyridin-3-yl)methyl)amino)pyrido[2,3-d]pyrimidin-7(8H)-one (14 mg), 20% palladium hydroxide-carbon (7 mg) and methanol (10 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a gray solid of 8-hydroxy-4-methyl-5-(((6-(trifluoromethyl)pyridin-3-yl)methyl)amino)pyrido[2,3-d]pyrimidine-7(8H)-one (9mg).

$^1$H-NMR (DMSO-$d_6$) δ value: 3.01 (s, 3H), 4.58-4.74 (m, 2H), 5.44 (s, 1H), 6.84-7.02 (m, 1H), 7.84-7.94 (m, 1H), 8.06-8.16 (m, 1H), 8.74-8.92 (m, 2H), 10.62 (brs, 1H).

Example 84

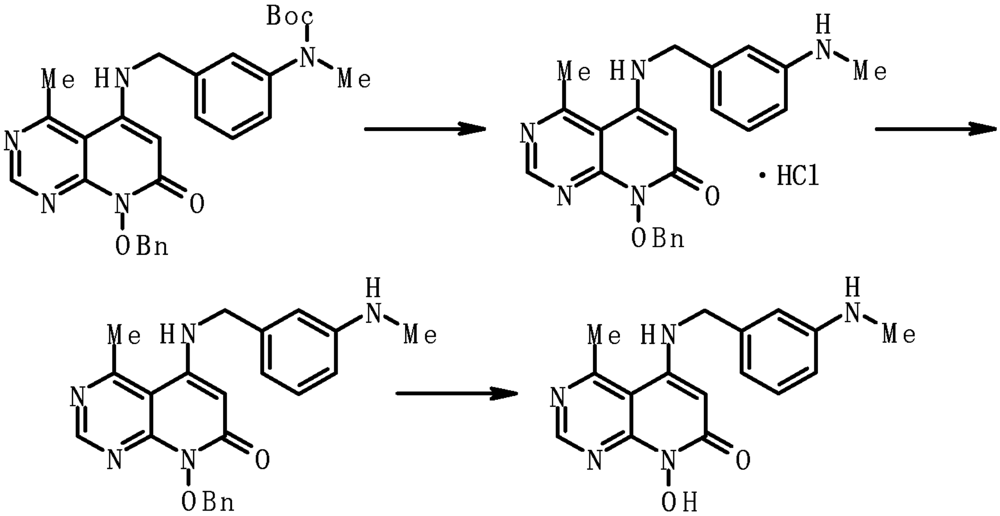

To tert-butyl (3-(((8-(benzyloxy)-4-methyl-7-oxo-7,8-dihydropyrido[2,3-d]pyrimidin-5-yl)amino)methyl)phenyl)(methyl)carbamate (64 mg), ethyl acetate (6 mL) and a 2.9 mol/L solution of hydrogen chloride in ethyl acetate (0.9 mL) were added, and the mixture was stirred at room temperature for 2 hours. The solvent in the reaction mixture was distilled off under reduced pressure. To the obtained residue, ethyl acetate and a saturated aqueous solution of sodium bicarbonate were added. An organic layer was separated, washed with a saturated aqueous solution of sodium chloride and then dried over sodium sulfate, and the solvent was distilled off under reduced pressure. To the obtained residue, ethyl acetate (2 mL), methanol (2 mL) and a 2.9 mol/L solution of hydrogen chloride in ethyl acetate (4 mL) were added, and the mixture was stirred at room temperature for 1 hour. The solvent in the reaction mixture was distilled off under reduced pressure. The obtained residue was suspended in a mixed solvent of ethyl acetate and methanol, and the deposit was collected by filtration to obtain a yellow solid (53 mg). To the obtained yellow solid (49 mg), ethyl acetate and a saturated aqueous solution of sodium bicarbonate were added. An organic layer was separated, washed with a saturated aqueous solution of sodium chloride and then dried over sodium sulfate, and the solvent was distilled off under reduced pressure. To the obtained residue, 20% palladium hydroxide-carbon (25 mg) and methanol (15 mL) were added, and the mixture was stirred at room temperature for 15 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-4-methyl-5-((3-(methylamino)benzyl)amino)pyrido[2,3-d]pyrimidin-7(8H)-one (26 mg).
$^1$H-NMR (DMSO-$d_6$) δ value: 2.65 (d, J=4.9 Hz, 3H), 3.00 (s, 3H), 4.33 (d, J=5.4 Hz, 2H), 5.40 (s, 1H), 5.60-5.72 (m, 1H), 6.36-6.48 (m, 1H), 6.52-6.62 (m, 2H), 6.73-6.86 (m, 1H), 7.00-7.12 (m, 1H), 8.79 (s, 1H).

Example 85

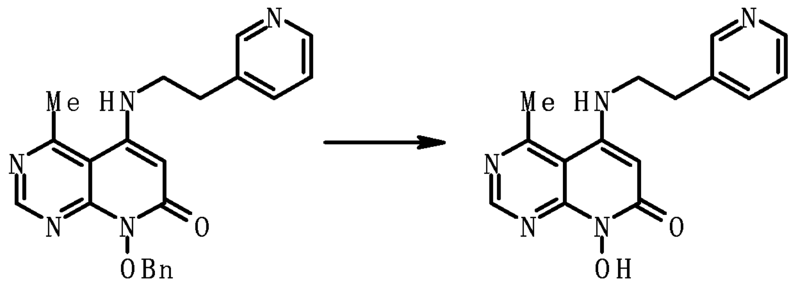

To 8-(benzyloxy)-4-methyl-5-((2-(pyridin-3-yl)ethyl)amino)pyrido[2,3-d]pyrimidin-7(8H)-one (65 mg), 20% palladium hydroxide-carbon (33 mg) and methanol (10 mL) were added, and the mixture was stirred at room temperature for 20 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a gray solid of 8-hydroxy-4-methyl-5-((2-(pyridin-3-yl)ethyl)amino)pyrido[2,3-d]pyrimidin-7(8H)-one (31 mg).
$^1$H-NMR (DMSO-$d_6$) δ value: 2.71 (s, 3H), 3.01 (t, J=7.0 Hz, 2H), 3.35-3.55 (m, 2H), 5.65 (s, 1H), 6.10-6.24 (m, 1H), 7.36 (dd, J=7.8, 4.9 Hz, 1H), 7.76 (d, J=7.8 Hz, 1H), 8.40-8.48 (m, 1H), 8.48-8.60 (m, 1H), 8.77 (s, 1H), 10.60 (s, 1H).

Example 86

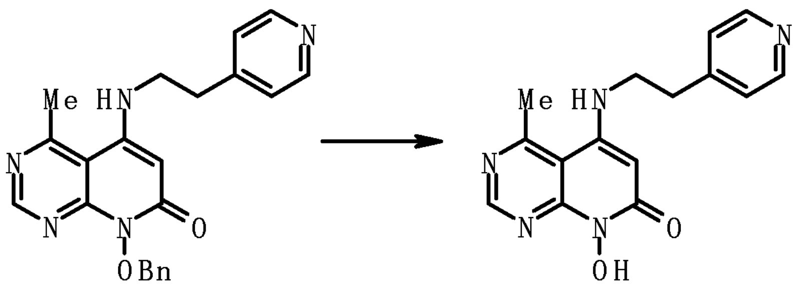

To 8-(benzyloxy)-4-methyl-5-((2-(pyridin-4-yl)ethyl)amino)pyrido[2,3-d]pyrimidin-7(8H)-one (17 mg), 20% palladium hydroxide-carbon (9 mg) and methanol (8 mL) were added, and the mixture was stirred at room temperature for 15 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a gray solid of 8-hydroxy-4-methyl-5-((2-(pyridin-4-yl)ethyl)amino)pyrido[2,3-d]pyrimidin-7(8H)-one (12 mg).
$^1$H-NMR (DMSO-$d_6$) δ value: 2.71 (s, 3H), 3.01 (t, J=7.0 Hz, 2H), 3.42-3.54 (m, 2H), 5.66 (s, 1H), 6.09-6.22 (m, 1H), 7.32-7.39 (m, 2H), 8.45-8.53 (m, 2H), 8.77 (s, 1H), 10.60 (brs, 1H).

Example 87

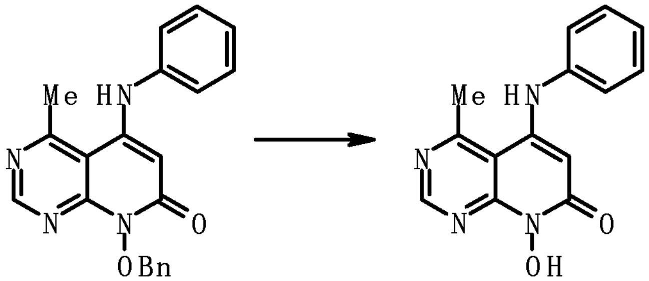

To 5-anilino-8-(benzyloxy)-4-methylpyrido[2,3-d]pyrimidin-7(8H)-one (4 mg), 20% palladium hydroxide-carbon (2 mg) and methanol (6 mL) were added, and the mixture was stirred at room temperature for 15 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale green solid of 5-anilino-8-hydroxy-4-methylpyrido[2,3-d]pyrimidin-7(8H)-one (2 mg).
$^1$H-NMR (DMSO-$d_6$) δ value: 2.94 (s, 3H), 5.99 (s, 1H), 7.11-7.19 (m, 1H), 7.22-7.31 (m, 2H), 7.36-7.47 (m, 2H), 8.15 (s, 1H), 8.86 (s, 1H), 10.82 (brs, 1H).

Example 88

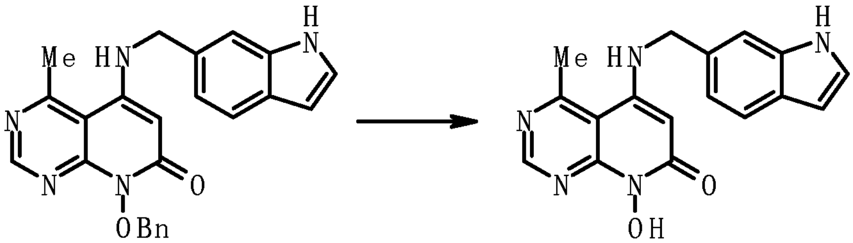

To 8-(benzyloxy)-5-(((1H-indol-6-yl)methyl)amino)-4-methylpyrido[2,3-d]pyrimidin-7(8H)-one (102 mg), 20% palladium hydroxide-carbon (51 mg), methanol (15 mL) and THF (15 mL) were added, and the mixture was stirred at room temperature for 30 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-5-(((1H-indol-6-yl)methyl)amino)-4-methylpyrido[2,3-d]pyrimidin-7(8H)-one (66 mg). $^1$H-NMR (DMSO-$d_6$) δ value: 3.01 (s, 3H), 4.53 (d, J=5.1 Hz, 2H), 5.49 (s, 1H), 6.39 (s, 1H), 6.81-6.89 (m, 1H), 7.07 (d, J=8.0 Hz, 1H), 7.27-7.34 (m, 1H), 7.42 (s, 1H), 7.52 (d, J=8.0 Hz, 1H), 8.79 (s, 1H), 10.56 (brs, 1H), 11.02 (brs, 1H).

Example 89

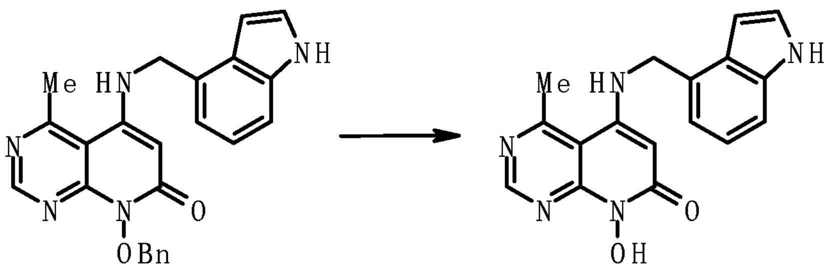

To 8-(benzyloxy)-5-(((1H-indol-4-yl)methyl)amino)-4-methylpyrido[2,3-d]pyrimidin-7(8H)-one (116 mg), 20% palladium hydroxide-carbon (58 mg), methanol (15 mL) and THF (15 mL) were added, and the mixture was stirred at

room temperature for 25 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a yellow solid of 8-hydroxy-5-(((1H-indol-4-yl)methyl)amino)-4-methylpyrido[2,3-d]pyrimidin-7(8H)-one (65 mg).
$^1$H-NMR (DMSO-$d_6$) δ value: 2.99 (s, 3H), 4.60-4.80 (m, 2H), 5.46 (s, 1H), 6.60-6.75 (m, 1H), 6.80-6.86 (m, 1H), 6.90-7.15 (m, 2H), 7.25-7.55 (m, 2H), 8.79 (s, 1H), 10.56 (brs, 1H), 11.23 (brs, 1H).

Example 90

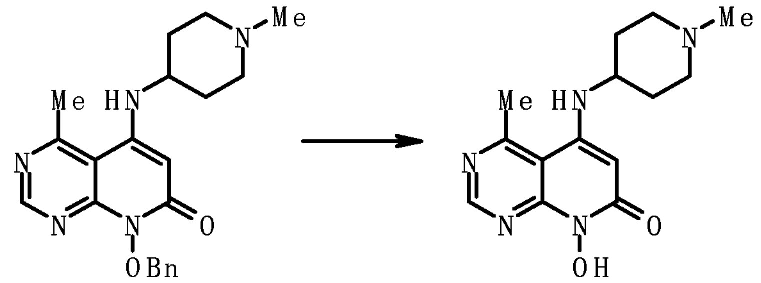

To 8-(benzyloxy)-4-methyl-5-((1-methylpiperidin-4-yl)amino)pyrido[2,3-d]pyrimidin-7(8H)-one (42 mg), 20% palladium hydroxide-carbon (21 mg) and methanol (10 mL) were added, and the mixture was stirred at room temperature for 20 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a yellow solid of 8-hydroxy-4-methyl-5-((1-methylpiperidin-4-yl)amino)pyrido[2,3-d]pyrimidin-7(8H)-one (28 mg).
$^1$H-NMR (DMSO-$d_6$) δ value: 1.50-1.70 (m, 2H), 1.82-2.04 (m, 2H), 2.06-2.20 (m, 2H), 2.18 (s, 3H), 2.60-2.80 (m, 2H), 2.90 (s, 3H), 3.15-3.55 (m, 1H), 5.60 (s, 1H), 5.86 (d, J=6.8 Hz, 1H), 8.77 (s, 1H), 10.63 (brs, 1H).

Example 91

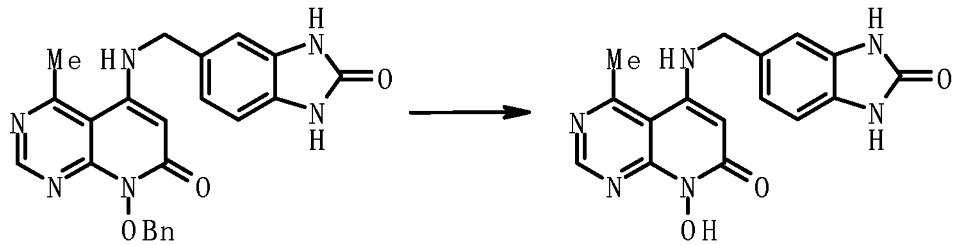

To 8-(benzyloxy)-4-methyl-5-(((2-oxo-2,3-dihydro-1H-benzimidazol-5-yl)methyl)amino)pyrido[2,3-d]pyrimidin-7(8H)-one (37 mg), 20% palladium hydroxide-carbon (19 mg), methanol (15 mL) and THF (15 mL) were added, and the mixture was stirred at room temperature for 20 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-4-methyl-5-(((2-oxo-2,3-dihydro-1H-benzimidazol-5-yl)methyl)amino)pyrido[2,3-d]pyrimidin-7(8H)-one (21 mg).
$^1$H-NMR (DMSO-$d_6$) δ value: 2.98 (s, 3H), 4.42 (d, J=5.4 Hz, 2H), 5.45 (s, 1H), 6.77-6.84 (m, 1H), 6.86-6.93 (m, 1H), 6.96-7.04 (m, 2H), 8.79 (s, 1H), 10.53-10.65 (m, 3H).

Example 92

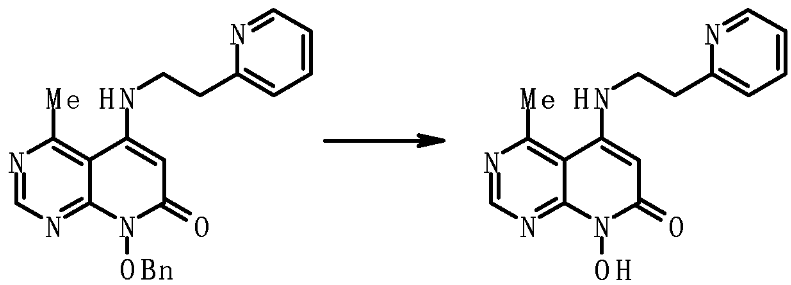

To 8-(benzyloxy)-4-methyl-5-((2-(pyridin-2-yl)ethyl)amino)pyrido[2,3-d]pyrimidin-7(8H)-one (60 mg), 20% palladium hydroxide-carbon (30 mg) and methanol (10 mL) were added, and the mixture was stirred at room temperature for 30 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a gray solid of 8-hydroxy-4-methyl-5-((2-(pyridin-2-yl)ethyl)amino)pyrido[2,3-d]pyrimidin-7(8H)-one (25 mg).
$^1$H-NMR (DMSO-$d_6$) δ value: 2.81 (s, 3H), 3.09-3.20 (m, 2H), 3.45-3.59 (m, 2H), 5.61 (s, 1H), 6.50-6.60 (m, 1H), 7.20-7.33 (m, 1H), 7.38 (d, J=7.8 Hz, 1H), 7.75 (ddd, J=7.6, 7.6, 1.9 Hz, 1H), 8.52-8.58 (m, 1H), 8.78 (s, 1H), 10.58 (brs, 1H).

Example 93

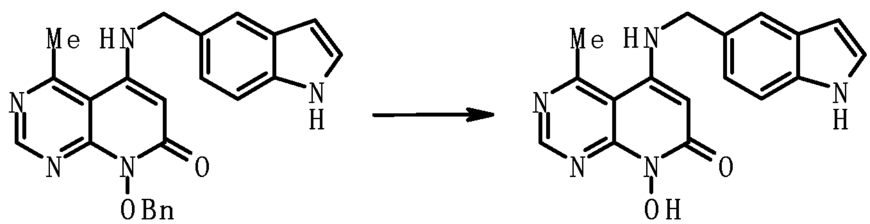

To 8-(benzyloxy)-5-(((1H-indol-5-yl)methyl)amino)-4-methylpyrido[2,3-d]pyrimidin-7(8H)-one (54 mg), 20% palladium hydroxide-carbon (27 mg), methanol (7 mL) and THF (7 mL) were added, and the mixture was stirred at room temperature for 20 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-5-(((1H-indol-5-yl)methyl)amino)-4-methylpyrido[2,3-d]pyrimidin-7(8H)-one (11 mg).
$^1$H-NMR (DMSO-$d_6$) δ value: 3.00 (s, 3H), 4.50 (d, J=5.1 Hz, 2H), 5.49 (s, 1H), 6.33-6.43 (m, 1H), 6.76-6.86 (m, 1H), 7.16 (dd, J=8.4, 1.3 Hz, 1H), 7.30-7.42 (m, 2H), 7.58 (s, 1H), 8.79 (s, 1H), 10.55 (brs, 1H), 11.08 (brs, 1H).

Example 94

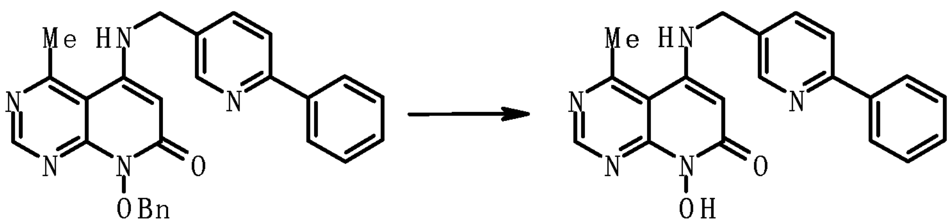

To 8-(benzyloxy)-4-methyl-5-(((6-phenylpyridin-3-yl)methyl)amino)pyrido[2,3-d]pyrimidin-7(8H)-one (80 mg), 20% palladium hydroxide-carbon (40 mg), methanol (10 mL) and THF (10 mL) were added, and the mixture was stirred at room temperature for 2 hours and 5 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit

was collected by filtration to obtain a pale yellow solid of 8-hydroxy-4-methyl-5-(((6-phenylpyridin-3-yl)methyl)amino)pyrido[2,3-d]pyrimidin-7(8H)-one (51 mg).
$^1$H-NMR (DMSO-$d_6$) δ value: 3.02 (s, 3H), 4.56 (d, J=5.4 Hz, 2H), 5.50 (s, 1H), 6.86-6.95 (m, 1H), 7.38-7.55 (m, 3H), 7.89-7.99 (m, 2H), 8.05-8.12 (m, 2H), 8.72-8.77 (m, 1H), 8.81 (s, 1H), 10.61 (brs, 1H).

Example 95

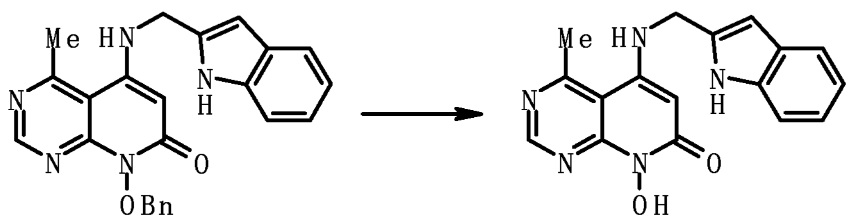

To 8-(benzyloxy)-5-(((1H-indol-2-yl)methyl)amino)-4-methylpyrido[2,3-d]pyrimidin-7(8H)-one (66 mg), 20% palladium hydroxide-carbon (33 mg), methanol (5 mL) and dioxane (5 mL) were added, and the mixture was stirred at room temperature for 30 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a light brown solid of 8-hydroxy-5-(((1H-indol-2-yl)methyl)amino)-4-methylpyrido[2,3-d]pyrimidin-7(8H)-one (14mg).
$^1$H-NMR (DMSO-$d_6$) δ value: 3.04 (s, 3H), 4.60 (d, J=5.1 Hz, 2H), 5.62 (s, 1H), 6.43 (s, 1H), 6.68-6.82 (m, 1H), 6.90-7.10 (m, 2H), 7.28-7.38 (m, 1H), 7.42-7.52 (m, 1H), 8.81 (s, 1H), 11.04 (brs, 1H).

Example 96

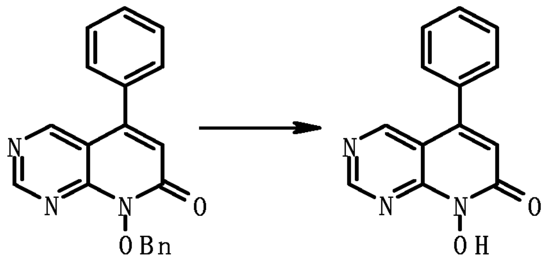

To 8-(benzyloxy)-5-phenylpyrido[2,3-d]pyrimidin-7(8H)-one (24 mg), 20% palladium hydroxide-carbon (12 mg), methanol (4.5 mL) and dioxane (3.0 mL) were added, and the mixture was stirred at room temperature for 5 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a yellow solid of 8-hydroxy-5-phenylpyrido[2,3-d]pyrimidin-7(8H)-one (15 mg).
$^1$H-NMR (DMSO-$d_6$) δ value: 6.79 (s, 1H), 7.55-7.63 (m, 5H), 8.75 (s, 1H), 9.14 (s, 1H), 11.47 (brs, 1H).

Example 97

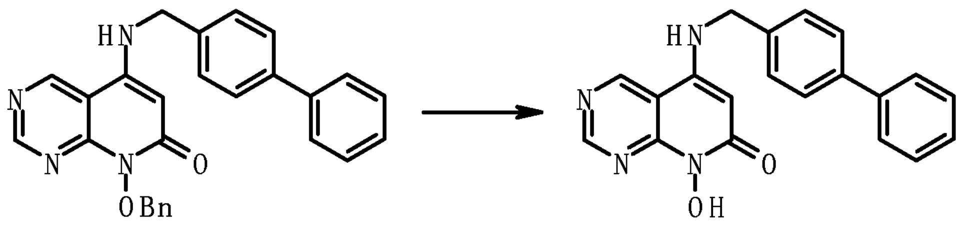

To 8-(benzyloxy)-5-(((biphenyl-4-yl)methyl)amino)pyrido[2,3-d]pyrimidin-7(8H)-one (16 mg), 20% palladium hydroxide-carbon (8 mg), methanol (6.0 mL) and dioxane (4.0 mL) were added, and the mixture was stirred at room temperature for 5 minutes in a hydrogen atmosphere. To the reaction mixture, 20% palladium hydroxide-carbon (8 mg) was added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a pale yellow solid of 5-(((biphenyl-4-yl)methyl)amino)-8-hydroxypyrido[2,3-d]pyrimidin-7(8H)-one (8 mg).
$^1$H-NMR (DMSO-$d_6$) δ value: 4.49-4.55 (m, 2H), 5.43 (s, 1H), 7.32-7.39 (m, 1H), 7.42-7.52 (m, 4H), 7.62-7.69 (m, 4H), 8.11-8.18 (m, 1H), 9.02 (s, 1H), 9.41 (s, 1H).

Example 98

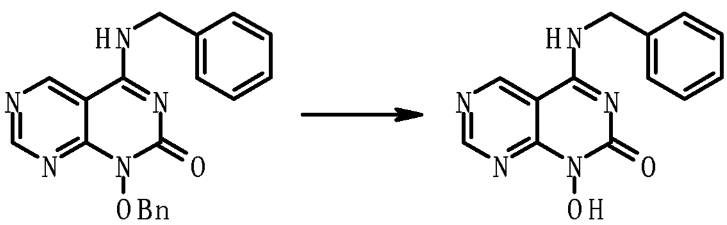

To 4-(benzylamino)-1-(benzyloxy)pyrimido[4,5-d]pyrimidin-2(1H)-one (38 mg), 20% palladium hydroxide-carbon (19 mg), methanol (11 mL) and dioxane (3.7 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a yellow solid of 4-(benzylamino)-1-hydroxypyrimido[4,5-d]pyrimidin-2(1H)-one (27 mg).
$^1$H-NMR (DMSO-$d_6$) δ value: 4.71 (d, J=5.8 Hz, 2H), 7.16-7.44 (m, 5H), 9.06 (s, 1H), 9.29-9.40 (m, 2H), 10.70 (brs, 1H).

Example 99

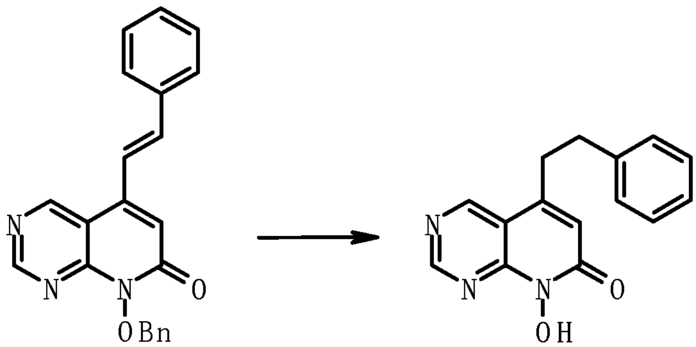

To 8-(benzyloxy)-5-((E)-2-phenylvinyl)pyrido[2,3-d]pyrimidin-7(8H)-one (15 mg), 20% palladium hydroxide-carbon (8 mg), methanol (4.5 mL) and dioxane (3.0 mL) were added, and the mixture was stirred at room temperature for

10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a pale yellow solid of 8-hydroxy-5-(2-phenylethyl)pyrido[2,3-d]pyrimidin-7(8H)-one (9 mg).
$^1$H-NMR (DMSO-$d_6$) δ value: 2.93-3.01 (m, 2H), 3.18-3.25 (m, 2H), 6.64 (s, 1H), 7.15-7.33 (m, 5H), 9.08 (s, 1H), 9.24 (s, 1H).

Example 100

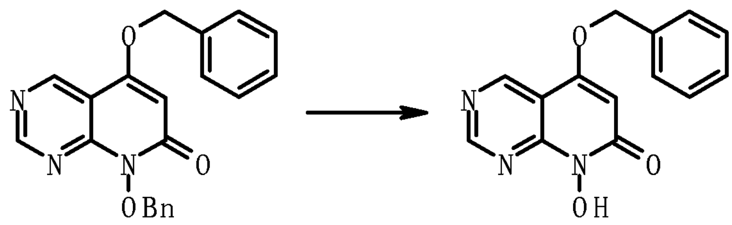

To 5,8-bis(benzyloxy)pyrido[2,3-d]pyrimidin-7(8H)-one (95 mg), a palladium-carbon-ethylenediamine complex (Pd: 3.5-6.5%) (48 mg), methanol (19 mL) and dioxane (19 mL) were added, and the mixture was stirred at room temperature for 1 hour and 50 minutes in a hydrogen atmosphere. The palladium-carbon-ethylenediamine complex was filtered off and washed with a mixed solvent of methanol and dioxane. The filtrate and the washes were combined, and the solvent was distilled off under reduced pressure. To the obtained residue, ethyl acetate and a saturated aqueous solution of sodium bicarbonate were added, and insoluble matter was filtered off, followed by separation of an aqueous layer. The obtained aqueous layer was pH-adjusted to 4.5 by the addition of 3 mol/L hydrochloric acid and stirred at room temperature for 30 minutes. The deposit was collected by filtration to obtain a pale red solid (19 mg). The obtained pale red solid (19 mg) was purified with a PLC glass plate [Merck Japan Ltd., PLC glass plate silica gel 60 $F_{254}$, eluent: chloroform:methanol = 5:1]. Then, a saturated aqueous solution of sodium bicarbonate was added thereto, and insoluble matter was filtered off. The filtrate was pH-adjusted to 4.5 by the addition of 3 mol/L hydrochloric acid, and the deposit was then collected by filtration to obtain a pale yellow solid of 5-(benzyloxy)-8-hydroxypyrido[2,3-d]pyrimidin-7(8H)-one (2 mg).
$^1$H-NMR (DMSO-$d_6$) δ value: 5.34 (s, 2H), 6.35 (s, 1H), 7.35-7.48 (m, 3H), 7.52-7.58 (m, 2H), 9.12 (s, 1H), 9.12 (s, 1H).

Example 101

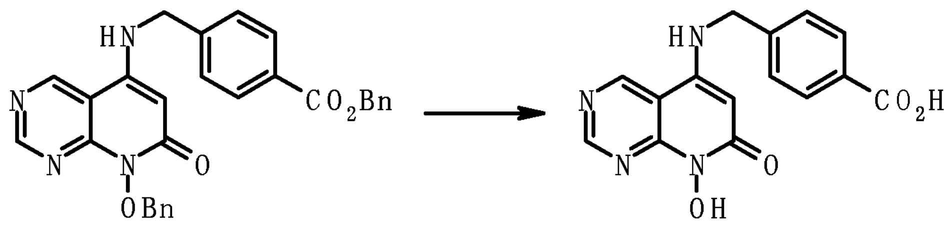

To benzyl 4-(((8-(benzyloxy)-7-oxo-7,8-dihydropyrido[2,3-d]pyrimidin-5-yl)amino)methyl)benzoate (11 mg), 20% palladium hydroxide-carbon (6 mg), methanol (5.5 mL) and dioxane (3.3 mL) were added, and the mixture was stirred at room temperature for 15 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a yellow solid of 4-(((8-hydroxy-7-oxo-7,8-dihydropyrido[2,3-d]pyrimidin-5-yl)amino)methyl)benzoic acid (5 mg). $^1$H-NMR (DMSO-$d_6$) δ value: 4.52-4.59 (m, 2H), 5.36 (s, 1H), 7.44-7.51 (m, 2H), 7.88-7.95 (m, 2H), 8.12-8.18 (m, 1H), 9.03 (s, 1H), 9.39 (s, 1H).

Example 102

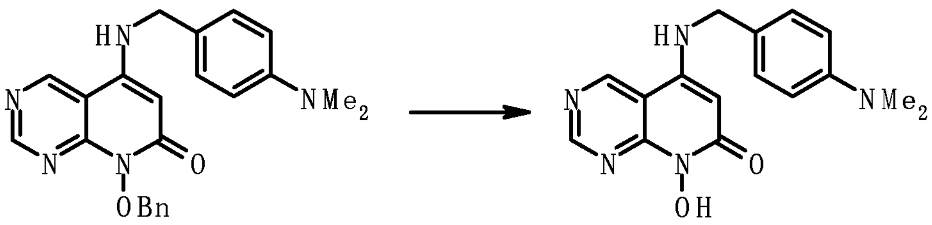

To 8-(benzyloxy)-5-((4-(dimethylamino)benzyl)amino)pyrido[2,3-d]pyrimidin-7(8H)-one (25 mg), 20% palladium hydroxide-carbon (13 mg), methanol (5.0 mL) and dioxane (2.5 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a yellow solid of 5-((4-(dimethylamino)benzyl)amino)-8-hydroxypyrido[2,3-d]pyrimidin-7(8H)-one (14mg). $^1$H-NMR (DMSO-$d_6$) δ value: 2.86 (s, 6H), 4.32 (d, J=5.6 Hz, 2H), 5.40 (s, 1H), 6.68-6.74 (m, 2H), 7.17-7.24 (m, 2H), 7.97 (t, J=5.7 Hz, 1H), 9.00 (s, 1H), 9.37 (s, 1H), 10.60 (brs, 1H).

Example 103

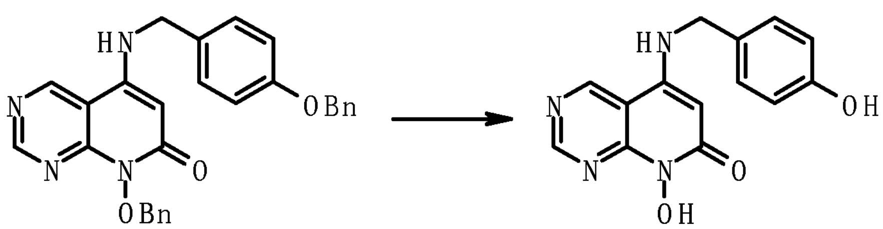

To 8-(benzyloxy)-5-((4-(benzyloxy)benzyl)amino)pyrido[2,3-d]pyrimidin-7(8H)-one (29 mg), 20% palladium hydroxide-carbon (29 mg), methanol (8.7 mL) and dioxane (4.4 mL) were added, and the mixture was stirred at room temperature for 20 minutes in a hydrogen atmosphere. Palladium hydroxide-carbon was filtered off and washed with methanol. The filtrate and the washes were combined, and the solvent was distilled off under reduced pressure. The obtained residue was suspended in diisopropyl ether, and the deposit was collected by filtration to obtain a yellow solid of 8-hydroxy-5-((4-hydroxybenzyl)amino)pyrido[2,3-d]pyrimidin-7(8H)-one (13 mg).
$^1$H-NMR (DMSO-$d_6$) δ value: 4.32 (d, J=5.6 Hz, 2H), 5.40 (s, 1H), 6.73 (d, J=8.3 Hz, 2H), 7.18 (d, J=8.3 Hz, 2H), 7.94-8.01 (m, 1H), 9.01 (s, 1H), 9.33 (brs, 1H), 9.37 (s, 1H), 10.61 (brs, 1H).

Example 104

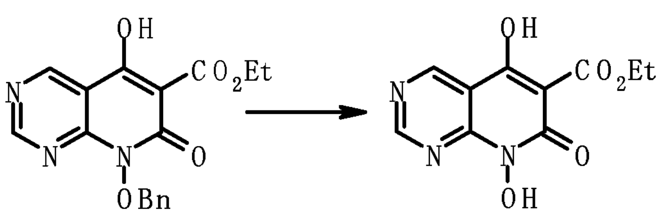

To ethyl 8-(benzyloxy)-5-hydroxy-7-oxo-7,8-dihydropyrido[2,3-d]pyrimidine-6-carboxylate (30 mg), 20% palladium hydroxide-carbon (15 mg), methanol (6.0 mL) and dioxane (6.0 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a greenish yellow solid of ethyl 5,8-dihydroxy-7-oxo-7,8-dihydropyrido[2,3-d]pyrimidine-6-carboxylate (18 mg).
$^1$H-NMR (DMSO-$d_6$) δ value: 1.28 (t, J=7.1 Hz, 3H), 4.28 (q, J=7.2 Hz, 2H), 9.10 (s, 1H), 9.19 (s, 1H), 10.96 (brs, 1H).

Example 105

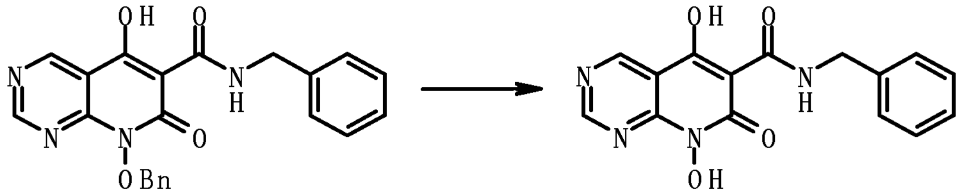

To N-benzyl-8-(benzyloxy)-5-hydroxy-7-oxo-7,8-dihydropyrido[2,3-d]pyrimidine-6-carboxamide (56 mg), 20% palladium hydroxide-carbon (28 mg), methanol (10 mL) and dioxane (14 mL) were added, and the mixture was stirred at room temperature for 8 minutes in a hydrogen atmosphere. Palladium hydroxide-carbon was filtered off and washed with a mixed solvent of methanol and dioxane. The filtrate and the washes were combined, and the solvent was distilled off under reduced pressure. To the obtained residue, ethyl acetate was added, and insoluble matter was filtered off. The solvent was distilled off under reduced pressure. The obtained residue was suspended in diisopropyl ether, and the deposit was collected by filtration to obtain a pale yellow solid of N-benzyl-5,8-dihydroxy-7-oxo-7,8-dihydropyrido[2,3-d]pyrimidine-6-carboxamide (30 mg).
$^1$H-NMR (DMSO-$d_6$) δ value: 4.63 (d, J=6.1 Hz, 2H), 7.25-7.43 (m, 5H), 9.24 (s, 1H), 9.31 (s, 1H), 10.29 (brs, 1H), 11.30 (brs, 1H).

Example 106

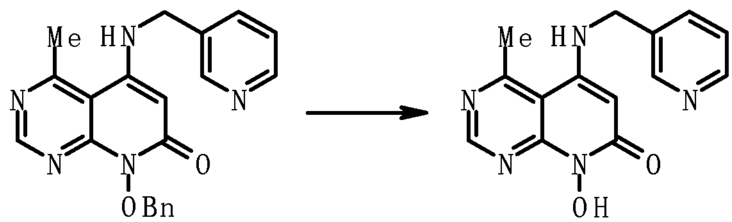

To 8-(benzyloxy)-4-methyl-5-(((pyridin-3-yl)methyl)amino)pyrido[2,3-d]pyrimidin-7(8H)-one (17 mg), 10% palladium-carbon (9 mg) and methanol (8.5 mL) were added, and the mixture was stirred at room temperature for 15 minutes in a hydrogen atmosphere. Palladium-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-4-methyl-5-(((pyridin-3-yl)methyl)amino)pyrido[2,3-d]pyrimidin-7(8H)-one (12 mg).
$^1$H-NMR (DMSO-$d_6$) δ value: 2.99 (s, 3H), 4.52 (d, J=5.4 Hz, 2H), 5.44 (s, 1H), 6.80-6.90 (m, 1H), 7.38 (dd, J=8.1, 5.1 Hz, 1H), 7.80-7.87 (m, 1H), 8.48 (dd, J=4.8, 1.6 Hz, 1H), 8.65 (d, J=1.4 Hz, 1H), 8.80 (s, 1H), 10.59 (brs, 1H).

Example 107

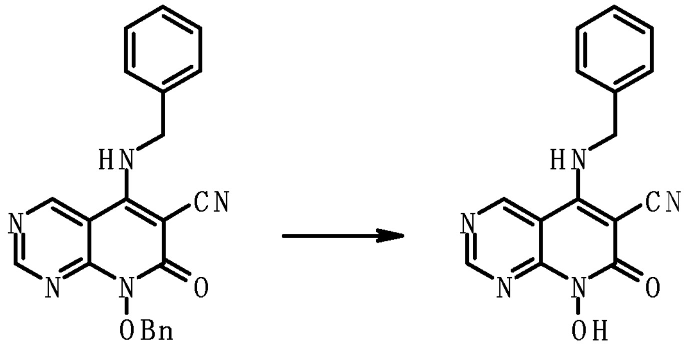

To 5-(benzylamino)-8-(benzyloxy)-7-oxo-7,8-dihydropyrido[2,3-d]pyrimidine-6-carbonitrile (10 mg), 10% palladium-carbon (5 mg), methanol (8.0 mL) and dioxane (2.0 mL) were added, and the mixture was stirred at room temperature for 10 minutes in a hydrogen atmosphere. Palladium-carbon was filtered off and washed with methanol. The filtrate and the washes were combined, and the solvent was distilled off under reduced pressure. The obtained residue was suspended in diisopropyl ether, and the deposit was collected by filtration to obtain a yellow solid of 5-(benzylamino)-8-hydroxy-7-oxo-7,8-dihydropyrido[2,3-d]pyrimidine-6-carbonitrile (5 mg).
$^1$H-NMR (DMSO-$d_6$) $\delta$ value: 5.04 (s, 2H), 7.22-7.50 (m, 5H), 9.10 (s, 1H), 9.52 (s, 1H).

Example 108

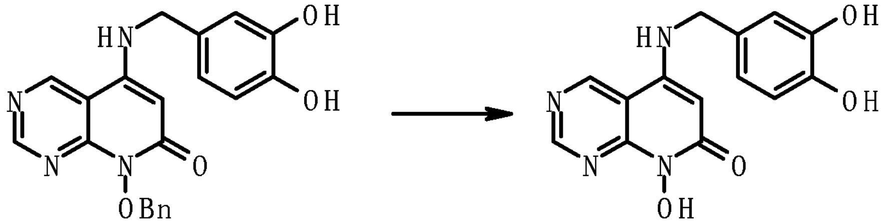

To 8-(benzyloxy)-5-((3,4-dihydroxybenzyl)amino)pyrido[2,3-d]pyrimidin-7(8H)-one (12mg), 10% palladium-carbon (6 mg), methanol (5.0 mL) and dioxane (2.0 mL) were added, and the mixture was stirred at room temperature for 10 minutes in a hydrogen atmosphere. Palladium-carbon was filtered off and washed with methanol. The filtrate and the washes were combined, and the solvent was distilled off under reduced pressure. The obtained residue was suspended in diisopropyl ether, and the deposit was collected by filtration to obtain a yellow solid of 5-((3,4-dihydroxybenzyl)amino)-8-hydroxypyrido[2,3-d]pyrimidin-7(8H)-one (5 mg).
$^1$H-NMR (DMSO-$d_6$) $\delta$ value: 4.22-4.32 (m, 2H), 5.39 (s, 1H), 6.59-6.78 (m, 3H), 7.97 (brs, 1H), 9.01 (s, 1H), 9.37 (s, 1H).

Example 109

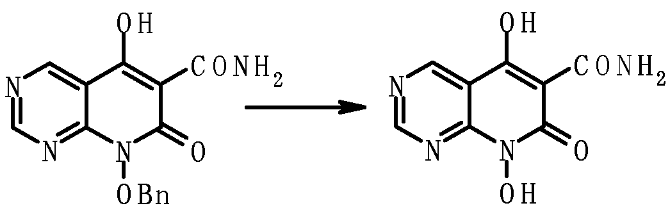

To 8-(benzyloxy)-5-hydroxy-7-oxo-7,8-dihydropyrido[2,3-d]pyrimidine-6-carboxamide (15 mg), 20% palladium hydroxide-carbon (8 mg), methanol (6.0 mL) and dioxane (6.0 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off

under reduced pressure. The obtained residue was suspended in diisopropyl ether, and the deposit was collected by filtration to obtain a yellow solid of 5,8-dihydroxy-7-oxo-7,8-dihydropyrido[2,3-d]pyrimidine-6-carboxamide (9 mg).
$^1$H-NMR (DMSO-$d_6$) δ value: 8.94 (brs, 1H), 9.19-9.34 (m, 1H), 9.21 (s, 1H), 9.27 (s, 1H), 11.21 (brs, 1H).

Example 110

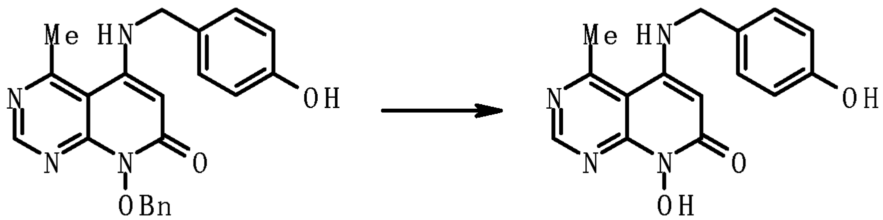

To 8-(benzyloxy)-5-((4-hydroxybenzyl)amino)-4-methylpyrido[2,3-d]pyrimidin-7(8H)-one (20 mg), 10% palladium-carbon (10 mg), methanol (8.0 mL) and dioxane (4.0 mL) were added, and the mixture was stirred at room temperature for 10 minutes in a hydrogen atmosphere. Palladium-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-5-((4-hydroxybenzyl)amino)-4-methylpyrido[2,3-d]pyrimidin-7(8H)-one (10 mg).
$^1$H-NMR (DMSO-$d_6$) δ value: 2.96 (s, 3H), 4.31 (d, J=5.4 Hz, 2H), 5.43 (s, 1H), 6.66-6.78 (m, 3H), 7.19-7.25 (m, 2H), 8.78 (s, 1H), 9.35 (brs, 1H).

Example 111

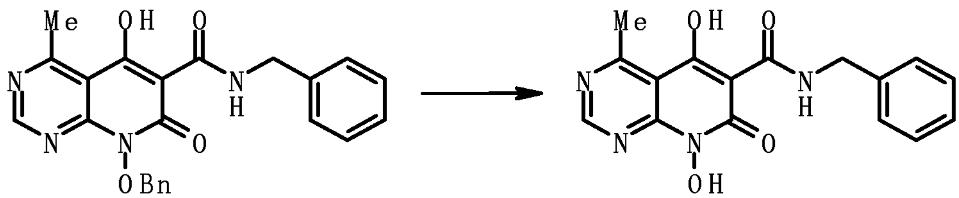

To N-benzyl-8-(benzyloxy)-5-hydroxy-4-methyl-7-oxo-7,8-dihydropyrido[2,3-d]pyrimidine-6-carboxamide (26 mg), 20% palladium hydroxide-carbon (13 mg), methanol (6.0 mL) and dioxane (6.0 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. To the obtained residue, ethyl acetate was added, and insoluble matter was filtered off. The solvent was distilled off under reduced pressure. The obtained residue was suspended in diisopropyl ether, and the deposit was collected by filtration to obtain a pale yellow solid of N-benzyl-5,8-dihydroxy-4-methyl -7-oxo-7,8-dihydropyrido[2,3-d]pyrimidine-6-carboxamide (14 mg).
$^1$H-NMR (DMSO-$d_6$) δ value: 2.92 (s, 3H), 4.63 (d, J=5.8 Hz, 2H), 7.26-7.33 (m, 1H), 7.33-7.40(m, 4H), 9.01 (s, 1H), 10.46 (brs, 1H), 11.18 (s, 1H).

Example 112

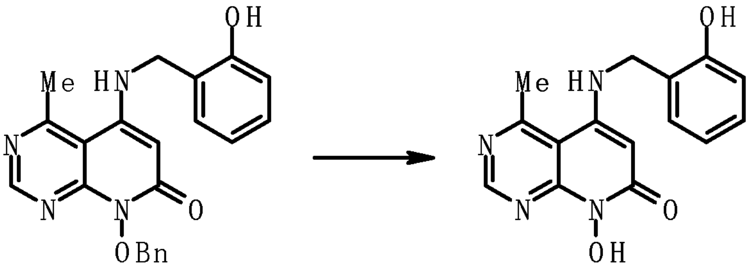

To 8-(benzyloxy)-5-((2-hydroxybenzyl)amino)-4-methylpyrido[2,3-d]pyrimidin-7(8H)-one (15 mg), 10% palladium-carbon (8 mg), methanol (4.0 mL) and dioxane (2.0 mL) were added, and the mixture was stirred at room temperature for 10 minutes in a hydrogen atmosphere. Palladium-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-5-((2-hydroxybenzyl)amino)-4-methylpyrido[2,3-d]pyrimidin-7(8H)-one (6 mg).
$^1$H-NMR (DMSO-$d_6$) δ value: 2.97 (s, 3H), 4.32-4.40 (m, 2H), 5.43 (s, 1H), 6.69-6.80 (m, 2H), 6.84-6.89 (m, 1H), 7.06-7.12 (m, 1H), 7.21-7.26 (m, 1H), 8.78 (s, 1H).

Example 113

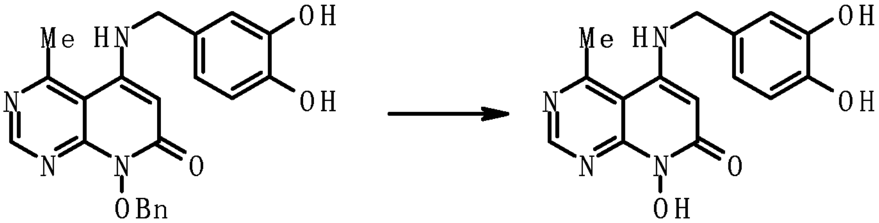

To 8-(benzyloxy)-5-((3,4-dihydroxybenzyl)amino)-4-methylpyrido[2,3-d]pyrimidin-7(8H)-one (17 mg), 10% palladium-carbon (9 mg), methanol (12 mL) and dioxane (12 mL) were added, and the mixture was stirred at room temperature for 10 minutes in a hydrogen atmosphere. Palladium-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a pale yellow solid of 5-((3,4-dihydroxybenzyl)amino)-8-hydroxy-4-methylpyrido[2,3-d]pyrimidin-7(8H)-one (10 mg).
$^1$H-NMR (DMSO-$d_6$) δ value: 2.97 (s, 3H), 4.26 (d, J=5.4 Hz, 2H), 5.42 (s, 1H), 6.62-6.74 (m, 3H), 6.78 (d, J=2.0 Hz, 1H), 8.74-8.93 (m, 2H), 8.79 (s, 1H), 10.58 (brs, 1H).

Example 114

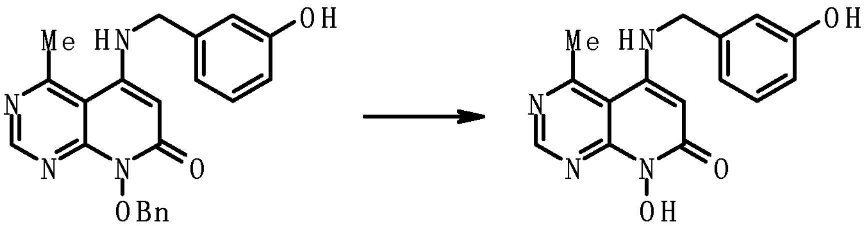

To 8-(benzyloxy)-5-((3-hydroxybenzyl)amino)-4-methylpyrido[2,3-d]pyrimidin-7(8H)-one (12 mg), 10% palladium-carbon (6 mg) and methanol (8.0 mL) were added, and the mixture was stirred at room temperature for 10 minutes in a hydrogen atmosphere. Palladium-carbon was filtered off and washed with methanol. The filtrate and the washes

were combined, and the solvent was distilled off under reduced pressure. The obtained residue was suspended in diisopropyl ether, and the deposit was collected by filtration to obtain a yellow solid of 8-hydroxy-5-((3-hydroxybenzyl)amino)-4-methylpyrido[2,3-d]pyrimidin-7(8H)-one (7 mg).
$^1$H-NMR (DMSO-$d_6$) δ value: 3.00 (s, 3H), 4.38 (d, J=5.4 Hz, 2H), 5.39 (s, 1H), 6.61-6.68 (m, 1H), 6.77-6.86 (m, 3H), 7.15 (dd, J=7.7, 7.7 Hz, 1H), 8.80 (s, 1H), 9.38 (brs, 1H).

Example 115

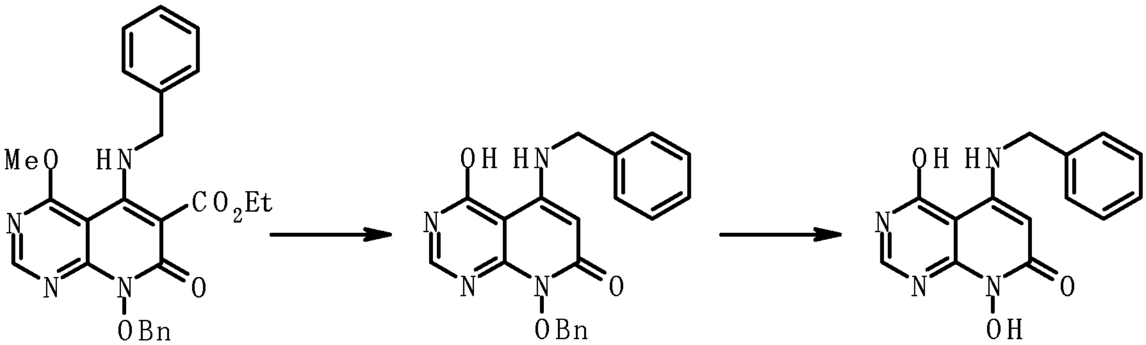

To ethyl 5-(benzylamino)-8-(benzyloxy)-4-methoxy-7-oxo-7,8-dihydropyrido[2,3-d]pyrimidine-6-carboxylate (35 mg), methanol (1.8 mL) and a 1 mol/L aqueous sodium hydroxide solution (1.8 mL) were added, and the mixture was heated with stirring in a nitrogen atmosphere at 70 to 80°C for 20 minutes and at 80 to 90°C for 1 hour and then heated to reflux for 1 hour. After cooling of the reaction mixture, a 5 mol/L aqueous sodium hydroxide solution (0.9 mL) was added thereto, and the mixture was heated to reflux for 2 hours in a nitrogen atmosphere. After cooling of the reaction mixture, a 5 mol/L aqueous sodium hydroxide solution (0.9 mL) was added thereto, and the mixture was heated to reflux for 3 hours in a nitrogen atmosphere. The reaction mixture was pH-adjusted to 7 by the addition of 2 mol/L hydrochloric acid under ice cooling, and the deposit was collected by filtration. To the obtained solid, methanol was added, and insoluble matter was filtered off. Then, the solvent was distilled off under reduced pressure to obtain a pale yellow solid (30 mg). To the obtained pale yellow solid (30 mg), 20% palladium hydroxide-carbon (15 mg), methanol (6.0 mL) and dioxane (2.0 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a pale yellow solid of 5-(benzylamino)-4,8-dihydroxypyrido[2,3-d]pyrimidin-7(8H)-one (9 mg).
$^1$H-NMR (DMSO-$d_6$) δ value: 4.33 (d, J=5.6 Hz, 2H), 5.01 (s, 1H), 7.22-7.40 (m, 5H), 8.04 (s, 1H), 10.10 (brs, 1H).

Example 116

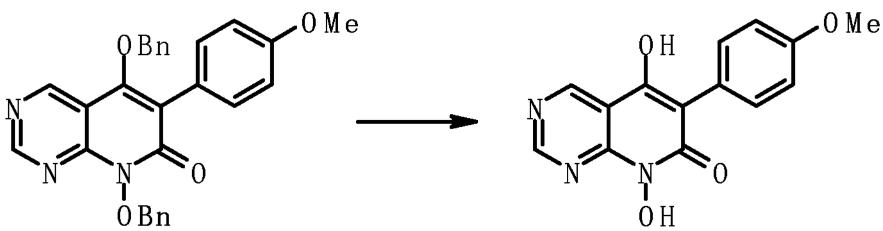

To 5,8-bis(benzyloxy)-6-(4-methoxyphenyl)pyrido[2,3-d]pyrimidin-7(8H)-one (9 mg), 20% palladium hydroxide-carbon (5 mg), methanol (5.0 mL) and dioxane (2.5 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain

a yellow solid of 5,8-dihydroxy-6-(4-methoxyphenyl)pyrido[2,3-d]pyrimidin-7(8H)-one (5 mg).
$^1$H-NMR (DMSO-$d_6$) δ value: 3.80 (s, 3H), 6.94-7.02 (m, 2H), 7.32-7.40 (m, 2H), 9.04 (s, 1H), 9.18 (s, 1H), 10.89 (brs, 1H).

Example 117

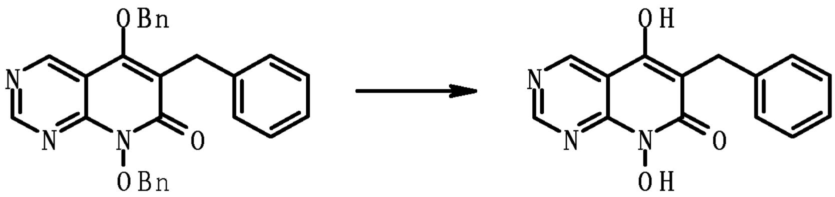

To 6-benzyl-5,8-bis(benzyloxy)pyrido[2,3-d]pyrimidin-7(8H)-one (35 mg), 20% palladium hydroxide-carbon (18 mg), methanol (7.0 mL) and dioxane (7.0 mL) were added, and the mixture was stirred at room temperature for 10 minutes in a hydrogen atmosphere. To the reaction mixture, 20% palladium hydroxide-carbon (18 mg) was added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a yellow solid of 6-benzyl-5,8-dihydroxypyrido[2,3-d]pyrimidin-7(8H)-one (15 mg).
$^1$H-NMR (DMSO-$d_6$) δ value: 3.96 (s, 2H), 7.10-7.29 (m, 5H), 9.04 (s, 1H), 9.20 (s, 1H), 10.92 (brs, 1H).

Example 118

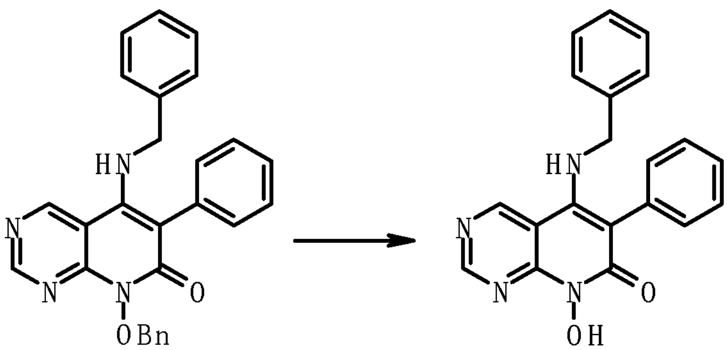

To 5-(benzylamino)-8-(benzyloxy)-6-phenylpyrido[2,3-d]pyrimidin-7(8H)-one (12 mg), 20% palladium hydroxide-carbon (6 mg) and methanol (8.0 mL) were added, and the mixture was stirred at room temperature for 10 minutes in a hydrogen atmosphere. To the reaction mixture, dioxane was added, and palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a yellow solid of 5-(benzylamino)-8-hydroxy-6-phenylpyrido[2,3-d]pyrimidin-7(8H)-one (9 mg). $^1$H-NMR (DMSO-$d_6$) δ value: 3.86 (d, J=6.6 Hz, 2H), 6.85-6.92 (m, 2H), 7.04-7.23 (m, 6H), 7.30-7.37 (m, 3H), 9.04 (s, 1H), 9.48 (s, 1H), 10.78 (brs, 1H).

Example 119

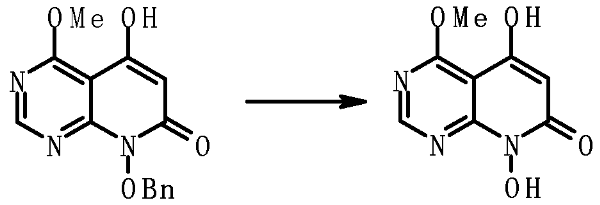

To 8-(benzyloxy)-5-hydroxy-4-methoxypyrido[2,3-d]pyrimidin-7(8H)-one (6 mg), a palladium-carbon-ethylenediamine complex (Pd: 3.5-6.5%) (3 mg), methanol (3.0 mL) and dioxane (1.5 mL) were added, and the mixture was stirred at room temperature for 10 minutes in a hydrogen atmosphere. The palladium-carbon-ethylenediamine complex was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a pale yellow solid of 5,8-dihydroxy-4-methoxypyrido[2,3-d]pyrimidin-7(8H)-one (3 mg).
$^1$H-NMR (DMSO-$d_6$) $\delta$ value: 3.49 (s, 3H), 5.78 (s, 1H), 8.64 (s, 1H), 11.10 (brs, 1H), 11.72 (brs, 1H).

Example 120

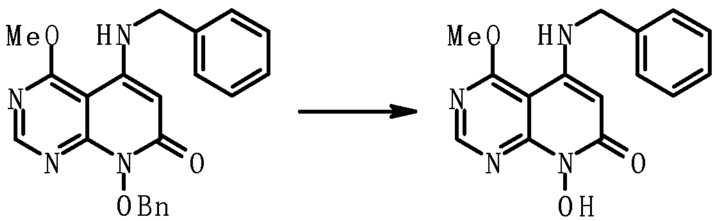

To 5-(benzylamino)-8-(benzyloxy)-4-methoxypyrido[2,3-d]pyrimidin-7(8H)-one (21 mg), a palladium-carbon-ethylenediamine complex (Pd: 3.5-6.5%) (11 mg), methanol (5.0 mL) and dioxane (5.0 mL) were added, and the mixture was stirred at room temperature for 15 minutes in a hydrogen atmosphere. The palladium-carbon-ethylenediamine complex was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a pale yellow solid of 5-(benzylamino)-8-hydroxy-4-methoxypyrido[2,3-d]pyrimidin-7(8H)-one (6 mg).
$^1$H-NMR (DMSO-$d_6$) $\delta$ value: 3.43 (s, 3H), 4.40 (d, J=5.6 Hz, 2H), 5.26 (s, 1H), 7.25-7.41 (m, 5H), 8.53 (s, 1H), 8.99-9.05 (m, 1H).

Example 121

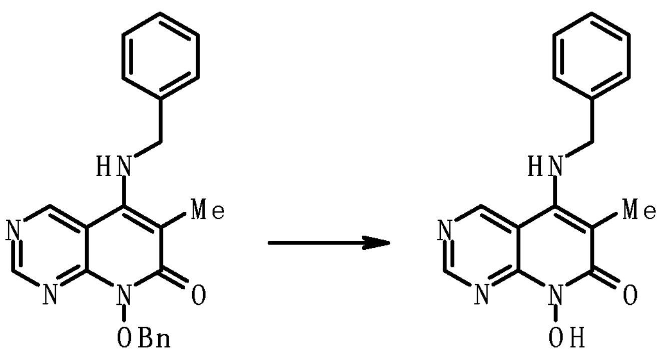

To 5-(benzylamino)-8-(benzyloxy)-6-methylpyrido[2,3-d]pyrimidin-7(8H)-one (9 mg), 20% palladium hydroxide-carbon (5 mg) and methanol (5.0 mL) were added, and the mixture was stirred at room temperature for 10 minutes in

a hydrogen atmosphere. Palladium hydroxide-carbon was filtered off and washed with methanol. The filtrate and the washes were combined, and the solvent was distilled off under reduced pressure. The obtained residue was suspended in diisopropyl ether, and the deposit was collected by filtration to obtain a yellow solid of 5-(benzylamino)-8-hydroxy-6-methylpyrido[2,3-d]pyrimidin-7(8H)-one (5 mg). $^1$H-NMR (DMSO-$d_6$) δ value: 2.07 (s, 3H), 4.60 (d, J=6.8 Hz, 2H), 6.55-6.63 (m, 1H), 7.18-7.36 (m, 5H), 8.95 (s, 1H), 9.26 (s, 1H).

Example 122

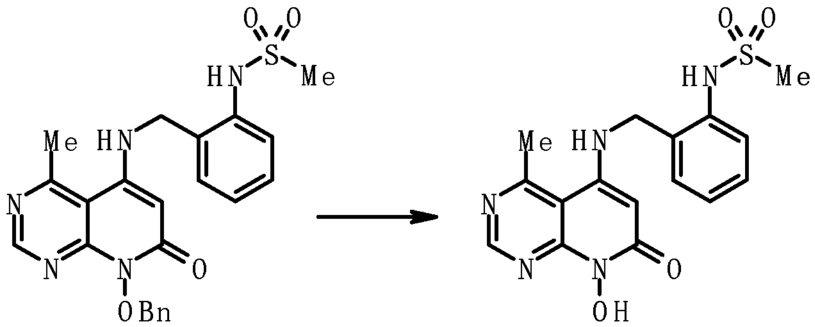

To N-(2-(((8-(benzyloxy)-4-methyl-7-oxo-7,8-dihydropyrido[2,3-d]pyrimidin-5-yl)amino)methyl)phenyl)methanesulfonamide (8 mg), 20% palladium hydroxide-carbon (4 mg), methanol (4.0 mL) and dioxane (4.0 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a pale yellow solid of N-(2-(((8-hydroxy-4-methyl-7-oxo-7,8-dihydropyrido[2,3-d]pyrimidin-5-yl)amino)methyl)phenyl)methanesulfonamide (5 mg).
$^1$H-NMR (DMSO-$d_6$) δ value: 3.01 (s, 6H), 4.46-4.60 (m, 2H), 5.31 (s, 1H), 6.95 (brs, 1H), 7.12-7.43 (m, 4H), 8.80 (s, 1H), 9.34 (brs, 1H), 10.57 (brs, 1H).

Example 123

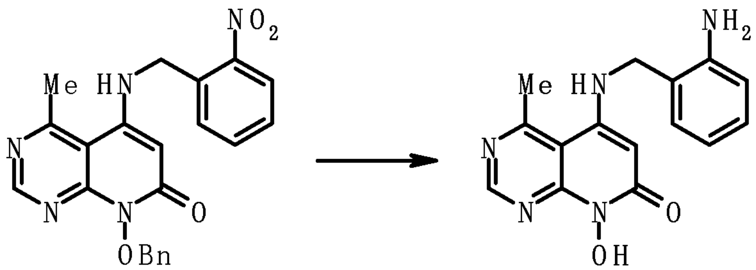

To 8-(benzyloxy)-4-methyl-5-((2-nitrobenzyl)amino)pyrido[2,3-d]pyrimidin-7(8H)-one (15mg), 20% palladium hydroxide-carbon (8 mg), methanol (5.0 mL) and dioxane (5.0 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a light brown solid of 5-((2-aminobenzyl)amino)-8-hydroxy-4-methylpyrido[2,3-d]pyrimidin-7(8H)-one (10mg).
$^1$H-NMR (DMSO-$d_6$) δ value: 2.97 (s, 3H), 4.24 (d, J=5.1 Hz, 2H), 5.11 (brs, 2H), 5.44 (s, 1H), 6.50-6.57 (m, 1H), 6.57-6.63 (m, 1H), 6.66-6.72 (m, 1H), 6.95-7.01 (m, 1H), 7.05-7.11 (m, 1H), 8.79 (s, 1H).

Example 124

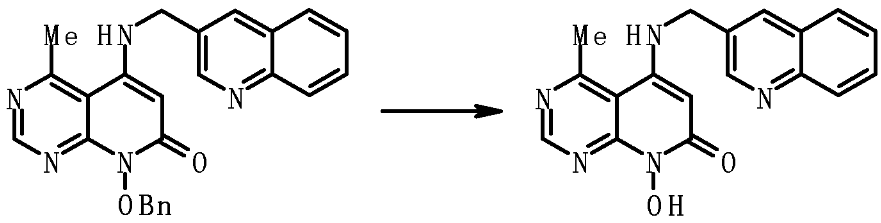

To 8-(benzyloxy)-4-methyl-5-(((quinolin-3-yl)methyl)amino)pyrido[2,3-d]pyrimidin-7(8H)-one (19 mg), 20% palladium hydroxide-carbon (10 mg) and methanol (8.0 mL) were added, and the mixture was stirred at room temperature for 10 minutes in a hydrogen atmosphere. To the reaction mixture, 20% palladium hydroxide-carbon (19 mg) was added, and the mixture was stirred at room temperature for 10 minutes in a hydrogen atmosphere. To the reaction mixture, 20% palladium hydroxide-carbon (19 mg) was added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-4-methyl-5-(((quinolin-3-yl)methyl)amino)pyrido[2,3-d]pyrimidin-7(8H)-one (5 mg).
$^1$H-NMR (DMSO-$d_6$) δ value: 3.04 (s, 3H), 4.67-4.75 (m, 2H), 5.50 (s, 1H), 6.91-7.00 (m, 1H), 7.56-7.64 (m, 1H), 7.70-7.78 (m, 1H), 7.95-8.06 (m, 2H), 8.31-8.36 (m, 1H), 8.80 (s, 1H), 9.00 (d, J=2.0 Hz, 1H).

Example 125

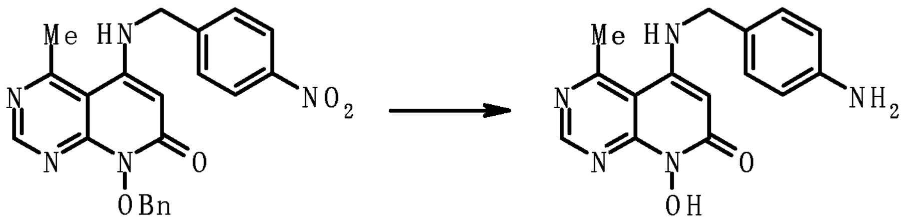

To 8-(benzyloxy)-4-methyl-5-((4-nitrobenzyl)amino)pyrido[2,3-d]pyrimidin-7(8H)-one (14mg), 20% palladium hydroxide-carbon (7 mg), methanol (5.0 mL) and dioxane (5.0 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a pale yellow solid of 5-((4-aminobenzyl)amino)-8-hydroxy-4-methylpyrido[2,3-d]pyrimidin-7(8H)-one (9mg).
$^1$H-NMR (DMSO-$d_6$) δ value: 2.95 (s, 3H), 4.23 (d, J=4.9 Hz, 2H), 5.01 (brs, 2H), 5.45 (s, 1H), 6.52-6.57 (m, 2H), 6.57-6.64 (m, 1H), 7.04-7.10 (m, 2H), 8.78 (s, 1H).

Example 126

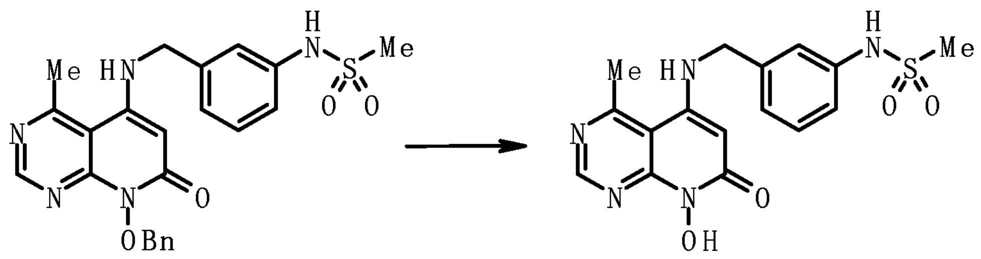

To N-(3-(((8-(benzyloxy)-4-methyl-7-oxo-7,8-dihydropyrido[2,3-d]pyrimidin-5-yl)amino)methyl)phenyl)methanesulfonamide (19 mg), 20% palladium hydroxide-carbon (10 mg), methanol (5.0 mL) and dioxane (5.0 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of N-(3-(((8-hydroxy-4-methyl-7-oxo-7,8-dihydropyrido[2,3-d]pyrimidin-5-yl)amino)methyl)phenyl)methanesulfonamide (11 mg). $^1$H-NMR (DMSO-$d_6$) δ value: 2.95 (s, 3H), 3.00 (s, 3H), 4.40-4.47 (m, 2H), 5.39 (s, 1H), 6.82-6.88 (m, 1H), 7.06-7.15 (m, 2H), 7.22-7.27 (m, 1H), 7.31 (dd, J=7.9, 7.9 Hz, 1H), 8.80 (s, 1H).

Example 127

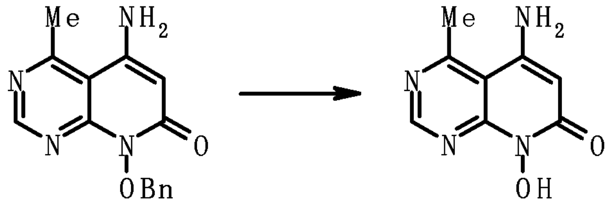

To 5-amino-8-(benzyloxy)-4-methylpyrido[2,3-d]pyrimidin-7(8H)-one (7 mg), a palladium-carbon-ethylenediamine complex (Pd: 3.5-6.5%) (4 mg) and methanol (5.0 mL) were added, and the mixture was stirred at room temperature for 1 hour and 20 minutes in a hydrogen atmosphere. The palladium-carbon-ethylenediamine complex was filtered off and washed with methanol. The filtrate and the washes were combined, and the solvent was distilled off under reduced pressure to obtain a yellow solid of 5-amino-8-hydroxy-4-methylpyrido[2,3-d]pyrimidin-7(8H)-one (4 mg).
$^1$H-NMR (DMSO-$d_6$) δ value: 2.88 (s, 3H), 5.73 (s, 1H), 6.32 (brs, 2H), 8.73 (s, 1H).

Example 128

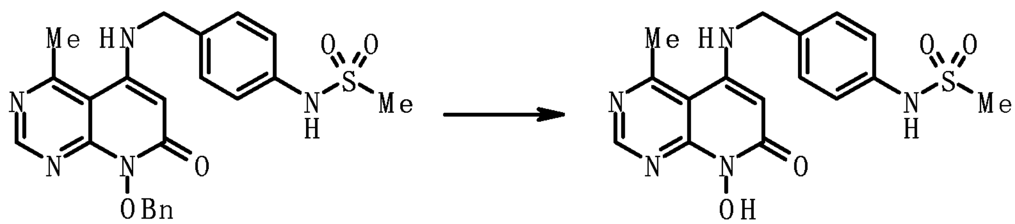

To N-(4-(((8-(benzyloxy)-4-methyl-7-oxo-7,8-dihydropyrido[2,3-d]pyrimidin-5-yl)amino)methyl)phenyl)methanesulfonamide (11 mg), 20% palladium hydroxide-carbon (6 mg), methanol (5.0 mL) and dioxane (5.0 mL) were added, and the mixture was stirred at room temperature for 10 minutes in a hydrogen atmosphere. To the reaction mixture, 20% palladium hydroxide-carbon (6 mg) was added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a pale yellow

solid of N-(4-(((8-hydroxy-4-methyl-7-oxo-7,8-dihydropyrido[2,3-d]pyrimidin-5-yl)amino)methyl)phenyl)methanesulfonamide (6 mg).
$^1$H-NMR (DMSO-$d_6$) δ value: 2.96 (s, 3H), 2.99 (s, 3H), 4.37-4.43 (m, 2H), 5.41 (s, 1H), 6.76-6.82 (m, 1H), 7.15-7.21 (m, 2H), 7.34-7.41 (m, 2H), 8.79 (s, 1H).

Example 129

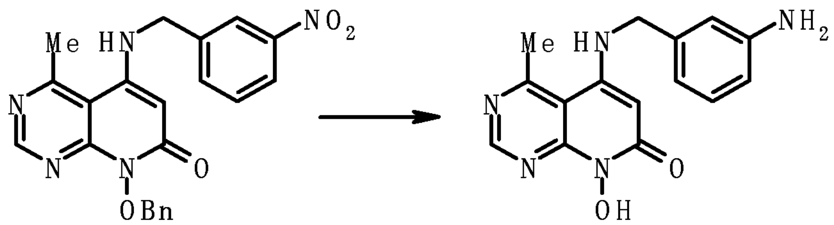

To 8-(benzyloxy)-4-methyl-5-((3-nitrobenzyl)amino)pyrido[2,3-d]pyrimidin-7(8H)-one (13 mg), 20% palladium hydroxide-carbon (7 mg), methanol (5.0 mL) and dioxane (5.0 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in diisopropyl ether, and the deposit was collected by filtration to obtain a pale yellow solid of 5-((3-aminobenzyl)amino)-8-hydroxy-4-methylpyrido[2,3-d]pyrimidin-7(8H)-one (7 mg). $^1$H-NMR (DMSO-$d_6$) δ value: 2.99 (s, 3H), 4.30 (d, J=5.6 Hz, 2H), 5.07 (brs, 2H), 5.38 (s, 1H), 6.41-6.48 (m, 1H), 6.50-6.61 (m, 2H), 6.72-6.79 (m, 1H), 6.99 (dd, J=7.7, 7.7 Hz, 1H), 8.78 (s, 1H).

Example 130

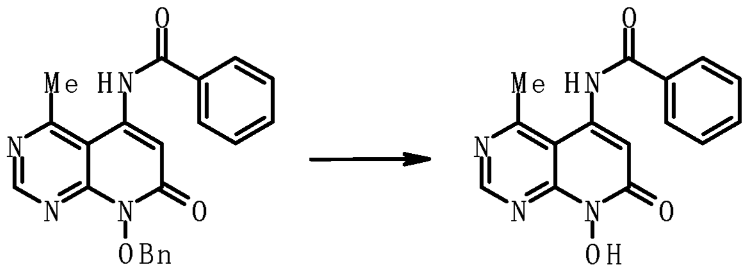

To N-(8-(benzyloxy)-4-methyl-7-oxo-7,8-dihydropyrido[2,3-d]pyrimidin-5-yl)benzamide (4 mg), 20% palladium hydroxide-carbon (2 mg), methanol (2.5 mL) and dioxane (2.5 mL) were added, and the mixture was stirred at room temperature for 10 minutes in a hydrogen atmosphere. To the reaction mixture, 20% palladium hydroxide-carbon (2 mg) was added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure to obtain a yellow solid of N-(8-hydroxy-4-methyl-7-oxo-7,8-dihydropyrido[2,3-d]pyrimidin-5-yl)benzamide (3 mg).
$^1$H-NMR ($CD_3OD$) δ value: 2.89 (s, 3H), 7.02 (s, 1H), 7.54-7.62 (m, 2H), 7.63-7.70 (m, 1H), 8.00-8.06 (m, 2H), 8.91 (s, 1H).

Example 131

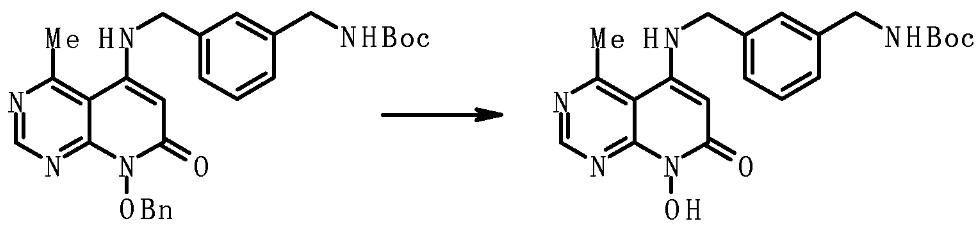

To tert-butyl (3-(((8-(benzyloxy)-4-methyl-7-oxo-7,8-dihydropyrido[2,3-d]pyrimidin-5-yl)amino)methyl)benzyl)carbamate (19 mg), 10% palladium-carbon (10 mg) and methanol (5.0 mL) were added, and the mixture was stirred at room temperature for 10 minutes in a hydrogen atmosphere. Palladium-carbon was filtered off and washed with methanol. The filtrate and the washes were combined, and the solvent was distilled off under reduced pressure to obtain a yellow oil of tert-butyl (3-(((8-hydroxy-4-methyl-7-oxo-7,8-dihydropyrido[2,3-d]pyrimidin-5-yl)amino)methyl)benzyl)carbamate (11 mg).
$^1$H-NMR ($CD_3OD$) δ value: 1.39 (s, 9H), 3.07 (s, 3H), 4.22 (s, 2H), 4.52 (s, 2H), 5.61 (s, 1H), 7.16-7.22 (m, 1H), 7.27-7.37 (m, 3H), 8.80 (s, 1H).

Example 132

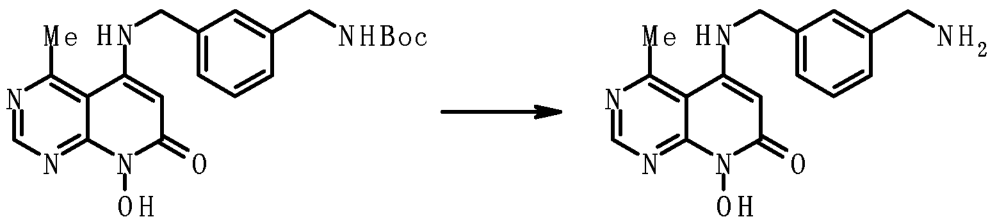

To a solution of tert-butyl (3-(((8-hydroxy-4-methyl-7-oxo-7,8-dihydropyrido[2,3-d]pyrimidin-5-yl)amino)methyl)benzyl)carbamate (11 mg) in methylene chloride (2.0 mL), TFA (0.50 mL) was added under ice cooling, and the mixture was stirred at room temperature for 3 hours. The solvent in the reaction mixture was distilled off under reduced pressure. To the obtained residue, ethanol (1.0 mL) and a 2.9 mol/L solution of hydrogen chloride in ethanol (0.20 mL) were added, and the solvent was distilled off under reduced pressure. The obtained residue was suspended in ethyl acetate, and the deposit was collected by filtration. To the obtained solid, methanol (2.0 mL) and water (2.0 mL) were added, and the mixture was pH-adjusted to 6.8 by the dropwise addition of a 1 mol/L aqueous sodium hydroxide solution. The deposit was collected by filtration to obtain a brown solid of 5-((3-(aminomethyl)benzyl)amino)-8-hydroxy-4-methylpyrido[2,3-d]pyrimidin-7(8H)-one (6 mg). $^1$H-NMR (DMSO-$d_6$) δ value: 3.01 (s, 3H), 3.77 (s, 2H), 4.38-4.52 (m, 2H), 5.38 (s, 1H), 6.84 (brs, 1H), 7.20-7.35 (m, 3H), 7.35-7.45 (m, 1H), 8.80 (s, 1H).

Example 133

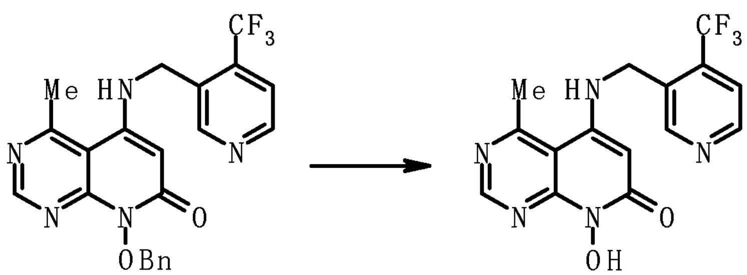

To 8-(benzyloxy)-4-methyl-5-(((4-(trifluoromethyl)pyridin-3-yl)methyl)amino)pyrido[2,3-d]pyrimidin-7(8H)-one (7 mg), 20% palladium hydroxide-carbon (4 mg), methanol (3.0 mL) and dioxane (3.0 mL) were added, and the mixture was stirred at room temperature for 10 minutes in a hydrogen atmosphere. To the reaction mixture, 20% palladium

hydroxide-carbon (4 mg) was added, and the mixture was stirred at room temperature for 5 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-4-methyl-5-(((4-(trifluoromethyl)pyridin-3-yl)methyl)amino)pyrido[2,3-d]pyrimidin-7(8H)-one (4 mg).
$^1$H-NMR (DMSO-$d_6$) δ value: 2.99 (s, 3H), 4.62-4.72 (m, 2H), 5.32 (s, 1H), 6.87 (brs, 1H), 7.80 (d, J=5.1 Hz, 1H), 8.77-8.83 (m, 2H), 8.85 (s, 1H).

Example 134

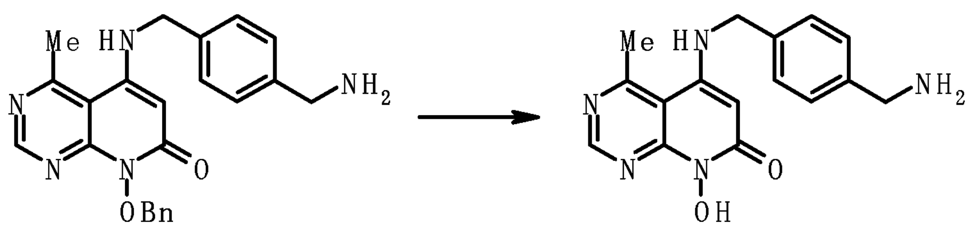

To 5-((4-(aminomethyl)benzyl)amino)-8-(benzyloxy)-4-methylpyrido[2,3-d]pyrimidin-7(8H)-one (23 mg), 20% palladium hydroxide-carbon (12 mg), methanol (5.0 mL) and dioxane (2.5 mL) were added, and the mixture was stirred at room temperature for 10 minutes in a hydrogen atmosphere. To the reaction mixture, 20% palladium hydroxide-carbon (23 mg) was added, and the mixture was stirred at room temperature for 10 minutes in a hydrogen atmosphere. To the reaction mixture, 20% palladium hydroxide-carbon (35 mg) was added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 5-((4-(aminomethyl)benzyl)amino)-8-hydroxy-4-methylpyrido[2,3-d]pyrimidin-7(8H)-one (9 mg).
$^1$H-NMR ($CD_3OD$) δ value: 3.07 (s, 3H), 4.06 (s, 2H), 4.57 (s, 2H), 5.55 (s, 1H), 7.42-7.47 (m, 2H), 7.48-7.53 (m, 2H), 8.81 (s, 1H).

Example 135

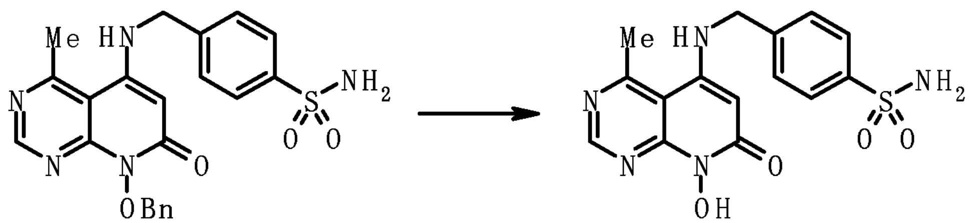

To 4-(((8-(benzyloxy)-4-methyl-7-oxo-7,8-dihydropyrido[2,3-d]pyrimidin-5-yl)amino)methyl)benzenesulfonamide (23 mg), 20% palladium hydroxide-carbon (11 mg), methanol (15 mL) and dioxane (15 mL) were added, and the mixture was stirred at room temperature for 10 minutes in a hydrogen atmosphere. To the reaction mixture, 20% palladium hydroxide-carbon (11 mg) was added, and the mixture was stirred at room temperature for 5 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 4-(((8-hydroxy-4-methyl-7-oxo-7,8-dihydropyrido[2,3-d]pyrimidin-5-yl)amino)methyl)benzenesulfonamide (18 mg).
$^1$H-NMR (DMSO-$d_6$) δ value: 3.02 (s, 3H), 4.56 (d, J=5.4 Hz, 2H), 5.33 (s, 1H), 6.90-6.97 (m, 1H), 7.31 (brs, 2H), 7.58-7.63 (m, 2H), 7.78-7.83 (m, 2H), 8.81 (s, 1H).

Example 136

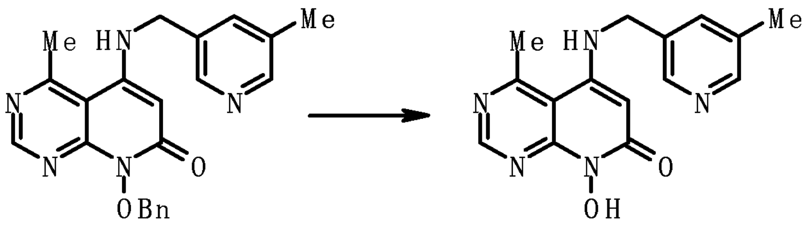

To 8-(benzyloxy)-4-methyl-5-(((5-methylpyridin-3-yl)methyl)amino)pyrido[2,3-d]pyrimidin-7(8H)-one (8 mg), 20% palladium hydroxide-carbon (4 mg), methanol (5.0 mL) and dioxane (2.5 mL) were added, and the mixture was stirred at room temperature for 10 minutes in a hydrogen atmosphere. To the reaction mixture, 20% palladium hydroxide-carbon (4 mg) was added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-4-methyl-5-(((5-methylpyridin-3-yl)methyl)amino)pyrido[2,3-d]pyrimidin-7(8H)-one (5 mg).
$^1$H-NMR (DMSO-$d_6$) δ value: 2.29 (s, 3H), 2.99 (s, 3H), 4.47 (d, J=5.4 Hz, 2H), 5.42 (s, 1H), 6.79-6.86 (m, 1H), 7.62-7.66 (m, 1H), 8.30-8.34 (m, 1H), 8.42-8.47 (m, 1H), 8.79 (s, 1H).

Example 137

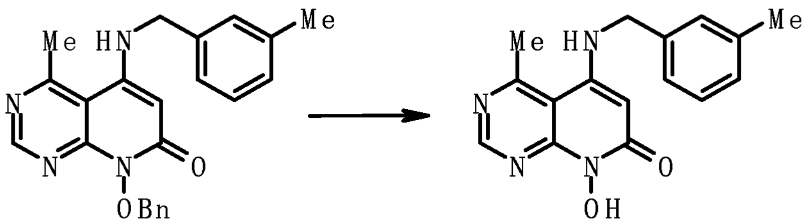

To 8-(benzyloxy)-4-methyl-5-((3-methylbenzyl)amino)pyrido[2,3-d]pyrimidin-7(8H)-one (42 mg), 20% palladium hydroxide-carbon (21 mg), methanol (5.0 mL) and dioxane (5.0 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-4-methyl-5-((3-methylbenzyl)amino)pyrido[2,3-d]pyrimidin-7(8H)-one (22mg).
$^1$H-NMR (DMSO-$d_6$) δ value: 2.30 (s, 3H), 3.00 (s, 3H), 4.42 (d, J=5.6 Hz, 2H), 5.39 (s, 1H), 6.78-6.85 (m, 1H), 7.05-7.11 (m, 1H), 7.18-7.28 (m, 3H), 8.79 (s, 1H), 10.55 (brs, 1H).

Example 138

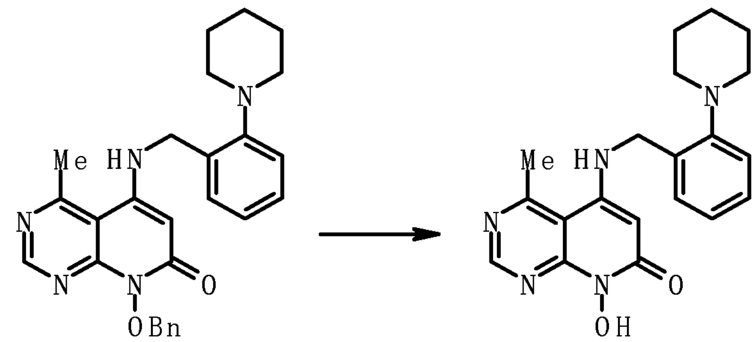

To 8-(benzyloxy)-4-methyl-5-((2-(piperidin-1-yl)benzyl)amino)pyrido[2,3-d]pyrimidin-7(8H)-one (96 mg), 20% palladium hydroxide-carbon (48 mg), methanol (7.5 mL) and dioxane (7.5 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in ethyl acetate, and the deposit was collected by filtration to obtain a pale yellow solid of 8-hydroxy-4-methyl-5-((2-(piperidin-1-yl)benzyl)amino)pyrido[2,3-d]pyrimidin-7(8H)-one (67 mg).

$^1$H-NMR (DMSO-$d_6$) δ value: 1.50-1.62 (m, 2H), 1.67-1.78 (m, 4H), 2.80-2.91 (m, 4H), 2.98 (s, 3H), 4.43 (d, J=5.6 Hz, 2H), 5.49 (s, 1H), 6.85-6.93 (m, 1H), 7.01-7.08 (m, 1H), 7.12-7.17 (m, 1H), 7.20-7.27 (m, 1H), 7.38-7.44 (m, 1H), 8.78 (s, 1H), 10.52 (brs, 1H).

Example 139

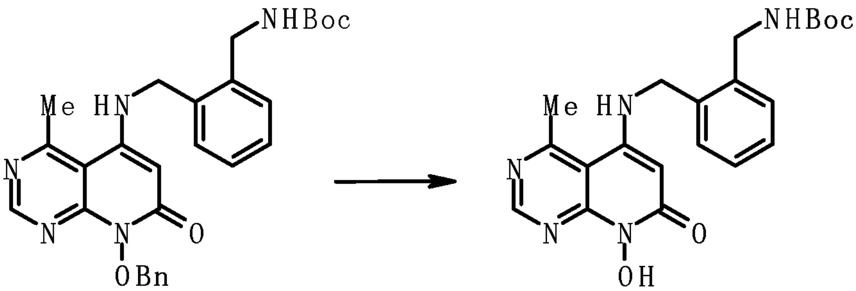

To tert-butyl (2-(((8-(benzyloxy)-4-methyl-7-oxo-7,8-dihydropyrido[2,3-d]pyrimidin-5-yl)amino)methyl)benzyl)carbamate (30 mg), 10% palladium-carbon (15 mg) and methanol (5.0 mL) were added, and the mixture was stirred at room temperature for 10 minutes in a hydrogen atmosphere. To the reaction mixture, 10% palladium-carbon (8 mg) was added, and the mixture was stirred at room temperature for 5 minutes in a hydrogen atmosphere. Palladium-carbon was filtered off and washed with a mixed solvent of methanol and dioxane. The filtrate and the washes were combined, and the solvent was distilled off under reduced pressure to obtain a light brown solid of tert-butyl (2-(((8-hydroxy-4-methyl-7-oxo-7,8-dihydropyrido[2,3-d]pyrimidin-5-yl)amino)methyl)benzyl)carbamate (23 mg).

$^1$H-NMR ($CD_3OD$) δ value: 1.39 (s, 9H), 3.00 (s, 3H), 4.35 (s, 2H), 4.53 (s, 2H), 5.68 (s, 1H), 7.24-7.42 (m, 4H), 8.81 (s, 1H).

Example 140

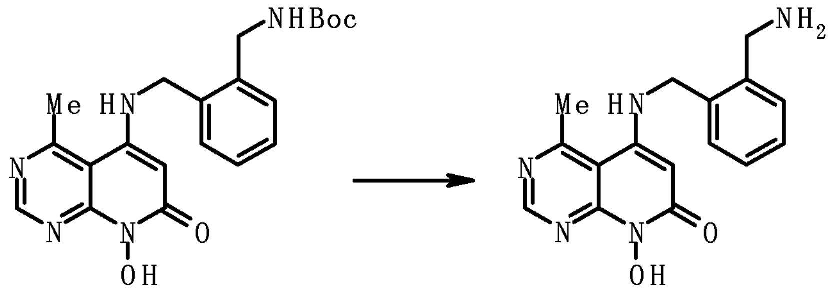

To a suspension of tert-butyl (2-(((8-hydroxy-4-methyl-7-oxo-7,8-dihydropyrido[2,3-d]pyrimidin-5-yl)amino)methyl)benzyl)carbamate (22 mg) in methylene chloride (2.0 mL), TFA (0.50 mL) was added under ice cooling, and the mixture was stirred at room temperature for 3 hours. The solvent in the reaction mixture was distilled off under reduced pressure. To the obtained residue, ethanol (5.0 mL) and a 2.9 mol/L solution of hydrogen chloride in ethanol (0.40 mL) were added, and the solvent was distilled off under reduced pressure. To the obtained residue, methanol (3.0 mL) and water (3.0 mL) were added, and the mixture was pH-adjusted to 7.0 by the dropwise addition of a 2 mol/L aqueous sodium hydroxide solution. The solvent was distilled off under reduced pressure. To the obtained residue, methanol and dioxane were added, and insoluble matter was filtered off. Then, the solvent was distilled off under reduced pressure. The obtained residue was suspended in a mixed solvent of 2-propanol and diisopropyl ether, and the deposit was collected by filtration to obtain a pale yellow solid of 5-((2-(aminomethyl)benzyl)amino)-8-hydroxy-4-methylpyrido[2,3-d]pyrimidin-7(8H)-one (16 mg).
$^1$H-NMR (DMSO-$d_6$) δ value: 2.97 (s, 3H), 4.11 (s, 2H), 4.52-4.58 (m, 2H), 5.54 (s, 1H), 6.85 (brs, 1H), 7.32-7.40 (m, 3H), 7.45-7.51 (m, 1H), 8.81 (s, 1H).

Example 141

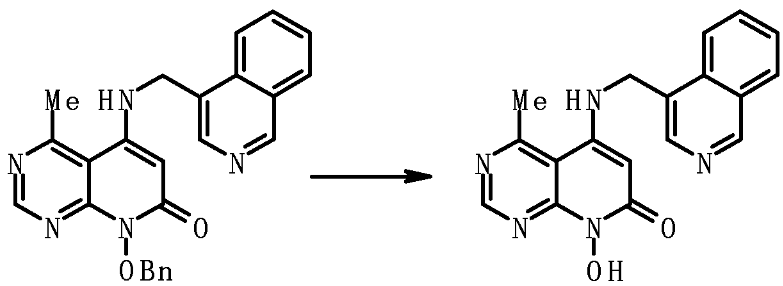

To 8-(benzyloxy)-5-(((isoquinolin-4-yl)methyl)amino)-4-methylpyrido[2,3-d]pyrimidin-7(8H)-one (32 mg), 20% palladium hydroxide-carbon (48 mg), methanol (5.0 mL) and dioxane (5.0 mL) were added, and the mixture was stirred at room temperature for 15 minutes in a hydrogen atmosphere. Palladium hydroxide-carbon was filtered off and washed with a mixed solvent of methanol and dioxane. The filtrate and the washes were combined, and the solvent was distilled off under reduced pressure. To the obtained residue, chloroform and a 0.5 mol/L aqueous sodium hydroxide solution were added, followed by separation of an aqueous layer. An organic layer was extracted twice with a 0.5 mol/L aqueous sodium hydroxide solution, combined with the aqueous layer, and pH-adjusted to 6.5 by the addition of 2 mol/L hydrochloric acid. The deposit was collected by filtration to obtain a yellow solid of 8-hydroxy-5-(((isoquinolin-4-yl)methyl)amino)-4-methylpyrido[2,3-d]pyrimidin-7(8H)-one (2 mg).
$^1$H-NMR (DMSO-$d_6$) δ value: 2.90 (s, 3H), 4.84-4.93 (m, 2H), 5.64 (s, 1H), 6.78-6.87 (m, 1H), 7.71-7.79 (m, 1H), 7.85-7.93 (m, 1H), 8.20 (d, J=7.8 Hz, 1H), 8.27 (d, J=8.8 Hz, 1H), 8.56 (s, 1H), 8.80 (s, 1H), 9.29 (s, 1H), 10.62 (brs, 1H).

Example 142

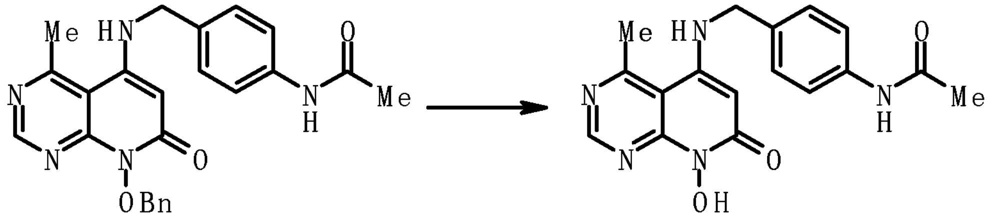

To N-(4-(((8-(benzyloxy)-4-methyl-7-oxo-7,8-dihydropyrido[2,3-d]pyrimidin-5-yl)amino)methyl)phenyl)acetamide (22 mg), 20% palladium hydroxide-carbon (11 mg), methanol (5.0 mL) and dioxane (2.5 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of N-(4-(((8-hydroxy-4-methyl-7-oxo-7,8-dihydropyrido[2,3-d]pyrimidin-5-yl)amino)methyl)phenyl)acetamide (16 mg).
$^1$H-NMR (DMSO-$d_6$) δ value: 2.02 (s, 3H), 2.98 (s, 3H), 4.39 (d, J=5.1 Hz, 2H), 5.40 (s, 1H), 6.73-6.81 (m, 1H), 7.33 (d, J=8.3 Hz, 2H), 7.55 (d, J=8.6 Hz, 2H), 8.78 (s, 1H), 9.93 (s, 1H), 10.61 (brs, 1H).

Example 143

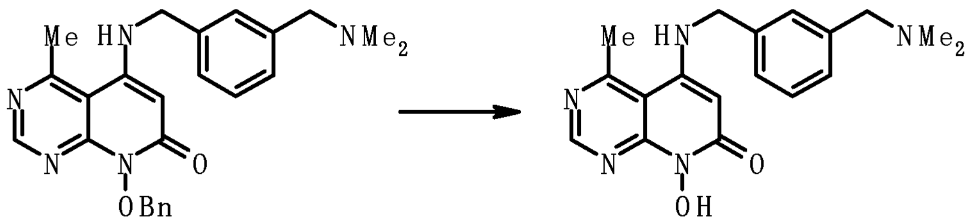

To 8-(benzyloxy)-5-((3-((dimethylamino)methyl)benzyl)amino)-4-methylpyrido[2,3-d]pyrimidin-7(8H)-one (39 mg), a palladium-carbon-ethylenediamine complex (Pd: 3.5-6.5%) (20 mg), methanol (5.0 mL) and dioxane (5.0 mL) were added, and the mixture was stirred at room temperature for 12 hours in a hydrogen atmosphere. The palladium-carbon-ethylenediamine complex was filtered off and washed with a mixed solvent of methanol and dioxane. The filtrate and the washes were combined, and the solvent was distilled off under reduced pressure. To the obtained residue, chloroform and a 0.5 mol/L aqueous sodium hydroxide solution were added, followed by separation of an aqueous layer. An organic layer was extracted with a 0.5 mol/L aqueous sodium hydroxide solution, combined with the aqueous layer, and pH-adjusted to 7.0 by the addition of 1 mol/L hydrochloric acid, and the solvent was distilled off under reduced pressure. To the obtained residue, methanol and dioxane were added, and insoluble matter was filtered off. Then, the solvent was distilled off under reduced pressure. The obtained residue was suspended in a mixed solvent of 2-propanol and diisopropyl ether, and the deposit was collected by filtration to obtain a yellow solid of 5-((3-((dimethylamino)methyl)benzyl)amino)-8-hydroxy-4-methylpyrido[2,3-d]pyrimidin-7(8H)-one (27 mg).
$^1$H-NMR ($CD_3OD$) δ value: 2.42 (s, 6H), 3.07 (s, 3H), 3.71-3.79 (m, 2H), 4.56 (s, 2H), 5.59 (s, 1H), 7.26-7.32 (m, 1H), 7.36-7.48 (m, 3H), 8.81 (s, 1H).

Example 144

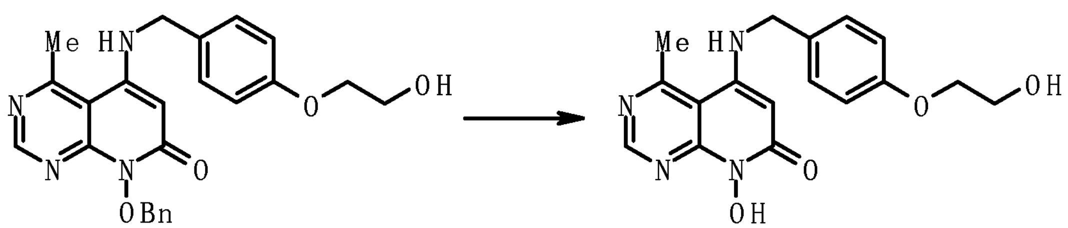

To 8-(benzyloxy)-5-((4-(2-hydroxyethoxy)benzyl)amino)-4-methylpyrido[2,3-d]pyrimidin-7(8H)-one (15 mg),

20% palladium hydroxide-carbon (8 mg), methanol (5.0 mL) and dioxane (5.0 mL) were added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-5-((4-(2-hydroxyethoxy)benzyl)amino)-4-methylpyrido[2,3-d]pyrimidin-7(8H)-one (8 mg).
$^1$H-NMR (DMSO-$d_6$) δ value: 2.98 (s, 3H), 3.64-3.75 (m, 2H), 3.92-4.00 (m, 2H), 4.32-4.42 (m, 2H), 4.80-4.88 (m, 1H), 5.42 (s, 1H), 6.72-6.81 (m, 1H), 6.93 (d, J=8.5 Hz, 2H), 7.33 (d, J=8.3 Hz, 2H), 8.79 (s, 1H), 10.52 (brs, 1H).

Example 145

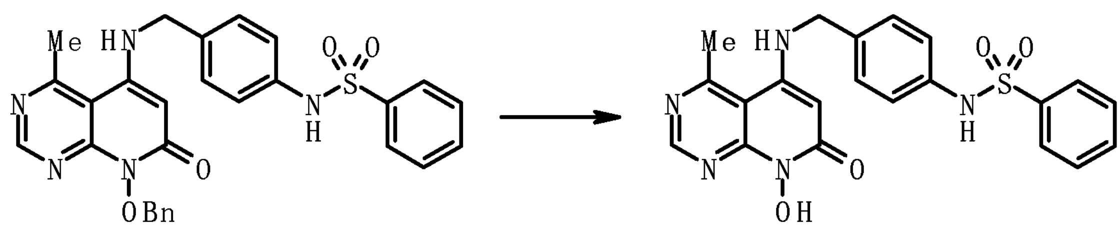

To N-(4-(((8-(benzyloxy)-4-methyl-7-oxo-7,8-dihydropyrido[2,3-d]pyrimidin-5-yl)amino)methyl)phenyl)benzenesulfonamide (40 mg), 20% palladium hydroxide-carbon (20 mg), methanol (5.0 mL) and dioxane (5.0 mL) were added, and the mixture was stirred at room temperature for 10 minutes in a hydrogen atmosphere. To the reaction mixture, 20% palladium hydroxide-carbon (20 mg) was added, and the mixture was stirred at room temperature for 10 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of N-(4-(((8-hydroxy-4-methyl-7-oxo-7,8-dihydropyrido[2,3-d]pyrimidin-5-yl)amino)methyl)phenyl)benzenesulfonamide (29 mg).
$^1$H-NMR (DMSO-$d_6$) δ value: 2.95 (s, 3H), 4.33 (d, J=5.4 Hz, 2H), 5.33 (s, 1H), 6.69-6.76 (m, 1H), 7.05 (d, J=8.5 Hz, 2H), 7.26 (d, J=8.6 Hz, 2H), 7.49-7.63 (m, 3H), 7.70-7.77 (m, 2H), 8.78 (s, 1H), 10.26 (brs, 1H), 10.57 (brs, 1H).

Example 146

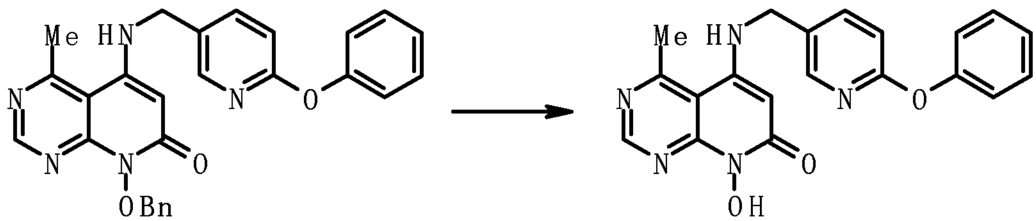

To 8-(benzyloxy)-4-methyl-5-(((6-phenoxypyridin-3-yl)methyl)amino)pyrido[2,3-d]pyrimidin-7(8H)-one (118 mg), 20% palladium hydroxide-carbon (59 mg), methanol (8.0 mL) and dioxane (8.0 mL) were added, and the mixture was stirred at room temperature for 15 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-4-methyl-5-(((6-phenoxypyridin-3-yl)methyl)amino)pyrido[2,3-d]pyrimidin-7(8H)-one (72 mg).
$^1$H-NMR (DMSO-$d_6$) δ value: 2.97 (s, 3H), 4.45 (d, J=5.4 Hz, 2H), 5.49 (s, 1H), 6.76-6.83 (m, 1H), 7.02 (d, J=8.5 Hz, 1H), 7.08-7.15 (m, 2H), 7.17-7.24 (m, 1H), 7.37-7.45 (m, 2H), 7.91 (dd, J=8.6, 2.4 Hz, 1H), 8.22 (d, J=2.2 Hz, 1H), 8.79 (s, 1H), 10.59 (brs, 1H).

Example 147

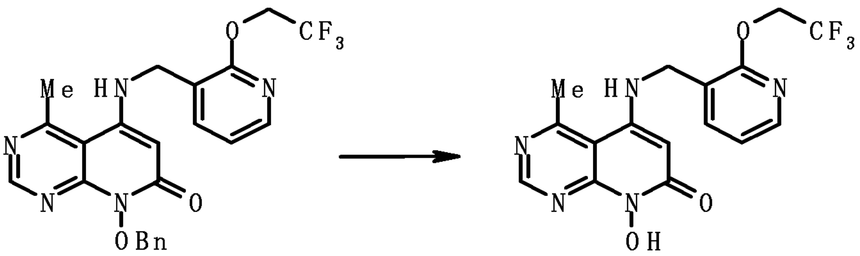

To 8-(benzyloxy)-4-methyl-5-(((2-(2,2,2-trifluoroethoxy)pyridin-3-yl)methyl)amino)pyrido[2,3-d]pyrimidin-7(8H)-one (122 mg), 20% palladium hydroxide-carbon (61 mg), methanol (6.0 mL) and dioxane (3.0 mL) were added, and the mixture was stirred at room temperature for 15 minutes in a hydrogen atmosphere. Palladium hydroxide-carbon was filtered off and washed with a mixed solvent of methanol and dioxane. The filtrate and the washes were combined, and the solvent was distilled off under reduced pressure. The obtained residue was suspended in a mixed solvent of ethyl acetate and diisopropyl ether, and the deposit was collected by filtration to obtain a light brown solid of 8-hydroxy-4-methyl-5-(((2-(2,2,2-trifluoroethoxy)pyridin-3-yl)methyl)amino)pyrido[2,3-d]pyrimidin-7(8H)-one (75 mg).
$^1$H-NMR (DMSO-$d_6$) $\delta$ value: 2.98 (s, 3H), 4.42 (d, J=5.4 Hz, 2H), 5.10 (q, J=9.0 Hz, 2H), 5.32 (s, 1H), 6.73-6.80 (m, 1H), 7.11 (dd, J=7.3, 5.1 Hz, 1H), 7.74-7.80 (m, 1H), 8.14 (dd, J=5.0, 1.8 Hz, 1H), 8.80 (s, 1H), 10.61 (brs, 1H).

Example 148

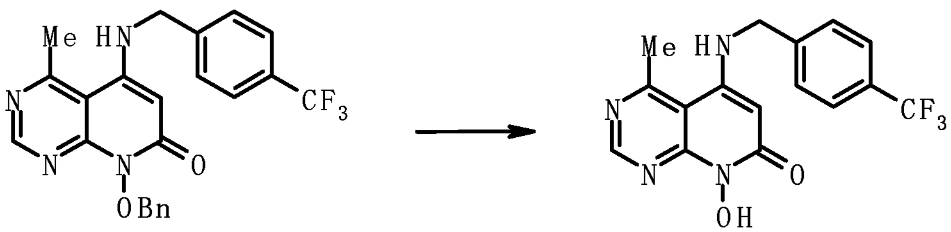

To 8-(benzyloxy)-4-methyl-5-((4-(trifluoromethyl)benzyl)amino)pyrido[2,3-d]pyrimidin-7(8H)-one (60 mg), 20% palladium hydroxide-carbon (30 mg) and methanol (60 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-4-methyl-5-((4-(trifluoromethyl)benzyl)amino)pyrido[2,3-d]pyrimidin-7(8H)-one (36 mg).
$^1$H-NMR (DMSO-$d_6$) $\delta$ value: 3.02 (s, 3H), 4.58 (d, J=5.1 Hz, 2H), 5.36 (s, 1H), 6.90-6.97 (m, 1H), 7.62-7.67 (m, 2H), 7.70-7.75 (m, 2H), 8.80 (s, 1H).

Example 149

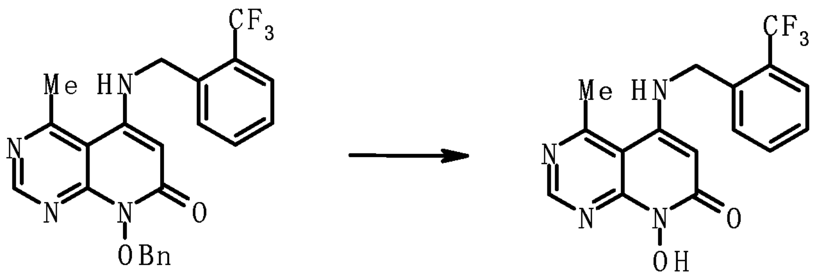

To 8-(benzyloxy)-4-methyl-5-((2-(trifluoromethyl)benzyl)amino)pyrido[2,3-d]pyrimidin-7(8H)-one (16 mg), 20% palladium hydroxide-carbon (8 mg) and methanol (16 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a dark yellow solid of 8-hydroxy-4-methyl-5-((2-(trifluoromethyl)benzyl)amino)pyrido[2,3-d]pyrimidin-7(8H)-one (12 mg).
$^1$H-NMR (DMSO-$d_6$) δ value: 2.98 (s, 3H), 4.60 (s, 2H), 5.23 (s, 1H), 6.60 (brs, 1H), 7.46-7.53 (m, 1H), 7.56-7.68 (m, 2H), 7.76-7.82 (m, 1H), 8.61 (brs, 1H).

Example 150

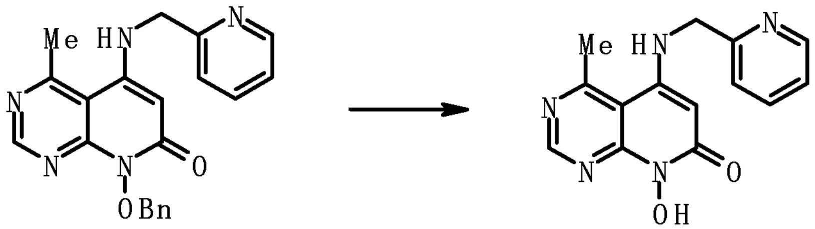

To 8-(benzyloxy)-4-methyl-5-(((pyridin-2-yl)methyl)amino)pyrido[2,3-d]pyrimidin-7(8H)-one (9 mg), 20% palladium hydroxide-carbon (4 mg) and methanol (9 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a light brown solid of 8-hydroxy-4-methyl-5-(((pyridin-2-yl)methyl)amino)pyrido[2,3-d]pyrimidin-7(8H)-one (4 mg).
$^1$H-NMR (DMSO-$d_6$) δ value: 3.03 (s, 3H), 4.50-4.60 (m, 2H), 5.46 (s, 1H), 7.00-7.10 (m, 1H), 7.29-7.37 (m, 1H), 7.44-7.51 (m, 1H), 7.77-7.86 (m, 1H), 8.57-8.61 (m, 1H), 8.74 (s, 1H).

Example 151

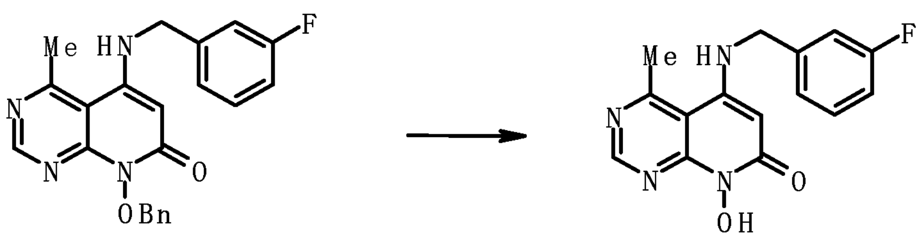

To 8-(benzyloxy)-5-((3-fluorobenzyl)amino)-4-methylpyrido[2,3-d]pyrimidin-7(8H)-one (30 mg), 20% palladium hydroxide-carbon (15 mg) and methanol (30 mL) were added, and the mixture was stirred at room temperature for 10 minutes in a hydrogen atmosphere. Palladium hydroxide-carbon was filtered off and washed with methanol. The filtrate

and the washes were combined, and the solvent was distilled off under reduced pressure. The obtained residue was suspended in a mixed solvent of 2-propanol and diisopropyl ether, and the deposit was collected by filtration to obtain a light brown solid of 5-((3-fluorobenzyl)amino)-8-hydroxy-4-methylpyrido[2,3-d]pyrimidin-7(8H)-one (10 mg).
$^{1}$H-NMR (DMSO-$d_6$) δ value: 3.00 (s, 3H), 4.49 (d, J=5.1 Hz, 2H), 5.38 (s, 1H), 6.79-6.88 (m, 1H), 7.06-7.13 (m, 1H), 7.23-7.30 (m, 2H), 7.37-7.45 (m, 1H), 8.77 (s, 1H).

Example 152

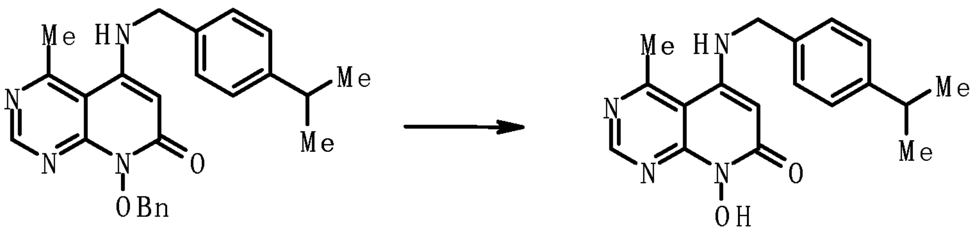

To 8-(benzyloxy)-5-((4-isopropylbenzyl)amino)-4-methylpyrido[2,3-d]pyrimidin-7(8H)-one (30 mg), 20% palladium hydroxide-carbon (15 mg) and methanol (30 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a light brown solid of 8-hydroxy-5-((4-isopropylbenzyl)amino)-4-methylpyrido[2,3-d]pyrimidin-7(8H)-one (16 mg).
$^{1}$H-NMR (DMSO-$d_6$) δ value: 1.19 (d, J=7.1 Hz, 6H), 2.82-2.92 (m, 1H), 2.98 (s, 3H), 4.40 (d, J=5.1 Hz, 2H), 5.42 (s, 1H), 6.71-6.79 (m, 1H), 7.21-7.26 (m, 2H), 7.31-7.37 (m, 2H), 8.73 (s, 1H).

Example 153

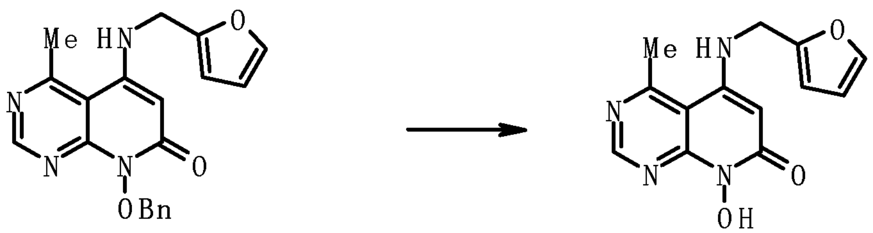

To 8-(benzyloxy)-5-((2-furylmethyl)amino)-4-methylpyrido[2,3-d]pyrimidin-7(8H)-one (28 mg), 20% palladium hydroxide-carbon (15 mg) and methanol (30 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a light brown solid of 5-((2-furylmethyl)amino)-8-hydroxy-4-methylpyrido[2,3-d]pyrimidin-7(8H)-one (9 mg).
$^{1}$H-NMR (DMSO-$d_6$) δ value: 2.91 (s, 3H), 4.43 (d, J=3.7 Hz, 2H), 5.65 (s, 1H), 6.39-6.46 (m, 2H), 6.58-6.69 (m, 1H), 7.63 (s, 1H), 8.74 (s, 1H).

Example 154

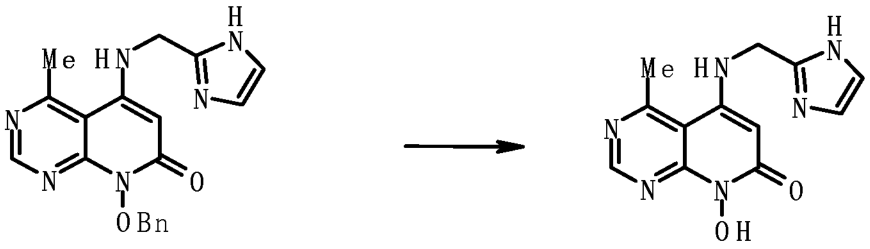

To 8-(benzyloxy)-5-(((1H-imidazol-2-yl)methyl)amino)-4-methylpyrido[2,3-d]pyrimidin-7(8H)-one (13 mg), 20% palladium hydroxide-carbon (6 mg) and methanol (30 mL) were added, and the mixture was stirred at room temperature for 10 minutes in a hydrogen atmosphere. To the reaction mixture, 20% palladium hydroxide-carbon (6 mg) was added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a light brown solid of 8-hydroxy-5-(((1H-imidazol-2-yl)methyl)amino)-4-methylpyrido[2,3-d]pyrimidin-7(8H)-one (2 mg).
$^1$H-NMR (DMSO-$d_6$) δ value: 2.99 (s, 3H), 4.41 (s, 2H), 5.56 (s, 1H), 6.69-6.87 (m, 1H), 6.88-7.05 (m, 2H), 8.72 (s, 1H).

Example 155

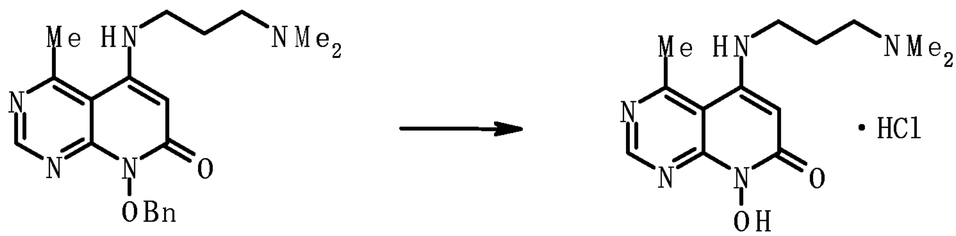

To 8-(benzyloxy)-5-((3-(dimethylamino)propyl)amino)-4-methylpyrido[2,3-d]pyrimidin-7(8H)-one (30 mg), 20% palladium hydroxide-carbon (15 mg) and methanol (30 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. To the obtained residue, methanol and a 2.9 mol/L solution of hydrogen chloride in ethanol were added, and the solvent was distilled off under reduced pressure. The obtained residue was suspended in methanol, and the deposit was collected by filtration to obtain a light brown solid of 5-((3-(dimethylamino)propyl)amino)-8-hydroxy-4-methylpyrido[2,3-d]pyrimidin-7(8H)-one hydrochloride (13 mg).
$^1$H-NMR (DMSO-$d_6$) δ value: 1.96-2.06 (m, 2H), 2.77 (s, 6H), 2.93 (s, 3H), 3.10-3.19 (m, 2H), 3.22-3.29 (m, 2H), 5.63 (s, 1H), 6.23-6.31 (m, 1H), 8.80 (s, 1H), 9.86 (brs, 1H), 10.63 (s, 1H).

Example 156

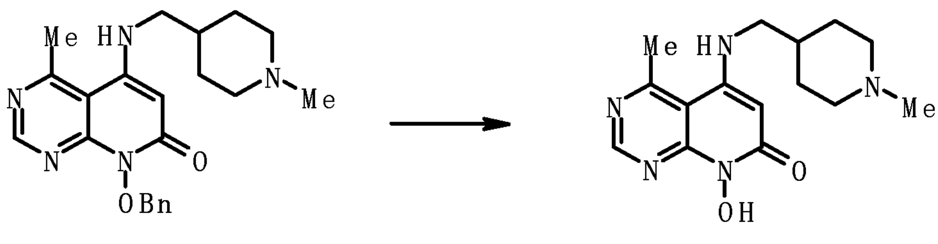

To 8-(benzyloxy)-4-methyl-5-(((1-methylpiperidin-4-yl)methyl)amino)pyrido[2,3-d]pyrimidin-7(8H)-one (16 mg), 20% palladium hydroxide-carbon (8 mg) and methanol (16 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and ethyl acetate, and the deposit was collected by filtration to obtain a yellow solid of 8-hydroxy-4-methyl-5-(((1-methylpiperidin-4-yl)methyl)amino)pyrido[2,3-d]pyrimidin-7(8H)-one (7 mg).
$^1$H-NMR (DMSO-$d_6$) δ value: 1.20-1.32 (m, 2H), 1.60-1.88 (m, 5H), 2.14 (s, 3H), 2.73-2.81 (m, 2H), 2.89 (s, 3H), 2.98-3.05 (m, 2H), 5.54 (s, 1H), 6.00-6.08 (m, 1H), 8.71 (s, 1H).

Example 157

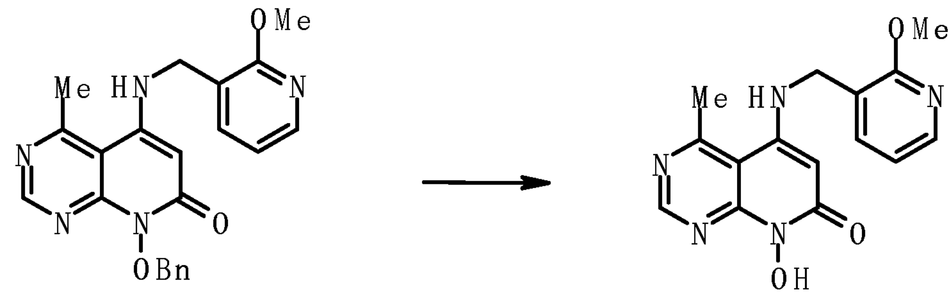

To 8-(benzyloxy)-5-(((2-methoxypyridin-3-yl)methyl)amino)-4-methylpyrido[2,3-d]pyrimidin-7(8H)-one (60 mg), 20% palladium hydroxide-carbon (30 mg) and methanol (60 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-5-(((2-methoxypyridin-3-yl)methyl)amino)-4-methylpyrido[2,3-d]pyrimidin-7(8H)-one (35 mg).
$^1$H-NMR (DMSO-$d_6$) δ value: 2.97 (s, 3H), 3.96 (s, 3H), 4.38 (d, J=4.9 Hz, 2H), 5.32 (s, 1H), 6.72-6.79 (m, 1H), 6.97 (dd, J=7.2, 5.0 Hz, 1H), 7.64-7.70 (m, 1H), 8.08-8.13 (m, 1H), 8.77 (s, 1H).

Example 158

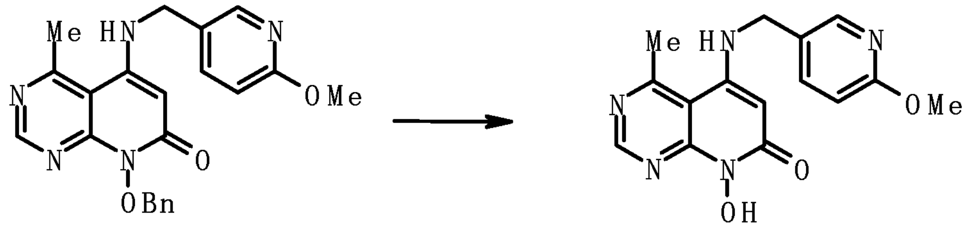

To 8-(benzyloxy)-5-(((2-methoxypiperidin-5-yl)methyl)amino)-4-methylpyrido[2,3-d]pyrimidin-7(8H)-one (40 mg), 20% palladium hydroxide-carbon (20 mg) and methanol (40 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-5-(((2-methoxypyridin-5-yl)methyl)amino)-4-methylpyrido[2,3-d]pyrimidin-7(8H)-one (12 mg).
$^1$H-NMR (DMSO-$d_6$) δ value: 2.96 (s, 3H), 3.84 (s, 3H), 4.39 (d, J=4.1 Hz, 2H), 5.48 (s, 1H), 6.67-6.75 (m, 1H), 6.82 (d, J=8.8 Hz, 1H), 7.74-7.80 (m, 1H), 8.22-8.26 (m, 1H), 8.74 (s, 1H).

Example 159

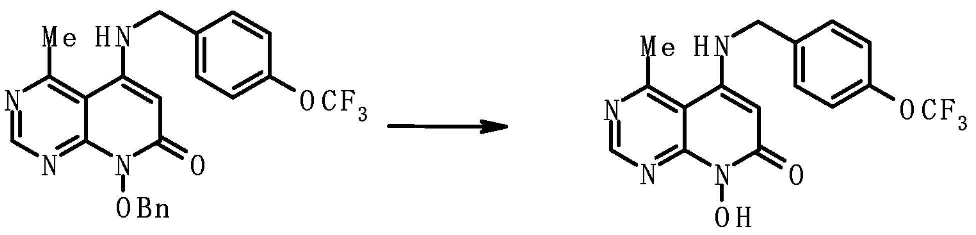

To 8-(benzyloxy)-4-methyl-5-((4-(trifluoromethoxy)benzyl)amino)pyrido[2,3-d]pyrimidin-7(8H)-one (70 mg), 20% palladium hydroxide-carbon (35 mg) and methanol (70 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-4-methyl-5-((4-(trifluoromethoxy)benzyl)amino)pyrido[2,3-d]pyrimidin-7(8H)-one (47 mg).
$^1$H-NMR (DMSO-$d_6$) δ value: 3.00 (s, 3H), 4.50 (d, J=4.9 Hz, 2H), 5.40 (s, 1H), 6.82-6.89 (m, 1H), 7.33-7.38 (m, 2H), 7.52-7.58 (m, 2H), 8.78 (s, 1H).

Example 160

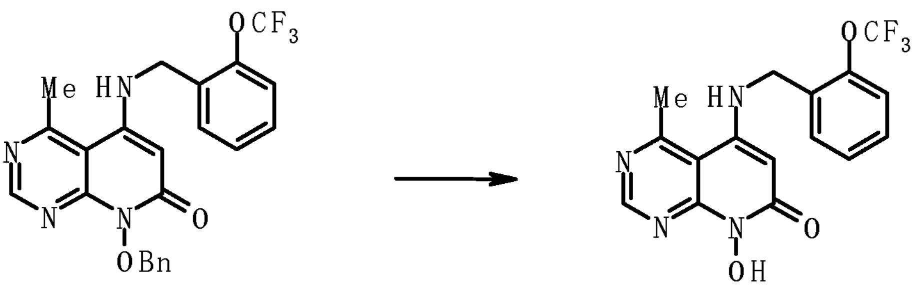

To 8-(benzyloxy)-4-methyl-5-((2-(trifluoromethoxy)benzyl)amino)pyrido[2,3-d]pyrimidin-7(8H)-one (30 mg), 20% palladium hydroxide-carbon (15 mg) and methanol (30 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a pale yellow solid of 8-hydroxy-4-methyl-5-((2-(trifluoromethoxy)benzyl)amino)pyrido[2,3-d]pyrimidin-7(8H)-one (14 mg).
$^1$H-NMR (DMSO-$d_6$) δ value: 2.98 (s, 3H), 4.52 (s, 2H), 5.28 (s, 1H), 6.72-6.85 (m, 1H), 7.34-7.46 (m, 3H), 7.50-7.55 (m, 1H), 8.75 (s, 1H).

Example 161

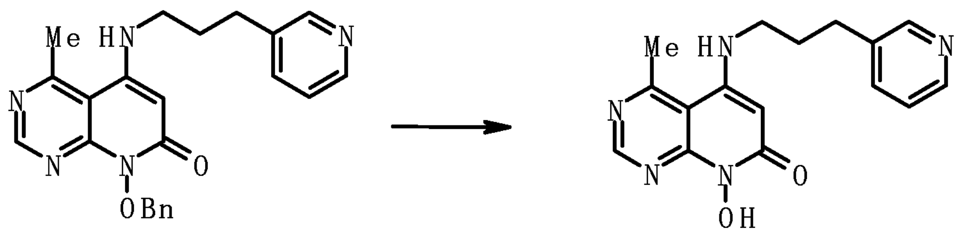

To 8-(benzyloxy)-4-methyl-5-((3-(pyridin-3-yl)propyl)amino)pyrido[2,3-d]pyrimidin-7(8H)-one (19 mg), 20% palladium hydroxide-carbon (10 mg) and methanol (20 mL) were added, and the mixture was stirred at room temperature for 20 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a yellow solid of 8-hydroxy-4-methyl-5-((3-(pyridin-3-yl)propyl)amino)pyrido[2,3-d]pyrimidin-7(8H)-one (5 mg).
$^1$H-NMR (DMSO-$d_6$) δ value: 1.94-2.03 (m, 2H), 2.72-2.78 (m, 2H), 2.88 (s, 3H), 3.08-3.19 (m, 2H), 5.55 (s, 1H), 5.91-6.03 (m, 1H), 7.29-7.36 (m, 1H), 7.65-7.72 (m, 1H), 8.39-8.43 (m, 1H), 8.47-8.51 (m, 1H), 8.67 (s, 1H).

Example 162

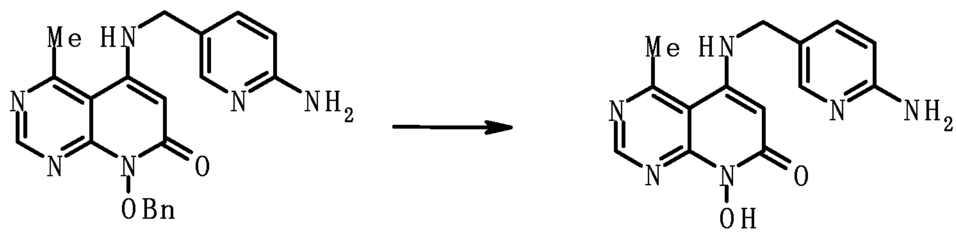

To 5-(((6-aminopyridin-3-yl)methyl)amino)-8-(benzyloxy)-4-methylpyrido[2,3-d]pyrimidin-7(8H)-one (30 mg), 20% palladium hydroxide-carbon (15 mg) and methanol (30 mL) were added, and the mixture was stirred at room temperature for 30 minutes in a hydrogen atmosphere. To the reaction mixture, 20% palladium hydroxide-carbon (15 mg) was added, and the mixture was stirred at room temperature for 30 minutes in a hydrogen atmosphere. Palladium hydroxide-carbon was filtered off and washed with methanol. The filtrate and the washes were combined, and the solvent was distilled off under reduced pressure. The obtained residue was suspended in 2-propanol, and the deposit was collected by filtration to obtain a yellow solid of 5-(((6-aminopyridin-3-yl)methyl)amino)-8-hydroxy-4-methylpyrido[2,3-d]pyrimidin-7(8H)-one (10 mg).
$^1$H-NMR (DMSO-$d_6$) δ value: 2.94 (s, 3H), 4.23 (d, J=4.9 Hz, 2H), 5.50 (s, 1H), 5.86 (s, 2H), 6.43 (d, J=8.3 Hz, 1H), 6.56-6.63 (m, 1H), 7.44 (dd, J=8.5, 2.4 Hz, 1H), 7.94-7.99 (m, 1H), 8.78 (s, 1H), 10.58 (brs, 1H).

Example 163

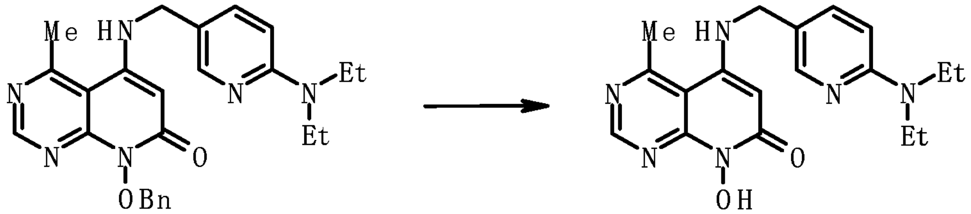

To 8-(benzyloxy)-5-(((6-(diethylamino)pyridin-3-yl)methyl)amino)-4-methylpyrido[2,3-d]pyrimidin-7(8H)-one (32 mg), 20% palladium hydroxide-carbon (16 mg) and methanol (32 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure.

The obtained residue was suspended in diisopropyl ether, and the deposit was collected by filtration to obtain a yellow solid of 5-(((6-(diethylamino)pyridin-3-yl)methyl)amino)-8-hydroxy-4-methylpyrido[2,3-d]pyrimidin-7(8H)-one (21 mg). $^1$H-NMR (DMSO-$d_6$) δ value: 1.08 (t, J=7.0 Hz, 6H), 2.89 (s, 3H), 3.46 (q, J=7.1 Hz, 4H), 4.14-4.22 (m, 2H), 5.62 (s, 1H), 6.09 (brs, 1H), 6.54-6.58 (m, 1H), 7.49-7.56 (m, 1H), 8.08-8.13 (m, 1H), 8.57 (s, 1H).

Example 164

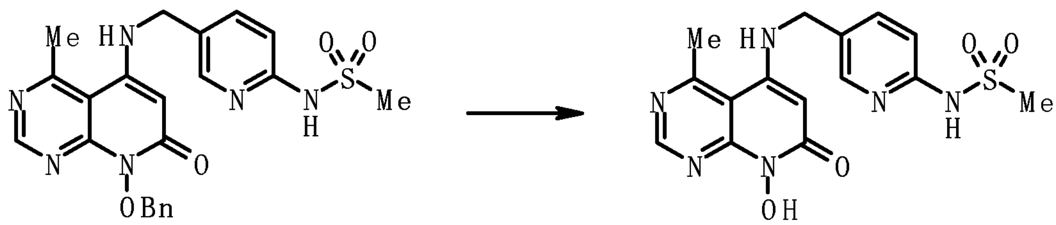

To N-(5-(((8-(benzyloxy)-4-methyl-7-oxo-7,8-dihydropyrido[2,3-d]pyrimidin-5-yl)amino)methyl)pyridin-2-yl)methanesulfonamide (15 mg), 20% palladium hydroxide-carbon (8 mg) and methanol (15 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in 2-propanol, and the deposit was collected by filtration to obtain a yellow solid of N-(5-(((8-hydroxy-4-methyl-7-oxo-7,8-dihydropyrido[2,3-d]pyrimidin-5-yl)amino)methyl)pyridin-2-yl)methanesulfonamide (5 mg). $^1$H-NMR (DMSO-$d_6$) δ value: 2.96 (s, 3H), 3.10 (s, 3H), 4.31-4.38 (m, 2H), 5.50 (s, 1H), 6.66-6.74 (m, 1H), 6.74-6.84 (m, 1H), 7.60-7.70 (m, 1H), 8.17-8.24 (m, 1H), 8.79 (s, 1H).

Example 165

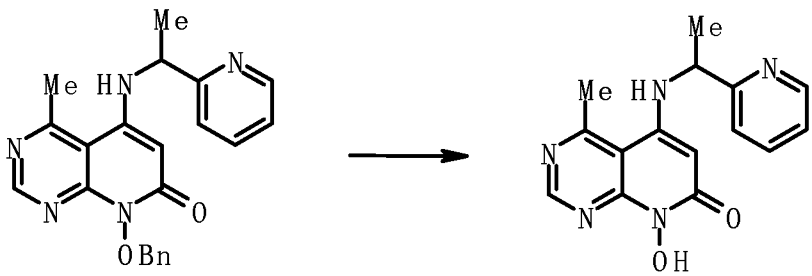

To 8-(benzyloxy)-4-methyl-5-((1-(pyridin-2-yl)ethyl)amino)pyrido[2,3-d]pyrimidin-7(8H)-one (78 mg), 20% palladium hydroxide-carbon (39 mg) and methanol (78 mL) were added, and the mixture was stirred at room temperature for 10 minutes in a hydrogen atmosphere. Palladium hydroxide-carbon was filtered off and washed with methanol. The filtrate and the washes were combined, and the solvent was distilled off under reduced pressure. The obtained residue was suspended in a mixed solvent of 2-propanol and diisopropyl ether, and the deposit was collected by filtration to obtain a yellow solid of 8-hydroxy-4-methyl-5-((1-(pyridin-2-yl)ethyl)amino)pyrido[2,3-d]pyrimidin-7(8H)-one (43 mg). $^1$H-NMR (DMSO-$d_6$) δ value: 1.55 (d, J=6.6 Hz, 3H), 3.06 (s, 3H), 4.70-4.78 (m, 1H), 5.40 (s, 1H), 6.75-6.84 (m, 1H), 7.28-7.36 (m, 1H), 7.49-7.56 (m, 1H), 7.78-7.85 (m, 1H), 8.56-8.61 (m, 1H), 8.79 (s, 1H).

[Industrial Applicability]

The heterocyclic compound of the present invention or the salt thereof has excellent anti-HIV activity and is therefore useful as an anti-HIV agent.

## Claims

1. A heterocyclic compound represented by a general formula, or a salt thereof:

[Formula 1]

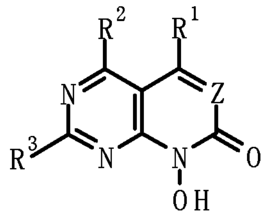

wherein $R^1$ and $R^2$ are the same or different and represent a hydrogen atom, a halogen atom, a cyano group, a nitro group, an optionally substituted $C_{1-12}$ alkyl group, an optionally substituted $C_{2-12}$ alkenyl group, an optionally substituted $C_{2-12}$ alkynyl group, an optionally substituted $C_{1-6}$ alkoxyl group, an optionally substituted acyl group, an optionally substituted $C_{1-6}$ alkylamino group, an optionally substituted di($C_{1-6}$ alkyl)amino group, an optionally substituted $C_{1-6}$ alkylthio group, an optionally substituted $C_{1-6}$ alkylsulfonyl group, an optionally substituted arylsulfonyl group, an optionally substituted $C_{3-8}$cycloalkyl group, an optionally substituted aryl group, an optionally substituted heterocyclic group, an optionally substituted carbamoyl group, an optionally protected amino group, an optionally protected carboxyl group, an optionally protected hydroxyl group or a general formula $X^1$-$Y^1$-$R^4$ (wherein $X^1$ represents a general formula $NR^5$ (wherein $R^5$ represents a hydrogen atom, an optionally substituted $C_{1-6}$ alkyl group or an imino protecting group), an oxygen atom or a sulfur atom; $Y^1$ represents an optionally substituted $C_{1-6}$ alkylene group, an optionally substituted $C_{2-6}$ alkenylene group, an optionally substituted $C_{2-6}$ alkynylene group or a bond; $R^4$ represents an optionally substituted $C_{3-8}$ cycloalkyl group, an optionally substituted aryl group or an optionally substituted heterocyclic group);
$R^3$ represents a hydrogen atom, a halogen atom, a cyano group, a nitro group, an optionally substituted $C_{1-6}$ alkyl group, an optionally substituted $C_{1-6}$ alkoxyl group, an amino group, an optionally protected carboxyl group or an optionally protected hydroxyl group;
Z represents a nitrogen atom or a general formula $CR^6$ (wherein $R^6$ represents a hydrogen atom, a halogen atom, a cyano group, a nitro group, an optionally substituted $C_{1-12}$ alkyl group, an optionally substituted $C_{2-12}$ alkenyl group, an optionally substituted $C_{2-12}$ alkynyl group, an optionally substituted $C_{1-6}$ alkoxyl group, an optionally substituted acyl group, an optionally substituted $C_{1-6}$ alkylamino group, an optionally substituted di($C_{1-6}$ alkyl)amino group, an optionally substituted $C_{1-6}$ alkylthio group, an optionally substituted $C_{1-6}$ alkylsulfonyl group, an optionally substituted arylsulfonyl group, an optionally substituted $C_{3-8}$cycloalkyl group, an optionally substituted aryl group, an optionally substituted heterocyclic group, an optionally substituted carbamoyl group, an optionally protected amino group, an optionally protected carboxyl group, an optionally protected hydroxyl group or a general formula $X^2$-$Y^2$-$R^7$ (wherein $X^2$ represents a general formula $NR^8$ (wherein $R^8$ represents a hydrogen atom, an optionally substituted $C_{1-6}$ alkyl group or an imino protecting group), an oxygen atom or a sulfur atom; $Y^2$ represents an optionally substituted $C_{1-6}$ alkylene group, an optionally substituted $C_{2-6}$ alkenylene group, an optionally substituted $C_{2-6}$ alkynylene group or a bond; $R^7$ represents an optionally substituted $C_{3-8}$cycloalkyl group, an optionally substituted aryl group or an optionally substituted heterocyclic group)).

2. The heterocyclic compound according to claim 1, or a salt thereof, wherein Z represents a general formula $CR^6$ (wherein $R^6$ represents a hydrogen atom, a halogen atom, a cyano group, a nitro group, an optionally substituted $C_{1-12}$ alkyl group, an optionally substituted $C_{2-12}$ alkenyl group, an optionally substituted $C_{2-12}$ alkynyl group, an optionally substituted $C_{1-6}$ alkoxyl group, an optionally substituted acyl group, an optionally substituted $C_{1-6}$ alkylamino group, an optionally substituted di($C_{1-6}$ alkyl)amino group, an optionally substituted $C_{1-6}$ alkylthio group, an optionally substituted $C_{1-6}$ alkylsulfonyl group, an optionally substituted arylsulfonyl group, an optionally substituted $C_{3-8}$cycloalkyl group, an optionally substituted aryl group, an optionally substituted heterocyclic group, an optionally substituted carbamoyl group, an optionally protected amino group, an optionally protected carboxyl group, an optionally protected hydroxyl group or a general formula $X^2$-$Y^2$-$R^7$ (wherein $X^2$ represents a general formula $NR^8$ (wherein $R^8$ represents a hydrogen atom, an optionally substituted $C_{1-6}$ alkyl group or an imino protecting group), an oxygen atom or a sulfur atom; $Y^2$ representsan optionally substituted $C_{1-6}$ alkylene group, an optionally substituted $C_{2-6}$ alkenylene group, an optionally substituted $C_{2-6}$ alkynylene group or a bond; $R^7$ represents an optionally substituted $C_{3-8}$ cycloalkyl group, an optionally substituted aryl group or an optionally substituted heterocyclic group)).

**3.** The heterocyclic compound according to claim 1 or 2, or a salt thereof, wherein $R^1$ represents a general formula $X^1$-$Y^1$-$R^4$ (wherein $X^1$ represents a general formula $NR^5$ (wherein $R^5$ represents a hydrogen atom, an optionally substituted $C_{1-6}$ alkyl group or an imino protecting group), an oxygen atom or a sulfur atom; $Y^1$ representsan optionally substituted $C_{1-6}$ alkylene group, an optionally substituted $C_{2-6}$ alkenylene group, an optionally substituted $C_{2-6}$ alkynylene group or a bond; $R^4$ represents an optionally substituted $C_{3-8}$cycloalkyl group, an optionally substituted aryl group or an optionally substituted heterocyclic group).

**4.** The heterocyclic compound according to any one of claims 1 to 3, or a salt thereof, wherein $R^2$ and $R^6$ are the same or different and represent a hydrogen atom, a halogen atom, a cyano group, a nitro group, an optionally substituted $C_{1-12}$ alkyl group, an optionally substituted $C_{1-6}$ alkoxyl group, an optionally substituted $C_{1-6}$ alkylamino group, an optionally substituted di($C_{1-6}$ alkyl)amino group, an optionally substituted $C_{1-6}$ alkylthio group, an optionally substituted aryl group, an optionally substituted heterocyclic group, an optionally substituted carbamoyl group, an optionally protected amino group, an optionally protected carboxyl group or an optionally protected hydroxyl group.

**5.** The heterocyclic compound according to any one of claims 1 to 3, or a salt thereof, wherein $R^2$ and $R^6$ are the same or different and represent a hydrogen atom, an optionally substituted $C_{1-6}$ alkyl group, an optionally substituted aryl group, an optionally substituted heterocyclic group, an optionally protected amino group or an optionally protected hydroxyl group.

**6.** The heterocyclic compound according to any one of claims 1 to 5, or a salt thereof, wherein $X^1$ represents a general formula $NR^5$ (wherein $R^5$ represents a hydrogen atom, an optionally substituted $C_{1-6}$ alkyl group or an imino protecting group).

**7.** The heterocyclic compound according to any one of claims 1 to 6, or a salt thereof, wherein $Y^1$ represents an optionally substituted $C_{1-6}$ alkylene group or a bond.

**8.** The heterocyclic compound according to any one of claims 1 to 7, or a salt thereof, wherein $R^4$ represents an optionally substituted aryl group or an optionally substituted heterocyclic group.

**9.** An anti-HIV agent comprising the heterocyclic compound according to any one of claims 1 to 8, or a salt thereof.

**INTERNATIONAL SEARCH REPORT**

International application No. PCT/JP2013/052077

A. CLASSIFICATION OF SUBJECT MATTER
*C07D471/04*(2006.01)i, *A61K31/519*(2006.01)i, *A61P31/18*(2006.01)i

According to International Patent Classification (IPC) or to both national classification and IPC

B. FIELDS SEARCHED

Minimum documentation searched (classification system followed by classification symbols)
C07D471/04, A61K31/519, A61P31/18

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 2009-543867 A (Merck & Co., Inc.), 10 December 2009 (10.12.2009), claims 1 to 19; examples & US 2010/0056516 A1 & EP 2044068 A & WO 2008/010964 A1 | 1-9 |
| Y | WO 2011/075747 A1 (GLAXOSMITHKLINE LLC), 23 June 2011 (23.06.2011), claims 1 to 9; examples (Family: none) | 1-9 |
| Y | Thomas J. DELIA et al, Fused Pyrimidines. 7. Nucleosides of Pyrimidopyrimidinediones and Pteridinediones as Potential Chemotherapeutic Agents, Journal of Heterocyclic Chemistry, 1997, 34, 1581-1585 | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 February, 2013 (14.02.13) | 26 February, 2013 (26.02.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2013/052077

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2005/105097 A2 (GPC BIOTECH AG), 10 November 2005 (10.11.2005), claims 1 to 18 (Family: none) | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Antibiotics & Chemotherapy,* 2011, vol. 27, 25-31
- *Antibiotics & Chemotherapy,* 2004, vol. 20, 58-68
- *Antibiotics & Chemotherapy,* 2009, vol. 25, 40-53
- *Antibiotics & Chemotherapy,* 2009, vol. 25, 54-61
- **W. GREENE et al.** Protective Groups in Organic Synthesis. John Wiley & Sons, INC, 2007, 696-926
- **W. GREENE et al.** Protective Groups in Organic Synthesis. John Wiley & Sons, INC, 2007, 696-868
- **W. GREENE et al.** Protective Groups in Organic Synthesis. John Wiley & Sons, INC, 2007, 16-299
- **W. GREENE et al.** Protective Groups in Organic Synthesis. John Wiley & Sons, INC, 2007, 533-643
- Protective Groups in Organic Synthesis. John Wiley & Sons, INC, 2007, 16-299
- The Fourth Series of Experimental Chemistry. Maruzen Co., Ltd, 1992, vol. 20, 279-282
- New Experimental Chemistry. Maruzen Co., Ltd, 1977, vol. 15, 808-811
- Protective Groups in Organic Synthesis. John Wiley & Sons, INC, 2007, 696-926
- The Fourth Series of Experimental Chemistry. Maruzen Co., Ltd, 1992, vol. 20, 424-427, 466-467
- *Tetrahedron,* 2002, vol. 58, 3323-3328
- *Journal of Clinical Microbiology,* 2007, vol. 45, 477-487